(19) Europäisches Patentamt
European Patent Office
Office européen des brevets

(11) EP 2 603 596 B1

(12) **EUROPEAN PATENT SPECIFICATION**

(45) Date of publication and mention of the grant of the patent:
**07.10.2020 Bulletin 2020/41**

(21) Application number: **11746730.8**

(22) Date of filing: **05.08.2011**

(51) Int Cl.:
***C12P 7/10*** *(2006.01)* ***C12P 19/14*** *(2006.01)*
***C12N 9/42*** *(2006.01)* ***C12P 19/02*** *(2006.01)*

(86) International application number:
**PCT/US2011/046795**

(87) International publication number:
**WO 2012/021410 (16.02.2012 Gazette 2012/07)**

(54) **COMPOSITIONS COMPRISING A POLYPEPTIDE HAVING CELLULOLYTIC ENHANCING ACTIVITY AND A LIQUOR AND USES THEREOF**

ZUSAMMENSETZUNGEN MIT EINEM POLYPEPTID MIT ZELLULOLYSEVERSTÄRKENDER WIRKUNG UND EINER LAUGE SOWIE IHRE VERWENDUNG

COMPOSITIONS CONTENANT UN POLYPEPTIDE À ACTIVITÉ CELLULOLYTIQUE RENFORCÉE ET UNE SOLUTION AQUEUSE ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**12.08.2010 US 373170 P**
**12.08.2010 US 373166 P**
**12.08.2010 US 373157 P**
**12.08.2010 US 373150 P**
**12.08.2010 US 373145 P**
**12.08.2010 US 373128 P**
**12.08.2010 US 373124 P**
**12.08.2010 US 373210 P**

(43) Date of publication of application:
**19.06.2013 Bulletin 2013/25**

(73) Proprietors:
- **Novozymes, Inc.**
  **Davis, CA 95618 (US)**
- **Novozymes North America, Inc.**
  **Franklinton, North Carolina 27525 (US)**

(72) Inventors:
- **QUINLAN, Jason**
  **Woodland, California 95695 (US)**
- **XU, Feng**
  **Davis, California 95616 (US)**
- **SWEENEY, Matthew**
  **Sacramento, California 95814 (US)**
- **HIGGINS, Don**
  **Franklinton, North Carolina 27525 (US)**
- **XU, Hui**
  **Wake Forest, North Carolina 27587 (US)**

(74) Representative: **NZ EPO Representatives**
**Krogshoejvej 36**
**2880 Bagsvaerd (DK)**

(56) References cited:
**WO-A1-2011/002832** **WO-A2-2009/090480**
**US-A1- 2009 056 889** **US-A1- 2010 129 860**

- **HARRIS PAUL V ET AL: "Stimulation of Lignocellulosic Biomass Hydrolysis by Proteins of Glycoside Hydrolase Family 61: Structure and Function of a Large, Enigmatic Family", BIOCHEMISTRY, AMERICAN CHEMICAL SOCIETY, US, vol. 49, no. 15, 1 April 2010 (2010-04-01) , pages 3305-3316, XP002608645, ISSN: 0006-2960, DOI: 10.1021/BI100009P [retrieved on 2010-03-15]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 603 596 B1

SEQUENCE LISTING

<110> NOVOZYMES, INC.
NOVOZYMES NORTH AMERICA, INC.
Quinlan, Jason
Xu, Feng
Sweeney, Matt
Higgins, Don
Xu, Hui

<120> Compositions comprising a polypeptide having cellulolytic enhancing activity and a liquor and uses thereof

<130> 12022-WO-PCT

<150> US 61/373,124
<151> 2010-08-12

<150> US 61/373,128
<151> 2010-08-12

<150> US 61/373,145
<151> 2010-08-12

<150> US 61/373,150
<151> 2010-08-12

<150> US 61/373,157
<151> 2010-08-12

<150> US 61/373,166
<151> 2010-08-12

<150> US 61/373,170
<151> 2010-08-12

<150> US 61/373,210
<151> 2010-08-12

<160> 166

<170> PatentIn version 3.5

<210> 1
<211> 1846
<212> DNA
<213> Thielavia terrestris

<400> 1

```
aattgaagga gggagtggcg gagtggccac caagtcaggc ggctgtcaac taaccaagga      60
tgggaacagt tcggctcgcc ttgcccgagg gcagcgttcc ctgatgggga cgaaccatgg     120
gactggggtc agctgctgta taaaagttca aatcgatgat ctctcagatg gcgctgctgg     180
ggtgttctgc gcttttccat cctcgcaacc tggtatccca ctagtccagc gttcggcacc     240
atgaagtcgt tcaccattgc cgccttggca gccctatggg cccaggaggc cgccgcccac     300
gcgaccttcc aggacctctg gattgatgga gtcgactacg gctcgcaatg tgtccgcctc     360
ccggcgtcca actcccccgt caccaatgtt gcgtccgacg atatccgatg caatgtcggc     420
acctcgaggc ccaccgtcaa gtgcccggtc aaggccggct ccacggtcac gatcgagatg     480
```

caccaggttc gcacgcctct ctgcgtaggc cccccagcta ctatatggca ctaacacgac 540
ctccagcaac ctggcgaccg gtcttgcgcc aacgaggcta tcggcggcga ccactacggc 600
cccgtaatgg tgtacatgtc caaggtcgat gacgcggtga cagccgacgg ttcatcgggc 660
tggttcaagg tgttccagga cagctgggcc aagaacccgt cgggttcgac gggcgacgac 720
gactactggg gcaccaagga cctcaactcg tgctgcggca agatgaacgt caagatcccc 780
gaagacatcg agccgggcga ctacctgctc cgcgccgagg ttatcgcgct gcacgtggcc 840
gccagctcgg gcggcgcgca gttctacatg tcctgctacc agctgaccgt gacgggctcc 900
ggcagcgcca cccccctcgac cgtgaatttc ccgggcgcct actcggccag cgacccgggc 960
atcctgatca acatccacgc gcccatgtcg acctacgtcg tcccgggccc gaccgtgtac 1020
gcgggcggct cgaccaagtc ggctggcagc tcctgctccg gctgcgaggc gacctgcacg 1080
gttggttccg gccccagcgc gacactgacg cagcccacct ccaccgcgac cgcgacctcc 1140
gcccctggcg gcggcggctc cggctgcacg gcggccaagt accagcagtg cggcggcacc 1200
ggctacactg ggtgcaccac ctgcgctgta agttccctcg tgatatgcag cggaacaccg 1260
tctggactgt tttgctaact cgcgtcgtag tccgggtcta cctgcagcgc cgtctcgcct 1320
ccgtactact cgcagtgcct ctaagccggg agcgcttgct cagcgggctg ctgtgaagga 1380
gctccatgtc cccatgccgc catggccgga gtaccgggct gagcgcccaa ttcttgtata 1440
tagttgagtt ttcccaatca tgaatacata tgcatctgca tggactgttg cgtcgtcagt 1500
ctacatcctt tgctccactg aactgtgaga ccccatgtca tccggaccat tcgatcggtg 1560
ctcgctctac catctcggtt gatgggtctg ggcttgagag tcactggcac gtcctcggcg 1620
gtaatgaaat gtggaggaaa gtgtgagctg tctgacgcac tcggcgctga tgagacgttg 1680
agcgcggccc acactggtgt tctgtaagcc agcacacaaa agaatactcc aggatggccc 1740
atagcggcaa atatacagta tcagggatgc aaaaagtgca aaagtaaggg gctcaatcgg 1800
ggatcgaacc cgagacctcg cacatgactt atttcaagtc aggggt 1846

<210> 2
<211> 326
<212> PRT
<213> Thielavia terrestris

<400> 2

```
Met Lys Ser Phe Thr Ile Ala Ala Leu Ala Ala Leu Trp Ala Gln Glu
1               5                   10                  15

Ala Ala Ala His Ala Thr Phe Gln Asp Leu Trp Ile Asp Gly Val Asp
            20                  25                  30

Tyr Gly Ser Gln Cys Val Arg Leu Pro Ala Ser Asn Ser Pro Val Thr
        35                  40                  45
```

```
Asn Val Ala Ser Asp Asp Ile Arg Cys Asn Val Gly Thr Ser Arg Pro
    50                  55                  60

Thr Val Lys Cys Pro Val Lys Ala Gly Ser Thr Val Thr Ile Glu Met
65                  70                  75                  80

His Gln Gln Pro Gly Asp Arg Ser Cys Ala Asn Glu Ala Ile Gly Gly
                85                  90                  95

Asp His Tyr Gly Pro Val Met Val Tyr Met Ser Lys Val Asp Asp Ala
            100                 105                 110

Val Thr Ala Asp Gly Ser Ser Gly Trp Phe Lys Val Phe Gln Asp Ser
        115                 120                 125

Trp Ala Lys Asn Pro Ser Gly Ser Thr Gly Asp Asp Asp Tyr Trp Gly
    130                 135                 140

Thr Lys Asp Leu Asn Ser Cys Cys Gly Lys Met Asn Val Lys Ile Pro
145                 150                 155                 160

Glu Asp Ile Glu Pro Gly Asp Tyr Leu Leu Arg Ala Glu Val Ile Ala
                165                 170                 175

Leu His Val Ala Ala Ser Ser Gly Gly Ala Gln Phe Tyr Met Ser Cys
            180                 185                 190

Tyr Gln Leu Thr Val Thr Gly Ser Gly Ser Ala Thr Pro Ser Thr Val
        195                 200                 205

Asn Phe Pro Gly Ala Tyr Ser Ala Ser Asp Pro Gly Ile Leu Ile Asn
    210                 215                 220

Ile His Ala Pro Met Ser Thr Tyr Val Val Pro Gly Pro Thr Val Tyr
225                 230                 235                 240

Ala Gly Gly Ser Thr Lys Ser Ala Gly Ser Ser Cys Ser Gly Cys Glu
                245                 250                 255

Ala Thr Cys Thr Val Gly Ser Gly Pro Ser Ala Thr Leu Thr Gln Pro
            260                 265                 270

Thr Ser Thr Ala Thr Ala Thr Ser Ala Pro Gly Gly Gly Gly Ser Gly
        275                 280                 285

Cys Thr Ala Ala Lys Tyr Gln Gln Cys Gly Gly Thr Gly Tyr Thr Gly
    290                 295                 300
```

Cys Thr Thr Cys Ala Ser Gly Ser Thr Cys Ser Ala Val Ser Pro Pro
305 310 315 320

Tyr Tyr Ser Gln Cys Leu
325

<210> 3
<211> 880
<212> DNA
<213> Thielavia terrestris

<400> 3

accccgggat cactgcccct aggaaccagc acacctcggt ccaatcatgc ggttcgacgc 60

cctctccgcc ctcgctcttg cgccgcttgt ggctggccac ggcgccgtga ccagctacat 120

catcggcggc aaaacctatc ccggctacga gggcttctcg cctgcctcga gcccgccgac 180

gatccagtac cagtggcccg actacaaccc gaccctgagc gtgaccgacc cgaagatgcg 240

ctgcaacggc ggcacctcgg cagagctcag cgcgcccgtc caggccggcg agaacgtgac 300

ggccgtctgg aagcagtgga cccaccagca aggccccgtc atggtctgga tgttcaagtg 360

ccccggcgac ttctcgtcgt gccacggcga cggcaagggc tggttcaaga tcgaccagct 420

gggcctgtgg ggcaacaacc tcaactcgaa caactggggc accgcgatcg tctacaagac 480

cctccagtgg agcaacccga tccccaagaa cctcgcgccg ggcaactacc tcatccgcca 540

cgagctgctc gccctgcacc aggccaacac gccgcagttc tacgccgagt gcgcccagct 600

ggtcgtctcc ggcagcggct ccgccctgcc cccgtccgac tacctctaca gcatccccgt 660

ctacgcgccc cagaacgacc ccggcatcac cgtgagtggg cttccgttcc gcggcgagct 720

ctgtggaaat cttgctgacg atgggctagg ttgacatcta caacggcggg cttacctcct 780

acaccccgcc cggcggcccc gtctggtctg gcttcgagtt ttaggcgcat tgagtcgggg 840

gctacgaggg gaaggcatct gttcgcatga gcgtgggtac 880

<210> 4
<211> 239
<212> PRT
<213> Thielavia terrestris

<400> 4

Met Arg Phe Asp Ala Leu Ser Ala Leu Ala Leu Ala Pro Leu Val Ala
1               5                   10                  15

Gly His Gly Ala Val Thr Ser Tyr Ile Ile Gly Gly Lys Thr Tyr Pro
            20                  25                  30

Gly Tyr Glu Gly Phe Ser Pro Ala Ser Ser Pro Pro Thr Ile Gln Tyr
        35                  40                  45

Gln Trp Pro Asp Tyr Asn Pro Thr Leu Ser Val Thr Asp Pro Lys Met

    50                  55                  60

Arg Cys Asn Gly Gly Thr Ser Ala Glu Leu Ser Ala Pro Val Gln Ala
65                  70                  75                  80

Gly Glu Asn Val Thr Ala Val Trp Lys Gln Trp Thr His Gln Gln Gly
                85                  90                  95

Pro Val Met Val Trp Met Phe Lys Cys Pro Gly Asp Phe Ser Ser Ser
            100                 105                 110

His Gly Asp Gly Lys Gly Trp Phe Lys Ile Asp Gln Leu Gly Leu Trp
        115                 120                 125

Gly Asn Asn Leu Asn Ser Asn Asn Trp Gly Thr Ala Ile Val Tyr Lys
    130                 135                 140

Thr Leu Gln Trp Ser Asn Pro Ile Pro Lys Asn Leu Ala Pro Gly Asn
145                 150                 155                 160

Tyr Leu Ile Arg His Glu Leu Leu Ala Leu His Gln Ala Asn Thr Pro
                165                 170                 175

Gln Phe Tyr Ala Glu Cys Ala Gln Leu Val Val Ser Gly Ser Gly Ser
            180                 185                 190

Ala Leu Pro Pro Ser Asp Tyr Leu Tyr Ser Ile Pro Val Tyr Ala Pro
        195                 200                 205

Gln Asn Asp Pro Gly Ile Thr Val Asp Ile Tyr Asn Gly Gly Leu Thr
    210                 215                 220

Ser Tyr Thr Pro Pro Gly Gly Pro Val Trp Ser Gly Phe Glu Phe
225                 230                 235

<210> 5
<211> 1000
<212> DNA
<213> Thielavia terrestris

<400> 5

```
ctcctgttcc tgggccaccg cttgttgcct gcactattgg tagagttggt ctattgctag      60
agttggccat gcttctcaca tcagtcctcg gctcggctgc cctgcttgct agcggcgctg     120
cggcacacgg cgccgtgacc agctacatca tcgccggcaa gaattacccg gggtgggtag     180
ctgattattg agggcgcatt caaggttcat accggtgtgc atggctgaca accggctggc     240
agataccaag gcttttctcc tgcgaactcg ccgaacgtca tccaatggca atggcatgac     300
tacaaccccg tcttgtcgtg cagcgactcg aagcttcgct gcaacggcgg cacgtcggcc     360
accctgaacg ccacggccgc accgggcgac accatcaccg ccatctgggc gcagtggacg     420
cacagccagg gccccatcct ggtgtggatg tacaagtgcc cgggctcctt cagctcctgt     480
gacggctccg gcgctggctg gttcaagatc gacgaggccg gcttccacgg cgacggcgtc     540
aaggtcttcc tcgacaccga gaacccgtcc ggctgggaca tcgccaagct cgtcggcggc     600
aacaagcagt ggagcagcaa ggtccccgag ggcctcgccc ccggcaacta cctcgtccgc     660
cacgagttga tcgccctgca ccaggccaac aacccgcagt tctacccgga gtgcgcccag     720
gtcgtcatca ccggctccgg caccgcgcag ccggatgcct catacaaggc ggctatcccc     780
ggctactgca accagaatga cccgaacatc aaggtgagat ccaggcgtaa tgcagtctac     840
tgctggaaag aaagtggtcc aagctaaacc gcgctccagg tgcccatcaa cgaccactcc     900
atccctcaga cctacaagat tcccggccct cccgtcttca agggcaccgc cagcaagaag     960
gcccgggact tcaccgcctg aagttgttga atcgatggag                           1000
```

<210> 6
<211> 258
<212> PRT
<213> Thielavia terrestris

<400> 6

```
Met Leu Leu Thr Ser Val Leu Gly Ser Ala Ala Leu Leu Ala Ser Gly
1               5                   10                  15

Ala Ala Ala His Gly Ala Val Thr Ser Tyr Ile Ile Ala Gly Lys Asn
            20                  25                  30

Tyr Pro Gly Tyr Gln Gly Phe Ser Pro Ala Asn Ser Pro Asn Val Ile
        35                  40                  45

Gln Trp Gln Trp His Asp Tyr Asn Pro Val Leu Ser Cys Ser Asp Ser
    50                  55                  60

Lys Leu Arg Cys Asn Gly Gly Thr Ser Ala Thr Leu Asn Ala Thr Ala
65                  70                  75                  80

Ala Pro Gly Asp Thr Ile Thr Ala Ile Trp Ala Gln Trp Thr His Ser
                85                  90                  95

Gln Gly Pro Ile Leu Val Trp Met Tyr Lys Cys Pro Gly Ser Phe Ser
            100                 105                 110

Ser Cys Asp Gly Ser Gly Ala Gly Trp Phe Lys Ile Asp Glu Ala Gly
        115                 120                 125

Phe His Gly Asp Gly Val Lys Val Phe Leu Asp Thr Glu Asn Pro Ser
    130                 135                 140
```

```
Gly Trp Asp Ile Ala Lys Leu Val Gly Gly Asn Lys Gln Trp Ser Ser
145                 150                 155                 160

Lys Val Pro Glu Gly Leu Ala Pro Gly Asn Tyr Leu Val Arg His Glu
                165                 170                 175

Leu Ile Ala Leu His Gln Ala Asn Asn Pro Gln Phe Tyr Pro Glu Cys
            180                 185                 190

Ala Gln Val Val Ile Thr Gly Ser Gly Thr Ala Gln Pro Asp Ala Ser
        195                 200                 205

Tyr Lys Ala Ala Ile Pro Gly Tyr Cys Asn Gln Asn Asp Pro Asn Ile
    210                 215                 220

Lys Val Pro Ile Asn Asp His Ser Ile Pro Gln Thr Tyr Lys Ile Pro
225                 230                 235                 240

Gly Pro Pro Val Phe Lys Gly Thr Ala Ser Lys Lys Ala Arg Asp Phe
                245                 250                 255

Thr Ala
```

<210> 7
<211> 681
<212> DNA
<213> Thielavia terrestris

<400> 7

```
atgctcgcaa acggtgccat cgtcttcctg gccgccgccc tcggcgtcag tggccactac      60
acctggccac gggttaacga cggcgccgac tggcaacagg tccgtaaggc ggacaactgg     120
caggacaacg gctacgtcgg ggatgtcacg tcgccacaga tccgctgttt ccaggcgacc     180
ccgtccccgg ccccatccgt cctcaacacc acggccggct cgaccgtgac ctactgggcc     240
aaccccgacg tctaccaccc cgggcctgtg cagttttaca tggcccgcgt gcccgatggc     300
gaggacatca actcgtggaa cggcgacggc gccgtgtggt tcaaggtgta cgaggaccat     360
cctacctttg gcgctcagct cacatggccc agcacgggca agagctcgtt cgcggttccc     420
atccccccgt gcatcaagtc cggctactac ctcctccggg cggagcaaat cggcctgcac     480
gtcgcccaga gcgtaggcgg agcgcagttc tacatctcat gcgcccagct cagcgtcacc     540
ggcggcggca gcaccgagcc gccgaacaag gtggccttcc ccggcgctta cagtgcgacg     600
gacccgggca ttctgatcaa catctactac cctgttccca cgtcctacca gaaccccggc     660
ccggccgtct tcagctgctg a                                               681
```

<210> 8
<211> 226
<212> PRT
<213> Thielavia terrestris

<400> 8

```
Met Leu Ala Asn Gly Ala Ile Val Phe Leu Ala Ala Ala Leu Gly Val
1               5                   10                  15

Ser Gly His Tyr Thr Trp Pro Arg Val Asn Asp Gly Ala Asp Trp Gln
            20                  25                  30

Gln Val Arg Lys Ala Asp Asn Trp Gln Asp Asn Gly Tyr Val Gly Asp
        35                  40                  45

Val Thr Ser Pro Gln Ile Arg Cys Phe Gln Ala Thr Pro Ser Pro Ala
    50                  55                  60

Pro Ser Val Leu Asn Thr Thr Ala Gly Ser Thr Val Thr Tyr Trp Ala
65                  70                  75                  80

Asn Pro Asp Val Tyr His Pro Gly Pro Val Gln Phe Tyr Met Ala Arg
                85                  90                  95

Val Pro Asp Gly Glu Asp Ile Asn Ser Trp Asn Gly Asp Gly Ala Val
            100                 105                 110

Trp Phe Lys Val Tyr Glu Asp His Pro Thr Phe Gly Ala Gln Leu Thr
        115                 120                 125

Trp Pro Ser Thr Gly Lys Ser Ser Phe Ala Val Pro Ile Pro Pro Cys
    130                 135                 140

Ile Lys Ser Gly Tyr Tyr Leu Leu Arg Ala Glu Gln Ile Gly Leu His
145                 150                 155                 160

Val Ala Gln Ser Val Gly Gly Ala Gln Phe Tyr Ile Ser Cys Ala Gln
                165                 170                 175

Leu Ser Val Thr Gly Gly Gly Ser Thr Glu Pro Pro Asn Lys Val Ala
            180                 185                 190

Phe Pro Gly Ala Tyr Ser Ala Thr Asp Pro Gly Ile Leu Ile Asn Ile
        195                 200                 205

Tyr Tyr Pro Val Pro Thr Ser Tyr Gln Asn Pro Gly Pro Ala Val Phe
    210                 215                 220

Ser Cys
```

225

<210> 9

<211> 960
<212> DNA
<213> Thielavia terrestris

<400> 9

```
atgaagggac ttttcagtgc cgccgccctc tccctggccg tcggccaggc ttcggcccat       60
tacatcttcc agcaactctc catcaacggg aaccagtttc cggtgtacca atatattcgc      120
aagaacacca attataacag tcccgttacc gatctcacgt ccgacgatct tcggtgcaat      180
gtcggcgccc agggtgctgg gacagacacc gtcacggtga aggccggcga ccagttcacc      240
ttcacccttg acacccctgt ttaccaccag gggcccatct ccatctacat gtccaaggcc      300
ccgggcgcgg cgtcagacta cgatggcagc ggcggctggt tcaagatcaa ggactggggc      360
ccgactttca acgccgacgg cacggccacc tgggacatgg ccggctcata cacctacaac      420
atcccgacct gcattcccga cggcgactat ctgctccgca tccagtcgct ggccatccac      480
aacccctggc cggcgggcat cccgcagttc tacatctcct gcgcccagat caccgtgacc      540
ggcggcggca acggcaaccc tggcccgacg gccctcatcc ccggcgcctt caaggacacc      600
gacccgggct acacggtgaa catctacacg aacttccaca actacacggt tcccggcccg      660
gaggtcttca gctgcaacgg cggcggctcg aacccgcccc cgccggtgag tagcagcacg      720
cccgcgacca cgacgctggt cacgtcgacg cgcaccacgt cctccacgtc ctccgcctcg      780
acgccggcct cgaccggcgg ctgcaccgtc gccaagtggg gccagtgcgg cggcaacggg      840
tacaccggct gcacgacctg cgcggccggg tccacctgca gcaagcagaa cgactactac      900
tcgcagtgct tgtaagggag gccgcaaagc atgaggtgtt tgaagaggag gagaggggtc      960
```

<210> 10
<211> 304
<212> PRT
<213> Thielavia terrestris

<400> 10

```
Met Lys Gly Leu Phe Ser Ala Ala Ala Leu Ser Leu Ala Val Gly Gln
1               5                   10                  15

Ala Ser Ala His Tyr Ile Phe Gln Gln Leu Ser Ile Asn Gly Asn Gln
            20                  25                  30

Phe Pro Val Tyr Gln Tyr Ile Arg Lys Asn Thr Asn Tyr Asn Ser Pro
        35                  40                  45

Val Thr Asp Leu Thr Ser Asp Asp Leu Arg Cys Asn Val Gly Ala Gln
    50                  55                  60

Gly Ala Gly Thr Asp Thr Val Thr Val Lys Ala Gly Asp Gln Phe Thr
65                  70                  75                  80
```

```
Phe Thr Leu Asp Thr Pro Val Tyr His Gln Gly Pro Ile Ser Ile Tyr
                85                  90                  95

Met Ser Lys Ala Pro Gly Ala Ala Ser Asp Tyr Asp Gly Ser Gly Gly
            100                 105                 110

Trp Phe Lys Ile Lys Asp Trp Gly Pro Thr Phe Asn Ala Asp Gly Thr
        115                 120                 125

Ala Thr Trp Asp Met Ala Gly Ser Tyr Thr Tyr Asn Ile Pro Thr Cys
    130                 135                 140

Ile Pro Asp Gly Asp Tyr Leu Leu Arg Ile Gln Ser Leu Ala Ile His
145                 150                 155                 160

Asn Pro Trp Pro Ala Gly Ile Pro Gln Phe Tyr Ile Ser Cys Ala Gln
                165                 170                 175

Ile Thr Val Thr Gly Gly Gly Asn Gly Asn Pro Gly Pro Thr Ala Leu
            180                 185                 190

Ile Pro Gly Ala Phe Lys Asp Thr Asp Pro Gly Tyr Thr Val Asn Ile
        195                 200                 205

Tyr Thr Asn Phe His Asn Tyr Thr Val Pro Gly Pro Glu Val Phe Ser
    210                 215                 220

Cys Asn Gly Gly Gly Ser Asn Pro Pro Pro Pro Val Ser Ser Ser Thr
225                 230                 235                 240

Pro Ala Thr Thr Thr Leu Val Thr Ser Thr Arg Thr Thr Ser Ser Thr
                245                 250                 255

Ser Ser Ala Ser Thr Pro Ala Ser Thr Gly Gly Cys Thr Val Ala Lys
            260                 265                 270

Trp Gly Gln Cys Gly Gly Asn Gly Tyr Thr Gly Cys Thr Thr Cys Ala
        275                 280                 285

Ala Gly Ser Thr Cys Ser Lys Gln Asn Asp Tyr Tyr Ser Gln Cys Leu
    290                 295                 300
```

<210> 11
<211> 954
<212> DNA
<213> Thielavia terrestris

<400> 11

```
atgaagggcc tcagcctcct cgccgctgcg tcggcagcga ctgctcatac catcttcgtg        60

cagctcgagt caggggggaac gacctatccg gtatcctacg gcatccggga ccctagctac      120
gacggtccca tcaccgacgt cacctccgac tcactggctt gcaatggtcc cccgaacccc       180
acgacgccgt ccccgtacat catcaacgtc accgccggca ccacggtcgc ggcgatctgg       240
aggcacaccc tcacatccgg ccccgacgat gtcatggacg ccagccacaa ggggccgacc       300
ctggcctacc tcaagaaggt cgatgatgcc ttgaccgaca cgggtatcgg cggcggctgg       360
ttcaagatcc aggaggccgg ttacgacaat ggcaattggg ctaccagcac ggtgatcacc       420
aacggtggct tccaatatat tgacatcccc gcctgcattc ccaacggcca gtatctgctc       480
cgcgccgaga tgatcgcgct ccacgccgcc agcacgcagg gtggtgccca gctctacatg       540
gagtgcgcgc agatcaacgt ggtgggcggc tccggcagcg ccagcccgca gacgtacagc       600
atcccgggca tctaccaggc aaccgacccg ggcctgctga tcaacatcta ctccatgacg       660
ccgtccagcc agtacaccat tccgggtccg cccctgttca cctgcagcgg cagcggcaac       720
aacggcggcg gcagcaaccc gtcgggcggg cagaccacga cggcgaagcc cacgacgacg       780
acggcggcga cgaccacctc ctccgccgct cctaccagca gccagggggg cagcagcggt       840
tgcaccgttc cccagtggca gcagtgcggt ggcatctcgt tcaccggctg caccacctgc       900
gcggcgggct acacctgcaa gtatctgaac gactattact cgcaatgcca gtaa             954
```

<210> 12
<211> 317
<212> PRT
<213> Thielavia terrestris

<400> 12

```
Met Lys Gly Leu Ser Leu Leu Ala Ala Ala Ser Ala Ala Thr Ala His
1               5                   10                  15

Thr Ile Phe Val Gln Leu Glu Ser Gly Gly Thr Thr Tyr Pro Val Ser
            20                  25                  30

Tyr Gly Ile Arg Asp Pro Ser Tyr Asp Gly Pro Ile Thr Asp Val Thr
        35                  40                  45

Ser Asp Ser Leu Ala Cys Asn Gly Pro Pro Asn Pro Thr Thr Pro Ser
    50                  55                  60

Pro Tyr Ile Ile Asn Val Thr Ala Gly Thr Thr Val Ala Ala Ile Trp
65                  70                  75                  80

Arg His Thr Leu Thr Ser Gly Pro Asp Asp Val Met Asp Ala Ser His
                85                  90                  95

Lys Gly Pro Thr Leu Ala Tyr Leu Lys Lys Val Asp Asp Ala Leu Thr
            100                 105                 110
```

```
Asp Thr Gly Ile Gly Gly Gly Trp Phe Lys Ile Gln Glu Ala Gly Tyr
        115                 120                 125

Asp Asn Gly Asn Trp Ala Thr Ser Thr Val Ile Thr Asn Gly Gly Phe
    130                 135                 140

Gln Tyr Ile Asp Ile Pro Ala Cys Ile Pro Asn Gly Gln Tyr Leu Leu
145                 150                 155                 160

Arg Ala Glu Met Ile Ala Leu His Ala Ala Ser Thr Gln Gly Gly Ala
                165                 170                 175

Gln Leu Tyr Met Glu Cys Ala Gln Ile Asn Val Val Gly Gly Ser Gly
            180                 185                 190

Ser Ala Ser Pro Gln Thr Tyr Ser Ile Pro Gly Ile Tyr Gln Ala Thr
        195                 200                 205

Asp Pro Gly Leu Leu Ile Asn Ile Tyr Ser Met Thr Pro Ser Ser Gln
    210                 215                 220

Tyr Thr Ile Pro Gly Pro Pro Leu Phe Thr Cys Ser Gly Ser Gly Asn
225                 230                 235                 240

Asn Gly Gly Gly Ser Asn Pro Ser Gly Gly Gln Thr Thr Thr Ala Lys
                245                 250                 255

Pro Thr Thr Thr Thr Ala Ala Thr Thr Thr Ser Ser Ala Ala Pro Thr
            260                 265                 270

Ser Ser Gln Gly Gly Ser Ser Gly Cys Thr Val Pro Gln Trp Gln Gln
        275                 280                 285

Cys Gly Gly Ile Ser Phe Thr Gly Cys Thr Thr Cys Ala Ala Gly Tyr
    290                 295                 300

Thr Cys Lys Tyr Leu Asn Asp Tyr Tyr Ser Gln Cys Gln
305                 310                 315
```

<210> 13
<211> 799
<212> DNA
<213> Thermoascus aurantiacus

<400> 13

```
atgtcctttt ccaagataat tgctactgcc ggcgttcttg cctctgcttc tctagtggct      60
ggccatggct tcgttcagaa catcgtgatt gatggtaaaa agtatgtcat tgcaagacgc     120
acataagcgg caacagctga caatcgacag ttatggcggg tatctagtga accagtatcc     180
atacatgtcc aatcctccag aggtcatcgc ctggtctact acggcaactg atcttggatt     240
tgtggacggt actggatacc aaaccccaga tatcatctgc catagggggcg ccaagcctgg    300
agccctgact gctccagtct ctccaggagg aactgttgag cttcaatgga ctccatggcc     360
tgattctcac catggcccag ttatcaacta ccttgctccg tgcaatggtg attgttccac     420
tgtggataag acccaattag aattcttcaa aattgccgag agcggtctca tcaatgatga     480
caatcctcct gggatctggg cttcagacaa tctgatagca gccaacaaca gctggactgt     540
caccattcca accacaattg cacctggaaa ctatgttctg aggcatgaga ttattgctct     600
tcactcagct cagaaccagg atggtgccca gaactatccc cagtgcatca atctgcaggt     660
cactggaggt ggttctgata accctgctgg aactcttgga acggcactct accacgatac     720
cgatcctgga attctgatca acatctatca gaaactttcc agctatatca tccctggtcc     780
tcctctgtat actggttaa                                                  799
```

<210> 14
<211> 249
<212> PRT
<213> Thermoascus aurantiacus

<400> 14

```
Met Ser Phe Ser Lys Ile Ile Ala Thr Ala Gly Val Leu Ala Ser Ala
1               5                   10                  15

Ser Leu Val Ala Gly His Gly Phe Val Gln Asn Ile Val Ile Asp Gly
            20                  25                  30

Lys Tyr Tyr Gly Gly Tyr Leu Val Asn Gln Tyr Pro Tyr Met Ser Asn
        35                  40                  45

Pro Pro Glu Val Ile Ala Trp Ser Thr Thr Ala Thr Asp Leu Gly Phe
    50                  55                  60

Val Asp Gly Thr Gly Tyr Gln Thr Pro Asp Ile Ile Cys His Arg Gly
65                  70                  75                  80

Ala Lys Pro Gly Ala Leu Thr Ala Pro Val Ser Pro Gly Gly Thr Val
                85                  90                  95

Glu Leu Gln Trp Thr Pro Trp Pro Asp Ser His His Gly Pro Val Ile
            100                 105                 110

Asn Tyr Leu Ala Pro Cys Asn Gly Asp Cys Ser Thr Val Asp Lys Thr
        115                 120                 125

Gln Leu Glu Phe Phe Lys Ile Ala Glu Ser Gly Leu Ile Asn Asp Asp
    130                 135                 140

Asn Pro Pro Gly Ile Trp Ala Ser Asp Asn Leu Ile Ala Ala Asn Asn
145                 150                 155                 160

Ser Trp Thr Val Thr Ile Pro Thr Thr Ile Ala Pro Gly Asn Tyr Val
                165                 170                 175

Leu Arg His Glu Ile Ile Ala Leu His Ser Ala Gln Asn Gln Asp Gly
            180                 185                 190

Ala Gln Asn Tyr Pro Gln Cys Ile Asn Leu Gln Val Thr Gly Gly Gly
        195                 200                 205

Ser Asp Asn Pro Ala Gly Thr Leu Gly Thr Ala Leu Tyr His Asp Thr
    210                 215                 220

Asp Pro Gly Ile Leu Ile Asn Ile Tyr Gln Lys Leu Ser Ser Tyr Ile
225                 230                 235                 240

Ile Pro Gly Pro Pro Leu Tyr Thr Gly
                245
```

<210> 15
<211> 1172
<212> DNA
<213> Trichoderma reesei

<400> 15

```
ggatctaagc cccatcgata tgaagtcctg cgccattctt gcagcccttg gctgtcttgc      60
cgggagcgtt ctcggccatg gacaagtcca aaacttcacg atcaatggac aatacaatca     120
gggtttcatt ctcgattact actatcagaa gcagaatact ggtcacttcc ccaacgttgc     180
tggctggtac gccgaggacc tagacctggg cttcatctcc cctgaccaat acaccacgcc     240
cgacattgtc tgtcacaaga acgcggcccc aggtgccatt tctgccactg cagcggccgg     300
cagcaacatc gtcttccaat ggggccctgg cgtctggcct cacccctacg gtcccatcgt     360
tacctacgtg gctgagtgca gcggatcgtg cacgaccgtg aacaagaaca acctgcgctg     420
ggtcaagatt caggaggccg gcatcaacta taacacccaa gtctgggcgc agcaggatct     480
gatcaaccag ggcaacaagt ggactgtgaa gatcccgtcg agcctcaggc ccggaaacta     540
tgtcttccgc catgaacttc ttgctgccca tggtgcctct agtgcgaacg gcatgcagaa     600
ctatcctcag tgcgtgaaca tcgccgtcac aggctcgggc acgaaagcgc tccctgccgg     660
aactcctgca actcagctct acaagcccac tgaccctggc atcttgttca acccttacac     720
aacaatcacg agctacacca tccctggccc agccctgtgg caaggctaga tccaggggta     780
cggtgttggc gttcgtgaag tcggagctgt tgacaaggat atctgatgat gaacggagag     840
gactgatggg cgtgactgag tgtatatatt tttgatgacc aaattgtata cgaaatccga     900
acgcatggtg atcattgttt atccctgtag tatattgtct ccaggctgct aagagcccac     960
cgggtgtatt acggcaacaa agtcaggaat ttgggtggca atgaacgcag gtctccatga    1020
atgtatatgt gaagaggcat cggctggcat gggcattacc agatataggc cctgtgaaac    1080
atatagtact tgaacgtgct actggaacgg atcataagca agtcatcaac atgtgaaaaa    1140
acactacatg taaaaaaaaa aaaaaaaaaa aa                                  1172
```

<210> 16
<211> 249
<212> PRT
<213> Trichoderma reesei

<400> 16

Met Lys Ser Cys Ala Ile Leu Ala Ala Leu Gly Cys Leu Ala Gly Ser
1               5                   10                  15

Val Leu Gly His Gly Gln Val Gln Asn Phe Thr Ile Asn Gly Gln Tyr
            20                  25                  30

Asn Gln Gly Phe Ile Leu Asp Tyr Tyr Tyr Gln Lys Gln Asn Thr Gly
        35                  40                  45

His Phe Pro Asn Val Ala Gly Trp Tyr Ala Glu Asp Leu Asp Leu Gly
    50                  55                  60

Phe Ile Ser Pro Asp Gln Tyr Thr Thr Pro Asp Ile Val Cys His Lys
65                  70                  75                  80

Asn Ala Ala Pro Gly Ala Ile Ser Ala Thr Ala Ala Ala Gly Ser Asn
                85                  90                  95

Ile Val Phe Gln Trp Gly Pro Gly Val Trp Pro His Pro Tyr Gly Pro
            100                 105                 110

Ile Val Thr Tyr Val Val Glu Cys Ser Gly Ser Cys Thr Thr Val Asn
        115                 120                 125

Lys Asn Asn Leu Arg Trp Val Lys Ile Gln Glu Ala Gly Ile Asn Tyr
    130                 135                 140

Asn Thr Gln Val Trp Ala Gln Gln Asp Leu Ile Asn Gln Gly Asn Lys
145                 150                 155                 160

Trp Thr Val Lys Ile Pro Ser Ser Leu Arg Pro Gly Asn Tyr Val Phe
                165                 170                 175

Arg His Glu Leu Leu Ala Ala His Gly Ala Ser Ser Ala Asn Gly Met
            180                 185                 190

Gln Asn Tyr Pro Gln Cys Val Asn Ile Ala Val Thr Gly Ser Gly Thr
        195                 200                 205

Lys Ala Leu Pro Ala Gly Thr Pro Ala Thr Gln Leu Tyr Lys Pro Thr
    210                 215                 220

Asp Pro Gly Ile Leu Phe Asn Pro Tyr Thr Thr Ile Thr Ser Tyr Thr
225                 230                 235                 240

Ile Pro Gly Pro Ala Leu Trp Gln Gly
                245

<210> 17
<211> 924
<212> DNA
<213> Myceliophthora thermophila

<400> 17

```
atgaagttca cctcgtccct cgctgtcctg gccgctgccg gcgcccaggc tcactgttag       60
tcgaccctcg aacccaacac ccccctcccc ccttttctcc tccatctcct cggcctcact      120
tagtagccgc tgacaacgac tagatacctt ccctagggcc ggcactggtg gctcgctctc      180
tggcgagtgg gaggtggtcc gcatgaccga gaaccattac tcgcacggcc cggtcaccga      240
tgtcaccagc cccgagatga cctgctatca gtccggcgtg cagggtgcgc cccagaccgt      300
ccaggtcaag gcgggctccc aattcacctt cagcgtggat ccctcgatcg gccaccccgg      360
ccctctccag ttctacatgg ctaaggtgcc gtcgggccag acggccgcca cctttgacgg      420
cacgggagcc gtgtggttca agatctacca agacggcccg aacggcctcg gcaccgacag      480
cattacctgg cccagcgccg gttcgtgact tcctccccac tcgctttttt ttttttattt      540
tttatttttt tttctttcgg aactcaagaa tctttctctc tctctcccgt ctttggcctt      600
gaacaacact aaaactcttc cttactgtat taattaggca aaaccgaggt ctcggtcacc      660
atccccagct gcatcgatga tggcgagtac ctgctccggg tcgagcacat cgcgctccac      720
agcgccagca gcgtgggcgg cgctcagttc tacattgcct gcgcccagct ctccgtcacc      780
ggcggctccg gcaccctcaa cacgggctcg ctcgtctccc tgcccggcgc ctacaaggcc      840
accgacccgg gcatcctctt ccagctctac tggcccatcc cgaccgagta catcaacccc      900
ggcccggccc ccgtctcttg ctaa                                             924
```

<210> 18
<211> 232
<212> PRT
<213> Myceliophthora thermophila

<400> 18

Met Lys Phe Thr Ser Ser Leu Ala Val Leu Ala Ala Ala Gly Ala Gln

```
1               5                   10                  15

Ala His Tyr Thr Phe Pro Arg Ala Gly Thr Gly Gly Ser Leu Ser Gly
            20                  25                  30

Glu Trp Glu Val Val Arg Met Thr Glu Asn His Tyr Ser His Gly Pro
        35                  40                  45

Val Thr Asp Val Thr Ser Pro Glu Met Thr Cys Tyr Gln Ser Gly Val
    50                  55                  60

Gln Gly Ala Pro Gln Thr Val Gln Val Lys Ala Gly Ser Gln Phe Thr
65                  70                  75                  80

Phe Ser Val Asp Pro Ser Ile Gly His Pro Gly Pro Leu Gln Phe Tyr
                85                  90                  95

Met Ala Lys Val Pro Ser Gly Gln Thr Ala Ala Thr Phe Asp Gly Thr
            100                 105                 110

Gly Ala Val Trp Phe Lys Ile Tyr Gln Asp Gly Pro Asn Gly Leu Gly
        115                 120                 125

Thr Asp Ser Ile Thr Trp Pro Ser Ala Gly Lys Thr Glu Val Ser Val
    130                 135                 140

Thr Ile Pro Ser Cys Ile Asp Asp Gly Glu Tyr Leu Leu Arg Val Glu
145                 150                 155                 160

His Ile Ala Leu His Ser Ala Ser Ser Val Gly Gly Ala Gln Phe Tyr
                165                 170                 175

Ile Ala Cys Ala Gln Leu Ser Val Thr Gly Gly Ser Gly Thr Leu Asn
            180                 185                 190

Thr Gly Ser Leu Val Ser Leu Pro Gly Ala Tyr Lys Ala Thr Asp Pro
        195                 200                 205

Gly Ile Leu Phe Gln Leu Tyr Trp Pro Ile Pro Thr Glu Tyr Ile Asn
    210                 215                 220

Pro Gly Pro Ala Pro Val Ser Cys
225                 230
```

<210> 19
<211> 854
<212> DNA

<213> Myceliophthora thermophila

<400> 19

```
atgaaggccc tctctctcct tgcggctgcc tcggcagtct ctgcgcatac catcttcgtc      60
cagctcgaag cagacggcac gaggtacccg gtctcgtacg ggatccggga cccaagctac     120
gacggcccca tcaccgacgt cacatccaac gacgttgctt gcaacggcgg gccgaacccg     180
acgacccccct ccagcgacgt catcaccgtc accgcgggca ccacggtcaa ggccatctgg    240
aggcacaccc tccaatccgg cccggacgat gtcatggacg ccagccacaa gggcccgacc     300
ctggcctacc tcaagaaggt cggcgatgcc accaaggact cgggcgtcgg cggtggctgg     360
ttcaagattc aggaggacgg ctacaacaac ggccagtggg gcaccagcac cgttatctcc     420
aacggcggcg agcactacat gtgagccatt cctccgagag aagaccaaga ctcttgacga     480
tctcgctgac ccgtgcaaca agtgacatcc cggcctgcat ccccgagggt cagtacctcc     540
tccgcgccga gatgatcgcc ctccacgcgg ccgggtcccc cggcggtgcc cagctctacg     600
taagcctctg cccttccccc cttcctcttg atcgaatcgg actgcccacc cccctttcg      660
actccgacta acaccgttgc cagatggaat gtgcccagat caacatcgtc ggcggctccg     720
gctcggtgcc cagctcgacc gtcagcttcc ccggcgcgta cagccccaac gacccgggtc     780
tcctcatcaa catctattcc atgtcgccct cgagctcgta caccatcccg ggcccgcccg     840
tcttcaagtg ctag                                                       854
```

<210> 20
<211> 235
<212> PRT
<213> Myceliophthora thermophila

<400> 20

```
Met Lys Ala Leu Ser Leu Leu Ala Ala Ala Ser Ala Val Ser Ala His
1               5                   10                  15

Thr Ile Phe Val Gln Leu Glu Ala Asp Gly Thr Arg Tyr Pro Val Ser
            20                  25                  30

Tyr Gly Ile Arg Asp Pro Ser Tyr Asp Gly Pro Ile Thr Asp Val Thr
        35                  40                  45

Ser Asn Asp Val Ala Cys Asn Gly Gly Pro Asn Pro Thr Thr Pro Ser
    50                  55                  60

Ser Asp Val Ile Thr Val Thr Ala Gly Thr Thr Val Lys Ala Ile Trp
65                  70                  75                  80

Arg His Thr Leu Gln Ser Gly Pro Asp Asp Val Met Asp Ala Ser His
                85                  90                  95

Lys Gly Pro Thr Leu Ala Tyr Leu Lys Lys Val Gly Asp Ala Thr Lys
            100                 105                 110

Asp Ser Gly Val Gly Gly Gly Trp Phe Lys Ile Gln Glu Asp Gly Tyr
        115                 120                 125

Asn Asn Gly Gln Trp Gly Thr Ser Thr Val Ile Ser Asn Gly Gly Glu
    130                 135                 140

His Tyr Ile Asp Ile Pro Ala Cys Ile Pro Glu Gly Gln Tyr Leu Leu
145                 150                 155                 160

Arg Ala Glu Met Ile Ala Leu His Ala Ala Gly Ser Pro Gly Gly Ala
                165                 170                 175

Gln Leu Tyr Met Glu Cys Ala Gln Ile Asn Ile Val Gly Gly Ser Gly
            180                 185                 190

Ser Val Pro Ser Ser Thr Val Ser Phe Pro Gly Ala Tyr Ser Pro Asn
        195                 200                 205

Asp Pro Gly Leu Leu Ile Asn Ile Tyr Ser Met Ser Pro Ser Ser Ser
    210                 215                 220

Tyr Thr Ile Pro Gly Pro Pro Val Phe Lys Cys
225                 230                 235
```

<210> 21
<211> 1242

<212> DNA
<213> Myceliophthora thermophila

<400> 21

```
atgaagtcct tcgccctcac cactctggcc gccctggccg gcaacgccgc cgctcacgcg      60
accttccagg ccctctgggt cgacggcgtc gactacggcg cgcagtgtgc ccgtctgccc     120
gcgtccaact ccccggtcac cgacgtgacc tccaacgcga tccgctgcaa cgccaacccg     180
tcgcccgctc ggggcaagtg cccggtcaag gccggctcga ccgttacggt cgagatgcat     240
caggtacgtt ggatgaatga aaggggaaag gaagcagagg cagaagggga aggcgaaggg     300
aaagaaaaag aaaaagaaat ggaaaagaaa aagaaatgga aaagaaaaag aaaaatgaaa     360
aagaaagtgg aaaccgtcag actaactggg gctcctcccc cccacccctc ctttgatatc     420
agcaacccgg tgaccggtcg tgcagcagcg aggcgatcgg cggggcgcac tacggccccg     480
tcatggtgta catgtccaag gtgtcggacg cggcgtcggc ggacgggtcg tcgggctggt     540
tcaaggtgtt cgaggacggc tgggccaaga acccgtccgg cgggtcgggc gacgacgact     600
actggggcac caaggacctg aactcgtgct gcgggaagat gaacgtcaag atccccgccg     660
acctgccctc gggcgactac ctgctccggg ccgaggccct cgcgctgcac acggcgggca     720
gcgccggcgg cgcccagttc tacatgacgt gctaccagct caccgtgacg ggctccggca     780
gcgccagccc gcccaccgtc tccttcccgg gcgcctacaa ggccaccgac ccgggcatcc     840
tcgtcaacat ccacgccccg ctgtccggct acaccgtgcc cggcccggcc gtctactccg     900
gcggctccac caagaaggcc ggcagcgcct gcaccggctg cgagtccacc tgcgccgtcg     960
gctccggccc caccgccacc gtctcccagt cgcccggttc caccgccacc tccgcccccg    1020
gcggcggcgg cggctgcacc gtccagaagt accagcagtg cggcggcgag ggctacaccg    1080
gctgcaccaa ctgcgcggta cgtttttcaa ccccgttttt ttttttcctt ccctacctta    1140
tttggttacc taattaatta ctttccggct gctgactttt tgctttagtc cggctctacc    1200
tgcagcgccg tctcgccgcc ctactactcg cagtgcgtct aa                       1242
```

<210> 22
<211> 323
<212> PRT
<213> Myceliophthora thermophila

<400> 22

```
Met Lys Ser Phe Ala Leu Thr Thr Leu Ala Ala Leu Ala Gly Asn Ala
1               5                   10                  15

Ala Ala His Ala Thr Phe Gln Ala Leu Trp Val Asp Gly Val Asp Tyr
            20                  25                  30

Gly Ala Gln Cys Ala Arg Leu Pro Ala Ser Asn Ser Pro Val Thr Asp
        35                  40                  45

Val Thr Ser Asn Ala Ile Arg Cys Asn Ala Asn Pro Ser Pro Ala Arg
    50                  55                  60

Gly Lys Cys Pro Val Lys Ala Gly Ser Thr Val Thr Val Glu Met His
65                  70                  75                  80

Gln Gln Pro Gly Asp Arg Ser Cys Ser Ser Glu Ala Ile Gly Gly Ala
                85                  90                  95

His Tyr Gly Pro Val Met Val Tyr Met Ser Lys Val Ser Asp Ala Ala
            100                 105                 110

Ser Ala Asp Gly Ser Ser Gly Trp Phe Lys Val Phe Glu Asp Gly Trp
        115                 120                 125

Ala Lys Asn Pro Ser Gly Gly Ser Gly Asp Asp Asp Tyr Trp Gly Thr
    130                 135                 140

Lys Asp Leu Asn Ser Cys Cys Gly Lys Met Asn Val Lys Ile Pro Ala
145                 150                 155                 160

Asp Leu Pro Ser Gly Asp Tyr Leu Leu Arg Ala Glu Ala Leu Ala Leu
```

165 170 175

His Thr Ala Gly Ser Ala Gly Gly Ala Gln Phe Tyr Met Thr Cys Tyr
180 185 190

Gln Leu Thr Val Thr Gly Ser Gly Ser Ala Ser Pro Pro Thr Val Ser
195 200 205

Phe Pro Gly Ala Tyr Lys Ala Thr Asp Pro Gly Ile Leu Val Asn Ile
210 215 220

His Ala Pro Leu Ser Gly Tyr Thr Val Pro Gly Pro Ala Val Tyr Ser
225 230 235 240

Gly Gly Ser Thr Lys Lys Ala Gly Ser Ala Cys Thr Gly Cys Glu Ser
245 250 255

Thr Cys Ala Val Gly Ser Gly Pro Thr Ala Thr Val Ser Gln Ser Pro
260 265 270

Gly Ser Thr Ala Thr Ser Ala Pro Gly Gly Gly Gly Gly Cys Thr Val
275 280 285

Gln Lys Tyr Gln Gln Cys Gly Gly Glu Gly Tyr Thr Gly Cys Thr Asn
290 295 300

Cys Ala Ser Gly Ser Thr Cys Ser Ala Val Ser Pro Pro Tyr Tyr Ser
305 310 315 320

Gln Cys Val

<210> 23
<211> 1253
<212> DNA
<213> Myceliophthora thermophila

<400> 23

atgaagcctt ttagcctcgt cgccctggcg accgccgtga gcggccatgc catcttccag 60
cgggtgtcgg tcaacgggca ggaccagggc cagctcaagg gggtgcgggc gccgtcgagc 120
aactccccga tccagaacgt caacgatgcc aacatggcct gcaacgccaa cattgtgtac 180
cacgacagca ccatcatcaa ggtgcccgcg ggagcccgcg tcggcgcgtg gtggcagcac 240
gtcatcggcg ggccgcaggg cgccaacgac ccggacaacc cgatcgcggc ctcccacaag 300
ggtatgatga tcgatgatgc ctctctcttc ccccgttctt gatggacagg cgatggctcc 360
caggaacacg cgtgactgac caccgaatcc aggccccatc caggtctacc tggccaaggt 420
ggacaacgcg gcgacggcgt cgccgtcggg cctcaggtgg ttcaaggtgg ccgagcgcgg 480

cctgaacaac ggcgtgtggg ccgtcgatga gctcatcgcc aacaacggct ggcactactt 540
cgacctgccg tcgtgcgtgg cccccggcca gtacctgatg cgcgtcgagc tgctcgccct 600
gcacagcgcc tcaagccccg gcggcgccca gttctacatg ggctgcgcac agatcgaagg 660
tgcgtcgatc tttgttctcc ttccgtgtcc tctctgatcc tttctctctt ctttttcttt 720
cttttactcc ctttccttcc atcttcggag aagcaacgaa gggggaaagg gatagaagag 780
aggaatgaga gacgacgaaa gagaggattg gggaaagaca agacagggaa aaaaagacaa 840
gaaaaaaaaa aaaaaaaaaa aacagagtga gctaacaaga acaatcagtc actggctccg 900
gcaccaactc gggctccgac tttgtctcgt tccccggcgc ctactcggcc aacgatccgg 960
gcatcttgct aagcatctac gacagctcgg gcaagcccac caacggcggg cgctcgtacc 1020
cgatccccgg cccgcgcccc atctcctgct ccggcagcgg cgacggcggc aacaacggcg 1080
gcggcggcga cgacaacaac aataacaacg gtggtggcaa caacggcggc ggcggcggcg 1140
gcagcgtccc cctgtacggg cagtgcggcg gcatcggcta cacgggcccg accacctgtg 1200
cccagggaac ttgcaaggtg tcgaacgaat actacagcca gtgcctcccc tag 1253

<210> 24
<211> 310
<212> PRT
<213> Myceliophthora thermophila

<400> 24

```
Met Lys Pro Phe Ser Leu Val Ala Leu Ala Thr Ala Val Ser Gly His
1               5                   10                  15

Ala Ile Phe Gln Arg Val Ser Val Asn Gly Gln Asp Gln Gly Gln Leu
            20                  25                  30

Lys Gly Val Arg Ala Pro Ser Ser Asn Ser Pro Ile Gln Asn Val Asn
        35                  40                  45

Asp Ala Asn Met Ala Cys Asn Ala Asn Ile Val Tyr His Asp Ser Thr
    50                  55                  60

Ile Ile Lys Val Pro Ala Gly Ala Arg Val Gly Ala Trp Trp Gln His
65                  70                  75                  80

Val Ile Gly Gly Pro Gln Gly Ala Asn Asp Pro Asp Asn Pro Ile Ala
                85                  90                  95

Ala Ser His Lys Gly Pro Ile Gln Val Tyr Leu Ala Lys Val Asp Asn
            100                 105                 110

Ala Ala Thr Ala Ser Pro Ser Gly Leu Arg Trp Phe Lys Val Ala Glu
        115                 120                 125
```

```
Arg Gly Leu Asn Asn Gly Val Trp Ala Val Asp Glu Leu Ile Ala Asn
    130                 135                 140

Asn Gly Trp His Tyr Phe Asp Leu Pro Ser Cys Val Ala Pro Gly Gln
145                 150                 155                 160

Tyr Leu Met Arg Val Glu Leu Leu Ala Leu His Ser Ala Ser Ser Pro
                165                 170                 175

Gly Gly Ala Gln Phe Tyr Met Gly Cys Ala Gln Ile Glu Val Thr Gly
            180                 185                 190

Ser Gly Thr Asn Ser Gly Ser Asp Phe Val Ser Phe Pro Gly Ala Tyr
        195                 200                 205

Ser Ala Asn Asp Pro Gly Ile Leu Leu Ser Ile Tyr Asp Ser Ser Gly
    210                 215                 220

Lys Pro Thr Asn Gly Gly Arg Ser Tyr Pro Ile Pro Gly Pro Arg Pro
225                 230                 235                 240

Ile Ser Cys Ser Gly Ser Gly Asp Gly Gly Asn Asn Gly Gly Gly Gly
                245                 250                 255

Asp Asp Asn Asn Asn Asn Asn Gly Gly Gly Asn Asn Gly Gly Gly Gly
            260                 265                 270

Gly Gly Ser Val Pro Leu Tyr Gly Gln Cys Gly Gly Ile Gly Tyr Thr
        275                 280                 285

Gly Pro Thr Thr Cys Ala Gln Gly Thr Cys Lys Val Ser Asn Glu Tyr
    290                 295                 300

Tyr Ser Gln Cys Leu Pro
305                 310
```

<210> 25
<211> 814
<212> DNA
<213> Myceliophthora thermophila

<400> 25

atgaagctct ccctcttctc cgtcctggcc actgccctca ccgtcgaggg gcatgccatc 60

ttccagaagg tctccgtcaa cggagcggac cagggctccc tcaccggcct ccgcgctccc 120

aacaacaaca accccgtgca ggatgtcaac agccaggaca tgatctgcgg ccagtcggga 180

tcgacgtcga acactatcat cgaggtcaag gccggcgata ggatcggtgc ctggtatcag 240

catgtcatcg gcggtgccca gttccccaac gacccagaca acccgattgc caagtcgcac 300

aagggccccg tcatggccta cctcgccaag gttgacaatg ccgcaaccgc cagcaagacg 360

ggcctgaagt ggtatgtatt cccgcggccc gagggacatc gggttgggca agtcgagact 420

gacggagctc gcttctccgt ataggttcaa gatttgggag gataccttta atcccagcac 480

caagacctgg ggtgtcgaca acctcatcaa taacaacggc tgggtgtact tcaacctccc 540

gcagtgcatc gccgacggca actacctcct ccgcgtcgag gtcctcgctc tgcactcggc 600

ctactctcag ggccaggctc agttctacca gtcctgcgcc cagatcaacg tatccggcgg 660

cggctccttc acaccgccgt cgactgtcag cttcccgggt gcctacagcg ccagcgaccc 720

cggtatcctg atcaacatct acggcgccac cggccagccc gacaacaacg gccagccgta 780

cactgcccct gggcccgcgc ccatctcctg ctga 814

<210> 26
<211> 246
<212> PRT
<213> Myceliophthora thermophila

<400> 26

```
Met Lys Leu Ser Leu Phe Ser Val Leu Ala Thr Ala Leu Thr Val Glu
1               5                   10                  15

Gly His Ala Ile Phe Gln Lys Val Ser Val Asn Gly Ala Asp Gln Gly
            20                  25                  30

Ser Leu Thr Gly Leu Arg Ala Pro Asn Asn Asn Asn Pro Val Gln Asp
        35                  40                  45

Val Asn Ser Gln Asp Met Ile Cys Gly Gln Ser Gly Ser Thr Ser Asn
    50                  55                  60

Thr Ile Ile Glu Val Lys Ala Gly Asp Arg Ile Gly Ala Trp Tyr Gln
65                  70                  75                  80

His Val Ile Gly Gly Ala Gln Phe Pro Asn Asp Pro Asp Asn Pro Ile
                85                  90                  95

Ala Lys Ser His Lys Gly Pro Val Met Ala Tyr Leu Ala Lys Val Asp
            100                 105                 110

Asn Ala Ala Thr Ala Ser Lys Thr Gly Leu Lys Trp Phe Lys Ile Trp
        115                 120                 125

Glu Asp Thr Phe Asn Pro Ser Thr Lys Thr Trp Gly Val Asp Asn Leu
    130                 135                 140

Ile Asn Asn Asn Gly Trp Val Tyr Phe Asn Leu Pro Gln Cys Ile Ala
145                 150                 155                 160

Asp Gly Asn Tyr Leu Leu Arg Val Glu Val Leu Ala Leu His Ser Ala
                165                 170                 175

Tyr Ser Gln Gly Gln Ala Gln Phe Tyr Gln Ser Cys Ala Gln Ile Asn
            180                 185                 190

Val Ser Gly Gly Gly Ser Phe Thr Pro Pro Ser Thr Val Ser Phe Pro
        195                 200                 205

Gly Ala Tyr Ser Ala Ser Asp Pro Gly Ile Leu Ile Asn Ile Tyr Gly
    210                 215                 220

Ala Thr Gly Gln Pro Asp Asn Asn Gly Gln Pro Tyr Thr Ala Pro Gly
225                 230                 235                 240

Pro Ala Pro Ile Ser Cys
                245
```

<210> 27
<211> 1115
<212> DNA
<213> Thermoascus aurantiacus

<400> 27

```
atgtcgttct cgaagattgc tgcgatcacc ggggccatta cctatgcgtc tctggccgcc      60
gctcacggtt atgttacagg aatcgtagcc gatggcacct agtatgtaac gctcatgcca     120
agatccgcat tgctgtacta acaattagca gctacggggg ctatatcgtg acccaatacc     180
cctacatgtc gacaccgccg gatgtcatcg cctggtctac caaagcaact gatcttggtt     240
tcgtggatcc cagtagctat gcttcgtctg atattatctg ccacaagggt gctgagcctg     300
gtgccctgag cgccaaggtg gctgctggag ggaccgtcga gctgcagtgg acggattggc     360
ctgagagtca caagggcccg gtcattgact acctcgccgc ctgtaacggg gactgctcga     420
ctgtcgacaa gaccaaacta gagttcttca agattgatga gagtggccta attgacggca     480
gcagcgcccc aggcacatgg gcctctgaca acttgattgc caataacaac agctggaccg     540
tcaccatccc gagcacgatt gctcccggca actatgtcct gagacatgaa atcattgccc     600
tccactccgc cggaaataca aatggtgctc agaactaccc ccagtgtatc aaccttgagg     660
tcacaggcag tggcaccgac acccctgccg gcaccctcgg aacggagctt tataaggcaa     720
cggaccctgg cattctggtc aacatctacc agaccctgac cagctacgat attcccggcc     780
ctgctctgta caccggtggt agctctggta gctctggttc ctccaacacc gccaaggcca     840
ccacttcgac ggcttctagc tctatcgtga ccccgacgcc tgttaacaac ccaaccgtta     900
ctcagactgc cgttgttgat gtcacccaga ctgtttccca gaatgctgcc gtcgccacca     960
cgactccggc ctccactgca gttgctacag ctgtcccaac gggaaccacc tttagctttg    1020

attcgatgac ctcggatgaa ttcgtcagcc tgatgcgtgc gaccgtgaat tggctgcttt    1080
ctaacaagaa gcatgcccgg gatctttctt actaa                               1115
```

<210> 28
<211> 354
<212> PRT
<213> Thermoascus aurantiacus

<400> 28

```
Met Ser Phe Ser Lys Ile Ala Ala Ile Thr Gly Ala Ile Thr Tyr Ala
1               5                   10                  15

Ser Leu Ala Ala Ala His Gly Tyr Val Thr Gly Ile Val Ala Asp Gly
            20                  25                  30

Thr Tyr Tyr Gly Gly Tyr Ile Val Thr Gln Tyr Pro Tyr Met Ser Thr
        35                  40                  45

Pro Pro Asp Val Ile Ala Trp Ser Thr Lys Ala Thr Asp Leu Gly Phe
    50                  55                  60

Val Asp Pro Ser Ser Tyr Ala Ser Ser Asp Ile Ile Cys His Lys Gly
65                  70                  75                  80

Ala Glu Pro Gly Ala Leu Ser Ala Lys Val Ala Ala Gly Gly Thr Val
                85                  90                  95

Glu Leu Gln Trp Thr Asp Trp Pro Glu Ser His Lys Gly Pro Val Ile
            100                 105                 110

Asp Tyr Leu Ala Ala Cys Asn Gly Asp Cys Ser Thr Val Asp Lys Thr
        115                 120                 125

Lys Leu Glu Phe Phe Lys Ile Asp Glu Ser Gly Leu Ile Asp Gly Ser
    130                 135                 140

Ser Ala Pro Gly Thr Trp Ala Ser Asp Asn Leu Ile Ala Asn Asn Asn
145                 150                 155                 160

Ser Trp Thr Val Thr Ile Pro Ser Thr Ile Ala Pro Gly Asn Tyr Val
                165                 170                 175

Leu Arg His Glu Ile Ile Ala Leu His Ser Ala Gly Asn Thr Asn Gly
            180                 185                 190

Ala Gln Asn Tyr Pro Gln Cys Ile Asn Leu Glu Val Thr Gly Ser Gly
        195                 200                 205

Thr Asp Thr Pro Ala Gly Thr Leu Gly Thr Glu Leu Tyr Lys Ala Thr
```

```
    210                 215                 220

Asp Pro Gly Ile Leu Val Asn Ile Tyr Gln Thr Leu Thr Ser Tyr Asp
225                 230                 235                 240

Ile Pro Gly Pro Ala Leu Tyr Thr Gly Gly Ser Ser Gly Ser Ser Gly
                245                 250                 255

Ser Ser Asn Thr Ala Lys Ala Thr Thr Ser Thr Ala Ser Ser Ser Ile
            260                 265                 270

Val Thr Pro Thr Pro Val Asn Asn Pro Thr Val Thr Gln Thr Ala Val
        275                 280                 285

Val Asp Val Thr Gln Thr Val Ser Gln Asn Ala Ala Val Ala Thr Thr
    290                 295                 300

Thr Pro Ala Ser Thr Ala Val Ala Thr Ala Val Pro Thr Gly Thr Thr
305                 310                 315                 320

Phe Ser Phe Asp Ser Met Thr Ser Asp Glu Phe Val Ser Leu Met Arg
                325                 330                 335

Ala Thr Val Asn Trp Leu Leu Ser Asn Lys Lys His Ala Arg Asp Leu
            340                 345                 350

Ser Tyr
```

<210> 29
<211> 862
<212> DNA
<213> Aspergillus fumigatus

<400> 29

```
atgactttgt ccaagatcac ttccattgct ggccttctgg cctcagcgtc tctcgtggct        60
ggccacggct ttgtttctgg cattgttgct gatgggaaat agtatgtgct tgaaccacac       120
aaatgacagc tgcaacagct aacttctatt ccagttacgg agggtacctt gttaaccaat       180
acccctacat gagcaaccct cccgacacca ttgcctggtc caccaccgcc accgacctcg       240
gctttgtgga cggcaccggc taccagtctc cggatattat ctgccacaga gacgcaaaga       300
atggcaagtt gaccgcaacc gttgcagccg gttcacagat cgaattccag tggacgacgt       360
ggccagagtc tcaccatgga ccggtacgac gccgaagaga agagaacata ttgtgaccag       420
ataggctaac atagcatagt tgattactta cctcgctcca tgcaacggcg actgtgccac       480
cgtggacaag accaccctga agtttgtcaa gatcgccgct caaggcttga tcgacggctc       540
caacccacct ggtgtttggg ctgatgatga aatgatcgcc aacaacaaca cggccacagt       600
gaccattcct gcctcctatg cccccggaaa ctacgtcctt cgccacgaga tcatcgccct       660
tcactctgcg ggtaacctga acggcgcgca gaactacccc cagtgtttca acatccaaat       720
caccggtggc ggcagtgctc agggatctgg caccgctggc acgtccctgt acaagaatac       780
tgatcctggc atcaagtttg acatctactc ggatctgagc ggtggatacc ctattcctgg       840
tcctgcactg ttcaacgctt aa                                                862
```

<210> 30
<211> 250
<212> PRT
<213> Aspergillus fumigatus

<400> 30

```
Met Thr Leu Ser Lys Ile Thr Ser Ile Ala Gly Leu Leu Ala Ser Ala
1               5                   10                  15

Ser Leu Val Ala Gly His Gly Phe Val Ser Gly Ile Val Ala Asp Gly
            20                  25                  30

Lys Tyr Tyr Gly Gly Tyr Leu Val Asn Gln Tyr Pro Tyr Met Ser Asn
        35                  40                  45

Pro Pro Asp Thr Ile Ala Trp Ser Thr Thr Ala Thr Asp Leu Gly Phe
    50                  55                  60

Val Asp Gly Thr Gly Tyr Gln Ser Pro Asp Ile Ile Cys His Arg Asp
65                  70                  75                  80

Ala Lys Asn Gly Lys Leu Thr Ala Thr Val Ala Ala Gly Ser Gln Ile
                85                  90                  95

Glu Phe Gln Trp Thr Thr Trp Pro Glu Ser His His Gly Pro Leu Ile
            100                 105                 110

Thr Tyr Leu Ala Pro Cys Asn Gly Asp Cys Ala Thr Val Asp Lys Thr
        115                 120                 125

Thr Leu Lys Phe Val Lys Ile Ala Ala Gln Gly Leu Ile Asp Gly Ser
    130                 135                 140

Asn Pro Pro Gly Val Trp Ala Asp Asp Glu Met Ile Ala Asn Asn Asn
145                 150                 155                 160

Thr Ala Thr Val Thr Ile Pro Ala Ser Tyr Ala Pro Gly Asn Tyr Val
                165                 170                 175

Leu Arg His Glu Ile Ile Ala Leu His Ser Ala Gly Asn Leu Asn Gly
            180                 185                 190

Ala Gln Asn Tyr Pro Gln Cys Phe Asn Ile Gln Ile Thr Gly Gly Gly
        195                 200                 205

Ser Ala Gln Gly Ser Gly Thr Ala Gly Thr Ser Leu Tyr Lys Asn Thr
    210                 215                 220

Asp Pro Gly Ile Lys Phe Asp Ile Tyr Ser Asp Leu Ser Gly Gly Tyr
225                 230                 235                 240

Pro Ile Pro Gly Pro Ala Leu Phe Asn Ala
                245                 250
```

<210> 31
<211> 1021
<212> DNA
<213> Penicillium pinophilum

<400> 31

```
atgccttcta ctaaagtcgc tgccctttct gctgttctag ctttggcctc cacggttgct      60
ggccatggtt ttgtgcaaaa catcgttatc gacggtaaat cgtaagcagt gatgcatcca     120
ttattaaact agacatgctt acaaaaaaat cagttactct ggataccttg tgaatcagtt     180
cccctacgag tccaacccac cagctgttat tgggtgggca acaactgcaa ccgacctggg     240
attcgtcgct cccagtgagt acaccaatgc agacattatc tgccacaaga acgccacacc     300
tggcgcgctt tctgctccag ttgctgcagg gggcactgtc gagctccagt ggactacatg     360
gcccgatagt catcacggtc ctgtcatcag ctacctcgcc aactgcaatg gcaattgttc     420
taccgtggat aagactaagc tagactttgt caagattgac caaggtggtt tgatcgacga     480
tactaccccc ccgggtacat gggcttccga caaacttatc gctgccaaca acagctggac     540
tgtaactatc ccctccacca tcgcgcctgg aaactacgtt ttgcgccacg aaatcattgc     600
tcttcactcc gctggaaacg cagacggtgc ccaaaactac cctcaatgca tcaacttgga     660
gatcaccggc agcggaaccg ccgctccctc tggtaccgct ggcgaaaagc tctacacctc     720
tactgacccc ggtatcttgg tcaatatcta ccaatccttg tcgacctacg ttattcccgg     780
accaactctg tggagcggtg ctgccaatgg cgctgttgcc actggttctg ctactgcggt     840
tgctacgact gccactgctt ctgcgaccgc tactcctacc acacttgtta cctctgtcgc     900
tccagcttca tctacctttg ccactgctgt tgtgaccact gtcgctcctg cagtaactga     960
tgtcgtgact gtcaccgatg tagttaccgt gaccaccgtc atcaccacta ctgtcctttg    1020
a                                                                    1021
```

<210> 32
<211> 322
<212> PRT
<213> Penicillium pinophilum

<400> 32

Met Pro Ser Thr Lys Val Ala Ala Leu Ser Ala Val Leu Ala Leu Ala
1 5 10 15

Ser Thr Val Ala Gly His Gly Phe Val Gln Asn Ile Val Ile Asp Gly
20 25 30

Lys Ser Tyr Ser Gly Tyr Leu Val Asn Gln Phe Pro Tyr Glu Ser Asn
35 40 45

Pro Pro Ala Val Ile Gly Trp Ala Thr Thr Ala Thr Asp Leu Gly Phe
50 55 60

Val Ala Pro Ser Glu Tyr Thr Asn Ala Asp Ile Ile Cys His Lys Asn
65 70 75 80

Ala Thr Pro Gly Ala Leu Ser Ala Pro Val Ala Ala Gly Gly Thr Val
85 90 95

Glu Leu Gln Trp Thr Thr Trp Pro Asp Ser His His Gly Pro Val Ile
100 105 110

Ser Tyr Leu Ala Asn Cys Asn Gly Asn Cys Ser Thr Val Asp Lys Thr
115 120 125

Lys Leu Asp Phe Val Lys Ile Asp Gln Gly Gly Leu Ile Asp Asp Thr
130 135 140

Thr Pro Pro Gly Thr Trp Ala Ser Asp Lys Leu Ile Ala Ala Asn Asn
145 150 155 160

Ser Trp Thr Val Thr Ile Pro Ser Thr Ile Ala Pro Gly Asn Tyr Val
165 170 175

Leu Arg His Glu Ile Ile Ala Leu His Ser Ala Gly Asn Ala Asp Gly
180 185 190

Ala Gln Asn Tyr Pro Gln Cys Ile Asn Leu Glu Ile Thr Gly Ser Gly
195 200 205

Thr Ala Ala Pro Ser Gly Thr Ala Gly Glu Lys Leu Tyr Thr Ser Thr
210 215 220

Asp Pro Gly Ile Leu Val Asn Ile Tyr Gln Ser Leu Ser Thr Tyr Val
225 230 235 240

Ile Pro Gly Pro Thr Leu Trp Ser Gly Ala Ala Asn Gly Ala Val Ala
245 250 255

Thr Gly Ser Ala Thr Ala Val Ala Thr Thr Ala Thr Ala Ser Ala Thr
260 265 270

Ala Thr Pro Thr Thr Leu Val Thr Ser Val Ala Pro Ala Ser Ser Thr
275 280 285

Phe Ala Thr Ala Val Val Thr Thr Val Ala Pro Ala Val Thr Asp Val
290 295 300

Val Thr Val Thr Asp Val Val Thr Val Thr Thr Val Ile Thr Thr Thr
305 310 315 320

Val Leu

<210> 33
<211> 1486
<212> DNA
<213> Thermoascus sp.

<400> 33

atgttgtcgt tcgcttctgc caagtcagct gtgctgacga cccttctact tcttggatcc 60
gctcaggctc acactttgat gaccaccctg tttgtggatg gcgtcaatca gggagatggt 120
gtctgtattc gcatgaacaa caacggtagt actgccaaca cctatatcca gcctgtcacg 180
agcaaggata ttgcctgcgg taagtacagt accggtccag atatcatact ctatttcaat 240
ccgacaacag tcagagctgg agagcaatgc taaacatccc caggcattca aggcgaaatt 300
ggcgccgctc gagtctgtcc agccaaggct tcatccaccc tcacgttcca attccgagag 360
cagccatcca acccgaattc cgctcctctc gatccctcgc acaaaggccc cgctgcggtg 420
tacctgaaaa aggtagactc cgccatcgcg agcaacaacg ccgctggaga cggctggttc 480
aagatctggg agtccgtcta cgacgagtcc acgggcaaat ggggtacgac caagatgatc 540
gagaacaacg ggcacatctc tgtcaaggtc cccgacgata tcgagggtgg gtattatctc 600
gcgcgtacgg agcttctggc gctgcacgcg gcgaacgaag gggatccgca gttctacgtt 660
ggctgcgcgc agctgttcat cgattcagcg gggacagcga aaccgcctac tgtctctatt 720
ggagagggga cctacgatct gagcatgcct gccatgacgt acaatatcta ccagactccg 780
ttggctctac catacccgat gtatgggcct cctgtctaca cacctggctc tggctcgggt 840
tctggctctg gttccgggtc agcttctgca acgagatctt ctgctattcc tactgccacc 900
gctgttacgg actgttcttc cgaagaggac agggaagact cagtcatggc aaccggtgtt 960
cccgttgcaa gaagcacact cagaacctgg gttgacagac tgtcatggca tggtaaggcc 1020
cgtgagaacg tgaaaccagc cgccaggaga agcgcccttg tccagaccga gggtctgaag 1080
ccggaaggct gcatcttcgt caacggcaac tggtgcggtt tcgaggtccc cgattacaac 1140
gatgcggaaa gctgctgggc tgtacgttcc cgtctaatta cttaaaacga aataaaagct 1200
aacagtactt ttctttttct aatcccaggc ctccgacaac tgctggaaac agtccgactc 1260
gtgctggaac cagacccagc ccaccggcta caacaactgc cagatctggc aagaccagaa 1320
atgcaagccc atccaggact cgtgtagcca atccaacccg actggaccgc cgaacaaggg 1380
caaggatata actccaacgt ggccgcccct ggagggctcg atgaagacct tcaccaagcg 1440
cactgtcagt taccgtgatt ggattatgaa aaggaaagga gcataa 1486

<210> 34
<211> 444
<212> PRT
<213> Thermoascus sp.

<400> 34

```
Met Leu Ser Phe Ala Ser Ala Lys Ser Ala Val Leu Thr Thr Leu Leu
1               5                   10                  15

Leu Leu Gly Ser Ala Gln Ala His Thr Leu Met Thr Thr Leu Phe Val
            20                  25                  30

Asp Gly Val Asn Gln Gly Asp Gly Val Cys Ile Arg Met Asn Asn Asn
        35                  40                  45

Gly Ser Thr Ala Asn Thr Tyr Ile Gln Pro Val Thr Ser Lys Asp Ile
    50                  55                  60

Ala Cys Gly Ile Gln Gly Glu Ile Gly Ala Ala Arg Val Cys Pro Ala
65                  70                  75                  80

Lys Ala Ser Ser Thr Leu Thr Phe Gln Phe Arg Glu Gln Pro Ser Asn
                85                  90                  95

Pro Asn Ser Ala Pro Leu Asp Pro Ser His Lys Gly Pro Ala Ala Val
            100                 105                 110

Tyr Leu Lys Lys Val Asp Ser Ala Ile Ala Ser Asn Asn Ala Ala Gly
        115                 120                 125

Asp Gly Trp Phe Lys Ile Trp Glu Ser Val Tyr Asp Glu Ser Thr Gly
    130                 135                 140

Lys Trp Gly Thr Thr Lys Met Ile Glu Asn Asn Gly His Ile Ser Val
145                 150                 155                 160

Lys Val Pro Asp Asp Ile Glu Gly Gly Tyr Tyr Leu Ala Arg Thr Glu
                165                 170                 175

Leu Leu Ala Leu His Ala Ala Asn Glu Gly Asp Pro Gln Phe Tyr Val
```

```
            180                 185                 190

Gly Cys Ala Gln Leu Phe Ile Asp Ser Ala Gly Thr Ala Lys Pro Pro
        195                 200                 205

Thr Val Ser Ile Gly Glu Gly Thr Tyr Asp Leu Ser Met Pro Ala Met
    210                 215                 220

Thr Tyr Asn Ile Tyr Gln Thr Pro Leu Ala Leu Pro Tyr Pro Met Tyr
225                 230                 235                 240

Gly Pro Pro Val Tyr Thr Pro Gly Ser Gly Ser Gly Ser Gly Ser Gly
                245                 250                 255

Ser Gly Ser Ala Ser Ala Thr Arg Ser Ser Ala Ile Pro Thr Ala Thr
            260                 265                 270

Ala Val Thr Asp Cys Ser Ser Glu Glu Asp Arg Glu Asp Ser Val Met
        275                 280                 285

Ala Thr Gly Val Pro Val Ala Arg Ser Thr Leu Arg Thr Trp Val Asp
    290                 295                 300

Arg Leu Ser Trp His Gly Lys Ala Arg Glu Asn Val Lys Pro Ala Ala
305                 310                 315                 320

Arg Arg Ser Ala Leu Val Gln Thr Glu Gly Leu Lys Pro Glu Gly Cys
                325                 330                 335

Ile Phe Val Asn Gly Asn Trp Cys Gly Phe Glu Val Pro Asp Tyr Asn
            340                 345                 350

Asp Ala Glu Ser Cys Trp Ala Ala Ser Asp Asn Cys Trp Lys Gln Ser
        355                 360                 365

Asp Ser Cys Trp Asn Gln Thr Gln Pro Thr Gly Tyr Asn Asn Cys Gln
    370                 375                 380

Ile Trp Gln Asp Gln Lys Cys Lys Pro Ile Gln Asp Ser Cys Ser Gln
385                 390                 395                 400

Ser Asn Pro Thr Gly Pro Pro Asn Lys Gly Lys Asp Ile Thr Pro Thr
                405                 410                 415

Trp Pro Pro Leu Glu Gly Ser Met Lys Thr Phe Thr Lys Arg Thr Val
            420                 425                 430

Ser Tyr Arg Asp Trp Ile Met Lys Arg Lys Gly Ala
        435                 440
```

<210> 35
<211> 835
<212> DNA
<213> Penicillium sp.

<400> 35

```
atgctgtctt cgacgactcg caccctcgcc tttacaggcc ttgcgggcct tctgtccgct      60
cccctggtca aggcccatgg ctttgtccag ggcattgtca tcggtgacca attgtaagtc     120
cctctcttgc agttctgtcg attaactgct ggactgcttg cttgactccc tgctgactcc     180
caacagctac agcgggtaca tcgtcaactc gttcccctac gaatccaacc cacccccgt      240
catcggctgg gccacgaccg ccaccgacct gggcttcgtc gacggcacag gataccaagg     300
cccggacatc atctgccacc ggaatgcgac gcccgcgccg ctgacagccc ccgtggccgc     360
cggcggcacc gtcgagctgc agtggacgcc gtggccggac agccaccacg gacccgtcat     420
cacctacctg gcgccgtgca acggcaactg ctcgaccgtc gacaagacga cgctggagtt     480
cttcaagatc gaccagcagg gcctgatcga cgacacgagc ccgccgggca cctgggcgtc     540
ggacaacctc atcgccaaca acaatagctg gaccgtcacc attcccaaca gcgtcgcccc     600
cggcaactac gtcctgcgcc acgagatcat cgccctgcac tcggccaaca acaaggacgg     660
cgcccagaac tacccccagt gcatcaacat cgaggtcacg ggcggcggct ccgacgcgcc     720
tgagggtact ctgggcgagg atctctacca tgacaccgac ccgggcattc tggtcgacat     780
ttacgagccc attgcgacgt ataccattcc ggggccgcct gagccgacgt tctag          835
```

<210> 36
<211> 253
<212> PRT
<213> Penicillium sp.

<400> 36

Met Leu Ser Ser Thr Thr Arg Thr Leu Ala Phe Thr Gly Leu Ala Gly
1               5                   10                  15

Leu Leu Ser Ala Pro Leu Val Lys Ala His Gly Phe Val Gln Gly Ile
            20                  25                  30

Val Ile Gly Asp Gln Phe Tyr Ser Gly Tyr Ile Val Asn Ser Phe Pro
        35                  40                  45

Tyr Glu Ser Asn Pro Pro Pro Val Ile Gly Trp Ala Thr Thr Ala Thr
    50                  55                  60

Asp Leu Gly Phe Val Asp Gly Thr Gly Tyr Gln Gly Pro Asp Ile Ile
65                  70                  75                  80

Cys His Arg Asn Ala Thr Pro Ala Pro Leu Thr Ala Pro Val Ala Ala

```
                85                  90                  95

Gly Gly Thr Val Glu Leu Gln Trp Thr Pro Trp Pro Asp Ser His His
            100                 105                 110

Gly Pro Val Ile Thr Tyr Leu Ala Pro Cys Asn Gly Asn Cys Ser Thr
        115                 120                 125

Val Asp Lys Thr Thr Leu Glu Phe Phe Lys Ile Asp Gln Gln Gly Leu
    130                 135                 140

Ile Asp Asp Thr Ser Pro Pro Gly Thr Trp Ala Ser Asp Asn Leu Ile
145                 150                 155                 160

Ala Asn Asn Asn Ser Trp Thr Val Thr Ile Pro Asn Ser Val Ala Pro
                165                 170                 175

Gly Asn Tyr Val Leu Arg His Glu Ile Ile Ala Leu His Ser Ala Asn
            180                 185                 190

Asn Lys Asp Gly Ala Gln Asn Tyr Pro Gln Cys Ile Asn Ile Glu Val
        195                 200                 205

Thr Gly Gly Gly Ser Asp Ala Pro Glu Gly Thr Leu Gly Glu Asp Leu
    210                 215                 220

Tyr His Asp Thr Asp Pro Gly Ile Leu Val Asp Ile Tyr Glu Pro Ile
225                 230                 235                 240

Ala Thr Tyr Thr Ile Pro Gly Pro Pro Glu Pro Thr Phe
                245                 250
```

<210> 37
<211> 977
<212> DNA
<213> Thielavia terrestris

<400> 37

atgaagctgt catcccagct cgccgccctc acgctggccg cggcctccgt gtcaggccac 60
tacatcttcg agcagattgc ccatggcggc accaagttcc caccttacga gtacatccga 120
agaaacacga actataacag ccctgtcacc agtctctcgt cgaacgacct gcgatgcaac 180
gtaggcggcg agacggctgg caacacgacc gtcctcgacg tgaaggcggg cgactccttc 240
accttctact cggacgtggc cgtgtaccac caggggccca tctcactgtg cgtgccccgg 300
gccaactttg atcagtccca agcggactgt ccgctcgcct ggataaccac aattgactga 360
cagcccgcac agctacatgt ccaaggctcc cggctccgtc gtggactacg acggctccgg 420
cgactggttc aagatccacg actggggccc gaccttcagc aacggccagg cctcgtggcc 480

gctgcggggt gcgtcccttc cctttccctc cccttcctc ccccttcctc ccccccttc 540
cccccttttc tgtctggtcg cacgccctgc tgacgtcccc gtagacaact accagtacaa 600
catcccgacg tgcatcccga acggcgagta cctgctgcgc atccagtcgc tggcgatcca 660
caacccgggc gccacgccgc agttctacat cagctgcgcg caggtccggg tctcgggcgg 720
cggcagcgcc tcccctccc caacggccaa gatccccggc gcgttcaagg cgaccgatcc 780
cgggtatacc gcgaatgtga gtgccctatg ttccttgcgc tccttgttcc ttgctccttg 840
ctcggcgtgc ttgaacgcta cgggctgtgg agggagggat ggatggatga ataggatgct 900
gactgatggt gggacaccag atttacaata acttccactc gtatacggtg ccgggtccgg 960
cggtctttca gtgctag 977

<210> 38
<211> 223
<212> PRT
<213> Thielavia terrestris

<400> 38

```
Met Lys Leu Ser Ser Gln Leu Ala Ala Leu Thr Leu Ala Ala Ala Ser
1               5                   10                  15

Val Ser Gly His Tyr Ile Phe Glu Gln Ile Ala His Gly Gly Thr Lys
            20                  25                  30

Phe Pro Pro Tyr Glu Tyr Ile Arg Arg Asn Thr Asn Tyr Asn Ser Pro
        35                  40                  45

Val Thr Ser Leu Ser Ser Asn Asp Leu Arg Cys Asn Val Gly Gly Glu
    50                  55                  60

Thr Ala Gly Asn Thr Thr Val Leu Asp Val Lys Ala Gly Asp Ser Phe
65                  70                  75                  80

Thr Phe Tyr Ser Asp Val Ala Val Tyr His Gln Gly Pro Ile Ser Leu
                85                  90                  95

Tyr Met Ser Lys Ala Pro Gly Ser Val Val Asp Tyr Asp Gly Ser Gly
            100                 105                 110

Asp Trp Phe Lys Ile His Asp Trp Gly Pro Thr Phe Ser Asn Gly Gln
        115                 120                 125

Ala Ser Trp Pro Leu Arg Asp Asn Tyr Gln Tyr Asn Ile Pro Thr Cys
    130                 135                 140

Ile Pro Asn Gly Glu Tyr Leu Leu Arg Ile Gln Ser Leu Ala Ile His
145                 150                 155                 160

Asn Pro Gly Ala Thr Pro Gln Phe Tyr Ile Ser Cys Ala Gln Val Arg
                165                 170                 175

Val Ser Gly Gly Gly Ser Ala Ser Pro Ser Pro Thr Ala Lys Ile Pro
            180                 185                 190

Gly Ala Phe Lys Ala Thr Asp Pro Gly Tyr Thr Ala Asn Ile Tyr Asn
        195                 200                 205

Asn Phe His Ser Tyr Thr Val Pro Gly Pro Ala Val Phe Gln Cys
    210                 215                 220
```

<210> 39
<211> 878
<212> DNA
<213> Thielavia terrestris

<400> 39

```
atgaagttct cactggtgtc tctgctggct tacggcctct cggtcgaggc gcactccatc      60
ttccaggttc gtctcgcaca tcacgctcaa ctcggctcgt ggcgtaaggg caaggattaa     120
cacggccggc agagagtctc ggtcaacggc caagaccaag gcctgctcac cggcctccgc     180
gctccaagca acaacaaccc agtgcaagat gtcaacagcc agaacatgat ttgcggccag     240
tcgggctcca agtcgcagac cgttatcaac gtcaaggccg gcgacaggat cggctcgctc     300
tggcagcatg tcatcggcgg cgcccagttt tcgggtgacc cggacaaccc gatcgcccac     360
tcgcacaagg gccccgtgat ggcgtacctt gctaaggtcg acaatgccgc gtccgcgagc     420
caaacgggtc tgaagtggta agtagcgggc gacgctcagg ggacggggat cgggggcctg     480
ctccatccga gactaacacc gtggacaggt tcaagatctg gcaggacggg ttcgatacca     540
gcagcaagac atggggcgtc gacaacctga tcaagaacaa cggctgggtg tacttccacc     600
tgccgcagtg cctcgctccg ggccagtatc tcctgcgcgt cgaggttctg gcgctgcact     660
cggcgtacca gcagggccag gcccagttct accagtcctg cgcccagatc aacgtctccg     720
gctccgggtc cttcagcccg tcccagacgg tcagcatccc gggcgtctac agcgccaccg     780
acccgagcat cctcatcaac atctacggca gcacggggca gcccgacaac ggcggcaagg     840
cttacaaccc ccctggaccc gccccgatct cctgctga                             878
```

<210> 40
<211> 246
<212> PRT
<213> Thielavia terrestris

<400> 40

```
Met Lys Phe Ser Leu Val Ser Leu Leu Ala Tyr Gly Leu Ser Val Glu
1               5                   10                  15
```

```
Ala His Ser Ile Phe Gln Arg Val Ser Val Asn Gly Gln Asp Gln Gly
            20                  25                  30

Leu Leu Thr Gly Leu Arg Ala Pro Ser Asn Asn Asn Pro Val Gln Asp
        35                  40                  45

Val Asn Ser Gln Asn Met Ile Cys Gly Gln Ser Gly Ser Lys Ser Gln
    50                  55                  60

Thr Val Ile Asn Val Lys Ala Gly Asp Arg Ile Gly Ser Leu Trp Gln
65                  70                  75                  80

His Val Ile Gly Gly Ala Gln Phe Ser Gly Asp Pro Asp Asn Pro Ile
                85                  90                  95

Ala His Ser His Lys Gly Pro Val Met Ala Tyr Leu Ala Lys Val Asp
            100                 105                 110

Asn Ala Ala Ser Ala Ser Gln Thr Gly Leu Lys Trp Phe Lys Ile Trp
        115                 120                 125

Gln Asp Gly Phe Asp Thr Ser Ser Lys Thr Trp Gly Val Asp Asn Leu
    130                 135                 140

Ile Lys Asn Asn Gly Trp Val Tyr Phe His Leu Pro Gln Cys Leu Ala
145                 150                 155                 160

Pro Gly Gln Tyr Leu Leu Arg Val Glu Val Leu Ala Leu His Ser Ala
                165                 170                 175

Tyr Gln Gln Gly Gln Ala Gln Phe Tyr Gln Ser Cys Ala Gln Ile Asn
            180                 185                 190

Val Ser Gly Ser Gly Ser Phe Ser Pro Ser Gln Thr Val Ser Ile Pro
        195                 200                 205

Gly Val Tyr Ser Ala Thr Asp Pro Ser Ile Leu Ile Asn Ile Tyr Gly
    210                 215                 220

Ser Thr Gly Gln Pro Asp Asn Gly Gly Lys Ala Tyr Asn Pro Pro Gly
225                 230                 235                 240

Pro Ala Pro Ile Ser Cys
                245
```

<210> 41
<211> 1253

<212> DNA
<213> Thielavia terrestris

<400> 41

```
atgaggacga cattcgccgc cgcgttggca gccttcgctg cgcaggaagt ggcaggccat      60
gccatcttcc aacagctctg ggtggacggc accgactata tacgtgctcc ccttttcctt     120
ttgtgtttgc ccatcctcga ttgataaccc gaggccatcc aatgctgact cttacagcac     180
ggctcctcct gcgtccgcat gccgctgtcg aactcgcccg tcacgaacgt cggcagcagg     240
gacatgatct gcaacgccgg cacgcgcccc gtcagcggga agtgccccgt caaggccggc     300
ggcaccgtga cggttgagat gcaccaggtg ggctgatttc ctgagcgtcc tattcctccc     360
ggaagcccct ttcccatcct ttgccctggc taacccctcc gcccctccca gcaacccggg     420
gatcggtcgt gtaacaacga agccatcggc ggcgcccact ggggaccggt gcaggtgtac     480
ctcagcaagg tggaggacgc gagcacggcg gacgggtcga cgggctggtt caagatcttc     540
gcggacacgt ggtccaagaa ggcgggcagc tcggtggggg acgacgacaa ctggggcacg     600
cgcgacctca acgcgtgctg cggcaagatg caggtcaaga tcccggcgga catcccgtcg     660
ggcgactacc tgctgcgggc ggaggcgctg gcgctgcaca cggcgggcca ggtgggcggc     720
gcgcagttct acatgagctg ctaccagatc accgtgtcgg gcggcggcag cgccagcccg     780
gccaccgtca agttccccgg cgcctacagc gccaacgacc cgggcatcca catcaacatc     840
cacgcggccg tgtccaacta cgtcgcgccc ggcccggccg tctattccgg cggcacgacc     900
aaggtggccg ggtccgggtg ccaaggctgc gagaacacgt gcaaggtcgg ctcgtcgccc     960
acggcgacgg cgccgtcggg caagagcggc gcgggttccg acggcggcgc tgggaccgac    1020
ggcgggtctt cgtcttcgag ccccgacacg ggcagcgcgt gcagcgtgca ggcctacggg    1080
cagtgcggcg ggaacgggta ctcgggttgc acccagtgcg cggtaagttc ggggtcgtct    1140
gtcttttgta ggaacatccg agaggcttgg ctgacgaggc gttgttgtag cccggctata    1200
cttgcaaggc ggtctctccg ccgtactatt cgcagtgcgc cccttcttct tag           1253
```

<210> 42
<211> 334
<212> PRT
<213> Thielavia terrestris

<400> 42

```
Met Arg Thr Thr Phe Ala Ala Ala Leu Ala Ala Phe Ala Ala Gln Glu
1               5                   10                  15

Val Ala Gly His Ala Ile Phe Gln Gln Leu Trp His Gly Ser Ser Cys
            20                  25                  30

Val Arg Met Pro Leu Ser Asn Ser Pro Val Thr Asn Val Gly Ser Arg
        35                  40                  45

Asp Met Ile Cys Asn Ala Gly Thr Arg Pro Val Ser Gly Lys Cys Pro
    50                  55                  60
```

```
Val Lys Ala Gly Gly Thr Val Thr Val Glu Met His Gln Gln Pro Gly
65                  70                  75                  80

Asp Arg Ser Cys Asn Asn Glu Ala Ile Gly Gly Ala His Trp Gly Pro
                85                  90                  95

Val Gln Val Tyr Leu Ser Lys Val Glu Asp Ala Ser Thr Ala Asp Gly
            100                 105                 110

Ser Thr Gly Trp Phe Lys Ile Phe Ala Asp Thr Trp Ser Lys Lys Ala
        115                 120                 125

Gly Ser Ser Val Gly Asp Asp Asp Asn Trp Gly Thr Arg Asp Leu Asn
    130                 135                 140

Ala Cys Cys Gly Lys Met Gln Val Lys Ile Pro Ala Asp Ile Pro Ser
145                 150                 155                 160

Gly Asp Tyr Leu Leu Arg Ala Glu Ala Leu Ala Leu His Thr Ala Gly
                165                 170                 175

Gln Val Gly Gly Ala Gln Phe Tyr Met Ser Cys Tyr Gln Ile Thr Val
            180                 185                 190

Ser Gly Gly Gly Ser Ala Ser Pro Ala Thr Val Lys Phe Pro Gly Ala
        195                 200                 205

Tyr Ser Ala Asn Asp Pro Gly Ile His Ile Asn Ile His Ala Ala Val
    210                 215                 220

Ser Asn Tyr Val Ala Pro Gly Pro Ala Val Tyr Ser Gly Gly Thr Thr
225                 230                 235                 240

Lys Val Ala Gly Ser Gly Cys Gln Gly Cys Glu Asn Thr Cys Lys Val
                245                 250                 255

Gly Ser Ser Pro Thr Ala Thr Ala Pro Ser Gly Lys Ser Gly Ala Gly
            260                 265                 270

Ser Asp Gly Gly Ala Gly Thr Asp Gly Gly Ser Ser Ser Ser Ser Pro
        275                 280                 285

Asp Thr Gly Ser Ala Cys Ser Val Gln Ala Tyr Gly Gln Cys Gly Gly
    290                 295                 300

Asn Gly Tyr Ser Gly Cys Thr Gln Cys Ala Pro Gly Tyr Thr Cys Lys
305                 310                 315                 320
```

Ala Val Ser Pro Pro Tyr Tyr Ser Gln Cys Ala Pro Ser Ser
325 330

<210> 43
<211> 798
<212> DNA
<213> Thielavia terrestris

<400> 43

```
atgaagctga gcgttgccat cgccgtgctg gcgtcggctc ttgccgaggc tcactgtgag      60
tgcatcgtct cactccagct actgcgaagc ttgctgacga tggtccctag acaccttccc     120
cagcatcgga aacaccgctg actggcagta tgtgcggatt acaacgaact accagagcaa     180
cgggccggtg acggacgtca cctcggatca aattcggtgc tacgaacgga acccaggcac     240
gggagcgcag ggcatataca acgtcaccgc cggccagacc atcaactaca acgcgaaggc     300
gtccatctcc cacccggggc ccatgtcctt ctacattgct aaggttcccg ccggccaaac     360
cgctgcgacc tgggacggta agggggctgt gtggaccaag atctaccagg acatgcccaa     420
gttcggcagc agcctgacct ggcccaccat gggtaagaat tctcaccctg gaaatgaacg     480
cacatttgca cagatctaac atggcctaca ggcgccaagt ctgtccccgt caccatccct     540
cgttgcctcc agaacggcga ttaccttctg cgagccgagc acatcgctct acacagcgcg     600
agcagcgtcg gtggcgccca gttctacctc tcgtgcgccc agcttactgt cagcggcggc     660
agtggcacct ggaaccccaa gaaccgggtc tccttccccg gcgcttacaa ggcaacagac     720
ccgggcatct tgatcaacat ctactacccc gtgccgacca gctactcgcc gcccggcccg     780
ccggctgaga cgtgctaa                                                   798
```

<210> 44
<211> 227
<212> PRT
<213> Thielavia terrestris

<400> 44

```
Met Lys Leu Ser Val Ala Ile Ala Val Leu Ala Ser Ala Leu Ala Glu
1               5                   10                  15

Ala His Tyr Thr Phe Pro Ser Ile Gly Asn Thr Ala Asp Trp Gln Tyr
            20                  25                  30

Val Arg Ile Thr Thr Asn Tyr Gln Ser Asn Gly Pro Val Thr Asp Val
        35                  40                  45

Thr Ser Asp Gln Ile Arg Cys Tyr Glu Arg Asn Pro Gly Thr Gly Ala
    50                  55                  60

Gln Gly Ile Tyr Asn Val Thr Ala Gly Gln Thr Ile Asn Tyr Asn Ala
65                  70                  75                  80

Lys Ala Ser Ile Ser His Pro Gly Pro Met Ser Phe Tyr Ile Ala Lys
                85                  90                  95

Val Pro Ala Gly Gln Thr Ala Ala Thr Trp Asp Gly Lys Gly Ala Val
            100                 105                 110

Trp Thr Lys Ile Tyr Gln Asp Met Pro Lys Phe Gly Ser Ser Leu Thr
        115                 120                 125

Trp Pro Thr Met Gly Ala Lys Ser Val Pro Val Thr Ile Pro Arg Cys
    130                 135                 140

Leu Gln Asn Gly Asp Tyr Leu Leu Arg Ala Glu His Ile Ala Leu His
145                 150                 155                 160

Ser Ala Ser Ser Val Gly Gly Ala Gln Phe Tyr Leu Ser Cys Ala Gln
                165                 170                 175

Leu Thr Val Ser Gly Gly Ser Gly Thr Trp Asn Pro Lys Asn Arg Val
            180                 185                 190

Ser Phe Pro Gly Ala Tyr Lys Ala Thr Asp Pro Gly Ile Leu Ile Asn
        195                 200                 205

Ile Tyr Tyr Pro Val Pro Thr Ser Tyr Ser Pro Pro Gly Pro Pro Ala
    210                 215                 220

Glu Thr Cys
225
```

<210> 45
<211> 1107
<212> DNA

<213> Thielavia terrestris

<400> 45

```
atgccttctt tcgcctccaa gactctcctt tccaccctgg cgggtgccgc atccgtggcc      60
gcccacgggc acgtgtcgaa catcgtcatc aacggggtct cgtaccaggg ttacgatccg     120
acctccttcc cttacatgca gaacccgccc atcgtggtcg gctggactgc cgccgacacg     180
gacaacggct ttgttgcccc ggatgccttc gccagtggcg atatcatctg ccacaagaac     240
gccaccaacg ccaagggcca cgccgtggtc gccgcgggag acaagatctt catccagtgg     300
aacacatggc ccgagtccca ccacggcccc gtcatcgact acctcgcgag ctgcggcagc     360
gcgtcctgcg agaccgtcga caagaccaag ctcgagttct tcaagatcga cgaggtcggc     420
ctggtcgacg gcagctcggc gcccggtgtg tggggctccg accagctcat cgccaacaac     480
aactcgtggc tcgtcgagat cccgcccacc atcgcgccgg gcaactacgt cctgcgccac     540
gagatcatcg cgctgcacag cgccgaaaac gccgacggcg cccagaacta cccgcagtgc     600
ttcaacctgc agatcaccgg caccggcacc gccacccct ccggcgtccc cggcacctcg     660
ctctacaccc cgaccgaccc gggcatcctc gtcaacatct acagcgcccc gatcacctac     720
accgtcccgg ggccggccct catctccggc gccgtcagca tcgcccagtc ctcctccgcc     780
atcaccgcct ccggcaccgc cctgaccggc tctgccaccg cacccgccgc cgccgctgct     840
accacaactt ccaccaccaa cgccgcggct gctgctacct ctgctgctgc tgctgctggt     900
acttccacaa ccaccaccag cgccgcggcc gtggtccaga cctcctcctc ctcctcctcc     960
gccccgtcct ctgccgccgc cgccgccacc accaccgcgg ctgccagcgc ccgcccgacc    1020
ggctgctcct ctggccgctc caggaagcag ccgcgccgcc acgcgcggga tatggtggtt    1080
gcgcgagggg ctgaggaggc aaactga                                        1107
```

<210> 46
<211> 368
<212> PRT
<213> Thielavia terrestris

<400> 46

```
Met Pro Ser Phe Ala Ser Lys Thr Leu Leu Ser Thr Leu Ala Gly Ala
1               5                   10                  15

Ala Ser Val Ala Ala His Gly His Val Ser Asn Ile Val Ile Asn Gly
            20                  25                  30

Val Ser Tyr Gln Gly Tyr Asp Pro Thr Ser Phe Pro Tyr Met Gln Asn
        35                  40                  45

Pro Pro Ile Val Val Gly Trp Thr Ala Ala Asp Thr Asp Asn Gly Phe
    50                  55                  60

Val Ala Pro Asp Ala Phe Ala Ser Gly Asp Ile Ile Cys His Lys Asn
65                  70                  75                  80

Ala Thr Asn Ala Lys Gly His Ala Val Val Ala Ala Gly Asp Lys Ile
                85                  90                  95

Phe Ile Gln Trp Asn Thr Trp Pro Glu Ser His His Gly Pro Val Ile
            100                 105                 110

Asp Tyr Leu Ala Ser Cys Gly Ser Ala Ser Cys Glu Thr Val Asp Lys
        115                 120                 125

Thr Lys Leu Glu Phe Phe Lys Ile Asp Glu Val Gly Leu Val Asp Gly
    130                 135                 140
```

```
Ser Ser Ala Pro Gly Val Trp Gly Ser Asp Gln Leu Ile Ala Asn Asn
145                 150                 155                 160

Asn Ser Trp Leu Val Glu Ile Pro Pro Thr Ile Ala Pro Gly Asn Tyr
                165                 170                 175

Val Leu Arg His Glu Ile Ile Ala Leu His Ser Ala Glu Asn Ala Asp
            180                 185                 190

Gly Ala Gln Asn Tyr Pro Gln Cys Phe Asn Leu Gln Ile Thr Gly Thr
        195                 200                 205

Gly Thr Ala Thr Pro Ser Gly Val Pro Gly Thr Ser Leu Tyr Thr Pro
    210                 215                 220

Thr Asp Pro Gly Ile Leu Val Asn Ile Tyr Ser Ala Pro Ile Thr Tyr
225                 230                 235                 240

Thr Val Pro Gly Pro Ala Leu Ile Ser Gly Ala Val Ser Ile Ala Gln
                245                 250                 255

Ser Ser Ser Ala Ile Thr Ala Ser Gly Thr Ala Leu Thr Gly Ser Ala
            260                 265                 270

Thr Ala Pro Ala Ala Ala Ala Ala Thr Thr Thr Ser Thr Thr Asn Ala
        275                 280                 285

Ala Ala Ala Ala Thr Ser Ala Ala Ala Ala Ala Gly Thr Ser Thr Thr
    290                 295                 300

Thr Thr Ser Ala Ala Ala Val Val Gln Thr Ser Ser Ser Ser Ser Ser
305                 310                 315                 320

Ala Pro Ser Ser Ala Ala Ala Ala Ala Thr Thr Thr Ala Ala Ala Ser
                325                 330                 335

Ala Arg Pro Thr Gly Cys Ser Ser Gly Arg Ser Arg Lys Gln Pro Arg
            340                 345                 350

Arg His Ala Arg Asp Met Val Val Ala Arg Gly Ala Glu Glu Ala Asn
        355                 360                 365
```

<210> 47
<211> 993
<212> DNA
<213> Thielavia terrestris

<400> 47

```
atgccgcccg cactccctca actcctaacc acggtcctga ccgccctcac cctcggttcc      60
accgccctcg cccactcaca cctcgcgtac attatcgtta acggcaagct ctaccagggc     120
ttcgacccgc gcccgcacca ggccaactac ccttcccggg tcgggtggtc caccggcgcc     180
gtcgacgacg gcttcgtcac gccggccaac tactccaccc cggacatcat ttgccacatc     240
gccggcacca gcccggccgg ccacgcgccc gtgcgcccgg gcgaccgcat ccacgtccag     300
tggaacggct ggccggtcgg ccacatcggt cccgtgctgt cgtacctcgc ccgctgcgag     360
tcggacacgg gctgcacggg ccagaacaag accgcgctgc ggtggaccaa gatcgacgac     420
tccagcccga ccatgcagaa cgtcgccggc gcgggcaccc agggcgaggg cacccccggc     480
aagcgctggg ccaccgacgt gctgatcgcc gccaacaaca gctggcaggt cgccgtgccg     540
gcggggctgc cgaccggcgc gtacgtgctg cgcaacgaga tcatcgcgct gcactacgcg     600
gcgaggaaga acggggcgca gaactatccg ctctgcatga acctgtgggt ggacgccagt     660
ggtgataata gtagtgtggc tgcaacgacg gcggcggtga cggcgggggg tctgcagatg     720
gatgcgtatg acgcgcgcgg gttctacaag gagaacgatc cgggcgtgct ggtcaatgtc     780
acggccgcgc tgtcgtcgta tgtcgtgccc gggccgacgg tggcggcggg cgccacgccg     840
gtgccgtacg cgcagcagag cccgagcgtg tcgacggcgg cgggcacgcc cgtcgtcgtt     900
acaaggacta gcgagacggc gccgtacacg ggcgccatga cgccgacggt tgcggcgagg     960
atgaagggga gggggtatga tcggcggggt tag                                  993
```

<210> 48
<211> 330
<212> PRT
<213> Thielavia terrestris

<400> 48

```
Met Pro Pro Ala Leu Pro Gln Leu Leu Thr Thr Val Leu Thr Ala Leu
1               5                   10                  15

Thr Leu Gly Ser Thr Ala Leu Ala His Ser His Leu Ala Tyr Ile Ile
            20                  25                  30

Val Asn Gly Lys Leu Tyr Gln Gly Phe Asp Pro Arg Pro His Gln Ala
        35                  40                  45

Asn Tyr Pro Ser Arg Val Gly Trp Ser Thr Gly Ala Val Asp Asp Gly
    50                  55                  60

Phe Val Thr Pro Ala Asn Tyr Ser Thr Pro Asp Ile Ile Cys His Ile
65                  70                  75                  80

Ala Gly Thr Ser Pro Ala Gly His Ala Pro Val Arg Pro Gly Asp Arg
                85                  90                  95

Ile His Val Gln Trp Asn Gly Trp Pro Val Gly His Ile Gly Pro Val
            100                 105                 110
```

```
Leu Ser Tyr Leu Ala Arg Cys Glu Ser Asp Thr Gly Cys Thr Gly Gln
        115                 120                 125

Asn Lys Thr Ala Leu Arg Trp Thr Lys Ile Asp Asp Ser Ser Pro Thr
    130                 135                 140

Met Gln Asn Val Ala Gly Ala Gly Thr Gln Gly Glu Gly Thr Pro Gly
145                 150                 155                 160

Lys Arg Trp Ala Thr Asp Val Leu Ile Ala Ala Asn Asn Ser Trp Gln
                165                 170                 175

Val Ala Val Pro Ala Gly Leu Pro Thr Gly Ala Tyr Val Leu Arg Asn
            180                 185                 190

Glu Ile Ile Ala Leu His Tyr Ala Ala Arg Lys Asn Gly Ala Gln Asn
        195                 200                 205

Tyr Pro Leu Cys Met Asn Leu Trp Val Asp Ala Ser Gly Asp Asn Ser
    210                 215                 220

Ser Val Ala Ala Thr Thr Ala Ala Val Thr Ala Gly Gly Leu Gln Met
225                 230                 235                 240

Asp Ala Tyr Asp Ala Arg Gly Phe Tyr Lys Glu Asn Asp Pro Gly Val
                245                 250                 255

Leu Val Asn Val Thr Ala Ala Leu Ser Ser Tyr Val Val Pro Gly Pro
            260                 265                 270

Thr Val Ala Ala Gly Ala Thr Pro Val Pro Tyr Ala Gln Gln Ser Pro
        275                 280                 285

Ser Val Ser Thr Ala Ala Gly Thr Pro Val Val Val Thr Arg Thr Ser
    290                 295                 300

Glu Thr Ala Pro Tyr Thr Gly Ala Met Thr Pro Thr Val Ala Ala Arg
305                 310                 315                 320

Met Lys Gly Arg Gly Tyr Asp Arg Arg Gly
                325                 330
```

<210> 49
<211> 1221
<212> DNA
<213> Thielavia terrestris

<400> 49

```
atgaagacat tcaccgccct cctggccgca gccggcctcg tcgccggcca tggatatgtc      60

gacaacgcca ccattggcgg ccagttttat caggtactct accgcttcac ccaaggtccg     120
ctggccacaa ctctataggt gtcataaatt aacaagccac cgtcccgcag ttctatcagg     180
tgtgctcgct accgaccatg tggtcccgtc tcagcaagcc actcacacgc ccatgatccc     240
ctagccttac gtcgacccgt atttagcaac cttggcacgt agtatttatt gtcccaaata     300
ttgagctgaa ctgcacctcc ctagaatccc gcggtgctaa cattctttca gcccgacagg     360
gtctctcgat ccatcccggg caacggcccg gtcacggacg tcactctcat cgacctgcag     420
tgcaacgcca attccacccc ggccaagctc cacgccactg ccgctgccgg ctcggacgtg     480
attctccgct ggacgctctg gcctgagtcg cacgttggcc ccgtcatcac ctacatggcc     540
cgctgccccg acacgggctg ccaggactgg atgccgggca cttcgtagga gcccatcttg     600
caccatatcc atttcaaccg gccacacgca ctgacccata tgtctgtcta cccctgcagt     660
gcggtctggt tcaagatcaa ggagggcggc cgcgacggca cttccaacac ctgggccgac     720
gtacgtgtac cccgtcccag agagccaaag ccccccctc aacaaagcaa acatctcaat      780
agcccgagcc tacgcactaa cccctctcct tccccctcga aaacacagac cccgctgatg     840
acggcgccca cctcgtacac gtacacgatc ccctcctgcc tgaagaaggg ctactacctg     900
gtccgccacg agatcatcgc gctgcacgcc gcctacacct accccggcgc gcagttctac     960
ccgggctgcc accagctcaa cgtcacgggc ggcgggtcca ccgtaccgtc gagcggcctg    1020
gtggcctttc ccggggcgta caagggcagt gaccccggga ttacgtacga tgcgtataaa    1080
ggtgggttgg ctggttggcc caggtcttgg tgatgggga atgtggtgat gaggtttatt     1140
atttgggatc ccgtggctaa cgtaaccctg ggtgtagcgc aaacgtacca gattcctggg    1200
ccggcggtct ttacttgctg a                                               1221
```

<210> 50
<211> 236
<212> PRT
<213> Thielavia terrestris

<400> 50

```
Met Lys Thr Phe Thr Ala Leu Leu Ala Ala Ala Gly Leu Val Ala Gly
1               5                   10                  15

His Gly Tyr Val Asp Asn Ala Thr Ile Gly Gly Gln Phe Tyr Gln Asn
            20                  25                  30

Pro Ala Val Leu Thr Phe Phe Gln Pro Asp Arg Val Ser Arg Ser Ile
        35                  40                  45

Pro Gly Asn Gly Pro Val Thr Asp Val Thr Leu Ile Asp Leu Gln Cys
    50                  55                  60

Asn Ala Asn Ser Thr Pro Ala Lys Leu His Ala Thr Ala Ala Ala Gly
65                  70                  75                  80

Ser Asp Val Ile Leu Arg Trp Thr Leu Trp Pro Glu Ser His Val Gly
                85                  90                  95

Pro Val Ile Thr Tyr Met Ala Arg Cys Pro Asp Thr Gly Cys Gln Asp
            100                 105                 110

Trp Met Pro Gly Thr Ser Ala Val Trp Phe Lys Ile Lys Glu Gly Gly
        115                 120                 125

Arg Asp Gly Thr Ser Asn Thr Trp Ala Asp Thr Pro Leu Met Thr Ala
    130                 135                 140

Pro Thr Ser Tyr Thr Tyr Thr Ile Pro Ser Cys Leu Lys Lys Gly Tyr
145                 150                 155                 160

Tyr Leu Val Arg His Glu Ile Ile Ala Leu His Ala Ala Tyr Thr Tyr
                165                 170                 175

Pro Gly Ala Gln Phe Tyr Pro Gly Cys His Gln Leu Asn Val Thr Gly
            180                 185                 190

Gly Gly Ser Thr Val Pro Ser Ser Gly Leu Val Ala Phe Pro Gly Ala
        195                 200                 205

Tyr Lys Gly Ser Asp Pro Gly Ile Thr Tyr Asp Ala Tyr Lys Ala Gln
    210                 215                 220

Thr Tyr Gln Ile Pro Gly Pro Ala Val Phe Thr Cys
225                 230                 235
```

<210> 51
<211> 933

<212> DNA
<213> Thielavia terrestris

<400> 51

```
atggccttgc tgctcttggc aggcttggcc attctggccg ggccggctca tgcccacggc     60
ggcctcgcca actacacagt gggcaacacc tggtataggg ggtgcgtaag gggggcaccg    120
acaacgcctg cttagtaact ccaccatttc gagcgggcta acaccgggcg cagctacgac    180
cccttcacgc cggcggccga ccagatcggc cagccgtgga tgatccaacg cgcgtgggac    240
tcgatcgacc cgatcttcag cgtcaacgac aaggcgctcg cctgcaacac cccggccacg    300
gcgccgacct cttacattcc catccgcgcg ggcgagaaca tcacggccgt gtactggtac    360
tggctgcacc cggtgggccc catgacggcg tggctggcgc ggtgcgacgg cgactgccgc    420
gacgccgacg tcaacgaggc gcgctggttc aagatctggg aggccggcct gctcagcggg    480
ccgaacctgg ccgagggcat gtggtaccag aaggcgttcc agaactggga cggcagcccg    540
gacctgtggc ccgtcacgat cccggccggg ctgaagagcg gcctgtacat gatccggcac    600
gagatcttgt cgatccacgt cgaggataaa ccgcagtttt atcccgagtg tgcgcatctg    660
aatgtgaccg gggtggggga cctgctgccg cctgatgagt ttttggtgaa gttcccgggc    720
gcttacaaag aagatagtga gtgaaacgcg aagcttcggt agccattggg ttgcgctgat    780
ggaggttaga cccgtcgatc aagatcaata tctactcgga ccagtacgcc aatacaacgg    840
tgagtgtaac aggtcgagca aaaccaaaca gatgccgatg actgatgatc tcagaattac    900
acaattcccg gagggccgat atgggatggg tga                                 933
```

<210> 52
<211> 250
<212> PRT
<213> Thielavia terrestris

<400> 52

```
Met Ala Leu Leu Leu Leu Ala Gly Leu Ala Ile Leu Ala Gly Pro Ala
1               5                   10                  15

His Ala His Gly Gly Leu Ala Asn Tyr Thr Val Gly Asn Thr Trp Tyr
            20                  25                  30

Arg Gly Tyr Asp Pro Phe Thr Pro Ala Ala Asp Gln Ile Gly Gln Pro
        35                  40                  45

Trp Met Ile Gln Arg Ala Trp Asp Ser Ile Asp Pro Ile Phe Ser Val
    50                  55                  60

Asn Asp Lys Ala Leu Ala Cys Asn Thr Pro Ala Thr Ala Pro Thr Ser
65                  70                  75                  80

Tyr Ile Pro Ile Arg Ala Gly Glu Asn Ile Thr Ala Val Tyr Trp Tyr
                85                  90                  95

Trp Leu His Pro Val Gly Pro Met Thr Ala Trp Leu Ala Arg Cys Asp
            100                 105                 110

Gly Asp Cys Arg Asp Ala Asp Val Asn Glu Ala Arg Trp Phe Lys Ile
        115                 120                 125

Trp Glu Ala Gly Leu Leu Ser Gly Pro Asn Leu Ala Glu Gly Met Trp
    130                 135                 140

Tyr Gln Lys Ala Phe Gln Asn Trp Asp Gly Ser Pro Asp Leu Trp Pro
145                 150                 155                 160

Val Thr Ile Pro Ala Gly Leu Lys Ser Gly Leu Tyr Met Ile Arg His
                165                 170                 175

Glu Ile Leu Ser Ile His Val Glu Asp Lys Pro Gln Phe Tyr Pro Glu
            180                 185                 190

Cys Ala His Leu Asn Val Thr Gly Gly Gly Asp Leu Leu Pro Pro Asp
        195                 200                 205

Glu Phe Leu Val Lys Phe Pro Gly Ala Tyr Lys Glu Asp Asn Pro Ser
    210                 215                 220

Ile Lys Ile Asn Ile Tyr Ser Asp Gln Tyr Ala Asn Thr Thr Asn Tyr
225                 230                 235                 240

Thr Ile Pro Gly Gly Pro Ile Trp Asp Gly
                245                 250
```

<210> 53
<211> 1584
<212> DNA
<213> Thielavia terrestris

<400> 53

```
atgatgccgt cccttgttcg cttctcaatg ggtctggcga ccgccttcgc ctcgctgtcc      60
acagcacata ccgtcttcac cacgcttttc atcaacggcg tcgaccaagg ggacgggacc     120
tgcatccgca tggccaagaa gggcagcgtt tgcacccatc ccattgctgg tggcctcgac     180
agcccagaca tggcttgtgg tatgccctct gcgtttcccc tgcgagagct ttcctcgagc     240
taacccaatg ccgcgttgcc caggccgaga cggacaacaa gccgtggcat tcacctgccc     300
agccccggcg ggctccaagt tgagcttcga gttccgcatg tgggccgacg cctctcagcc     360
cggctctatc gacccatccc acctcggctc gacggcaatc tacctcaaac aagtctccaa     420
catcagctcc gactcggctg ccggccctgg ctggttcaag atctacgccg agggctacga     480
cacagccgcc aagaagtggg ccacagagaa gctcatcgac aacggcggcc tgctgagcat     540
cgagcttccg cccactctgc cggcgggata ctacctcgcc cgcagcgaga tcgtcaccat     600
ccagaacgtc accaacgacc acgtcgaccc gcagttctac gttggctgcg cacagctctt     660
cgtccagggg cctccgacca cccccaccgt cccgccagac agactcgtct ccatcccggg     720
ccacgtccat gcctccgacc cggggctgac cttcaacatc tggcgcgacg accccctccaa     780
gacggcctac accgtcgtcg gcccggcccc cttctccccc accgccgccc ccaccccccac     840
ctccaccaac accaacgggc agcaacaaca acaacagcaa caggcgataa agcagacgga     900
cggcgtgatc cccgccgact gccagctcaa gaacgccaac tggtgcggcg ccgaggtgcc     960
cgcgtacgcc gacgaggccg gctgctgggc gtcgtcggcc gactgcttcg cccagctgga    1020
cgcctgctac acgtcggcgc cgcccacggg cagccgcggc tgccggctgt gggaggactg    1080

gtgcaccggc attcagcagg gctgccgcgc ggggcggtgg cgggggccgc cgccctttca    1140
tggggagggg gcagcagcgg aggtgtgaac ggttcgggga cgggtggcgg tggtggtggt    1200
ggtggtggtg gcactggctc ttcttcggct tctgccccga cggagacggc ctctgctggc    1260
cggggggcg caagaatagc tgccgtggcc ggctgcggag gcgggacagg agacatggtt    1320
gaagaggttt tcctctttta ttgggacgct tgcagcggct ggcgacggag ccgtggtggt    1380
ggttcgattc ttgcgaggct tatccttcat gtccttcttc cacttttgag accgaggcga    1440
gcccctcgag tccatttact tctcttccac ctgtacctca acttctgtta tccaggaacc    1500
agtggtttct ataatcgcct gagcattaaa ctaggcatat ggccaagcaa aatgtcgcct    1560
gatgtagcgc attacgtgaa ataa                                           1584
```

<210> 54
<211> 478
<212> PRT
<213> Thielavia terrestris

<400> 54

```
Met Met Pro Ser Leu Val Arg Phe Ser Met Gly Leu Ala Thr Ala Phe
1               5                   10                  15

Ala Ser Leu Ser Thr Ala His Thr Val Phe Thr Thr Leu Phe Ile Asn
            20                  25                  30

Gly Val Asp Gln Gly Asp Gly Thr Cys Ile Arg Met Ala Lys Lys Gly
        35                  40                  45

Ser Val Cys Thr His Pro Ile Ala Gly Gly Leu Asp Ser Pro Asp Met
    50                  55                  60

Ala Cys Gly Arg Asp Gly Gln Gln Ala Val Ala Phe Thr Cys Pro Ala
65                  70                  75                  80

Pro Ala Gly Ser Lys Leu Ser Phe Glu Phe Arg Met Trp Ala Asp Ala
                85                  90                  95

Ser Gln Pro Gly Ser Ile Asp Pro Ser His Leu Gly Ser Thr Ala Ile
            100                 105                 110

Tyr Leu Lys Gln Val Ser Asn Ile Ser Ser Asp Ser Ala Ala Gly Pro
        115                 120                 125

Gly Trp Phe Lys Ile Tyr Ala Glu Gly Tyr Asp Thr Ala Ala Lys Lys
    130                 135                 140

Trp Ala Thr Glu Lys Leu Ile Asp Asn Gly Gly Leu Leu Ser Ile Glu
145                 150                 155                 160
```

```
Leu Pro Pro Thr Leu Pro Ala Gly Tyr Tyr Leu Ala Arg Ser Glu Ile
                165                 170                 175

Val Thr Ile Gln Asn Val Thr Asn Asp His Val Asp Pro Gln Phe Tyr
            180                 185                 190

Val Gly Cys Ala Gln Leu Phe Val Gln Gly Pro Pro Thr Thr Pro Thr
        195                 200                 205

Val Pro Pro Asp Arg Leu Val Ser Ile Pro Gly His Val His Ala Ser
    210                 215                 220

Asp Pro Gly Leu Thr Phe Asn Ile Trp Arg Asp Asp Pro Ser Lys Thr
225                 230                 235                 240

Ala Tyr Thr Val Val Gly Pro Ala Pro Phe Ser Pro Thr Ala Ala Pro
                245                 250                 255

Thr Pro Thr Ser Thr Asn Thr Asn Gly Gln Gln Gln Gln Gln Gln Gln
            260                 265                 270

Gln Ala Ile Lys Gln Thr Asp Gly Val Ile Pro Ala Asp Cys Gln Leu
        275                 280                 285

Lys Asn Ala Asn Trp Cys Gly Ala Glu Val Pro Ala Tyr Ala Asp Glu
    290                 295                 300

Ala Gly Cys Trp Ala Ser Ser Ala Asp Cys Phe Ala Gln Leu Asp Ala
305                 310                 315                 320

Cys Tyr Thr Ser Ala Pro Pro Thr Gly Ser Arg Gly Cys Arg Leu Trp
                325                 330                 335

Glu Asp Trp Cys Thr Gly Ile Gln Gln Gly Cys Arg Ala Gly Arg Trp
            340                 345                 350

Arg Gly Pro Pro Pro Phe His Gly Glu Gly Ala Ala Ala Glu Thr Ala
        355                 360                 365

Ser Ala Gly Arg Gly Gly Ala Arg Ile Ala Ala Val Ala Gly Cys Gly
    370                 375                 380

Gly Gly Thr Gly Asp Met Val Glu Glu Val Phe Leu Phe Tyr Trp Asp
385                 390                 395                 400

Ala Cys Ser Gly Trp Arg Arg Ser Arg Gly Gly Gly Ser Ile Leu Ala
                405                 410                 415
```

Arg Leu Ile Leu His Val Leu Leu Pro Leu Leu Arg Pro Arg Arg Ala
420 425 430

Pro Arg Val His Leu Leu Leu Phe His Leu Tyr Leu Asn Phe Cys Tyr
435 440 445

Pro Gly Thr Ser Gly Phe Tyr Asn Arg Leu Ser Ile Lys Leu Gly Ile
450 455 460

Trp Pro Ser Lys Met Ser Pro Asp Val Ala His Tyr Val Lys
465 470 475

<210> 55
<211> 868
<212> DNA
<213> Thielavia terrestris

<400> 55

atgcagctcc tcgtgggctt gctgcttgca gccgtggctg ctcgagcaca ttgtatttct 60

accccttcc gcgtgcctcc cagcctcaag gcaagaagac gcacgcagca gctaacggac 120

cctatcagac acatttccca gactcgtggt aaatgggcag cccgaggaca aggactggtc 180

ggttacgcgc atgaccaaga acgcgcagag caagcaggga gtccaggacc cgaccagtcc 240

cgacattcgc tgctacacgt cgcagacggc gcctaacgtg gctacggtcc ctgccggagc 300

caccgtccat tacatatcga ctcagcagat caaccacccg ggcccgacgc agtactacct 360

cgccaaggta ccggcggggt cgtcggccaa gacgtgggac gggtcagggg ccgtctggtt 420

caagatctcg accaccatgc cttacttgga caacaacaag cagcttgtct ggccgaatca 480

gagtaggaac aattcccgct ccaatcttcg atttggcctt gagctacggc cgattgcatg 540

ggagagaccg ttgactgacg gggcaaccca accttcatca gacacgtaca cgacggtcaa 600

cacgaccatc cccgccgata cgcccagtgg ggaatacctc ctccgggtcg agcagatcgc 660

gctgcacctg gcctcgcagc ccaacggggc tcagttctac ctggcctgct cgcagatcca 720

gattacgggc ggcggcaacg gcacgcccgg cccgctagtc gcgttgccgg gggcgtacaa 780

gagcaacgac ccgggcattt tggtcaacat ctactctatg cagcccggcg attacaagcc 840

gcccgggccg ccggtgtgga gtggctga 868

<210> 56
<211> 230
<212> PRT
<213> Thielavia terrestris

<400> 56

```
Met Gln Leu Leu Val Gly Leu Leu Leu Ala Ala Val Ala Ala Arg Ala
1               5                   10                  15

His Tyr Thr Phe Pro Arg Leu Val Val Asn Gly Gln Pro Glu Asp Lys
            20                  25                  30

Asp Trp Ser Val Thr Arg Met Thr Lys Asn Ala Gln Ser Lys Gln Gly
        35                  40                  45

Val Gln Asp Pro Thr Ser Pro Asp Ile Arg Cys Tyr Thr Ser Gln Thr
    50                  55                  60

Ala Pro Asn Val Ala Thr Val Pro Ala Gly Ala Thr Val His Tyr Ile
65                  70                  75                  80

Ser Thr Gln Gln Ile Asn His Pro Gly Pro Thr Gln Tyr Tyr Leu Ala
                85                  90                  95

Lys Val Pro Ala Gly Ser Ser Ala Lys Thr Trp Asp Gly Ser Gly Ala
            100                 105                 110

Val Trp Phe Lys Ile Ser Thr Thr Met Pro Tyr Leu Asp Asn Asn Lys
        115                 120                 125

Gln Leu Val Trp Pro Asn Gln Asn Thr Tyr Thr Thr Val Asn Thr Thr
    130                 135                 140

Ile Pro Ala Asp Thr Pro Ser Gly Glu Tyr Leu Leu Arg Val Glu Gln
145                 150                 155                 160

Ile Ala Leu His Leu Ala Ser Gln Pro Asn Gly Ala Gln Phe Tyr Leu
                165                 170                 175

Ala Cys Ser Gln Ile Gln Ile Thr Gly Gly Gly Asn Gly Thr Pro Gly
            180                 185                 190

Pro Leu Val Ala Leu Pro Gly Ala Tyr Lys Ser Asn Asp Pro Gly Ile
        195                 200                 205

Leu Val Asn Ile Tyr Ser Met Gln Pro Gly Asp Tyr Lys Pro Pro Gly
    210                 215                 220

Pro Pro Val Trp Ser Gly
225                 230
```

<210> 57
<211> 1068
<212> DNA

<213> Thielavia terrestris

<400> 57

```
atgaagctgt acctggcggc ctttctaggc gccgtcgcca ccccgggagc gttcgctcat     60
cgtaggttcc ccgtctatct ccctaggggt agcaccacga ctaatttctc gtcgtccccc    120
tgtagaaatc cacgggattc tacttgtcaa cggcaccgaa acgccggaat ggaaatacgt    180
ccggtaatat ctaccttgct ctccttcttc cacaaccagc ctaacacatc atcagtgacg    240
tggcctggga gggcgcctac gaaccggaaa aataccccaa caccgagttc tttaagacgc    300
ccccgcagac ggacatcaac aacccgaaca tcacctgcgg caggaacgcg ttcgactcgg    360
ccagcaagac tgagacggcc gacatactgg ccggctcaga ggtcggcttc cgcgtctcgt    420
gggacggcaa cggcaagtac ggcgtgttct ggcatcccgg gccggggcag atctacctct    480
ctcgtgctcc gaacgacgac ctggaggact accgcggcga cggagactgg ttcaagatcg    540
caaccggcgc cgccgtctcc aataccgagt ggctgctgtg gaacaagcat gacgtgagcc    600
ccaacattcc tcgcccaatc gatccccaac ctggtcacca tggcggcgtc cgggatgcaa    660
agagactaac tccagaggaa cctacctagt tcaacttcac catccccaag acgacgccgc    720
cgggcaagta cctgatgcgc atcgagcagt tcatgccctc cacggtcgaa tacagccagt    780
ggtacgtcaa ctgcgcccac gtcaacatca tcggccccgg cggaggcacg ccgacgggct    840
ttgccaggtt tcccggcacc tacactgttg acgatcccgg taagccggac ctaccggaca    900
cagaggcctc gggatagctt gctaaccttg tttgctctct ctctttttct ctcccgacta    960
ggcatcaagg tgccgttgaa ccagatcgtc aacagcggag agttgccgca ggaccaactg   1020
aggctgctcg agtacaagcc cccgggccca gcgctgtgga ctggttga                1068
```

<210> 58
<211> 257
<212> PRT
<213> Thielavia terrestris

<400> 58

```
Met Lys Leu Tyr Leu Ala Ala Phe Leu Gly Ala Val Ala Thr Pro Gly
1               5                   10                  15

Ala Phe Ala His Gln Ile His Gly Ile Leu Leu Val Asn Gly Thr Glu
            20                  25                  30

Thr Pro Glu Trp Lys Tyr Val Arg Asp Val Ala Trp Glu Gly Ala Tyr
        35                  40                  45

Glu Pro Glu Lys Tyr Pro Asn Thr Glu Phe Phe Lys Thr Pro Pro Gln
    50                  55                  60

Thr Asp Ile Asn Asn Pro Asn Ile Thr Cys Gly Arg Asn Ala Phe Asp
65                  70                  75                  80

Ser Ala Ser Lys Thr Glu Thr Ala Asp Ile Leu Ala Gly Ser Glu Val
                85                  90                  95
```

```
Gly Phe Arg Val Ser Trp Asp Gly Asn Gly Lys Tyr Gly Val Phe Trp
            100                 105                 110

His Pro Gly Pro Gly Gln Ile Tyr Leu Ser Arg Ala Pro Asn Asp Asp
        115                 120                 125

Leu Glu Asp Tyr Arg Gly Asp Gly Asp Trp Phe Lys Ile Ala Thr Gly
    130                 135                 140

Ala Ala Val Ser Asn Thr Glu Trp Leu Leu Trp Asn Lys His Asp Phe
145                 150                 155                 160

Asn Phe Thr Ile Pro Lys Thr Thr Pro Pro Gly Lys Tyr Leu Met Arg
                165                 170                 175

Ile Glu Gln Phe Met Pro Ser Thr Val Glu Tyr Ser Gln Trp Tyr Val
            180                 185                 190

Asn Cys Ala His Val Asn Ile Ile Gly Pro Gly Gly Gly Thr Pro Thr
        195                 200                 205

Gly Phe Ala Arg Phe Pro Gly Thr Tyr Thr Val Asp Asp Pro Gly Ile
    210                 215                 220

Lys Val Pro Leu Asn Gln Ile Val Asn Ser Gly Glu Leu Pro Gln Asp
225                 230                 235                 240

Gln Leu Arg Leu Leu Glu Tyr Lys Pro Pro Gly Pro Ala Leu Trp Thr
                245                 250                 255

Gly
```

<210> 59
<211> 871
<212> DNA
<213> Thermoascus crustaceus

<400> 59

```
atggcctttt cccagataat ggctattacc ggcgtttttc ttgcctctgc ttccctggtg        60
gctggccatg gctttgttca gaatatcgtg attgatggta aaaggtacct aactacctac       120
cttactatct gatgtcattt acaagaaagg gcacagacac aagcggcaaa aaaaagaaag       180
aaagaaagaa agaaagaaag ctgacaaaaa ttcaacaagt tatggcgggt acatcgtgaa       240
ccaatatcca tacatgtcag atcctccgga ggtcgtcggc tggtctacca ccgcaaccga       300
cctcggattc gtggacggta ccggatacca aggacctgat atcatctgcc acaggggcgc       360
caagcctgca gccctgactg cccaagtggc cgccggagga accgtcaagc tggaatggac       420
tccatggcct gattctcacc acggcccggt gatcaactac cttgctcctt gcaacggtga       480

ctgttccacc gtggacaaga cccaattgaa attcttcaag atcgcccagg ccggtctcat       540
cgatgacaac agtcctcctg gtatctgggc ctcagacaat ctgatagcgg ccaacaacag       600
ctggactgtc accatcccaa ccacaactgc acctggaaac tatgttctaa ggcatgagat       660
cattgctctc cactcagctg ggaacaagga tggtgcgcag aactatcccc agtgcatcaa       720
cctgaaggtc actggaaatg gttctggcaa tcctcctgct ggtgctcttg gaacggcact       780
ctacaaggat acagatccgg gaattctgat caatatctac cagaaacttt ccagctatgt       840
tattcctggt cctgctttgt acactggtta g                                      871
```

<210> 60
<211> 251
<212> PRT
<213> Thermoascus crustaceus

<400> 60

```
Met Ala Phe Ser Gln Ile Met Ala Ile Thr Gly Val Phe Leu Ala Ser
1               5                   10                  15

Ala Ser Leu Val Ala Gly His Gly Phe Val Gln Asn Ile Val Ile Asp
            20                  25                  30

Gly Lys Ser Tyr Gly Gly Tyr Ile Val Asn Gln Tyr Pro Tyr Met Ser
        35                  40                  45

Asp Pro Pro Glu Val Val Gly Trp Ser Thr Thr Ala Thr Asp Leu Gly
    50                  55                  60

Phe Val Asp Gly Thr Gly Tyr Gln Gly Pro Asp Ile Ile Cys His Arg
65                  70                  75                  80

Gly Ala Lys Pro Ala Ala Leu Thr Ala Gln Val Ala Ala Gly Gly Thr
                85                  90                  95

Val Lys Leu Glu Trp Thr Pro Trp Pro Asp Ser His His Gly Pro Val
            100                 105                 110

Ile Asn Tyr Leu Ala Pro Cys Asn Gly Asp Cys Ser Thr Val Asp Lys
        115                 120                 125

Thr Gln Leu Lys Phe Phe Lys Ile Ala Gln Ala Gly Leu Ile Asp Asp
    130                 135                 140

Asn Ser Pro Pro Gly Ile Trp Ala Ser Asp Asn Leu Ile Ala Ala Asn
145                 150                 155                 160

Asn Ser Trp Thr Val Thr Ile Pro Thr Thr Thr Ala Pro Gly Asn Tyr
                165                 170                 175

Val Leu Arg His Glu Ile Ile Ala Leu His Ser Ala Gly Asn Lys Asp
            180                 185                 190

Gly Ala Gln Asn Tyr Pro Gln Cys Ile Asn Leu Lys Val Thr Gly Asn
        195                 200                 205

Gly Ser Gly Asn Pro Pro Ala Gly Ala Leu Gly Thr Ala Leu Tyr Lys
    210                 215                 220

Asp Thr Asp Pro Gly Ile Leu Ile Asn Ile Tyr Gln Lys Leu Ser Ser
225                 230                 235                 240

Tyr Val Ile Pro Gly Pro Ala Leu Tyr Thr Gly
                245                 250
```

<210> 61
<211> 1102
<212> DNA
<213> Thermoascus crustaceus

<400> 61

```
atgtcattct cgaagatact tgctatcgct ggggccatta cctacgcatc ttcagctgcc      60
gctcatggtt atgtccaggg aattgttgtc gatggcagct agtatgtcac tctggatgga     120
accttcagca cgtactgtac taacaatcag cagctacggg ggatatatgg tgacccaata     180
tccctacacc gctcaacctc cggaactcat cgcctggtcc actaaagcaa ccgatcttgg     240
gtttgtggac ggcagtggct atacttctcc tgatatcatc tgccataagg gtgctgagcc     300
tggtgcccag agcgccaaag tggcagctgg agggaccgtt gagctgcagt ggacggcatg     360
gcccgagtct cacaagggcc cagttattga ctacctcgcc gcctgcgacg gggactgctc     420
atctgttgat aagactgcac taaagttctt taagattgac gagagtggtc tgattgacgg     480
caacggtgct ggaacatggg cctctgatac gttgatcaaa aataacaaca gctggactgt     540
caccatccca agcacaattg cttccggaaa ctacgtacta agacacgaaa taattgcgct     600
ccattctgcc ggaaacaaag atggtgctca gaactatccc cagtgtatca acctcgaggt     660
cactggtagt ggcaccgaaa accctgctgg cactctcgga acagcgcttt acacagacac     720
tgatcctggc cttctggtca acatctacca gggtctgtcc aactattcaa tccctggtcc     780
tgctctgtat agcggcaaca gtgataacgc tggttccctc aaccctacca ccacgccgtc     840
aattcagaat gctgctgctg ctccctccac ttccacagca tctgttgtca ctgattcttc     900
gtcagccacc cagactgcta gtgtcgccgc cacgactcca gcctccactt cggctgttac     960
agcctcacca gctcccgata ctggaagcga cgtaaccaaa tatctggatt cgatgagctc    1020
ggatgaggtc ctcaccctgg tgcgcgggac cctgtcttgg ctggtttcta acaagaaaca    1080
tgcgcgggat ctttctcact ga                                             1102
```

<210> 62
<211> 349
<212> PRT
<213> Thermoascus crustaceus

<400> 62

```
Met Ser Phe Ser Lys Ile Leu Ala Ile Ala Gly Ala Ile Thr Tyr Ala
1               5                   10                  15

Ser Ser Ala Ala Ala His Gly Tyr Val Gln Gly Ile Val Val Asp Gly
            20                  25                  30

Ser Tyr Tyr Gly Gly Tyr Met Val Thr Gln Tyr Pro Tyr Thr Ala Gln
        35                  40                  45

Pro Pro Glu Leu Ile Ala Trp Ser Thr Lys Ala Thr Asp Leu Gly Phe
    50                  55                  60

Val Asp Gly Ser Gly Tyr Thr Ser Pro Asp Ile Ile Cys His Lys Gly
65                  70                  75                  80

Ala Glu Pro Gly Ala Gln Ser Ala Lys Val Ala Ala Gly Gly Thr Val
                85                  90                  95

Glu Leu Gln Trp Thr Ala Trp Pro Glu Ser His Lys Gly Pro Val Ile
            100                 105                 110

Asp Tyr Leu Ala Ala Cys Asp Gly Asp Cys Ser Ser Val Asp Lys Thr
        115                 120                 125

Ala Leu Lys Phe Phe Lys Ile Asp Glu Ser Gly Leu Ile Asp Gly Asn
    130                 135                 140

Gly Ala Gly Thr Trp Ala Ser Asp Thr Leu Ile Lys Asn Asn Asn Ser
145                 150                 155                 160

Trp Thr Val Thr Ile Pro Ser Thr Ile Ala Ser Gly Asn Tyr Val Leu
                165                 170                 175

Arg His Glu Ile Ile Ala Leu His Ser Ala Gly Asn Lys Asp Gly Ala
            180                 185                 190

Gln Asn Tyr Pro Gln Cys Ile Asn Leu Glu Val Thr Gly Ser Gly Thr
        195                 200                 205

Glu Asn Pro Ala Gly Thr Leu Gly Thr Ala Leu Tyr Thr Asp Thr Asp
    210                 215                 220
```

```
Pro Gly Leu Leu Val Asn Ile Tyr Gln Gly Leu Ser Asn Tyr Ser Ile
225                 230                 235                 240

Pro Gly Pro Ala Leu Tyr Ser Gly Asn Ser Asp Asn Ala Gly Ser Leu
                245                 250                 255

Asn Pro Thr Thr Thr Pro Ser Ile Gln Asn Ala Ala Ala Ala Pro Ser
            260                 265                 270

Thr Ser Thr Ala Ser Val Val Thr Asp Ser Ser Ser Ala Thr Gln Thr
        275                 280                 285

Ala Ser Val Ala Ala Thr Thr Pro Ala Ser Thr Ser Ala Val Thr Ala
    290                 295                 300

Ser Pro Ala Pro Asp Thr Gly Ser Asp Val Thr Lys Tyr Leu Asp Ser
305                 310                 315                 320

Met Ser Ser Asp Glu Val Leu Thr Leu Val Arg Gly Thr Leu Ser Trp
                325                 330                 335

Leu Val Ser Asn Lys Lys His Ala Arg Asp Leu Ser His
            340                 345
```

<210> 63
<211> 1493
<212> DNA
<213> Thermoascus crustaceus

<400> 63

```
atgttgtcat tcattcccac caagtcagct gcgctgacga ctcttctact tcttggaaca      60
gctcatgctc acactttgat gaccaccatg tttgtggacg gcgtcaacca gggagatggt     120
gtctgcattc gcatgaacaa tgacggcgga actgccaata cctatatcca gcctatcacg     180
agcaaggata tcgcctgcgg taagtaccca gatgtcatca tactctgcca taacatccgt     240
catatctact agaatcggag caatgttaag tatttccagg catccaaggc gaaatcggcg     300
cctcccgagt ctgcccagtc aaggcatctt ccaccctaac cttccaattc cgcgagcaac     360
ccaacaaccc aaactcctcc cctctcgatc catcgcacaa aggccccgcc gcggtgtacc     420
tgaaaaaggt cgactccgcc atcgcgagca acaacgccgc cggagacagc tggttcaaga     480
tctgggagtc cgtctacgac gagtccacgg gcaaatgggg cacgaccaag atgatcgaga     540
acaacgggca catctccgtc aaggtgcccg atgatatcga gggtggttac tatcttgccc     600
ggacggagct gctggcgcta cattctgcgg atcaggggga tccgcagttc tatgttggct     660
gtgcgcagct gtttatcgat tcggatggga cggcgaaacc gcccactgtt tctattggag     720
aggggacgta cgatctgagc atgcctgcca tgacgtataa tatctgggag acaccgttgg     780
ctctgccgta tccgatgtat gggcctcctg tctatacgcc tggctctggt tctggatcag     840
tccgtgcgac gagctcttct gctgtcccta ctgcaaccga atcctctttt gtagaggaaa     900
gagcaaaccc cgtcacggca aacagtgttt attctgcaag gggcaaattc aaaacctgga     960
ttgataaact gtcatggcgc gggaaggtcc gtgagaacgt cagacaagcc gcgggaagaa    1020
gaagcactct cgtccagact gtgggtctaa agccaaaagg ctgcatcttc gtcaatggaa    1080
actggtgcgg cttcgaggtt cccgactaca acgatgcgga gagctgctgg gctgtatgtt    1140
cccctcctta gcctcttaca tccctaagta ctacatttga aaacaacaaa aagaaatgta    1200
tatactaact acgtacgctc tactctaggc ctccgacaac tgctggaaac agtccgacgc    1260
ctgctggaac aagacccaac ccacgggcta caataactgc cagatctggc aggacaagaa    1320
atgcaaggtc atccaggatt cctgtagcgg acccaacccg catggaccac cgaataaggg    1380
caaggatttg actccggagt ggccgccact gaagggctcg atggatacgt tctccaagcg    1440
tactatcggt taccgcgatt ggattgttag aaggagaggt gcatgagggt gta           1493
```

<210> 64
<211> 436
<212> PRT
<213> Thermoascus crustaceus

<400> 64

```
Met Leu Ser Phe Ile Pro Thr Lys Ser Ala Ala Leu Thr Thr Leu Leu
1               5                   10                  15

Leu Leu Gly Thr Ala His Ala His Thr Leu Met Thr Thr Met Phe Val
            20                  25                  30

Asp Gly Val Asn Gln Gly Asp Gly Val Cys Ile Arg Met Asn Asn Asp
        35                  40                  45

Gly Gly Thr Ala Asn Thr Tyr Ile Gln Pro Ile Thr Ser Lys Asp Ile
    50                  55                  60

Ala Cys Gly Ile Gln Gly Glu Ile Gly Ala Ser Arg Val Cys Pro Val
65                  70                  75                  80

Lys Ala Ser Ser Thr Leu Thr Phe Gln Phe Arg Glu Gln Pro Asn Asn
                85                  90                  95

Pro Asn Ser Ser Pro Leu Asp Pro Ser His Lys Gly Pro Ala Ala Val
            100                 105                 110

Tyr Leu Lys Lys Val Asp Ser Ala Ile Ala Ser Asn Asn Ala Ala Gly
        115                 120                 125

Asp Ser Trp Phe Lys Ile Trp Glu Ser Val Tyr Asp Glu Ser Thr Gly
    130                 135                 140
```

```
Lys Trp Gly Thr Thr Lys Met Ile Glu Asn Asn Gly His Ile Ser Val
145                 150                 155                 160

Lys Val Pro Asp Asp Ile Glu Gly Gly Tyr Tyr Leu Ala Arg Thr Glu
                165                 170                 175

Leu Leu Ala Leu His Ser Ala Asp Gln Gly Asp Pro Gln Phe Tyr Val
            180                 185                 190

Gly Cys Ala Gln Leu Phe Ile Asp Ser Asp Gly Thr Ala Lys Pro Pro
        195                 200                 205

Thr Val Ser Ile Gly Glu Gly Thr Tyr Asp Leu Ser Met Pro Ala Met
    210                 215                 220

Thr Tyr Asn Ile Trp Glu Thr Pro Leu Ala Leu Pro Tyr Pro Met Tyr
225                 230                 235                 240

Gly Pro Pro Val Tyr Thr Pro Gly Ser Gly Ser Gly Ser Val Arg Ala
                245                 250                 255

Thr Ser Ser Ser Ala Val Pro Thr Ala Thr Glu Ser Ser Phe Val Glu
            260                 265                 270

Glu Arg Ala Asn Pro Val Thr Ala Asn Ser Val Tyr Ser Ala Arg Gly
        275                 280                 285

Lys Phe Lys Thr Trp Ile Asp Lys Leu Ser Trp Arg Gly Lys Val Arg
    290                 295                 300

Glu Asn Val Arg Gln Ala Ala Gly Arg Arg Ser Thr Leu Val Gln Thr
305                 310                 315                 320

Val Gly Leu Lys Pro Lys Gly Cys Ile Phe Val Asn Gly Asn Trp Cys
                325                 330                 335

Gly Phe Glu Val Pro Asp Tyr Asn Asp Ala Glu Ser Cys Trp Ala Ala
            340                 345                 350

Ser Asp Asn Cys Trp Lys Gln Ser Asp Ala Cys Trp Asn Lys Thr Gln
        355                 360                 365

Pro Thr Gly Tyr Asn Asn Cys Gln Ile Trp Gln Asp Lys Lys Cys Lys
    370                 375                 380

Val Ile Gln Asp Ser Cys Ser Gly Pro Asn Pro His Gly Pro Pro Asn
385                 390                 395                 400
```

Lys Gly Lys Asp Leu Thr Pro Glu Trp Pro Pro Leu Lys Gly Ser Met
405 410 415

Asp Thr Phe Ser Lys Arg Thr Ile Gly Tyr Arg Asp Trp Ile Val Arg
420 425 430

Arg Arg Gly Ala
435

<210> 65
<211> 1377
<212> DNA
<213> Trichoderma reesei

<400> 65

```
atggcgccct cagttacact gccgttgacc acggccatcc tggccattgc ccggctcgtc      60
gccgcccagc aaccgggtac cagcaccccc gaggtccatc ccaagttgac aacctacaag     120
tgtacaaagt ccgggggtg cgtggcccag gacacctcgg tggtccttga ctggaactac      180
cgctggatgc acgacgcaaa ctacaactcg tgcaccgtca acggcggcgt caacaccacg     240
ctctgccctg acgaggcgac ctgtggcaag aactgcttca tcgagggcgt cgactacgcc     300
gcctcgggcg tcacgacctc gggcagcagc ctcaccatga accagtacat gcccagcagc     360
tctggcggct acagcagcgt ctctcctcgg ctgtatctcc tggactctga cggtgagtac     420
gtgatgctga agctcaacgg ccaggagctg agcttcgacg tcgacctctc tgctctgccg     480
tgtggagaga acggctcgct ctacctgtct cagatggacg agaacggggg cgccaaccag     540
tataacacgg ccggtgccaa ctacgggagc ggctactgcg atgctcagtg ccccgtccag     600
acatggagga acggcaccct caacactagc caccagggct tctgctgcaa cgagatggat     660
atcctggagg gcaactcgag ggcgaatgcc ttgacccctc actcttgcac ggccacggcc     720
tgcgactctg ccggttgcgg cttcaacccc tatggcagcg gctacaaaag ctactacggc     780
cccggagata ccgttgacac ctccaagacc ttcaccatca tcacccagtt caacacggac     840
aacggctcgc cctcgggcaa ccttgtgagc atcacccgca agtaccagca aaacggcgtc     900
gacatcccca gcgcccagcc cggcggcgac accatctcgt cctgcccgtc cgcctcagcc     960
tacggcggcc tcgccaccat gggcaaggcc ctgagcagcg gcatggtgct cgtgttcagc    1020
atttggaacg acaacagcca gtacatgaac tggctcgaca gcggcaacgc cggcccctgc    1080
agcagcaccg agggcaaccc atccaacatc ctggccaaca accccaacac gcacgtcgtc    1140
ttctccaaca tccgctgggg agacattggg tctactacga actcgactgc gccccccgccc   1200
ccgcctgcgt ccagcacgac gttttcgact acacggagga gctcgacgac ttcgagcagc    1260
ccgagctgca cgcagactca ctgggggcag tgcggtggca ttgggtacag cgggtgcaag    1320
acgtgcacgt cgggcactac gtgccagtat agcaacgact actactcgca atgcctt       1377
```

<210> 66
<211> 459
<212> PRT
<213> Trichoderma reesei

<400> 66

```
Met Ala Pro Ser Val Thr Leu Pro Leu Thr Thr Ala Ile Leu Ala Ile
1               5                   10                  15

Ala Arg Leu Val Ala Ala Gln Gln Pro Gly Thr Ser Thr Pro Glu Val
            20                  25                  30

His Pro Lys Leu Thr Thr Tyr Lys Cys Thr Lys Ser Gly Gly Cys Val
        35                  40                  45

Ala Gln Asp Thr Ser Val Val Leu Asp Trp Asn Tyr Arg Trp Met His
    50                  55                  60

Asp Ala Asn Tyr Asn Ser Cys Thr Val Asn Gly Gly Val Asn Thr Thr
65                  70                  75                  80

Leu Cys Pro Asp Glu Ala Thr Cys Gly Lys Asn Cys Phe Ile Glu Gly
                85                  90                  95

Val Asp Tyr Ala Ala Ser Gly Val Thr Thr Ser Gly Ser Ser Leu Thr
            100                 105                 110

Met Asn Gln Tyr Met Pro Ser Ser Ser Gly Gly Tyr Ser Ser Val Ser
        115                 120                 125

Pro Arg Leu Tyr Leu Leu Asp Ser Asp Gly Glu Tyr Val Met Leu Lys
    130                 135                 140

Leu Asn Gly Gln Glu Leu Ser Phe Asp Val Asp Leu Ser Ala Leu Pro
145                 150                 155                 160

Cys Gly Glu Asn Gly Ser Leu Tyr Leu Ser Gln Met Asp Glu Asn Gly
                165                 170                 175

Gly Ala Asn Gln Tyr Asn Thr Ala Gly Ala Asn Tyr Gly Ser Gly Tyr
            180                 185                 190

Cys Asp Ala Gln Cys Pro Val Gln Thr Trp Arg Asn Gly Thr Leu Asn
        195                 200                 205

Thr Ser His Gln Gly Phe Cys Cys Asn Glu Met Asp Ile Leu Glu Gly
    210                 215                 220

Asn Ser Arg Ala Asn Ala Leu Thr Pro His Ser Cys Thr Ala Thr Ala
```

```
225                 230                 235                 240

Cys Asp Ser Ala Gly Cys Gly Phe Asn Pro Tyr Gly Ser Gly Tyr Lys
                245                 250                 255

Ser Tyr Tyr Gly Pro Gly Asp Thr Val Asp Thr Ser Lys Thr Phe Thr
            260                 265                 270

Ile Ile Thr Gln Phe Asn Thr Asp Asn Gly Ser Pro Ser Gly Asn Leu
        275                 280                 285

Val Ser Ile Thr Arg Lys Tyr Gln Gln Asn Gly Val Asp Ile Pro Ser
    290                 295                 300

Ala Gln Pro Gly Gly Asp Thr Ile Ser Ser Cys Pro Ser Ala Ser Ala
305                 310                 315                 320

Tyr Gly Gly Leu Ala Thr Met Gly Lys Ala Leu Ser Ser Gly Met Val
                325                 330                 335

Leu Val Phe Ser Ile Trp Asn Asp Asn Ser Gln Tyr Met Asn Trp Leu
            340                 345                 350

Asp Ser Gly Asn Ala Gly Pro Cys Ser Ser Thr Glu Gly Asn Pro Ser
        355                 360                 365

Asn Ile Leu Ala Asn Asn Pro Asn Thr His Val Val Phe Ser Asn Ile
    370                 375                 380

Arg Trp Gly Asp Ile Gly Ser Thr Thr Asn Ser Thr Ala Pro Pro Pro
385                 390                 395                 400

Pro Pro Ala Ser Ser Thr Thr Phe Ser Thr Thr Arg Arg Ser Ser Thr
                405                 410                 415

Thr Ser Ser Ser Pro Ser Cys Thr Gln Thr His Trp Gly Gln Cys Gly
            420                 425                 430

Gly Ile Gly Tyr Ser Gly Cys Lys Thr Cys Thr Ser Gly Thr Thr Cys
        435                 440                 445

Gln Tyr Ser Asn Asp Tyr Tyr Ser Gln Cys Leu
    450                 455
```

<210> 67
<211> 1254
<212> DNA

<213> Trichoderma reesei

<400> 67

```
atgaacaagt ccgtggctcc attgctgctt gcagcgtcca tactatatgg cggcgccgtc      60
gcacagcaga ctgtctgggg ccagtgtgga ggtattggtt ggagcggacc tacgaattgt     120
gctcctggct cagcttgttc gaccctcaat ccttattatg cgcaatgtat tccgggagcc     180
actactatca ccacttcgac ccggccacca tccggtccaa ccaccaccac cagggctacc     240
tcaacaagct catcaactcc acccacgagc tctggggtcc gatttgccgg cgttaacatc     300
gcgggttttg actttggctg taccacagat ggcacttgcg ttacctcgaa ggtttatcct     360
ccgttgaaga acttcaccgg ctcaaacaac taccccgatg gcatcggcca gatgcagcac     420
ttcgtcaacg aggacgggat gactattttc cgcttacctg tcggatggca gtacctcgtc     480
aacaacaatt tgggcggcaa tcttgattcc acgagcattt ccaagtatga tcagcttgtt     540
caggggtgcc tgtctctggg cgcatactgc atcgtcgaca tccacaatta tgctcgatgg     600
aacggtggga tcattggtca gggcggccct actaatgctc aattcacgag cctttggtcg     660
cagttggcat caaagtacgc atctcagtcg agggtgtggt tcggcatcat gaatgagccc     720
cacgacgtga acatcaacac ctgggctgcc acggtccaag aggttgtaac cgcaatccgc     780
aacgctggtg ctacgtcgca attcatctct ttgcctggaa atgattggca atctgctggg     840
gctttcatat ccgatggcag tgcagccgcc ctgtctcaag tcacgaaccc ggatgggtca     900
acaacgaatc tgatttttga cgtgcacaaa tacttggact cagacaactc cggtactcac     960
gccgaatgta ctacaaataa cattgacggc gccttttctc cgcttgccac ttggctccga    1020
cagaacaatc gccaggctat cctgacagaa accggtggtg gcaacgttca gtcctgcata    1080
caagacatgt gccagcaaat ccaatatctc aaccagaact cagatgtcta tcttggctat    1140
gttggttggg gtgccggatc atttgatagc acgtatgtcc tgacggaaac accgactagc    1200
agtggtaact catggacgga cacatccttg gtcagctcgt gtctcgcaag aaag          1254
```

<210> 68
<211> 418
<212> PRT
<213> Trichoderma reesei

<400> 68

```
Met Asn Lys Ser Val Ala Pro Leu Leu Leu Ala Ala Ser Ile Leu Tyr
1               5                   10                  15

Gly Gly Ala Val Ala Gln Gln Thr Val Trp Gly Gln Cys Gly Gly Ile
            20                  25                  30

Gly Trp Ser Gly Pro Thr Asn Cys Ala Pro Gly Ser Ala Cys Ser Thr
        35                  40                  45

Leu Asn Pro Tyr Tyr Ala Gln Cys Ile Pro Gly Ala Thr Thr Ile Thr
    50                  55                  60
```

```
Thr Ser Thr Arg Pro Pro Ser Gly Pro Thr Thr Thr Thr Arg Ala Thr
65                  70                  75                  80

Ser Thr Ser Ser Ser Thr Pro Pro Thr Ser Ser Gly Val Arg Phe Ala
                85                  90                  95

Gly Val Asn Ile Ala Gly Phe Asp Phe Gly Cys Thr Thr Asp Gly Thr
            100                 105                 110

Cys Val Thr Ser Lys Val Tyr Pro Pro Leu Lys Asn Phe Thr Gly Ser
        115                 120                 125

Asn Asn Tyr Pro Asp Gly Ile Gly Gln Met Gln His Phe Val Asn Glu
    130                 135                 140

Asp Gly Met Thr Ile Phe Arg Leu Pro Val Gly Trp Gln Tyr Leu Val
145                 150                 155                 160

Asn Asn Asn Leu Gly Gly Asn Leu Asp Ser Thr Ser Ile Ser Lys Tyr
                165                 170                 175

Asp Gln Leu Val Gln Gly Cys Leu Ser Leu Gly Ala Tyr Cys Ile Val
            180                 185                 190

Asp Ile His Asn Tyr Ala Arg Trp Asn Gly Gly Ile Ile Gly Gln Gly
        195                 200                 205

Gly Pro Thr Asn Ala Gln Phe Thr Ser Leu Trp Ser Gln Leu Ala Ser
    210                 215                 220

Lys Tyr Ala Ser Gln Ser Arg Val Trp Phe Gly Ile Met Asn Glu Pro
225                 230                 235                 240

His Asp Val Asn Ile Asn Thr Trp Ala Ala Thr Val Gln Glu Val Val
                245                 250                 255

Thr Ala Ile Arg Asn Ala Gly Ala Thr Ser Gln Phe Ile Ser Leu Pro
            260                 265                 270

Gly Asn Asp Trp Gln Ser Ala Gly Ala Phe Ile Ser Asp Gly Ser Ala
        275                 280                 285

Ala Ala Leu Ser Gln Val Thr Asn Pro Asp Gly Ser Thr Thr Asn Leu
    290                 295                 300

Ile Phe Asp Val His Lys Tyr Leu Asp Ser Asp Asn Ser Gly Thr His
305                 310                 315                 320
```

Ala Glu Cys Thr Thr Asn Asn Ile Asp Gly Ala Phe Ser Pro Leu Ala
325 330 335

Thr Trp Leu Arg Gln Asn Asn Arg Gln Ala Ile Leu Thr Glu Thr Gly
340 345 350

Gly Gly Asn Val Gln Ser Cys Ile Gln Asp Met Cys Gln Gln Ile Gln
355 360 365

Tyr Leu Asn Gln Asn Ser Asp Val Tyr Leu Gly Tyr Val Gly Trp Gly
370 375 380

Ala Gly Ser Phe Asp Ser Thr Tyr Val Leu Thr Glu Thr Pro Thr Ser
385 390 395 400

Ser Gly Asn Ser Trp Thr Asp Thr Ser Leu Val Ser Ser Cys Leu Ala
405 410 415

Arg Lys

<210> 69
<211> 702
<212> DNA
<213> Trichoderma reesei

<400> 69

atgaagttcc ttcaagtcct ccctgccctc ataccggccg ccctggccca aaccagctgt 60

gaccagtggg caaccttcac tggcaacggc tacacagtca gcaacaacct ttggggagca 120

tcagccggct ctggatttgg ctgcgtgacg gcggtatcgc tcagcggcgg ggcctcctgg 180

cacgcagact ggcagtggtc cggcggccag aacaacgtca agtcgtacca gaactctcag 240

attgccattc cccagaagag gaccgtcaac agcatcagca gcatgcccac cactgccagc 300

tggagctaca gcgggagcaa catccgcgct aatgttgcgt atgacttgtt caccgcagcc 360

aacccgaatc atgtcacgta ctcgggagac tacgaactca tgatctggct tggcaaatac 420

ggcgatattg ggccgattgg gtcctcacag ggaacagtca acgtcggtgg ccagagctgg 480

acgctctact atggctacaa cggagccatg caagtctatt cctttgtggc ccagaccaac 540

actaccaact acagcggaga tgtcaagaac ttcttcaatt atctccgaga caataaagga 600

tacaacgctg caggccaata tgttcttagc taccaatttg gtaccgagcc cttcacgggc 660

agtggaactc tgaacgtcgc atcctggacc gcatctatca ac 702

<210> 70
<211> 234
<212> PRT

<213> Trichoderma reesei

<400> 70

```
Met Lys Phe Leu Gln Val Leu Pro Ala Leu Ile Pro Ala Ala Leu Ala
1               5                   10                  15

Gln Thr Ser Cys Asp Gln Trp Ala Thr Phe Thr Gly Asn Gly Tyr Thr
            20                  25                  30

Val Ser Asn Asn Leu Trp Gly Ala Ser Ala Gly Ser Gly Phe Gly Cys
        35                  40                  45

Val Thr Ala Val Ser Leu Ser Gly Gly Ala Ser Trp His Ala Asp Trp
    50                  55                  60

Gln Trp Ser Gly Gly Gln Asn Asn Val Lys Ser Tyr Gln Asn Ser Gln
65                  70                  75                  80

Ile Ala Ile Pro Gln Lys Arg Thr Val Asn Ser Ile Ser Ser Met Pro
                85                  90                  95

Thr Thr Ala Ser Trp Ser Tyr Ser Gly Ser Asn Ile Arg Ala Asn Val
            100                 105                 110

Ala Tyr Asp Leu Phe Thr Ala Ala Asn Pro Asn His Val Thr Tyr Ser
        115                 120                 125

Gly Asp Tyr Glu Leu Met Ile Trp Leu Gly Lys Tyr Gly Asp Ile Gly
    130                 135                 140

Pro Ile Gly Ser Ser Gln Gly Thr Val Asn Val Gly Gly Gln Ser Trp
145                 150                 155                 160

Thr Leu Tyr Tyr Gly Tyr Asn Gly Ala Met Gln Val Tyr Ser Phe Val
                165                 170                 175

Ala Gln Thr Asn Thr Thr Asn Tyr Ser Gly Asp Val Lys Asn Phe Phe
            180                 185                 190

Asn Tyr Leu Arg Asp Asn Lys Gly Tyr Asn Ala Ala Gly Gln Tyr Val
        195                 200                 205

Leu Ser Tyr Gln Phe Gly Thr Glu Pro Phe Thr Gly Ser Gly Thr Leu
    210                 215                 220

Asn Val Ala Ser Trp Thr Ala Ser Ile Asn
225                 230
```

<210> 71
<211> 726

<212> DNA
<213> Trichoderma reesei

<400> 71

```
atgaaggcaa ctctggttct cggctccctc attgtaggcg ccgtttccgc gtacaaggcc        60
accaccacgc gctactacga tgggcaggag ggtgcttgcg gatgcggctc gagctccggc       120
gcattcccgt ggcagctcgg catcggcaac ggagtctaca cggctgccgg ctcccaggct       180
ctcttcgaca cggccggagc ttcatggtgc ggcgccggct gcggtaaatg ctaccagctc       240
acctcgacgg gccaggcgcc ctgctccagc tgcggcacgg gcggtgctgc tggccagagc       300
atcatcgtca tggtgaccaa cctgtgcccg aacaatggga acgcgcagtg gtgcccggtg       360
gtcggcggca ccaaccaata cggctacagc taccatttcg acatcatggc gcagaacgag       420
atctttggag acaatgtcgt cgtcgacttt gagcccattg cttgccccgg gcaggctgcc       480
tctgactggg ggacgtgcct ctgcgtggga cagcaagaga cggatcccac gcccgtcctc       540
ggcaacgaca cgggctcaac tcctcccggg agctcgccgc cagcgacatc gtcgagtccg       600
ccgtctggcg gcggccagca gacgctctat ggccagtgtg gaggtgccgg ctggacggga       660
cctacgacgt gccaggcccc agggacctgc aaggttcaga accagtggta ctcccagtgt       720
cttcct                                                                  726
```

<210> 72
<211> 242
<212> PRT
<213> Trichoderma reesei

<400> 72

```
Met Lys Ala Thr Leu Val Leu Gly Ser Leu Ile Val Gly Ala Val Ser
1               5                   10                  15

Ala Tyr Lys Ala Thr Thr Thr Arg Tyr Tyr Asp Gly Gln Glu Gly Ala
            20                  25                  30

Cys Gly Cys Gly Ser Ser Ser Gly Ala Phe Pro Trp Gln Leu Gly Ile
        35                  40                  45

Gly Asn Gly Val Tyr Thr Ala Ala Gly Ser Gln Ala Leu Phe Asp Thr
    50                  55                  60

Ala Gly Ala Ser Trp Cys Gly Ala Gly Cys Gly Lys Cys Tyr Gln Leu
65                  70                  75                  80

Thr Ser Thr Gly Gln Ala Pro Cys Ser Ser Cys Gly Thr Gly Gly Ala
                85                  90                  95

Ala Gly Gln Ser Ile Ile Val Met Val Thr Asn Leu Cys Pro Asn Asn
            100                 105                 110

Gly Asn Ala Gln Trp Cys Pro Val Val Gly Gly Thr Asn Gln Tyr Gly
```

```
            115                 120                 125

Tyr Ser Tyr His Phe Asp Ile Met Ala Gln Asn Glu Ile Phe Gly Asp
    130                 135                 140

Asn Val Val Val Asp Phe Glu Pro Ile Ala Cys Pro Gly Gln Ala Ala
145                 150                 155                 160

Ser Asp Trp Gly Thr Cys Leu Cys Val Gly Gln Gln Glu Thr Asp Pro
                165                 170                 175

Thr Pro Val Leu Gly Asn Asp Thr Gly Ser Thr Pro Pro Gly Ser Ser
            180                 185                 190

Pro Pro Ala Thr Ser Ser Ser Pro Pro Ser Gly Gly Gly Gln Gln Thr
        195                 200                 205

Leu Tyr Gly Gln Cys Gly Gly Ala Gly Trp Thr Gly Pro Thr Thr Cys
    210                 215                 220

Gln Ala Pro Gly Thr Cys Lys Val Gln Asn Gln Trp Tyr Ser Gln Cys
225                 230                 235                 240

Leu Pro
```

<210> 73
<211> 923
<212> DNA
<213> Trichoderma reesei

<400> 73

```
atgcgttcct ccccccctcct ccgctccgcc gttgtggccg ccctgccggt gttggccctt      60
gccgctgatg gcaggtccac ccgctactgg gactgctgca agccttcgtg cggctgggcc     120
aagaaggctc ccgtgaacca gcctgtcttt tcctgcaacg ccaacttcca gcgtatcacg     180
gacttcgacg ccaagtccgg ctgcgagccg ggcggtgtcg cctactcgtg cgccgaccag     240
accccatggg ctgtgaacga cgacttcgcg ctcggttttg ctgccacctc tattgccggc     300
agcaatgagg cgggctggtg ctgcgcctgc tacgagctca ccttcacatc cggtcctgtt     360
gctggcaaga agatggtcgt ccagtccacc agcactggcg gtgatcttgg cagcaaccac     420
ttcgatctca acatccccgg cggcggcgtc ggcatcttcg acggatgcac tccccagttc     480
ggcggtctgc ccggccagcg ctacggcggc atctcgtccc gcaacgagtg cgatcggttc     540
cccgacgccc tcaagcccgg ctgctactgg cgcttcgact ggttcaagaa cgccgacaat     600
ccgagcttca gcttccgtca ggtccagtgc ccagccgagc tcgtcgctcg caccggatgc     660
cgccgcaacg acgacggcaa cttccctgcc gtccagatcc cctccagcag caccagctct     720
ccggtcaacc agcctaccag caccagcacc acgtccacct ccaccacctc gagcccgcca     780
gtccagccta cgactcccag cggctgcact gctgagaggt gggctcagtg cggcggcaat     840
ggctggagcg gctgcaccac ctgcgtcgct ggcagcactt gcacgaagat taatgactgg     900
taccatcagt gcctgtagaa ttc                                             923
```

<210> 74
<211> 305
<212> PRT
<213> Trichoderma reesei

<400> 74

```
Met Arg Ser Ser Pro Leu Leu Arg Ser Ala Val Val Ala Ala Leu Pro
1               5                   10                  15

Val Leu Ala Leu Ala Ala Asp Gly Arg Ser Thr Arg Tyr Trp Asp Cys
            20                  25                  30

Cys Lys Pro Ser Cys Gly Trp Ala Lys Lys Ala Pro Val Asn Gln Pro
        35                  40                  45

Val Phe Ser Cys Asn Ala Asn Phe Gln Arg Ile Thr Asp Phe Asp Ala
    50                  55                  60

Lys Ser Gly Cys Glu Pro Gly Gly Val Ala Tyr Ser Cys Ala Asp Gln
65                  70                  75                  80

Thr Pro Trp Ala Val Asn Asp Asp Phe Ala Leu Gly Phe Ala Ala Thr
                85                  90                  95

Ser Ile Ala Gly Ser Asn Glu Ala Gly Trp Cys Cys Ala Cys Tyr Glu
            100                 105                 110

Leu Thr Phe Thr Ser Gly Pro Val Ala Gly Lys Lys Met Val Val Gln
        115                 120                 125

Ser Thr Ser Thr Gly Gly Asp Leu Gly Ser Asn His Phe Asp Leu Asn
    130                 135                 140

Ile Pro Gly Gly Gly Val Gly Ile Phe Asp Gly Cys Thr Pro Gln Phe
145                 150                 155                 160

Gly Gly Leu Pro Gly Gln Arg Tyr Gly Gly Ile Ser Ser Arg Asn Glu
                165                 170                 175

Cys Asp Arg Phe Pro Asp Ala Leu Lys Pro Gly Cys Tyr Trp Arg Phe
            180                 185                 190

Asp Trp Phe Lys Asn Ala Asp Asn Pro Ser Phe Ser Phe Arg Gln Val
```

```
        195                 200                 205

Gln Cys Pro Ala Glu Leu Val Ala Arg Thr Gly Cys Arg Arg Asn Asp
    210                 215                 220

Asp Gly Asn Phe Pro Ala Val Gln Ile Pro Ser Ser Ser Thr Ser Ser
225                 230                 235                 240

Pro Val Asn Gln Pro Thr Ser Thr Ser Thr Thr Ser Thr Ser Thr Thr
                245                 250                 255

Ser Ser Pro Pro Val Gln Pro Thr Thr Pro Ser Gly Cys Thr Ala Glu
            260                 265                 270

Arg Trp Ala Gln Cys Gly Gly Asn Gly Trp Ser Gly Cys Thr Thr Cys
        275                 280                 285

Val Ala Gly Ser Thr Cys Thr Lys Ile Asn Asp Trp Tyr His Gln Cys
    290                 295                 300

Leu
305
```

<210> 75
<211> 1188
<212> DNA
<213> Myceliophthora thermophila

<400> 75

```
cgacttgaaa cgccccaaat gaagtcctcc atcctcgcca gcgtcttcgc cacgggcgcc      60
gtggctcaaa gtggtccgtg gcagcaatgt ggtggcatcg gatggcaagg atcgaccgac     120
tgtgtgtcgg gctaccactg cgtctaccag aacgattggt acagccagtg cgtgcctggc     180
gcggcgtcga caacgctgca gacatcgacc acgtccaggc ccaccgccac cagcaccgcc     240
cctccgtcgt ccaccacctc gcctagcaag ggcaagctga agtggctcgg cagcaacgag     300
tcgggcgccg agttcgggga gggcaattac cccggcctct ggggcaagca cttcatcttc     360
ccgtcgactt cggcgattca gacgctcatc aatgatggat acaacatctt ccggatcgac     420
ttctcgatgg agcgtctggt gcccaaccag ttgacgtcgt ccttcgacca gggttacctc     480
cgcaacctga ccgaggtggt caacttcgtg acgaacgcgg gcaagtacgc cgtcctggac     540
ccgcacaact acggccggta ctacggcaac atcatcacgg acacgaacgc gttccggacc     600
ttctggacca acctggccaa gcagttcgcc tccaactcgc tcgtcatctt cgacaccaac     660
aacgagtaca acacgatgga ccagaccctg gtgctcaacc tcaaccaggc cgccatcgac     720
ggcatccggg ccgccggcgc gacctcgcag tacatcttcg tcgagggcaa cgcgtggagc     780
ggggcctgga gctggaacac gaccaacacc aacatggccg ccctgacgga cccgcagaac     840

aagatcgtgt acgagatgca ccagtacctc gactcggaca gctcgggcac ccacgccgag     900
tgcgtcagca gcaccatcgg cgcccagcgc gtcgtcggag ccacccagtg gctccgcgcc     960
aacggcaagc tcggcgtcct cggcgagttc gccggcggcg ccaacgccgt ctgccagcag    1020
gccgtcaccg gcctcctcga ccacctccag gacaacagcg acgtctggct gggtgccctc    1080
tggtgggccg ccggtccctg gtggggcgac tacatgtact cgttcgagcc tccttcgggc    1140
accggctatg tcaactacaa ctcgatcttg aagaagtact tgccgtaa                 1188
```

<210> 76
<211> 389
<212> PRT
<213> Myceliophthora thermophila

<400> 76

```
Met Lys Ser Ser Ile Leu Ala Ser Val Phe Ala Thr Gly Ala Val Ala
1               5                   10                  15

Gln Ser Gly Pro Trp Gln Gln Cys Gly Gly Ile Gly Trp Gln Gly Ser
            20                  25                  30

Thr Asp Cys Val Ser Gly Tyr His Cys Val Tyr Gln Asn Asp Trp Tyr
        35                  40                  45

Ser Gln Cys Val Pro Gly Ala Ala Ser Thr Thr Leu Gln Thr Ser Thr
    50                  55                  60

Thr Ser Arg Pro Thr Ala Thr Ser Thr Ala Pro Pro Ser Ser Thr Thr
65                  70                  75                  80

Ser Pro Ser Lys Gly Lys Leu Lys Trp Leu Gly Ser Asn Glu Ser Gly
                85                  90                  95

Ala Glu Phe Gly Glu Gly Asn Tyr Pro Gly Leu Trp Gly Lys His Phe
            100                 105                 110

Ile Phe Pro Ser Thr Ser Ala Ile Gln Thr Leu Ile Asn Asp Gly Tyr
        115                 120                 125

Asn Ile Phe Arg Ile Asp Phe Ser Met Glu Arg Leu Val Pro Asn Gln
    130                 135                 140

Leu Thr Ser Ser Phe Asp Gln Gly Tyr Leu Arg Asn Leu Thr Glu Val
145                 150                 155                 160

Val Asn Phe Val Thr Asn Ala Gly Lys Tyr Ala Val Leu Asp Pro His
                165                 170                 175

Asn Tyr Gly Arg Tyr Tyr Gly Asn Ile Ile Thr Asp Thr Asn Ala Phe
```

```
                180                 185                 190

Arg Thr Phe Trp Thr Asn Leu Ala Lys Gln Phe Ala Ser Asn Ser Leu
        195                 200                 205

Val Ile Phe Asp Thr Asn Asn Glu Tyr Asn Thr Met Asp Gln Thr Leu
    210                 215                 220

Val Leu Asn Leu Asn Gln Ala Ala Ile Asp Gly Ile Arg Ala Ala Gly
225                 230                 235                 240

Ala Thr Ser Gln Tyr Ile Phe Val Glu Gly Asn Ala Trp Ser Gly Ala
                245                 250                 255

Trp Ser Trp Asn Thr Thr Asn Thr Asn Met Ala Ala Leu Thr Asp Pro
            260                 265                 270

Gln Asn Lys Ile Val Tyr Glu Met His Gln Tyr Leu Asp Ser Asp Ser
        275                 280                 285

Ser Gly Thr His Ala Glu Cys Val Ser Ser Thr Ile Gly Ala Gln Arg
    290                 295                 300

Val Val Gly Ala Thr Gln Trp Leu Arg Ala Asn Gly Lys Leu Gly Val
305                 310                 315                 320

Leu Gly Glu Phe Ala Gly Gly Ala Asn Ala Val Cys Gln Gln Ala Val
                325                 330                 335

Thr Gly Leu Leu Asp His Leu Gln Asp Asn Ser Asp Val Trp Leu Gly
            340                 345                 350

Ala Leu Trp Trp Ala Ala Gly Pro Trp Trp Gly Asp Tyr Met Tyr Ser
        355                 360                 365

Phe Glu Pro Pro Ser Gly Thr Gly Tyr Val Asn Tyr Asn Ser Ile Leu
    370                 375                 380

Lys Lys Tyr Leu Pro
385
```

<210> 77
<211> 1232
<212> DNA
<213> BASIDIOMYCETE CBS 495.95

<400> 77

```
ggatccactt agtaacggcc gccagtgtgc tggaaagcat gaagtctctc ttcctgtcac     60
ttgtagcgac cgtcgcgctc agctcgccag tattctctgt cgcagtctgg gggcaatgcg    120
gcggcattgg cttcagcgga agcaccgtct gtgatgcagg cgccggctgt gtgaagctca    180
acgactatta ctctcaatgc caacccggcg ctcccactgc tacatccgcg gcgccaagta    240
gcaacgcacc gtccggcact tcgacggcct cggccccctc ctccagcctt tgctctggca    300
gccgcacgcc gttccagttc ttcggtgtca acgaatccgg cgcggagttc ggcaacctga    360
acatccccgg tgttctgggc accgactaca cctggccgtc gccatccagc attgacttct    420
tcatgggcaa gggaatgaat accttccgta ttccgttcct catggagcgt cttgtccccc    480
ctgccactgg catcacagga cctctcgacc agacgtactt gggcggcctg cagacgattg    540
tcaactacat caccggcaaa ggcggctttg ctctcattga cccgcacaac tttatgatct    600
acaatggcca gacgatctcc agtaccagcg acttccagaa gttctggcag aacctcgcag    660
gagtgtttaa atcgaacagt cacgtcatct tcgatgttat gaacgagcct cacgatattc    720
ccgcccagac cgtgttccaa ctgaaccaag ccgctgtcaa tggcatccgt gcgagcggtg    780
cgacgtcgca gctcattctg gtcgagggca caagctggac tggagcctgg acctggacga    840
cctctggcaa cagcgatgca ttcggtgcca ttaaggatcc caacaacaac gtcgcgatcc    900
agatgcatca gtacctggat agcgatggct ctggcacttc gcagacctgc gtgtctccca    960
ccatcggtgc cgagcggttg caggctgcga ctcaatggtt gaagcagaac aacctcaagg   1020
gcttcctggg cgagatcggc gccggctcta actccgcttg catcagcgct gtgcagggtg   1080
cgttgtgttc gatgcagcaa tctggtgtgt ggctcggcgc tctctggtgg gctgcgggcc   1140
cgtggtgggg cgactactac cagtccatcg agccgccctc tggcccggcg gtgtccgcga   1200
tcctcccgca ggccctgctg ccgttcgcgt aa                                 1232
```

<210> 78
<211> 397
<212> PRT
<213> BASIDIOMYCETE CBS 495.95

<400> 78

```
Met Lys Ser Leu Phe Leu Ser Leu Val Ala Thr Val Ala Leu Ser Ser
1               5                   10                  15

Pro Val Phe Ser Val Ala Val Trp Gly Gln Cys Gly Gly Ile Gly Phe
            20                  25                  30

Ser Gly Ser Thr Val Cys Asp Ala Gly Ala Gly Cys Val Lys Leu Asn
        35                  40                  45

Asp Tyr Tyr Ser Gln Cys Gln Pro Gly Ala Pro Thr Ala Thr Ser Ala
    50                  55                  60

Ala Pro Ser Ser Asn Ala Pro Ser Gly Thr Ser Thr Ala Ser Ala Pro
65                  70                  75                  80
```

```
Ser Ser Ser Leu Cys Ser Gly Ser Arg Thr Pro Phe Gln Phe Phe Gly
                85                  90                  95

Val Asn Glu Ser Gly Ala Glu Phe Gly Asn Leu Asn Ile Pro Gly Val
            100                 105                 110

Leu Gly Thr Asp Tyr Thr Trp Pro Ser Pro Ser Ser Ile Asp Phe Phe
        115                 120                 125

Met Gly Lys Gly Met Asn Thr Phe Arg Ile Pro Phe Leu Met Glu Arg
    130                 135                 140

Leu Val Pro Pro Ala Thr Gly Ile Thr Gly Pro Leu Asp Gln Thr Tyr
145                 150                 155                 160

Leu Gly Gly Leu Gln Thr Ile Val Asn Tyr Ile Thr Gly Lys Gly Gly
                165                 170                 175

Phe Ala Leu Ile Asp Pro His Asn Phe Met Ile Tyr Asn Gly Gln Thr
            180                 185                 190

Ile Ser Ser Thr Ser Asp Phe Gln Lys Phe Trp Gln Asn Leu Ala Gly
        195                 200                 205

Val Phe Lys Ser Asn Ser His Val Ile Phe Asp Val Met Asn Glu Pro
    210                 215                 220

His Asp Ile Pro Ala Gln Thr Val Phe Gln Leu Asn Gln Ala Ala Val
225                 230                 235                 240

Asn Gly Ile Arg Ala Ser Gly Ala Thr Ser Gln Leu Ile Leu Val Glu
                245                 250                 255

Gly Thr Ser Trp Thr Gly Ala Trp Thr Trp Thr Thr Ser Gly Asn Ser
            260                 265                 270

Asp Ala Phe Gly Ala Ile Lys Asp Pro Asn Asn Asn Val Ala Ile Gln
        275                 280                 285

Met His Gln Tyr Leu Asp Ser Asp Gly Ser Gly Thr Ser Gln Thr Cys
    290                 295                 300

Val Ser Pro Thr Ile Gly Ala Glu Arg Leu Gln Ala Ala Thr Gln Trp
305                 310                 315                 320

Leu Lys Gln Asn Asn Leu Lys Gly Phe Leu Gly Glu Ile Gly Ala Gly
                325                 330                 335
```

```
Ser Asn Ser Ala Cys Ile Ser Ala Val Gln Gly Ala Leu Cys Ser Met
            340                 345                 350

Gln Gln Ser Gly Val Trp Leu Gly Ala Leu Trp Trp Ala Ala Gly Pro
        355                 360                 365

Trp Trp Gly Asp Tyr Tyr Gln Ser Ile Glu Pro Pro Ser Gly Pro Ala
    370                 375                 380

Val Ser Ala Ile Leu Pro Gln Ala Leu Leu Pro Phe Ala
385                 390                 395
```

<210> 79
<211> 1303
<212> DNA
<213> BASIDIOMYCETE CBS 495.95

<400> 79

```
ggaaagcgtc agtatggtga aatttgcgct tgtggcaact gtcggcgcaa tcttgagcgc      60
ttctgcggcc aatgcggctt ctatctacca gcaatgtgga ggcattggat ggtctgggtc     120
cactgtttgc gacgccggtc tcgcttgcgt tatcctcaat gcgtactact ttcagtgctt     180
gacgcccgcc gcgggccaga caacgacggg ctcgggcgca ccggcgtcaa catcaacctc     240
tcactcaacg gtcactacgg ggagctcaca ctcaacaacc gggacgacgg cgacgaaaac     300
aactaccact ccgtcgacca ccacgaccct acccgccatc tctgtgtctg gtcgcgtctg     360
ctctggctcc aggacgaagt tcaagttctt cggtgtgaat gaaagcggcg ccgaattcgg     420
gaacactgct tggccagggc agctcgggaa agactataca tggccttcgc ctagcagcgt     480
ggactacttc atgggggctg gattcaatac attccgtatc accttcttga tggagcgtat     540
gagccctccg gctaccggac tcactggccc attcaaccag acgtacctgt cgggcctcac     600
caccattgtc gactacatca cgaacaaagg aggatacgct cttattgacc cccacaactt     660
catgcgttac aacaacggca taatcagcag cacatctgac ttcgcgactt ggtggagcaa     720
tttggccact gtattcaaat ccacgaagaa cgccatcttc gacatccaga acgagccgta     780
cggaatcgat gcgcagaccg tatacgaact gaatcaagct gccatcaatt cgatccgcgc     840
cgctggcgct acgtcacagt tgattctggt tgaaggaacg tcatacactg gagcttggac     900
gtgggtctcg tccggaaacg gagctgcttt cgcggccgtt acggatcctt acaacaacac     960
ggcaattgaa atgcaccaat acctcgacag cgacggttct gggacaaacg aagactgtgt    1020
ctcctccacc attgggtcgc aacgtctcca agctgccact gcgtggctgc aacaaacagg    1080
actcaaggga ttcctcggag agacgggtgc tgggtcgaat tcccagtgca tcgacgccgt    1140
gttcgatgaa ctttgctata tgcaacagca aggcggctcc tggatcggtg cactctggtg    1200
ggctgcgggt ccctggtggg gcacgtacat ttactcgatt gaacctccga gcggtgccgc    1260
tatcccagaa gtccttcctc agggtctcgc tccattcctc tag                      1303
```

<210> 80
<211> 429
<212> PRT
<213> BASIDIOMYCETE CBS 495.95

<400> 80

```
Met Val Lys Phe Ala Leu Val Ala Thr Val Gly Ala Ile Leu Ser Ala
1               5                   10                  15

Ser Ala Ala Asn Ala Ala Ser Ile Tyr Gln Gln Cys Gly Gly Ile Gly
            20                  25                  30

Trp Ser Gly Ser Thr Val Cys Asp Ala Gly Leu Ala Cys Val Ile Leu
        35                  40                  45

Asn Ala Tyr Tyr Phe Gln Cys Leu Thr Pro Ala Ala Gly Gln Thr Thr
    50                  55                  60

Thr Gly Ser Gly Ala Pro Ala Ser Thr Ser Thr Ser His Ser Thr Val
65                  70                  75                  80

Thr Thr Gly Ser Ser His Ser Thr Thr Gly Thr Thr Ala Thr Lys Thr
                85                  90                  95

Thr Thr Thr Pro Ser Thr Thr Thr Thr Leu Pro Ala Ile Ser Val Ser
            100                 105                 110

Gly Arg Val Cys Ser Gly Ser Arg Thr Lys Phe Lys Phe Phe Gly Val
        115                 120                 125

Asn Glu Ser Gly Ala Glu Phe Gly Asn Thr Ala Trp Pro Gly Gln Leu
    130                 135                 140

Gly Lys Asp Tyr Thr Trp Pro Ser Pro Ser Ser Val Asp Tyr Phe Met
145                 150                 155                 160

Gly Ala Gly Phe Asn Thr Phe Arg Ile Thr Phe Leu Met Glu Arg Met
                165                 170                 175

Ser Pro Pro Ala Thr Gly Leu Thr Gly Pro Phe Asn Gln Thr Tyr Leu
            180                 185                 190

Ser Gly Leu Thr Thr Ile Val Asp Tyr Ile Thr Asn Lys Gly Gly Tyr
        195                 200                 205

Ala Leu Ile Asp Pro His Asn Phe Met Arg Tyr Asn Asn Gly Ile Ile
    210                 215                 220
```

```
Ser Ser Thr Ser Asp Phe Ala Thr Trp Trp Ser Asn Leu Ala Thr Val
225                 230                 235                 240

Phe Lys Ser Thr Lys Asn Ala Ile Phe Asp Ile Gln Asn Glu Pro Tyr
                245                 250                 255

Gly Ile Asp Ala Gln Thr Val Tyr Glu Leu Asn Gln Ala Ala Ile Asn
            260                 265                 270

Ser Ile Arg Ala Ala Gly Ala Thr Ser Gln Leu Ile Leu Val Glu Gly
        275                 280                 285

Thr Ser Tyr Thr Gly Ala Trp Thr Trp Val Ser Ser Gly Asn Gly Ala
    290                 295                 300

Ala Phe Ala Ala Val Thr Asp Pro Tyr Asn Asn Thr Ala Ile Glu Met
305                 310                 315                 320

His Gln Tyr Leu Asp Ser Asp Gly Ser Gly Thr Asn Glu Asp Cys Val
                325                 330                 335

Ser Ser Thr Ile Gly Ser Gln Arg Leu Gln Ala Ala Thr Ala Trp Leu
            340                 345                 350

Gln Gln Thr Gly Leu Lys Gly Phe Leu Gly Glu Thr Gly Ala Gly Ser
        355                 360                 365

Asn Ser Gln Cys Ile Asp Ala Val Phe Asp Glu Leu Cys Tyr Met Gln
    370                 375                 380

Gln Gln Gly Gly Ser Trp Ile Gly Ala Leu Trp Trp Ala Ala Gly Pro
385                 390                 395                 400

Trp Trp Gly Thr Tyr Ile Tyr Ser Ile Glu Pro Pro Ser Gly Ala Ala
                405                 410                 415

Ile Pro Glu Val Leu Pro Gln Gly Leu Ala Pro Phe Leu
            420                 425
```

<210> 81
<211> 1580
<212> DNA
<213> Thielavia terrestris

<400> 81

agcccccgt tcaggcacac ttggcatcag atcagcttag cagcgcctgc acagcatgaa 60
gctctcgcag tcggccgcgc tggcggcact caccgcgacg gcgctcgccg ccccctcgcc 120
cacgacgccg caggcgccga ggcaggcttc agccggctgc tcgtctgcgg tcacgctcga 180
cgccagcacc aacgtttgga agaagtacac gctgcacccc aacagctact accgcaagga 240
ggttgaggcc gcggtggcgc agatctcgga cccggacctc gccgccaagg ccaagaaggt 300
ggccgacgtc ggcaccttcc tgtggctcga ctcgatcgag aacatcggca agctggagcc 360
ggcgatccag gacgtgccct gcgagaacat cctgggcctg gtcatctacg acctgccggg 420
ccgcgactgc gcggccaagg cgtccaacgg cgagctcaag gtcggcgaga tcgaccgcta 480
caagaccgag tacatcgaca gtgagtgctg ccccccgggt tcgagaagag cgtgggggaa 540
agggaaaggg ttgactgact gacacggcgc actgcagaga tcgtgtcgat cctcaaggca 600
caccccaaca cggcgttcgc gctggtcatc gagccggact cgctgcccaa cctggtgacc 660
aacagcaact tggacacgtg ctcgagcagc gcgtcgggct accgcgaagg cgtggcttac 720
gccctcaaga acctcaacct gcccaacgtg atcatgtacc tcgacgccgg ccacggcggc 780
tggctcggct gggacgccaa cctgcagccc ggcgcgcagg agctagccaa ggcgtacaag 840
aacgccggct cgcccaagca gctccgcggc ttctcgacca acgtggccgg ctggaactcc 900
tggtgagctt ttttccattc catttcttct tcctcttctc tcttcgctcc cactctgcag 960
cccccctcc cccaagcacc cactggcgtt ccggcttgct gactcggcct ccctttcccc 1020
gggcaccagg gatcaatcgc ccggcgaatt ctcccaggcg tccgacgcca agtacaacaa 1080
gtgccagaac gagaagatct acgtcagcac cttcggctcc gcgctccagt cggccggcat 1140
gcccaaccac gccatcgtcg acacgggccg caacggcgtc accggcctgc gcaaggagtg 1200
ggtgactgg tgcaacgtca acggtgcagg ttcgttgtct tctttttctc ctcttttgtt 1260
tgcacgtcgt ggtccttttc aagcagccgt gtttggttgg gggagatgga ctccggctga 1320
tgttctgctt cctctctagg cttcggcgtg cgcccgacga gcaacacggg cctcgagctg 1380
gccgacgcgt tcgtgtgggt caagcccggc ggcgagtcgg acggcaccag cgacagctcg 1440
tcgccgcgct acgacagctt ctgcggcaag gacgacgcct tcaagccctc gcccgaggcc 1500
ggcacctgga acgaggccta cttcgagatg ctgctcaaga acgccgtgcc gtcgttctaa 1560
gacggtccag catcatccgg 1580

<210> 82
<211> 396
<212> PRT
<213> Thielavia terrestris

<400> 82

```
Met Lys Leu Ser Gln Ser Ala Ala Leu Ala Ala Leu Thr Ala Thr Ala
1               5                   10                  15

Leu Ala Ala Pro Ser Pro Thr Thr Pro Gln Ala Pro Arg Gln Ala Ser
            20                  25                  30

Ala Gly Cys Ser Ser Ala Val Thr Leu Asp Ala Ser Thr Asn Val Trp
        35                  40                  45
```

```
Lys Lys Tyr Thr Leu His Pro Asn Ser Tyr Tyr Arg Lys Glu Val Glu
    50                  55                  60

Ala Ala Val Ala Gln Ile Ser Asp Pro Asp Leu Ala Ala Lys Ala Lys
65                  70                  75                  80

Lys Val Ala Asp Val Gly Thr Phe Leu Trp Leu Asp Ser Ile Glu Asn
                85                  90                  95

Ile Gly Lys Leu Glu Pro Ala Ile Gln Asp Val Pro Cys Glu Asn Ile
            100                 105                 110

Leu Gly Leu Val Ile Tyr Asp Leu Pro Gly Arg Asp Cys Ala Ala Lys
        115                 120                 125

Ala Ser Asn Gly Glu Leu Lys Val Gly Glu Ile Asp Arg Tyr Lys Thr
    130                 135                 140

Glu Tyr Ile Asp Lys Ile Val Ser Ile Leu Lys Ala His Pro Asn Thr
145                 150                 155                 160

Ala Phe Ala Leu Val Ile Glu Pro Asp Ser Leu Pro Asn Leu Val Thr
                165                 170                 175

Asn Ser Asn Leu Asp Thr Cys Ser Ser Ser Ala Ser Gly Tyr Arg Glu
            180                 185                 190

Gly Val Ala Tyr Ala Leu Lys Asn Leu Asn Leu Pro Asn Val Ile Met
        195                 200                 205

Tyr Leu Asp Ala Gly His Gly Gly Trp Leu Gly Trp Asp Ala Asn Leu
    210                 215                 220

Gln Pro Gly Ala Gln Glu Leu Ala Lys Ala Tyr Lys Asn Ala Gly Ser
225                 230                 235                 240

Pro Lys Gln Leu Arg Gly Phe Ser Thr Asn Val Ala Gly Trp Asn Ser
                245                 250                 255

Trp Asp Gln Ser Pro Gly Glu Phe Ser Gln Ala Ser Asp Ala Lys Tyr
            260                 265                 270

Asn Lys Cys Gln Asn Glu Lys Ile Tyr Val Ser Thr Phe Gly Ser Ala
        275                 280                 285

Leu Gln Ser Ala Gly Met Pro Asn His Ala Ile Val Asp Thr Gly Arg
    290                 295                 300
```

Asn Gly Val Thr Gly Leu Arg Lys Glu Trp Gly Asp Trp Cys Asn Val
305 310 315 320

Asn Gly Ala Gly Phe Gly Val Arg Pro Thr Ser Asn Thr Gly Leu Glu
325 330 335

Leu Ala Asp Ala Phe Val Trp Val Lys Pro Gly Gly Glu Ser Asp Gly
340 345 350

Thr Ser Asp Ser Ser Ser Pro Arg Tyr Asp Ser Phe Cys Gly Lys Asp
355 360 365

Asp Ala Phe Lys Pro Ser Pro Glu Ala Gly Thr Trp Asn Glu Ala Tyr
370 375 380

Phe Glu Met Leu Leu Lys Asn Ala Val Pro Ser Phe
385 390 395

<210> 83
<211> 1203
<212> DNA
<213> Thielavia terrestris

<400> 83

```
atgaagtacc tcaacctcct cgcagctctc ctcgccgtcg ctcctctctc cctcgctgca      60
cccagcatcg aggccagaca gtcgaacgtc aacccataca tcggcaagag cccgctcgtt     120
attaggtcgt acgcccaaaa gcttgaggag accgtcagga ccttccagca acgtggcgac     180
cagctcaacg ctgcgaggac acggacggtg cagaacgttg cgactttcgc ctggatctcg     240
gataccaatg gtattggagc cattcgacct ctcatccaag atgctctcgc ccagcaggct     300
cgcactggac agaaggtcat cgtccaaatc gtcgtctaca acctcccaga tcgcgactgc     360
tctgccaacg cctcgactgg agagttcacc gtaggaaacg acggtctcaa ccgatacaag     420
aactttgtca acaccatcgc ccgcgagctc tcgactgctg acgctgacaa gctccacttt     480
gccctcctcc tcgaacccga cgcacttgcc aacctcgtca ccaacgcgaa tgcccccagg     540
tgccgaatcg ccgctcccgc ttacaaggag ggtatcgcct acaccctcgc caccttgtcc     600
aagcccaacg tcgacgtcta catcgacgcc gccaacggtg gctggctcgg ctggaacgac     660
aacctccgcc ccttcgccga actcttcaag gaagtctacg acctcgcccg ccgcatcaac     720
cccaacgcca aggtccgcgg cgtccccgtc aacgtctcca actacaacca gtaccgcgct     780
gaagtccgcg agcccttcac cgagtggaag gacgcctggg acgagagccg ctacgtcaac     840
gtcctcaccc cgcacctcaa cgccgtcggc ttctccgcgc acttcatcgt tgaccaggga     900
cgcggtggca agggcggtat caggacggag tggggccagt ggtgcaacgt taggaacgct     960
gggttcggta tcaggcctac tgcggatcag ggcgtgctcc agaacccgaa tgtggatgcg    1020

attgtgtggg ttaagccggg tggagagtcg gatggcacga gtgatttgaa ctcgaacagg    1080
tatgatccta cgtgcaggag tccggtggcg catgttcccg ctcctgaggc tggccagtgg    1140
ttcaacgagt atgttgttaa cctcgttttg aacgctaacc cccctcttga gcctacctgg    1200
taa                                                                  1203
```

<210> 84
<211> 400
<212> PRT
<213> Thielavia terrestris

<400> 84

```
Met Lys Tyr Leu Asn Leu Leu Ala Ala Leu Leu Ala Val Ala Pro Leu
1               5                   10                  15

Ser Leu Ala Ala Pro Ser Ile Glu Ala Arg Gln Ser Asn Val Asn Pro
            20                  25                  30

Tyr Ile Gly Lys Ser Pro Leu Val Ile Arg Ser Tyr Ala Gln Lys Leu
        35                  40                  45

Glu Glu Thr Val Arg Thr Phe Gln Gln Arg Gly Asp Gln Leu Asn Ala
    50                  55                  60

Ala Arg Thr Arg Thr Val Gln Asn Val Ala Thr Phe Ala Trp Ile Ser
65                  70                  75                  80

Asp Thr Asn Gly Ile Gly Ala Ile Arg Pro Leu Ile Gln Asp Ala Leu
                85                  90                  95

Ala Gln Gln Ala Arg Thr Gly Gln Lys Val Ile Val Gln Ile Val Val
            100                 105                 110

Tyr Asn Leu Pro Asp Arg Asp Cys Ser Ala Asn Ala Ser Thr Gly Glu
        115                 120                 125

Phe Thr Val Gly Asn Asp Gly Leu Asn Arg Tyr Lys Asn Phe Val Asn
    130                 135                 140

Thr Ile Ala Arg Glu Leu Ser Thr Ala Asp Ala Asp Lys Leu His Phe
145                 150                 155                 160

Ala Leu Leu Leu Glu Pro Asp Ala Leu Ala Asn Leu Val Thr Asn Ala
                165                 170                 175

Asn Ala Pro Arg Cys Arg Ile Ala Ala Pro Ala Tyr Lys Glu Gly Ile
            180                 185                 190

Ala Tyr Thr Leu Ala Thr Leu Ser Lys Pro Asn Val Asp Val Tyr Ile
```

```
            195                 200                 205

Asp Ala Ala Asn Gly Gly Trp Leu Gly Trp Asn Asp Asn Leu Arg Pro
    210                 215                 220

Phe Ala Glu Leu Phe Lys Glu Val Tyr Asp Leu Ala Arg Arg Ile Asn
225                 230                 235                 240

Pro Asn Ala Lys Val Arg Gly Val Pro Val Asn Val Ser Asn Tyr Asn
                245                 250                 255

Gln Tyr Arg Ala Glu Val Arg Glu Pro Phe Thr Glu Trp Lys Asp Ala
            260                 265                 270

Trp Asp Glu Ser Arg Tyr Val Asn Val Leu Thr Pro His Leu Asn Ala
        275                 280                 285

Val Gly Phe Ser Ala His Phe Ile Val Asp Gln Gly Arg Gly Gly Lys
    290                 295                 300

Gly Gly Ile Arg Thr Glu Trp Gly Gln Trp Cys Asn Val Arg Asn Ala
305                 310                 315                 320

Gly Phe Gly Ile Arg Pro Thr Ala Asp Gln Gly Val Leu Gln Asn Pro
                325                 330                 335

Asn Val Asp Ala Ile Val Trp Val Lys Pro Gly Gly Glu Ser Asp Gly
            340                 345                 350

Thr Ser Asp Leu Asn Ser Asn Arg Tyr Asp Pro Thr Cys Arg Ser Pro
        355                 360                 365

Val Ala His Val Pro Ala Pro Glu Ala Gly Gln Trp Phe Asn Glu Tyr
    370                 375                 380

Val Val Asn Leu Val Leu Asn Ala Asn Pro Pro Leu Glu Pro Thr Trp
385                 390                 395                 400
```

<210> 85
<211> 1501
<212> DNA
<213> Thielavia terrestris

<400> 85

```
gccgttgtca agatgggcca gaagacgctg cacggattcg ccgccacggc tttggccgtt      60
ctcccctttg tgaaggctca gcagcccggc aacttcacgc cggaggtgca cccgcaactg     120
ccaacgtgga agtgcacgac cgccggcggc tgcgttcagc aggacacttc ggtggtgctc     180
gactggaact accgttggat ccacaatgcc gacggcaccg cctcgtgcac gacgtccagc     240
ggggtcgacc acacgctgtg tccagatgag gcgacctgcg cgaagaactg cttcgtggaa     300
ggcgtcaact acacgagcag cggtgtcacc acatccggca gttcgctgac gatgaggcag     360
tatttcaagg ggagcaacgg gcagaccaac agcgtttcgc ctcgtctcta cctgctcggc     420
tcggatggaa actacgtaat gctcaagctg ctcggccagg agctgagctt cgatgtcgat     480
ctctccacgc tcccctgcgg cgagaacggc gcgctgtacc tgtccgagat ggacgcgacc     540
ggtggcagga accagtacaa caccggcggt gccaactacg gctcgggcta ctgtgacgcc     600
cagtgtcccg tgcagacgtg gatgaacggc acgctgaaca ccaacgggca gggctactgc     660
tgcaacgaga tggacatcct cgaggccaac tcccgcgcca acgcgatgac acctcacccc     720
tgcgccaacg gcagctgcga caagagcggg tgcggactca acccctacgc cgagggctac     780
aagagctact acggaccggg cctcacggtt gacacgtcga agcccttcac catcattacc     840
cgcttcatca ccgacgacgg cacgaccagc ggcaccctca accagatcca gcggatctat     900
gtgcagaatg gcaagacggt cgcgtcggct gcgtccggag gcgacatcat cacggcatcc     960
ggctgcacct cggcccaggc gttcggcggg ctggccaaca tgggcgcggc gcttggacgg    1020
ggcatggtgc tgaccttcag catctggaac gacgctgggg gctacatgaa ctggctcgac    1080
agcggcaaca acggcccgtg cagcagcacc gagggcaacc cgtccaacat cctggccaac    1140
tacccggaca cccacgtggt cttctccaac atccgctggg gagacatcgg ctcgacggtc    1200
caggtctcgg gaggcggcaa cggcggctcg accaccacca cgtcgaccac cacgctgagg    1260
acctcgacca cgaccaccac caccgccccg acggccactg ccacgcactg ggacaatgc     1320
ggcggaatcg ggtacgtca accgcctcct gcattctgtt gaggaagtta actaacgtgg     1380
cctacgcagt ggactggacc gaccgtctgc gaatcgccgt acgcatgcaa ggagctgaac    1440
ccctggtact accagtgcct ctaaagtatt gcagtgaagc catactccgt gctcggcatg    1500
g                                                                    1501
```

<210> 86
<211> 464
<212> PRT
<213> Thielavia terrestris

<400> 86

```
Met Gly Gln Lys Thr Leu His Gly Phe Ala Ala Thr Ala Leu Ala Val
1               5                   10                  15

Leu Pro Phe Val Lys Ala Gln Gln Pro Gly Asn Phe Thr Pro Glu Val
            20                  25                  30

His Pro Gln Leu Pro Thr Trp Lys Cys Thr Thr Ala Gly Gly Cys Val
        35                  40                  45

Gln Gln Asp Thr Ser Val Val Leu Asp Trp Asn Tyr Arg Trp Ile His
```

50 55 60

Asn Ala Asp Gly Thr Ala Ser Cys Thr Thr Ser Ser Gly Val Asp His
65 70 75 80

Thr Leu Cys Pro Asp Glu Ala Thr Cys Ala Lys Asn Cys Phe Val Glu
85 90 95

Gly Val Asn Tyr Thr Ser Ser Gly Val Thr Thr Ser Gly Ser Ser Leu
100 105 110

Thr Met Arg Gln Tyr Phe Lys Gly Ser Asn Gly Gln Thr Asn Ser Val
115 120 125

Ser Pro Arg Leu Tyr Leu Leu Gly Ser Asp Gly Asn Tyr Val Met Leu
130 135 140

Lys Leu Leu Gly Gln Glu Leu Ser Phe Asp Val Asp Leu Ser Thr Leu
145 150 155 160

Pro Cys Gly Glu Asn Gly Ala Leu Tyr Leu Ser Glu Met Asp Ala Thr
165 170 175

Gly Gly Arg Asn Gln Tyr Asn Thr Gly Gly Ala Asn Tyr Gly Ser Gly
180 185 190

Tyr Cys Asp Ala Gln Cys Pro Val Gln Thr Trp Met Asn Gly Thr Leu
195 200 205

Asn Thr Asn Gly Gln Gly Tyr Cys Cys Asn Glu Met Asp Ile Leu Glu
210 215 220

Ala Asn Ser Arg Ala Asn Ala Met Thr Pro His Pro Cys Ala Asn Gly
225 230 235 240

Ser Cys Asp Lys Ser Gly Cys Gly Leu Asn Pro Tyr Ala Glu Gly Tyr
245 250 255

Lys Ser Tyr Tyr Gly Pro Gly Leu Thr Val Asp Thr Ser Lys Pro Phe
260 265 270

Thr Ile Ile Thr Arg Phe Ile Thr Asp Asp Gly Thr Thr Ser Gly Thr
275 280 285

Leu Asn Gln Ile Gln Arg Ile Tyr Val Gln Asn Gly Lys Thr Val Ala
290 295 300

Ser Ala Ala Ser Gly Gly Asp Ile Ile Thr Ala Ser Gly Cys Thr Ser
305 310 315 320

Ala Gln Ala Phe Gly Gly Leu Ala Asn Met Gly Ala Ala Leu Gly Arg
325 330 335

Gly Met Val Leu Thr Phe Ser Ile Trp Asn Asp Ala Gly Gly Tyr Met
340 345 350

Asn Trp Leu Asp Ser Gly Asn Asn Gly Pro Cys Ser Ser Thr Glu Gly
355 360 365

Asn Pro Ser Asn Ile Leu Ala Asn Tyr Pro Asp Thr His Val Val Phe
370 375 380

Ser Asn Ile Arg Trp Gly Asp Ile Gly Ser Thr Val Gln Val Ser Gly
385 390 395 400

Gly Gly Asn Gly Gly Ser Thr Thr Thr Thr Ser Thr Thr Thr Leu Arg
405 410 415

Thr Ser Thr Thr Thr Thr Thr Thr Ala Pro Thr Ala Thr Ala Thr His
420 425 430

Trp Gly Gln Cys Gly Gly Ile Gly Trp Thr Gly Pro Thr Val Cys Glu
435 440 445

Ser Pro Tyr Ala Cys Lys Glu Leu Asn Pro Trp Tyr Tyr Gln Cys Leu
450 455 460

<210> 87
<211> 1368
<212> DNA
<213> Thielavia terrestris

<400> 87

accgatccgc tcgaagatgg cgcccaagtc tacagttctg gccgcctggc tgctctcctc 60
gctggccgcg gcccagcaga tcggcaaagc cgtgcccgag gtccacccca aactgacaac 120
gcagaagtgc actctccgcg gcgggtgcaa gcctgtccgc acctcggtcg tgctcgactc 180
gtccgcgcgc tcgctgcaca aggtcgggga ccccaacacc agctgcagcg tcggcggcga 240
cctgtgctcg gacgcgaagt cgtgcggcaa gaactgcgcg ctcgagggcg tcgactacgc 300
ggcccacggc gtggcgacca agggcgacgc cctcacgctg caccagtggc tcaagggggc 360
cgacggcacc tacaggaccg tctcgccgcg cgtatacctc ctgggcgagg acgggaagaa 420
ctacgaggac ttcaagctgc tcaacgccga gctcagcttc gacgtcgacg tgtcccagct 480
cgtctgcggc atgaacggcg ccctgtactt ctccgagatg gagatggacg gcggccgcag 540
cccgctgaac ccggcgggcg ccacgtacgg cacgggctac tgcgacgcgc agtgccccaa 600
gttggacttt atcaacggcg aggtatttct tctctcttct gtttttcttt tccatcgctt 660
tttctgaccg gaatccgccc tcttagctca acaccaacca cacgtacggg gcgtgctgca 720
acgagatgga catctgggag gccaacgcgc tggcgcaggc gctcacgccg cacccgtgca 780
acgcgacgcg ggtgtacaag tgcgacacgg cggacgagtg cgggcagccg gtgggcgtgt 840
gcgacgaatg ggggtgctcg tacaacccgt ccaacttcgg ggtcaaggac tactacgggc 900
gcaacctgac ggtggacacg aaccgcaagt tcacggtgac gacgcagttc gtgacgtcca 960
acgggcgggc ggacggcgag ctgaccgaga tccggcggct gtacgtgcag gacggcgtgg 1020
tgatccagaa ccacgcggtc acggcgggcg gggcgacgta cgacagcatc acggacggct 1080
tctgcaacgc gacggccacc tggacgcagc agcggggcgg gctcgcgcgc atgggcgagg 1140
ccatcggccg cggcatggtg ctcatcttca gcctgtgggt tgacaacggc ggcttcatga 1200
actggctcga cagcggcaac gccgggccct gcaacgccac cgagggcgac ccggccctga 1260
tcctgcagca gcacccggac gccagcgtca ccttctccaa catccgatgg ggcgagatcg 1320
gcagcacgta caagagcgag tgcagccact agagtagagc ttgtaatt 1368

<210> 88
<211> 423
<212> PRT
<213> Thielavia terrestris

<400> 88

```
Met Ala Pro Lys Ser Thr Val Leu Ala Ala Trp Leu Leu Ser Ser Leu
1               5                   10                  15

Ala Ala Ala Gln Gln Ile Gly Lys Ala Val Pro Glu Val His Pro Lys
            20                  25                  30

Leu Thr Thr Gln Lys Cys Thr Leu Arg Gly Gly Cys Lys Pro Val Arg
        35                  40                  45

Thr Ser Val Val Leu Asp Ser Ser Ala Arg Ser Leu His Lys Val Gly
    50                  55                  60

Asp Pro Asn Thr Ser Cys Ser Val Gly Gly Asp Leu Cys Ser Asp Ala
65                  70                  75                  80

Lys Ser Cys Gly Lys Asn Cys Ala Leu Glu Gly Val Asp Tyr Ala Ala
                85                  90                  95

His Gly Val Ala Thr Lys Gly Asp Ala Leu Thr Leu His Gln Trp Leu
            100                 105                 110

Lys Gly Ala Asp Gly Thr Tyr Arg Thr Val Ser Pro Arg Val Tyr Leu
        115                 120                 125
```

Leu Gly Glu Asp Gly Lys Asn Tyr Glu Asp Phe Lys Leu Leu Asn Ala
    130                 135                 140

Glu Leu Ser Phe Asp Val Asp Val Ser Gln Leu Val Cys Gly Met Asn
145                 150                 155                 160

Gly Ala Leu Tyr Phe Ser Glu Met Glu Met Asp Gly Gly Arg Ser Pro
                165                 170                 175

Leu Asn Pro Ala Gly Ala Thr Tyr Gly Thr Gly Tyr Cys Asp Ala Gln
            180                 185                 190

Cys Pro Lys Leu Asp Phe Ile Asn Gly Glu Leu Asn Thr Asn His Thr
        195                 200                 205

Tyr Gly Ala Cys Cys Asn Glu Met Asp Ile Trp Glu Ala Asn Ala Leu
    210                 215                 220

Ala Gln Ala Leu Thr Pro His Pro Cys Asn Ala Thr Arg Val Tyr Lys
225                 230                 235                 240

Cys Asp Thr Ala Asp Glu Cys Gly Gln Pro Val Gly Val Cys Asp Glu
                245                 250                 255

Trp Gly Cys Ser Tyr Asn Pro Ser Asn Phe Gly Val Lys Asp Tyr Tyr
            260                 265                 270

Gly Arg Asn Leu Thr Val Asp Thr Asn Arg Lys Phe Thr Val Thr Thr
        275                 280                 285

Gln Phe Val Thr Ser Asn Gly Arg Ala Asp Gly Glu Leu Thr Glu Ile
    290                 295                 300

Arg Arg Leu Tyr Val Gln Asp Gly Val Val Ile Gln Asn His Ala Val
305                 310                 315                 320

Thr Ala Gly Gly Ala Thr Tyr Asp Ser Ile Thr Asp Gly Phe Cys Asn
                325                 330                 335

Ala Thr Ala Thr Trp Thr Gln Gln Arg Gly Gly Leu Ala Arg Met Gly
            340                 345                 350

Glu Ala Ile Gly Arg Gly Met Val Leu Ile Phe Ser Leu Trp Val Asp
        355                 360                 365

Asn Gly Gly Phe Met Asn Trp Leu Asp Ser Gly Asn Ala Gly Pro Cys
    370                 375                 380

Asn Ala Thr Glu Gly Asp Pro Ala Leu Ile Leu Gln Gln His Pro Asp

385 390 395 400

Ala Ser Val Thr Phe Ser Asn Ile Arg Trp Gly Glu Ile Gly Ser Thr
405 410 415

Tyr Lys Ser Glu Cys Ser His
420

<210> 89
<211> 1011
<212> DNA
<213> Thielavia terrestris

<400> 89

atgaccctac ggctccctgt catcagcctg ctggcctcgc tggcagcagg cgccgtcgtc 60

gtcccacggg cggagtttca cccccctctc ccgacttgga aatgcacgac ctccgggggc 120

tgcgtgcagc agaacaccag cgtcgtcctg gaccgtgact cgaagtacgc cgcacacagc 180

gccggctcgc ggacggaatc ggattacgcg gcaatgggag tgtccacttc gggcaatgcc 240

gtgacgctgt accactacgt caagaccaac ggcaccctcg tccccgcttc gccgcgcatc 300

tacctcctgg gcgcggacgg caagtacgtg cttatggacc tcctcaacca ggagctgtcg 360

gtggacgtcg acttctcggc gctgccgtgc ggcgagaacg gggccttcta cctgtccgag 420

atggcggcgg acgggcgggg cgacgcgggg gcgggcgacg ggtactgcga cgcgcagtgc 480

cagggctact gctgcaacga gatggacatc ctcgaggcca actcgatggc gacggccatg 540

acgccgcacc cgtgcaaggg caacaactgc gaccgcagcg gctgcggcta caacccgtac 600

gccagcggcc agcgcggctt ctacgggccc ggcaagacgg tcgacacgag caagcccttc 660

accgtcgtca cgcagttcgc cgccagcggc ggcaagctga cccagatcac ccgcaagtac 720

atccagaacg gccgggagat cggcggcggc ggcaccatct ccagctgcgg ctccgagtct 780

tcgacgggcg gcctgaccgg catgggcgag gcgctggggc gcggaatggt gctggccatg 840

agcatctgga acgacgcggc ccaggagatg gcatggctcg atgccggcaa caacggccct 900

tgcgccagtg gccagggcag cccgtccgtc attcagtcgc agcatcccga cacccacgtc 960

gtcttctcca acatcaggtg gggcgacatc gggtctacca cgaagaacta g 1011

<210> 90
<211> 336
<212> PRT
<213> Thielavia terrestris

<400> 90

```
Met Thr Leu Arg Leu Pro Val Ile Ser Leu Leu Ala Ser Leu Ala Ala
1               5                   10                  15

Gly Ala Val Val Val Pro Arg Ala Glu Phe His Pro Pro Leu Pro Thr
            20                  25                  30
```

```
Trp Lys Cys Thr Thr Ser Gly Gly Cys Val Gln Gln Asn Thr Ser Val
        35                  40                  45

Val Leu Asp Arg Asp Ser Lys Tyr Ala Ala His Ser Ala Gly Ser Arg
    50                  55                  60

Thr Glu Ser Asp Tyr Ala Ala Met Gly Val Ser Thr Ser Gly Asn Ala
65                  70                  75                  80

Val Thr Leu Tyr His Tyr Val Lys Thr Asn Gly Thr Leu Val Pro Ala
                85                  90                  95

Ser Pro Arg Ile Tyr Leu Leu Gly Ala Asp Gly Lys Tyr Val Leu Met
            100                 105                 110

Asp Leu Leu Asn Gln Glu Leu Ser Val Asp Val Asp Phe Ser Ala Leu
        115                 120                 125

Pro Cys Gly Glu Asn Gly Ala Phe Tyr Leu Ser Glu Met Ala Ala Asp
    130                 135                 140

Gly Arg Gly Asp Ala Gly Ala Gly Asp Gly Tyr Cys Asp Ala Gln Cys
145                 150                 155                 160

Gln Gly Tyr Cys Cys Asn Glu Met Asp Ile Leu Glu Ala Asn Ser Met
                165                 170                 175

Ala Thr Ala Met Thr Pro His Pro Cys Lys Gly Asn Asn Cys Asp Arg
            180                 185                 190

Ser Gly Cys Gly Tyr Asn Pro Tyr Ala Ser Gly Gln Arg Gly Phe Tyr
        195                 200                 205

Gly Pro Gly Lys Thr Val Asp Thr Ser Lys Pro Phe Thr Val Val Thr
    210                 215                 220

Gln Phe Ala Ala Ser Gly Gly Lys Leu Thr Gln Ile Thr Arg Lys Tyr
225                 230                 235                 240

Ile Gln Asn Gly Arg Glu Ile Gly Gly Gly Gly Thr Ile Ser Ser Cys
                245                 250                 255

Gly Ser Glu Ser Ser Thr Gly Gly Leu Thr Gly Met Gly Glu Ala Leu
            260                 265                 270

Gly Arg Gly Met Val Leu Ala Met Ser Ile Trp Asn Asp Ala Ala Gln
        275                 280                 285
```

Glu Met Ala Trp Leu Asp Ala Gly Asn Asn Gly Pro Cys Ala Ser Gly
290 295 300

Gln Gly Ser Pro Ser Val Ile Gln Ser Gln His Pro Asp Thr His Val
305 310 315 320

Val Phe Ser Asn Ile Arg Trp Gly Asp Ile Gly Ser Thr Thr Lys Asn
325 330 335

<210> 91
<211> 1480
<212> DNA
<213> Cladorrhinum foecundissimum

<400> 91

```
gatccgaatt cctcctctcg ttctttagtc acagaccaga catctgccca cgatggttca        60
caagttcgcc ctcctcaccg gcctcgccgc ctccctcgca tctgcccagc agatcggcac       120
cgtcgtcccc gagtctcacc ccaagcttcc caccaagcgc tgcactctcg ccggtggctg       180
ccagaccgtc gacacctcca tcgtcatcga cgccttccag cgtcccctcc acaagatcgg       240
cgacccttcc actccttgcg tcgtcggcgg ccctctctgc cccgacgcca agtcctgcgc       300
tgagaactgc gcgctcgagg gtgtcgacta tgcctcctgg ggcatcaaga ccgagggcga       360
cgccctaact ctcaaccagt ggatgcccga cccggcgaac cctggccagt acaagacgac       420
tactccccgt acttaccttg ttgctgagga cggcaagaac tacgaggatg tgaagctcct       480
ggctaaggag atctcgtttg atgccgatgt cagcaacctt ccctgcggca tgaacggtgc       540
tttctacttg tctgagatgt tgatggatgg tggacgtggc gacctcaacc ctgctggtgc       600
cgagtatggt accggttact gtgatgcgca gtgcttcaag ttggatttca tcaacggcga       660
ggccaacatc gaccaaaagc acggcgcctg ctgcaacgaa atggacattt tcgaatccaa       720
ctcgcgcgcc aagaccttcg tcccccaccc ctgcaacatc acgcaggtct acaagtgcga       780
aggcgaagac gagtgcggcc agcccgtcgg cgtgtgcgac aagtgggggt gcggcttcaa       840
cgagtacaaa tggggcgtcg agtccttcta cggccggggc tcgcagttcg ccatcgactc       900
ctccaagaag ttcaccgtca ccacgcagtt cctgaccgac aacggcaagg aggacggcgt       960
cctcgtcgag atccgccgct tgtggcacca ggatggcaag ctgatcaaga acaccgctat      1020
ccaggttgag gagaactaca gcacggactc ggtgagcacc gagttctgcg agaagactgc      1080
ttctttcacc atgcagcgcg gtggtctcaa ggcgatgggc gaggctatcg gtcgtggtat      1140
ggtgctggtt ttcagcatct gggcggatga ttcgggtttt atgaactggt tggatgcgga      1200
gggtaatggc ccttgcagcg cgactgaggg cgatccgaag gagattgtca agaataagcc      1260
ggatgctagg gttacgttct caaacattag gattggtgag gttggtagca cgtatgctcc      1320
gggtgggaag tgcggtgtta agagcagggt tgctaggggg cttactgctt cttaaggggg      1380

gtgtgaagag aggaggaggt gttgttgggg gttggagatg ataattgggc gagatggtgt      1440
agagcgggtt ggttggatat gaatacgttg aattggatgt                            1480
```

<210> 92
<211> 440
<212> PRT
<213> Cladorrhinum foecundissimum

<400> 92

```
Met Val His Lys Phe Ala Leu Leu Thr Gly Leu Ala Ala Ser Leu Ala
1               5                   10                  15

Ser Ala Gln Gln Ile Gly Thr Val Val Pro Glu Ser His Pro Lys Leu
            20                  25                  30

Pro Thr Lys Arg Cys Thr Leu Ala Gly Gly Cys Gln Thr Val Asp Thr
        35                  40                  45

Ser Ile Val Ile Asp Ala Phe Gln Arg Pro Leu His Lys Ile Gly Asp
    50                  55                  60

Pro Ser Thr Pro Cys Val Val Gly Gly Pro Leu Cys Pro Asp Ala Lys
65                  70                  75                  80

Ser Cys Ala Glu Asn Cys Ala Leu Glu Gly Val Asp Tyr Ala Ser Trp
                85                  90                  95

Gly Ile Lys Thr Glu Gly Asp Ala Leu Thr Leu Asn Gln Trp Met Pro
            100                 105                 110

Asp Pro Ala Asn Pro Gly Gln Tyr Lys Thr Thr Thr Pro Arg Thr Tyr
        115                 120                 125

Leu Val Ala Glu Asp Gly Lys Asn Tyr Glu Asp Val Lys Leu Leu Ala
    130                 135                 140

Lys Glu Ile Ser Phe Asp Ala Asp Val Ser Asn Leu Pro Cys Gly Met
145                 150                 155                 160

Asn Gly Ala Phe Tyr Leu Ser Glu Met Leu Met Asp Gly Gly Arg Gly
                165                 170                 175

Asp Leu Asn Pro Ala Gly Ala Glu Tyr Gly Thr Gly Tyr Cys Asp Ala
            180                 185                 190

Gln Cys Phe Lys Leu Asp Phe Ile Asn Gly Glu Ala Asn Ile Asp Gln
        195                 200                 205

Lys His Gly Ala Cys Cys Asn Glu Met Asp Ile Phe Glu Ser Asn Ser
```

```
    210                 215                 220

Arg Ala Lys Thr Phe Val Pro His Pro Cys Asn Ile Thr Gln Val Tyr
225                 230                 235                 240

Lys Cys Glu Gly Glu Asp Glu Cys Gly Gln Pro Val Gly Val Cys Asp
                245                 250                 255

Lys Trp Gly Cys Gly Phe Asn Glu Tyr Lys Trp Gly Val Glu Ser Phe
            260                 265                 270

Tyr Gly Arg Gly Ser Gln Phe Ala Ile Asp Ser Ser Lys Lys Phe Thr
        275                 280                 285

Val Thr Thr Gln Phe Leu Thr Asp Asn Gly Lys Glu Asp Gly Val Leu
    290                 295                 300

Val Glu Ile Arg Arg Leu Trp His Gln Asp Gly Lys Leu Ile Lys Asn
305                 310                 315                 320

Thr Ala Ile Gln Val Glu Glu Asn Tyr Ser Thr Asp Ser Val Ser Thr
                325                 330                 335

Glu Phe Cys Glu Lys Thr Ala Ser Phe Thr Met Gln Arg Gly Gly Leu
            340                 345                 350

Lys Ala Met Gly Glu Ala Ile Gly Arg Gly Met Val Leu Val Phe Ser
        355                 360                 365

Ile Trp Ala Asp Asp Ser Gly Phe Met Asn Trp Leu Asp Ala Glu Gly
    370                 375                 380

Asn Gly Pro Cys Ser Ala Thr Glu Gly Asp Pro Lys Glu Ile Val Lys
385                 390                 395                 400

Asn Lys Pro Asp Ala Arg Val Thr Phe Ser Asn Ile Arg Ile Gly Glu
                405                 410                 415

Val Gly Ser Thr Tyr Ala Pro Gly Gly Lys Cys Gly Val Lys Ser Arg
            420                 425                 430

Val Ala Arg Gly Leu Thr Ala Ser
        435                 440
```

<210> 93
<211> 1380
<212> DNA

<213> Trichoderma reesei

<400> 93

```
atggcgccct cagttacact gccgttgacc acggccatcc tggccattgc ccggctcgtc      60
gccgcccagc aaccgggtac cagcaccccc gaggtccatc ccaagttgac aacctacaag     120
tgtacaaagt ccgggggtg cgtggcccag gacacctcgg tggtccttga ctggaactac     180
cgctggatgc acgacgcaaa ctacaactcg tgcaccgtca acggcggcgt caacaccacg     240
ctctgccctg acgaggcgac ctgtggcaag aactgcttca tcgagggcgt cgactacgcc     300
gcctcgggcg tcacgacctc gggcagcagc ctcaccatga accagtacat gcccagcagc     360
tctggcggct acagcagcgt ctctcctcgg ctgtatctcc tggactctga cggtgagtac     420
gtgatgctga agctcaacgg ccaggagctg agcttcgacg tcgacctctc tgctctgccg     480
tgtggagaga acggctcgct ctacctgtct cagatggacg agaacggggg cgccaaccag     540
tataacacgg ccggtgccaa ctacgggagc ggctactgcg atgctcagtg ccccgtccag     600
acatggagga acggcaccct caacactagc caccagggct tctgctgcaa cgagatggat     660
atcctggagg gcaactcgag ggcgaatgcc ttgacccctc actcttgcac ggccacggcc     720
tgcgactctg ccggttgcgg cttcaacccc tatggcagcg gctacaaaag ctactacggc     780
cccggagata ccgttgacac ctccaagacc ttcaccatca tcacccagtt caacacggac     840
aacggctcgc cctcgggcaa ccttgtgagc atcacccgca agtaccagca aaacggcgtc     900
gacatcccca gcgcccagcc cggcggcgac accatctcgt cctgcccgtc cgcctcagcc     960
tacggcggcc tcgccaccat gggcaaggcc ctgagcagcg gcatggtgct cgtgttcagc    1020
atttggaacg acaacagcca gtacatgaac tggctcgaca gcggcaacgc cggcccctgc    1080
agcagcaccg agggcaaccc atccaacatc ctggccaaca accccaacac gcacgtcgtc    1140
ttctccaaca tccgctgggg agacattggg tctactacga actcgactgc gcccccgccc    1200
ccgcctgcgt ccagcacgac gttttcgact acacggagga gctcgacgac ttcgagcagc    1260
ccgagctgca cgcagactca ctgggggcag tgcggtggca ttgggtacag cgggtgcaag    1320
acgtgcacgt cgggcactac gtgccagtat agcaacgact actactcgca atgcctttag    1380
```

<210> 94
<211> 459
<212> PRT
<213> Trichoderma reesei

<400> 94

```
Met Ala Pro Ser Val Thr Leu Pro Leu Thr Thr Ala Ile Leu Ala Ile
1               5                   10                  15

Ala Arg Leu Val Ala Ala Gln Gln Pro Gly Thr Ser Thr Pro Glu Val
            20                  25                  30

His Pro Lys Leu Thr Thr Tyr Lys Cys Thr Lys Ser Gly Gly Cys Val
        35                  40                  45
```

Ala Gln Asp Thr Ser Val Val Leu Asp Trp Asn Tyr Arg Trp Met His
    50                  55                  60

Asp Ala Asn Tyr Asn Ser Cys Thr Val Asn Gly Gly Val Asn Thr Thr
65                  70                  75                  80

Leu Cys Pro Asp Glu Ala Thr Cys Gly Lys Asn Cys Phe Ile Glu Gly
                85                  90                  95

Val Asp Tyr Ala Ala Ser Gly Val Thr Thr Ser Gly Ser Ser Leu Thr
            100                 105                 110

Met Asn Gln Tyr Met Pro Ser Ser Ser Gly Gly Tyr Ser Ser Val Ser
        115                 120                 125

Pro Arg Leu Tyr Leu Leu Asp Ser Asp Gly Glu Tyr Val Met Leu Lys
    130                 135                 140

Leu Asn Gly Gln Glu Leu Ser Phe Asp Val Asp Leu Ser Ala Leu Pro
145                 150                 155                 160

Cys Gly Glu Asn Gly Ser Leu Tyr Leu Ser Gln Met Asp Glu Asn Gly
                165                 170                 175

Gly Ala Asn Gln Tyr Asn Thr Ala Gly Ala Asn Tyr Gly Ser Gly Tyr
            180                 185                 190

Cys Asp Ala Gln Cys Pro Val Gln Thr Trp Arg Asn Gly Thr Leu Asn
        195                 200                 205

Thr Ser His Gln Gly Phe Cys Cys Asn Glu Met Asp Ile Leu Glu Gly
    210                 215                 220

Asn Ser Arg Ala Asn Ala Leu Thr Pro His Ser Cys Thr Ala Thr Ala
225                 230                 235                 240

Cys Asp Ser Ala Gly Cys Gly Phe Asn Pro Tyr Gly Ser Gly Tyr Lys
                245                 250                 255

Ser Tyr Tyr Gly Pro Gly Asp Thr Val Asp Thr Ser Lys Thr Phe Thr
            260                 265                 270

Ile Ile Thr Gln Phe Asn Thr Asp Asn Gly Ser Pro Ser Gly Asn Leu
        275                 280                 285

Val Ser Ile Thr Arg Lys Tyr Gln Gln Asn Gly Val Asp Ile Pro Ser
    290                 295                 300

```
Ala Gln Pro Gly Gly Asp Thr Ile Ser Ser Cys Pro Ser Ala Ser Ala
305                 310                 315                 320

Tyr Gly Gly Leu Ala Thr Met Gly Lys Ala Leu Ser Ser Gly Met Val
                325                 330                 335

Leu Val Phe Ser Ile Trp Asn Asp Asn Ser Gln Tyr Met Asn Trp Leu
            340                 345                 350

Asp Ser Gly Asn Ala Gly Pro Cys Ser Ser Thr Glu Gly Asn Pro Ser
        355                 360                 365

Asn Ile Leu Ala Asn Asn Pro Asn Thr His Val Val Phe Ser Asn Ile
    370                 375                 380

Arg Trp Gly Asp Ile Gly Ser Thr Thr Asn Ser Thr Ala Pro Pro Pro
385                 390                 395                 400

Pro Pro Ala Ser Ser Thr Thr Phe Ser Thr Thr Arg Arg Ser Ser Thr
                405                 410                 415

Thr Ser Ser Ser Pro Ser Cys Thr Gln Thr His Trp Gly Gln Cys Gly
            420                 425                 430

Gly Ile Gly Tyr Ser Gly Cys Lys Thr Cys Thr Ser Gly Thr Thr Cys
        435                 440                 445

Gln Tyr Ser Asn Asp Tyr Tyr Ser Gln Cys Leu
    450                 455
```

<210> 95
<211> 1545
<212> DNA
<213> Trichoderma reesei

<400> 95

atgtatcgga agttggccgt catctcggcc ttcttggcca cagctcgtgc tcagtcggcc 60
tgcactctcc aatcggagac tcacccgcct ctgacatggc agaaatgctc gtctggtggc 120
acgtgcactc aacagacagg ctccgtggtc atcgacgcca actggcgctg gactcacgct 180
acgaacagca gcacgaactg ctacgatggc aacacttgga gctcgaccct atgtcctgac 240
aacgagacct gcgcgaagaa ctgctgtctg gacggtgccg cctacgcgtc cacgtacgga 300
gttaccacga gcggtaacag cctctccatt ggctttgtca cccagtctgc gcagaagaac 360
gttggcgctc gcctttacct tatggcgagc gacacgacct accaggaatt caccctgctt 420
ggcaacgagt tctctttcga tgttgatgtt tcgcagctgc cgtgcggctt gaacggagct 480
ctctacttcg tgtccatgga cgcggatggt ggcgtgagca agtatcccac caacaccgct 540
ggcgccaagt acggcacggg gtactgtgac agccagtgtc cccgcgatct gaagttcatc 600
aatggccagg ccaacgttga gggctgggag ccgtcatcca acaacgcgaa cacgggcatt 660
ggaggacacg gaagctgctg ctctgagatg gatatctggg aggccaactc catctccgag 720
gctcttaccc cccacccttg cacgactgtc ggccaggaga tctgcgaggg tgatgggtgc 780
ggcggaactt actccgataa cagatatggc ggcacttgcg atcccgatgg ctgcgactgg 840
aacccatacc gcctgggcaa caccagcttc tacggccctg gctcaagctt taccctcgat 900
accaccaaga aattgaccgt tgtcacccag ttcgagacgt cgggtgccat caaccgatac 960
tatgtccaga atggcgtcac tttccagcag cccaacgccg agcttggtag ttactctggc 1020
aacgagctca acgatgatta ctgcacagct gaggaggcag aattcggcgg atcctctttc 1080
tcagacaagg gcggcctgac tcagttcaag aaggctacct ctggcggcat ggttctggtc 1140
atgagtctgt gggatgatta ctacgccaac atgctgtggc tggactccac ctacccgaca 1200
aacgagacct cctccacacc cggtgccgtg cgcggaagct gctccaccag ctccggtgtc 1260
cctgctcagg tcgaatctca gtctcccaac gccaaggtca ccttctccaa catcaagttc 1320
ggacccattg gcagcaccgg caaccctagc ggcggcaacc ctcccggcgg aaacccgcct 1380
ggcaccacca ccacccgccg cccagccact accactggaa gctctcccgg acctacccag 1440
tctcactacg gccagtgcgg cggtattggc tacagcggcc ccacggtctg cgccagcggc 1500
acaacttgcc aggtcctgaa cccttactac tctcagtgcc tgtaa 1545

<210> 96
<211> 514
<212> PRT
<213> Trichoderma reesei

<400> 96

```
Met Tyr Arg Lys Leu Ala Val Ile Ser Ala Phe Leu Ala Thr Ala Arg
1               5                   10                  15

Ala Gln Ser Ala Cys Thr Leu Gln Ser Glu Thr His Pro Pro Leu Thr
            20                  25                  30

Trp Gln Lys Cys Ser Ser Gly Gly Thr Cys Thr Gln Gln Thr Gly Ser
        35                  40                  45

Val Val Ile Asp Ala Asn Trp Arg Trp Thr His Ala Thr Asn Ser Ser
    50                  55                  60

Thr Asn Cys Tyr Asp Gly Asn Thr Trp Ser Ser Thr Leu Cys Pro Asp
65                  70                  75                  80

Asn Glu Thr Cys Ala Lys Asn Cys Cys Leu Asp Gly Ala Ala Tyr Ala
                85                  90                  95
```

```
Ser Thr Tyr Gly Val Thr Thr Ser Gly Asn Ser Leu Ser Ile Gly Phe
            100                 105                 110

Val Thr Gln Ser Ala Gln Lys Asn Val Gly Ala Arg Leu Tyr Leu Met
        115                 120                 125

Ala Ser Asp Thr Thr Tyr Gln Glu Phe Thr Leu Leu Gly Asn Glu Phe
    130                 135                 140

Ser Phe Asp Val Asp Val Ser Gln Leu Pro Cys Gly Leu Asn Gly Ala
145                 150                 155                 160

Leu Tyr Phe Val Ser Met Asp Ala Asp Gly Gly Val Ser Lys Tyr Pro
                165                 170                 175

Thr Asn Thr Ala Gly Ala Lys Tyr Gly Thr Gly Tyr Cys Asp Ser Gln
            180                 185                 190

Cys Pro Arg Asp Leu Lys Phe Ile Asn Gly Gln Ala Asn Val Glu Gly
        195                 200                 205

Trp Glu Pro Ser Ser Asn Asn Ala Asn Thr Gly Ile Gly Gly His Gly
    210                 215                 220

Ser Cys Cys Ser Glu Met Asp Ile Trp Glu Ala Asn Ser Ile Ser Glu
225                 230                 235                 240

Ala Leu Thr Pro His Pro Cys Thr Thr Val Gly Gln Glu Ile Cys Glu
                245                 250                 255

Gly Asp Gly Cys Gly Gly Thr Tyr Ser Asp Asn Arg Tyr Gly Gly Thr
            260                 265                 270

Cys Asp Pro Asp Gly Cys Asp Trp Asn Pro Tyr Arg Leu Gly Asn Thr
        275                 280                 285

Ser Phe Tyr Gly Pro Gly Ser Ser Phe Thr Leu Asp Thr Thr Lys Lys
    290                 295                 300

Leu Thr Val Val Thr Gln Phe Glu Thr Ser Gly Ala Ile Asn Arg Tyr
305                 310                 315                 320

Tyr Val Gln Asn Gly Val Thr Phe Gln Gln Pro Asn Ala Glu Leu Gly
                325                 330                 335

Ser Tyr Ser Gly Asn Glu Leu Asn Asp Asp Tyr Cys Thr Ala Glu Glu
            340                 345                 350

Ala Glu Phe Gly Gly Ser Ser Phe Ser Asp Lys Gly Gly Leu Thr Gln
```

```
            355                 360                 365

Phe Lys Lys Ala Thr Ser Gly Gly Met Val Leu Val Met Ser Leu Trp
    370                 375                 380

Asp Asp Tyr Tyr Ala Asn Met Leu Trp Leu Asp Ser Thr Tyr Pro Thr
385                 390                 395                 400

Asn Glu Thr Ser Ser Thr Pro Gly Ala Val Arg Gly Ser Cys Ser Thr
                405                 410                 415

Ser Ser Gly Val Pro Ala Gln Val Glu Ser Gln Ser Pro Asn Ala Lys
            420                 425                 430

Val Thr Phe Ser Asn Ile Lys Phe Gly Pro Ile Gly Ser Thr Gly Asn
        435                 440                 445

Pro Ser Gly Gly Asn Pro Pro Gly Gly Asn Pro Pro Gly Thr Thr Thr
    450                 455                 460

Thr Arg Arg Pro Ala Thr Thr Thr Gly Ser Ser Pro Gly Pro Thr Gln
465                 470                 475                 480

Ser His Tyr Gly Gln Cys Gly Gly Ile Gly Tyr Ser Gly Pro Thr Val
                485                 490                 495

Cys Ala Ser Gly Thr Thr Cys Gln Val Leu Asn Pro Tyr Tyr Ser Gln
            500                 505                 510

Cys Leu
```

<210> 97
<211> 1611
<212> DNA
<213> Trichoderma reesei

<400> 97

```
atgattgtcg gcattctcac cacgctggct acgctggcca cactcgcagc tagtgtgcct      60
ctagaggagc ggcaagcttg ctcaagcgtc tggtaattat gtgaaccctc tcaagagacc     120
caaatactga gatatgtcaa ggggccaatg tggtggccag aattggtcgg gtccgacttg     180
ctgtgcttcc ggaagcacat gcgtctactc caacgactat tactcccagt gtcttcccgg     240
cgctgcaagc tcaagctcgt ccacgcgcgc cgcgtcgacg acttctcgag tatcccccac     300
aacatcccgg tcgagctccg cgacgcctcc acctggttct actactacca gagtacctcc     360
agtcggatcg ggaaccgcta cgtattcagg caaccctttt gttggggtca ctccttgggc     420
caatgcatat tacgcctctg aagttagcag cctcgctatt cctagcttga ctggagccat     480
ggccactgct gcagcagctg tcgcaaaggt tccctctttt atgtggctgt aggtcctccc     540
ggaaccaagg caatctgtta ctgaaggctc atcattcact gcagagatac tcttgacaag     600
acccctctca tggagcaaac cttggccgac atccgcaccg ccaacaagaa tggcggtaac     660
tatgccggac agtttgtggt gtatgacttg ccggatcgcg attgcgctgc ccttgcctcg     720
aatggcgaat actctattgc cgatggtggc gtcgccaaat ataagaacta tatcgacacc     780
attcgtcaaa ttgtcgtgga atattccgat atccggaccc tcctggttat tggtatgagt     840
ttaaacacct gcctcccccc ccccttccct tcctttcccg ccggcatctt gtcgttgtgc     900
taactattgt tccctcttcc agagcctgac tctcttgcca acctggtgac caacctcggt     960
actccaaagt gtgccaatgc tcagtcagcc taccttgagt gcatcaacta cgccgtcaca    1020
cagctgaacc ttccaaatgt tgcgatgtat ttggacgctg gccatgcagg atggcttggc    1080
tggccggcaa accaagaccc ggccgctcag ctatttgcaa atgtttacaa gaatgcatcg    1140
tctccgagag ctcttcgcgg attggcaacc aatgtcgcca actacaacgg gtggaacatt    1200
accagccccc catcgtacac gcaaggcaac gctgtctaca acgagaagct gtacatccac    1260
gctattggac gtcttcttgc caatcacggc tggtccaacg ccttcttcat cactgatcaa    1320
ggtcgatcgg gaaagcagcc taccggacag caacagtggg gagactggtg caatgtgatc    1380
ggcaccggat ttggtattcg cccatccgca aacactgggg actcgttgct ggattcgttt    1440
gtctgggtca agccaggcgg cgagtgtgac ggcaccagcg acagcagtgc gccacgattt    1500
gactcccact gtgcgctccc agatgccttg caaccggcgc ctcaagctgg tgcttggttc    1560
caagcctact ttgtgcagct tctcacaaac gcaaacccat cgttcctgta a             1611
```

<210> 98
<211> 471
<212> PRT
<213> Trichoderma reesei

<400> 98

```
Met Ile Val Gly Ile Leu Thr Thr Leu Ala Thr Leu Ala Thr Leu Ala
1               5                   10                  15

Ala Ser Val Pro Leu Glu Glu Arg Gln Ala Cys Ser Ser Val Trp Gly
            20                  25                  30

Gln Cys Gly Gly Gln Asn Trp Ser Gly Pro Thr Cys Cys Ala Ser Gly
        35                  40                  45

Ser Thr Cys Val Tyr Ser Asn Asp Tyr Tyr Ser Gln Cys Leu Pro Gly
    50                  55                  60

Ala Ala Ser Ser Ser Ser Ser Thr Arg Ala Ala Ser Thr Thr Ser Arg
65                  70                  75                  80
```

```
Val Ser Pro Thr Thr Ser Arg Ser Ser Ser Ala Thr Pro Pro Pro Gly
                85                  90                  95

Ser Thr Thr Thr Arg Val Pro Pro Val Gly Ser Gly Thr Ala Thr Tyr
            100                 105                 110

Ser Gly Asn Pro Phe Val Gly Val Thr Pro Trp Ala Asn Ala Tyr Tyr
        115                 120                 125

Ala Ser Glu Val Ser Ser Leu Ala Ile Pro Ser Leu Thr Gly Ala Met
    130                 135                 140

Ala Thr Ala Ala Ala Ala Val Ala Lys Val Pro Ser Phe Met Trp Leu
145                 150                 155                 160

Asp Thr Leu Asp Lys Thr Pro Leu Met Glu Gln Thr Leu Ala Asp Ile
                165                 170                 175

Arg Thr Ala Asn Lys Asn Gly Gly Asn Tyr Ala Gly Gln Phe Val Val
            180                 185                 190

Tyr Asp Leu Pro Asp Arg Asp Cys Ala Ala Leu Ala Ser Asn Gly Glu
        195                 200                 205

Tyr Ser Ile Ala Asp Gly Gly Val Ala Lys Tyr Lys Asn Tyr Ile Asp
    210                 215                 220

Thr Ile Arg Gln Ile Val Val Glu Tyr Ser Asp Ile Arg Thr Leu Leu
225                 230                 235                 240

Val Ile Glu Pro Asp Ser Leu Ala Asn Leu Val Thr Asn Leu Gly Thr
                245                 250                 255

Pro Lys Cys Ala Asn Ala Gln Ser Ala Tyr Leu Glu Cys Ile Asn Tyr
            260                 265                 270

Ala Val Thr Gln Leu Asn Leu Pro Asn Val Ala Met Tyr Leu Asp Ala
        275                 280                 285

Gly His Ala Gly Trp Leu Gly Trp Pro Ala Asn Gln Asp Pro Ala Ala
    290                 295                 300

Gln Leu Phe Ala Asn Val Tyr Lys Asn Ala Ser Ser Pro Arg Ala Leu
305                 310                 315                 320

Arg Gly Leu Ala Thr Asn Val Ala Asn Tyr Asn Gly Trp Asn Ile Thr
                325                 330                 335
```

Ser Pro Pro Ser Tyr Thr Gln Gly Asn Ala Val Tyr Asn Glu Lys Leu
340 345 350

Tyr Ile His Ala Ile Gly Arg Leu Leu Ala Asn His Gly Trp Ser Asn
355 360 365

Ala Phe Phe Ile Thr Asp Gln Gly Arg Ser Gly Lys Gln Pro Thr Gly
370 375 380

Gln Gln Gln Trp Gly Asp Trp Cys Asn Val Ile Gly Thr Gly Phe Gly
385 390 395 400

Ile Arg Pro Ser Ala Asn Thr Gly Asp Ser Leu Leu Asp Ser Phe Val
405 410 415

Trp Val Lys Pro Gly Gly Glu Cys Asp Gly Thr Ser Asp Ser Ser Ala
420 425 430

Pro Arg Phe Asp Ser His Cys Ala Leu Pro Asp Ala Leu Gln Pro Ala
435 440 445

Pro Gln Ala Gly Ala Trp Phe Gln Ala Tyr Phe Val Gln Leu Leu Thr
450 455 460

Asn Ala Asn Pro Ser Phe Leu
465 470

<210> 99
<211> 2046
<212> DNA
<213> Humicola insolens

<400> 99

```
gccgtgacct tgcgcgcttt gggtggcggt ggcgagtcgt ggacggtgct tgctggtcgc      60
cggccttccc ggcgatccgc gtgatgagag ggccaccaac ggcgggatga tgctccatgg     120
ggaacttccc catggagaag agagagaaac ttgcggagcc gtgatctggg gaaagatgct     180
ccgtgtctcg tctatataac tcgagtctcc ccgagccctc aacaccacca gctctgatct     240
caccatcccc atcgacaatc acgcaaacac agcagttgtc gggccattcc ttcagacaca     300
tcagtcaccc tccttcaaaa tgcgtaccgc caagttcgcc accctcgccg cccttgtggc     360
ctcggccgcc gcccagcagg cgtgcagtct caccaccgag aggcaccctt ccctctcttg     420
gaacaagtgc accgccggcg gccagtgcca gaccgtccag gcttccatca ctctcgactc     480
caactggcgc tggactcacc aggtgtctgg ctccaccaac tgctacacgg gcaacaagtg     540
ggatactagc atctgcactg atgccaagtc gtgcgctcag aactgctgcg tcgatggtgc     600
cgactacacc agcacctatg gcatcaccac caacggtgat tccctgagcc tcaagttcgt     660
caccaagggc cagcactcga ccaacgtcgg ctcgcgtacc tacctgatgg acggcgagga     720
```

```
caagtatcag agtacgttct atcttcagcc ttctcgcgcc ttgaatcctg gctaacgttt    780
acacttcaca gccttcgagc tcctcggcaa cgagttcacc ttcgatgtcg atgtctccaa    840
catcggctgc ggtctcaacg gcgccctgta cttcgtctcc atggacgccg atggtggtct    900
cagccgctat cctggcaaca aggctggtgc caagtacggt accggctact gcgatgctca    960
gtgcccccgt gacatcaagt tcatcaacgg cgaggccaac attgagggct ggaccggctc   1020
caccaacgac cccaacgccg gcgcgggccg ctatggtacc tgctgctctg agatggatat   1080
ctgggaagcc aacaacatgg ctactgcctt cactcctcac ccttgcacca tcattggcca   1140
gagccgctgc gagggcgact cgtgcggtgg cacctacagc aacgagcgct acgccggcgt   1200
ctgcgacccc gatggctgcg acttcaactc gtaccgccag ggcaacaaga ccttctacgg   1260
caagggcatg accgtcgaca ccaccaagaa gatcactgtc gtcacccagt tcctcaagga   1320
tgccaacggc gatctcggcg agatcaagcg cttctacgtc caggatggca agatcatccc   1380
caactccgag tccaccatcc ccggcgtcga gggcaattcc atcacccagg actggtgcga   1440
ccgccagaag gttgcctttg gcgacattga cgacttcaac cgcaagggcg gcatgaagca   1500
gatgggcaag gccctcgccg gccccatggt cctggtcatg tccatctggg atgaccacgc   1560
ctccaacatg ctctggctcg actcgacctt ccctgtcgat gccgctggca agcccggcgc   1620
cgagcgcggt gcctgcccga ccacctcggg tgtccctgct gaggttgagg ccgaggcccc   1680
caacagcaac gtcgtcttct ccaacatccg cttcggcccc atcggctcga ccgttgctgg   1740
tctccccggc gcgggcaacg gcggcaacaa cggcggcaac ccccccgcccc ccaccaccac   1800
cacctcctcg gctccggcca ccaccaccac cgccagcgct ggccccaagg ctggccgctg   1860
gcagcagtgc ggcggcatcg gcttcactgg cccgacccag tgcgaggagc cctacatttg   1920
caccaagctc aacgactggt actctcagtg cctgtaaatt ctgagtcgct gactcgacga   1980
tcacggccgg tttttgcatg aaaggaaaca aacgaccgcg ataaaaatgg agggtaatga   2040
gatgtc                                                              2046
```

<210> 100
<211> 525
<212> PRT
<213> Humicola insolens

<400> 100

```
Met Arg Thr Ala Lys Phe Ala Thr Leu Ala Ala Leu Val Ala Ser Ala
1               5                   10                  15

Ala Ala Gln Gln Ala Cys Ser Leu Thr Thr Glu Arg His Pro Ser Leu
            20                  25                  30

Ser Trp Asn Lys Cys Thr Ala Gly Gly Gln Cys Gln Thr Val Gln Ala
        35                  40                  45
```

```
Ser Ile Thr Leu Asp Ser Asn Trp Arg Trp Thr His Gln Val Ser Gly
    50                  55                  60

Ser Thr Asn Cys Tyr Thr Gly Asn Lys Trp Asp Thr Ser Ile Cys Thr
65                  70                  75                  80

Asp Ala Lys Ser Cys Ala Gln Asn Cys Cys Val Asp Gly Ala Asp Tyr
                85                  90                  95

Thr Ser Thr Tyr Gly Ile Thr Thr Asn Gly Asp Ser Leu Ser Leu Lys
            100                 105                 110

Phe Val Thr Lys Gly Gln His Ser Thr Asn Val Gly Ser Arg Thr Tyr
        115                 120                 125

Leu Met Asp Gly Glu Asp Lys Tyr Gln Thr Phe Glu Leu Leu Gly Asn
    130                 135                 140

Glu Phe Thr Phe Asp Val Asp Val Ser Asn Ile Gly Cys Gly Leu Asn
145                 150                 155                 160

Gly Ala Leu Tyr Phe Val Ser Met Asp Ala Asp Gly Gly Leu Ser Arg
                165                 170                 175

Tyr Pro Gly Asn Lys Ala Gly Ala Lys Tyr Gly Thr Gly Tyr Cys Asp
            180                 185                 190

Ala Gln Cys Pro Arg Asp Ile Lys Phe Ile Asn Gly Glu Ala Asn Ile
        195                 200                 205

Glu Gly Trp Thr Gly Ser Thr Asn Asp Pro Asn Ala Gly Ala Gly Arg
    210                 215                 220

Tyr Gly Thr Cys Cys Ser Glu Met Asp Ile Trp Glu Ala Asn Asn Met
225                 230                 235                 240

Ala Thr Ala Phe Thr Pro His Pro Cys Thr Ile Ile Gly Gln Ser Arg
                245                 250                 255

Cys Glu Gly Asp Ser Cys Gly Gly Thr Tyr Ser Asn Glu Arg Tyr Ala
            260                 265                 270

Gly Val Cys Asp Pro Asp Gly Cys Asp Phe Asn Ser Tyr Arg Gln Gly
        275                 280                 285

Asn Lys Thr Phe Tyr Gly Lys Gly Met Thr Val Asp Thr Thr Lys Lys
    290                 295                 300
```

```
Ile Thr Val Val Thr Gln Phe Leu Lys Asp Ala Asn Gly Asp Leu Gly
305                 310                 315                 320

Glu Ile Lys Arg Phe Tyr Val Gln Asp Gly Lys Ile Ile Pro Asn Ser
                325                 330                 335

Glu Ser Thr Ile Pro Gly Val Glu Gly Asn Ser Ile Thr Gln Asp Trp
            340                 345                 350

Cys Asp Arg Gln Lys Val Ala Phe Gly Asp Ile Asp Asp Phe Asn Arg
        355                 360                 365

Lys Gly Gly Met Lys Gln Met Gly Lys Ala Leu Ala Gly Pro Met Val
    370                 375                 380

Leu Val Met Ser Ile Trp Asp Asp His Ala Ser Asn Met Leu Trp Leu
385                 390                 395                 400

Asp Ser Thr Phe Pro Val Asp Ala Ala Gly Lys Pro Gly Ala Glu Arg
                405                 410                 415

Gly Ala Cys Pro Thr Thr Ser Gly Val Pro Ala Glu Val Glu Ala Glu
            420                 425                 430

Ala Pro Asn Ser Asn Val Val Phe Ser Asn Ile Arg Phe Gly Pro Ile
        435                 440                 445

Gly Ser Thr Val Ala Gly Leu Pro Gly Ala Gly Asn Gly Gly Asn Asn
    450                 455                 460

Gly Gly Asn Pro Pro Pro Pro Thr Thr Thr Thr Ser Ser Ala Pro Ala
465                 470                 475                 480

Thr Thr Thr Thr Ala Ser Ala Gly Pro Lys Ala Gly Arg Trp Gln Gln
                485                 490                 495

Cys Gly Gly Ile Gly Phe Thr Gly Pro Thr Gln Cys Glu Glu Pro Tyr
            500                 505                 510

Ile Cys Thr Lys Leu Asn Asp Trp Tyr Ser Gln Cys Leu
        515                 520                 525
```

<210> 101
<211> 1812
<212> DNA
<213> Myceliophthora thermophila

<400> 101

atggccaaga agcttttcat caccgccgcc cttgcggctg ccgtgttggc ggcccccgtc 60

attgaggagc gccagaactg cggcgctgtg tggtaagaaa gcccggtctg agtttcccat 120
gactttctca tcgagtaatg gcataaggcc cacccccttcg actgactgtg agaatcgatc 180
aaatccagga ctcaatgcgg cggcaacggg tggcagggtc ccacatgctg cgcctcgggc 240
tcgacctgcg ttgcgcagaa cgagtggtac tctcagtgcc tgcccaacaa tcaggtgacg 300
agttccaaca ctccgtcgtc gacttccacc tcgcagcgca gcagcagcac ctccagcagc 360
agcaccagga gcggcagctc ctcctcctcc accaccacgc cccctcccgt ctccagcccc 420
gtgactagca ttcccggcgg tgcgaccacc acggcgagct actctggcaa cccccttctcg 480
ggcgtccggc tcttcgccaa cgactactac aggtccgagg tccacaatct cgccattcct 540
agcatgaccg gtactctggc ggccaaggct tccgccgtcg ccgaagtccc tagcttccag 600
tggctcgacc ggaacgtcac catcgacacc ctgatggtcc agactctgtc ccagatccgg 660
gctgccaata atgccggtgc caatcctccc tatgctggtg agttacatgg cggcgacttg 720
ccttctcgtc ccccaccttt cttgacggga tcggttacct gacctggagg caaaacaaaa 780
ccagcccaac ttgtcgtcta cgacctcccc gaccgtgact gcgccgccgc tgcgtccaac 840
ggcgagtttt cgattgcaaa cggcggcgcc gccaactaca ggagctacat cgacgctatc 900
cgcaagcaca tcattgagta ctcggacatc cggatcatcc tggttatcga gcccgactcg 960
atggccaaca tggtgaccaa catgaacgtg gccaagtgca gcaacgccgc gtcgacgtac 1020
cacgagttga ccgtgtacgc gctcaagcag ctgaacctgc ccaacgtcgc catgtatctc 1080
gacgccggcc acgccggctg gctcggctgg cccgccaaca tccagcccgc cgccgacctg 1140
tttgccggca tctacaatga cgccggcaag ccggctgccg tccgcggcct ggccactaac 1200
gtcgccaact acaacgcctg gagtatcgct tcggccccgt cgtacacgtc cctaaccct 1260
aactacgacg agaagcacta catcgaggcc ttcagcccgc tcctgaacgc ggccggcttc 1320
cccgcacgct tcattgtcga cactggccgc aacggcaaac aacctaccgg tatggttttt 1380
ttcttttttt ttctctgttc ccctccccct tccccttcag ttggcgtcca caaggtctct 1440
tagtcttgct tcttctcgga ccaaccttcc cccaccccca aaacgcaccg cccacaaccg 1500
ttcgactcta tactcttggg aatgggcgcc gaaactgacc gttcgacagg ccaacaacag 1560
tggggtgact ggtgcaatgt caagggcact ggctttggcg tgcgcccgac ggccaacacg 1620
ggccacgacc tggtcgatgc ctttgtctgg gtcaagcccg gcggcgagtc cgacggcaca 1680
agcgacacca gcgccgcccg ctacgactac cactgcggcc tgtccgatgc cctgcagcct 1740
gctccggagg ctggacagtg gttccaggcc tacttcgagc agctgctcac caacgccaac 1800
ccgcccttct aa 1812

<210> 102

<211> 482
<212> PRT
<213> Myceliophthora thermophila

<400> 102

```
Met Ala Lys Lys Leu Phe Ile Thr Ala Ala Leu Ala Ala Ala Val Leu
1               5                   10                  15

Ala Ala Pro Val Ile Glu Glu Arg Gln Asn Cys Gly Ala Val Trp Thr
            20                  25                  30

Gln Cys Gly Gly Asn Gly Trp Gln Gly Pro Thr Cys Cys Ala Ser Gly
        35                  40                  45

Ser Thr Cys Val Ala Gln Asn Glu Trp Tyr Ser Gln Cys Leu Pro Asn
    50                  55                  60

Asn Gln Val Thr Ser Ser Asn Thr Pro Ser Ser Thr Ser Thr Ser Gln
65                  70                  75                  80

Arg Ser Ser Ser Thr Ser Ser Ser Ser Thr Arg Ser Gly Ser Ser Ser
                85                  90                  95

Ser Ser Thr Thr Thr Pro Pro Pro Val Ser Ser Pro Val Thr Ser Ile
            100                 105                 110

Pro Gly Gly Ala Thr Thr Thr Ala Ser Tyr Ser Gly Asn Pro Phe Ser
        115                 120                 125

Gly Val Arg Leu Phe Ala Asn Asp Tyr Tyr Arg Ser Glu Val His Asn
    130                 135                 140

Leu Ala Ile Pro Ser Met Thr Gly Thr Leu Ala Ala Lys Ala Ser Ala
145                 150                 155                 160

Val Ala Glu Val Pro Ser Phe Gln Trp Leu Asp Arg Asn Val Thr Ile
                165                 170                 175

Asp Thr Leu Met Val Gln Thr Leu Ser Gln Ile Arg Ala Ala Asn Asn
            180                 185                 190

Ala Gly Ala Asn Pro Pro Tyr Ala Ala Gln Leu Val Val Tyr Asp Leu
        195                 200                 205

Pro Asp Arg Asp Cys Ala Ala Ala Ala Ser Asn Gly Glu Phe Ser Ile
    210                 215                 220

Ala Asn Gly Gly Ala Ala Asn Tyr Arg Ser Tyr Ile Asp Ala Ile Arg
225                 230                 235                 240

Lys His Ile Ile Glu Tyr Ser Asp Ile Arg Ile Ile Leu Val Ile Glu
                245                 250                 255
```

```
Pro Asp Ser Met Ala Asn Met Val Thr Asn Met Asn Val Ala Lys Cys
            260                 265                 270

Ser Asn Ala Ala Ser Thr Tyr His Glu Leu Thr Val Tyr Ala Leu Lys
        275                 280                 285

Gln Leu Asn Leu Pro Asn Val Ala Met Tyr Leu Asp Ala Gly His Ala
    290                 295                 300

Gly Trp Leu Gly Trp Pro Ala Asn Ile Gln Pro Ala Ala Asp Leu Phe
305                 310                 315                 320

Ala Gly Ile Tyr Asn Asp Ala Gly Lys Pro Ala Ala Val Arg Gly Leu
                325                 330                 335

Ala Thr Asn Val Ala Asn Tyr Asn Ala Trp Ser Ile Ala Ser Ala Pro
            340                 345                 350

Ser Tyr Thr Ser Pro Asn Pro Asn Tyr Asp Glu Lys His Tyr Ile Glu
        355                 360                 365

Ala Phe Ser Pro Leu Leu Asn Ala Ala Gly Phe Pro Ala Arg Phe Ile
    370                 375                 380

Val Asp Thr Gly Arg Asn Gly Lys Gln Pro Thr Gly Gln Gln Gln Trp
385                 390                 395                 400

Gly Asp Trp Cys Asn Val Lys Gly Thr Gly Phe Gly Val Arg Pro Thr
                405                 410                 415

Ala Asn Thr Gly His Asp Leu Val Asp Ala Phe Val Trp Val Lys Pro
            420                 425                 430

Gly Gly Glu Ser Asp Gly Thr Ser Asp Thr Ser Ala Ala Arg Tyr Asp
        435                 440                 445

Tyr His Cys Gly Leu Ser Asp Ala Leu Gln Pro Ala Pro Glu Ala Gly
    450                 455                 460

Gln Trp Phe Gln Ala Tyr Phe Glu Gln Leu Leu Thr Asn Ala Asn Pro
465                 470                 475                 480

Pro Phe
```

<210> 103
<211> 1802
<212> DNA

<213> Myceliophthora thermophila

<400> 103

atggccaaga agcttttcat caccgccgcg cttgcggctg ccgtgttggc ggcccccgtc 60
attgaggagc gccagaactg cggcgctgtg tggtaagaaa gcccggtccg agtctcccat 120
gattttctcg tcgagtaatg gcataagggc caccccttcg actgaccgtg agaatcgatc 180
aaatccagga ctcaatgcgg cggtaacggg tggcaaggtc ccacatgctg cgcctcgggc 240
tcgacctgcg ttgcgcagaa cgagtggtac tctcagtgcc tgcccaacag ccaggtgacg 300
agttccacca ctccgtcgtc gacttccacc tcgcagcgca gcaccagcac ctccagcagc 360
accaccagga gcggcagctc ctcctcctcc tccaccacgc ccccgcccgt ctccagcccc 420
gtgaccagca ttcccggcgg tgcgacctcc acggcgagct actctggcaa ccccttctcg 480
ggcgtccggc tcttcgccaa cgactactac aggtccgagg tccacaatct cgccattcct 540
agcatgactg gtactctggc ggccaaggct tccgccgtcg ccgaagtccc tagcttccag 600
tggctcgacc ggaacgtcac catcgacacc ctgatggtcc agactctgtc ccaggtccgg 660
gctctcaata aggccggtgc caatcctccc tatgctggtg agttacatgg cgacttgcct 720
tctcgtcccc tacctttctt gacgggatcg gttacctgac ctggaggcaa aacaacaaca 780
gcccaactcg tcgtctacga cctccccgac cgtgactgtg ccgccgctgc gtccaacggc 840
gagttttcga ttgcaaacgg cggcgccgcc aactacagga gctacatcga cgctatccgc 900
aagcacatca ttgagtactc ggacatccgg atcatcctgg ttatcgagcc cgactcgatg 960
gccaacatgg tgaccaacat gaacgtggcc aagtgcagca acgccgcgtc gacgtaccac 1020
gagttgaccg tgtacgcgct caagcagctg aacctgccca acgtcgccat gtatctcgac 1080
gccggccacg ccggctggct cggctggccc gccaacatcc agcccgccgc cgagctgttt 1140
gccggcatct acaatgatgc cggcaagccg gctgccgtcc gcggcctggc cactaacgtc 1200
gccaactaca acgcctggag catcgcttcg gccccgtcgt acacgtcgcc taaccctaac 1260
tacgacgaga agcactacat cgaggccttc agcccgctct tgaactcggc cggcttcccc 1320
gcacgcttca ttgtcgacac tggccgcaac ggcaaacaac ctaccggtat gttttttttt 1380
cttttgtctc tgtccccccc ttttctcccc cttcagttgg cgtccacaag gtctcttagt 1440
cctgcttcat ctgtgaccaa cctccccccc cccggcaccg cccacaaccg tttgactcta 1500
tactcttggg aatgggcgcc gaaactgacc gttccacagg ccaacaacag tggggtgact 1560
ggtgcaatgt caagggcacc ggctttggcg tgcgcccgac ggccaacacg ggccacgagc 1620
tggtcgatgc ctttgtctgg gtcaagcccg gcggcgagtc cgacggcaca agcgacacca 1680
gcgccgcccg ctacgactac cactgcggcc tgtccgatgc cctgcagcct gcccccgagg 1740
ctggacagtg gttccaggcc tacttcgagc agctgctcac caacgccaac ccgcccttct 1800
aa 1802

<210> 104
<211> 481
<212> PRT
<213> Myceliophthora thermophila

<400> 104

```
Met Ala Lys Lys Leu Phe Ile Thr Ala Ala Leu Ala Ala Ala Val Leu
1               5                   10                  15

Ala Ala Pro Val Ile Glu Glu Arg Gln Asn Cys Gly Ala Val Trp Thr
            20                  25                  30

Gln Cys Gly Gly Asn Gly Trp Gln Gly Pro Thr Cys Cys Ala Ser Gly
        35                  40                  45

Ser Thr Cys Val Ala Gln Asn Glu Trp Tyr Ser Gln Cys Leu Pro Asn
    50                  55                  60

Ser Gln Val Thr Ser Ser Thr Thr Pro Ser Ser Thr Ser Thr Ser Gln
65                  70                  75                  80

Arg Ser Thr Ser Thr Ser Ser Ser Thr Thr Arg Ser Gly Ser Ser Ser
                85                  90                  95

Ser Ser Ser Thr Thr Pro Pro Pro Val Ser Ser Pro Val Thr Ser Ile
            100                 105                 110

Pro Gly Gly Ala Thr Ser Thr Ala Ser Tyr Ser Gly Asn Pro Phe Ser
        115                 120                 125

Gly Val Arg Leu Phe Ala Asn Asp Tyr Tyr Arg Ser Glu Val His Asn
    130                 135                 140

Leu Ala Ile Pro Ser Met Thr Gly Thr Leu Ala Ala Lys Ala Ser Ala
145                 150                 155                 160

Val Ala Glu Val Pro Ser Phe Gln Trp Leu Asp Arg Asn Val Thr Ile
                165                 170                 175

Asp Thr Leu Met Val Gln Thr Leu Ser Gln Val Arg Ala Leu Asn Lys
            180                 185                 190

Ala Gly Ala Asn Pro Pro Tyr Ala Ala Gln Leu Val Val Tyr Asp Leu
        195                 200                 205

Pro Asp Arg Asp Cys Ala Ala Ala Ala Ser Asn Gly Glu Phe Ser Ile
    210                 215                 220

Ala Asn Gly Gly Ala Ala Asn Tyr Arg Ser Tyr Ile Asp Ala Ile Arg
```

225 230 235 240

Lys His Ile Ile Glu Tyr Ser Asp Ile Arg Ile Ile Leu Val Ile Glu
245 250 255

Pro Asp Ser Met Ala Asn Met Val Thr Asn Met Asn Val Ala Lys Cys
260 265 270

Ser Asn Ala Ala Ser Thr Tyr His Glu Leu Thr Val Tyr Ala Leu Lys
275 280 285

Gln Leu Asn Leu Pro Asn Val Ala Met Tyr Leu Asp Ala Gly His Ala
290 295 300

Gly Trp Leu Gly Trp Pro Ala Asn Ile Gln Pro Ala Ala Glu Leu Phe
305 310 315 320

Ala Gly Ile Tyr Asn Asp Ala Gly Lys Pro Ala Ala Val Arg Gly Leu
325 330 335

Ala Thr Asn Val Ala Asn Tyr Asn Ala Trp Ser Ile Ala Ser Ala Pro
340 345 350

Ser Tyr Thr Ser Pro Asn Pro Asn Tyr Asp Glu Lys His Tyr Ile Glu
355 360 365

Ala Phe Ser Pro Leu Leu Asn Ser Ala Gly Phe Pro Ala Arg Phe Ile
370 375 380

Val Asp Thr Gly Arg Asn Gly Lys Gln Pro Thr Gly Gln Gln Gln Trp
385 390 395 400

Gly Asp Trp Cys Asn Val Lys Gly Thr Gly Phe Gly Val Arg Pro Thr
405 410 415

Ala Asn Thr Gly His Glu Leu Val Asp Ala Phe Val Trp Val Lys Pro
420 425 430

Gly Gly Glu Ser Asp Gly Thr Ser Asp Thr Ser Ala Ala Arg Tyr Asp
435 440 445

Tyr His Cys Gly Leu Ser Asp Ala Leu Gln Pro Ala Pro Glu Ala Gly
450 455 460

Gln Trp Phe Gln Ala Tyr Phe Glu Gln Leu Leu Thr Asn Ala Asn Pro
465 470 475 480

Pro

<210> 105
<211> 1446
<212> DNA
<213> Thielavia terrestris

<400> 105

```
atggctcaga agctccttct cgccgccgcc cttgcggcca gcgccctcgc tgctcccgtc      60
gtcgaggagc gccagaactg cggttccgtc tggagccaat gcggcggcat tggctggtcc     120
ggcgcgacct gctgcgcttc gggcaatacc tgcgttgagc tgaacccgta ctactcgcag     180
tgcctgccca acagccaggt gactacctcg accagcaaga ccacctccac caccaccagg     240
agcagcacca ccagccacag cagcggtccc accagcacga gcaccaccac caccagcagt     300
cccgtggtca ctaccccgcc gagtacctcc atccccggcg gtgcctcgtc aacggccagc     360
tggtccggca acccgttctc gggcgtgcag atgtgggcca acgactacta cgcctccgag     420
gtctcgtcgc tggccatccc cagcatgacg ggcgccatgg ccaccaaggc ggccgaggtg     480
gccaaggtgc ccagcttcca gtggcttgac cgcaacgtca ccatcgacac gctgttcgcc     540
cacacgctgt cgcagatccg cgcggccaac cagaaaggcg ccaacccgcc ctacgcgggc     600
atcttcgtgg tctacgacct tccggaccgc gactgcgccg ccgccgcgtc caacggcgag     660
ttctccatcg cgaacaacgg ggcggccaac tacaagacgt acatcgacgc gatccggagc     720
ctcgtcatcc agtactcaga catccgcatc atcttcgtca tcgagcccga ctcgctggcc     780
aacatggtga ccaacctgaa cgtggccaag tgcgccaacg ccgagtcgac ctacaaggag     840
ttgaccgtct acgcgctgca gcagctgaac ctgcccaacg tggccatgta cctggacgcc     900
ggccacgccg gctggctcgg ctggcccgcc aacatccagc cggccgccaa cctcttcgcc     960
gagatctaca cgagcgccgg caagccggcc gccgtgcgcg gcctcgccac caacgtggcc    1020
aactacaacg gctggagcct ggccacgccg ccctcgtaca cccagggcga ccccaactac    1080
gacgagagcc actacgtcca ggccctcgcc ccgctgctca ccgccaacgg cttccccgcc    1140
cacttcatca ccgacaccgg ccgcaacggc aagcagccga ccggacaacg gcaatgggga    1200
gactggtgca acgttatcgg aactggcttc ggcgtgcgcc cgacgacaaa caccggcctc    1260
gacatcgagg acgccttcgt ctgggtcaag cccggcggcg agtgcgacgg cacgagcaac    1320
acgacctctc cccgctacga ctaccactgc ggcctgtcgg acgcgctgca gcctgctccg    1380
gaggccggca cttggttcca ggcctacttc gagcagctcc tgaccaacgc caacccgccc    1440
ttttaa                                                                1446
```

<210> 106
<211> 481
<212> PRT
<213> Thielavia terrestris

<400> 106

```
Met Ala Gln Lys Leu Leu Leu Ala Ala Ala Leu Ala Ala Ser Ala Leu
1               5                   10                  15

Ala Ala Pro Val Val Glu Glu Arg Gln Asn Cys Gly Ser Val Trp Ser
            20                  25                  30

Gln Cys Gly Gly Ile Gly Trp Ser Gly Ala Thr Cys Cys Ala Ser Gly
        35                  40                  45

Asn Thr Cys Val Glu Leu Asn Pro Tyr Tyr Ser Gln Cys Leu Pro Asn
    50                  55                  60

Ser Gln Val Thr Thr Ser Thr Ser Lys Thr Thr Ser Thr Thr Thr Arg
65                  70                  75                  80

Ser Ser Thr Thr Ser His Ser Ser Gly Pro Thr Ser Thr Ser Thr Thr
                85                  90                  95

Thr Thr Ser Ser Pro Val Val Thr Thr Pro Pro Ser Thr Ser Ile Pro
            100                 105                 110

Gly Gly Ala Ser Ser Thr Ala Ser Trp Ser Gly Asn Pro Phe Ser Gly
        115                 120                 125

Val Gln Met Trp Ala Asn Asp Tyr Tyr Ala Ser Glu Val Ser Ser Leu
    130                 135                 140

Ala Ile Pro Ser Met Thr Gly Ala Met Ala Thr Lys Ala Ala Glu Val
145                 150                 155                 160

Ala Lys Val Pro Ser Phe Gln Trp Leu Asp Arg Asn Val Thr Ile Asp
                165                 170                 175

Thr Leu Phe Ala His Thr Leu Ser Gln Ile Arg Ala Ala Asn Gln Lys
            180                 185                 190

Gly Ala Asn Pro Pro Tyr Ala Gly Ile Phe Val Val Tyr Asp Leu Pro
        195                 200                 205

Asp Arg Asp Cys Ala Ala Ala Ala Ser Asn Gly Glu Phe Ser Ile Ala
    210                 215                 220

Asn Asn Gly Ala Ala Asn Tyr Lys Thr Tyr Ile Asp Ala Ile Arg Ser
225                 230                 235                 240

Leu Val Ile Gln Tyr Ser Asp Ile Arg Ile Ile Phe Val Ile Glu Pro
                245                 250                 255
```

Asp Ser Leu Ala Asn Met Val Thr Asn Leu Asn Val Ala Lys Cys Ala
260 265 270

Asn Ala Glu Ser Thr Tyr Lys Glu Leu Thr Val Tyr Ala Leu Gln Gln
275 280 285

Leu Asn Leu Pro Asn Val Ala Met Tyr Leu Asp Ala Gly His Ala Gly
290 295 300

Trp Leu Gly Trp Pro Ala Asn Ile Gln Pro Ala Ala Asn Leu Phe Ala
305 310 315 320

Glu Ile Tyr Thr Ser Ala Gly Lys Pro Ala Ala Val Arg Gly Leu Ala
325 330 335

Thr Asn Val Ala Asn Tyr Asn Gly Trp Ser Leu Ala Thr Pro Pro Ser
340 345 350

Tyr Thr Gln Gly Asp Pro Asn Tyr Asp Glu Ser His Tyr Val Gln Ala
355 360 365

Leu Ala Pro Leu Leu Thr Ala Asn Gly Phe Pro Ala His Phe Ile Thr
370 375 380

Asp Thr Gly Arg Asn Gly Lys Gln Pro Thr Gly Gln Arg Gln Trp Gly
385 390 395 400

Asp Trp Cys Asn Val Ile Gly Thr Gly Phe Gly Val Arg Pro Thr Thr
405 410 415

Asn Thr Gly Leu Asp Ile Glu Asp Ala Phe Val Trp Val Lys Pro Gly
420 425 430

Gly Glu Cys Asp Gly Thr Ser Asn Thr Thr Ser Pro Arg Tyr Asp Tyr
435 440 445

His Cys Gly Leu Ser Asp Ala Leu Gln Pro Ala Pro Glu Ala Gly Thr
450 455 460

Trp Phe Gln Ala Tyr Phe Glu Gln Leu Leu Thr Asn Ala Asn Pro Pro
465 470 475 480

Phe

<210> 107
<211> 1593
<212> DNA

<213> Chaetomium thermophilum

<400> 107

```
atgatgtaca agaagttcgc cgctctcgcc gccctcgtgg ctggcgccgc cgcccagcag      60
gcttgctccc tcaccactga gacccacccc agactcactt ggaagcgctg cacctctggc     120
ggcaactgct cgaccgtgaa cggcgccgtc accatcgatg ccaactggcg ctggactcac     180
actgtttccg gctcgaccaa ctgctacacc ggcaacgagt gggatacctc catctgctct     240
gatggcaaga gctgcgccca gacctgctgc gtcgacggcg ctgactactc ttcgacctat     300
ggtatcacca ccagcggtga ctccctgaac ctcaagttcg tcaccaagca ccagcacggc     360
accaatgtcg gctctcgtgt ctacctgatg gagaacgaca ccaagtacca gatgttcgag     420
ctcctcggca acgagttcac cttcgatgtc gatgtctcta acctgggctg cggtctcaac     480
ggcgccctct acttcgtctc catggacgct gatggtggta tgagcaagta ctctggcaac     540
aaggctggcg ccaagtacgg taccggctac tgcgatgctc agtgcccgcg cgaccttaag     600
ttcatcaacg gcgaggccaa cattgagaac tggacccctt cgaccaatga tgccaacgcc     660
ggtttcggcc gctatggcag ctgctgctct gagatggata tctgggatgc caacaacatg     720
gctactgcct tcactcctca cccttgcacc attatcggcc agagccgctg cgagggcaac     780
agctgcggtg gcacctacag ctctgagcgc tatgctggtg tttgcgatcc tgatggctgc     840
gacttcaacg cctaccgcca gggcgacaag accttctacg gcaagggcat gaccgtcgac     900
accaccaaga agatgaccgt cgtcacccag ttccacaaga actcggctgg cgtcctcagc     960
gagatcaagc gcttctacgt tcaggacggc aagatcattg ccaacgccga gtccaagatc    1020
cccggcaacc ccggcaactc catcacccag gagtggtgcg atgcccagaa ggtcgccttc    1080
ggtgacatcg atgacttcaa ccgcaagggc ggtatggctc agatgagcaa ggccctcgag    1140
ggccctatgg tcctggtcat gtccgtctgg gatgaccact acgccaacat gctctggctc    1200
gactcgacct accccattga caaggccggc acccccggcg ccgagcgcgg tgcttgcccg    1260
accacctccg gtgtccctgc cgagattgag gcccaggtcc ccaacagcaa cgttatcttc    1320
tccaacatcc gcttcggccc catcggctcg accgtccctg gcctcgacgg cagcaccccc    1380
agcaacccga ccgccaccgt tgctcctccc acttctacca ccaccagcgt gagaagcagc    1440
actactcaga tttccacccc gactagccag cccggcggct gcaccaccca gaagtggggc    1500
cagtgcggtg gtatcggcta caccggctgc actaactgcg ttgctggcac tacctgcact    1560
gagctcaacc cctggtacag ccagtgcctg taa                                 1593
```

<210> 108
<211> 530
<212> PRT

<213> Chaetomium thermophilum

<400> 108

```
Met Met Tyr Lys Lys Phe Ala Ala Leu Ala Ala Leu Val Ala Gly Ala
1               5                   10                  15
```

```
Ala Ala Gln Gln Ala Cys Ser Leu Thr Thr Glu Thr His Pro Arg Leu
            20                  25                  30

Thr Trp Lys Arg Cys Thr Ser Gly Gly Asn Cys Ser Thr Val Asn Gly
        35                  40                  45

Ala Val Thr Ile Asp Ala Asn Trp Arg Trp Thr His Thr Val Ser Gly
    50                  55                  60

Ser Thr Asn Cys Tyr Thr Gly Asn Glu Trp Asp Thr Ser Ile Cys Ser
65                  70                  75                  80

Asp Gly Lys Ser Cys Ala Gln Thr Cys Cys Val Asp Gly Ala Asp Tyr
                85                  90                  95

Ser Ser Thr Tyr Gly Ile Thr Thr Ser Gly Asp Ser Leu Asn Leu Lys
            100                 105                 110

Phe Val Thr Lys His Gln His Gly Thr Asn Val Gly Ser Arg Val Tyr
        115                 120                 125

Leu Met Glu Asn Asp Thr Lys Tyr Gln Met Phe Glu Leu Leu Gly Asn
    130                 135                 140

Glu Phe Thr Phe Asp Val Asp Val Ser Asn Leu Gly Cys Gly Leu Asn
145                 150                 155                 160

Gly Ala Leu Tyr Phe Val Ser Met Asp Ala Asp Gly Gly Met Ser Lys
                165                 170                 175

Tyr Ser Gly Asn Lys Ala Gly Ala Lys Tyr Gly Thr Gly Tyr Cys Asp
            180                 185                 190

Ala Gln Cys Pro Arg Asp Leu Lys Phe Ile Asn Gly Glu Ala Asn Ile
        195                 200                 205

Glu Asn Trp Thr Pro Ser Thr Asn Asp Ala Asn Ala Gly Phe Gly Arg
    210                 215                 220

Tyr Gly Ser Cys Cys Ser Glu Met Asp Ile Trp Asp Ala Asn Asn Met
225                 230                 235                 240

Ala Thr Ala Phe Thr Pro His Pro Cys Thr Ile Ile Gly Gln Ser Arg
                245                 250                 255

Cys Glu Gly Asn Ser Cys Gly Gly Thr Tyr Ser Ser Glu Arg Tyr Ala
            260                 265                 270
```

Gly Val Cys Asp Pro Asp Gly Cys Asp Phe Asn Ala Tyr Arg Gln Gly
275 280 285

Asp Lys Thr Phe Tyr Gly Lys Gly Met Thr Val Asp Thr Thr Lys Lys
290 295 300

Met Thr Val Val Thr Gln Phe His Lys Asn Ser Ala Gly Val Leu Ser
305 310 315 320

Glu Ile Lys Arg Phe Tyr Val Gln Asp Gly Lys Ile Ile Ala Asn Ala
325 330 335

Glu Ser Lys Ile Pro Gly Asn Pro Gly Asn Ser Ile Thr Gln Glu Trp
340 345 350

Cys Asp Ala Gln Lys Val Ala Phe Gly Asp Ile Asp Asp Phe Asn Arg
355 360 365

Lys Gly Gly Met Ala Gln Met Ser Lys Ala Leu Glu Gly Pro Met Val
370 375 380

Leu Val Met Ser Val Trp Asp Asp His Tyr Ala Asn Met Leu Trp Leu
385 390 395 400

Asp Ser Thr Tyr Pro Ile Asp Lys Ala Gly Thr Pro Gly Ala Glu Arg
405 410 415

Gly Ala Cys Pro Thr Thr Ser Gly Val Pro Ala Glu Ile Glu Ala Gln
420 425 430

Val Pro Asn Ser Asn Val Ile Phe Ser Asn Ile Arg Phe Gly Pro Ile
435 440 445

Gly Ser Thr Val Pro Gly Leu Asp Gly Ser Thr Pro Ser Asn Pro Thr
450 455 460

Ala Thr Val Ala Pro Pro Thr Ser Thr Thr Thr Ser Val Arg Ser Ser
465 470 475 480

Thr Thr Gln Ile Ser Thr Pro Thr Ser Gln Pro Gly Gly Cys Thr Thr
485 490 495

Gln Lys Trp Gly Gln Cys Gly Gly Ile Gly Tyr Thr Gly Cys Thr Asn
500 505 510

Cys Val Ala Gly Thr Thr Cys Thr Glu Leu Asn Pro Trp Tyr Ser Gln
515 520 525

Cys Leu
530

<210> 109
<211> 1434
<212> DNA
<213> Chaetomium thermophilum

<400> 109

```
atggctaagc agctgctgct cactgccgct cttgcggcca cttcgctggc tgcccctctc      60
cttgaggagc gccagagctg ctcctccgtc tggggtcaat gcggtggcat caattacaac     120
ggcccgacct gctgccagtc cggcagtgtt tgcacttacc tgaatgactg gtacagccag     180
tgcattcccg gtcaggctca gcccggcacg actagcacca cggctcggac caccagcacc     240
agcaccacca gcacttcgtc ggtccgcccg accacctcga ataccccctgt gacgactgct     300
cccccgacga ccaccatccc gggcggcgcc tcgagcacgg ccagctacaa cggcaacccg     360
ttttcgggtg ttcaactttg ggccaacacc tactactcgt ccgaggtgca cactttggcc     420
atccccagct tgtctcctga gctggctgcc aaggccgcca aggtcgctga ggttcccagc     480
ttccagtggc tcgaccgcaa tgtgactgtt gacactctct tctccggcac tcttgccgaa     540
atccgcgccg ccaaccagcg cggtgccaac ccgccttatg ccggcatttt cgtggtttat     600
gacttaccag accgtgattg cgcggctgct gcttcgaacg gcgagtggtc tatcgccaac     660
aatggtgcca acaactacaa gcgctacatc gaccggatcc gtgagctcct tatccagtac     720
tccgatatcc gcactattct ggtcattgaa cctgattccc tggccaacat ggtcaccaac     780
atgaacgtcc agaagtgctc gaacgctgcc tccacttaca aggagcttac tgtctatgcc     840
ctcaaacagc tcaatcttcc tcacgttgcc atgtacatgg atgctggcca cgctggctgg     900
cttggctggc ccgccaacat ccagcctgct gctgagctct ttgctcaaat ctaccgcgac     960
gctggcaggc ccgctgctgt ccgcggtctt gcgaccaacg ttgccaacta caatgcttgg    1020
tcgatcgcca gccctccgtc ctacacctct cctaacccga actacgacga gaagcactat    1080
attgaggcct ttgctcctct tctccgcaac cagggcttcg acgcaaagtt catcgtcgac    1140
accggccgta acggcaagca gcccactggc cagcttgaat ggggtcactg gtgcaatgtc    1200
aagggaactg gcttcggtgt gcgccctact gctaacactg ggcatgaact tgttgatgct    1260
ttcgtgtggg tcaagcccgg tggcgagtcc gacggcacca gtgcggacac cagcgctgct    1320
cgttatgact atcactgcgg cctttccgac gcactgactc cggcgcctga ggctggccaa    1380
tggttccagg cttatttcga acagctgctc atcaatgcca accctccgct ctga          1434
```

<210> 110
<211> 477
<212> PRT
<213> Chaetomium thermophilum

<400> 110

```
Met Ala Lys Gln Leu Leu Leu Thr Ala Ala Leu Ala Ala Thr Ser Leu
1               5                   10                  15

Ala Ala Pro Leu Leu Glu Glu Arg Gln Ser Cys Ser Ser Val Trp Gly
            20                  25                  30

Gln Cys Gly Gly Ile Asn Tyr Asn Gly Pro Thr Cys Cys Gln Ser Gly
        35                  40                  45

Ser Val Cys Thr Tyr Leu Asn Asp Trp Tyr Ser Gln Cys Ile Pro Gly
    50                  55                  60

Gln Ala Gln Pro Gly Thr Thr Ser Thr Thr Ala Arg Thr Thr Ser Thr
65                  70                  75                  80

Ser Thr Thr Ser Thr Ser Ser Val Arg Pro Thr Thr Ser Asn Thr Pro
                85                  90                  95

Val Thr Thr Ala Pro Pro Thr Thr Thr Ile Pro Gly Gly Ala Ser Ser
            100                 105                 110

Thr Ala Ser Tyr Asn Gly Asn Pro Phe Ser Gly Val Gln Leu Trp Ala
        115                 120                 125

Asn Thr Tyr Tyr Ser Ser Glu Val His Thr Leu Ala Ile Pro Ser Leu
    130                 135                 140

Ser Pro Glu Leu Ala Ala Lys Ala Ala Lys Val Ala Glu Val Pro Ser
145                 150                 155                 160

Phe Gln Trp Leu Asp Arg Asn Val Thr Val Asp Thr Leu Phe Ser Gly
                165                 170                 175

Thr Leu Ala Glu Ile Arg Ala Ala Asn Gln Arg Gly Ala Asn Pro Pro
            180                 185                 190

Tyr Ala Gly Ile Phe Val Val Tyr Asp Leu Pro Asp Arg Asp Cys Ala
        195                 200                 205

Ala Ala Ala Ser Asn Gly Glu Trp Ser Ile Ala Asn Asn Gly Ala Asn
    210                 215                 220

Asn Tyr Lys Arg Tyr Ile Asp Arg Ile Arg Glu Leu Leu Ile Gln Tyr
225                 230                 235                 240

Ser Asp Ile Arg Thr Ile Leu Val Ile Glu Pro Asp Ser Leu Ala Asn
                245                 250                 255
```

```
Met Val Thr Asn Met Asn Val Gln Lys Cys Ser Asn Ala Ala Ser Thr
            260                 265                 270

Tyr Lys Glu Leu Thr Val Tyr Ala Leu Lys Gln Leu Asn Leu Pro His
        275                 280                 285

Val Ala Met Tyr Met Asp Ala Gly His Ala Gly Trp Leu Gly Trp Pro
    290                 295                 300

Ala Asn Ile Gln Pro Ala Ala Glu Leu Phe Ala Gln Ile Tyr Arg Asp
305                 310                 315                 320

Ala Gly Arg Pro Ala Ala Val Arg Gly Leu Ala Thr Asn Val Ala Asn
                325                 330                 335

Tyr Asn Ala Trp Ser Ile Ala Ser Pro Pro Ser Tyr Thr Ser Pro Asn
            340                 345                 350

Pro Asn Tyr Asp Glu Lys His Tyr Ile Glu Ala Phe Ala Pro Leu Leu
        355                 360                 365

Arg Asn Gln Gly Phe Asp Ala Lys Phe Ile Val Asp Thr Gly Arg Asn
    370                 375                 380

Gly Lys Gln Pro Thr Gly Gln Leu Glu Trp Gly His Trp Cys Asn Val
385                 390                 395                 400

Lys Gly Thr Gly Phe Gly Val Arg Pro Thr Ala Asn Thr Gly His Glu
                405                 410                 415

Leu Val Asp Ala Phe Val Trp Val Lys Pro Gly Gly Glu Ser Asp Gly
            420                 425                 430

Thr Ser Ala Asp Thr Ser Ala Ala Arg Tyr Asp Tyr His Cys Gly Leu
        435                 440                 445

Ser Asp Ala Leu Thr Pro Ala Pro Glu Ala Gly Gln Trp Phe Gln Ala
    450                 455                 460

Tyr Phe Glu Gln Leu Leu Ile Asn Ala Asn Pro Pro Leu
465                 470                 475
```

<210> 111
<211> 2586
<212> DNA
<213> Aspergillus oryzae

<400> 111

atgaagcttg gttggatcga ggtggccgca ttggcggctg cctcagtagt cagtgccaag 60

gatgatctcg cgtactcccc tcctttctac ccttccccat gggcagatgg tcagggtgaa 120

tgggcggaag tatacaaacg cgctgtagac atagtttccc agatgacgtt gacagagaaa 180
gtcaacttaa cgactggaac aggatggcaa ctagagaggt gtgttggaca aactggcagt 240
gttcccagac tcaacatccc cagcttgtgt ttgcaggata gtcctcttgg tattcgtttc 300
tcggactaca attcagcttt ccctgcgggt gttaatgtcg ctgccacctg ggacaagacg 360
ctcgcctacc ttcgtggtca ggcaatgggt gaggagttca gtgataaggg tattgacgtt 420
cagctgggtc ctgctgctgg ccctctcggt gctcatccgg atggcggtag aaactgggaa 480
ggtttctcac cagatccagc cctcaccggt gtactttttg cggagacgat taagggtatt 540
caagatgctg gtgtcattgc gacagctaag cattatatca tgaacgaaca agagcatttc 600
cgccaacaac ccgaggctgc gggttacgga ttcaacgtaa gcgacagttt gagttccaac 660
gttgatgaca agactatgca tgaattgtac ctctggccct tcgcggatgc agtacgcgct 720
ggagtcggtg ctgtcatgtg ctcttacaac caaatcaaca acagctacgg ttgcgagaat 780
agcgaaactc tgaacaagct tttgaaggcg gagcttggtt tccaaggctt cgtcatgagt 840
gattggaccg ctcatcacag cggcgtaggc gctgctttag caggtctgga tatgtcgatg 900
cccggtgatg ttaccttcga tagtggtacg tctttctggg gtgcaaactt gacggtcggt 960
gtccttaacg gtacaatccc ccaatggcgt gttgatgaca tggctgtccg tatcatggcc 1020
gcttattaca aggttggccg cgacaccaaa tacacccctc ccaacttcag ctcgtggacc 1080
agggacgaat atggtttcgc gcataaccat gtttcggaag gtgcttacga gagggtcaac 1140
gaattcgtgg acgtgcaacg cgatcatgcc gacctaatcc gtcgcatcgg cgcgcagagc 1200
actgttctgc tgaagaacaa gggtgccttg cccttgagcc gcaaggaaaa gctggtcgcc 1260
cttctgggag aggatgcggg ttccaactcg tggggcgcta acggctgtga tgaccgtggt 1320
tgcgataacg gtacccttgc catggcctgg ggtagcggta ctgcgaattt cccatacctc 1380
gtgacaccag agcaggcgat tcagaacgaa gttcttcagg gccgtggtaa tgtcttcgcc 1440
gtgaccgaca gttgggcgct cgacaagatc gctgcggctg cccgccaggc cagcgtatct 1500
ctcgtgttcg tcaactccga ctcaggagaa ggctatctta gtgtggatgg aaatgagggc 1560
gatcgtaaca acatcactct gtggaagaac ggcgacaatg tggtcaagac cgcagcgaat 1620
aactgtaaca acaccgttgt catcatccac tccgtcggac cagttttgat cgatgaatgg 1680
tatgaccacc ccaatgtcac tggtattctc tgggctggtc tgccaggcca ggagtctggt 1740
aactccattg ccgatgtgct gtacggtcgt gtcaaccctg gcgccaagtc tcctttcact 1800
tggggcaaga cccgggagtc gtatggttct cccttggtca aggatgccaa caatggcaac 1860
ggagcgcccc agtctgattt cacccagggt gttttcatcg attaccgcca tttcgataag 1920
ttcaatgaga cccctatcta cgagtttggc tacggcttga gctacaccac cttcgagctc 1980
tccgacctcc atgttcagcc cctgaacgcg tcccgataca ctcccaccag tggcatgact 2040

gaagctgcaa agaactttgg tgaaattggc gatgcgtcgg agtacgtgta tccggagggg 2100

ctggaaagga tccatgagtt tatctatccc tggatcaact ctaccgacct gaaggcatcg 2160

tctgacgatt ctaactacgg ctgggaagac tccaagtata ttcccgaagg cgccacggat 2220

gggtctgccc agccccgttt gcccgctagt ggtggtgccg gaggaaaccc cggtctgtac 2280

gaggatcttt tccgcgtctc tgtgaaggtc aagaacacgg gcaatgtcgc cggtgatgaa 2340

gttcctcagc tgtacgtttc cctaggcggc ccgaatgagc ccaaggtggt actgcgcaag 2400

tttgagcgta ttcacttggc cccttcgcag gaggccgtgt ggacaacgac ccttacccgt 2460

cgtgaccttg caaactggga cgtttcggct caggactgga ccgtcactcc ttaccccaag 2520

acgatctacg ttggaaactc ctcacggaaa ctgccgctcc aggcctcgct gcctaaggcc 2580

cagtaa 2586

<210> 112
<211> 861
<212> PRT
<213> Aspergillus oryzae

<400> 112

```
Met Lys Leu Gly Trp Ile Glu Val Ala Ala Leu Ala Ala Ala Ser Val
1               5                   10                  15

Val Ser Ala Lys Asp Asp Leu Ala Tyr Ser Pro Pro Phe Tyr Pro Ser
            20                  25                  30

Pro Trp Ala Asp Gly Gln Gly Glu Trp Ala Glu Val Tyr Lys Arg Ala
        35                  40                  45

Val Asp Ile Val Ser Gln Met Thr Leu Thr Glu Lys Val Asn Leu Thr
    50                  55                  60

Thr Gly Thr Gly Trp Gln Leu Glu Arg Cys Val Gly Gln Thr Gly Ser
65                  70                  75                  80

Val Pro Arg Leu Asn Ile Pro Ser Leu Cys Leu Gln Asp Ser Pro Leu
                85                  90                  95

Gly Ile Arg Phe Ser Asp Tyr Asn Ser Ala Phe Pro Ala Gly Val Asn
            100                 105                 110

Val Ala Ala Thr Trp Asp Lys Thr Leu Ala Tyr Leu Arg Gly Gln Ala
        115                 120                 125

Met Gly Glu Glu Phe Ser Asp Lys Gly Ile Asp Val Gln Leu Gly Pro
    130                 135                 140

Ala Ala Gly Pro Leu Gly Ala His Pro Asp Gly Gly Arg Asn Trp Glu
```

145 150 155 160

Gly Phe Ser Pro Asp Pro Ala Leu Thr Gly Val Leu Phe Ala Glu Thr
165 170 175

Ile Lys Gly Ile Gln Asp Ala Gly Val Ile Ala Thr Ala Lys His Tyr
180 185 190

Ile Met Asn Glu Gln Glu His Phe Arg Gln Gln Pro Glu Ala Ala Gly
195 200 205

Tyr Gly Phe Asn Val Ser Asp Ser Leu Ser Ser Asn Val Asp Asp Lys
210 215 220

Thr Met His Glu Leu Tyr Leu Trp Pro Phe Ala Asp Ala Val Arg Ala
225 230 235 240

Gly Val Gly Ala Val Met Cys Ser Tyr Asn Gln Ile Asn Asn Ser Tyr
245 250 255

Gly Cys Glu Asn Ser Glu Thr Leu Asn Lys Leu Leu Lys Ala Glu Leu
260 265 270

Gly Phe Gln Gly Phe Val Met Ser Asp Trp Thr Ala His His Ser Gly
275 280 285

Val Gly Ala Ala Leu Ala Gly Leu Asp Met Ser Met Pro Gly Asp Val
290 295 300

Thr Phe Asp Ser Gly Thr Ser Phe Trp Gly Ala Asn Leu Thr Val Gly
305 310 315 320

Val Leu Asn Gly Thr Ile Pro Gln Trp Arg Val Asp Asp Met Ala Val
325 330 335

Arg Ile Met Ala Ala Tyr Tyr Lys Val Gly Arg Asp Thr Lys Tyr Thr
340 345 350

Pro Pro Asn Phe Ser Ser Trp Thr Arg Asp Glu Tyr Gly Phe Ala His
355 360 365

Asn His Val Ser Glu Gly Ala Tyr Glu Arg Val Asn Glu Phe Val Asp
370 375 380

Val Gln Arg Asp His Ala Asp Leu Ile Arg Arg Ile Gly Ala Gln Ser
385 390 395 400

Thr Val Leu Leu Lys Asn Lys Gly Ala Leu Pro Leu Ser Arg Lys Glu
405 410 415

Lys Leu Val Ala Leu Leu Gly Glu Asp Ala Gly Ser Asn Ser Trp Gly
420 425 430

Ala Asn Gly Cys Asp Asp Arg Gly Cys Asp Asn Gly Thr Leu Ala Met
435 440 445

Ala Trp Gly Ser Gly Thr Ala Asn Phe Pro Tyr Leu Val Thr Pro Glu
450 455 460

Gln Ala Ile Gln Asn Glu Val Leu Gln Gly Arg Gly Asn Val Phe Ala
465 470 475 480

Val Thr Asp Ser Trp Ala Leu Asp Lys Ile Ala Ala Ala Ala Arg Gln
485 490 495

Ala Ser Val Ser Leu Val Phe Val Asn Ser Asp Ser Gly Glu Gly Tyr
500 505 510

Leu Ser Val Asp Gly Asn Glu Gly Asp Arg Asn Asn Ile Thr Leu Trp
515 520 525

Lys Asn Gly Asp Asn Val Val Lys Thr Ala Ala Asn Asn Cys Asn Asn
530 535 540

Thr Val Val Ile Ile His Ser Val Gly Pro Val Leu Ile Asp Glu Trp
545 550 555 560

Tyr Asp His Pro Asn Val Thr Gly Ile Leu Trp Ala Gly Leu Pro Gly
565 570 575

Gln Glu Ser Gly Asn Ser Ile Ala Asp Val Leu Tyr Gly Arg Val Asn
580 585 590

Pro Gly Ala Lys Ser Pro Phe Thr Trp Gly Lys Thr Arg Glu Ser Tyr
595 600 605

Gly Ser Pro Leu Val Lys Asp Ala Asn Asn Gly Asn Gly Ala Pro Gln
610 615 620

Ser Asp Phe Thr Gln Gly Val Phe Ile Asp Tyr Arg His Phe Asp Lys
625 630 635 640

Phe Asn Glu Thr Pro Ile Tyr Glu Phe Gly Tyr Gly Leu Ser Tyr Thr
645 650 655

Thr Phe Glu Leu Ser Asp Leu His Val Gln Pro Leu Asn Ala Ser Arg
660 665 670

```
Tyr Thr Pro Thr Ser Gly Met Thr Glu Ala Ala Lys Asn Phe Gly Glu
        675                 680                 685

Ile Gly Asp Ala Ser Glu Tyr Val Tyr Pro Glu Gly Leu Glu Arg Ile
    690                 695                 700

His Glu Phe Ile Tyr Pro Trp Ile Asn Ser Thr Asp Leu Lys Ala Ser
705                 710                 715                 720

Ser Asp Asp Ser Asn Tyr Gly Trp Glu Asp Ser Lys Tyr Ile Pro Glu
                725                 730                 735

Gly Ala Thr Asp Gly Ser Ala Gln Pro Arg Leu Pro Ala Ser Gly Gly
            740                 745                 750

Ala Gly Gly Asn Pro Gly Leu Tyr Glu Asp Leu Phe Arg Val Ser Val
        755                 760                 765

Lys Val Lys Asn Thr Gly Asn Val Ala Gly Asp Glu Val Pro Gln Leu
    770                 775                 780

Tyr Val Ser Leu Gly Gly Pro Asn Glu Pro Lys Val Val Leu Arg Lys
785                 790                 795                 800

Phe Glu Arg Ile His Leu Ala Pro Ser Gln Glu Ala Val Trp Thr Thr
                805                 810                 815

Thr Leu Thr Arg Arg Asp Leu Ala Asn Trp Asp Val Ser Ala Gln Asp
            820                 825                 830

Trp Thr Val Thr Pro Tyr Pro Lys Thr Ile Tyr Val Gly Asn Ser Ser
        835                 840                 845

Arg Lys Leu Pro Leu Gln Ala Ser Leu Pro Lys Ala Gln
    850                 855                 860
```

<210> 113
<211> 3060
<212> DNA
<213> Aspergillus fumigatus

<400> 113

```
atgagattcg gttggctcga ggtggccgct ctgacggccg cttctgtagc caatgcccag        60
gtttgtgatg ctttcccgtc attgtttcgg atatagttga caatagtcat ggaaataatc       120
aggaattggc tttctctcca ccattctacc cttcgccttg ggctgatggc cagggagagt       180
gggcagatgc ccatcgacgc gccgtcgaga tcgtttctca gatgacactg gcggagaagg       240
ttaaccttac aacgggtact gggtgggttg cgactttttt gttgacagtg agctttcttc       300
```

actgaccatc tacacagatg ggaaatggac cgatgcgtcg gtcaaaccgg cagcgttccc 360
aggtaagctt gcaattctgc aacaacgtgc aagtgtagtt gctaaaacgc ggtggtgcag 420
acttggtatc aactggggtc tttgtggcca ggattcccct ttgggtatcc gtttctgtga 480
gctatacccg cggagtcttt cagtccttgt attatgtgct gatgattgtc tctgtatagc 540
tgacctcaac tccgccttcc ctgctggtac taatgtcgcc gcgacatggg acaagacact 600
cgcctacctt cgtggcaagg ccatgggtga ggaattcaac gacaagggcg tggacatttt 660
gctggggcct gctgctggtc ctctcggcaa atacccggac ggcggcagaa tctgggaagg 720
cttctctcct gatccggttc tcactggtgt acttttcgcc gaaactatca agggtatcca 780
agacgcgggt gtgattgcta ctgccaagca ttacattctg aatgaacagg agcatttccg 840
acaggttggc gaggcccagg gatatggtta caacatcacg gagacgatca gctccaacgt 900
ggatgacaag accatgcacg agttgtacct ttggtgagta gttgacactg caaatgagga 960
ccttgattga tttgactgac ctggaatgca ggccctttgc agatgctgtg cgcggtaaga 1020
ttttccgtag acttgacctc gcgacgaaga aatcgctgac gaaccatcgt agctggcgtt 1080
ggcgctgtca tgtgttccta caatcaaatc aacaacagct acggttgtca aaacagtcaa 1140
actctcaaca agctcctcaa ggctgagctg ggcttccaag gcttcgtcat gagtgactgg 1200
agcgctcacc acagcggtgt cggcgctgcc ctcgctgggt tggatatgtc gatgcctgga 1260
gacatttcct tcgacgacgg actctccttc tggggcacga acctaactgt cagtgttctt 1320
aacggcaccg ttccagcctg gcgtgtcgat gacatggctg ttcgtatcat gaccgcgtac 1380
tacaaggttg gtcgtgaccg tcttcgtatt ccccctaact tcagctcctg gacccgggat 1440
gagtacggct gggagcattc tgctgtctcc gagggagcct ggaccaaggt gaacgacttc 1500
gtcaatgtgc agcgcagtca ctctcagatc atccgtgaga ttggtgccgc tagtacagtg 1560
ctcttgaaga acacgggtgc tcttcctttg accggcaagg aggttaaagt gggtgttctc 1620
ggtgaagacg ctggttccaa cccgtggggt gctaacggct gccccgaccg cggctgtgat 1680
aacggcactc ttgctatggc ctggggtagt ggtactgcca acttccctta ccttgtcacc 1740
cccgagcagg ctatccagcg agaggtcatc agcaacggcg gcaatgtctt tgctgtgact 1800
gataacgggg ctctcagcca gatggcagat gttgcatctc aatccaggtg agtgcgggct 1860
cttagaaaaa gaacgttctc tgaatgaagt tttttaacca ttgcgaacag cgtgtctttg 1920
gtgtttgtca acgccgactc tggagagggt ttcatcagtg tcgacggcaa cgagggtgac 1980
cgcaaaaatc tcactctgtg gaagaacggc gaggccgtca ttgacactgt tgtcagccac 2040
tgcaacaaca cgattgtggt tattcacagt gttgggcccg tcttgatcga ccggtggtat 2100
gataacccca acgtcactgc catcatctgg gccggcttgc ccggtcagga gagtggcaac 2160
tccctggtcg acgtgctcta tggccgcgtc aaccccagcg ccaagacccc gttcacctgg 2220
ggcaagactc gggagtctta cggggctccc ttgctcaccg agcctaacaa tggcaatggt 2280

gctccccagg atgatttcaa cgagggcgtc ttcattgact accgtcactt tgacaagcgc 2340

aatgagaccc ccatttatga gtttggccat ggcttgagct acaccacctt tggttactct 2400

caccttcggg ttcaggccct caatagttcg agttcggcat atgtcccgac tagcggagag 2460

accaagcctg cgccaaccta tggtgagatc ggtagtgccg ccgactacct gtatcccgag 2520

ggtctcaaaa gaattaccaa gtttatttac ccttggctca actcgaccga cctcgaggat 2580

tcttctgacg acccgaacta cggctgggag gactcggagt acattcccga aggcgctagg 2640

gatgggtctc ctcaacccct cctgaaggct ggcggcgctc ctggtggtaa ccctaccctt 2700

tatcaggatc ttgttagggt gtcggccacc ataaccaaca ctggtaacgt cgccggttat 2760

gaagtccctc aattggtgag tgacccgcat gttccttgcg ttgcaatttg gctaactcgc 2820

ttctagtatg tttcactggg cggaccgaac gagcctcggg tcgttctgcg caagttcgac 2880

cgaatcttcc tggctcctgg ggagcaaaag gtttggacca cgactcttaa ccgtcgtgat 2940

ctcgccaatt gggatgtgga ggctcaggac tgggtcatca caaagtaccc caagaaagtg 3000

cacgtcggca gctcctcgcg taagctgcct ctgagagcgc ctctgccccg tgtctactag 3060

<210> 114
<211> 863
<212> PRT
<213> Aspergillus fumigatus

<400> 114

```
Met Arg Phe Gly Trp Leu Glu Val Ala Ala Leu Thr Ala Ala Ser Val
1               5                   10                  15

Ala Asn Ala Gln Glu Leu Ala Phe Ser Pro Pro Phe Tyr Pro Ser Pro
            20                  25                  30

Trp Ala Asp Gly Gln Gly Glu Trp Ala Asp Ala His Arg Arg Ala Val
        35                  40                  45

Glu Ile Val Ser Gln Met Thr Leu Ala Glu Lys Val Asn Leu Thr Thr
    50                  55                  60

Gly Thr Gly Trp Glu Met Asp Arg Cys Val Gly Gln Thr Gly Ser Val
65                  70                  75                  80

Pro Arg Leu Gly Ile Asn Trp Gly Leu Cys Gly Gln Asp Ser Pro Leu
                85                  90                  95

Gly Ile Arg Phe Ser Asp Leu Asn Ser Ala Phe Pro Ala Gly Thr Asn
            100                 105                 110

Val Ala Ala Thr Trp Asp Lys Thr Leu Ala Tyr Leu Arg Gly Lys Ala
        115                 120                 125
```

Met Gly Glu Glu Phe Asn Asp Lys Gly Val Asp Ile Leu Leu Gly Pro
130 135 140

Ala Ala Gly Pro Leu Gly Lys Tyr Pro Asp Gly Gly Arg Ile Trp Glu
145 150 155 160

Gly Phe Ser Pro Asp Pro Val Leu Thr Gly Val Leu Phe Ala Glu Thr
165 170 175

Ile Lys Gly Ile Gln Asp Ala Gly Val Ile Ala Thr Ala Lys His Tyr
180 185 190

Ile Leu Asn Glu Gln Glu His Phe Arg Gln Val Gly Glu Ala Gln Gly
195 200 205

Tyr Gly Tyr Asn Ile Thr Glu Thr Ile Ser Ser Asn Val Asp Asp Lys
210 215 220

Thr Met His Glu Leu Tyr Leu Trp Pro Phe Ala Asp Ala Val Arg Ala
225 230 235 240

Gly Val Gly Ala Val Met Cys Ser Tyr Asn Gln Ile Asn Asn Ser Tyr
245 250 255

Gly Cys Gln Asn Ser Gln Thr Leu Asn Lys Leu Leu Lys Ala Glu Leu
260 265 270

Gly Phe Gln Gly Phe Val Met Ser Asp Trp Ser Ala His His Ser Gly
275 280 285

Val Gly Ala Ala Leu Ala Gly Leu Asp Met Ser Met Pro Gly Asp Ile
290 295 300

Ser Phe Asp Asp Gly Leu Ser Phe Trp Gly Thr Asn Leu Thr Val Ser
305 310 315 320

Val Leu Asn Gly Thr Val Pro Ala Trp Arg Val Asp Asp Met Ala Val
325 330 335

Arg Ile Met Thr Ala Tyr Tyr Lys Val Gly Arg Asp Arg Leu Arg Ile
340 345 350

Pro Pro Asn Phe Ser Ser Trp Thr Arg Asp Glu Tyr Gly Trp Glu His
355 360 365

Ser Ala Val Ser Glu Gly Ala Trp Thr Lys Val Asn Asp Phe Val Asn
370 375 380

```
Val Gln Arg Ser His Ser Gln Ile Ile Arg Glu Ile Gly Ala Ala Ser
385                 390                 395                 400

Thr Val Leu Leu Lys Asn Thr Gly Ala Leu Pro Leu Thr Gly Lys Glu
                405                 410                 415

Val Lys Val Gly Val Leu Gly Glu Asp Ala Gly Ser Asn Pro Trp Gly
            420                 425                 430

Ala Asn Gly Cys Pro Asp Arg Gly Cys Asp Asn Gly Thr Leu Ala Met
        435                 440                 445

Ala Trp Gly Ser Gly Thr Ala Asn Phe Pro Tyr Leu Val Thr Pro Glu
    450                 455                 460

Gln Ala Ile Gln Arg Glu Val Ile Ser Asn Gly Gly Asn Val Phe Ala
465                 470                 475                 480

Val Thr Asp Asn Gly Ala Leu Ser Gln Met Ala Asp Val Ala Ser Gln
                485                 490                 495

Ser Ser Val Ser Leu Val Phe Val Asn Ala Asp Ser Gly Glu Gly Phe
            500                 505                 510

Ile Ser Val Asp Gly Asn Glu Gly Asp Arg Lys Asn Leu Thr Leu Trp
        515                 520                 525

Lys Asn Gly Glu Ala Val Ile Asp Thr Val Val Ser His Cys Asn Asn
    530                 535                 540

Thr Ile Val Val Ile His Ser Val Gly Pro Val Leu Ile Asp Arg Trp
545                 550                 555                 560

Tyr Asp Asn Pro Asn Val Thr Ala Ile Ile Trp Ala Gly Leu Pro Gly
                565                 570                 575

Gln Glu Ser Gly Asn Ser Leu Val Asp Val Leu Tyr Gly Arg Val Asn
            580                 585                 590

Pro Ser Ala Lys Thr Pro Phe Thr Trp Gly Lys Thr Arg Glu Ser Tyr
        595                 600                 605

Gly Ala Pro Leu Leu Thr Glu Pro Asn Asn Gly Asn Gly Ala Pro Gln
    610                 615                 620

Asp Asp Phe Asn Glu Gly Val Phe Ile Asp Tyr Arg His Phe Asp Lys
625                 630                 635                 640
```

```
Arg Asn Glu Thr Pro Ile Tyr Glu Phe Gly His Gly Leu Ser Tyr Thr
                645                 650                 655

Thr Phe Gly Tyr Ser His Leu Arg Val Gln Ala Leu Asn Ser Ser Ser
            660                 665                 670

Ser Ala Tyr Val Pro Thr Ser Gly Glu Thr Lys Pro Ala Pro Thr Tyr
        675                 680                 685

Gly Glu Ile Gly Ser Ala Ala Asp Tyr Leu Tyr Pro Glu Gly Leu Lys
    690                 695                 700

Arg Ile Thr Lys Phe Ile Tyr Pro Trp Leu Asn Ser Thr Asp Leu Glu
705                 710                 715                 720

Asp Ser Ser Asp Asp Pro Asn Tyr Gly Trp Glu Asp Ser Glu Tyr Ile
                725                 730                 735

Pro Glu Gly Ala Arg Asp Gly Ser Pro Gln Pro Leu Leu Lys Ala Gly
            740                 745                 750

Gly Ala Pro Gly Gly Asn Pro Thr Leu Tyr Gln Asp Leu Val Arg Val
        755                 760                 765

Ser Ala Thr Ile Thr Asn Thr Gly Asn Val Ala Gly Tyr Glu Val Pro
    770                 775                 780

Gln Leu Tyr Val Ser Leu Gly Gly Pro Asn Glu Pro Arg Val Val Leu
785                 790                 795                 800

Arg Lys Phe Asp Arg Ile Phe Leu Ala Pro Gly Glu Gln Lys Val Trp
                805                 810                 815

Thr Thr Thr Leu Asn Arg Arg Asp Leu Ala Asn Trp Asp Val Glu Ala
            820                 825                 830

Gln Asp Trp Val Ile Thr Lys Tyr Pro Lys Lys Val His Val Gly Ser
        835                 840                 845

Ser Ser Arg Lys Leu Pro Leu Arg Ala Pro Leu Pro Arg Val Tyr
    850                 855                 860
```

<210> 115
<211> 2800
<212> DNA
<213> Penicillium brasilianum

<400> 115

```
tgaaaatgca gggttctaca atctttctgg ctttcgcctc atgggcgagc caggttgctg        60
ccattgcgca gcccatacag aagcacgagg tttgttttat cttgctcatg gacgtgcttt       120
```

gacttgacta attgttttac atacagcccg gatttctgca cgggccccaa gccatagaat 180

cgttctcaga accgttctac ccgtcgccct ggatgaatcc tcacgccgag ggctgggagg 240

ccgcatatca gaaagctcaa gattttgtct cgcaactcac tatcttggag aaaataaatc 300

tgaccaccgg tgttgggtaa gtctctccga ctgcttctgg gtcacggtgc gacgagccac 360

tgactttttg aagctgggaa aatgggccgt gtgtaggaaa cactggatca attcctcgtc 420

tcggattcaa aggattttgt acccaggatt caccacaggg tgttcggttc gcagattatt 480

cctccgcttt cacatctagc caaatggccg ccgcaacatt tgaccgctca attctttatc 540

aacgaggcca agccatggca caggaacaca aggctaaggg tatcacaatt caattgggcc 600

ctgttgccgg ccctctcggt cgcatccccg agggcggccg caactgggaa ggattctccc 660

ctgatcctgt cttgactggt atagccatgg ctgagacaat taagggcatg caggatactg 720

gagtgattgc ttgcgctaaa cattatattg gaaacgagca ggagcacttc cgtcaagtgg 780

gtgaagctgc gggtcacgga tacactattt ccgatactat ttcatctaat attgacgacc 840

gtgctatgca tgagctatac ttgtggccat ttgctgatgc cgttcgcgct ggtgtgggtt 900

ctttcatgtg ctcatactct cagatcaaca actcctacgg atgccaaaac agtcagaccc 960

tcaacaagct cctcaagagc gaattgggct tccaaggctt tgtcatgagc gattggggtg 1020

cccatcactc tggagtgtca tcggcgctag ctggacttga tatgagcatg ccgggtgata 1080

ccgaatttga ttctggcttg agcttctggg gctctaacct caccattgca attctgaacg 1140

gcacggttcc cgaatggcgc ctggatgaca tggcgatgcg aattatggct gcatacttca 1200

aagttggcct tactattgag gatcaaccag atgtcaactt caatgcctgg acccatgaca 1260

cctacggata taaatacgct tatagcaagg aagattacga gcaggtcaac tggcatgtcg 1320

atgttcgcag cgaccacaat aagctcattc gcgagactgc cgcgaagggt acagttctgc 1380

tgaagaacaa ctttcatgct ctccctctga agcagcccag gttcgtggcc gtcgttggtc 1440

aggatgccgg gccaaacccc aagggcccta acggctgcgc agaccgagga tgcgaccaag 1500

gcactctcgc aatgggatgg ggctcagggt ctaccgaatt cccttacctg gtcactcctg 1560

acactgctat tcagtcaaag gtcctcgaat acgggggtcg atacgagagt atttttgata 1620

actatgacga caatgctatc ttgtcgcttg tctcacagcc tgatgcaacc tgtatcgttt 1680

ttgcaaatgc cgattccggt gaaggctaca tcactgtcga caacaactgg ggtgaccgca 1740

acaatctgac cctctggcaa aatgccgatc aagtgattag cactgtcagc tcgcgatgca 1800

acaacacaat cgttgttctc cactctgtcg gaccagtgtt gctaaatggt atatatgagc 1860

acccgaacat cacagctatt gtctgggcag ggatgccagg cgaagaatct ggcaatgctc 1920

tcgtggatat tctttggggc aatgttaacc ctgccggtcg cactccgttc acctgggcca 1980

aaagtcgaga ggactatggc actgatataa tgtacgagcc caacaacggc cagcgtgcgc 2040

ctcagcagga tttcaccgag agcatctacc tcgactaccg ccatttcgac aaagctggta 2100

tcgagccaat ttacgagttt ggattcggcc tctcctatac caccttcgaa tactctgacc 2160

tccgtgttgt gaagaagtat gttcaaccat acagtcccac gaccggcacc ggtgctcaag 2220

caccttccat cggacagcca cctagccaga acctggatac ctacaagttc cctgctacat 2280

acaagtacat caaaaccttc atttatccct acctgaacag cactgtctcc ctccgcgctg 2340

cttccaagga tcccgaatac ggtcgtacag actttatccc accccacgcg cgtgatggct 2400

cccctcaacc tctcaacccc gctggagacc cagtggccag tggtggaaac aacatgctct 2460

acgacgaact ttacgaggtc actgcacaga tcaaaaacac tggcgacgtg gccggcgacg 2520

aagtcgtcca gctttacgta gatctcgggg gtgacaaccc gcctcgtcag ttgagaaact 2580

ttgacaggtt ttatctgctg cccggtcaga gctcaacatt ccgggctaca ttgacgcgcc 2640

gtgatttgag caactgggat attgaggcgc agaactggcg agttacggaa tcgcctaaga 2700

gagtgtatgt tggacggtcg agtcgggatt tgccgctgag ctcacaattg gagtaatgat 2760

catgtctacc aatagatgtt gaatgtctgg tgtggatatt 2800

<210> 116
<211> 878
<212> PRT
<213> Penicillium brasilianum

<400> 116

```
Met Gln Gly Ser Thr Ile Phe Leu Ala Phe Ala Ser Trp Ala Ser Gln
1               5                   10                  15

Val Ala Ala Ile Ala Gln Pro Ile Gln Lys His Glu Pro Gly Phe Leu
            20                  25                  30

His Gly Pro Gln Ala Ile Glu Ser Phe Ser Glu Pro Phe Tyr Pro Ser
        35                  40                  45

Pro Trp Met Asn Pro His Ala Glu Gly Trp Glu Ala Ala Tyr Gln Lys
    50                  55                  60

Ala Gln Asp Phe Val Ser Gln Leu Thr Ile Leu Glu Lys Ile Asn Leu
65                  70                  75                  80

Thr Thr Gly Val Gly Trp Glu Asn Gly Pro Cys Val Gly Asn Thr Gly
                85                  90                  95

Ser Ile Pro Arg Leu Gly Phe Lys Gly Phe Cys Thr Gln Asp Ser Pro
            100                 105                 110

Gln Gly Val Arg Phe Ala Asp Tyr Ser Ser Ala Phe Thr Ser Ser Gln
        115                 120                 125
```

```
Met Ala Ala Ala Thr Phe Asp Arg Ser Ile Leu Tyr Gln Arg Gly Gln
    130                 135                 140

Ala Met Ala Gln Glu His Lys Ala Lys Gly Ile Thr Ile Gln Leu Gly
145                 150                 155                 160

Pro Val Ala Gly Pro Leu Gly Arg Ile Pro Glu Gly Gly Arg Asn Trp
                165                 170                 175

Glu Gly Phe Ser Pro Asp Pro Val Leu Thr Gly Ile Ala Met Ala Glu
            180                 185                 190

Thr Ile Lys Gly Met Gln Asp Thr Gly Val Ile Ala Cys Ala Lys His
        195                 200                 205

Tyr Ile Gly Asn Glu Gln Glu His Phe Arg Gln Val Gly Glu Ala Ala
    210                 215                 220

Gly His Gly Tyr Thr Ile Ser Asp Thr Ile Ser Ser Asn Ile Asp Asp
225                 230                 235                 240

Arg Ala Met His Glu Leu Tyr Leu Trp Pro Phe Ala Asp Ala Val Arg
                245                 250                 255

Ala Gly Val Gly Ser Phe Met Cys Ser Tyr Ser Gln Ile Asn Asn Ser
            260                 265                 270

Tyr Gly Cys Gln Asn Ser Gln Thr Leu Asn Lys Leu Leu Lys Ser Glu
        275                 280                 285

Leu Gly Phe Gln Gly Phe Val Met Ser Asp Trp Gly Ala His His Ser
    290                 295                 300

Gly Val Ser Ser Ala Leu Ala Gly Leu Asp Met Ser Met Pro Gly Asp
305                 310                 315                 320

Thr Glu Phe Asp Ser Gly Leu Ser Phe Trp Gly Ser Asn Leu Thr Ile
                325                 330                 335

Ala Ile Leu Asn Gly Thr Val Pro Glu Trp Arg Leu Asp Asp Met Ala
            340                 345                 350

Met Arg Ile Met Ala Ala Tyr Phe Lys Val Gly Leu Thr Ile Glu Asp
        355                 360                 365

Gln Pro Asp Val Asn Phe Asn Ala Trp Thr His Asp Thr Tyr Gly Tyr
    370                 375                 380
```

Lys Tyr Ala Tyr Ser Lys Glu Asp Tyr Glu Gln Val Asn Trp His Val
385 390 395 400

Asp Val Arg Ser Asp His Asn Lys Leu Ile Arg Glu Thr Ala Ala Lys
405 410 415

Gly Thr Val Leu Leu Lys Asn Asn Phe His Ala Leu Pro Leu Lys Gln
420 425 430

Pro Arg Phe Val Ala Val Val Gly Gln Asp Ala Gly Pro Asn Pro Lys
435 440 445

Gly Pro Asn Gly Cys Ala Asp Arg Gly Cys Asp Gln Gly Thr Leu Ala
450 455 460

Met Gly Trp Gly Ser Gly Ser Thr Glu Phe Pro Tyr Leu Val Thr Pro
465 470 475 480

Asp Thr Ala Ile Gln Ser Lys Val Leu Glu Tyr Gly Gly Arg Tyr Glu
485 490 495

Ser Ile Phe Asp Asn Tyr Asp Asp Asn Ala Ile Leu Ser Leu Val Ser
500 505 510

Gln Pro Asp Ala Thr Cys Ile Val Phe Ala Asn Ala Asp Ser Gly Glu
515 520 525

Gly Tyr Ile Thr Val Asp Asn Asn Trp Gly Asp Arg Asn Asn Leu Thr
530 535 540

Leu Trp Gln Asn Ala Asp Gln Val Ile Ser Thr Val Ser Ser Arg Cys
545 550 555 560

Asn Asn Thr Ile Val Val Leu His Ser Val Gly Pro Val Leu Leu Asn
565 570 575

Gly Ile Tyr Glu His Pro Asn Ile Thr Ala Ile Val Trp Ala Gly Met
580 585 590

Pro Gly Glu Glu Ser Gly Asn Ala Leu Val Asp Ile Leu Trp Gly Asn
595 600 605

Val Asn Pro Ala Gly Arg Thr Pro Phe Thr Trp Ala Lys Ser Arg Glu
610 615 620

Asp Tyr Gly Thr Asp Ile Met Tyr Glu Pro Asn Asn Gly Gln Arg Ala
625 630 635 640

Pro Gln Gln Asp Phe Thr Glu Ser Ile Tyr Leu Asp Tyr Arg His Phe

```
                645                 650                 655

Asp Lys Ala Gly Ile Glu Pro Ile Tyr Glu Phe Gly Phe Gly Leu Ser
            660                 665                 670

Tyr Thr Thr Phe Glu Tyr Ser Asp Leu Arg Val Val Lys Lys Tyr Val
        675                 680                 685

Gln Pro Tyr Ser Pro Thr Thr Gly Thr Gly Ala Gln Ala Pro Ser Ile
    690                 695                 700

Gly Gln Pro Pro Ser Gln Asn Leu Asp Thr Tyr Lys Phe Pro Ala Thr
705                 710                 715                 720

Tyr Lys Tyr Ile Lys Thr Phe Ile Tyr Pro Tyr Leu Asn Ser Thr Val
                725                 730                 735

Ser Leu Arg Ala Ala Ser Lys Asp Pro Glu Tyr Gly Arg Thr Asp Phe
            740                 745                 750

Ile Pro Pro His Ala Arg Asp Gly Ser Pro Gln Pro Leu Asn Pro Ala
        755                 760                 765

Gly Asp Pro Val Ala Ser Gly Gly Asn Asn Met Leu Tyr Asp Glu Leu
    770                 775                 780

Tyr Glu Val Thr Ala Gln Ile Lys Asn Thr Gly Asp Val Ala Gly Asp
785                 790                 795                 800

Glu Val Val Gln Leu Tyr Val Asp Leu Gly Gly Asp Asn Pro Pro Arg
                805                 810                 815

Gln Leu Arg Asn Phe Asp Arg Phe Tyr Leu Leu Pro Gly Gln Ser Ser
            820                 825                 830

Thr Phe Arg Ala Thr Leu Thr Arg Arg Asp Leu Ser Asn Trp Asp Ile
        835                 840                 845

Glu Ala Gln Asn Trp Arg Val Thr Glu Ser Pro Lys Arg Val Tyr Val
    850                 855                 860

Gly Arg Ser Ser Arg Asp Leu Pro Leu Ser Ser Gln Leu Glu
865                 870                 875
```

<210> 117
<211> 2583
<212> DNA

<213> Aspergillus niger

<400> 117

atgaggttca ctttgatcga ggcggtggct ctgactgccg tctcgctggc cagcgctgat 60
gaattggcct actccccacc gtattaccca tccccttggg ccaatggcca gggcgactgg 120
gcgcaggcat accagcgcgc tgttgatatt gtctcgcaaa tgacattgga tgagaaggtc 180
aatctgacca caggaactgg atgggaattg gaactatgtg ttggtcagac tggcggtgtt 240
ccccgattgg gagttccggg aatgtgttta caggatagcc ctctgggcgt tcgcgactcc 300
gactacaact ctgctttccc tgccggcatg aacgtggctg caacctggga caagaatctg 360
gcataccttc gcggcaaggc tatgggtcag gaatttagtg acaagggtgc cgatatccaa 420
ttgggtccag ctgccggccc tctcggtaga agtcccgacg gtggtcgtaa ctgggagggc 480
ttctccccag accctgccct aagtggtgtg ctctttgccg agaccatcaa gggtatccaa 540
gatgctggtg tggttgcgac ggctaagcac tacattgctt acgagcaaga gcatttccgt 600
caggcgcctg aagcccaagg ttttggattt aatatttccg agagtggaag tgcgaacctc 660
gatgataaga ctatgcacga gctgtacctc tggcccttcg cggatgccat ccgtgcaggt 720
gctggcgctg tgatgtgctc ctacaaccag atcaacaaca gttatggctg ccagaacagc 780
tacactctga acaagctgct caaggccgag ctgggcttcc agggctttgt catgagtgat 840
tgggctgctc accatgctgg tgtgagtggt gctttggcag gattggatat gtctatgcca 900
ggagacgtcg actacgacag tggtacgtct tactggggta caaacttgac cattagcgtg 960
ctcaacggaa cggtgcccca atggcgtgtt gatgacatgg ctgtccgcat catggccgcc 1020
tactacaagg tcggccgtga ccgtctgtgg actcctccca acttcagctc atggaccaga 1080
gatgaatacg gctacaagta ctactacgtg tcggagggac cgtacgagaa ggtcaaccag 1140
tacgtgaatg tgcaacgcaa ccacagcgaa ctgattcgcc gcattggagc ggacagcacg 1200
gtgctcctca agaacgacgg cgctctgcct ttgactggta aggagcgcct ggtcgcgctt 1260
atcggagaag atgcgggctc caacccttat ggtgccaacg gctgcagtga ccgtggatgc 1320
gacaatggaa cattggcgat gggctgggga agtggtactg ccaacttccc atacctggtg 1380
acccccgagc aggccatctc aaacgaggtg cttaagcaca agaatggtgt attcaccgcc 1440
accgataact gggctatcga tcagattgag gcgcttgcta agaccgccag tgtctctctt 1500
gtctttgtca acgccgactc tggtgagggt tacatcaatg tggacggaaa cctgggtgac 1560
cgcaggaacc tgaccctgtg gaggaacggc gataatgtga tcaaggctgc tgctagcaac 1620
tgcaacaaca caatcgttgt cattcactct gtcggaccag tcttggttaa cgagtggtac 1680
gacaacccca atgttaccgc tatcctctgg ggtggtttgc ccggtcagga gtctggcaac 1740
tctcttgccg acgtcctcta tggccgtgtc aaccccggtg ccaagtcgcc ctttacctgg 1800
ggcaagactc gtgaggccta ccaagactac ttggtcaccg agcccaacaa cggcaacgga 1860
gcccctcagg aagactttgt cgagggcgtc ttcattgact accgtggatt tgacaagcgc 1920
aacgagaccc cgatctacga gttcggctat ggtctgagct acaccacttt caactactcg 1980

aaccttgagg tgcaggtgct gagcgcccct gcatacgagc ctgcttcggg tgagaccgag 2040

gcagcgccaa ccttcggaga ggttggaaat gcgtcggatt acctctaccc cagcggattg 2100

cagagaatta ccaagttcat ctacccctgg ctcaacggta ccgatctcga ggcatcttcc 2160

ggggatgcta gctacgggca ggactcctcc gactatcttc ccgagggagc caccgatggc 2220

tctgcgcaac cgatcctgcc tgccggtggc ggtcctggcg gcaaccctcg cctgtacgac 2280

gagctcatcc gcgtgtcagt gaccatcaag aacaccggca aggttgctgg tgatgaagtt 2340

ccccaactgt atgtttccct tggcggtccc aatgagccca agatcgtgct gcgtcaattc 2400

gagcgcatca cgctgcagcc gtcggaggag acgaagtgga gcacgactct gacgcgccgt 2460

gaccttgcaa actggaatgt tgagaagcag gactgggaga ttacgtcgta tcccaagatg 2520

gtgtttgtcg gaagctcctc gcggaagctg ccgctccggg cgtctctgcc tactgttcac 2580

taa 2583

<210> 118
<211> 860
<212> PRT
<213> Aspergillus niger

<400> 118

```
Met Arg Phe Thr Leu Ile Glu Ala Val Ala Leu Thr Ala Val Ser Leu
1               5                   10                  15

Ala Ser Ala Asp Glu Leu Ala Tyr Ser Pro Pro Tyr Tyr Pro Ser Pro
            20                  25                  30

Trp Ala Asn Gly Gln Gly Asp Trp Ala Gln Ala Tyr Gln Arg Ala Val
        35                  40                  45

Asp Ile Val Ser Gln Met Thr Leu Asp Glu Lys Val Asn Leu Thr Thr
    50                  55                  60

Gly Thr Gly Trp Glu Leu Glu Leu Cys Val Gly Gln Thr Gly Gly Val
65                  70                  75                  80

Pro Arg Leu Gly Val Pro Gly Met Cys Leu Gln Asp Ser Pro Leu Gly
                85                  90                  95

Val Arg Asp Ser Asp Tyr Asn Ser Ala Phe Pro Ala Gly Met Asn Val
            100                 105                 110

Ala Ala Thr Trp Asp Lys Asn Leu Ala Tyr Leu Arg Gly Lys Ala Met
        115                 120                 125

Gly Gln Glu Phe Ser Asp Lys Gly Ala Asp Ile Gln Leu Gly Pro Ala
    130                 135                 140
```

Ala Gly Pro Leu Gly Arg Ser Pro Asp Gly Gly Arg Asn Trp Glu Gly
145 150 155 160

Phe Ser Pro Asp Pro Ala Leu Ser Gly Val Leu Phe Ala Glu Thr Ile
165 170 175

Lys Gly Ile Gln Asp Ala Gly Val Val Ala Thr Ala Lys His Tyr Ile
180 185 190

Ala Tyr Glu Gln Glu His Phe Arg Gln Ala Pro Glu Ala Gln Gly Phe
195 200 205

Gly Phe Asn Ile Ser Glu Ser Gly Ser Ala Asn Leu Asp Asp Lys Thr
210 215 220

Met His Glu Leu Tyr Leu Trp Pro Phe Ala Asp Ala Ile Arg Ala Gly
225 230 235 240

Ala Gly Ala Val Met Cys Ser Tyr Asn Gln Ile Asn Asn Ser Tyr Gly
245 250 255

Cys Gln Asn Ser Tyr Thr Leu Asn Lys Leu Leu Lys Ala Glu Leu Gly
260 265 270

Phe Gln Gly Phe Val Met Ser Asp Trp Ala Ala His His Ala Gly Val
275 280 285

Ser Gly Ala Leu Ala Gly Leu Asp Met Ser Met Pro Gly Asp Val Asp
290 295 300

Tyr Asp Ser Gly Thr Ser Tyr Trp Gly Thr Asn Leu Thr Ile Ser Val
305 310 315 320

Leu Asn Gly Thr Val Pro Gln Trp Arg Val Asp Asp Met Ala Val Arg
325 330 335

Ile Met Ala Ala Tyr Tyr Lys Val Gly Arg Asp Arg Leu Trp Thr Pro
340 345 350

Pro Asn Phe Ser Ser Trp Thr Arg Asp Glu Tyr Gly Tyr Lys Tyr Tyr
355 360 365

Tyr Val Ser Glu Gly Pro Tyr Glu Lys Val Asn Gln Tyr Val Asn Val
370 375 380

Gln Arg Asn His Ser Glu Leu Ile Arg Arg Ile Gly Ala Asp Ser Thr
385 390 395 400

```
Val Leu Leu Lys Asn Asp Gly Ala Leu Pro Leu Thr Gly Lys Glu Arg
                405                 410                 415

Leu Val Ala Leu Ile Gly Glu Asp Ala Gly Ser Asn Pro Tyr Gly Ala
            420                 425                 430

Asn Gly Cys Ser Asp Arg Gly Cys Asp Asn Gly Thr Leu Ala Met Gly
        435                 440                 445

Trp Gly Ser Gly Thr Ala Asn Phe Pro Tyr Leu Val Thr Pro Glu Gln
    450                 455                 460

Ala Ile Ser Asn Glu Val Leu Lys His Lys Asn Gly Val Phe Thr Ala
465                 470                 475                 480

Thr Asp Asn Trp Ala Ile Asp Gln Ile Glu Ala Leu Ala Lys Thr Ala
                485                 490                 495

Ser Val Ser Leu Val Phe Val Asn Ala Asp Ser Gly Glu Gly Tyr Ile
            500                 505                 510

Asn Val Asp Gly Asn Leu Gly Asp Arg Arg Asn Leu Thr Leu Trp Arg
        515                 520                 525

Asn Gly Asp Asn Val Ile Lys Ala Ala Ala Ser Asn Cys Asn Asn Thr
    530                 535                 540

Ile Val Val Ile His Ser Val Gly Pro Val Leu Val Asn Glu Trp Tyr
545                 550                 555                 560

Asp Asn Pro Asn Val Thr Ala Ile Leu Trp Gly Gly Leu Pro Gly Gln
                565                 570                 575

Glu Ser Gly Asn Ser Leu Ala Asp Val Leu Tyr Gly Arg Val Asn Pro
            580                 585                 590

Gly Ala Lys Ser Pro Phe Thr Trp Gly Lys Thr Arg Glu Ala Tyr Gln
        595                 600                 605

Asp Tyr Leu Val Thr Glu Pro Asn Asn Gly Asn Gly Ala Pro Gln Glu
    610                 615                 620

Asp Phe Val Glu Gly Val Phe Ile Asp Tyr Arg Gly Phe Asp Lys Arg
625                 630                 635                 640

Asn Glu Thr Pro Ile Tyr Glu Phe Gly Tyr Gly Leu Ser Tyr Thr Thr
                645                 650                 655
```

Phe Asn Tyr Ser Asn Leu Glu Val Gln Val Leu Ser Ala Pro Ala Tyr
660 665 670

Glu Pro Ala Ser Gly Glu Thr Glu Ala Ala Pro Thr Phe Gly Glu Val
675 680 685

Gly Asn Ala Ser Asp Tyr Leu Tyr Pro Ser Gly Leu Gln Arg Ile Thr
690 695 700

Lys Phe Ile Tyr Pro Trp Leu Asn Gly Thr Asp Leu Glu Ala Ser Ser
705 710 715 720

Gly Asp Ala Ser Tyr Gly Gln Asp Ser Ser Asp Tyr Leu Pro Glu Gly
725 730 735

Ala Thr Asp Gly Ser Ala Gln Pro Ile Leu Pro Ala Gly Gly Gly Pro
740 745 750

Gly Gly Asn Pro Arg Leu Tyr Asp Glu Leu Ile Arg Val Ser Val Thr
755 760 765

Ile Lys Asn Thr Gly Lys Val Ala Gly Asp Glu Val Pro Gln Leu Tyr
770 775 780

Val Ser Leu Gly Gly Pro Asn Glu Pro Lys Ile Val Leu Arg Gln Phe
785 790 795 800

Glu Arg Ile Thr Leu Gln Pro Ser Glu Glu Thr Lys Trp Ser Thr Thr
805 810 815

Leu Thr Arg Arg Asp Leu Ala Asn Trp Asn Val Glu Lys Gln Asp Trp
820 825 830

Glu Ile Thr Ser Tyr Pro Lys Met Val Phe Val Gly Ser Ser Ser Arg
835 840 845

Lys Leu Pro Leu Arg Ala Ser Leu Pro Thr Val His
850 855 860

<210> 119
<211> 2583
<212> DNA
<213> Aspergillus aculeatus

<400> 119

```
atgaagctca gttggcttga ggcggctgcc ttgacggctg cttcagtcgt cagcgctgat       60
gaactggcgt tctctcctcc tttctacccc tctccgtggg ccaatggcca gggagagtgg      120
gcggaagcct accagcgtgc agtggccatt gtatcccaga tgactctgga tgagaaggtc      180
aacctgacca ccggaactgg atgggagctg gagaagtgcg tcggtcagac tggtggtgtc      240
```

ccaagactga acatcggtgg catgtgtctt caggacagtc ccttgggaat tcgtgatagt 300
gactacaatt cggctttccc tgctggtgtc aacgttgctg cgacatggga caagaacctt 360
gcttatctac gtggtcaggc tatgggtcaa gagttcagtg acaaaggaat tgatgttcaa 420
ttgggaccgg ccgcgggtcc cctcggcagg agccctgatg gaggtcgcaa ctgggaaggt 480
ttctctccag acccggctct tactggtgtg ctctttgcgg agacgattaa gggtattcaa 540
gacgctggtg tcgtggcgac agccaagcat tacattctca atgagcaaga gcatttccgc 600
caggtcgcag aggctgcggg ctacggattc aatatctccg acacgatcag ctctaacgtt 660
gatgacaaga ccattcatga aatgtacctc tggcccttcg cggatgccgt tcgcgccggc 720
gttggcgcca tcatgtgttc ctacaaccag atcaacaaca gctacggttg ccagaacagt 780
tacactctga acaagcttct gaaggccgag ctcggcttcc agggctttgt gatgtctgac 840
tggggtgctc accacagtgg tgttggctct gctttggccg gcttggatat gtcaatgcct 900
ggcgatatca ccttcgattc tgccactagt ttctggggta ccaacctgac cattgctgtg 960
ctcaacggta ccgtcccgca gtggcgcgtt gacgacatgg ctgtccgtat catggctgcc 1020
tactacaagg ttggccgcga ccgcctgtac cagccgccta acttcagctc ctggactcgc 1080
gatgaatacg gcttcaagta tttctacccc caggaagggc cctatgagaa ggtcaatcac 1140
tttgtcaatg tgcagcgcaa ccacagcgag gttattcgca agttgggagc agacagtact 1200
gttctactga agaacaacaa tgccctgccg ctgaccggaa aggagcgcaa agttgcgatc 1260
ctgggtgaag atgctggatc caactcgtac ggtgccaatg gctgctctga ccgtggctgt 1320
gacaacggta ctcttgctat ggcttggggt agcggcactg ccgaattccc atatctcgtg 1380
acccctgagc aggctattca agccgaggtg ctcaagcata agggcagcgt ctacgccatc 1440
acggacaact gggcgctgag ccaggtggag accctcgcta aacaagccag tgtctctctt 1500
gtatttgtca actcggacgc gggagagggc tatatctccg tggacggaaa cgagggcgac 1560
cgcaacaacc tcaccctctg gaagaacggc gacaacctca tcaaggctgc tgcaaacaac 1620
tgcaacaaca ccatcgttgt catccactcc gttggacctg ttttggttga cgagtggtat 1680
gaccacccca acgttactgc catcctctgg gcgggcttgc ctggccagga gtctggcaac 1740
tccttggctg acgtgctcta cggccgcgtc aacccgggcg ccaaatctcc attcacctgg 1800
ggcaagacga gggaggcgta cggggattac cttgtccgtg agctcaacaa cggcaacgga 1860
gctccccaag atgatttctc ggaaggtgtt ttcattgact accgcggatt cgacaagcgc 1920
aatgagaccc cgatctacga gttcggacat ggtctgagct acaccacttt caactactct 1980
ggccttcaca tccaggttct caacgcttcc tccaacgctc aagtagccac tgagactggc 2040
gccgctccca ccttcggaca agtcggcaat gcctctgact acgtgtaccc tgagggattg 2100
accagaatca gcaagttcat ctatccctgg cttaattcca cagacctgaa ggcctcatct 2160

ggcgacccgt actatggagt cgacaccgcg gagcacgtgc ccgagggtgc tactgatggc 2220

tctccgcagc ccgttctgcc tgccggtggt ggctctggtg gtaacccgcg cctctacgat 2280

gagttgatcc gtgtttcggt gacagtcaag aacactggtc gtgttgccgg tgatgctgtg 2340

cctcaattgt atgtttccct tggtggaccc aatgagccca aggttgtgtt gcgcaaattc 2400

gaccgcctca ccctcaagcc ctccgaggag acggtgtgga cgactaccct gacccgccgc 2460

gatctgtcta actgggacgt tgcggctcag gactgggtca tcacttctta cccgaagaag 2520

gtccatgttg gtagctcttc gcgtcagctg ccccttcacg cggcgctccc gaaggtgcaa 2580

tga 2583

<210> 120
<211> 860
<212> PRT
<213> Aspergillus aculeatus

<400> 120

Met Lys Leu Ser Trp Leu Glu Ala Ala Ala Leu Thr Ala Ala Ser Val
1               5                   10                  15

Val Ser Ala Asp Glu Leu Ala Phe Ser Pro Pro Phe Tyr Pro Ser Pro
            20                  25                  30

Trp Ala Asn Gly Gln Gly Glu Trp Ala Glu Ala Tyr Gln Arg Ala Val
        35                  40                  45

Ala Ile Val Ser Gln Met Thr Leu Asp Glu Lys Val Asn Leu Thr Thr
    50                  55                  60

Gly Thr Gly Trp Glu Leu Glu Lys Cys Val Gly Gln Thr Gly Gly Val
65                  70                  75                  80

Pro Arg Leu Asn Ile Gly Gly Met Cys Leu Gln Asp Ser Pro Leu Gly
                85                  90                  95

Ile Arg Asp Ser Asp Tyr Asn Ser Ala Phe Pro Ala Gly Val Asn Val
            100                 105                 110

Ala Ala Thr Trp Asp Lys Asn Leu Ala Tyr Leu Arg Gly Gln Ala Met
        115                 120                 125

Gly Gln Glu Phe Ser Asp Lys Gly Ile Asp Val Gln Leu Gly Pro Ala
    130                 135                 140

Ala Gly Pro Leu Gly Arg Ser Pro Asp Gly Gly Arg Asn Trp Glu Gly
145                 150                 155                 160

Phe Ser Pro Asp Pro Ala Leu Thr Gly Val Leu Phe Ala Glu Thr Ile

165 170 175

Lys Gly Ile Gln Asp Ala Gly Val Val Ala Thr Ala Lys His Tyr Ile
180 185 190

Leu Asn Glu Gln Glu His Phe Arg Gln Val Ala Glu Ala Ala Gly Tyr
195 200 205

Gly Phe Asn Ile Ser Asp Thr Ile Ser Ser Asn Val Asp Asp Lys Thr
210 215 220

Ile His Glu Met Tyr Leu Trp Pro Phe Ala Asp Ala Val Arg Ala Gly
225 230 235 240

Val Gly Ala Ile Met Cys Ser Tyr Asn Gln Ile Asn Asn Ser Tyr Gly
245 250 255

Cys Gln Asn Ser Tyr Thr Leu Asn Lys Leu Leu Lys Ala Glu Leu Gly
260 265 270

Phe Gln Gly Phe Val Met Ser Asp Trp Gly Ala His His Ser Gly Val
275 280 285

Gly Ser Ala Leu Ala Gly Leu Asp Met Ser Met Pro Gly Asp Ile Thr
290 295 300

Phe Asp Ser Ala Thr Ser Phe Trp Gly Thr Asn Leu Thr Ile Ala Val
305 310 315 320

Leu Asn Gly Thr Val Pro Gln Trp Arg Val Asp Asp Met Ala Val Arg
325 330 335

Ile Met Ala Ala Tyr Tyr Lys Val Gly Arg Asp Arg Leu Tyr Gln Pro
340 345 350

Pro Asn Phe Ser Ser Trp Thr Arg Asp Glu Tyr Gly Phe Lys Tyr Phe
355 360 365

Tyr Pro Gln Glu Gly Pro Tyr Glu Lys Val Asn His Phe Val Asn Val
370 375 380

Gln Arg Asn His Ser Glu Val Ile Arg Lys Leu Gly Ala Asp Ser Thr
385 390 395 400

Val Leu Leu Lys Asn Asn Asn Ala Leu Pro Leu Thr Gly Lys Glu Arg
405 410 415

Lys Val Ala Ile Leu Gly Glu Asp Ala Gly Ser Asn Ser Tyr Gly Ala
420 425 430

```
Asn Gly Cys Ser Asp Arg Gly Cys Asp Asn Gly Thr Leu Ala Met Ala
        435                 440                 445

Trp Gly Ser Gly Thr Ala Glu Phe Pro Tyr Leu Val Thr Pro Glu Gln
    450                 455                 460

Ala Ile Gln Ala Glu Val Leu Lys His Lys Gly Ser Val Tyr Ala Ile
465                 470                 475                 480

Thr Asp Asn Trp Ala Leu Ser Gln Val Glu Thr Leu Ala Lys Gln Ala
                485                 490                 495

Ser Val Ser Leu Val Phe Val Asn Ser Asp Ala Gly Glu Gly Tyr Ile
            500                 505                 510

Ser Val Asp Gly Asn Glu Gly Asp Arg Asn Asn Leu Thr Leu Trp Lys
        515                 520                 525

Asn Gly Asp Asn Leu Ile Lys Ala Ala Ala Asn Asn Cys Asn Asn Thr
    530                 535                 540

Ile Val Val Ile His Ser Val Gly Pro Val Leu Val Asp Glu Trp Tyr
545                 550                 555                 560

Asp His Pro Asn Val Thr Ala Ile Leu Trp Ala Gly Leu Pro Gly Gln
                565                 570                 575

Glu Ser Gly Asn Ser Leu Ala Asp Val Leu Tyr Gly Arg Val Asn Pro
            580                 585                 590

Gly Ala Lys Ser Pro Phe Thr Trp Gly Lys Thr Arg Glu Ala Tyr Gly
        595                 600                 605

Asp Tyr Leu Val Arg Glu Leu Asn Asn Gly Asn Gly Ala Pro Gln Asp
    610                 615                 620

Asp Phe Ser Glu Gly Val Phe Ile Asp Tyr Arg Gly Phe Asp Lys Arg
625                 630                 635                 640

Asn Glu Thr Pro Ile Tyr Glu Phe Gly His Gly Leu Ser Tyr Thr Thr
                645                 650                 655

Phe Asn Tyr Ser Gly Leu His Ile Gln Val Leu Asn Ala Ser Ser Asn
            660                 665                 670

Ala Gln Val Ala Thr Glu Thr Gly Ala Ala Pro Thr Phe Gly Gln Val
        675                 680                 685
```

```
Gly Asn Ala Ser Asp Tyr Val Tyr Pro Glu Gly Leu Thr Arg Ile Ser
    690                 695                 700

Lys Phe Ile Tyr Pro Trp Leu Asn Ser Thr Asp Leu Lys Ala Ser Ser
705                 710                 715                 720

Gly Asp Pro Tyr Tyr Gly Val Asp Thr Ala Glu His Val Pro Glu Gly
                725                 730                 735

Ala Thr Asp Gly Ser Pro Gln Pro Val Leu Pro Ala Gly Gly Gly Ser
            740                 745                 750

Gly Gly Asn Pro Arg Leu Tyr Asp Glu Leu Ile Arg Val Ser Val Thr
        755                 760                 765

Val Lys Asn Thr Gly Arg Val Ala Gly Asp Ala Val Pro Gln Leu Tyr
    770                 775                 780

Val Ser Leu Gly Gly Pro Asn Glu Pro Lys Val Val Leu Arg Lys Phe
785                 790                 795                 800

Asp Arg Leu Thr Leu Lys Pro Ser Glu Glu Thr Val Trp Thr Thr Thr
                805                 810                 815

Leu Thr Arg Arg Asp Leu Ser Asn Trp Asp Val Ala Ala Gln Asp Trp
            820                 825                 830

Val Ile Thr Ser Tyr Pro Lys Lys Val His Val Gly Ser Ser Ser Arg
        835                 840                 845

Gln Leu Pro Leu His Ala Ala Leu Pro Lys Val Gln
    850                 855                 860
```

<210> 121
<211> 3294
<212> DNA
<213> Aspergillus oryzae

<400> 121

atgcgttcct ccccccctcct ccgctccgcc gttgtggccg ccctgccggt gttggccctt 60
gccgctgatg gcaggtccac ccgctactgg gactgctgca agccttcgtg cggctgggcc 120
aagaaggctc ccgtgaacca gcctgtcttt tcctgcaacg ccaacttcca gcgtatcacg 180
gacttcgacg ccaagtccgg ctgcgagccg ggcggtgtcg cctactcgtg cgccgaccag 240
accccatggg ctgtgaacga cgacttcgcg ctcggttttg ctgccacctc tattgccggc 300
agcaatgagg cgggctggtg ctgcgcctgc tacgagctca ccttcacatc cggtcctgtt 360
gctggcaaga agatggtcgt ccagtccacc agcactggcg gtgatcttgg cagcaaccac 420

```
ttcgatctca acatccccgg cggcggcgtc ggcatcttcg acggatgcac tccccagttc      480
ggtggtctgc ccggccagcg ctacggcggc atctcgtccc gcaacgagtg cgatcggttc      540
cccgacgccc tcaagcccgg ctgctactgg cgcttcgact ggttcaagaa cgccgacaat      600
ccgagcttca gcttccgtca ggtccagtgc ccagccgagc tcgtcgctcg caccggatgc      660
cgccgcaacg acgacggcaa cttccctgcc gtccagatcc ccatgcgttc ctccccctc       720
ctccgctccg ccgttgtggc cgccctgccg gtgttggccc ttgccaagga tgatctcgcg      780
tactcccctc ctttctaccc ttccccatgg gcagatggtc agggtgaatg ggcggaagta      840
tacaaacgcg ctgtagacat agtttcccag atgacgttga cagagaaagt caacttaacg      900
actggaacag gatggcaact agagaggtgt gttggacaaa ctggcagtgt tcccagactc      960
aacatcccca gcttgtgttt gcaggatagt cctcttggta ttcgtttctc ggactacaat     1020
tcagctttcc ctgcgggtgt taatgtcgct gccacctggg acaagacgct cgcctacctt     1080
cgtggtcagg caatgggtga ggagttcagt gataagggta ttgacgttca gctgggtcct     1140
gctgctggcc ctctcggtgc tcatccggat ggcggtagaa actgggaagg tttctcacca     1200
gatccagccc tcaccggtgt actttttgcg gagacgatta agggtattca agatgctggt     1260
gtcattgcga cagctaagca ttatatcatg aacgaacaag agcatttccg ccaacaaccc     1320
gaggctgcgg gttacggatt caacgtaagc gacagtttga gttccaacgt tgatgacaag     1380
actatgcatg aattgtacct ctggcccttc gcggatgcag tacgcgctgg agtcggtgct     1440
gtcatgtgct cttacaacca aatcaacaac agctacggtt gcgagaatag cgaaactctg     1500
aacaagcttt tgaaggcgga gcttggtttc caaggcttcg tcatgagtga ttggaccgct     1560
catcacagcg gcgtaggcgc tgctttagca ggtctggata tgtcgatgcc cggtgatgtt     1620
accttcgata gtggtacgtc tttctggggt gcaaacttga cggtcggtgt ccttaacggt     1680
acaatccccc aatggcgtgt tgatgacatg gctgtccgta tcatggccgc ttattacaag     1740
gttggccgcg acaccaaata cacccctccc aacttcagct cgtggaccag ggacgaatat     1800
ggtttcgcgc ataaccatgt ttcggaaggt gcttacgaga gggtcaacga attcgtggac     1860
gtgcaacgcg atcatgccga cctaatccgt cgcatcggcg cgcagagcac tgttctgctg     1920
aagaacaagg gtgccttgcc cttgagccgc aaggaaaagc tggtcgccct tctgggagag     1980
gatgcgggtt ccaactcgtg ggcgctaac ggctgtgatg accgtggttg cgataacggt       2040
acccttgcca tggcctgggg tagcggtact gcgaatttcc catacctcgt gacaccagag     2100
caggcgattc agaacgaagt tcttcagggc cgtggtaatg tcttcgccgt gaccgacagt     2160
tgggcgctcg acaagatcgc tgcggctgcc cgccaggcca gcgtatctct cgtgttcgtc     2220
aactccgact caggagaagg ctatcttagt gtggatggaa atgagggcga tcgtaacaac     2280
atcactctgt ggaagaacgg cgacaatgtg gtcaagaccg cagcgaataa ctgtaacaac     2340
accgttgtca tcatccactc cgtcggacca gttttgatcg atgaatggta tgaccacccc     2400
```

aatgtcactg gtattctctg ggctggtctg ccaggccagg agtctggtaa ctccattgcc 2460
gatgtgctgt acggtcgtgt caaccctggc gccaagtctc ctttcacttg gggcaagacc 2520
cgggagtcgt atggttctcc cttggtcaag gatgccaaca atggcaacgg agcgccccag 2580
tctgatttca cccagggtgt tttcatcgat taccgccatt tcgataagtt caatgagacc 2640
cctatctacg agtttggcta cggcttgagc tacaccacct tcgagctctc cgacctccat 2700
gttcagcccc tgaacgcgtc ccgatacact cccaccagtg gcatgactga agctgcaaag 2760
aactttggtg aaattggcga tgcgtcggag tacgtgtatc cggaggggct ggaaaggatc 2820
catgagttta tctatccctg gatcaactct accgacctga aggcatcgtc tgacgattct 2880
aactacggct gggaagactc caagtatatt cccgaaggcg ccacggatgg gtctgcccag 2940
ccccgtttgc ccgctagtgg tggtgccgga ggaaaccccg gtctgtacga ggatcttttc 3000
cgcgtctctg tgaaggtcaa gaacacgggc aatgtcgccg gtgatgaagt tcctcagctg 3060
tacgtttccc taggcggccc gaatgagccc aaggtggtac tgcgcaagtt tgagcgtatt 3120
cacttggccc cttcgcagga ggccgtgtgg acaacgaccc ttacccgtcg tgaccttgca 3180
aactgggacg tttcggctca ggactggacc gtcactcctt accccaagac gatctacgtt 3240
ggaaactcct cacggaaact gccgctccag gcctcgctgc ctaaggccca gtaa 3294

<210> 122
<211> 1097
<212> PRT
<213> Aspergillus oryzae

<400> 122

```
Met Arg Ser Ser Pro Leu Leu Arg Ser Ala Val Val Ala Ala Leu Pro
1               5                   10                  15

Val Leu Ala Leu Ala Ala Asp Gly Arg Ser Thr Arg Tyr Trp Asp Cys
            20                  25                  30

Cys Lys Pro Ser Cys Gly Trp Ala Lys Lys Ala Pro Val Asn Gln Pro
        35                  40                  45

Val Phe Ser Cys Asn Ala Asn Phe Gln Arg Ile Thr Asp Phe Asp Ala
    50                  55                  60

Lys Ser Gly Cys Glu Pro Gly Gly Val Ala Tyr Ser Cys Ala Asp Gln
65                  70                  75                  80

Thr Pro Trp Ala Val Asn Asp Asp Phe Ala Leu Gly Phe Ala Ala Thr
                85                  90                  95

Ser Ile Ala Gly Ser Asn Glu Ala Gly Trp Cys Cys Ala Cys Tyr Glu
            100                 105                 110
```

```
Leu Thr Phe Thr Ser Gly Pro Val Ala Gly Lys Lys Met Val Val Gln
        115                 120                 125

Ser Thr Ser Thr Gly Gly Asp Leu Gly Ser Asn His Phe Asp Leu Asn
    130                 135                 140

Ile Pro Gly Gly Gly Val Gly Ile Phe Asp Gly Cys Thr Pro Gln Phe
145                 150                 155                 160

Gly Gly Leu Pro Gly Gln Arg Tyr Gly Gly Ile Ser Ser Arg Asn Glu
                165                 170                 175

Cys Asp Arg Phe Pro Asp Ala Leu Lys Pro Gly Cys Tyr Trp Arg Phe
            180                 185                 190

Asp Trp Phe Lys Asn Ala Asp Asn Pro Ser Phe Ser Phe Arg Gln Val
        195                 200                 205

Gln Cys Pro Ala Glu Leu Val Ala Arg Thr Gly Cys Arg Arg Asn Asp
    210                 215                 220

Asp Gly Asn Phe Pro Ala Val Gln Ile Pro Met Arg Ser Ser Pro Leu
225                 230                 235                 240

Leu Arg Ser Ala Val Val Ala Ala Leu Pro Val Leu Ala Leu Ala Lys
                245                 250                 255

Asp Asp Leu Ala Tyr Ser Pro Pro Phe Tyr Pro Ser Pro Trp Ala Asp
            260                 265                 270

Gly Gln Gly Glu Trp Ala Glu Val Tyr Lys Arg Ala Val Asp Ile Val
        275                 280                 285

Ser Gln Met Thr Leu Thr Glu Lys Val Asn Leu Thr Thr Gly Thr Gly
    290                 295                 300

Trp Gln Leu Glu Arg Cys Val Gly Gln Thr Gly Ser Val Pro Arg Leu
305                 310                 315                 320

Asn Ile Pro Ser Leu Cys Leu Gln Asp Ser Pro Leu Gly Ile Arg Phe
                325                 330                 335

Ser Asp Tyr Asn Ser Ala Phe Pro Ala Gly Val Asn Val Ala Ala Thr
            340                 345                 350

Trp Asp Lys Thr Leu Ala Tyr Leu Arg Gly Gln Ala Met Gly Glu Glu
        355                 360                 365
```

Phe Ser Asp Lys Gly Ile Asp Val Gln Leu Gly Pro Ala Ala Gly Pro
370 375 380

Leu Gly Ala His Pro Asp Gly Gly Arg Asn Trp Glu Gly Phe Ser Pro
385 390 395 400

Asp Pro Ala Leu Thr Gly Val Leu Phe Ala Glu Thr Ile Lys Gly Ile
405 410 415

Gln Asp Ala Gly Val Ile Ala Thr Ala Lys His Tyr Ile Met Asn Glu
420 425 430

Gln Glu His Phe Arg Gln Gln Pro Glu Ala Ala Gly Tyr Gly Phe Asn
435 440 445

Val Ser Asp Ser Leu Ser Ser Asn Val Asp Asp Lys Thr Met His Glu
450 455 460

Leu Tyr Leu Trp Pro Phe Ala Asp Ala Val Arg Ala Gly Val Gly Ala
465 470 475 480

Val Met Cys Ser Tyr Asn Gln Ile Asn Asn Ser Tyr Gly Cys Glu Asn
485 490 495

Ser Glu Thr Leu Asn Lys Leu Leu Lys Ala Glu Leu Gly Phe Gln Gly
500 505 510

Phe Val Met Ser Asp Trp Thr Ala His His Ser Gly Val Gly Ala Ala
515 520 525

Leu Ala Gly Leu Asp Met Ser Met Pro Gly Asp Val Thr Phe Asp Ser
530 535 540

Gly Thr Ser Phe Trp Gly Ala Asn Leu Thr Val Gly Val Leu Asn Gly
545 550 555 560

Thr Ile Pro Gln Trp Arg Val Asp Asp Met Ala Val Arg Ile Met Ala
565 570 575

Ala Tyr Tyr Lys Val Gly Arg Asp Thr Lys Tyr Thr Pro Pro Asn Phe
580 585 590

Ser Ser Trp Thr Arg Asp Glu Tyr Gly Phe Ala His Asn His Val Ser
595 600 605

Glu Gly Ala Tyr Glu Arg Val Asn Glu Phe Val Asp Val Gln Arg Asp
610 615 620

His Ala Asp Leu Ile Arg Arg Ile Gly Ala Gln Ser Thr Val Leu Leu
625 630 635 640

Lys Asn Lys Gly Ala Leu Pro Leu Ser Arg Lys Glu Lys Leu Val Ala
645 650 655

Leu Leu Gly Glu Asp Ala Gly Ser Asn Ser Trp Gly Ala Asn Gly Cys
660 665 670

Asp Asp Arg Gly Cys Asp Asn Gly Thr Leu Ala Met Ala Trp Gly Ser
675 680 685

Gly Thr Ala Asn Phe Pro Tyr Leu Val Thr Pro Glu Gln Ala Ile Gln
690 695 700

Asn Glu Val Leu Gln Gly Arg Gly Asn Val Phe Ala Val Thr Asp Ser
705 710 715 720

Trp Ala Leu Asp Lys Ile Ala Ala Ala Ala Arg Gln Ala Ser Val Ser
725 730 735

Leu Val Phe Val Asn Ser Asp Ser Gly Glu Gly Tyr Leu Ser Val Asp
740 745 750

Gly Asn Glu Gly Asp Arg Asn Asn Ile Thr Leu Trp Lys Asn Gly Asp
755 760 765

Asn Val Val Lys Thr Ala Ala Asn Asn Cys Asn Asn Thr Val Val Ile
770 775 780

Ile His Ser Val Gly Pro Val Leu Ile Asp Glu Trp Tyr Asp His Pro
785 790 795 800

Asn Val Thr Gly Ile Leu Trp Ala Gly Leu Pro Gly Gln Glu Ser Gly
805 810 815

Asn Ser Ile Ala Asp Val Leu Tyr Gly Arg Val Asn Pro Gly Ala Lys
820 825 830

Ser Pro Phe Thr Trp Gly Lys Thr Arg Glu Ser Tyr Gly Ser Pro Leu
835 840 845

Val Lys Asp Ala Asn Asn Gly Asn Gly Ala Pro Gln Ser Asp Phe Thr
850 855 860

Gln Gly Val Phe Ile Asp Tyr Arg His Phe Asp Lys Phe Asn Glu Thr
865 870 875 880

Pro Ile Tyr Glu Phe Gly Tyr Gly Leu Ser Tyr Thr Thr Phe Glu Leu

```
                885                 890                 895

Ser Asp Leu His Val Gln Pro Leu Asn Ala Ser Arg Tyr Thr Pro Thr
            900                 905                 910

Ser Gly Met Thr Glu Ala Ala Lys Asn Phe Gly Glu Ile Gly Asp Ala
        915                 920                 925

Ser Glu Tyr Val Tyr Pro Glu Gly Leu Glu Arg Ile His Glu Phe Ile
    930                 935                 940

Tyr Pro Trp Ile Asn Ser Thr Asp Leu Lys Ala Ser Ser Asp Asp Ser
945                 950                 955                 960

Asn Tyr Gly Trp Glu Asp Ser Lys Tyr Ile Pro Glu Gly Ala Thr Asp
                965                 970                 975

Gly Ser Ala Gln Pro Arg Leu Pro Ala Ser Gly Gly Ala Gly Gly Asn
            980                 985                 990

Pro Gly Leu Tyr Glu Asp Leu Phe  Arg Val Ser Val Lys  Val Lys Asn
        995                 1000                1005

Thr Gly  Asn Val Ala Gly Asp  Glu Val Pro Gln Leu  Tyr Val Ser
    1010                 1015                 1020

Leu Gly  Gly Pro Asn Glu Pro  Lys Val Val Leu Arg  Lys Phe Glu
    1025                 1030                 1035

Arg Ile  His Leu Ala Pro Ser  Gln Glu Ala Val Trp  Thr Thr Thr
    1040                 1045                 1050

Leu Thr  Arg Arg Asp Leu Ala  Asn Trp Asp Val Ser  Ala Gln Asp
    1055                 1060                 1065

Trp Thr  Val Thr Pro Tyr Pro  Lys Thr Ile Tyr Val  Gly Asn Ser
    1070                 1075                 1080

Ser Arg  Lys Leu Pro Leu Gln  Ala Ser Leu Pro Lys  Ala Gln
    1085                 1090                 1095
```

<210> 123
<211> 3294
<212> DNA
<213> Aspergillus oryzae

<400> 123

atgcgttcct ccccccctcct ccgctccgcc gttgtggccg ccctgccggt gttggccctt 60

gccgctgatg gcaggtccac ccgctactgg gactgctgca agccttcgtg cggctgggcc 120

aagaaggctc ccgtgaacca gcctgtcttt tcctgcaacg ccaacttcca gcgtatcacg 180
gacttcgacg ccaagtccgg ctgcgagccg ggcggtgtcg cctactcgtg cgccgaccag 240
accccatggg ctgtgaacga cgacttcgcg ctcggttttg ctgccacctc tattgccggc 300
agcaatgagg cgggctggtg ctgcgcctgc tacgagctca ccttcacatc cggtcctgtt 360
gctggcaaga agatggtcgt ccagtccacc agcactggcg gtgatcttgg cagcaaccac 420
ttcgatctca acatccccgg cggcggcgtc ggcatcttcg acggatgcac tccccagttc 480
ggtggtctgc ccggccagcg ctacggcggc atctcgtccc gcaacgagtg cgatcggttc 540
cccgacgccc tcaagcccgg ctgctactgg cgcttcgact ggttcaagaa cgccgacaat 600
ccgagcttca gcttccgtca ggtccagtgc ccagccgagc tcgtcgctcg caccggatgc 660
cgccgcaacg acgacggcaa cttccctgcc gtccagatcc ccatgcgttc ctcccccctc 720
ctccgctccg ccgttgtggc cgccctgccg gtgttggccc ttgccaagga tgatctcgcg 780
tactcccctc ctttctaccc ttccccatgg gcagatggtc agggtgaatg ggcggaagta 840
tacaaacgcg ctgtagacat agtttcccag atgacgttga cagagaaagt caacttaacg 900
actggaacag gatggcaact agagaggtgt gttggacaaa ctggcagtgt tcccagactc 960
aacatcccca gcttgtgttt gcaggatagt cctcttggta ttcgtttctc ggactacaat 1020
tcagctttcc ctgcgggtgt taatgtcgct gccacctggg acaagacgct cgcctacctt 1080
cgtggtcagg caatgggtga ggagttcagt gataagggta ttgacgttca gctgggtcct 1140
gctgctggcc ctctcggtgc tcatccggat ggcggtagaa actgggaaag tttctcacca 1200
gatccagccc tcaccggtgt acttttgcg gagacgatta agggtattca agatgctggt 1260
gtcattgcga cagctaagca ttatatcatg aacgaacaag agcatttccg ccaacaaccc 1320
gaggctgcgg gttacggatt caacgtaagc gacagtttga gttccaacgt tgatgacaag 1380
actatgcatg aattgtacct ctggcccttc gcggatgcag tacgcgctgg agtcggtgct 1440
gttatgtgct cttacaacca aatcaacaac agctacggtt gcgagaatag cgaaactctg 1500
aacaagcttt tgaaggcgga gcttggtttc caaggcttcg tcatgagtga ttggaccgct 1560
caacacagcg gcgtaggcgc tgctttagca ggtctggata tgtcgatgcc cggtgatgtt 1620
accttcgata gtggtacgtc tttctggggt gcaaacttga cggtcggtgt ccttaacggt 1680
acaatccccc aatggcgtgt tgatgacatg gctgtccgta tcatggccgc ttattacaag 1740
gttggccgcg acaccaaata cacccctccc aacttcagct cgtggaccag ggacgaatat 1800
ggtttcgcgc ataaccatgt ttcggaaggt gcttacgaga gggtcaacga attcgtggac 1860
gtgcaacgcg atcatgccga cctaatccgt cgcatcggcg cgcagagcac tgttctgctg 1920
aagaacaagg gtgccttgcc cttgagccgc aaggaaaagc tggtcgccct tctgggagag 1980
gatgcgggtt ccaactcgtg gggcgctaac ggctgtgatg accgtggttg cgataacggt 2040
acccttgcca tggcctgggg tagcggtact gcgaatttcc catacctcgt gacaccagag 2100

caggcgattc agaacgaagt tcttcagggc cgtggtaatg tcttcgccgt gaccgacagt 2160
tgggcgctcg acaagatcgc tgcggctgcc cgccaggcca gcgtatctct cgtgttcgtc 2220
aactccgact caggagaagg ctatcttagt gtggatggaa atgagggcga tcgtaacaac 2280
atcactctgt ggaagaacgg cgacaatgtg gtcaagaccg cagcgaataa ctgtaacaac 2340
accgttgtca tcatccactc cgtcggacca gttttgatcg atgaatggta tgaccacccc 2400
aatgtcactg gtattctctg ggctggtctg ccaggccagg agtctggtaa ctccattgcc 2460
gatgtgctgt acggtcgtgt caaccctggc gccaagtctc ctttcacttg ggcaagacc 2520
cgggagtcgt atggttctcc cttggtcaag gatgccaaca atggcaacgg agcgccccag 2580
tctgatttca cccagggtgt tttcatcgat taccgccatt tcgataagtt caatgagacc 2640
cctatctacg agtttggcta cggcttgagc tacaccacct tcgagctctc cgacctccat 2700
gttcagcccc tgaacgcgtc ccgatacact cccaccagtg gcatgactga agctgcaaag 2760
aactttggtg aaattggcga tgcgtcggag tacgtgtatc cggaggggct ggaaaggatc 2820
catgagttta tctatccctg gatcaactct accgacctga aggcatcgtc tgacgattct 2880
aactacggct gggaagactc caagtatatt cccgaaggcg ccacggatgg gtctgcccag 2940
ccccgtttgc ccgctagtgg tggtgccgga ggaaaccccg gtctgtacga ggatcttttc 3000
cgcgtctctg tgaaggtcaa gaacacgggc aatgtcgccg gtgatgaagt tcctcagctg 3060
tacgtttccc taggcggccc gaatgagccc aaggtggtac tgcgcaagtt tgagcgtatt 3120
cacttggccc cttcgcagga ggccgtgtgg acaacgaccc ttacccgtcg tgaccttgca 3180
aactgggacg tttcggctca ggactggacc gtcactcctt accccaagac gatctacgtt 3240
ggaaactcct cacggaaact gccgctccag gcctcgctgc ctaaggccca gtaa 3294

<210> 124
<211> 1097
<212> PRT
<213> Aspergillus oryzae

<400> 124

Met Arg Ser Ser Pro Leu Leu Arg Ser Ala Val Val Ala Ala Leu Pro
1               5                   10                  15

Val Leu Ala Leu Ala Ala Asp Gly Arg Ser Thr Arg Tyr Trp Asp Cys
            20                  25                  30

Cys Lys Pro Ser Cys Gly Trp Ala Lys Lys Ala Pro Val Asn Gln Pro
        35                  40                  45

Val Phe Ser Cys Asn Ala Asn Phe Gln Arg Ile Thr Asp Phe Asp Ala
    50                  55                  60

```
Lys Ser Gly Cys Glu Pro Gly Gly Val Ala Tyr Ser Cys Ala Asp Gln
65                  70                  75                  80

Thr Pro Trp Ala Val Asn Asp Asp Phe Ala Leu Gly Phe Ala Ala Thr
                85                  90                  95

Ser Ile Ala Gly Ser Asn Glu Ala Gly Trp Cys Cys Ala Cys Tyr Glu
            100                 105                 110

Leu Thr Phe Thr Ser Gly Pro Val Ala Gly Lys Lys Met Val Val Gln
        115                 120                 125

Ser Thr Ser Thr Gly Gly Asp Leu Gly Ser Asn His Phe Asp Leu Asn
    130                 135                 140

Ile Pro Gly Gly Gly Val Gly Ile Phe Asp Gly Cys Thr Pro Gln Phe
145                 150                 155                 160

Gly Gly Leu Pro Gly Gln Arg Tyr Gly Gly Ile Ser Ser Arg Asn Glu
                165                 170                 175

Cys Asp Arg Phe Pro Asp Ala Leu Lys Pro Gly Cys Tyr Trp Arg Phe
            180                 185                 190

Asp Trp Phe Lys Asn Ala Asp Asn Pro Ser Phe Ser Phe Arg Gln Val
        195                 200                 205

Gln Cys Pro Ala Glu Leu Val Ala Arg Thr Gly Cys Arg Arg Asn Asp
    210                 215                 220

Asp Gly Asn Phe Pro Ala Val Gln Ile Pro Met Arg Ser Ser Pro Leu
225                 230                 235                 240

Leu Arg Ser Ala Val Val Ala Ala Leu Pro Val Leu Ala Leu Ala Lys
                245                 250                 255

Asp Asp Leu Ala Tyr Ser Pro Pro Phe Tyr Pro Ser Pro Trp Ala Asp
            260                 265                 270

Gly Gln Gly Glu Trp Ala Glu Val Tyr Lys Arg Ala Val Asp Ile Val
        275                 280                 285

Ser Gln Met Thr Leu Thr Glu Lys Val Asn Leu Thr Thr Gly Thr Gly
    290                 295                 300

Trp Gln Leu Glu Arg Cys Val Gly Gln Thr Gly Ser Val Pro Arg Leu
305                 310                 315                 320

Asn Ile Pro Ser Leu Cys Leu Gln Asp Ser Pro Leu Gly Ile Arg Phe
```

325 330 335

Ser Asp Tyr Asn Ser Ala Phe Pro Ala Gly Val Asn Val Ala Ala Thr
340 345 350

Trp Asp Lys Thr Leu Ala Tyr Leu Arg Gly Gln Ala Met Gly Glu Glu
355 360 365

Phe Ser Asp Lys Gly Ile Asp Val Gln Leu Gly Pro Ala Ala Gly Pro
370 375 380

Leu Gly Ala His Pro Asp Gly Gly Arg Asn Trp Glu Ser Phe Ser Pro
385 390 395 400

Asp Pro Ala Leu Thr Gly Val Leu Phe Ala Glu Thr Ile Lys Gly Ile
405 410 415

Gln Asp Ala Gly Val Ile Ala Thr Ala Lys His Tyr Ile Met Asn Glu
420 425 430

Gln Glu His Phe Arg Gln Gln Pro Glu Ala Ala Gly Tyr Gly Phe Asn
435 440 445

Val Ser Asp Ser Leu Ser Ser Asn Val Asp Asp Lys Thr Met His Glu
450 455 460

Leu Tyr Leu Trp Pro Phe Ala Asp Ala Val Arg Ala Gly Val Gly Ala
465 470 475 480

Val Met Cys Ser Tyr Asn Gln Ile Asn Asn Ser Tyr Gly Cys Glu Asn
485 490 495

Ser Glu Thr Leu Asn Lys Leu Leu Lys Ala Glu Leu Gly Phe Gln Gly
500 505 510

Phe Val Met Ser Asp Trp Thr Ala Gln His Ser Gly Val Gly Ala Ala
515 520 525

Leu Ala Gly Leu Asp Met Ser Met Pro Gly Asp Val Thr Phe Asp Ser
530 535 540

Gly Thr Ser Phe Trp Gly Ala Asn Leu Thr Val Gly Val Leu Asn Gly
545 550 555 560

Thr Ile Pro Gln Trp Arg Val Asp Asp Met Ala Val Arg Ile Met Ala
565 570 575

Ala Tyr Tyr Lys Val Gly Arg Asp Thr Lys Tyr Thr Pro Pro Asn Phe
580 585 590

Ser Ser Trp Thr Arg Asp Glu Tyr Gly Phe Ala His Asn His Val Ser
595 600 605

Glu Gly Ala Tyr Glu Arg Val Asn Glu Phe Val Asp Val Gln Arg Asp
610 615 620

His Ala Asp Leu Ile Arg Arg Ile Gly Ala Gln Ser Thr Val Leu Leu
625 630 635 640

Lys Asn Lys Gly Ala Leu Pro Leu Ser Arg Lys Glu Lys Leu Val Ala
645 650 655

Leu Leu Gly Glu Asp Ala Gly Ser Asn Ser Trp Gly Ala Asn Gly Cys
660 665 670

Asp Asp Arg Gly Cys Asp Asn Gly Thr Leu Ala Met Ala Trp Gly Ser
675 680 685

Gly Thr Ala Asn Phe Pro Tyr Leu Val Thr Pro Glu Gln Ala Ile Gln
690 695 700

Asn Glu Val Leu Gln Gly Arg Gly Asn Val Phe Ala Val Thr Asp Ser
705 710 715 720

Trp Ala Leu Asp Lys Ile Ala Ala Ala Ala Arg Gln Ala Ser Val Ser
725 730 735

Leu Val Phe Val Asn Ser Asp Ser Gly Glu Gly Tyr Leu Ser Val Asp
740 745 750

Gly Asn Glu Gly Asp Arg Asn Asn Ile Thr Leu Trp Lys Asn Gly Asp
755 760 765

Asn Val Val Lys Thr Ala Ala Asn Asn Cys Asn Asn Thr Val Val Ile
770 775 780

Ile His Ser Val Gly Pro Val Leu Ile Asp Glu Trp Tyr Asp His Pro
785 790 795 800

Asn Val Thr Gly Ile Leu Trp Ala Gly Leu Pro Gly Gln Glu Ser Gly
805 810 815

Asn Ser Ile Ala Asp Val Leu Tyr Gly Arg Val Asn Pro Gly Ala Lys
820 825 830

Ser Pro Phe Thr Trp Gly Lys Thr Arg Glu Ser Tyr Gly Ser Pro Leu
835 840 845

Val Lys Asp Ala Asn Asn Gly Asn Gly Ala Pro Gln Ser Asp Phe Thr
    850                 855                 860

Gln Gly Val Phe Ile Asp Tyr Arg His Phe Asp Lys Phe Asn Glu Thr
865                 870                 875                 880

Pro Ile Tyr Glu Phe Gly Tyr Gly Leu Ser Tyr Thr Thr Phe Glu Leu
                885                 890                 895

Ser Asp Leu His Val Gln Pro Leu Asn Ala Ser Arg Tyr Thr Pro Thr
            900                 905                 910

Ser Gly Met Thr Glu Ala Ala Lys Asn Phe Gly Glu Ile Gly Asp Ala
        915                 920                 925

Ser Glu Tyr Val Tyr Pro Glu Gly Leu Glu Arg Ile His Glu Phe Ile
    930                 935                 940

Tyr Pro Trp Ile Asn Ser Thr Asp Leu Lys Ala Ser Ser Asp Asp Ser
945                 950                 955                 960

Asn Tyr Gly Trp Glu Asp Ser Lys Tyr Ile Pro Glu Gly Ala Thr Asp
                965                 970                 975

Gly Ser Ala Gln Pro Arg Leu Pro Ala Ser Gly Gly Ala Gly Gly Asn
            980                 985                 990

Pro Gly Leu Tyr Glu Asp Leu Phe Arg Val Ser Val Lys Val Lys Asn
        995                 1000                1005

Thr Gly Asn Val Ala Gly Asp Glu Val Pro Gln Leu Tyr Val Ser
    1010                1015                1020

Leu Gly Gly Pro Asn Glu Pro Lys Val Val Leu Arg Lys Phe Glu
    1025                1030                1035

Arg Ile His Leu Ala Pro Ser Gln Glu Ala Val Trp Thr Thr Thr
    1040                1045                1050

Leu Thr Arg Arg Asp Leu Ala Asn Trp Asp Val Ser Ala Gln Asp
    1055                1060                1065

Trp Thr Val Thr Pro Tyr Pro Lys Thr Ile Tyr Val Gly Asn Ser
    1070                1075                1080

Ser Arg Lys Leu Pro Leu Gln Ala Ser Leu Pro Lys Ala Gln
    1085                1090                1095

<210> 125
<211> 38
<212> DNA
<213> Trichoderma reesei

<400> 125
actggattta ccatgaacaa gtccgtggct ccattgct 38

<210> 126
<211> 38
<212> DNA
<213> Trichoderma reesei

<400> 126
tcacctctag ttaattaact actttcttgc gagacacg 38

<210> 127
<211> 19
<212> PRT
<213> Thielavia terrestris

<220>
<221> MISC_FEATURE
<222> (1)..(1)
<223> X=I,L,M, OR V

<220>
<221> misc_feature
<222> (3)..(6)
<223> Xaa can be any naturally occurring amino acid

<220>
<221> misc_feature
<222> (8)..(8)
<223> Xaa can be any naturally occurring amino acid

<220>
<221> MISC_FEATURE
<222> (10)..(10)
<223> X=I,L,M, OR V

<220>
<221> misc_feature
<222> (11)..(11)
<223> Xaa can be any naturally occurring amino acid

<220>
<221> misc_feature
<222> (13)..(13)
<223> Xaa can be any naturally occurring amino acid

<220>
<221> MISC_FEATURE
<222> (14)..(14)
<223> X=E OR Q

<220>
<221> misc_feature

<222> (15)..(18)
<223> Xaa can be any naturally occurring amino acid

<220>
<221> MISC_FEATURE
<222> (19)..(19)
<223> X=H,N, OR Q

<400> 127

```
Xaa Pro Xaa Xaa Xaa Xaa Gly Xaa Tyr Xaa Xaa Arg Xaa Xaa Xaa Xaa
1               5                   10                  15


Xaa Xaa Xaa
```

<210> 128
<211> 20
<212> PRT
<213> Thielavia terrestris

<220>
<221> MISC_FEATURE
<222> (1)..(1)
<223> X=I,L,M, OR V

<220>
<221> misc_feature
<222> (3)..(7)
<223> Xaa can be any naturally occurring amino acid

<220>
<221> misc_feature
<222> (9)..(9)
<223> Xaa can be any naturally occurring amino acid

<220>
<221> MISC_FEATURE
<222> (11)..(11)
<223> X=I,L,M, OR V

<220>
<221> misc_feature
<222> (12)..(12)
<223> Xaa can be any naturally occurring amino acid

<220>
<221> misc_feature
<222> (14)..(14)
<223> Xaa can be any naturally occurring amino acid

<220>
<221> MISC_FEATURE
<222> (15)..(15)
<223> X=E OR Q

<220>
<221> misc_feature

<222> (16)..(19)
<223> Xaa can be any naturally occurring amino acid

<220>
<221> MISC_FEATURE
<222> (20)..(20)
<223> X=H,N, OR Q

<400> 128

```
Xaa Pro Xaa Xaa Xaa Xaa Xaa Gly Xaa Tyr Xaa Xaa Arg Xaa Xaa Xaa
1               5                   10                  15

Xaa Xaa Xaa Xaa
            20
```

<210> 129
<211> 9
<212> PRT
<213> Thielavia terrestris

<220>
<221> misc_feature
<222> (2)..(2)
<223> Xaa can be any naturally occurring amino acid

<220>
<221> misc_feature
<222> (5)..(7)
<223> Xaa can be any naturally occurring amino acid

<220>
<221> MISC_FEATURE
<222> (8)..(8)
<223> X= Y OR W

<220>
<221> MISC_FEATURE
<222> (9)..(9)
<223> X= A,I,L,M OR V

<400> 129

```
His Xaa Gly Pro Xaa Xaa Xaa Xaa Xaa
1               5
```

<210> 130
<211> 10
<212> PRT
<213> Thielavia terrestris

<220>
<221> misc_feature
<222> (2)..(3)
<223> Xaa can be any naturally occurring amino acid

<220>

<221> misc_feature
<222> (6)..(8)
<223> Xaa can be any naturally occurring amino acid

<220>
<221> MISC_FEATURE
<222> (9)..(9)
<223> X= Y OR W

<220>
<221> MISC_FEATURE
<222> (10)..(10)
<223> X= Y OR W

<400> 130

```
His Xaa Xaa Gly Pro Xaa Xaa Xaa Xaa Xaa
1               5                   10
```

<210> 131
<211> 11
<212> PRT
<213> Thielavia terrestris

<220>
<221> MISC_FEATURE
<222> (1)..(1)
<223> X= E OR Q

<220>
<221> misc_feature
<222> (2)..(2)
<223> Xaa can be any naturally occurring amino acid

<220>
<221> misc_feature
<222> (4)..(5)
<223> Xaa can be any naturally occurring amino acid

<220>
<221> misc_feature
<222> (7)..(7)
<223> Xaa can be any naturally occurring amino acid

<220>
<221> MISC_FEATURE
<222> (8)..(8)
<223> X= E,H,Q OR N

<220>
<221> MISC_FEATURE
<222> (9)..(9)
<223> X=F,I,L, OR V

<220>
<221> misc_feature
<222> (10)..(10)
<223> Xaa can be any naturally occurring amino acid

<220>
<221> MISC_FEATURE
<222> (11)..(11)
<223> X=I,L,OR V

<400> 131

Xaa Xaa Tyr Xaa Xaa Cys Xaa Xaa Xaa Xaa Xaa
1 5 10

<210> 132
<211> 9
<212> PRT
<213> Thielavia terrestris

<220>
<221> misc_feature
<222> (2)..(2)
<223> Xaa can be any naturally occurring amino acid

<220>
<221> misc_feature
<222> (5)..(7)
<223> Xaa can be any naturally occurring amino acid

<220>
<221> MISC_FEATURE
<222> (8)..(8)
<223> X= Y OR W

<220>
<221> MISC_FEATURE
<222> (9)..(9)
<223> X= A,I,L,M OR V

<400> 132

His Xaa Gly Pro Xaa Xaa Xaa Xaa Xaa
1 5

<210> 133
<211> 10
<212> PRT
<213> Thielavia terrestris

<220>
<221> misc_feature
<222> (2)..(3)
<223> Xaa can be any naturally occurring amino acid

<220>
<221> misc_feature
<222> (6)..(8)
<223> Xaa can be any naturally occurring amino acid

<220>
<221> MISC_FEATURE
<222> (9)..(9)

<223> X= Y OR W

<220>
<221> MISC_FEATURE
<222> (10)..(10)
<223> X= A,I,L,M OR V

<400> 133

His Xaa Xaa Gly Pro Xaa Xaa Xaa Xaa Xaa
1               5                   10

<210> 134
<211> 11
<212> PRT
<213> Thielavia terrestris

<220>
<221> MISC_FEATURE
<222> (1)..(1)
<223> X= E OR Q

<220>
<221> misc_feature
<222> (2)..(2)
<223> Xaa can be any naturally occurring amino acid

<220>
<221> misc_feature
<222> (4)..(5)
<223> Xaa can be any naturally occurring amino acid

<220>
<221> misc_feature
<222> (7)..(7)
<223> Xaa can be any naturally occurring amino acid

<220>
<221> MISC_FEATURE
<222> (8)..(8)
<223> X= E,H,Q OR N

<220>
<221> MISC_FEATURE
<222> (9)..(9)
<223> X=F,I,L, OR V

<220>
<221> misc_feature
<222> (10)..(10)
<223> Xaa can be any naturally occurring amino acid

<220>
<221> MISC_FEATURE
<222> (11)..(11)
<223> X=I,L,OR V

<400> 134

Xaa Xaa Tyr Xaa Xaa Cys Xaa Xaa Xaa Xaa Xaa
1 5 10

<210> 135
<211> 9
<212> PRT
<213> Thielavia terrestris

<220>
<221> misc_feature
<222> (2)..(2)
<223> Xaa can be any naturally occurring amino acid

<220>
<221> misc_feature
<222> (5)..(7)
<223> Xaa can be any naturally occurring amino acid

<220>
<221> MISC_FEATURE
<222> (8)..(8)
<223> X= Y OR W

<220>
<221> MISC_FEATURE
<222> (9)..(9)
<223> X= A,I,L,M OR V

<400> 135

His Xaa Gly Pro Xaa Xaa Xaa Xaa Xaa
1 5

<210> 136
<211> 10
<212> PRT
<213> Thielavia terrestris

<220>
<221> misc_feature
<222> (2)..(3)
<223> Xaa can be any naturally occurring amino acid

<220>
<221> misc_feature
<222> (6)..(8)
<223> Xaa can be any naturally occurring amino acid

<220>
<221> MISC_FEATURE
<222> (9)..(9)
<223> X= Y OR W

<220>
<221> MISC_FEATURE
<222> (10)..(10)
<223> X= A,I,L,M OR V

<400> 136

```
His Xaa Xaa Gly Pro Xaa Xaa Xaa Xaa Xaa
1               5                   10
```

<210> 137
<211> 11
<212> PRT
<213> Thielavia terrestris

<220>
<221> MISC_FEATURE
<222> (1)..(1)
<223> X= E OR Q

<220>
<221> misc_feature
<222> (2)..(2)
<223> Xaa can be any naturally occurring amino acid

<220>
<221> misc_feature
<222> (4)..(5)
<223> Xaa can be any naturally occurring amino acid

<220>
<221> misc_feature
<222> (7)..(7)
<223> Xaa can be any naturally occurring amino acid

<220>
<221> MISC_FEATURE
<222> (8)..(8)
<223> X= E,H,Q OR N

<220>
<221> MISC_FEATURE
<222> (9)..(9)
<223> X=F,I,L, OR V

<220>
<221> misc_feature
<222> (10)..(10)
<223> Xaa can be any naturally occurring amino acid

<220>
<221> MISC_FEATURE
<222> (11)..(11)
<223> X=I,L,OR V

<400> 137

```
Xaa Xaa Tyr Xaa Xaa Cys Xaa Xaa Xaa Xaa Xaa
1               5                   10
```

<210> 138
<211> 9
<212> PRT

<213> Thielavia terrestris

<220>
<221> misc_feature
<222> (2)..(2)
<223> Xaa can be any naturally occurring amino acid

<220>
<221> misc_feature
<222> (5)..(7)
<223> Xaa can be any naturally occurring amino acid

<220>
<221> MISC_FEATURE
<222> (8)..(8)
<223> X= Y OR W

<220>
<221> MISC_FEATURE
<222> (9)..(9)
<223> X= A,I,L,M OR V

<400> 138

```
His Xaa Gly Pro Xaa Xaa Xaa Xaa Xaa
1               5
```

<210> 139
<211> 10
<212> PRT
<213> Thielavia terrestris

<220>
<221> misc_feature
<222> (2)..(3)
<223> Xaa can be any naturally occurring amino acid

<220>
<221> misc_feature
<222> (6)..(8)
<223> Xaa can be any naturally occurring amino acid

<220>
<221> MISC_FEATURE
<222> (9)..(9)
<223> X= Y OR W

<220>
<221> MISC_FEATURE
<222> (10)..(10)
<223> X= A,I,L,M OR V

<400> 139

```
His Xaa Xaa Gly Pro Xaa Xaa Xaa Xaa Xaa
1               5                   10
```

<210> 140

<211> 11
<212> PRT
<213> Thielavia terrestris

<220>
<221> MISC_FEATURE
<222> (1)..(1)
<223> X= E OR Q

<220>
<221> misc_feature
<222> (2)..(2)
<223> Xaa can be any naturally occurring amino acid

<220>
<221> misc_feature
<222> (4)..(5)
<223> Xaa can be any naturally occurring amino acid

<220>
<221> misc_feature
<222> (7)..(7)
<223> Xaa can be any naturally occurring amino acid

<220>
<221> MISC_FEATURE
<222> (8)..(8)
<223> X= E,H,Q OR N

<220>
<221> MISC_FEATURE
<222> (9)..(9)
<223> X=F,I,L, OR V

<220>
<221> misc_feature
<222> (10)..(10)
<223> Xaa can be any naturally occurring amino acid

<220>
<221> MISC_FEATURE
<222> (11)..(11)
<223> X=I,L,OR V

<400> 140

```
Xaa Xaa Tyr Xaa Xaa Cys Xaa Xaa Xaa Xaa Xaa
1               5                   10
```

<210> 141
<211> 19
<212> PRT
<213> Thielavia terrestris

<220>
<221> MISC_FEATURE
<222> (1)..(1)
<223> X=I,L,M OR V

<220>
<221> misc_feature
<222> (3)..(6)
<223> Xaa can be any naturally occurring amino acid

<220>
<221> misc_feature
<222> (8)..(8)
<223> Xaa can be any naturally occurring amino acid

<220>
<221> MISC_FEATURE
<222> (10)..(10)
<223> X=I,L,M OR V

<220>
<221> misc_feature
<222> (11)..(11)
<223> Xaa can be any naturally occurring amino acid

<220>
<221> misc_feature
<222> (13)..(13)
<223> Xaa can be any naturally occurring amino acid

<220>
<221> MISC_FEATURE
<222> (14)..(14)
<223> X= E OR Q

<220>
<221> misc_feature
<222> (15)..(17)
<223> Xaa can be any naturally occurring amino acid

<220>
<221> MISC_FEATURE
<222> (19)..(19)
<223> X= H,N, OR Q

<400> 141

```
Xaa Pro Xaa Xaa Xaa Xaa Gly Xaa Tyr Xaa Xaa Arg Xaa Xaa Xaa Xaa
1               5                   10                  15

Xaa Ala Xaa
```

<210> 142
<211> 20
<212> PRT
<213> Thielavia terrestris

<220>
<221> MISC_FEATURE
<222> (1)..(1)
<223> X=I,L,M OR V

<220>
<221> misc_feature
<222> (3)..(7)
<223> Xaa can be any naturally occurring amino acid

<220>
<221> misc_feature
<222> (9)..(9)
<223> Xaa can be any naturally occurring amino acid

<220>
<221> MISC_FEATURE
<222> (11)..(11)
<223> X=I,L,M OR V

<220>
<221> misc_feature
<222> (12)..(12)
<223> Xaa can be any naturally occurring amino acid

<220>
<221> misc_feature
<222> (14)..(14)
<223> Xaa can be any naturally occurring amino acid

<220>
<221> MISC_FEATURE
<222> (15)..(15)
<223> X=E OR Q

<220>
<221> misc_feature
<222> (16)..(18)
<223> Xaa can be any naturally occurring amino acid

<220>
<221> MISC_FEATURE
<222> (20)..(20)
<223> X= H,N, OR Q

<400> 142

```
Xaa Pro Xaa Xaa Xaa Xaa Xaa Gly Xaa Tyr Xaa Xaa Arg Xaa Xaa Xaa
1               5                   10                  15

Xaa Xaa Ala Xaa
            20
```

<210> 143
<211> 1035
<212> DNA
<213> Aspergillus aculeatus

<400> 143

```
atgaagtata ttcctctcgt tattgcagtt gctgccggcc tggcacgtcc ggctactgcc      60
cactacatct tcagcaagct cgtgctgaac ggagaggcat ctgcggactg gcaatacatc     120
cgcgagacta ctcgcagcat agtctatgag ccgaccaagt acacctctac cttcgataac     180
ctaacaccca gcgatagcga cttccgctgt aatctcggtt ccttcagcaa tgctgcgaag     240
accgaggtcg ctgaggttgc ggcaggcgat accatcgcaa tgaagctatt ctacgacacc     300
agtattgcgc atcctggccc gggacaagtt tatatgtcca aggcaccgac cggcaatgtt     360
caggaatacc aaggagacgg ggattggttc aaaatctggg aaaagaccct ttgcaacacg     420
gatggtgatc tgactacaga ggcctggtgc acctggggca tgtcacagtt tgaatttcaa     480
atcccagctg cgaccccggc aggagagtac ctagtgcgcg ccgagcatat aggcctgcat     540
ggcgctcaag cgaacgaggc cgaattcttc tacagctgtg cgcagatcaa ggttacaggc     600
tcgggaactg gatctcccag tctcacgtat caaattcctg gtctctataa cgacactatg     660
accctgttca atggcctcaa tctttggact gattcagccg agaaggtgca gctggatttc     720
ctggagacgc caattgggga cgacgtgtgg agcggagcag gctcggggag cccatctgct     780
gccacctctt cgaccagcgg tgcaactctt gcagctcagg gtacaactac ctctgccgcg     840
catgctcagg cccagaccac cattaccacc agcaccagca ccatcacgtc tctcgaatca     900
gccagctcaa ccgatctcgt tgcgcagtat ggtcagtgcg gaggccttaa ctggtccggt     960
ccaaccgagt gtgagacacc ttatacctgt gtgcagcaga acccttacta ccatcaatgc    1020
gtgaattcgt gctga                                                     1035
```

<210> 144
<211> 344
<212> PRT
<213> Aspergillus aculeatus

<400> 144

```
Met Lys Tyr Ile Pro Leu Val Ile Ala Val Ala Ala Gly Leu Ala Arg
1               5                   10                  15
```

```
Pro Ala Thr Ala His Tyr Ile Phe Ser Lys Leu Val Leu Asn Gly Glu
            20                  25                  30

Ala Ser Ala Asp Trp Gln Tyr Ile Arg Glu Thr Thr Arg Ser Ile Val
        35                  40                  45

Tyr Glu Pro Thr Lys Tyr Thr Ser Thr Phe Asp Asn Leu Thr Pro Ser
    50                  55                  60

Asp Ser Asp Phe Arg Cys Asn Leu Gly Ser Phe Ser Asn Ala Ala Lys
65                  70                  75                  80

Thr Glu Val Ala Glu Val Ala Ala Gly Asp Thr Ile Ala Met Lys Leu
                85                  90                  95

Phe Tyr Asp Thr Ser Ile Ala His Pro Gly Pro Gly Gln Val Tyr Met
            100                 105                 110

Ser Lys Ala Pro Thr Gly Asn Val Gln Glu Tyr Gln Gly Asp Gly Asp
        115                 120                 125

Trp Phe Lys Ile Trp Glu Lys Thr Leu Cys Asn Thr Asp Gly Asp Leu
    130                 135                 140

Thr Thr Glu Ala Trp Cys Thr Trp Gly Met Ser Gln Phe Glu Phe Gln
145                 150                 155                 160

Ile Pro Ala Ala Thr Pro Ala Gly Glu Tyr Leu Val Arg Ala Glu His
                165                 170                 175

Ile Gly Leu His Gly Ala Gln Ala Asn Glu Ala Glu Phe Phe Tyr Ser
            180                 185                 190

Cys Ala Gln Ile Lys Val Thr Gly Ser Gly Thr Gly Ser Pro Ser Leu
        195                 200                 205

Thr Tyr Gln Ile Pro Gly Leu Tyr Asn Asp Thr Met Thr Leu Phe Asn
    210                 215                 220

Gly Leu Asn Leu Trp Thr Asp Ser Ala Glu Lys Val Gln Leu Asp Phe
225                 230                 235                 240

Leu Glu Thr Pro Ile Gly Asp Asp Val Trp Ser Gly Ala Gly Ser Gly
                245                 250                 255

Ser Pro Ser Ala Ala Thr Ser Ser Thr Ser Gly Ala Thr Leu Ala Ala
            260                 265                 270

Gln Gly Thr Thr Thr Ser Ala Ala His Ala Gln Ala Gln Thr Thr Ile
```

275 280 285

Thr Thr Ser Thr Ser Thr Ile Thr Ser Leu Glu Ser Ala Ser Ser Thr
290 295 300

Asp Leu Val Ala Gln Tyr Gly Gln Cys Gly Gly Leu Asn Trp Ser Gly
305 310 315 320

Pro Thr Glu Cys Glu Thr Pro Tyr Thr Cys Val Gln Gln Asn Pro Tyr
325 330 335

Tyr His Gln Cys Val Asn Ser Cys
340

<210> 145
<211> 1170
<212> DNA
<213> Aspergillus aculeatus

<400> 145

```
atgaagtcct ctactttcgg tatgctcgct ctggcagcag cagccaagat ggtcgatgcc      60
cacaccaccg tcttcgccgt ctggatcaac ggcgaggacc agggtctggg caacagtgcc     120
agtggctaca tccggtctcc ccccagcaac agccccgtca aggacgtgac ctcgaccgac     180
atcacctgca acgtcaacgg cgaccaggcg gcggctaaga ccctctccgt caagggcggc     240
gacgtcgtca ccttcgagtg gcaccacgac agccgggacg cctccgacga catcatcgcc     300
tcctcccaca agggccccgt catggtctac atggccccga ccaccgccgg cagcagcggc     360
aagaactggg tcaagatcgc cgaggacgga tactccgacg gcacctgggc cgtcgacacc     420
ctgatcgcca acagcggcaa gcacaacatc accgtccccg acgtccccgc cggcgactac     480
ctcttccgcc cggagatcat cgccctccac gaggccgaga acgagggcgg cgcccagttc     540
tacatggagt gtgtccagtt caaggtcacc tccgacggtg ccaacactct gcccgacggt     600
gtcagcctgc ccggcgccta ctccgccact gaccccggta tcctcttcaa catgtacggc     660
tccttcgaca gctatcccat ccccggtccc tccgtctggg atggcactag ctctggctct     720
tcctcttctt cctcttcttc ctcttccagc tcttccgccg ccgctgccgt tgttgccacc     780
tcctcttcct cttcctctgc ttccatcgag gccgtgacca ccaagggtgc cgtcgccgcc     840
gtctccaccg ccgccgccgt ggctcctacc accaccaccg ctgcccccac caccttcgcc     900
acggccgtcg cctccaccaa gaaggccact gcctgccgca acaagaccaa gtcctcctcc     960
gctgccacca ccgccgccgc cgtcgccgag accacctctt ccaccgctgc cgccaccgct    1020
gctgcttcct ctgcctcttc cgcctccggc accgccggca agtacgagcg ctgcggtggc    1080
cagggctgga ccggtgccac cacctgcgtt gatggctgga cctgcaagca gtggaaccct    1140
tactactacc agtgcgttga gtctgcctag                                      1170
```

<210> 146
<211> 389
<212> PRT
<213> Aspergillus aculeatus

<400> 146

Met Lys Ser Ser Thr Phe Gly Met Leu Ala Leu Ala Ala Ala Ala Lys
1               5                   10                  15

Met Val Asp Ala His Thr Thr Val Phe Ala Val Trp Ile Asn Gly Glu
            20                  25                  30

Asp Gln Gly Leu Gly Asn Ser Ala Ser Gly Tyr Ile Arg Ser Pro Pro
        35                  40                  45

Ser Asn Ser Pro Val Lys Asp Val Thr Ser Thr Asp Ile Thr Cys Asn
    50                  55                  60

Val Asn Gly Asp Gln Ala Ala Ala Lys Thr Leu Ser Val Lys Gly Gly
65                  70                  75                  80

Asp Val Val Thr Phe Glu Trp His His Asp Ser Arg Asp Ala Ser Asp
                85                  90                  95

Asp Ile Ile Ala Ser Ser His Lys Gly Pro Val Met Val Tyr Met Ala
            100                 105                 110

Pro Thr Thr Ala Gly Ser Ser Gly Lys Asn Trp Val Lys Ile Ala Glu
        115                 120                 125

Asp Gly Tyr Ser Asp Gly Thr Trp Ala Val Asp Thr Leu Ile Ala Asn
    130                 135                 140

Ser Gly Lys His Asn Ile Thr Val Pro Asp Val Pro Ala Gly Asp Tyr
145                 150                 155                 160

Leu Phe Arg Pro Glu Ile Ile Ala Leu His Glu Ala Glu Asn Glu Gly
                165                 170                 175

Gly Ala Gln Phe Tyr Met Glu Cys Val Gln Phe Lys Val Thr Ser Asp
            180                 185                 190

Gly Ala Asn Thr Leu Pro Asp Gly Val Ser Leu Pro Gly Ala Tyr Ser
        195                 200                 205

Ala Thr Asp Pro Gly Ile Leu Phe Asn Met Tyr Gly Ser Phe Asp Ser
    210                 215                 220

Tyr Pro Ile Pro Gly Pro Ser Val Trp Asp Gly Thr Ser Ser Gly Ser

225 230 235 240

Ser Ser Ser Ser Ser Ser Ser Ser Ser Ser Ser Ser Ala Ala Ala Ala
245 250 255

Val Val Ala Thr Ser Ser Ser Ser Ser Ser Ala Ser Ile Glu Ala Val
260 265 270

Thr Thr Lys Gly Ala Val Ala Ala Val Ser Thr Ala Ala Ala Val Ala
275 280 285

Pro Thr Thr Thr Thr Ala Ala Pro Thr Thr Phe Ala Thr Ala Val Ala
290 295 300

Ser Thr Lys Lys Ala Thr Ala Cys Arg Asn Lys Thr Lys Ser Ser Ser
305 310 315 320

Ala Ala Thr Thr Ala Ala Ala Val Ala Glu Thr Thr Ser Ser Thr Ala
325 330 335

Ala Ala Thr Ala Ala Ala Ser Ser Ala Ser Ser Ala Ser Gly Thr Ala
340 345 350

Gly Lys Tyr Glu Arg Cys Gly Gly Gln Gly Trp Thr Gly Ala Thr Thr
355 360 365

Cys Val Asp Gly Trp Thr Cys Lys Gln Trp Asn Pro Tyr Tyr Tyr Gln
370 375 380

Cys Val Glu Ser Ala
385

<210> 147
<211> 1221
<212> DNA
<213> Aspergillus aculeatus

<400> 147

atgcgtcagg ctcagtcttt gtccctcttg acagctcttc tgtctgccac gcgtgtggct 60
ggacacggtc acgtcactaa cgttgtcgtc aacggtgttt actacgaggg cttcgatatc 120
aacagcttcc cctacgagtc cgatcccct aaggtggcgg cttggaccac tcctaacact 180
ggcaacggtt tcatttcccc cagcgactac ggtaccgatg acattatttg ccaccagaat 240
gccaccaacg cccaggccca cattgttgtt gcggctggtg acaagatcaa catccagtgg 300
accgcgtggc ccgattccca ccacggtcct gtccttgact acctcgctcg ctgcgacggt 360
gagtgtgaga cggttgataa gaccactctt gagtttttca agatcgacgg cgtcggtctc 420
atcagtgaca ccgaagtgcc cggtacctgg ggagatgacc agctgatcgc caacaacaac 480

agctggttgg tcgagatccc cccgaccatt gctcctggca actatgttct tcgccacgag 540
cttatcgctc tccacagcgc cggcactgaa gatggtgctc agaactaccc ccagtgtttc 600
aacctccagg tcactggctc cggtactgac gagcccgctg gtaccctcgg caccaagctc 660
tacactgagg atgaggctgg tatcgttgtg aacatctaca cctctctgtc ttcctatgcc 720
gtccccggcc ccacccagta cagcggcgcc gtctctgtca gccaatccac ttcggccatt 780
acctccaccg gaactgctgt tgtcggtagc ggcagcgctg ttgccacctc tgccgccgcg 840
gctaccacca gcgctgctgc ttcttctgcc gctgctgcta ccaccgctgc tgccgttacc 900
agcgccaatg ccaacactca gattgcccag cccagcagca gctcttctta ctcccagatc 960
gccgtgcagg tgccctcctc ctggaccacc cttgtgaccg tcactcctcc cgccgccgcc 1020
gccaccaccc ctgctgccgt ccctgagcct cagaccccct ctgccagctc tggagccacc 1080
actaccagca gcagcagcgg cgccgcccag tctctctacg gccagtgcgg tggtatcaac 1140
tggaccggag ctacctcttg cgttgagggc gctacttgct accagtacaa cccttactac 1200
taccagtgca tctctgccta a 1221

<210> 148
<211> 406
<212> PRT
<213> Aspergillus aculeatus

<400> 148

```
Met Arg Gln Ala Gln Ser Leu Ser Leu Leu Thr Ala Leu Leu Ser Ala
1               5                   10                  15

Thr Arg Val Ala Gly His Gly His Val Thr Asn Val Val Val Asn Gly
            20                  25                  30

Val Tyr Tyr Glu Gly Phe Asp Ile Asn Ser Phe Pro Tyr Glu Ser Asp
        35                  40                  45

Pro Pro Lys Val Ala Ala Trp Thr Thr Pro Asn Thr Gly Asn Gly Phe
    50                  55                  60

Ile Ser Pro Ser Asp Tyr Gly Thr Asp Asp Ile Ile Cys His Gln Asn
65                  70                  75                  80

Ala Thr Asn Ala Gln Ala His Ile Val Val Ala Ala Gly Asp Lys Ile
                85                  90                  95

Asn Ile Gln Trp Thr Ala Trp Pro Asp Ser His His Gly Pro Val Leu
            100                 105                 110

Asp Tyr Leu Ala Arg Cys Asp Gly Glu Cys Glu Thr Val Asp Lys Thr
        115                 120                 125
```

```
Thr Leu Glu Phe Phe Lys Ile Asp Gly Val Gly Leu Ile Ser Asp Thr
    130                 135                 140

Glu Val Pro Gly Thr Trp Gly Asp Asp Gln Leu Ile Ala Asn Asn Asn
145                 150                 155                 160

Ser Trp Leu Val Glu Ile Pro Pro Thr Ile Ala Pro Gly Asn Tyr Val
                165                 170                 175

Leu Arg His Glu Leu Ile Ala Leu His Ser Ala Gly Thr Glu Asp Gly
            180                 185                 190

Ala Gln Asn Tyr Pro Gln Cys Phe Asn Leu Gln Val Thr Gly Ser Gly
        195                 200                 205

Thr Asp Glu Pro Ala Gly Thr Leu Gly Thr Lys Leu Tyr Thr Glu Asp
    210                 215                 220

Glu Ala Gly Ile Val Val Asn Ile Tyr Thr Ser Leu Ser Ser Tyr Ala
225                 230                 235                 240

Val Pro Gly Pro Thr Gln Tyr Ser Gly Ala Val Ser Val Ser Gln Ser
                245                 250                 255

Thr Ser Ala Ile Thr Ser Thr Gly Thr Ala Val Val Gly Ser Gly Ser
            260                 265                 270

Ala Val Ala Thr Ser Ala Ala Ala Ala Thr Thr Ser Ala Ala Ala Ser
        275                 280                 285

Ser Ala Ala Ala Ala Thr Thr Ala Ala Ala Val Thr Ser Ala Asn Ala
    290                 295                 300

Asn Thr Gln Ile Ala Gln Pro Ser Ser Ser Ser Ser Tyr Ser Gln Ile
305                 310                 315                 320

Ala Val Gln Val Pro Ser Ser Trp Thr Thr Leu Val Thr Val Thr Pro
                325                 330                 335

Pro Ala Ala Ala Ala Thr Thr Pro Ala Ala Val Pro Glu Pro Gln Thr
            340                 345                 350

Pro Ser Ala Ser Ser Gly Ala Thr Thr Thr Ser Ser Ser Ser Gly Ala
        355                 360                 365

Ala Gln Ser Leu Tyr Gly Gln Cys Gly Gly Ile Asn Trp Thr Gly Ala
    370                 375                 380
```

```
Thr Ser Cys Val Glu Gly Ala Thr Cys Tyr Gln Tyr Asn Pro Tyr Tyr
385                 390                 395                 400

Tyr Gln Cys Ile Ser Ala
                405
```

<210> 149
<211> 1284
<212> DNA
<213> Aspergillus aculeatus

<400> 149

```
atgtctcttt ccaagattgc cactcttctg ctgggctcgg tctcgctggt cgctggtcat      60
gggtatgtct cgagcatcga ggtggacggt accacctatg gagggtactt ggtcgacact     120
tattactacg aatccgaccc gcccgagtta atcgcctggt ccacaaatgc cacggatgat     180
ggctatgtat cgccctccga ctacgagagc gtgaacatca tctgccacaa ggggtctgcg     240
cccggcgcgt tgtcggcccc tgtcgcgccc ggaggctggg tgcagatgac ctggaacacc     300
tggcccaccg accatcacgg ccctgtcatc acgtatatgg ccaattgcca cggttcttgc     360
gcagatgtgg acaagaccac cctcgagttc ttcaagatcg atgctggcgg cttgatcgat     420
gacacggacg tgcctggaac ttgggcgacc gatgagctca ttgaagatag ctatagtcgc     480
aacatcacta tccccagcga tattgccccc gggtactatg ttttgcgaca cgagatcatt     540
gctctgcaca gcgccgagaa cctggacgga gcccagaact acccccagtg catcaatctg     600
gaagtcaccg gcagcgagac agcaaccccg agtggcacct tgggcactgc tctgtacaag     660
gagaccgacc ccggcatcta tgttgacatc tggaacacgt tgagcacgta tactattccc     720
ggccccgcgc tgtacactgc tggtagcact gcgaccgcag ccgctgctgc cgataccacc     780
actacttctg ctggcaccac cgctgaggcc accaccgctg ccgccgccgt gagtaccacc     840
gcggacgctg ttccgaccga gtcttcagct ccttccgaga ccagcgcgac taccgcgaac     900
cctgctcggc ccactgccgg cagcgacatc cgcttccagc ccggtcaggt caaggctggt     960
gcttcagtca acaactcggc tactgagact tcctctggtg agtctgccac gacgaccaca    1020
acatcagtgg ccactgcggc ttcgagcgcg gattcgtcga cgacttctgg ggttttgagt    1080
ggcgcctgca gccaggaggg ctactggtac tgcaacgggg gcactgcgtt ccagcgctgt    1140
gtcaacgggg aatgggatgc gtcccagagt gtggctgcgg gcacggtctg caccgccggt    1200
atctcggaga ccatcaccat ttcagccgcc gccacgcgcc gggatgccat gcgtcgtcat    1260
ctggcgcgtc ccaagcgtca ctga                                           1284
```

<210> 150
<211> 427

<212> PRT
<213> Aspergillus aculeatus

<400> 150

```
Met Ser Leu Ser Lys Ile Ala Thr Leu Leu Leu Gly Ser Val Ser Leu
1               5                   10                  15

Val Ala Gly His Gly Tyr Val Ser Ser Ile Glu Val Asp Gly Thr Thr
            20                  25                  30

Tyr Gly Gly Tyr Leu Val Asp Thr Tyr Tyr Tyr Glu Ser Asp Pro Pro
        35                  40                  45

Glu Leu Ile Ala Trp Ser Thr Asn Ala Thr Asp Asp Gly Tyr Val Ser
    50                  55                  60

Pro Ser Asp Tyr Glu Ser Val Asn Ile Ile Cys His Lys Gly Ser Ala
65                  70                  75                  80

Pro Gly Ala Leu Ser Ala Pro Val Ala Pro Gly Gly Trp Val Gln Met
                85                  90                  95

Thr Trp Asn Thr Trp Pro Thr Asp His His Gly Pro Val Ile Thr Tyr
            100                 105                 110

Met Ala Asn Cys His Gly Ser Cys Ala Asp Val Asp Lys Thr Thr Leu
        115                 120                 125

Glu Phe Phe Lys Ile Asp Ala Gly Gly Leu Ile Asp Asp Thr Asp Val
    130                 135                 140

Pro Gly Thr Trp Ala Thr Asp Glu Leu Ile Glu Asp Ser Tyr Ser Arg
145                 150                 155                 160

Asn Ile Thr Ile Pro Ser Asp Ile Ala Pro Gly Tyr Tyr Val Leu Arg
                165                 170                 175

His Glu Ile Ile Ala Leu His Ser Ala Glu Asn Leu Asp Gly Ala Gln
            180                 185                 190

Asn Tyr Pro Gln Cys Ile Asn Leu Glu Val Thr Gly Ser Glu Thr Ala
        195                 200                 205

Thr Pro Ser Gly Thr Leu Gly Thr Ala Leu Tyr Lys Glu Thr Asp Pro
    210                 215                 220

Gly Ile Tyr Val Asp Ile Trp Asn Thr Leu Ser Thr Tyr Thr Ile Pro
225                 230                 235                 240

Gly Pro Ala Leu Tyr Thr Ala Gly Ser Thr Ala Thr Ala Ala Ala Ala
                245                 250                 255
```

```
Ala Asp Thr Thr Thr Thr Ser Ala Gly Thr Thr Ala Glu Ala Thr Thr
            260                 265                 270

Ala Ala Ala Ala Val Ser Thr Thr Ala Asp Ala Val Pro Thr Glu Ser
        275                 280                 285

Ser Ala Pro Ser Glu Thr Ser Ala Thr Thr Ala Asn Pro Ala Arg Pro
    290                 295                 300

Thr Ala Gly Ser Asp Ile Arg Phe Gln Pro Gly Gln Val Lys Ala Gly
305                 310                 315                 320

Ala Ser Val Asn Asn Ser Ala Thr Glu Thr Ser Ser Gly Glu Ser Ala
                325                 330                 335

Thr Thr Thr Thr Thr Ser Val Ala Thr Ala Ala Ser Ser Ala Asp Ser
            340                 345                 350

Ser Thr Thr Ser Gly Val Leu Ser Gly Ala Cys Ser Gln Glu Gly Tyr
        355                 360                 365

Trp Tyr Cys Asn Gly Gly Thr Ala Phe Gln Arg Cys Val Asn Gly Glu
    370                 375                 380

Trp Asp Ala Ser Gln Ser Val Ala Ala Gly Thr Val Cys Thr Ala Gly
385                 390                 395                 400

Ile Ser Glu Thr Ile Thr Ile Ser Ala Ala Ala Thr Arg Arg Asp Ala
                405                 410                 415

Met Arg Arg His Leu Ala Arg Pro Lys Arg His
            420                 425
```

<210> 151
<211> 804
<212> DNA
<213> Aspergillus aculeatus

<400> 151

```
atgcttgtca aactcatctc ttttctttca gctgctacca gcgtagctgc tcatggtcat      60
gtgtcaaaca ttgtgatcaa cggggtgtcc taccgcggat gggacatcaa ttcggaccct     120
tacaattcca accctccggt ggtggttgca tggcaaacac ccaacacagc taatggcttc     180
atctcccctg atgcatacga cacagatgat gttatttgcc atctgagcgc tacgaatgcc     240
agaggccacg cagtcgtcgc tgctggcgac aagatcagcc tccagtggac gacctggcct     300
gacagtcacc atggccctgt catcagctac ctagccaact gcggctccag ctgcgagaca     360
gtcgataaga ccaccctcga gttcttcaag atcgatggtg ttggcttggt ggatgagagc     420
aatcccccctg gtatctgggg agacgatgag ctcattgcca acaacaactc ttggctggta     480
gagattccag ctagtatcgc gccaggatac tatgtgctgc gtcacgagtt gatcgctctg     540
catggagcag ggagtgagaa tggagcccag aattacatgc aatgtttcaa ccttcaggtt     600
actgggactg gcacggtcca gccttccggg gtcctgggca cggagctgta caaacccaca     660
gacgctggaa ttcttgtcaa tatctaccag tcgctctcca cctatgttgt tcctggcccg     720
accctgatcc cccaggccgt ttccctcgtt cagtcgagct ccaccattac cgcctcgggc     780
acggcagtga caaccacggc ttga                                             804
```

<210> 152
<211> 267
<212> PRT
<213> Aspergillus aculeatus

<400> 152

```
Met Leu Val Lys Leu Ile Ser Phe Leu Ser Ala Ala Thr Ser Val Ala
1               5                   10                  15

Ala His Gly His Val Ser Asn Ile Val Ile Asn Gly Val Ser Tyr Arg
            20                  25                  30

Gly Trp Asp Ile Asn Ser Asp Pro Tyr Asn Ser Asn Pro Pro Val Val
        35                  40                  45

Val Ala Trp Gln Thr Pro Asn Thr Ala Asn Gly Phe Ile Ser Pro Asp
    50                  55                  60

Ala Tyr Asp Thr Asp Asp Val Ile Cys His Leu Ser Ala Thr Asn Ala
65                  70                  75                  80

Arg Gly His Ala Val Val Ala Ala Gly Asp Lys Ile Ser Leu Gln Trp
                85                  90                  95

Thr Thr Trp Pro Asp Ser His His Gly Pro Val Ile Ser Tyr Leu Ala
            100                 105                 110

Asn Cys Gly Ser Ser Cys Glu Thr Val Asp Lys Thr Thr Leu Glu Phe
        115                 120                 125

Phe Lys Ile Asp Gly Val Gly Leu Val Asp Glu Ser Asn Pro Pro Gly
    130                 135                 140

Ile Trp Gly Asp Asp Glu Leu Ile Ala Asn Asn Asn Ser Trp Leu Val
145                 150                 155                 160

Glu Ile Pro Ala Ser Ile Ala Pro Gly Tyr Tyr Val Leu Arg His Glu
                165                 170                 175
```

Leu Ile Ala Leu His Gly Ala Gly Ser Glu Asn Gly Ala Gln Asn Tyr
180 185 190

Met Gln Cys Phe Asn Leu Gln Val Thr Gly Thr Gly Thr Val Gln Pro
195 200 205

Ser Gly Val Leu Gly Thr Glu Leu Tyr Lys Pro Thr Asp Ala Gly Ile
210 215 220

Leu Val Asn Ile Tyr Gln Ser Leu Ser Thr Tyr Val Val Pro Gly Pro
225 230 235 240

Thr Leu Ile Pro Gln Ala Val Ser Leu Val Gln Ser Ser Ser Thr Ile
245 250 255

Thr Ala Ser Gly Thr Ala Val Thr Thr Thr Ala
260 265

<210> 153
<211> 822
<212> DNA
<213> Aspergillus aculeatus

<400> 153

atgaagtatc ttgcgatctt cgcggcagca gcagctggac tggcccgccc gacagcagcg 60

cactacatct tcagcaagct gattctggac ggcgaagtct ctgaggactg gcagtatatt 120

cgtaaaacca cccgggagac atgctatttg ccgaccaagt tcaccgacac cttcgacaac 180

ttgactccga acgaccagga tttccggtgc aatctcggct cgttcagcaa cgccgccaag 240

accgaagtgg ccgaggtgga agcgggctcc acgattggca tgcagctttt cgctggtagc 300

cacatgcgtc acccgggacc tgcgcaagtc ttcatgtcta aggccccgtc cggcaacgta 360

cagagctacg agggtgacgg ctcctggttc aagatctggg agcgtacact ctgcgacaaa 420

agtggcgatc tgactggaga tgcgtggtgt acatacggcc agaccgagat cgagtttcaa 480

atccccgagg cgaccccgac gggcgaatac ctggtccgag cggagcacat cggtcttcac 540

cgcgcacaga gtaatcaagc cgagttctac tacagctgcg cccaggtcaa ggtcacgggc 600

aatggtaccg ggtgccgag ccagacatat cagatccctg gcatgtacaa tgaccgctcg 660

gagcttttca acgggctgaa cttgtggtcc tactcggtgg agaacgtcga ggcagccatg 720

aagaattcta tcgtgggtga tgaaatttgg aatggaagtt ctgttccctc tgagtcccat 780

gtcccgaagt ataagaagag tcatgcttgt cgtgtttatt ga 822

<210> 154
<211> 273

<212> PRT
<213> Aspergillus aculeatus

<400> 154

```
Met Lys Tyr Leu Ala Ile Phe Ala Ala Ala Ala Ala Gly Leu Ala Arg
1               5                   10                  15

Pro Thr Ala Ala His Tyr Ile Phe Ser Lys Leu Ile Leu Asp Gly Glu
            20                  25                  30

Val Ser Glu Asp Trp Gln Tyr Ile Arg Lys Thr Thr Arg Glu Thr Cys
        35                  40                  45

Tyr Leu Pro Thr Lys Phe Thr Asp Thr Phe Asp Asn Leu Thr Pro Asn
    50                  55                  60

Asp Gln Asp Phe Arg Cys Asn Leu Gly Ser Phe Ser Asn Ala Ala Lys
65                  70                  75                  80

Thr Glu Val Ala Glu Val Glu Ala Gly Ser Thr Ile Gly Met Gln Leu
                85                  90                  95

Phe Ala Gly Ser His Met Arg His Pro Gly Pro Ala Gln Val Phe Met
            100                 105                 110

Ser Lys Ala Pro Ser Gly Asn Val Gln Ser Tyr Glu Gly Asp Gly Ser
        115                 120                 125

Trp Phe Lys Ile Trp Glu Arg Thr Leu Cys Asp Lys Ser Gly Asp Leu
    130                 135                 140

Thr Gly Asp Ala Trp Cys Thr Tyr Gly Gln Thr Glu Ile Glu Phe Gln
145                 150                 155                 160

Ile Pro Glu Ala Thr Pro Thr Gly Glu Tyr Leu Val Arg Ala Glu His
                165                 170                 175

Ile Gly Leu His Arg Ala Gln Ser Asn Gln Ala Glu Phe Tyr Tyr Ser
            180                 185                 190

Cys Ala Gln Val Lys Val Thr Gly Asn Gly Thr Gly Val Pro Ser Gln
        195                 200                 205

Thr Tyr Gln Ile Pro Gly Met Tyr Asn Asp Arg Ser Glu Leu Phe Asn
    210                 215                 220

Gly Leu Asn Leu Trp Ser Tyr Ser Val Glu Asn Val Glu Ala Ala Met
225                 230                 235                 240

Lys Asn Ser Ile Val Gly Asp Glu Ile Trp Asn Gly Ser Ser Val Pro
                245                 250                 255
```

Ser Glu Ser His Val Pro Lys Tyr Lys Lys Ser His Ala Cys Arg Val
260 265 270

Tyr

<210> 155
<211> 969
<212> DNA
<213> Aurantiporus alborubescens

<400> 155

atgcgaacca tcgccacgtt tgttacgctt gtagcctcag ttctccctgc ggtcctcgca 60

cacggaggtg tcctctccta ttcsaacggg gggaattggt actggggatg gaagccttac 120

aattcacctg acgggcagac caccatccaa cgcccgtggg caacatacaa tccgatcact 180

gatgcgacgg atcctaccat tgcttgcaac aacgacggga catctggagc tctgcagttg 240

actgcgacag tcgcggcggg atctgccatc acggcgtatt ggaaccaggt gtggccgcat 300

gataaagggc cgatgacgac atacctcgca caatgccccg gcagtacctg cacaggagtc 360

aacgcgaaga ctctgaaatg gttcaagatc gatcacgccg ggttgctttc tggtactgtc 420

tacagtggct cgtgggcatc aggcaagatg attgcacaga actcgacctg gacaactacc 480

attccagcga cggtgccttc agggaactat ctgatacgtt tcgagactat tgccctgcac 540

tctttgccag cgcaatttta ccctgagtgc gcacaaattc aaatcacggg cggaggttcc 600

cgtgctccaa ccgctgcaga gcttgttagc ttccctggcg cgtacagcaa caatgatcct 660

ggtgtcaaca ttgacatcta ctccaatgcc gcgcagagtg caaccacata cgtaatacca 720

ggacctccat tgtacggcgg tgcttccgga tctggtccat cttccgcgcc tccatcaagt 780

accccaggta gttcgtccac ttcccacggt cccacgtccg tcagcacgtc cagcagtgct 840

gcaccatcga cgacaggaac cgtgacgcag tacggtcagt gcggtggcat tggttgggct 900

ggagctaccg gctgtatctc accattcaag tgcacggtca tcaacgatta ttactaccag 960

tgcctctga 969

<210> 156
<211> 322
<212> PRT
<213> Aurantiporus alborubescens

<400> 156

Met Arg Thr Ile Ala Thr Phe Val Thr Leu Val Ala Ser Val Leu Pro
1               5                   10                  15

Ala Val Leu Ala His Gly Gly Val Leu Ser Tyr Ser Asn Gly Gly Asn
            20                  25                  30

Trp Tyr Trp Gly Trp Lys Pro Tyr Asn Ser Pro Asp Gly Gln Thr Thr

35 40 45

Ile Gln Arg Pro Trp Ala Thr Tyr Asn Pro Ile Thr Asp Ala Thr Asp
50 55 60

Pro Thr Ile Ala Cys Asn Asn Asp Gly Thr Ser Gly Ala Leu Gln Leu
65 70 75 80

Thr Ala Thr Val Ala Ala Gly Ser Ala Ile Thr Ala Tyr Trp Asn Gln
85 90 95

Val Trp Pro His Asp Lys Gly Pro Met Thr Thr Tyr Leu Ala Gln Cys
100 105 110

Pro Gly Ser Thr Cys Thr Gly Val Asn Ala Lys Thr Leu Lys Trp Phe
115 120 125

Lys Ile Asp His Ala Gly Leu Leu Ser Gly Thr Val Tyr Ser Gly Ser
130 135 140

Trp Ala Ser Gly Lys Met Ile Ala Gln Asn Ser Thr Trp Thr Thr Thr
145 150 155 160

Ile Pro Ala Thr Val Pro Ser Gly Asn Tyr Leu Ile Arg Phe Glu Thr
165 170 175

Ile Ala Leu His Ser Leu Pro Ala Gln Phe Tyr Pro Glu Cys Ala Gln
180 185 190

Ile Gln Ile Thr Gly Gly Gly Ser Arg Ala Pro Thr Ala Ala Glu Leu
195 200 205

Val Ser Phe Pro Gly Ala Tyr Ser Asn Asn Asp Pro Gly Val Asn Ile
210 215 220

Asp Ile Tyr Ser Asn Ala Ala Gln Ser Ala Thr Thr Tyr Val Ile Pro
225 230 235 240

Gly Pro Pro Leu Tyr Gly Gly Ala Ser Gly Ser Gly Pro Ser Ser Ala
245 250 255

Pro Pro Ser Ser Thr Pro Gly Ser Ser Ser Thr Ser His Gly Pro Thr
260 265 270

Ser Val Ser Thr Ser Ser Ser Ala Ala Pro Ser Thr Thr Gly Thr Val
275 280 285

Thr Gln Tyr Gly Gln Cys Gly Gly Ile Gly Trp Ala Gly Ala Thr Gly
290 295 300

Cys Ile Ser Pro Phe Lys Cys Thr Val Ile Asn Asp Tyr Tyr Tyr Gln
305 310 315 320

Cys Leu

<210> 157
<211> 705
<212> DNA
<213> Aurantiporus alborubescens

<400> 157

```
atgaaggcta tcttggctat tttctcggcc cttgctccac ttgccgctgc gcattatacc      60
ttccctgatt ttattgtcaa cggaacaaca actgccgatt gggtctacat ccgagagacc     120
gcgaaccact actcgaatgg tcctgtaacc aacgtgaacg atccagaatt ccgatgctac     180
gagctggacc tgcaaaacac ggcagcgagt accctcaccg ccacggtctc tgcaggctcc     240
agcgtcggct ttaaagctaa cagcgccctt taccatcctg gttatctcga tgtgtatatg     300
tccaaagcga ccccagctgc taattcaccc agtgctggaa cggaccaaag ctggttcaag     360
gtctatgaat ccgctccggt cttcgcgaat ggggccctaa gcttcccttc ggagaacatc     420
caatctttca cgttcacaat cccgaagtcc cttcccagtg gccaatatct catccgtgtg     480
gaacacatcg ctctccactc cgccagtagc tacggaggtg cacaattcta catcagctgc     540
gctcaagtca atgtcgtcaa cggcgggaac ggaaacccag gaccgttagt caagattccc     600
ggcgtttaca ctgggaacga gcctggcatc ctcatcaaca tctacagctt cccaccgggt     660
ttcagtggct accaatcccc gggacctgct gtgtggcgtg gttga                     705
```

<210> 158
<211> 234
<212> PRT
<213> Aurantiporus alborubescens

<400> 158

```
Met Lys Ala Ile Leu Ala Ile Phe Ser Ala Leu Ala Pro Leu Ala Ala
1               5                   10                  15

Ala His Tyr Thr Phe Pro Asp Phe Ile Val Asn Gly Thr Thr Thr Ala
            20                  25                  30

Asp Trp Val Tyr Ile Arg Glu Thr Ala Asn His Tyr Ser Asn Gly Pro
        35                  40                  45

Val Thr Asn Val Asn Asp Pro Glu Phe Arg Cys Tyr Glu Leu Asp Leu
    50                  55                  60

Gln Asn Thr Ala Ala Ser Thr Leu Thr Ala Thr Val Ser Ala Gly Ser

65                  70                  75                  80

Ser Val Gly Phe Lys Ala Asn Ser Ala Leu Tyr His Pro Gly Tyr Leu
                85                  90                  95

Asp Val Tyr Met Ser Lys Ala Thr Pro Ala Ala Asn Ser Pro Ser Ala
            100                 105                 110

Gly Thr Asp Gln Ser Trp Phe Lys Val Tyr Glu Ser Ala Pro Val Phe
        115                 120                 125

Ala Asn Gly Ala Leu Ser Phe Pro Ser Glu Asn Ile Gln Ser Phe Thr
    130                 135                 140

Phe Thr Ile Pro Lys Ser Leu Pro Ser Gly Gln Tyr Leu Ile Arg Val
145                 150                 155                 160

Glu His Ile Ala Leu His Ser Ala Ser Ser Tyr Gly Gly Ala Gln Phe
                165                 170                 175

Tyr Ile Ser Cys Ala Gln Val Asn Val Val Asn Gly Gly Asn Gly Asn
            180                 185                 190

Pro Gly Pro Leu Val Lys Ile Pro Gly Val Tyr Thr Gly Asn Glu Pro
        195                 200                 205

Gly Ile Leu Ile Asn Ile Tyr Ser Phe Pro Pro Gly Phe Ser Gly Tyr
    210                 215                 220

Gln Ser Pro Gly Pro Ala Val Trp Arg Gly
225                 230
```

<210> 159
<211> 702
<212> DNA
<213> Trichophaea saccata

<400> 159

```
atgacgcccc tgaaactccg cccccttctc ctcctggtgc tttccacgac cctcagcctc      60
gtgcacgcgc actatcgctt ctacgaactg atcgccaacg gggccaccca cgcttccttc     120
gaatacatcc gccaatgggt gcccatctac agcaactctc ccgtaaccga cgtcaccagc     180
gtcaacctcc gctgcaacgt caacgccact cccgccgccg aggtgatcac cgttgctgcc     240
ggtagcaccg tcggcttcgt agcagacaca acagtaacgc accccggtgc gttcaccgcg     300
tacatggcga aagcgcccga agacatcacg gaatgggatg gcaacgggga ctggttcaag     360
atctgggaga agggtccaac gagtataacc agtagcggga taacctggga cgtcacggat     420
acccaatgga ccttcaccat cccttccgcg acaccaaacg gtcaatacct actccgcttc     480
gagcacatag cgctccacgc cgccagcacc gtgggggtg ctcaattcta catgtcgtgc      540
gcgcagatac aagtaacgaa cggcggcaac gggagtcccg ggcccaccat caagttcccg     600
ggcggataca gcgccacaga ccccggtatc ctgatcaata tctattatcc catccccact     660
agttacacta ttcctggtcc accggtttgg accggtaagt aa                        702
```

<210> 160
<211> 233
<212> PRT
<213> Trichophaea saccata

<400> 160

```
Met Thr Pro Leu Lys Leu Arg Pro Leu Leu Leu Leu Val Leu Ser Thr
1               5                   10                  15

Thr Leu Ser Leu Val His Ala His Tyr Arg Phe Tyr Glu Leu Ile Ala
            20                  25                  30

Asn Gly Ala Thr His Ala Ser Phe Glu Tyr Ile Arg Gln Trp Val Pro
        35                  40                  45

Ile Tyr Ser Asn Ser Pro Val Thr Asp Val Thr Ser Val Asn Leu Arg
    50                  55                  60

Cys Asn Val Asn Ala Thr Pro Ala Ala Glu Val Ile Thr Val Ala Ala
65                  70                  75                  80

Gly Ser Thr Val Gly Phe Val Ala Asp Thr Thr Val Thr His Pro Gly
                85                  90                  95

Ala Phe Thr Ala Tyr Met Ala Lys Ala Pro Glu Asp Ile Thr Glu Trp
            100                 105                 110

Asp Gly Asn Gly Asp Trp Phe Lys Ile Trp Glu Lys Gly Pro Thr Ser
        115                 120                 125

Ile Thr Ser Ser Gly Ile Thr Trp Asp Val Thr Asp Thr Gln Trp Thr
    130                 135                 140

Phe Thr Ile Pro Ser Ala Thr Pro Asn Gly Gln Tyr Leu Leu Arg Phe
145                 150                 155                 160

Glu His Ile Ala Leu His Ala Ala Ser Thr Val Gly Gly Ala Gln Phe
                165                 170                 175

Tyr Met Ser Cys Ala Gln Ile Gln Val Thr Asn Gly Gly Asn Gly Ser
            180                 185                 190

Pro Gly Pro Thr Ile Lys Phe Pro Gly Gly Tyr Ser Ala Thr Asp Pro


        195                 200                 205

Gly Ile Leu Ile Asn Ile Tyr Tyr Pro Ile Pro Thr Ser Tyr Thr Ile
    210                 215                 220

Pro Gly Pro Pro Val Trp Thr Gly Lys
225                 230
```

<210> 161

<211> 714
<212> DNA
<213> Trichophaea saccata

<400> 161

```
atgaaatgcc ttctctccct ccttctcgcc gcgacagcgg tctccgctca cacgatcttc      60
caagaaatcg gcataaacgg ggtgatgcaa gctcgctacg actacatgcg gctgccgtcc     120
tacgacggtc ccattacgga cgtaacgagc acctacatgg cgtgcaacgg tggtcccaat     180
ccattggtcc aaatctcgaa cgacgtcgct ttcgtaaaag ccggcgacag catcacgctg     240
caatgggcgc aaacgttgac gacagatttc aacacggggc tgatcatcga tccatcgcac     300
ttgggtcctg tgatggtcta catggccaaa gtaccctccg ccaccggtcc gatccccaac     360
agcggctggt tcaaaatcta cgaagacggc tacgacccga caacaaagac atgggcggta     420
accaagctca tcaacaacaa gggaaaagtg accgtcacca tcccatcgtg tctaccggca     480
ggggactact tgctgcgcgg tgaaatcatt gccttgcacg cggctagtac ctatccaggc     540
gcacagtttt acatggagtg tgcgcagttg cggcttacca gtggcggcac taagatgcct     600
accacgtata acattccggg gatctattcg cccactgatc cgggtgttac gttcaatctt     660
tacaatggat tcacgagtta taccattcct ggcccaaggc cgtttacatg ctag           714
```

<210> 162
<211> 237
<212> PRT
<213> Trichophaea saccata

<400> 162

```
Met Lys Cys Leu Leu Ser Leu Leu Leu Ala Ala Thr Ala Val Ser Ala
1               5                   10                  15

His Thr Ile Phe Gln Glu Ile Gly Ile Asn Gly Val Met Gln Ala Arg
            20                  25                  30

Tyr Asp Tyr Met Arg Leu Pro Ser Tyr Asp Gly Pro Ile Thr Asp Val
        35                  40                  45

Thr Ser Thr Tyr Met Ala Cys Asn Gly Gly Pro Asn Pro Leu Val Gln
    50                  55                  60
```

```
Ile Ser Asn Asp Val Ala Phe Val Lys Ala Gly Asp Ser Ile Thr Leu
65                  70                  75                  80

Gln Trp Ala Gln Thr Leu Thr Thr Asp Phe Asn Thr Gly Leu Ile Ile
                85                  90                  95

Asp Pro Ser His Leu Gly Pro Val Met Val Tyr Met Ala Lys Val Pro
            100                 105                 110

Ser Ala Thr Gly Pro Ile Pro Asn Ser Gly Trp Phe Lys Ile Tyr Glu
        115                 120                 125

Asp Gly Tyr Asp Pro Thr Thr Lys Thr Trp Ala Val Thr Lys Leu Ile
    130                 135                 140

Asn Asn Lys Gly Lys Val Thr Val Thr Ile Pro Ser Cys Leu Pro Ala
145                 150                 155                 160

Gly Asp Tyr Leu Leu Arg Gly Glu Ile Ile Ala Leu His Ala Ala Ser
                165                 170                 175

Thr Tyr Pro Gly Ala Gln Phe Tyr Met Glu Cys Ala Gln Leu Arg Leu
            180                 185                 190

Thr Ser Gly Gly Thr Lys Met Pro Thr Thr Tyr Asn Ile Pro Gly Ile
        195                 200                 205

Tyr Ser Pro Thr Asp Pro Gly Val Thr Phe Asn Leu Tyr Asn Gly Phe
    210                 215                 220

Thr Ser Tyr Thr Ile Pro Gly Pro Arg Pro Phe Thr Cys
225                 230                 235
```

<210> 163
<211> 1455
<212> DNA
<213> Penicillium thomii

<400> 163

```
atgtctctgt ctaagatttc tggattgatc ctcggatctg ctgccttggt ggctggccac      60
ggttacgtga gcggaatcgt cgttgacgat acctactatg gtggatacct tgtcacccag     120
tacccttatg agagtgacgc cccagagctc attgcctggt cggagcaaga gaccgatctg     180
ggttacatcg atggctctga gtatgccaac tccaacatca tctgtcacaa ggaggccaaa     240
cctggtgctt tggaagcacc cgttaaggct ggtggctccg tcgagctcca gtggaccact     300
tggcctacca gccaccacgg tcctgtcatt acctacatgg ccaactgtaa cggcgactgt     360
gacgacgttg acaagactac tttgcagttc ttcaagattg accagggtgg tttgatcagc     420
gataccaccg agcccggtac ctgggcaact gacaacctca tcgccaacaa caatagccgt     480

actgtcaccg tccccagcga cattgccgat ggaaactacg tcctccgtca cgagatcatt     540
gccctccact ccgccgggga gaccaacggt gcccagaact acccccaatg tatcaacttg     600
aaggtcactg gcggcggtag cgctactcct tctggtaccc tgggtaccgc cctgtacaag     660
aacaccgacc ccggtatcct gatcaacatc tacacttccc tcagcaccta cgatatcccc     720
ggcccaaccc tgtacactgc cggcgccgcc gctgctaccg ctgcctccac ggctgcctct     780
tccaccgccg ctgccgttac tactgccgac gccgtcacta ccgccgctgc cgtcaccagc     840
agctctgcat ccgtggaagt tgtgcccaca actactccca gctcatcaat cgtcagtgcc     900
ttcccaacct ggagcccctc ttctacccca cccttctcca actcttccaa cggatggcgt     960
ccgtcattca gccgcggacc tggtggcccc cgcttcacat ctgctcctgc tcctcagttc    1020
tccgctccta gcggcgctca gcagaagcag tctgccactg ctacccccat cgtggctacc    1080
cctgtcgtga tcaccatgac cgagaccagc acctcctggg tcaccgaaat ggttactctt    1140
actgacaagt ctgttgtgca gaccaccagc gctgtcccag tcgtcgtcgc cgccaccact    1200
acccttaccg agggaagcga gcctgctcag acagcctccc ccagcgttgt ctccggctcc    1260
tctagctccg gctctagctc ctcatctacc accaccacct caaagacctc aactggatcc    1320
gactacgtct ccagcgactg gatgtcttac ctcagctcct tgagcgctgc tgaggtcctc    1380
cagatgctgc gccagacctt ccgttggatg gtcagcaacg acaaggtgca cgctcgtgat    1440
attaccatca actag                                                     1455
```

<210> 164
<211> 484
<212> PRT
<213> Penicillium thomii

<400> 164

```
Met Ser Leu Ser Lys Ile Ser Gly Leu Ile Leu Gly Ser Ala Ala Leu
1               5                   10                  15

Val Ala Gly His Gly Tyr Val Ser Gly Ile Val Val Asp Asp Thr Tyr
            20                  25                  30

Tyr Gly Gly Tyr Leu Val Thr Gln Tyr Pro Tyr Glu Ser Asp Ala Pro
        35                  40                  45

Glu Leu Ile Ala Trp Ser Glu Gln Glu Thr Asp Leu Gly Tyr Ile Asp
    50                  55                  60

Gly Ser Glu Tyr Ala Asn Ser Asn Ile Ile Cys His Lys Glu Ala Lys
65                  70                  75                  80

Pro Gly Ala Leu Glu Ala Pro Val Lys Ala Gly Gly Ser Val Glu Leu
                85                  90                  95
```

```
Gln Trp Thr Thr Trp Pro Thr Ser His His Gly Pro Val Ile Thr Tyr
            100                 105                 110

Met Ala Asn Cys Asn Gly Asp Cys Asp Asp Val Asp Lys Thr Thr Leu
        115                 120                 125

Gln Phe Phe Lys Ile Asp Gln Gly Gly Leu Ile Ser Asp Thr Thr Glu
    130                 135                 140

Pro Gly Thr Trp Ala Thr Asp Asn Leu Ile Ala Asn Asn Asn Ser Arg
145                 150                 155                 160

Thr Val Thr Val Pro Ser Asp Ile Ala Asp Gly Asn Tyr Val Leu Arg
                165                 170                 175

His Glu Ile Ile Ala Leu His Ser Ala Gly Glu Thr Asn Gly Ala Gln
            180                 185                 190

Asn Tyr Pro Gln Cys Ile Asn Leu Lys Val Thr Gly Gly Gly Ser Ala
        195                 200                 205

Thr Pro Ser Gly Thr Leu Gly Thr Ala Leu Tyr Lys Asn Thr Asp Pro
    210                 215                 220

Gly Ile Leu Ile Asn Ile Tyr Thr Ser Leu Ser Thr Tyr Asp Ile Pro
225                 230                 235                 240

Gly Pro Thr Leu Tyr Thr Ala Gly Ala Ala Ala Ala Thr Ala Ala Ser
                245                 250                 255

Thr Ala Ala Ser Ser Thr Ala Ala Ala Val Thr Thr Ala Asp Ala Val
            260                 265                 270

Thr Thr Ala Ala Ala Val Thr Ser Ser Ser Ala Ser Val Glu Val Val
        275                 280                 285

Pro Thr Thr Thr Pro Ser Ser Ser Ile Val Ser Ala Phe Pro Thr Trp
    290                 295                 300

Ser Pro Ser Ser Thr Pro Pro Phe Ser Asn Ser Ser Asn Gly Trp Arg
305                 310                 315                 320

Pro Ser Phe Ser Arg Gly Pro Gly Gly Pro Arg Phe Thr Ser Ala Pro
                325                 330                 335

Ala Pro Gln Phe Ser Ala Pro Ser Gly Ala Gln Gln Lys Gln Ser Ala
            340                 345                 350
```

Thr Ala Thr Pro Ile Val Ala Thr Pro Val Val Ile Thr Met Thr Glu
355 360 365

Thr Ser Thr Ser Trp Val Thr Glu Met Val Thr Leu Thr Asp Lys Ser
370 375 380

Val Val Gln Thr Thr Ser Ala Val Pro Val Val Val Ala Ala Thr Thr
385 390 395 400

Thr Leu Thr Glu Gly Ser Glu Pro Ala Gln Thr Ala Ser Pro Ser Val
405 410 415

Val Ser Gly Ser Ser Ser Ser Gly Ser Ser Ser Ser Ser Thr Thr Thr
420 425 430

Thr Ser Lys Thr Ser Thr Gly Ser Asp Tyr Val Ser Ser Asp Trp Met
435 440 445

Ser Tyr Leu Ser Ser Leu Ser Ala Ala Glu Val Leu Gln Met Leu Arg
450 455 460

Gln Thr Phe Arg Trp Met Val Ser Asn Asp Lys Val His Ala Arg Asp
465 470 475 480

Ile Thr Ile Asn

<210> 165
<211> 1021
<212> DNA
<213> Talaromyces stipitatus

<400> 165

atgccttcca ctaaagttgc tgctctatct gccgtcctgg ctttggcctc cacggttgct 60
ggccatggct ttgtgcaaaa tattgtcatt gacggtaaat cgtaagtgac ttgcttttgt 120
actatagagc tagataaata cttatactaa ataattcagc tacactggct acctcgtgaa 180
ccagtatcct taccagtcca acccaccagc tgttattggg tggtcaacca ctgcaaccga 240
cttgggattt gtcgatggat ctggatacac caacccggat atcatctgcc acaaaaacgc 300
caaacccggt cagctttctg ctccggttgc cgcaggaggc aaggttgagc tcgaatggac 360
aacatggccc gagagccatc acggccctgt catcagctat ctcgccaatt gcaatggcga 420
ttgtactacc gtggataaga cgaagctcga atttgtcaaa atcgatcagc ggggtctgat 480
cgacgacagc aatcctcccg gtacatgggc cgccgaccag ctcatcgccg ccaacaacag 540
ctggactgta actattcccg agagcatcgc gcctggaaac tacgtccttc gccacgaaat 600
catcgctctt cactccgcca acaacgcaac cggagctcaa aactaccctc aatgcatcaa 660
cttgcaaatc actggcagcg ggacggccaa cccatctggt acccctggcg agaaactcta 720
taccccaact gacccaggta tcttggtcaa catctaccag tcattgtcgt cttatgttat 780
tcccggtccg actttgtgga gtggtgctgc agcgcacgtt gttgccactg cagccggttc 840
tgctactggg gttgcttctg ccaccgctac tccgaccact cttgtgactg ccgtttcatc 900
gcctaccggt gctccttcag tggtgactcc tgaggctcct tcagtaacct cgttcgcccc 960
agtggtgact gttactgatg tcgttactgt gactaccgtc atcactacta ctatctctta 1020
g 1021

<210> 166
<211> 320
<212> PRT
<213> Talaromyces stipitatus

<400> 166

```
Met Pro Ser Thr Lys Val Ala Ala Leu Ser Ala Val Leu Ala Leu Ala
1               5                   10                  15

Ser Thr Val Ala Gly His Gly Phe Val Gln Asn Ile Val Ile Asp Gly
            20                  25                  30

Lys Ser Tyr Thr Gly Tyr Leu Val Asn Gln Tyr Pro Tyr Gln Ser Asn
        35                  40                  45

Pro Pro Ala Val Ile Gly Trp Ser Thr Thr Ala Thr Asp Leu Gly Phe
    50                  55                  60

Val Asp Gly Ser Gly Tyr Thr Asn Pro Asp Ile Ile Cys His Lys Asn
65                  70                  75                  80

Ala Lys Pro Gly Gln Leu Ser Ala Pro Val Ala Ala Gly Gly Lys Val
                85                  90                  95

Glu Leu Glu Trp Thr Thr Trp Pro Glu Ser His His Gly Pro Val Ile
            100                 105                 110

Ser Tyr Leu Ala Asn Cys Asn Gly Asp Cys Thr Thr Val Asp Lys Thr
        115                 120                 125

Lys Leu Glu Phe Val Lys Ile Asp Gln Arg Gly Leu Ile Asp Asp Ser
    130                 135                 140

Asn Pro Pro Gly Thr Trp Ala Ala Asp Gln Leu Ile Ala Ala Asn Asn
145                 150                 155                 160

Ser Trp Thr Val Thr Ile Pro Glu Ser Ile Ala Pro Gly Asn Tyr Val
                165                 170                 175
```

```
Leu Arg His Glu Ile Ile Ala Leu His Ser Ala Asn Asn Ala Thr Gly
            180                 185                 190

Ala Gln Asn Tyr Pro Gln Cys Ile Asn Leu Gln Ile Thr Gly Ser Gly
        195                 200                 205

Thr Ala Asn Pro Ser Gly Thr Pro Gly Glu Lys Leu Tyr Thr Pro Thr
    210                 215                 220

Asp Pro Gly Ile Leu Val Asn Ile Tyr Gln Ser Leu Ser Ser Tyr Val
225                 230                 235                 240

Ile Pro Gly Pro Thr Leu Trp Ser Gly Ala Ala Ala His Val Val Ala
                245                 250                 255

Thr Ala Ala Gly Ser Ala Thr Gly Val Ala Ser Ala Thr Ala Thr Pro
            260                 265                 270

Thr Thr Leu Val Thr Ala Val Ser Ser Pro Thr Gly Ala Pro Ser Val
        275                 280                 285

Val Thr Pro Glu Ala Pro Ser Val Thr Ser Phe Ala Pro Val Val Thr
    290                 295                 300

Val Thr Asp Val Val Thr Val Thr Thr Val Ile Thr Thr Thr Ile Ser
305                 310                 315                 320
```

## Description

This invention was made with Government support under Cooperative Agreement DE-FC36-08GO18080 awarded by the Department of Energy. The government has certain rights in this invention.

## Reference to a Sequence Listing

This patent contains a Sequence Listing.

## Background of the Invention

### Field of the Invention

The present invention relates to methods for degrading or converting a cellulosic material; methods for producing a fermentation product from cellulosic material; and methods of fermenting a cellulosic material.

### Description of the Related Art

Cellulose is a polymer of the simple sugar glucose covalently linked by beta-1,4-bonds. Many microorganisms produce enzymes that hydrolyze beta-linked glucans. These enzymes include endoglucanases, cellobiohydrolases, and beta-glucosidases. Endoglucanases digest the cellulose polymer at random locations, opening it to attack by cellobiohydrolases. Cellobiohydrolases sequentially release molecules of cellobiose from the ends of the cellulose polymer. Cellobiose is a water-soluble beta-1,4-linked dimer of glucose. Beta-glucosidases hydrolyze cellobiose to glucose.

The conversion of lignocellulosic feedstocks into ethanol has the advantages of the ready availability of large amounts of feedstock, the desirability of avoiding burning or land filling the materials, and the cleanliness of the ethanol fuel. Wood, agricultural residues, herbaceous crops, and municipal solid wastes have been considered as feedstocks for ethanol production. These materials primarily consist of cellulose, hemicellulose, and lignin. Once the lignocellulose is converted to fermentable sugars, *e.g.,* glucose, the fermentable sugars are easily fermented by yeast into ethanol.

WO 2005/074647, WO 2008/148131, WO 2011/035027 disclose isolated GH61 polypeptides having cellulolytic enhancing activity and the polynucleotides thereof from *Thielavia terrestris.* WO 2005/074656 and WO 2010/065830 disclose isolated GH61 polypeptides having cellulolytic enhancing activity and the polynucleotides thereof from *Thermoascus aurantiacus.* WO 2007/089290 discloses an isolated GH61 polypeptide having cellulolytic enhancing activity and the polynucleotide thereof from *Trichoderma reesei.* WO 2009/085935, WO 2009/085859, WO 2009/085864, and WO 2009/085868 disclose isolated GH61 polypeptides having cellulolytic enhancing activity and the polynucleotides thereof from *Myceliophthora thermophila.* WO 2010/138754 discloses isolated GH61 polypeptides having cellulolytic enhancing activity and the polynucleotides thereof from *Aspergillus fumigatus.* WO 2011/005867 discloses isolated GH61 polypeptides having cellulolytic enhancing activity and the polynucleotides thereof from *Penicillium pinophilum.* WO 2011/039319 discloses isolated GH61 polypeptides having cellulolytic enhancing activity and the polynucleotides thereof from *Thermoascus* sp. WO 2011/041397 discloses isolated GH61 polypeptides having cellulolytic enhancing activity and the polynucleotides thereof from *Penicillium* sp. WO 2011/041504 discloses isolated GH61 polypeptides having cellulolytic enhancing activity and the polynucleotides thereof from *Thermoascus crustaceous.* WO 2008/151043 discloses methods of increasing the activity of a GH61 polypeptide having cellulolytic enhancing activity by adding a soluble activating divalent metal cation to a composition comprising the polypeptide.

It would be advantageous in the art to improve the ability of polypeptides having cellulolytic enhancing activity to enhance enzymatic hydrolysis of lignocellulosic feedstocks.

## Summary of the Invention

The present invention relates to methods for degrading or converting a cellulosic material, comprising: treating the cellulosic material with an enzyme composition comprising a cellulase in the presence of a GH61 polypeptide having cellulolytic enhancing activity and a liquor arising from treatment of a lignocellulose material, a hemicellulose material, a lignacious material, monosaccharides of the lignocellulose material, monosaccharides of the hemicellulose material, or a combination thereof, wherein the liquor is present in an amount that minimizes inhibition of a cellulase composition of 1 to 20% (v/v), wherein the combination of the GH61 polypeptide having cellulolytic enhancing activity and the liquor enhances hydrolysis of the cellulosic material by the enzyme composition, wherein the enhanced hydrolysis is assessed according to Equation 3:

$$GH61\ effect = \frac{\%\ conversion_{(+\ GH61+\ liquor)}}{\%\ conversion_{(no\ GH61+\ liquor)}} \quad \text{(Equation 3)}$$

and the equation ratio is greater than 1, wherein the GH61 polypeptide having cellulolytic enhancing activity comprises the following motifs: [ILMV]-P-X(4,5)-G-X-Y-[ILMV]-X-R-X-[EQ]-X(4)-[HNQ] (SEQ ID NO: 127 or SEQ ID NO: 128) and [FW]-[TF]-K-[AIV], wherein X is any amino acid, X(4,5) is any amino acid at 4 or 5 contiguous positions, and X(4) is any amino acid at 4 contiguous positions.

The present invention also relates to methods for producing a fermentation product, comprising:

(a) saccharifying a cellulosic material with an enzyme composition comprising a cellulase in the presence of a GH61 polypeptide having cellulolytic enhancing activity and a liquor arising from treatment of a lignocellulose material, a hemicellulose material, a lignacious material, monosaccharides of the lignocellulose material, monosaccharides of the hemicellulose material, or a combination thereof, wherein the liquor is present in an amount that minimizes inhibition of a cellulase composition of about 1 to about 20% (v/v), wherein the liquor is present in an amount that minimizes inhibition of a cellulase composition of 1 to 20% (v/v), wherein the combination of the GH61 polypeptide having cellulolytic enhancing activity and the liquor enhances hydrolysis of the cellulosic material by the enzyme composition, wherein the enhanced hydrolysis is assessed according to Equation 3:

$$GH61\ effect = \frac{\%\ conversion_{(+\ GH61+\ liquor)}}{\%\ conversion_{(no\ GH61+\ liquor)}} \quad \text{(Equation 3)}$$

and the equation ratio is greater than 1, wherein the polypeptide having cellulolytic enhancing activity comprises the following motifs: [ILMV]-P-X(4,5)-G-X-Y-[ILMV]-X-R-X-[EQ]-X(4)-[HNQ] (SEQ ID NO: 127 or SEQ ID NO: 128) and [FW]-[TF]-K-[AIV], wherein X is any amino acid, X(4,5) is any amino acid at 4 or 5 contiguous positions, and X(4) is any amino acid at 4 contiguous positions;
(b) fermenting the saccharified cellulosic material with one or more fermenting microorganisms to produce the fermentation product; and
(c) recovering the fermentation product from the fermentation.

The present invention also relates to methods of fermenting a cellulosic material, comprising:

(a) saccharifying a cellulosic material with an enzyme composition comprising a cellulase in the presence of a GH61 polypeptide having cellulolytic enhancing activity and a liquor arising from treatment of a lignocellulose material, a hemicellulose material, a lignacious material, monosaccharides of the lignocellulose material, monosaccharides of the hemicellulose material, or a combination thereof, wherein the liquor is present in an amount that minimizes inhibition of a cellulase composition of about 1 to about 20% (v/v), wherein the liquor is present in an amount that minimizes inhibition of a cellulase composition of 1 to 20% (v/v), wherein the combination of the GH61 polypeptide having cellulolytic enhancing activity and the liquor enhances hydrolysis of the cellulosic material by the enzyme composition, wherein the enhanced hydrolysis is assessed according to Equation 3:

$$GH61\ effect = \frac{\%\ conversion_{(+\ GH61+\ liquor)}}{\%\ conversion_{(no\ GH61+\ liquor)}} \quad \text{(Equation 3)}$$

and the equation ratio is greater than 1, wherein the polypeptide having cellulolytic enhancing activity comprises the following motifs: [ILMV]-P-X(4,5)-G-X-Y-[ILMV]-X-R-X-[EQ]-X(4)-[HNQ] (SEQ ID NO: 127 or SEQ ID NO: 128) and [FW]-[TF]-K-[AIV], wherein X is any amino acid, X(4,5) is any amino acid at 4 or 5 contiguous positions, and X(4) is any amino acid at 4 contiguous positions;
(b) fermenting the saccharified cellulosic material with one or more fermenting microorganisms to produce the fermentation product; and
(c) recovering the fermentation product from the fermentation.

**Brief Description of the Figures**

Figure 1 shows the fractional hydrolysis of washed and unwashed pretreated corn stover by a *Trichoderma reesei* cellulase composition with various concentrations of *Thermoascus aurantiacus* GH61A polypeptide having cellulolytic enhancement activity. Open symbols: 1 day of hydrolysis; closed symbols: 3 days of hydrolysis; squares: milled, water-washed pretreated corn stover; circles: milled, unwashed pretreated corn stover; triangles: hot-water washed, milled pretreated corn stover.

Figures 2A and 2B show the effect of acid-pretreated corn stover liquor on GH61 polypeptide-enhancement of cellulolysis of milled, water-washed pretreated corn stover by a *T. reesei* cellulase composition. Panel A: fractional hydrolysis. White bars: 1 day hydrolysis; gray bars: 3 day hydrolysis. Panel B: open symbols, 1 day hydrolysis; solid symbols: 3 day hydrolysis. Circles: no added liquor; squares: 2% (v/v) liquor; diamonds: 5% (v/v) liquor; triangles: 10% liquor; inverted triangles: 15% liquor. Data were fit linearly or by a modified saturation-binding model as described.

Figures 3A and 3B show acid-pretreated corn stover liquor dependence of *T. aurantiacus* GH61A polypeptide-enhancement of hydrolysis of pretreated corn stover. Panel A: AVICEL® + various concentrations of the acid-pretreated corn stover liquor as a function of GH61 polypeptide concentrations. Panel B: AVICEL® + synthetic liquor as a function of GH61 polypeptide concentration. Open symbols: 1 day hydrolysis; closed symbols: 3 days hydrolysis. Circles: no liquor; diamonds: 5% (v/v) liquor; triangles: 10% liquor; inverted triangles: 15% liquor; squares: 5% synthetic liquor containing no phenol; right triangles: 15% synthetic liquor containing no phenol. Data were fit linearly or using Equation 2, as described.

Figures 4A, 4B, and 4C show the effect of enzymatically treated or not enzymatically-treated pretreated corn stover liquors on enhancement of cellulolysis of pretreated corn stover by the *Thelavia terrestris* GH61E polypeptide. Panel A: un-treated liquor; Panel B: *T. reesei* cellulase-treated liquor; and Panel C: *T. reesei* cellulase and *Thelavia terrestris* GH61E polypeptide-treated liquor. Circles: no added liquor; squares: 5% (v/v) liquor; diamonds: 10% (v/v) liquor; triangles: 15% (v/v) liquor. Data were fit linearly or using Equation 2, as described.

Figures 5A and 5B show the fractional hydrolysis of microcrystalline cellulose by the *T. reesei* cellulase composition with various *T. aurantiacus* GH61A polypeptide concentrations, comparing impact of addition of dilute-acid and steam pretreatment liquors at 5 days of hydrolysis. Panel A: NREL acid-pretreated corn stover liquor; Panel B: steam explosion-pretreated corn stover liquor. Open symbols: 5% (v/v) liquor; closed symbols: 15% (v/v) liquor. Circles: whole liquor; squares: low molecular weight fraction, triangles: high molecular weight fraction. Data were fit using Equation 2, as described.

Figure 6 shows the effect of retentates and flow-through samples of molecular weight-filtered acid-pretreated corn stover liquor on GH61 polypeptide cellulolytic enhancing activity. White bars: 1 day of saccharification; gray bars: 6 days of saccharification. Concentrations listed refer to the *T. reesei* cellulase composition and the GH61 polypeptide concentration, respectively, in mg per gram cellulose.

Figure 7 shows (A) the fractional hydrolysis of microcrystalline cellulose by individual *T. reesei* cellulase monocomponents and mixtures thereof, and the effects of the *T. aurantiacus* GH61A polypeptide and NREL acid-pretreated corn stover liquor thereon and (B) the *GH61 effect* on the individual cellulases and mixtures of cellulases. White bars: 3 days of hydrolysis; gray bars: 5 days of hydrolysis; black bars: 7 days of hydrolysis.

Figure 8A shows HPLC chromatography of NREL acid-pretreated corn stover liquor. Fractional hydrolysis and absorbance are plotted for the various HPLC fractions. Solid line: fractional hydrolysis; light dashed line: absorbance at 210 nm; heavy dashed line: absorbance at 280 nm. The average hydrolysis for all samples is indicated by the solid, horizontal line. Figure 8B shows the fractional hydrolysis for the *T. reesei* cellulase composition with increasing GH61A polypeptide concentrations in the presence of 3 kDa MWCO flow-through fractions of NREL acid-pretreated corn stover liquor incubated with microcrystalline cellulose and eluted with successive water and organic solvent washes. Gray bars: 3 days of hydrolysis; black bars: 7 days of hydrolysis.

Figure 9 shows a standard curve of AVICEL® height vs. added mass.

Figure 10 shows the effect of various pooled, HPLC-separated NREL acid-pretreated corn stover liquor fractions and the *T. aurantiacus* GH61A polypeptide on hydrolysis of microcrystalline cellulose by the *T. reesei* cellulase composition. Solid lines: height of AVICEL® incubated with the GH61 polypeptide; dashed lines: $A_{(600nm)}$ of washed, BCA reagent-reacted AVICEL®.

Figure 11 shows (A) a standard curve of reducing sugar equivalents and (B) the reducing sugar equivalents measured in the solid microcrystalline cellulose incubated with the *T. aurantiacus* GH61A polypeptide and the indicated HPLC fractions.

Figure 12 shows the results of a microcrystalline cellulose hydrolysis assay with the *T. reesei* cellulase composition in the presence of the *T. aurantiacus* GH61A polypeptide and chromatographed fractions of acid-pretreated xylan.

Solid line: fractional hydrolysis with GH61; dashed line: mean fractional hydrolysis.

Figure 13 shows a LC-MS chromatogram of a representative HPLC fraction of acid-pretreated xylan.

Figure 14 shows liquid chromatography-mass spectrometry chromatograms of GH61 polypeptide-affinity enriched acid-pretreated corn stover. Panel A: diode array detection; Panel B: TOF MS/MS total ion current 17.5; Panel C: TOF MS/MS ES-total ion current 273; Panel D: TOF MS ES-total ion survey.

Figure 15 shows ion chromatograms of microcrystalline cellulose or phosphoric acid-swollen cellulose incubated with the *T. aurantiacus* GH61A polypeptide and NREL acid-pretreated corn stover liquor. Panel A: reaction samples; Panel B: comparison to analytical standards. Panel A: solid lines: AVICEL® incubations; dashed lines: PASC incubations; light gray: AVICEL®; medium gray: AVICEL® + NREL acid-pretreated corn stover liquor; dark gray: AVICEL® + GH61 polypeptide; black: AVICEL® + GH61 polypeptide + liquor. Panel B: black: AVICEL® + GH61 polypeptide + liquor; dark gray solid lines: liquor; dark gray dashed lines: cellopentaose, cellotetraose and cellotriose; light gray: cellopentaonic acid, cellotetraonic acid, gluconic acid, galactonic acid and xylotetraose, peaks as indicated.

Figure 16A shows the fractional hydrolysis of AVICEL® by a *T. reesei* cellulase composition with the indicated *T. aurantiacus* GH61A polypeptide concentration in the presence of alkaline-pretreated corn stover generated using the indicated pretreatment concentration of sodium hydroxide. White bars: 1 day of hydrolysis; gray bars: 3 days of hydrolysis; black bars: 7 days of hydrolysis. Figure 16B shows the fractional hydrolysis of AVICEL® by the *T. reesei* cellulase composition with the indicated GH61 polypeptide concentration in the presence of water-extracted, acid-pretreated corn stover liquors generated using the indicated extraction temperature. Gray bars: 1 day of hydrolysis; white bars: 3 days of hydrolysis.

Figures 17A and 17B show the fractional hydrolysis of AVICEL® by a *T. reesei* cellulase composition with various concentrations of the *T. aurantiacus* GH61A polypeptide in the presence of acid-pretreated components of biomass or mixtures thereof. White bars: 1 day of hydrolysis; dark gray bars: 5 days of hydrolysis.

Figure 18 shows the fractional hydrolysis of AVICEL® by a *T. reesei* cellulase composition with various concentrations of the *T. aurantiacus* GH61A polypeptide in the presence of post-fermentation liquors. White bars: 1 day of hydrolysis; gray bars: 3 days of hydrolysis; black bars: 7 days of hydrolysis.

Figures 19A and 19B show the fractional hydrolysis of AVICEL® by a *T. reesei* cellulase composition with various concentrations of the *T. aurantiacus* GH61A polypeptide in the presence of various severity acid-pretreatments of corn stover. Gray bars: 3 days of hydrolysis; black bars: 7 days of hydrolysis. Figure 19C shows the fractional hydrolysis of AVICEL® by the *T. reesei* cellulase composition with *T. aurantiacus* GH61A polypeptide and liquors generated by acid pretreatment of cellulose at various severities between 110°C and 190°C at GH61A polypeptide concentrations of 50% ( ), 24% ( ); 8% ( ); 4% ( ); 2% ( ); and 0 ( ) (w/w).

Figure 20A, 20B, and 20C shows the fractional hydrolysis of AVICEL® by *T. reesei* cellulase compositions with increasing GH61 polypeptide concentrations plus added acid-pretreated xylan of various severities. Panel A shows 7 days of hydrolysis data for a broad range of severities. Panel B shows more severe pretreatments, gray bars: 3 days of hydrolysis; black bars: 7 days of hydrolysis. Panel C shows the fractional hydrolysis of AVICEL® by the *T. reesei* cellulase composition with *T. aurantiacus* GH61A polypeptide and liquors generated by acid pretreatment of xylan at various severities between 110°C and 190°C at GH61A polypeptide concentrations of 50% (-), 24% (); 8% (--); 4% (--); 2% (...);... and 0 (--) (w/w).

Figure 21 shows the fractional hydrolysis of AVICEL® by *T. reesei* cellulase compositions with and without 50% (w/w) *T. aurantiacus* GH61A polypeptide concentrations plus added solid-phase extracted NREL acid-pretreated corn stover or acid-pretreated xylan as indicated. Gray bars: 3 days of hydrolysis; black bars: 7 days of hydrolysis.

Figure 22 shows the fractional hydrolysis of AVICEL® by a *T. reesei* cellulase composition with various concentrations of *T. aurantiacus* GH61A in the presence of 10% (v/v) of the indicated pretreatment liquor or electrodialyzed pretreatment liquor. Gray bars: 3 days of hydrolysis; black bars: 7 days of hydrolysis.

Figure 23 shows the fractional hydrolysis of various biomass substrates by the *T. reesei* cellulase composition with increasing concentrations of the *T. aurantiacus* GH61A polypeptide with and without NREL acid-pretreated corn stover liquor. Panel A: low and medium severity organosolv ethanol pretreated corn stover; Panel B: medium severity glycerol and water pretreated corn stover, and 5% total solids water pretreated corn stover; Panel C: 5% total solids sugarcane bagasse; Panel D: alkaline pretreated corn stover plus no liquor and NREL milled washed pretreated corn stover controls. White bars: 1 day of hydrolysis; gray bars: 3 days of hydrolysis; black bars: 7 days of hydrolysis.

Figure 24 shows the fractional hydrolysis of AVICEL® by the *T. reesei* cellulase composition with either zero or 24% *T. aurantiacus* GH61A polypeptide in the presence of the indicated acid-pretreated monosaccharides. Gray bars: 3 days of hydrolysis; black bars: 7 days of hydrolysis.

Figure 25 shows the fractional hydrolysis of AVICEL® by the *T. reesei* cellulase composition with various concentrations of the indicated GH61 polypeptides with 10% (v/v) NREL PCS liquor. White symbols: 3 days of hydrolysis; black symbols: 7 days of hydrolysis; *Thermoascus aurantiacus* GH61A: circles; *Aspergillus fumigatus* GH61B polypeptide: diamonds; *Penicillium pinophilum* GH61 polypeptide: squares.

Figure 26 shows the glucose produced by hydrolysis of AVICEL® by a *T. reesei* cellulase composition with *T.*

*aurantiacus* GH61A polypeptide in the presence or absence of Kraft lignin. Solid symbols: *T. reesei* cellulase composition + *T. aurantiacus* GH61A polypeptide. Open symbols: *T. reesei* cellulase composition + *T. aurantiacus* GH61A polypeptide supplemented with additional 15% (w/w) *T. aurantiacus* GH61A polypeptide. Circles: no lignin; squares: 0.1% (w/w) Kraft lignin; diamonds: 0.1% (w/w) oxidized Kraft lignin.

Figure 27 shows the concentrations of glucose and xylose from 120 hours of saccharification of washed, milled alkaline pretreated corn stover by the *T. reesei* cellulase composition supplemented with *T. aurantiacus* GH61A polypeptide, replaced with increasing concentrations of *T. aurantiacus* GH61A. Solid squares: xylose, open diamonds: glucose.

Figure 28 shows the conversion of high total solids (15% TS) dilute acid pretreated corn stover of various pretreatment severities as indicated. The pretreated corn stovers were hydrolyzed by either a composition containing a blend of an *Aspergillus aculeatus* GH10 xylanase and a *Trichoderma reesei* cellulase preparation containing *Aspergillus fumigatus* beta-glucosidase and *Thermoascus aurantiacus* GH61A polypeptide or this mixture replaced with 20% additional *T. aurantiacus* GH61A. For each severity pretreatment other than the least severe, replacement of the cellulase-GH61A polypeptide mixture with additional GH61A polypeptide yielded a greater conversion. Grey bars: 120 hours of saccharification; black bars: 216 hours of saccharification.

Figure 29 shows the conversion of high total solids (15% TS) dilute acid pretreated *Arundo donax* of various pretreatment severities as indicated. The variously pretreated A. *donax* were hydrolyzed by either a composition containing a blend of an *Aspergillus aculeatus* GH10 xylanase (WO 94/021785) and a *Trichoderma reesei* cellulase preparation containing *Aspergillus fumigatus* beta-glucosidase (WO 2005/047499) and *Thermoascus aurantiacus* GH61A polypeptide (WO 2005/074656) or this mixture replaced by 20% additional *T. aurantiacus* GH61A polypeptide. For each severity pretreatment, replacement of the cellulase-GH61A polypeptide mixture with additional GH61A polypeptide yielded a greater conversion. Grey bars: 120 hours of saccharification; black bars: 216 hours of saccharification.

## Definitions

**Acetylxylan esterase:** The term "acetylxylan esterase" means a carboxylesterase (EC 3.1.1.72) that catalyzes the hydrolysis of acetyl groups from polymeric xylan, acetylated xylose, acetylated glucose, alpha-napthyl acetate, and p-nitrophenyl acetate. For purposes of the present invention, acetylxylan esterase activity is determined using 0.5 mM *p*-nitrophenylacetate as substrate in 50 mM sodium acetate pH 5.0 containing 0.01% TWEEN™ 20 (polyoxyethylene sorbitan monolaurate). One unit of acetylxylan esterase is defined as the amount of enzyme capable of releasing 1 μmole of *p*-nitrophenolate anion per minute at pH 5, 25°C.

**Allelic variant:** The term "allelic variant" means any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

**Alpha-L-arabinofuranosidase:** The term "alpha-L-arabinofuranosidase" means an alpha-L-arabinofuranoside arabinofuranohydrolase (EC 3.2.1.55) that catalyzes the hydrolysis of terminal non-reducing alpha-L-arabinofuranoside residues in alpha-L-arabinosides. The enzyme acts on alpha-L-arabinofuranosides, alpha-L-arabinans containing (1,3)- and/or (1,5)-linkages, arabinoxylans, and arabinogalactans. Alpha-L-arabinofuranosidase is also known as arabinosidase, alpha-arabinosidase, alpha-L-arabinosidase, alpha-arabinofuranosidase, polysaccharide alpha-L-arabinofuranosidase, alpha-L-arabinofuranoside hydrolase, L-arabinosidase, or alpha-L-arabinanase. For purposes of the present invention, alpha-L-arabinofuranosidase activity is determined using 5 mg of medium viscosity wheat arabinoxylan (Megazyme International Ireland, Ltd., Bray, Co. Wicklow, Ireland) per ml of 100 mM sodium acetate pH 5 in a total volume of 200 μl for 30 minutes at 40°C followed by arabinose analysis by AMINEX® HPX-87H column chromatography (Bio-Rad Laboratories, Inc., Hercules, CA, USA).

**Alpha-glucuronidase:** The term "alpha-glucuronidase" means an alpha-D-glucosiduronate glucuronohydrolase (EC 3.2.1.139) that catalyzes the hydrolysis of an alpha-D-glucuronoside to D-glucuronate and an alcohol. For purposes of the present invention, alpha-glucuronidase activity is determined according to de Vries, 1998, J. Bacteriol. 180: 243-249. One unit of alpha-glucuronidase equals the amount of enzyme capable of releasing 1 μmole of glucuronic or 4-O-methylglucuronic acid per minute at pH 5, 40°C.

**Beta-glucosidase:** The term "beta-glucosidase" means a beta-D-glucoside glucohydrolase (E.C. 3.2.1.21) that catalyzes the hydrolysis of terminal non-reducing beta-D-glucose residues with the release of beta-D-glucose. For purposes of the present invention, beta-glucosidase activity is determined using *p*-nitrophenyl-beta-D-glucopyranoside as substrate according to the procedure of Venturi et al., 2002, Extracellular beta-D-glucosidase from Chaetomium thermophilum var. coprophilum: production, purification and some biochemical properties, J. Basic Microbiol. 42: 55-66. One unit of beta-glucosidase is defined as 1.0 μmole of *p*-nitrophenolate anion produced per minute at 25°C, pH 4.8 from 1 mM *p*-nitrophenyl-beta-D-glucopyranoside as substrate in 50 mM sodium citrate containing 0.01% TWEEN® 20.

**Beta-xylosidase:** The term "beta-xylosidase" means a beta-D-xyloside xylohydrolase (E.C. 3.2.1.37) that catalyzes the exo-hydrolysis of short beta (1→4)-xylooligosaccharides, to remove successive D-xylose residues from non-reducing termini. For purposes of the present invention, one unit of beta-xylosidase is defined as 1.0 μmole of *p*-nitrophenolate anion produced per minute at 40°C, pH 5 from 1 mM p-nitrophenyl-beta-D-xyloside as substrate in 100 mM sodium citrate containing 0.01% TWEEN® 20.

**cDNA:** The term "cDNA" means a DNA molecule that can be prepared by reverse transcription from a mature, spliced, mRNA molecule obtained from a eukaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Cellobiohydrolase:** The term "cellobiohydrolase" means a 1,4-beta-D-glucan cellobiohydrolase (E.C. 3.2.1.91) that catalyzes the hydrolysis of 1,4-beta-D-glucosidic linkages in cellulose, cellooligosaccharides, or any beta-1,4-linked glucose containing polymer, releasing cellobiose from the reducing or non-reducing ends of the chain (Teeri, 1997, Crystalline cellulose degradation: New insight into the function of cellobiohydrolases, Trends in Biotechnology 15: 160-167; Teeri et al., 1998, Trichoderma reesei cellobiohydrolases: why so efficient on crystalline cellulose?, Biochem. Soc. Trans. 26: 173-178). For purposes of the present invention, cellobiohydrolase activity is determined according to the procedures described by Lever et al., 1972, Anal. Biochem. 47: 273-279; van Tilbeurgh et al., 1982, FEBS Letters, 149: 152-156; van Tilbeurgh and Claeyssens, 1985, FEBS Letters, 187: 283-288; and Tomme et al., 1988, Eur. J. Biochem. 170: 575-581. In the present invention, the Lever *et al.* method can be employed to assess hydrolysis of cellulose in corn stover, while the methods of van Tilbeurgh *et al.* and Tomme *et al.* can be used to determine the cellobiohydrolase activity on a fluorescent disaccharide derivative, 4-methylumbelliferyl-β-D-lactoside.

**Cellulolytic enhancing activity:** The term "cellulolytic enhancing activity" means a biological activity catalyzed by a GH61 polypeptide that enhances the hydrolysis of a cellulosic material by enzyme having cellulolytic activity. For purposes of the present invention, cellulolytic enhancing activity is determined by measuring the increase in reducing sugars or the increase of the total of cellobiose and glucose from the hydrolysis of a cellulosic material by cellulolytic enzyme under the following conditions: 1-50 mg of total protein/g of cellulose in PCS, wherein total protein is comprised of 50-99.5% w/w cellulolytic enzyme protein and 0.5-50% w/w protein of a GH61 polypeptide having cellulolytic enhancing activity for 1-7 days at 50°C compared to a control hydrolysis with equal total protein loading without cellulolytic enhancing activity (1-50 mg of cellulolytic protein/g of cellulose in PCS). In a preferred aspect, a mixture of CELLUCLAST® 1.5L (Novozymes A/S, Bagsværd, Denmark) in the presence of 2-3% of total protein weight *Aspergillus oryzae* beta-glucosidase (recombinantly produced in *Aspergillus oryzae* according to WO 02/095014) or 2-3% of total protein weight *Aspergillus fumigatus* beta-glucosidase (recombinantly produced in *Aspergillus oryzae* as described in WO 2002/095014) of cellulase protein loading is used as the source of the cellulolytic activity.

The GH61 polypeptides having cellulolytic enhancing activity enhance the hydrolysis of a cellulosic material catalyzed by enzyme having cellulolytic activity by reducing the amount of cellulolytic enzyme required to reach the same degree of hydrolysis preferably at least 1.01-fold, more preferably at least 1.05-fold, more preferably at least 1.10-fold, more preferably at least 1.25-fold, more preferably at least 1.5-fold, more preferably at least 2-fold, more preferably at least 3-fold, more preferably at least 4-fold, more preferably at least 5-fold, even more preferably at least 10-fold, and most preferably at least 20-fold.

**Cellulolytic enzyme or cellulase:** The term "cellulolytic enzyme" or "cellulase" means one or more (*e.g.*, several) enzymes that hydrolyze a cellulosic material. Such enzymes include endoglucanase(s), cellobiohydrolase(s), beta-glucosidase(s), or combinations thereof. The two basic approaches for measuring cellulolytic activity include: (1) measuring the total cellulolytic activity, and (2) measuring the individual cellulolytic activities (endoglucanases, cellobiohydrolases, and beta-glucosidases) as reviewed in Zhang et al., Outlook for cellulase improvement: Screening and selection strategies, 2006, Biotechnology Advances 24: 452-481. Total cellulolytic activity is usually measured using insoluble substrates, including Whatman №1 filter paper, microcrystalline cellulose, bacterial cellulose, algal cellulose, cotton, pretreated lignocellulose, *etc.* The most common total cellulolytic activity assay is the filter paper assay using Whatman №1 filter paper as the substrate. The assay was established by the International Union of Pure and Applied Chemistry (IUPAC) (Ghose, 1987, Measurement of cellulase activities, Pure Appl. Chem. 59: 257-68).

For purposes of the present invention, cellulolytic enzyme activity is determined by measuring the increase in hydrolysis of a cellulosic material by cellulolytic enzyme(s) under the following conditions: 1-20 mg of cellulolytic enzyme protein/g of cellulose in PCS for 3-7 days at 50°C compared to a control hydrolysis without addition of cellulolytic enzyme protein. Typical conditions are 1 ml reactions, washed or unwashed PCS, 5% insoluble solids, 50 mM sodium acetate pH 5, 1 mM $MnSO_4$, 50°C, 72 hours, sugar analysis by AMINEX® HPX-87H column (Bio-Rad Laboratories, Inc., Hercules, CA, USA).

**Cellulosic material:** The term "cellulosic material" means any material containing cellulose. The predominant polysaccharide in the primary cell wall of biomass is cellulose, the second most abundant is hemicellulose, and the third is pectin. The secondary cell wall, produced after the cell has stopped growing, also contains polysaccharides and is strengthened by polymeric lignin covalently cross-linked to hemicellulose. Cellulose is a homopolymer of anhydrocellobiose and thus a linear beta-(1-4)-D-glucan, while hemicelluloses include a variety of compounds, such as xylans, xyloglucans, arabinoxylans, and mannans in complex branched structures with a spectrum of substituents. Although generally polymorphous, cellulose is found in plant tissue primarily as an insoluble crystalline matrix of parallel glucan chains. Hemicelluloses usually hydrogen bond to cellulose, as well as to other hemicelluloses, which help stabilize the cell wall matrix.

Cellulose is generally found, for example, in the stems, leaves, hulls, husks, and cobs of plants or leaves, branches, and wood of trees. The cellulosic material can be, but is not limited to, agricultural residue, herbaceous material (including energy crops), municipal solid waste, pulp and paper mill residue, waste paper, and wood (including forestry residue) (see, for example, Wiselogel et al., 1995, in Handbook on Bioethanol (Charles E. Wyman, editor), pp.105-118, Taylor & Francis, Washington D.C.; Wyman, 1994, Bioresource Technology 50: 3-16; Lynd, 1990, Applied Biochemistry and Biotechnology 24/25: 695-719; Mosier et al., 1999, Recent Progress in Bioconversion of Lignocellulosics, in Advances in Biochemical Engineering/Biotechnology, T. Scheper, managing editor, Volume 65, pp.23-40, Springer-Verlag, New York). It is understood herein that the cellulose may be in the form of lignocellulose, a plant cell wall material containing lignin, cellulose, and hemicellulose in a mixed matrix. In a preferred aspect, the cellulosic material is any biomass material. In another preferred aspect, the cellulosic material is lignocellulose, which comprises cellulose, hemicelluloses, and lignin.

In one aspect, the cellulosic material is agricultural residue. In another aspect, the cellulosic material is herbaceous material (including energy crops). In another aspect, the cellulosic material is municipal solid waste. In another aspect, the cellulosic material is pulp and paper mill residue. In another aspect, the cellulosic material is waste paper. In another aspect, the cellulosic material is wood (including forestry residue).

In another aspect, the cellulosic material is arundo. In another aspect, the cellulosic material is bagasse. In another aspect, the cellulosic material is bamboo. In another aspect, the cellulosic material is corn cob. In another aspect, the cellulosic material is corn fiber. In another aspect, the cellulosic material is corn stover. In another aspect, the cellulosic material is miscanthus. In another aspect, the cellulosic material is orange peel. In another aspect, the cellulosic material is rice straw. In another aspect, the cellulosic material is switchgrass. In another aspect, the cellulosic material is wheat straw.

In another aspect, the cellulosic material is aspen. In another aspect, the cellulosic material is eucalyptus. In another aspect, the cellulosic material is fir. In another aspect, the cellulosic material is pine. In another aspect, the cellulosic material is poplar. In another aspect, the cellulosic material is spruce. In another aspect, the cellulosic material is willow.

In another aspect, the cellulosic material is algal cellulose. In another aspect, the cellulosic material is bacterial cellulose. In another aspect, the cellulosic material is cotton linter. In another aspect, the cellulosic material is filter paper. In another aspect, the cellulosic material is microcrystalline cellulose. In another aspect, the cellulosic material is phosphoric-acid treated cellulose.

In another aspect, the cellulosic material is an aquatic biomass. As used herein the term "aquatic biomass" means biomass produced in an aquatic environment by a photosynthesis process. The aquatic biomass can be algae, emergent plants, floating-leaf plants, or submerged plants.

The cellulosic material may be used as is or may be subjected to pretreatment, using conventional methods known in the art, as described herein. In a preferred aspect, the cellulosic material is pretreated.

**Coding sequence:** The term "coding sequence" means a polynucleotide, which directly specifies the amino acid sequence of a polypeptide. The boundaries of the coding sequence are generally determined by an open reading frame, which usually begins with the ATG start codon or alternative start codons such as GTG and TTG and ends with a stop codon such as TAA, TAG, and TGA. The coding sequence may be a DNA, cDNA, synthetic, or recombinant polynucleotide.

**Control sequences:** The term "control sequences" means all components necessary for the expression of a polynucleotide encoding a polypeptide. Each control sequence may be native or foreign to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Endoglucanase:** The term "endoglucanase" means an endo-1,4-(1,3;1,4)-beta-D-glucan 4-glucanohydrolase (E.C.

3.2.1.4) that catalyzes endohydrolysis of 1,4-beta-D-glycosidic linkages in cellulose, cellulose derivatives (such as carboxymethyl cellulose and hydroxyethyl cellulose), lichenin, beta-1,4 bonds in mixed beta-1,3 glucans such as cereal beta-D-glucans or xyloglucans, and other plant material containing cellulosic components. Endoglucanase activity can be determined by measuring reduction in substrate viscosity or increase in reducing ends determined by a reducing sugar assay (Zhang et al., 2006, Biotechnology Advances 24: 452-481). For purposes of the present invention, endoglucanase activity is determined using carboxymethyl cellulose (CMC) as substrate according to the procedure of Ghose, 1987, Pure and Appl. Chem. 59: 257-268, at pH 5, 40°C.

**Expression:** The term "expression" includes any step involved in the production of the polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to additional nucleotides that provide for its expression.

**Family 61 glycoside hydrolase:** The term "Family 61 glycoside hydrolase" or "Family GH61" or"GH61" means a polypeptide falling into the glycoside hydrolase Family 61 according to Henrissat B., 1991, A classification of glycosyl hydrolases based on amino-acid sequence similarities, Biochem. J. 280: 309-316, and Henrissat B., and Bairoch A., 1996, Updating the sequence-based classification of glycosyl hydrolases, Biochem. J. 316: 695-696. The enzymes in this family were originally classified as a glycoside hydrolase family based on measurement of very weak endo-1,4-beta-D-glucanase activity in one family member. The structure and mode of action of these enzymes are non-canonical and they cannot be considered as bona fide glycosidases. However, they are kept in the CAZy classification on the basis of their capacity to enhance the breakdown of lignocellulose when used in conjunction with a cellulase or a mixture of cellulases.

**Feruloyl esterase:** The term "feruloyl esterase" means a 4-hydroxy-3-methoxycinnamoyl-sugar hydrolase (EC 3.1.1.73) that catalyzes the hydrolysis of 4-hydroxy-3-methoxycinnamoyl (feruloyl) groups from esterified sugar, which is usually arabinose in "natural" substrates, to produce ferulate (4-hydroxy-3-methoxycinnamate). Feruloyl esterase is also known as ferulic acid esterase, hydroxycinnamoyl esterase, FAE-III, cinnamoyl ester hydrolase, FAEA, cinnAE, FAE-I, or FAE-II. For purposes of the present invention, feruloyl esterase activity is determined using 0.5 mM p-nitrophenylferulate as substrate in 50 mM sodium acetate pH 5.0. One unit of feruloyl esterase equals the amount of enzyme capable of releasing 1 μmole of *p*-nitrophenolate anion per minute at pH 5, 25°C.

**Hemicellulolytic enzyme or hemicellulase:** The term "hemicellulolytic enzyme" or "hemicellulase" means one or more (*e.g.*, several) enzymes that hydrolyze a hemicellulosic material. See, for example, Shallom, D. and Shoham, Y. Microbial hemicellulases. Current Opinion In Microbiology, 2003, 6(3): 219-228). Hemicellulases are key components in the degradation of plant biomass. Examples of hemicellulases include, but are not limited to, an acetylmannan esterase, an acetyxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase. The substrates of these enzymes, the hemicelluloses, are a heterogeneous group of branched and linear polysaccharides that are bound via hydrogen bonds to the cellulose microfibrils in the plant cell wall, crosslinking them into a robust network. Hemicelluloses are also covalently attached to lignin, forming together with cellulose a highly complex structure. The variable structure and organization of hemicelluloses require the concerted action of many enzymes for its complete degradation. The catalytic modules of hemicellulases are either glycoside hydrolases (GHs) that hydrolyze glycosidic bonds, or carbohydrate esterases (CEs), which hydrolyze ester linkages of acetate or ferulic acid side groups. These catalytic modules, based on homology of their primary sequence, can be assigned into GH and CE families marked by numbers. Some families, with an overall similar fold, can be further grouped into clans, marked alphabetically (*e.g.*, GH-A). A most informative and updated classification of these and other carbohydrate active enzymes is available in the Carbohydrate-Active Enzymes (CAZy) database. Hemicellulolytic enzyme activities can be measured according to Ghose and Bisaria, 1987, Pure & Appl. Chem. 59: 1739-1752.

**Host cell:** The term "host cell" means any cell type that is susceptible to transformation, transfection, transduction, and the like with a nucleic acid construct or expression vector comprising a polynucleotide. The term "host cell" encompasses any progeny of a parent cell that is not identical to the parent cell due to mutations that occur during replication.

**Isolated or Purified:** The term "isolated" or "purified" means a polypeptide or polynucleotide that is removed from at least one component with which it is naturally associated. For example, a polypeptide may be at least 1% pure, e.g., at least 5% pure, at least 10% pure, at least 20% pure, at least 40% pure, at least 60% pure, at least 80% pure, at least 90% pure, or at least 95% pure, as determined by SDS-PAGE, and a polynucleotide may be at least 1% pure, *e.g.*, at least 5% pure, at least 10% pure, at least 20% pure, at least 40% pure, at least 60% pure, at least 80% pure, at least 90% pure, or at least 95% pure, as determined by agarose electrophoresis.

**Liquor:** The term "liquor" means the solution phase, either aqueous, organic, or a combination thereof, arising from treatment of a lignocellulose and/or hemicellulose and/or lignacious material or feedstock, or monosaccharides thereof, *e.g.,* xylose, arabinose, mannose, *etc.,* under conditions as described herein, and the soluble contents thereof.

**Mature polypeptide:** The term "mature polypeptide" means a polypeptide in its final form following translation and any post-translational modifications, such as N-terminal processing, C-terminal truncation, glycosylation, phosphorylation, etc. It is known in the art that a host cell may produce a mixture of two of more different mature polypeptides (*i.e.,* with a different C-terminal and/or N-terminal amino acid) expressed by the same polynucleotide. The mature polypeptide can be predicted using the SignalP program (Nielsen et al., 1997, Protein Engineering 10: 1-6).

**Mature polypeptide coding sequence:** The term "mature polypeptide coding sequence" is defined herein as a nucleotide sequence that encodes a mature polypeptide having biological activity. The mature polypeptide coding sequence can be predicted using the SignalP program (Nielsen *et al.,* 1997, *supra*).

**Nucleic** acid construct: The term "nucleic acid construct" means a nucleic acid molecule, either single- or double-stranded, which is isolated from a naturally occurring gene or is modified to contain segments of nucleic acids in a manner that would not otherwise exist in nature or which is synthetic. The term nucleic acid construct is synonymous with the term "expression cassette" when the nucleic acid construct contains the control sequences required for expression of a coding sequence.

Operably linked: The term "operably linked" means a configuration in which a control sequence is placed at an appropriate position relative to the coding sequence of a polynucleotide such that the control sequence directs the expression of the coding sequence.

**Polypeptide fragment:** The term "fragment" means a polypeptide having one or more (*e.g.*, several) amino acids deleted from the amino and/or carboxyl terminus of a mature polypeptide; wherein the fragment has biological activity.

**Pretreated corn stover:** The term "PCS" or "Pretreated Corn Stover" means a cellulosic material derived from corn stover by treatment with heat and dilute sulfuric acid, alkaline pretreatment, or neutral pretreatment..

**Sequence Identity:** The relatedness between two amino acid sequences or between two nucleotide sequences is described by the parameter "sequence identity".

For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0, 5.0.0, or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment)

For purposes of the present invention, the degree of sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice *et al.,* 2000, *supra*), preferably version 3.0.0 or later. The optional parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment)

**Subsequence:** The term "subsequence" means a polynucleotide having one or more (*e.g.*, several) nucleotides deleted from the 5' and/or 3' end of a mature polypeptide coding sequence; wherein the subsequence encodes a fragment having biological activity.

**Variant:** The term "variant" means a polypeptide having cellulolytic enhancing activity comprising an alteration, *i.e.,* a substitution, insertion, and/or deletion of one or more (*e.g.,* several) amino acid residues at one or more (*e.g.*, several) positions. A substitution means a replacement of an amino acid occupying a position with a different amino acid; a deletion means removal of an amino acid occupying a position; and an insertion means adding one or more (*e.g.,* several) amino acids, *e.g.,* 1-5 amino acids, adjacent to an amino acid occupying a position.

**Xylan-containing material:** The term "xylan-containing material" means any material comprising a plant cell wall polysaccharide containing a backbone of beta-(1-4)-linked xylose residues. Xylans of terrestrial plants are heteropolymers possessing a beta-(1-4)-D-xylopyranose backbone, which is branched by short carbohydrate chains.

They comprise D-glucuronic acid or its 4-O-methyl ether, L-arabinose, and/or various oligosaccharides, composed of D-xylose, L-arabinose, D- or L-galactose, and D-glucose. Xylan-type polysaccharides can be divided into homoxylans and heteroxylans, which include glucuronoxylans, (arabino)glucuronoxylans, (glucurono)arabinoxylans, arabinoxylans, and complex heteroxylans. See, for example, Ebringerova et al., 2005, Adv. Polym. Sci. 186: 1-67. In the methods of the present invention, any material containing xylan may be used. In a preferred aspect, the xylan-containing material is lignocellulose.

**Xylan degrading activity or xylanolytic activity:** The term "xylan degrading activity" or "xylanolytic activity" means a biological activity that hydrolyzes xylan-containing material. The two basic approaches for measuring xylanolytic activity include: (1) measuring the total xylanolytic activity, and (2) measuring the individual xylanolytic activities (*e.g.*, endoxylanases, beta-xylosidases, arabinofuranosidases, alpha-glucuronidases, acetylxylan esterases, feruloyl esterases, and alpha-glucuronyl esterases). Recent progress in assays of xylanolytic enzymes was summarized in several publications including Biely and Puchard, Recent progress in the assays of xylanolytic enzymes, 2006, Journal of the Science of Food and Agriculture 86(11): 1636-1647; Spanikova and Biely, 2006, Glucuronoyl esterase - Novel carbohydrate esterase produced by Schizophyllum commune, FEBS Letters 580(19): 4597-4601; Herrmann, Vrsanska, Jurickova, Hirsch, Biely, and Kubicek, 1997, The beta-D-xylosidase of Trichoderma reesei is a multifunctional beta-D-xylan xylohydrolase, Biochemical Journal 321: 375-381.

Total xylan degrading activity can be measured by determining the reducing sugars formed from various types of xylan, including, for example, oat spelt, beechwood, and larchwood xylans, or by photometric determination of dyed xylan fragments released from various covalently dyed xylans. The most common total xylanolytic activity assay is based on production of reducing sugars from polymeric 4-O-methyl glucuronoxylan as described in Bailey, Biely, Poutanen, 1992, Interlaboratory testing of methods for assay of xylanase activity, Journal of Biotechnology 23(3): 257-270. Xylanase activity can also be determined with 0.2% AZCL-arabinoxylan as substrate in 0.01% TRITON® X-100 (4-(1,1,3,3-tetramethylbutyl)phenyl-polyethylene glycol) and 200 mM sodium phosphate buffer pH 6 at 37°C. One unit of xylanase activity is defined as 1.0 μmole of azurine produced per minute at 37°C, pH 6 from 0.2% AZCL-arabinoxylan as substrate in 200 mM sodium phosphate pH 6 buffer.

For purposes of the present invention, xylan degrading activity is determined by measuring the increase in hydrolysis of birchwood xylan (Sigma Chemical Co., Inc., St. Louis, MO, USA) by xylan-degrading enzyme(s) under the following typical conditions: 1 ml reactions, 5 mg/ml substrate (total solids), 5 mg of xylanolytic protein/g of substrate, 50 mM sodium acetate pH 5, 50°C, 24 hours, sugar analysis using p-hydroxybenzoic acid hydrazide (PHBAH) assay as described by Lever, 1972, A new reaction for colorimetric determination of carbohydrates, Anal. Biochem 47: 273-279.

**Xylanase:** The term "xylanase" means a 1,4-beta-D-xylan-xylohydrolase (E.C. 3.2.1.8) that catalyzes the endohydrolysis of 1,4-beta-D-xylosidic linkages in xylans. For purposes of the present invention, xylanase activity is determined with 0.2% AZCL-arabinoxylan as substrate in 0.01% TRITON® X-100 and 200 mM sodium phosphate buffer pH 6 at 37°C. One unit of xylanase activity is defined as 1.0 μmole of azurine produced per minute at 37°C, pH 6 from 0.2% AZCL-arabinoxylan as substrate in 200 mM sodium phosphate pH 6 buffer.

### Detailed Description of the Invention

Disclosed are compositions comprising: (a) a polypeptide having cellulolytic enhancing activity; and (b) a liquor, wherein the combination of the polypeptide having cellulolytic enhancing activity and the liquor enhances hydrolysis of a cellulosic material by a cellulolytic enzyme. The compositions may further comprise (c) one or more (*e.g.*, several) enzymes selected from the group consisting of a cellulase, a hemicellulase, an esterase, an expansin, a laccase, a ligninolytic enzyme, a pectinase, a peroxidase, a protease, and a swollenin.

The present invention relates to methods for degrading or converting a cellulosic material, comprising: treating the cellulosic material with an enzyme composition comprising a cellulase in the presence of a GH61 polypeptide having cellulolytic enhancing activity and a liquor arising from treatment of a lignocellulose material, a hemicellulose material, a lignacious material, monosaccharides of the lignocellulose material, monosaccharides of the hemicellulose material, or a combination thereof, wherein the liquor is present in an amount that minimizes inhibition of a cellulase composition of 1 to 20% (v/v), wherein the combination of the GH61 polypeptide having cellulolytic enhancing activity and the liquor enhances hydrolysis of the cellulosic material by the enzyme composition, wherein the enhanced hydrolysis is assessed according to Equation 3:

$$\textit{GH61 effect} = \frac{\%\ \text{conversion}_{(+\ \text{GH61+ liquor})}}{\%\ \text{conversion}_{(\text{no GH61+ liquor})}} \qquad \text{(Equation 3)}$$

and the equation ratio is greater than 1,

wherein the GH61 polypeptide having cellulolytic enhancing activity comprises the following motifs:

[ILMV]-P-X(4,5)-G-X-Y-[ILMV]-X-R-X-[EQ]-X(4)-[HNQ] (SEQ ID NO: 127 or SEQ ID NO: 128) and [FW]-[TF]-K-[AIV], wherein X is any amino acid, X(4,5) is any amino acid at 4 or 5 contiguous positions, and X(4) is any amino acid at 4 contiguous positions.

In an embodiment the method further comprises recovering the degraded cellulosic material. The present invention also relates to methods for producing a fermentation product, comprising:

(a) saccharifying a cellulosic material with an enzyme composition comprising a cellulase in the presence of a GH61 polypeptide having cellulolytic enhancing activity and a liquor arising from treatment of a lignocellulose material, a hemicellulose material, a lignacious material, monosaccharides of the lignocellulose material, monosaccharides of the hemicellulose material, or a combination thereof, wherein the liquor is present in an amount that minimizes inhibition of a cellulase composition of about 1 to about 20% (v/v), wherein the liquor is present in an amount that minimizes inhibition of a cellulase composition of 1 to 20% (v/v), wherein the combination of the GH61 polypeptide having cellulolytic enhancing activity and the liquor enhances hydrolysis of the cellulosic material by the enzyme composition, wherein the enhanced hydrolysis is assessed according to Equation 3:

$$GH61\ effect = \frac{\%\ conversion_{(+\ GH61+\ liquor)}}{\%\ conversion_{(no\ GH61+\ liquor)}} \qquad \text{(Equation 3)}$$

and the equation ratio is greater than 1, wherein the polypeptide having cellulolytic enhancing activity comprises the following motifs:
[ILMV]-P-X(4,5)-G-X-Y-[ILMV]-X-R-X-[EQ]-X(4)-[HNQ] (SEQ ID NO: 127 or SEQ ID NO: 128) and [FW]-[TF]-K-[AIV], wherein X is any amino acid, X(4,5) is any amino acid at 4 or 5 contiguous positions, and X(4) is any amino acid at 4 contiguous positions;
(b) fermenting the saccharified cellulosic material with one or more fermenting microorganisms to produce the fermentation product; and
(c) recovering the fermentation product from the fermentation.

The present invention also relates to methods of fermenting a cellulosic material, comprising: fermenting the cellulosic material with one or more fermenting microorganisms, wherein the cellulosic material is saccharified with an enzyme composition comprising a cellulase in the presence of a GH61 polypeptide having cellulolytic enhancing activity and a liquor arising from treatment of a lignocellulose material, a hemicellulose material, a lignacious material, monosaccharides of the lignocellulose material, monosaccharides of the hemicellulose material, or a combination thereof, wherein the liquor is present in an amount that minimizes inhibition of a cellulase composition of 1 to 20% (v/v), wherein the combination of the GH61 polypeptide having cellulolytic enhancing activity and the liquor enhances hydrolysis of the cellulosic material by the enzyme composition, wherein the enhanced hydrolysis is assessed according to Equation 3:

$$GH61\ effect = \frac{\%\ conversion_{(+\ GH61+\ liquor)}}{\%\ conversion_{(no\ GH61+\ liquor)}} \qquad \text{(Equation 3)}$$

and the equation ratio is greater than 1,
wherein the polypeptide having cellulolytic enhancing activity comprises the following motifs:
[ILMV]-P-X(4,5)-G-X-Y-[ILMV]-X-R-X-[EQ]-X(4)-[HNQ] (SEQ ID NO: 127 or SEQ ID NO: 128) and [FW]-[TF]-K-[AIV], wherein X is any amino acid, X(4,5) is any amino acid at 4 or 5 contiguous positions, and X(4) is any amino acid at 4 contiguous positions.

**Liquors**

The term "liquor" means the solution phase, either aqueous, organic, ionic liquid, or combinations thereof, arising from treatment of a lignocellulose and/or hemicellulose and/or lignacious material or feedstock in a slurry, or monosaccharides thereof, *e.g.*, xylose, arabinose, mannose, *etc.,* under conditions as described herein, and the soluble contents thereof. A liquor for cellulolytic enhancement of a GH61 polypeptide can be produced by treating a lignocellulosic, hemicellulosic, or lignacious material (or feedstock) by applying heat and/or pressure, optionally in the presence of a catalyst, *e.g.*, acid, optionally in the presence of an organic solvent, and optionally in combination with physical disruption of the material, and then separating the solution from the residual solids. Alternatively, the lignocellulosic, hemicellulosic, or lignacious material can be slurried and incubated in aqueous, organic, or ionic liquids or combinations thereof as either solutions or suspensions without addition of heat or pressure. Such conditions determine the degree of cellulolytic enhancement obtainable through the combination of liquor and a GH61 polypeptide during hydrolysis of a cellulosic substrate by a cellulase preparation. The liquor can be separated from the treated material using methods standard in the art, such as filtration, sedimentation, or centifugation.

In one aspect, the material for producing the liquor is herbaceous material. In another aspect, the material is agricultural residue. In another aspect, the material is forestry residue. In another aspect, the material is municipal solid waste. In another aspect, the material is waste paper. In another aspect, the material is pulp and paper mill residue. In another aspect, the material is pulping liquor. In another aspect, the material is mixed wood waste.

In another aspect, the material for producing the liquor is corn stover. In another aspect, the material is corn fiber. In another aspect, the material is corn cob. In another aspect, the material is orange peel. In another aspect, the material is rice straw. In another aspect, the material is wheat straw. In another aspect, the material is switch grass. In another aspect, the material is miscanthus. In another aspect, the material is sugar cane bagasse. In another aspect, the material is energy cane. In another aspect, the material is sorghum. In another aspect, the material is algae biomass.

In another aspect, the material for producing the liquor is softwood. In another aspect, the material is hardwood. In another aspect, the material is poplar. In another aspect, the material is pine. In another aspect, the material is spruce. In another aspect, the material is fir. In another aspect, the material is willow. In another aspect, the material is eucalyptus.

In another aspect, the material for producing the liquor is a hemicellulose. In another aspect, the material is a hemicellulose-rich lignocellulose. In another aspect, the material is a xylan. In another aspect, the material is beechwood xylan. In another aspect, the material is birch xylan. In another aspect, the material is spruce xylan. In another aspect, the material is arabinoxylan. In another aspect, the material is mannan. In another aspect, the material is glucomannan. In another aspect, the material contains beta-(1,4)-linked xylan. In another aspect, the material contains beta-(1,4)-linked mannan. In another aspect, the material contains branched beta-(1,4)-linked xylan, such as arabinoxylan and arabino-(glucoryono-)xylan. In another aspect, the material contains branched beta-(1,4)-linked mannan, such as galactoglucomannan.

In another aspect, the material for producing the liquor is a C5 monosaccharide (pentose). In another aspect, the material is arabinose. In another aspect, the material is xylose. In another aspect, the material is xylulose. In another aspect, the material is ribose. In another aspect, the material is ribulose. In another aspect, the material is acetyl-xylose. In another aspect, the material is ferulyl-xylose. In another aspect, the material is glucurono-xylose or methyl-glucurono-xylose.

In another aspect, the material for producing the liquor is a C6 monosaccharide (hexose). In another aspect, the material is glucose. In another aspect, the material is mannose. In another aspect, the material is fructose. In another aspect, the material is gulose. In another aspect, the material is allose. In another aspect, the material is altrose. In another aspect, the material is idose. In another aspect, the material is talose. In another aspect, the material is galactose. In another aspect, the material is gluconic acid. In another aspect, the material is glucuronic acid. In another aspect, the material is galactonic acid. In another aspect, the material is galacturonic acid. In another aspect, the material is psicose. In another aspect, the material is fructose. In another aspect, the material is sorbose. In another aspect, the material is tagatose.

In another aspect, the material for producing the liquor is a C4 monosaccharide. In another aspect, the material is erythrose. In another aspect, the material is threose. In another aspect, the material is erythrulose.

In another aspect, the material for producing the liquor is a C3 monosaccharide. In another aspect, the material is glyceraldehyde. In another aspect, the material is dihydroxyacetone.

In another aspect, the material for producing the liquor is lignin. In another aspect, the material is Kraft (Indulin) lignin. In another aspect, the material is *p*-hydroxyphenyl (H) rich lignin. In another aspect, the material is guaiacyl (G) rich lignin. In another aspect, the material is syringal (S) rich lignin. In another aspect, the material is lignosulfonate. In another aspect the material is black liquor or components thereof. In another aspect, the material is tall oil or components thereof.

In another aspect, the material for producing the liquor is a post-pretreatment residue of biomass (the solid waste after pretreatment. In another aspect, the material for producing the liquor is a post-saccharification residue of biomass (the solid waste after saccharification). In another aspect, the material for producing the liquor is a post-fermentation residue of biomass (the solid waste after fermentation). In another aspect, the material for producing the liquor is a post-distillation residue of biomass (the solid waste after distillation).

In a non-limiting aspect, the material is treated using acid in the range of about 0.5 to about 5% (w/v), *e.g.,* about 0.5 to about 4.5% (w/v), about 0.75 to about 4% (w/v), about 1.0 to about 3.5% (w/v), about 1.25 to about 3.0% (w/v), or about 1.5 to about 2.5% (w/v); a pH of about 0 to about 3, *e.g.,* about 0.5 to about 3, about 0.5 to about 2.5, about 1 to about 2, or about 1 to about 1.5; a time period of about 1 to about 15 minutes, *e.g.,* about 1 to about 12 minutes, about 2 to about 10 minutes, about 3 to about 9 minutes, about 4 to about 9 minutes, about 5 to about 9 minutes, or about 6 to about 8 minutes; at a temperature at about 130°C to about 250°C, *e.g.,* about 140°C to about 220°C, about 150°C to about 200°C, about 160°C to about 190°C, about 160°C to about 185°C, about 165°C to about 180°C, about 165°C to about 175°C, or about 165°C to about 170°C; and a pressure of about 100 to about 600 psi, *e.g.,* about 50 to about 1700 psi, about 100 to about 1500 psi, about 100 to about 1200 psi, about 100 to about 1000 psi, about 100 to about 500 psi, about 100 to about 400 psi, about 100 to about 300 psi, about 100 to about 200 psi, about 100 to about 150 psi, or about 100 to about 120 psi. In another aspect, the acid is sulfuric acid. In another aspect, the acid is hydrochloric acid. In another aspect, the acid is nitric acid. In another aspect, the acid is phosphoric acid. In another aspect, the acid is acetic acid. In another aspect, the acid is citric acid. In another aspect, the acid is succinic acid. In another aspect, the acid is tartaric acid. In another aspect, the acid is mixtures of any of the above acids. It is understood herein that the conditions described above may need to be optimized depending on the material being treated and the reactor used to produce the liquor. Such optimization is well within the skill in the art. Conditions used to pretreat a cellulosic material as described herein may also be used to generate liquor from a particular feedstock.

In a preferred aspect, the material is treated using 1.4% (w/v) sulfuric acid for 8 minutes at 165°C and 107 psi.

Treatment of a material to produce such liquor may also generate other compounds that are inhibitory to cellulases and/or hemicellulases, *e.g.,* organic acids and lignin-derived compounds. Conditions can be selected that balance cellulolytic enhancing activity of a GH61 polypeptide with production of inhibitor compounds of cellulases and/or hemicellulases. Such conditions can vary depending on the material used for producing the liquor. However, in methods not covered by the methods of the present invention, the liquor can be subjected to a molecular weight filter(s) or dialysis, *e.g.* electrodialysis, using a membrane with nominal molecular weight cut-off of in the range of about 0.1 kDa to about 10 kDa, *e.g.,* about 0.5 kDa to about 7 kDa, about 0.5 kDa to about 5 kDa, and about 1 kDa to about 3 kDa, to reduce the amount of the inhibitory compounds. In methods not covered by the methods of the present invention, any method known in the art can be used to reduce the amount of the inhibitory compounds. In such methods not falling under the scope of the present invention, the liquor is further processed to remove inhibitors of a cellulase, a hemicellulase, or a combination thereof.

In other aspects of the present invention, the liquor can be generated *in situ* by pretreating a cellulosic material that will be saccharified by a cellulase preparation. However, in such instances the amount of effective liquor generated *in situ* may be insufficient with regard to the GH61 polypeptide having cellulolytic enhancing activity, cellulolytic enzyme(s), and cellulose. For example, pretreatment of a cellulosic material under mild conditions, *e.g.,* auto-catalyzed steam explosion, alkaline pretreatment, auto-hydrolysis, jet cooking, hot water-pretreatment, organosolv using ethanol, glycerol, *etc.,* dilute acid pretreatment, and the like ("low severity conditions") or a pretreatment that includes a wash or rinse step or unpretreated cellulosic material compared to harsh conditions ("high severity conditions") to produce *in situ* a liquor may be inadequate for optimizing the cellulolytic enhancing activity of a GH61 polypeptide. Conditions employed to produce NREL preatreated corn stover, *i.e.,* 1.4 wt % sulfuric acid for 8 minutes at 165°C and 107 psi (Example 1) would be considered high severity conditions. In such circumstances a liquor obtained using treatment conditions different from the pretreatment conditions of the cellulosic material can be added to the saccharification reaction. In other aspects of the invention, a low-severity extraction treatment that extracts liquor can be used instead of conventional treatment techniques listed herein, and this liquor can then be added to the saccharification reaction.

In one aspect, the liquor is obtained from a material that is the same as the cellulosic material to be subjected to saccharification by a cellulase composition. In another aspect, the liquor is obtained from a material that is different than the cellulosic material to be subjected to saccharification by a cellulase composition.

In another aspect, the liquor is obtained from a material that is the same as the cellulosic material to be subjected to saccharification by a cellulase composition, but the treatment conditions used to produce the liquor are different from the pretreatment conditions of the cellulosic material. In another aspect, the liquor is obtained from a material that is the same as the cellulosic material to be subjected to saccharification by a cellulase composition, and the treatment conditions used to produce the liquor are the same as the pretreatment conditions of the cellulosic material. In another aspect, the liquor is obtained from a material that is the same as the cellulosic material to be subjected to saccharification by a cellulase composition, and the treatment conditions used to produce the liquor are the same as the pretreatment conditions of the cellulosic material, and the liquor is further processed, e.g., concentrated, filtered to remove cellulase inhibitors, filtered and concentrated, *etc.*

In another aspect, the liquor is obtained from a material that is different than the cellulosic material to be subjected to saccharification by a cellulase composition, and the treatment conditions used to produce the liquor are different from the pretreatment conditions of the cellulosic material. In another aspect, the liquor is obtained from a material that is different than the cellulosic material to be subjected to saccharification by a cellulase composition, and the treatment conditions used to produce the liquor are the same as the pretreatment conditions of the cellulosic material. In another aspect, the liquor is obtained from a material that is different than the cellulosic material to be subjected to saccharification by a cellulase composition, and the treatment conditions used to produce the liquor are the same as the pretreatment conditions of the cellulosic material, and the liquor is further processed, *e.g.,* concentrated, filtered to remove cellulase inhibitors, filtered and concentrated, *etc.* Further processing to remove cellulose inhibitors can be accomplished using any method known in the art.

In another aspect, liquors generated *in situ* may be washed or diluted and replaced with liquors generated *ex situ* to a greater or lesser extent, so the liquor composition/content is optimized for the cellulolytic enhancing effect of a GH61 polypeptide. In another aspect, the solids content of subsequent saccharifications is altered to optimize the liquor content for the cellulolytic enhancing effect of a GH61 polypeptide.

The effective amount of the liquor can depend on one or more (*e.g.*, several) factors including, but not limited to, the mixture of component cellulolytic enzymes, the cellulosic substrate, the concentration of cellulosic substrate, the pretreatment(s) of the cellulosic substrate, non-cellulosic components (*e.g.*, native or degraded lignin or hemicellulose), non-cellulase components, temperature, reaction time, and the liquor (*e.g.*, filtered to remove cellulase and/or hemicellulase inhibitors).

The liquor is preferably present in an amount that is not limiting with regard to the GH61 polypeptide having cellulolytic enhancing activity, cellulolytic enzyme(s), and cellulose. In one aspect, the liquor is present in an amount that is not limiting with regard to the GH61 polypeptide having cellulolytic enhancing activity. In another aspect, the liquor is present in an amount that is not limiting with regard to the cellulolytic enzyme(s). In another aspect, the liquor is present in an amount that is not limiting with regard to the cellulose. In another aspect, the liquor is present in an amount that is not limiting with regard to the GH61 polypeptide having cellulolytic enhancing activity and the cellulolytic enzyme(s). In another aspect, the liquor is present in an amount that is not limiting with regard to the GH61 polypeptide having cellulolytic enhancing activity and the cellulose. In another aspect, the liquor is present in an amount that is not limiting with regard to the cellulolytic enzyme(s) and the cellulose. In another aspect, the liquor is present in an amount that is not limiting with regard to the GH61 polypeptide having cellulolytic enhancing activity, the cellulolytic enzyme(s), and the cellulose.

The liquor is preferably present in an amount that optimizes the cellulolytic enhancing activity of a GH61 polypeptide during saccharification with a cellulase composition. In one aspect, the liquor optimizes the cellulolytic enhancing activity of a GH61 polypeptide with a *GH61 effect* as defined by Equation 3 (the ratio of fractional hydrolysis in the presence to the absence of the GH61 polypeptide) of preferably at least 1.05, more preferably at least 1.10, more preferably at least 1.15, more prefereably at least 1.2, more preferably at least 1.25, more preferably at least 1.3, more preferably at least 1.35, more preferably at least 1.4, more preferably at least 1.45, more preferably at least 1.5, more preferably at least 1.55, more preferably at least 1.6, more preferably at least 1.65, more preferably at least 1.7, more preferably at least 1.75, more preferably at least 1.8, more preferably at least 1.85, more preferably at least 1.9, most preferably at least 1.95, and even most preferably at least 2. An increase in the GH61 effect is obtained when liquor is added, relative to when liquor is not added, during hydrolysis. In another aspect, the amount of GH61 polypeptide is optimized for a given concentration of liquor. Such optimization is accomplished by varying the concentration of each component to determine the optimal ratio of the components during saccharification.

In another aspect, an effective amount of the liquor to cellulose is about $10^{-6}$ to about 10 g per g of cellulose, e.g., about $10^{-6}$ to about 7.5 g, about $10^{-6}$ to about 5, about $10^{-6}$ to about 2.5 g, about $10^{-6}$ to about 1 g, about $10^{-5}$ to about 1 g, about $10^{-5}$ to about $10^{-1}$ g, about $10^{-4}$ to about $10^{-1}$ g, about $10^{-3}$ to about $10^{-1}$ g, and about $10^{-3}$ to about $10^{-2}$ g per g of cellulose.

In another aspect, the amount of liquor present that minimizes inhibition of a cellulase composition and in combination with a GH61 polypeptide enhances hydrolysis by an enzyme composition is about 1 to about 20% (v/v), *e.g.,* about 1 to about 15%, about 1 to about 10%, about 2 to about 7%, about 2 to about 5%, and about 3 to about 5%.

In the methods of the present invention, the term "liquor" encompasses one or more (*e.g.*, several) liquors from different materials based on the same or different conditions of treatment to produce the liquors.

In the methods of the present invention, the liquor is preferably present when a GH61 polypeptide is present, for example, is added with the GH61 polypeptide. The liquor can also be added at different stages of a saccharification. The liquor can also be redosed at different stages of saccharification, *e.g.,* daily, to maintain the presence of an effective concentration of the liquor. The liquor can also be removed or washed to various degrees, or may be diluted at different stages of saccharification. Liquors generated *in situ* may be washed and replaced with liquors generated *ex situ* to a greater or lesser extent, at various times during saccharification

In another aspect of the present invention, the liquor may be recycled from a completed saccharification or completed saccharification and fermentation to a new saccharification. The liquor can be recovered using standard methods in the art, *e.g.,* filtration/centrifugation, sedimentation, and/or flocculation of solids materials pre- or post-distillation, to remove residual solids, cellular debris, *etc.* and then recirculated to the new saccharification.

**Polypeptides Having Cellulolytic Enhancing Activity and Polynucleotides Thereof**

In the methods of the present invention, any GH61 polypeptide having cellulolytic enhancing activity and below defined motifs can be used.

In a first aspect, the polypeptide having cellulolytic enhancing activity comprises the following motifs: [ILMV]-P-X(4,5)-G-X-Y-[ILMV]-X-R-X-[EQ]-X(4)-[HNQ] (SEQ ID NO: 127 or SEQ ID NO: 128) and [FW]-[TF]-K-[AIV], wherein X is any amino acid, X(4,5) is any amino acid at 4 or 5 contiguous positions, and X(4) is any amino acid at 4 contiguous positions.

The isolated polypeptide comprising the above-noted motifs may further comprise:

H-X(1,2)-G-P-X(3)-[YW]-[AILMV] (SEQ ID NO: 129 or SEQ ID NO: 130),
[EQ]-X-Y-X(2)-C-X-[EHQN]-[FILV]-X-[ILV] (SEQ ID NO: 131), or
H-X(1,2)-G-P-X(3)-[YW]-[AILMV] (SEQ ID NO: 132 or SEQ ID NO: 133) and [EQ]-X-Y-X(2)-C-X-[EHQN]-[FILV]-X-[ILV] (SEQ ID NO: 134),

wherein X is any amino acid, X(1,2) is any amino acid at 1 position or 2 contiguous positions, X(3) is any amino acid at 3 contiguous positions, and X(2) is any amino acid at 2 contiguous positions. In the above motifs, the accepted IUPAC single letter amino acid abbreviation is employed.

In a preferred embodiment, the isolated GH61 polypeptide having cellulolytic enhancing activity further comprises H-X(1,2)-G-P-X(3)-[YW]-[AILMV] (SEQ ID NO: 135 or SEQ ID NO: 136). In another preferred embodiment, the isolated GH61 polypeptide having cellulolytic enhancing activity further comprises [EQ]-X-Y-X(2)-C-X-[EHQN]-[FILV]-X-[ILV] (SEQ ID NO: 137). In another preferred embodiment, the isolated GH61 polypeptide having cellulolytic enhancing activity further comprises H-X(1,2)-G-P-X(3)-[YW]-[AILMV] (SEQ ID NO: 138 or SEQ ID NO: 139) and [EQ]-X-Y-X(2)-C-X-[EHQN]-[FILV]-X-[ILV] (SEQ ID NO: 140).

In a second aspect, isolated polypeptides having cellulolytic enhancing activity, comprise the following motif:
[ILMV]-P-X(4,5)-G-X-Y-[ILMV]-X-R-X-[EQ]-X(3)-A-[HNQ] (SEQ ID NO: 141 or SEQ ID NO: 142),
wherein X is any amino acid, X(4,5) is any amino acid at 4 or 5 contiguous positions, and X(3) is any amino acid at 3 contiguous positions. In the above motif, the accepted IUPAC single letter amino acid abbreviation is employed.

In a third aspect, the polypeptide having cellulolytic enhancing activity comprises an amino acid sequence that has a degree of identity to the mature polypeptide of SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 144, SEQ ID NO: 146, SEQ ID NO: 148, SEQ ID NO: 150, SEQ ID NO: 152, SEQ ID NO: 154, SEQ ID NO: 156, SEQ ID NO: 158, SEQ ID NO: 160, SEQ ID NO: 162, SEQ ID NO: 164, or SEQ ID NO: 166 of preferably at least 60%, more preferably at least 65%, more preferably at least 70%, more preferably at least 75%, more preferably at least 80%, more preferably at least 85%, even more preferably at least 90%, most preferably at least 91%, at least 92%, at least 93%, at least 94%, or at least 95%, or at least 100% and even most preferably at least 96%, at least 97%, at least 98%, at least 99%, or at least 100%.

In a preferred aspect, the mature polypeptide is amino acids 20 to 326 of SEQ ID NO: 2, amino acids 18 to 239 of SEQ ID NO: 4, amino acids 20 to 258 of SEQ ID NO: 6, amino acids 19 to 226 of SEQ ID NO: 8, amino acids 20 to 304 of SEQ ID NO: 10, amino acids 23 to 250 of SEQ ID NO: 12, amino acids 22 to 249 of SEQ ID NO: 14, amino acids 20 to 249 of SEQ ID NO: 16, amino acids 18 to 232 of SEQ ID NO: 18, amino acids 16 to 235 of SEQ ID NO: 20, amino acids 19 to 323 of SEQ ID NO: 22, amino acids 16 to 310 of SEQ ID NO: 24, amino acids 20 to 246 of SEQ ID NO: 26, amino acids 22 to 354 of SEQ ID NO: 28, amino acids 22 to 250 of SEQ ID NO: 30, or amino acids 22 to 322 of SEQ ID NO: 32, amino acids 24 to 444 of SEQ ID NO: 34, amino acids 26 to 253 of SEQ ID NO: 36, amino acids 20 to 223 of SEQ ID NO: 38, amino acids 18 to 246 of SEQ ID NO: 40, amino acids 20 to 334 of SEQ ID NO: 42, amino acids 18 to 227 of SEQ ID NO: 44, amino acids 22 to 368 of SEQ ID NO: 46, amino acids 25 to 330 of SEQ ID NO: 48, amino acids 17 to 236 of SEQ ID NO: 50, amino acids 17 to 250 of SEQ ID NO: 52, amino acids 23 to 478 of SEQ ID NO: 54, amino acids 17 to 230 of SEQ ID NO: 56, amino acids 20 to 257 of SEQ ID NO: 58, amino acids 23 to 251 of SEQ ID NO: 60, amino acids 19 to 349 of SEQ ID NO: 62, amino acids 24 to 436 of SEQ ID NO: 64, amino acids 21 to 344 of SEQ ID NO: 144, amino acids 21 to 389 of SEQ ID NO: 146, amino acids 22 to 406 of SEQ ID NO: 148, amino acids 20 to 427 of SEQ ID NO: 150, amino acids 18 to 267 of SEQ ID NO: 152, amino acids 21 to 273 of SEQ ID NO: 154, amino acids 21 to 322 of SEQ ID NO: 156, amino acids 18 to 234 of SEQ ID NO: 158, amino acids 24 to 233 of SEQ ID NO: 160, amino acids 17 to 237 of SEQ ID NO: 162, amino acids 20 to 484 of SEQ ID NO: 164, or amino acids 22 to 320 of SEQ ID NO: 166.

A polypeptide having cellulolytic enhancing activity preferably comprises or consists of the amino acid sequence of SEQ ID NO: 2 or an allelic variant thereof; or a fragment thereof that has cellulolytic enhancing activity. In a preferred aspect, the polypeptide comprises or consists of the amino acid sequence of SEQ ID NO: 2. In another preferred aspect, the polypeptide comprises or consists of the mature polypeptide of SEQ ID NO: 2. In another preferred aspect, the polypeptide comprises or consists of amino acids 20 to 326 of SEQ ID NO: 2, or an allelic variant thereof; or a fragment thereof that has cellulolytic enhancing activity. In another preferred aspect, the polypeptide comprises or consists of amino acids 20 to 326 of SEQ ID NO: 2.

A polypeptide having cellulolytic enhancing activity preferably comprises or consists of the amino acid sequence of SEQ ID NO: 4 or an allelic variant thereof; or a fragment thereof that has cellulolytic enhancing activity. In a preferred aspect, the polypeptide comprises or consists of the amino acid sequence of SEQ ID NO: 4. In another preferred aspect, the polypeptide comprises or consists of the mature polypeptide of SEQ ID NO: 4. In another preferred aspect, the polypeptide comprises or consists of amino acids 18 to 239 of SEQ ID NO: 4, or an allelic variant thereof; or a fragment thereof that has cellulolytic enhancing activity. In another preferred aspect, the polypeptide comprises or consists of amino acids 18 to 239 of SEQ ID NO: 4.

A polypeptide having cellulolytic enhancing activity preferably comprises or consists of the amino acid sequence of SEQ ID NO: 6 or an allelic variant thereof; or a fragment thereof that has cellulolytic enhancing activity. In a preferred aspect, the polypeptide comprises or consists of the amino acid sequence of SEQ ID NO: 6. In another preferred aspect, the polypeptide comprises or consists of the mature polypeptide of SEQ ID NO: 6. In another preferred aspect, the polypeptide comprises or consists of amino acids 20 to 258 of SEQ ID NO: 6, or an allelic variant thereof; or a fragment thereof that has cellulolytic enhancing activity. In another preferred aspect, the polypeptide comprises or consists of amino acids 20 to 258 of SEQ ID NO: 6.

A polypeptide having cellulolytic enhancing activity preferably comprises or consists of the amino acid sequence of SEQ ID NO: 8 or an allelic variant thereof; or a fragment thereof that has cellulolytic enhancing activity. In a preferred aspect, the polypeptide comprises or consists of the amino acid sequence of SEQ ID NO: 8. In another preferred aspect, the polypeptide comprises or consists of the mature polypeptide of SEQ ID NO: 8. In another preferred aspect, the polypeptide comprises or consists of amino acids 19 to 226 of SEQ ID NO: 8, or an allelic variant thereof; or a fragment thereof that has cellulolytic enhancing activity. In another preferred aspect, the polypeptide comprises or consists of amino acids 19 to 226 of SEQ ID NO: 8.

A polypeptide having cellulolytic enhancing activity preferably comprises or consists of the amino acid sequence of SEQ ID NO: 10 or an allelic variant thereof; or a fragment thereof that has cellulolytic enhancing activity. In a preferred aspect, the polypeptide comprises or consists of the amino acid sequence of SEQ ID NO: 10. In another preferred aspect, the polypeptide comprises or consists of the mature polypeptide of SEQ ID NO: 10. In another preferred aspect, the polypeptide comprises or consists of amino acids 20 to 304 of SEQ ID NO: 10, or an allelic variant thereof; or a fragment thereof that has cellulolytic enhancing activity. In another preferred aspect, the polypeptide comprises or consists of amino acids 20 to 304 of SEQ ID NO: 10.

A polypeptide having cellulolytic enhancing activity preferably comprises or consists of the amino acid sequence of SEQ ID NO: 12 or an allelic variant thereof; or a fragment thereof having cellulolytic enhancing activity. In a preferred aspect, the polypeptide comprises or consists of the amino acid sequence of SEQ ID NO: 12. In another preferred aspect, the polypeptide comprises or consists of the mature polypeptide of SEQ ID NO: 12. In another preferred aspect, the polypeptide comprises or consists of amino acids 16 to 317 of SEQ ID NO: 12, or an allelic variant thereof; or a fragment thereof having cellulolytic enhancing activity. In another preferred aspect, the polypeptide comprises or consists of amino acids 16 to 317 of SEQ ID NO: 12.

A polypeptide having cellulolytic enhancing activity preferably comprises or consists of the amino acid sequence of SEQ ID NO: 14 or an allelic variant thereof; or a fragment thereof that has cellulolytic enhancing activity. In a preferred aspect, the polypeptide comprises or consists of the amino acid sequence of SEQ ID NO: 14. In another preferred aspect, the polypeptide comprises or consists of the mature polypeptide of SEQ ID NO: 14. In another preferred aspect, the polypeptide comprises or consists of amino acids 23 to 250 of SEQ ID NO: 14, or an allelic variant thereof; or a fragment thereof that has cellulolytic enhancing activity. In another preferred aspect, the polypeptide comprises or consists of amino acids 23 to 250 of SEQ ID NO: 14.

A polypeptide having cellulolytic enhancing activity preferably comprises or consists of the amino acid sequence of SEQ ID NO: 16 or an allelic variant thereof; or a fragment thereof that has cellulolytic enhancing activity. In a preferred aspect, the polypeptide comprises or consists of the amino acid sequence of SEQ ID NO: 16. In another preferred aspect, the polypeptide comprises or consists of the mature polypeptide of SEQ ID NO: 16. In another preferred aspect, the polypeptide comprises or consists of amino acids 20 to 249 of SEQ ID NO: 16, or an allelic variant thereof; or a fragment thereof that has cellulolytic enhancing activity. In another preferred aspect, the polypeptide comprises or consists of amino acids 20 to 249 of SEQ ID NO: 16.

A polypeptide having cellulolytic enhancing activity preferably comprises or consists of the amino acid sequence of SEQ ID NO: 18 or an allelic variant thereof; or a fragment thereof that has cellulolytic enhancing activity. In a preferred aspect, the polypeptide comprises or consists of the amino acid sequence of SEQ ID NO: 18. In another preferred aspect, the polypeptide comprises or consists of the mature polypeptide of SEQ ID NO: 18. In another preferred aspect, the polypeptide comprises or consists of amino acids 18 to 232 of SEQ ID NO: 18, or an allelic variant thereof; or a fragment thereof that has cellulolytic enhancing activity. In another preferred aspect, the polypeptide comprises or consists of amino acids 18 to 232 of SEQ ID NO: 18.

A polypeptide having cellulolytic enhancing activity preferably comprises or consists of the amino acid sequence of SEQ ID NO: 20 or an allelic variant thereof; or a fragment thereof that has cellulolytic enhancing activity. In a preferred aspect, the polypeptide comprises or consists of the amino acid sequence of SEQ ID NO: 20. In another preferred aspect, the polypeptide comprises or consists of the mature polypeptide of SEQ ID NO: 20. In another preferred aspect, the polypeptide comprises or consists of amino acids 16 to 235 of SEQ ID NO: 20, or an allelic variant thereof; or a fragment thereof that has cellulolytic enhancing activity. In another preferred aspect, the polypeptide comprises or consists of amino acids 16 to 235 of SEQ ID NO: 20.

A polypeptide having cellulolytic enhancing activity preferably comprises or consists of the amino acid sequence of SEQ ID NO: 22 or an allelic variant thereof; or a fragment thereof that has cellulolytic enhancing activity. In a preferred aspect, the polypeptide comprises or consists of the amino acid sequence of SEQ ID NO: 22. In another preferred aspect, the polypeptide comprises or consists of the mature polypeptide of SEQ ID NO: 22. In another preferred aspect, the polypeptide comprises or consists of amino acids 19 to 323 of SEQ ID NO: 22, or an allelic variant thereof; or a fragment thereof that has cellulolytic enhancing activity. In another preferred aspect, the polypeptide comprises or consists of amino acids 19 to 323 of SEQ ID NO: 22.

A polypeptide having cellulolytic enhancing activity preferably comprises or consists of the amino acid sequence of SEQ ID NO: 24 or an allelic variant thereof; or a fragment thereof that has cellulolytic enhancing activity. In a preferred aspect, the polypeptide comprises or consists of the amino acid sequence of SEQ ID NO: 24. In another preferred aspect, the polypeptide comprises or consists of the mature polypeptide of SEQ ID NO: 24. In another preferred aspect, the polypeptide comprises or consists of amino acids 16 to 310 of SEQ ID NO: 24, or an allelic variant thereof; or a fragment thereof that has cellulolytic enhancing activity. In another preferred aspect, the polypeptide comprises or consists of amino acids 16 to 310 of SEQ ID NO: 24.

A polypeptide having cellulolytic enhancing activity preferably comprises or consists of the amino acid sequence of SEQ ID NO: 26 or an allelic variant thereof; or a fragment thereof that has cellulolytic enhancing activity. In a preferred aspect, the polypeptide comprises or consists of the amino acid sequence of SEQ ID NO: 26. In another preferred aspect, the polypeptide comprises or consists of the mature polypeptide of SEQ ID NO: 26. In another preferred aspect, the polypeptide comprises or consists of amino acids 20 to 246 of SEQ ID NO: 26, or an allelic variant thereof; or a fragment thereof that has cellulolytic enhancing activity. In another preferred aspect, the polypeptide comprises or consists of amino acids 20 to 246 of SEQ ID NO: 26.

A polypeptide having cellulolytic enhancing activity preferably comprises or consists of the amino acid sequence of SEQ ID NO: 28 or an allelic variant thereof; or a fragment thereof that has cellulolytic enhancing activity. In a preferred aspect, the polypeptide comprises or consists of the amino acid sequence of SEQ ID NO: 28. In another preferred aspect, the polypeptide comprises or consists of the mature polypeptide of SEQ ID NO: 28. In another preferred aspect, the polypeptide comprises or consists of amino acids 22 to 354 of SEQ ID NO: 28, or an allelic variant thereof; or a fragment thereof that has cellulolytic enhancing activity. In another preferred aspect, the polypeptide comprises or consists of amino acids 22 to 354 of SEQ ID NO: 28.

A polypeptide having cellulolytic enhancing activity preferably comprises or consists of the amino acid sequence of SEQ ID NO: 30 or an allelic variant thereof; or a fragment thereof that has cellulolytic enhancing activity. In a preferred aspect, the polypeptide comprises or consists of the amino acid sequence of SEQ ID NO: 30. In another preferred aspect, the polypeptide comprises or consists of the mature polypeptide of SEQ ID NO: 30. In another preferred aspect, the polypeptide comprises or consists of amino acids 22 to 250 of SEQ ID NO: 30, or an allelic variant thereof; or a fragment thereof that has cellulolytic enhancing activity. In another preferred aspect, the polypeptide comprises or consists of amino acids 22 to 250 of SEQ ID NO: 30.

A polypeptide having cellulolytic enhancing activity preferably comprises or consists of the amino acid sequence of SEQ ID NO: 32 or an allelic variant thereof; or a fragment thereof that has cellulolytic enhancing activity. In a preferred aspect, the polypeptide comprises or consists of the amino acid sequence of SEQ ID NO: 32. In another preferred aspect, the polypeptide comprises or consists of the mature polypeptide of SEQ ID NO: 32. In another preferred aspect, the polypeptide comprises or consists of amino acids 22 to 322 of SEQ ID NO: 32, or an allelic variant thereof; or a fragment thereof that has cellulolytic enhancing activity. In another preferred aspect, the polypeptide comprises or consists of amino acids 22 to 322 of SEQ ID NO: 32.

A polypeptide having cellulolytic enhancing activity preferably comprises or consists of the amino acid sequence of SEQ ID NO: 34 or an allelic variant thereof; or a fragment thereof that has cellulolytic enhancing activity. In a preferred aspect, the polypeptide comprises or consists of the amino acid sequence of SEQ ID NO: 34. In another preferred aspect, the polypeptide comprises or consists of the mature polypeptide of SEQ ID NO: 34. In another preferred aspect, the polypeptide comprises or consists of amino acids 24 to 444 of SEQ ID NO: 34, or an allelic variant thereof; or a fragment thereof that has cellulolytic enhancing activity. In another preferred aspect, the polypeptide comprises or consists of amino acids 24 to 444 of SEQ ID NO: 34.

A polypeptide having cellulolytic enhancing activity preferably comprises or consists of the amino acid sequence of SEQ ID NO: 36 or an allelic variant thereof; or a fragment thereof that has cellulolytic enhancing activity. In a preferred aspect, the polypeptide comprises or consists of the amino acid sequence of SEQ ID NO: 36. In another preferred aspect, the polypeptide comprises or consists of the mature polypeptide of SEQ ID NO: 36. In another preferred aspect, the polypeptide comprises or consists of amino acids 26 to 253 of SEQ ID NO: 36, or an allelic variant thereof; or a fragment thereof that has cellulolytic enhancing activity. In another preferred aspect, the polypeptide comprises or consists of amino acids 26 to 253 of SEQ ID NO: 36.

A polypeptide having cellulolytic enhancing activity preferably comprises or consists of the amino acid sequence of SEQ ID NO: 38 or an allelic variant thereof; or a fragment thereof that has cellulolytic enhancing activity. In a preferred aspect, the polypeptide comprises or consists of the amino acid sequence of SEQ ID NO: 38. In another preferred aspect, the polypeptide comprises or consists of the mature polypeptide of SEQ ID NO: 38. In another preferred aspect, the polypeptide comprises or consists of amino acids 20 to 223 of SEQ ID NO: 38, or an allelic variant thereof; or a fragment thereof that has cellulolytic enhancing activity. In another preferred aspect, the polypeptide comprises or consists of amino acids 20 to 223 of SEQ ID NO: 38.

A polypeptide having cellulolytic enhancing activity preferably comprises or consists of the amino acid sequence of SEQ ID NO: 40 or an allelic variant thereof; or a fragment thereof that has cellulolytic enhancing activity. In a preferred aspect, the polypeptide comprises or consists of the amino acid sequence of SEQ ID NO: 40. In another preferred aspect, the polypeptide comprises or consists of the mature polypeptide of SEQ ID NO: 40. In another preferred aspect, the polypeptide comprises or consists of amino acids 18 to 246 of SEQ ID NO: 40, or an allelic variant thereof; or a fragment thereof that has cellulolytic enhancing activity. In another preferred aspect, the polypeptide comprises or consists of amino acids 18 to 246 of SEQ ID NO: 40.

A polypeptide having cellulolytic enhancing activity preferably comprises or consists of the amino acid sequence of SEQ ID NO: 42 or an allelic variant thereof; or a fragment thereof that has cellulolytic enhancing activity. In a preferred aspect, the polypeptide comprises or consists of the amino acid sequence of SEQ ID NO: 42. In another preferred aspect, the polypeptide comprises or consists of the mature polypeptide of SEQ ID NO: 42. In another preferred aspect, the polypeptide comprises or consists of amino acids 20 to 334 of SEQ ID NO: 42, or an allelic variant thereof; or a fragment thereof that has cellulolytic enhancing activity. In another preferred aspect, the polypeptide comprises or consists of amino acids 20 to 334 of SEQ ID NO: 42.

A polypeptide having cellulolytic enhancing activity preferably comprises or consists of the amino acid sequence of SEQ ID NO: 44 or an allelic variant thereof; or a fragment thereof that has cellulolytic enhancing activity. In a preferred aspect, the polypeptide comprises or consists of the amino acid sequence of SEQ ID NO: 44. In another preferred aspect, the polypeptide comprises or consists of the mature polypeptide of SEQ ID NO: 44. In another preferred aspect, the polypeptide comprises or consists of amino acids 18 to 227 of SEQ ID NO: 44, or an allelic variant thereof; or a fragment thereof that has cellulolytic enhancing activity. In another preferred aspect, the polypeptide comprises or consists of amino acids 18 to 227 of SEQ ID NO: 44.

A polypeptide having cellulolytic enhancing activity preferably comprises or consists of the amino acid sequence of SEQ ID NO: 46 or an allelic variant thereof; or a fragment thereof that has cellulolytic enhancing activity. In a preferred aspect, the polypeptide comprises or consists of the amino acid sequence of SEQ ID NO: 46. In another preferred aspect, the polypeptide comprises or consists of the mature polypeptide of SEQ ID NO: 46. In another preferred aspect, the polypeptide comprises or consists of amino acids 22 to 368 of SEQ ID NO: 46, or an allelic variant thereof; or a fragment thereof that has cellulolytic enhancing activity. In another preferred aspect, the polypeptide comprises or consists of amino acids 22 to 368 of SEQ ID NO: 46.

A polypeptide having cellulolytic enhancing activity preferably comprises or consists of the amino acid sequence of SEQ ID NO: 48 or an allelic variant thereof; or a fragment thereof that has cellulolytic enhancing activity. In a preferred aspect, the polypeptide comprises or consists of the amino acid sequence of SEQ ID NO: 48. In another preferred aspect, the polypeptide comprises or consists of the mature polypeptide of SEQ ID NO: 48. In another preferred aspect, the polypeptide comprises or consists of amino acids 25 to 330 of SEQ ID NO: 48, or an allelic variant thereof; or a fragment thereof that has cellulolytic enhancing activity. In another preferred aspect, the polypeptide comprises or consists of amino acids 25 to 330 of SEQ ID NO: 48.

A polypeptide having cellulolytic enhancing activity preferably comprises or consists of the amino acid sequence of SEQ ID NO: 50 or an allelic variant thereof; or a fragment thereof that has cellulolytic enhancing activity. In a preferred aspect, the polypeptide comprises or consists of the amino acid sequence of SEQ ID NO: 50. In another preferred aspect, the polypeptide comprises or consists of the mature polypeptide of SEQ ID NO: 50. In another preferred aspect, the polypeptide comprises or consists of amino acids 17 to 236 of SEQ ID NO: 50, or an allelic variant thereof; or a fragment thereof that has cellulolytic enhancing activity. In another preferred aspect, the polypeptide comprises or consists of amino acids 17 to 236 of SEQ ID NO: 50.

A polypeptide having cellulolytic enhancing activity preferably comprises or consists of the amino acid sequence of SEQ ID NO: 52 or an allelic variant thereof; or a fragment thereof that has cellulolytic enhancing activity. In a preferred aspect, the polypeptide comprises or consists of the amino acid sequence of SEQ ID NO: 52. In another preferred aspect, the polypeptide comprises or consists of the mature polypeptide of SEQ ID NO: 52. In another preferred aspect, the polypeptide comprises or consists of amino acids 19 to 250 of SEQ ID NO: 52, or an allelic variant thereof; or a fragment thereof that has cellulolytic enhancing activity. In another preferred aspect, the polypeptide comprises or consists of amino acids 19 to 250 of SEQ ID NO: 52.

A polypeptide having cellulolytic enhancing activity preferably comprises or consists of the amino acid sequence of SEQ ID NO: 54 or an allelic variant thereof; or a fragment thereof that has cellulolytic enhancing activity. In a preferred aspect, the polypeptide comprises or consists of the amino acid sequence of SEQ ID NO: 54. In another preferred aspect, the polypeptide comprises or consists of the mature polypeptide of SEQ ID NO: 54. In another preferred aspect, the polypeptide comprises or consists of amino acids 23 to 478 of SEQ ID NO: 54, or an allelic variant thereof; or a fragment thereof that has cellulolytic enhancing activity. In another preferred aspect, the polypeptide comprises or consists of amino acids 23 to 478 of SEQ ID NO: 54.

A polypeptide having cellulolytic enhancing activity preferably comprises or consists of the amino acid sequence of SEQ ID NO: 56 or an allelic variant thereof; or a fragment thereof that has cellulolytic enhancing activity. In a preferred aspect, the polypeptide comprises or consists of the amino acid sequence of SEQ ID NO: 56. In another preferred aspect, the polypeptide comprises or consists of the mature polypeptide of SEQ ID NO: 56. In another preferred aspect, the polypeptide comprises or consists of amino acids 17 to 230 of SEQ ID NO: 56, or an allelic variant thereof; or a fragment thereof that has cellulolytic enhancing activity. In another preferred aspect, the polypeptide comprises or consists of amino acids 17 to 230 of SEQ ID NO: 56.

A polypeptide having cellulolytic enhancing activity preferably comprises or consists of the amino acid sequence of SEQ ID NO: 58 or an allelic variant thereof; or a fragment thereof that has cellulolytic enhancing activity. In a preferred aspect, the polypeptide comprises or consists of the amino acid sequence of SEQ ID NO: 58. In another preferred aspect, the polypeptide comprises or consists of the mature polypeptide of SEQ ID NO: 58. In another preferred aspect, the polypeptide comprises or consists of amino acids 20 to 257 of SEQ ID NO: 58, or an allelic variant thereof; or a fragment thereof that has cellulolytic enhancing activity. In another preferred aspect, the polypeptide comprises or consists of amino acids 20 to 257 of SEQ ID NO: 58.

A polypeptide having cellulolytic enhancing activity preferably comprises or consists of the amino acid sequence of SEQ ID NO: 60 or an allelic variant thereof; or a fragment thereof that has cellulolytic enhancing activity. In a preferred aspect, the polypeptide comprises or consists of the amino acid sequence of SEQ ID NO: 60. In another preferred aspect, the polypeptide comprises or consists of the mature polypeptide of SEQ ID NO: 60. In another preferred aspect, the polypeptide comprises or consists of amino acids 23 to 251 of SEQ ID NO: 60, or an allelic variant thereof; or a fragment thereof that has cellulolytic enhancing activity. In another preferred aspect, the polypeptide comprises or consists of amino acids 23 to 251 of SEQ ID NO: 60.

A polypeptide having cellulolytic enhancing activity preferably comprises or consists of the amino acid sequence of SEQ ID NO: 62 or an allelic variant thereof; or a fragment thereof that has cellulolytic enhancing activity. In a preferred aspect, the polypeptide comprises or consists of the amino acid sequence of SEQ ID NO: 62. In another preferred aspect, the polypeptide comprises or consists of the mature polypeptide of SEQ ID NO: 62. In another preferred aspect, the polypeptide comprises or consists of amino acids 19 to 349 of SEQ ID NO: 62, or an allelic variant thereof; or a fragment thereof that has cellulolytic enhancing activity. In another preferred aspect, the polypeptide comprises or consists of amino acids 19 to 349 of SEQ ID NO: 62.

A polypeptide having cellulolytic enhancing activity preferably comprises or consists of the amino acid sequence of SEQ ID NO: 64 or an allelic variant thereof; or a fragment thereof that has cellulolytic enhancing activity. In a preferred aspect, the polypeptide comprises or consists of the amino acid sequence of SEQ ID NO: 64. In another preferred aspect, the polypeptide comprises or consists of the mature polypeptide of SEQ ID NO: 64. In another preferred aspect, the polypeptide comprises or consists of amino acids 24 to 436 of SEQ ID NO: 64, or an allelic variant thereof; or a fragment thereof that has cellulolytic enhancing activity. In another preferred aspect, the polypeptide comprises or consists of amino acids 24 to 436 of SEQ ID NO: 64.

A polypeptide having cellulolytic enhancing activity preferably comprises or consists of the amino acid sequence of SEQ ID NO: 144 or an allelic variant thereof; or a fragment thereof that has cellulolytic enhancing activity. In a preferred aspect, the polypeptide comprises or consists of the amino acid sequence of SEQ ID NO: 144. In another preferred aspect, the polypeptide comprises or consists of the mature polypeptide of SEQ ID NO: 144. In another preferred aspect, the polypeptide comprises or consists of amino acids 21 to 344 of SEQ ID NO: 144, or an allelic variant thereof; or a fragment thereof that has cellulolytic enhancing activity. In another preferred aspect, the polypeptide comprises or consists of amino acids 21 to 344 of SEQ ID NO: 144.

A polypeptide having cellulolytic enhancing activity preferably comprises or consists of the amino acid sequence of SEQ ID NO: 146 or an allelic variant thereof; or a fragment thereof that has cellulolytic enhancing activity. In a preferred aspect, the polypeptide comprises or consists of the amino acid sequence of SEQ ID NO: 146. In another preferred aspect, the polypeptide comprises or consists of the mature polypeptide of SEQ ID NO: 146. In another preferred aspect, the polypeptide comprises or consists of amino acids 21 to 389 of SEQ ID NO: 146, or an allelic variant thereof; or a fragment thereof that has cellulolytic enhancing activity. In another preferred aspect, the polypeptide comprises or consists of amino acids 21 to 389 of SEQ ID NO: 146.

A polypeptide having cellulolytic enhancing activity preferably comprises or consists of the amino acid sequence of SEQ ID NO: 148 or an allelic variant thereof; or a fragment thereof that has cellulolytic enhancing activity. In a preferred aspect, the polypeptide comprises or consists of the amino acid sequence of SEQ ID NO: 148. In another preferred aspect, the polypeptide comprises or consists of the mature polypeptide of SEQ ID NO: 148. In another preferred aspect, the polypeptide comprises or consists of amino acids 22 to 406 of SEQ ID NO: 148, or an allelic variant thereof; or a fragment thereof that has cellulolytic enhancing activity. In another preferred aspect, the polypeptide comprises or consists of amino acids 22 to 406 of SEQ ID NO: 148.

A polypeptide having cellulolytic enhancing activity preferably comprises or consists of the amino acid sequence of SEQ ID NO: 150 or an allelic variant thereof; or a fragment thereof that has cellulolytic enhancing activity. In a preferred aspect, the polypeptide comprises or consists of the amino acid sequence of SEQ ID NO: 150. In another preferred aspect, the polypeptide comprises or consists of the mature polypeptide of SEQ ID NO: 150. In another preferred aspect, the polypeptide comprises or consists of amino acids 20 to 427 of SEQ ID NO: 150, or an allelic variant thereof; or a fragment thereof that has cellulolytic enhancing activity. In another preferred aspect, the polypeptide comprises or consists of amino acids 20 to 427 of SEQ ID NO: 150.

A polypeptide having cellulolytic enhancing activity preferably comprises or consists of the amino acid sequence of SEQ ID NO: 152 or an allelic variant thereof; or a fragment thereof that has cellulolytic enhancing activity. In a preferred aspect, the polypeptide comprises or consists of the amino acid sequence of SEQ ID NO: 152. In another preferred aspect, the polypeptide comprises or consists of the mature polypeptide of SEQ ID NO: 152. In another preferred aspect, the polypeptide comprises or consists of amino acids 18 to 267 of SEQ ID NO: 152, or an allelic variant thereof; or a fragment thereof that has cellulolytic enhancing activity. In another preferred aspect, the polypeptide comprises or consists of amino acids 18 to 267 of SEQ ID NO: 152.

A polypeptide having cellulolytic enhancing activity preferably comprises or consists of the amino acid sequence of SEQ ID NO: 154 or an allelic variant thereof; or a fragment thereof that has cellulolytic enhancing activity. In a preferred aspect, the polypeptide comprises or consists of the amino acid sequence of SEQ ID NO: 154. In another preferred aspect, the polypeptide comprises or consists of the mature polypeptide of SEQ ID NO: 154. In another preferred aspect, the polypeptide comprises or consists of amino acids 21 to 273 of SEQ ID NO: 154, or an allelic variant thereof; or a fragment thereof that has cellulolytic enhancing activity. In another preferred aspect, the polypeptide comprises or consists of amino acids 21 to 273 of SEQ ID NO: 154.

A polypeptide having cellulolytic enhancing activity preferably comprises or consists of the amino acid sequence of SEQ ID NO: 156 or an allelic variant thereof; or a fragment thereof that has cellulolytic enhancing activity. In a preferred aspect, the polypeptide comprises or consists of the amino acid sequence of SEQ ID NO: 156. In another preferred aspect, the polypeptide comprises or consists of the mature polypeptide of SEQ ID NO: 156. In another preferred aspect, the polypeptide comprises or consists of amino acids 21 to 322 of SEQ ID NO: 156, or an allelic variant thereof; or a fragment thereof that has cellulolytic enhancing activity. In another preferred aspect, the polypeptide comprises or consists of amino acids 21 to 322 of SEQ ID NO: 156.

A polypeptide having cellulolytic enhancing activity preferably comprises or consists of the amino acid sequence of SEQ ID NO: 158 or an allelic variant thereof; or a fragment thereof that has cellulolytic enhancing activity. In a preferred aspect, the polypeptide comprises or consists of the amino acid sequence of SEQ ID NO: 158. In another preferred aspect, the polypeptide comprises or consists of the mature polypeptide of SEQ ID NO: 158. In another preferred aspect, the polypeptide comprises or consists of amino acids 18 to 234 of SEQ ID NO: 158, or an allelic variant thereof; or a fragment thereof that has cellulolytic enhancing activity. In another preferred aspect, the polypeptide comprises or consists of amino acids 18 to 234 of SEQ ID NO: 158.

A polypeptide having cellulolytic enhancing activity preferably comprises or consists of the amino acid sequence of SEQ ID NO: 160 or an allelic variant thereof; or a fragment thereof that has cellulolytic enhancing activity. In a preferred aspect, the polypeptide comprises or consists of the amino acid sequence of SEQ ID NO: 160. In another preferred aspect, the polypeptide comprises or consists of the mature polypeptide of SEQ ID NO: 160. In another preferred aspect, the polypeptide comprises or consists of amino acids 24 to 233 of SEQ ID NO: 160, or an allelic variant thereof; or a fragment thereof that has cellulolytic enhancing activity. In another preferred aspect, the polypeptide comprises or consists of amino acids 24 to 233 of SEQ ID NO: 160.

A polypeptide having cellulolytic enhancing activity preferably comprises or consists of the amino acid sequence of SEQ ID NO: 162 or an allelic variant thereof; or a fragment thereof that has cellulolytic enhancing activity. In a preferred aspect, the polypeptide comprises or consists of the amino acid sequence of SEQ ID NO: 162. In another preferred aspect, the polypeptide comprises or consists of the mature polypeptide of SEQ ID NO: 162. In another preferred aspect, the polypeptide comprises or consists of amino acids 17 to 237 of SEQ ID NO: 162, or an allelic variant thereof; or a fragment thereof that has cellulolytic enhancing activity. In another preferred aspect, the polypeptide comprises or consists of amino acids 17 to 237 of SEQ ID NO: 162.

A polypeptide having cellulolytic enhancing activity preferably comprises or consists of the amino acid sequence of SEQ ID NO: 164 or an allelic variant thereof; or a fragment thereof that has cellulolytic enhancing activity. In a preferred aspect, the polypeptide comprises or consists of the amino acid sequence of SEQ ID NO: 164. In another preferred aspect, the polypeptide comprises or consists of the mature polypeptide of SEQ ID NO: 164. In another preferred aspect, the polypeptide comprises or consists of amino acids 20 to 484 of SEQ ID NO: 164, or an allelic variant thereof; or a fragment thereof that has cellulolytic enhancing activity. In another preferred aspect, the polypeptide comprises or consists of amino acids 20 to 484 of SEQ ID NO: 164.

A polypeptide having cellulolytic enhancing activity preferably comprises or consists of the amino acid sequence of SEQ ID NO: 166 or an allelic variant thereof; or a fragment thereof that has cellulolytic enhancing activity. In a preferred aspect, the polypeptide comprises or consists of the amino acid sequence of SEQ ID NO: 166. In another preferred aspect, the polypeptide comprises or consists of the mature polypeptide of SEQ ID NO: 166. In another preferred aspect, the polypeptide comprises or consists of amino acids 22 to 320 of SEQ ID NO: 166, or an allelic variant thereof; or a fragment thereof that has cellulolytic enhancing activity. In another preferred aspect, the polypeptide comprises or consists of amino acids 22 to 320 of SEQ ID NO: 166.

Preferably, a fragment of the mature polypeptide of SEQ ID NO: 2 contains at least 277 amino acid residues, more preferably at least 287 amino acid residues, and most preferably at least 297 amino acid residues. Preferably, a fragment of the mature polypeptide of SEQ ID NO: 4 contains at least 185 amino acid residues, more preferably at least 195 amino acid residues, and most preferably at least 205 amino acid residues. Preferably, a fragment of the mature polypeptide of SEQ ID NO: 6 contains at least 200 amino acid residues, more preferably at least 212 amino acid residues, and most preferably at least 224 amino acid residues. Preferably, a fragment of the mature polypeptide of SEQ ID NO: 8 contains at least 175 amino acid residues, more preferably at least 185 amino acid residues, and most preferably at least 195 amino acid residues. Preferably, a fragment of the mature polypeptide of SEQ ID NO: 10 contains at least 240 amino acid residues, more preferably at least 255 amino acid residues, and most preferably at least 270 amino acid residues. Preferably, a fragment of the mature polypeptide of SEQ ID NO: 12 contains at least 255 amino acid residues, more preferably at least 270 amino acid residues, and most preferably at least 285 amino acid residues. Preferably, a fragment of the mature polypeptide of SEQ ID NO: 14 contains at least 175 amino acid residues, more preferably at least 190 amino acid residues, and most preferably at least 205 amino acid residues. Preferably, a fragment of the mature polypeptide of SEQ ID NO: 16 contains at least 200 amino acid residues, more preferably at least 210 amino acid residues, and most preferably at least 220 amino acid residues. Preferably, a fragment of the mature polypeptide of SEQ ID NO: 18 contains at least 185 amino acid residues, more preferably at least 195 amino acid residues, and most preferably at least 205 amino acid residues. Preferably, a fragment of the mature polypeptide of SEQ ID NO: 20 contains at least 190 amino acid residues, more preferably at least 200 amino acid residues, and most preferably at least 210 amino acid residues. Preferably, a fragment of the mature polypeptide of SEQ ID NO: 22 contains at least 260 amino acid residues, more preferably at least 275 amino acid residues, and most preferably at least 290 amino acid residues. Preferably, a fragment of the mature polypeptide of SEQ ID NO: 24 contains at least 250 amino acid residues, more preferably at least 265 amino acid residues, and most preferably at least 280 amino acid residues. Preferably, a fragment of the mature polypeptide of SEQ ID NO: 26 contains at least 195 amino acid residues, more preferably at least 205 amino acid residues, and most preferably at least 214 amino acid residues. Preferably, a fragment of the mature polypeptide of SEQ ID NO: 28 contains at least 285 amino acid residues, more preferably at least 300 amino acid residues, and most preferably at least 315 amino acid residues. Preferably, a fragment of the mature polypeptide of SEQ ID NO: 30 contains at least 200 amino acid residues, more preferably at least 210 amino acid residues, and most preferably at least 220 amino acid residues. Preferably, a fragment of the mature polypeptide of SEQ ID NO: 32 contains at least 255 amino acid residues, more preferably at least 270 amino acid residues, and most preferably at least 285 amino acid residues. Preferably, a fragment of the mature polypeptide of SEQ ID NO: 34 contains at least 360 amino acid residues, more preferably at least 380 amino acid residues, and most preferably at least 400 amino acid residues. Preferably, a fragment of the mature polypeptide of SEQ ID NO: 36 contains at least 200 amino acid residues, more preferably at least 210 amino acid residues, and most preferably at least 220 amino acid residues. Preferably, a fragment of the mature polypeptide of SEQ ID NO: 38 contains at least 170 amino acid residues, more preferably at least 180 amino acid residues, and most preferably at least 190 amino acid residues. Preferably, a fragment of the mature polypeptide of SEQ ID NO: 40 contains at least 190 amino acid residues, more preferably at least 200 amino acid residues, and most preferably at least 210 amino acid residues. Preferably, a fragment of the mature polypeptide of SEQ ID NO: 42 contains at least 265 amino acid residues, more preferably at least 280 amino acid residues, and most preferably at least 295 amino acid residues. Preferably, a fragment of the mature polypeptide of SEQ ID NO: 44 contains at least 180 amino acid residues, more preferably at least 190 amino acid residues, and most preferably at least 200 amino acid residues. Preferably, a fragment of the mature polypeptide of SEQ ID NO: 46 contains at least 320 amino acid residues, more preferably at least 335 amino acid residues, and most preferably at least 350 amino acid residues. Preferably, a fragment of the mature polypeptide of SEQ ID NO: 48 contains at least 255 amino acid residues, more preferably at least 270 amino acid residues, and most preferably at least 285 amino acid residues. Preferably, a fragment of the mature polypeptide of SEQ ID NO: 50 contains at least 190 amino acid residues, more preferably at least 200 amino acid residues, and most preferably at least 210 amino acid residues. Preferably, a fragment of the mature polypeptide of SEQ ID NO: 52 contains at least 200 amino acid residues, more preferably at least 210 amino acid residues, and most preferably at least 220 amino acid residues. Preferably, a fragment of the mature polypeptide of SEQ ID NO: 54 contains at least 380 amino acid residues, more preferably at least 400 amino acid residues, and most preferably at least 420 amino acid residues. Preferably, a fragment of the mature polypeptide of SEQ ID NO: 56 contains at least 180 amino acid residues, more preferably at least 190 amino acid residues, and most preferably at least 200 amino acid residues. Preferably, a fragment of the mature polypeptide of SEQ ID NO: 58 contains at least 210 amino acid residues, more preferably at least 220 amino acid residues, and most preferably at least 230 amino acid residues. Preferably, a fragment of the mature polypeptide of SEQ ID NO: 60 contains at least 190 amino acid residues, more preferably at least 200 amino acid residues, and most preferably at least 210 amino acid residues. Preferably, a fragment of the mature polypeptide of SEQ ID NO: 62 contains at least 270 amino acid residues, more preferably at least 290 amino acid residues, and most preferably at least 310 amino acid residues. Preferably, a fragment of the mature polypeptide of SEQ ID NO: 64 contains at least 340 amino acid residues, more preferably at least 360 amino acid residues, and most preferably at least 380 amino acid residues. Preferably, a fragment of the mature polypeptide of SEQ ID NO: 144 contains at least 280 amino acid residues, more preferably at least 295 amino acid residues, and most preferably at least 310 amino acid residues. Preferably, a fragment of the mature polypeptide of SEQ ID NO: 146 contains at least 310 amino acid residues, more preferably at least 330 amino acid residues, and most preferably at least 350 amino acid residues. Preferably, a fragment of the mature polypeptide of SEQ ID NO: 148 contains at least 320 amino acid residues, more preferably at least 340 amino acid residues, and most preferably at least 360 amino acid residues. Preferably, a fragment of the mature polypeptide of SEQ ID NO: 150 contains at least 350 amino acid residues, more preferably at least 370 amino acid residues, and most preferably at least 390 amino acid residues. Preferably, a fragment of the mature polypeptide of SEQ ID NO: 152 contains at least 220 amino acid residues, more preferably at least 230 amino acid residues, and most preferably at least 240 amino acid residues. Preferably, a fragment of the mature polypeptide of SEQ ID NO: 154 contains at least 220 amino acid residues, more preferably at least 230 amino acid residues, and most preferably at least 240 amino acid residues. Preferably, a fragment of the mature polypeptide of SEQ ID NO: 156 contains at least 255 amino acid residues, more preferably at least 270 amino acid residues, and most preferably at least 285 amino acid residues. Preferably, a fragment of the mature polypeptide of SEQ ID NO: 158 contains at least 185 amino acid residues, more preferably at least 195 amino acid residues, and most preferably at least 205 amino acid residues. Preferably, a fragment of the mature polypeptide of SEQ ID NO: 160 contains at least 180 amino acid residues, more preferably at least 190 amino acid residues, and most preferably at least 200 amino acid residues. Preferably, a fragment of the mature polypeptide of SEQ ID NO: 162 contains at least 190 amino acid residues, more preferably at least 200 amino acid residues, and most preferably at least 210 amino acid residues. Preferably, a fragment of the mature polypeptide of SEQ ID NO: 164 contains at least 385 amino acid residues, more preferably at least 410 amino acid residues, and most preferably at least 435 amino acid residues. Preferably, a fragment of the mature polypeptide of SEQ ID NO: 166 contains at least 255 amino acid residues, more preferably at least 270 amino acid residues, and most preferably at least 285 amino acid residues.

Preferably, a subsequence of the mature polypeptide coding sequence of SEQ ID NO: 1 contains at least 831 nucleotides, more preferably at least 861 nucleotides, and most preferably at least 891 nucleotides. Preferably, a subsequence of the mature polypeptide coding sequence of SEQ ID NO: 3 contains at least 555 nucleotides, more preferably at least 585 nucleotides, and most preferably at least 615 nucleotides. Preferably, a subsequence of the mature polypeptide coding sequence of SEQ ID NO: 5 contains at least 600 nucleotides, more preferably at least 636 nucleotides, and most preferably at least 672 nucleotides. Preferably, a subsequence of the mature polypeptide coding sequence of SEQ ID NO: 7 contains at least 525 nucleotides, more preferably at least 555 nucleotides, and most preferably at least 585 nucleotides. Preferably, a subsequence of the mature polypeptide coding sequence of SEQ ID NO: 9 contains at least 720 nucleotides, more preferably at least 765 nucleotides, and most preferably at least 810 nucleotides. Preferably, a subsequence of the mature polypeptide coding sequence of SEQ ID NO: 11 contains at least 765 nucleotides, more preferably at least 810 nucleotides, and most preferably at least 855 nucleotides. Preferably, a subsequence of the mature polypeptide coding sequence of nucleotides 67 to 796 of SEQ ID NO: 13 contains at least 525 nucleotides, more preferably at least 570 nucleotides, and most preferably at least 615 nucleotides. Preferably, a subsequence of the mature polypeptide coding sequence of SEQ ID NO: 15 contains at least 600 nucleotides, more preferably at least 630 nucleotides, and most preferably at least 660 nucleotides. Preferably, a subsequence of the mature polypeptide coding sequence of SEQ ID NO: 17 contains at least 555 nucleotides, more preferably at least 585 nucleotides, and most preferably at least 615 nucleotides. Preferably, a subsequence of the mature polypeptide coding sequence of SEQ ID NO: 19 contains at least 570 nucleotides, more preferably at least 600 nucleotides, and most preferably at least 630 nucleotides. Preferably, a subsequence of the mature polypeptide coding sequence of SEQ ID NO: 21 contains at least 780 nucleotides, more preferably at least 825 nucleotides, and most preferably at least 870 nucleotides. Preferably, a subsequence of the mature polypeptide coding sequence of SEQ ID NO: 23 contains at least 750 nucleotides, more preferably at least 795 nucleotides, and most preferably at least 840 nucleotides. Preferably, a subsequence of the mature polypeptide coding sequence of SEQ ID NO: 25 contains at least 585 nucleotides, more preferably at least 615 nucleotides, and most preferably at least 645 nucleotides. Preferably, a subsequence of the mature polypeptide coding sequence of SEQ ID NO: 27 contains at least 855 nucleotides, more preferably at least 900 nucleotides, and most preferably at least 945 nucleotides. Preferably, a subsequence of the mature polypeptide coding sequence of SEQ ID NO: 29 contains at least 600 nucleotides, more preferably at least 630 nucleotides, and most preferably at least 660 nucleotides. Preferably, a subsequence of the mature polypeptide coding sequence of SEQ ID NO: 31 contains at least 765 nucleotides, more preferably at least 810 nucleotides, and most preferably at least 855 nucleotides. Preferably, a subsequence of the mature polypeptide coding sequence of SEQ ID NO: 33 contains at least 1180 nucleotides, more preferably at least 1140 nucleotides, and most preferably at least 1200 nucleotides. Preferably, a subsequence of the mature polypeptide coding sequence of SEQ ID NO: 35 contains at least 600 nucleotides, more preferably at least 630 nucleotides, and most preferably at least 660 nucleotides. Preferably, a subsequence of the mature polypeptide coding sequence of SEQ ID NO: 37 contains at least 170 amino acid residues, more preferably at least 180 amino acid residues, and most preferably at least 190 nucleotides. Preferably, a subsequence of the mature polypeptide coding sequence of SEQ ID NO: 39 contains at least 570 nucleotides, more preferably at least 600 nucleotides, and most preferably at least 630 nucleotides. Preferably, a subsequence of the mature polypeptide coding sequence of SEQ ID NO: 41 contains at least 795 nucleotides, more preferably at least 840 nucleotides, and most preferably at least 885 nucleotides. Preferably, a subsequence of the mature polypeptide coding sequence of SEQ ID NO: 43 contains at least 540 nucleotides, more preferably at least 570 nucleotides, and most preferably at least 600 nucleotides. Preferably, a subsequence of the mature polypeptide coding sequence of SEQ ID NO: 45 contains at least 960 nucleotides, more preferably at least 1005 nucleotides, and most preferably at least 1050 nucleotides. Preferably, a subsequence of the mature polypeptide coding sequence of SEQ ID NO: 47 contains at least 765 nucleotides, more preferably at least 810 nucleotides, and most preferably at least 855 nucleotides. Preferably, a subsequence of the mature polypeptide coding sequence of SEQ ID NO: 49 contains at least 570 nucleotides, more preferably at least 600 nucleotides, and most preferably at least 630 nucleotides. Preferably, a subsequence of the mature polypeptide coding sequence of SEQ ID NO: 51 contains at least 600 nucleotides, more preferably at least 630 nucleotides, and most preferably at least 660 nucleotides. Preferably, a subsequence of the mature polypeptide coding sequence of SEQ ID NO: 53 contains at least 1140 nucleotides, more preferably at least 1200 nucleotides, and most preferably at least 1260 nucleotides. Preferably, a subsequence of the mature polypeptide coding sequence of SEQ ID NO: 55 contains at least 540 nucleotides, more preferably at least 570 nucleotides, and most preferably at least 600 nucleotides. Preferably, a subsequence of the mature polypeptide coding sequence of SEQ ID NO: 57 contains at least 630 nucleotides, more preferably at least 690 nucleotides, and most preferably at least 720 nucleotides. Preferably, a subsequence of the mature polypeptide coding sequence of SEQ ID NO: 59 contains at least 570 nucleotides, more preferably at least 600 nucleotides, and most preferably at least 630 nucleotides. Preferably, a subsequence of the mature polypeptide coding sequence of SEQ ID NO: 61 contains at least 810 nucleotides, more preferably at least 870 nucleotides, and most preferably at least 930 nucleotides. Preferably, a subsequence of the mature polypeptide coding sequence of SEQ ID NO: 63 contains at least 1020 nucleotides, more preferably at least 1080 nucleotides, and most preferably at least 1140 nucleotides. Preferably, a subsequence of the mature polypeptide coding sequence of SEQ ID NO: 143 contains at least 840 nucleotides, more preferably at least 885 nucleotides, and most preferably at least 930 nucleotides. Preferably, a subsequence of the mature polypeptide coding sequence of SEQ ID NO: 145 contains at least 930 nucleotides, more preferably at least 960 nucleotides, and most preferably at least 1050 nucleotides. Preferably, a subsequence of the mature polypeptide coding sequence of SEQ ID NO: 147 contains at least 960 nucleotides, more preferably at least 1020 nucleotides, and most preferably at least 1080 nucleotides. Preferably, a subsequence of the mature polypeptide coding sequence of SEQ ID NO: 149 contains at least 1050 nucleotides, more preferably at least 1110 nucleotides, and most preferably at least 1170 nucleotides. Preferably, a subsequence of the mature polypeptide coding sequence of SEQ ID NO: 151 contains at least 660 nucleotides, more preferably at least 690 nucleotides, and most preferably at least 720 nucleotides. Preferably, a subsequence of the mature polypeptide coding sequence of SEQ ID NO: 153 contains at least 660 nucleotides, more preferably at least 690 nucleotides, and most preferably at least 720 nucleotides. Preferably, a subsequence of the mature polypeptide coding sequence of SEQ ID NO: 155 contains at least 765 nucleotides, more preferably at least 810 nucleotides, and most preferably at least 855 nucleotides. Preferably, a subsequence of the mature polypeptide coding sequence of SEQ ID NO: 157 contains at least 555 nucleotides, more preferably at least 585 nucleotides, and most preferably at least 615 nucleotides. Preferably, a subsequence of the mature polypeptide coding sequence of SEQ ID NO: 159 contains at least 540 nucleotides, more preferably at least 570 nucleotides, and most preferably at least 600 nucleotides. Preferably, a subsequence of the mature polypeptide coding sequence of SEQ ID NO: 161 contains at least 570 nucleotides, more preferably at least 600 nucleotides, and most preferably at least 630 nucleotides. Preferably, a subsequence of the mature polypeptide coding sequence of SEQ ID NO: 163 contains at least 1155 nucleotides, more preferably at least 1230 nucleotides, and most preferably at least 1305 nucleotides. Preferably, a subsequence of the mature polypeptide coding sequence of SEQ ID NO: 165 contains at least 765 nucleotides, more preferably at least 810 nucleotides, and most preferably at least 855 nucleotides.

In a fourth aspect, the polypeptide having cellulolytic enhancing activity is encoded by a polynucleotide that hybridizes under at least very low stringency conditions, preferably at least low stringency conditions, more preferably at least medium stringency conditions, more preferably at least medium-high stringency conditions, even more preferably at least high stringency conditions, and most preferably at least very high stringency conditions with (i) the mature polypeptide coding sequence of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, or SEQ ID NO: 163, (ii) the genomic DNA sequence of the mature polypeptide coding sequence of SEQ ID NO: 7, SEQ ID NO: 9, SEQ ID NO: 11, SEQ ID NO: 15, SEQ ID NO: 155, SEQ ID NO: 157, or SEQ ID NO: 159, or the cDNA sequence of the mature polypeptide coding sequence of SEQ ID NO: 1, SEQ ID NO: 3, SEQ ID NO: 5, SEQ ID NO: 13, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 143, SEQ ID NO: 145, SEQ ID NO: 147, SEQ ID NO: 149, SEQ ID NO: 151, SEQ ID NO: 153, SEQ ID NO: 161, SEQ ID NO: 163, or SEQ ID NO: 165, (iii) a subsequence of (i) or (ii), or (iv) a full-length complementary strand of (i), (ii), or (iii) (J. Sambrook, E.F. Fritsch, and T. Maniatus, 1989, *supra*). A subsequence of the mature polypeptide coding sequence of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, or SEQ ID NO: 163 contains at least 100 contiguous nucleotides or preferably at least 200 contiguous nucleotides. Moreover, the subsequence may encode a polypeptide fragment that has cellulolytic enhancing activity. In a preferred aspect, the mature polypeptide coding sequence is nucleotides 388 to 1332 of SEQ ID NO: 1, nucleotides 98 to 821 of SEQ ID NO: 3, nucleotides 126 to 978 of SEQ ID NO: 5, nucleotides 55 to 678 of SEQ ID NO: 7, nucleotides 58 to 912 of SEQ ID NO: 9, nucleotides 46 to 951 of SEQ ID NO: 11, nucleotides 67 to 796 of SEQ ID NO: 13, nucleotides 77 to 766 of SEQ ID NO: 15, nucleotides 52 to 921 of SEQ ID NO: 17, nucleotides 46 to 851 of SEQ ID NO: 19, nucleotides 55 to 1239 of SEQ ID NO: 21, nucleotides 46 to 1250 of SEQ ID NO: 23, nucleotides 58 to 811 of SEQ ID NO: 25, nucleotides 64 to 1112 of SEQ ID NO: 27, nucleotides 64 to 859 of SEQ ID NO: 29, nucleotides 64 to 1018 of SEQ ID NO: 31, nucleotides 70 to 1483 of SEQ ID NO: 33, nucleotides 76 to 832 of SEQ ID NO: 35, nucleotides 58 to 974 of SEQ ID NO: 37, nucleotides 52 to 875 of SEQ ID NO: 39, nucleotides 58 to 1250 of SEQ ID NO: 41, nucleotides 52 to 795 of SEQ ID NO: 43, nucleotides 64 to 1104 of SEQ ID NO: 45, nucleotides 73 to 990 of SEQ ID NO: 47, nucleotides 49 to 1218 of SEQ ID NO: 49, nucleotides 55 to 930 of SEQ ID NO: 51, nucleotides 67 to 1581 of SEQ ID NO: 53, nucleotides 49 to 865 of SEQ ID NO: 55, nucleotides 58 to 1065 of SEQ ID NO: 57, nucleotides 67 to 868 of SEQ ID NO: 59, nucleotides 55 to 1099 of SEQ ID NO: 61, nucleotides 70 to 1483 of SEQ ID NO: 63, nucleotides 61 to 1032 of SEQ ID NO: 143, nucleotides 61 to 1167 of SEQ ID NO: 145, nucleotides 64 to 1218 of SEQ ID NO: 147, nucleotides 58 to 1281 of SEQ ID NO: 149, nucleotides 52 to 801 of SEQ ID NO: 151, nucleotides 61 to 819 of SEQ ID NO: 153, nucleotides 61 to 966 of SEQ ID NO: 155, nucleotides 52 to 702 of SEQ ID NO: 157, nucleotides 70 to 699 of SEQ ID NO: 159, nucleotides 49 to 711 of SEQ ID NO: 161, nucleotides 76 to 1452 of SEQ ID NO: 163, or nucleotides 64 to 1018 of SEQ ID NO: 165.

The nucleotide sequence of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, or SEQ ID NO: 163, or a subsequence thereof; as well as the amino acid sequence of SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 144, SEQ ID NO: 146, SEQ ID NO: 148, SEQ ID NO: 150, SEQ ID NO: 152, SEQ ID NO: 154, SEQ ID NO: 156, SEQ ID NO: 158, SEQ ID NO: 160, SEQ ID NO: 162, SEQ ID NO: 164, or SEQ ID NO: 166, or a fragment thereof, may be used to design a nucleic acid probe to identify and clone DNA encoding polypeptides having cellulolytic enhancing activity from strains of different genera or species according to methods well known in the art. In particular, such probes can be used for hybridization with the genomic DNA or cDNA of the genus or species of interest, following standard Southern blotting procedures, in order to identify and isolate the corresponding gene therein. Such probes can be considerably shorter than the entire sequence, but should be at least 14, preferably at least 25, more preferably at least 35, and most preferably at least 70 nucleotides in length. It is, however, preferred that the nucleic acid probe is at least 100 nucleotides in length. For example, the nucleic acid probe may be at least 200 nucleotides, preferably at least 300 nucleotides, more preferably at least 400 nucleotides, or most preferably at least 500 nucleotides in length. Even longer probes may be used, *e.g.,* nucleic acid probes that are preferably at least 600 nucleotides, more preferably at least 700 nucleotides, even more preferably at least 800 nucleotides, or most preferably at least 900 nucleotides in length. Both DNA and RNA probes can be used. The probes are typically labeled for detecting the corresponding gene (for example, with $^{32}P$, $^{3}H$, $^{35}S$, biotin, or avidin). Such probes are encompassed by the present invention.

A genomic DNA or cDNA library prepared from such other strains may, therefore, be screened for DNA that hybridizes with the probes described above and encodes a polypeptide having cellulolytic enhancing activity. Genomic or other DNA from such other strains may be separated by agarose or polyacrylamide gel electrophoresis, or other separation techniques. DNA from the libraries or the separated DNA may be transferred to and immobilized on nitrocellulose or other suitable carrier material. In order to identify a clone or DNA that is homologous with SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, or SEQ ID NO: 163, or a subsequence thereof, the carrier material is preferably used in a Southern blot.

For purposes of the present invention, hybridization indicates that the nucleotide sequence hybridizes to a labeled nucleic acid probe corresponding to the mature polypeptide coding sequence of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, or SEQ ID NO: 163; the genomic DNA sequence of the mature polypeptide coding sequence of SEQ ID NO: 7, SEQ ID NO: 9, SEQ ID NO: 11, SEQ ID NO: 15, SEQ ID NO: 155, SEQ ID NO: 157, or SEQ ID NO: 159, or the cDNA sequence of the mature polypeptide coding sequence of SEQ ID NO: 1, SEQ ID NO: 3, SEQ ID NO: 5, SEQ ID NO: 13, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 143, SEQ ID NO: 145, SEQ ID NO: 147, SEQ ID NO: 149, SEQ ID NO: 151, SEQ ID NO: 153, SEQ ID NO: 161, SEQ ID NO: 163, or SEQ ID NO: 165; the full-length complementary strand thereof; or a subsequence thereof, under very low to very high stringency conditions, as described *supra.*

In a preferred aspect, the nucleic acid probe is the mature polypeptide coding sequence of SEQ ID NO: 1. In another preferred aspect, the nucleic acid probe is nucleotides 388 to 1332 of SEQ ID NO: 1. In another preferred aspect, the nucleic acid probe is a polynucleotide sequence that encodes the polypeptide of SEQ ID NO: 2, or a subsequence thereof. In another preferred aspect, the nucleic acid probe is SEQ ID NO: 1. In another preferred aspect, the nucleic acid probe is the polynucleotide sequence contained in plasmid pEJG120 which is contained in *E. coli* NRRL B-30699, wherein the polynucleotide sequence thereof encodes a polypeptide having cellulolytic enhancing activity. In another preferred aspect, the nucleic acid probe is the mature polypeptide coding sequence contained in plasmid pEJG120 which is contained in *E. coli* NRRL B-30699.

In another preferred aspect, the nucleic acid probe is the mature polypeptide coding sequence of SEQ ID NO: 3. In another preferred aspect, the nucleic acid probe is nucleotides 98 to 821 of SEQ ID NO: 3. In another preferred aspect, the nucleic acid probe is a polynucleotide sequence that encodes the polypeptide of SEQ ID NO: 4, or a subsequence thereof. In another preferred aspect, the nucleic acid probe is SEQ ID NO: 3. In another preferred aspect, the nucleic acid probe is the polynucleotide sequence contained in plasmid pTter61C which is contained in *E. coli* NRRL B-30813, wherein the polynucleotide sequence thereof encodes a polypeptide having cellulolytic enhancing activity. In another preferred aspect, the nucleic acid probe is the mature polypeptide coding sequence contained in plasmid pTter61C which is contained in *E. coli* NRRL B-30813.

In another preferred aspect, the nucleic acid probe is the mature polypeptide coding sequence of SEQ ID NO: 5. In another preferred aspect, the nucleic acid probe is nucleotides 126 to 978 of SEQ ID NO: 5. In another preferred aspect, the nucleic acid probe is a polynucleotide sequence that encodes the polypeptide of SEQ ID NO: 6, or a subsequence thereof. In another preferred aspect, the nucleic acid probe is SEQ ID NO: 5. In another preferred aspect, the nucleic acid probe is the polynucleotide sequence contained in plasmid pTter61D which is contained in *E. coli* NRRL B-30812, wherein the polynucleotide sequence thereof encodes a polypeptide having cellulolytic enhancing activity. In another preferred aspect, the nucleic acid probe is the mature polypeptide coding sequence contained in plasmid pTter61D which is contained in *E. coli* NRRL B-30812.

In another preferred aspect, the nucleic acid probe is the mature polypeptide coding sequence of SEQ ID NO: 7. In another preferred aspect, the nucleic acid probe is nucleotides 55 to 678 of SEQ ID NO: 7. In another preferred aspect, the nucleic acid probe is a polynucleotide sequence that encodes the polypeptide of SEQ ID NO: 8, or a subsequence thereof. In another preferred aspect, the nucleic acid probe is SEQ ID NO: 7. In another preferred aspect, the nucleic acid probe is the polynucleotide sequence contained in plasmid pTter61E which is contained in *E. coli* NRRL B-30814, wherein the polynucleotide sequence thereof encodes a polypeptide having cellulolytic enhancing activity. In another preferred aspect, the nucleic acid probe is the mature polypeptide coding sequence contained in plasmid pTter61E which is contained in *E. coli* NRRL B-30814.

In another preferred aspect, the nucleic acid probe is the mature polypeptide coding sequence of SEQ ID NO: 9. In another preferred aspect, the nucleic acid probe is nucleotides 58 to 912 of SEQ ID NO: 9 In another preferred aspect, the nucleic acid probe is a polynucleotide sequence that encodes the polypeptide of SEQ ID NO: 10, or a subsequence thereof. In another preferred aspect, the nucleic acid probe is SEQ ID NO: 9. In another preferred aspect, the nucleic acid probe is the polynucleotide sequence contained in plasmid pTter61G which is contained in *E. coli* NRRL B-30811, wherein the polynucleotide sequence thereof encodes a polypeptide having cellulolytic enhancing activity. In another preferred aspect, the nucleic acid probe is the mature polypeptide coding sequence contained in plasmid pTter61G which is contained in *E. coli* NRRL B-30811.

In another preferred aspect, the nucleic acid probe is the mature polypeptide coding sequence of SEQ ID NO: 11. In another preferred aspect, the nucleic acid probe is nucleotides 46 to 951 of SEQ ID NO: 11. In another preferred aspect, the nucleic acid probe is a polynucleotide sequence that encodes the polypeptide of SEQ ID NO: 12, or a subsequence thereof. In another preferred aspect, the nucleic acid probe is SEQ ID NO: 11. In another preferred aspect, the nucleic acid probe is the polynucleotide sequence contained in plasmid pTter61F which is contained in *E. coli* NRRL B-50044, wherein the polynucleotide sequence thereof encodes a polypeptide having cellulolytic enhancing activity. In another preferred aspect, the nucleic acid probe is the mature polypeptide coding region contained in plasmid pTter61F which is contained in *E. coli* NRRL B-50044.

In another preferred aspect, the nucleic acid probe is the mature polypeptide coding sequence of SEQ ID NO: 13. In another preferred aspect, the nucleic acid probe is nucleotides 67 to 796 of SEQ ID NO: 13. In another preferred aspect, the nucleic acid probe is a polynucleotide sequence that encodes the polypeptide of SEQ ID NO: 14, or a subsequence thereof. In another preferred aspect, the nucleic acid probe is SEQ ID NO: 13. In another preferred aspect, the nucleic acid probe is the polynucleotide sequence contained in plasmid pDZA2-7 which is contained in *E. coli* NRRL B-30704, wherein the polynucleotide sequence thereof encodes a polypeptide having cellulolytic enhancing activity. In another preferred aspect, the nucleic acid probe is the mature polypeptide coding sequence contained in plasmid pDZA2-7 which is contained in *E. coli* NRRL B-30704.

In another preferred aspect, the nucleic acid probe is the mature polypeptide coding sequence of SEQ ID NO: 15. In another preferred aspect, the nucleic acid probe is nucleotides 77 to 766 of SEQ ID NO: 15. In another preferred aspect, the nucleic acid probe is a polynucleotide sequence that encodes the polypeptide of SEQ ID NO: 16, or a subsequence thereof. In another preferred aspect, the nucleic acid probe is SEQ ID NO: 15. In another preferred aspect, the nucleic acid probe is the polynucleotide sequence contained in plasmid pTr3337 which is contained in *E. coli* NRRL B-30878, wherein the polynucleotide sequence thereof encodes a polypeptide having cellulolytic enhancing activity. In another preferred aspect, the nucleic acid probe is the mature polypeptide coding sequence contained in plasmid pTr3337 which is contained in *E. coli* NRRL B-30878.

In another preferred aspect, the nucleic acid probe is the mature polypeptide coding sequence of SEQ ID NO: 17. In another preferred aspect, the nucleic acid probe is nucleotides 52 to 921 of SEQ ID NO: 17. In another preferred aspect, the nucleic acid probe is a polynucleotide sequence that encodes the polypeptide of SEQ ID NO: 18, or a subsequence thereof. In another preferred aspect, the nucleic acid probe is SEQ ID NO: 17. In another preferred aspect, the nucleic acid probe is the polynucleotide sequence contained in plasmid pSMai190 which is contained in *E. coli* NRRL B-50084, wherein the polynucleotide sequence thereof encodes a polypeptide having cellulolytic enhancing activity. In another preferred aspect, the nucleic acid probe is the mature polypeptide coding sequence contained in plasmid pSMai190 which is contained in *E. coli* NRRL B-50084.

In another preferred aspect, the nucleic acid probe is the mature polypeptide coding sequence of SEQ ID NO: 19. In another preferred aspect, the nucleic acid probe is nucleotides 46 to 851 of SEQ ID NO: 19. In another preferred aspect, the nucleic acid probe is a polynucleotide sequence that encodes the polypeptide of SEQ ID NO: 20, or a subsequence thereof. In another preferred aspect, the nucleic acid probe is SEQ ID NO: 19. In another preferred aspect, the nucleic acid probe is the polynucleotide sequence contained in plasmid pSMai192 which is contained in *E. coli* NRRL B-50086, wherein the polynucleotide sequence thereof encodes a polypeptide having cellulolytic enhancing activity. In another preferred aspect, the nucleic acid probe is the mature polypeptide coding sequence contained in plasmid pSMai192 which is contained in *E. coli* NRRL B-50086.

In another preferred aspect, the nucleic acid probe is the mature polypeptide coding sequence of SEQ ID NO:

21. In another preferred aspect, the nucleic acid probe is nucleotides 55 to 1239 of SEQ ID NO: 21. In another preferred aspect, the nucleic acid probe is a polynucleotide sequence that encodes the polypeptide of SEQ ID NO: 22, or a subsequence thereof. In another preferred aspect, the nucleic acid probe is SEQ ID NO: 21. In another preferred aspect, the nucleic acid probe is the polynucleotide sequence contained in plasmid pSMai191 which is contained in *E. coli* NRRL B-50085, wherein the polynucleotide sequence thereof encodes a polypeptide having cellulolytic enhancing activity. In another preferred aspect, the nucleic acid probe is the mature polypeptide coding sequence contained in plasmid pSMai191 which is contained in *E. coli* NRRL B-50085.

In another preferred aspect, the nucleic acid probe is the mature polypeptide coding sequence of SEQ ID NO: 23. In another preferred aspect, the nucleic acid probe is nucleotides 46 to 1250 of SEQ ID NO: 23. In another preferred aspect, the nucleic acid probe is a polynucleotide sequence that encodes the polypeptide of SEQ ID NO: 24, or a subsequence thereof. In another preferred aspect, the nucleic acid probe is SEQ ID NO: 23. In another preferred aspect, the nucleic acid probe is the polynucleotide sequence contained in plasmid pSMai193 which is contained in *E. coli* NRRL B-50087, wherein the polynucleotide sequence thereof encodes a polypeptide having cellulolytic enhancing activity. In another preferred aspect, the nucleic acid probe is the mature polypeptide coding sequence contained in plasmid pSMai193 which is contained in *E. coli* NRRL B-50087.

In another preferred aspect, the nucleic acid probe is the mature polypeptide coding sequence of SEQ ID NO: 25. In another preferred aspect, the nucleic acid probe is nucleotides 58 to 811 of SEQ ID NO: 25. In another preferred aspect, the nucleic acid probe is a polynucleotide sequence that encodes the polypeptide of SEQ ID NO: 26, or a subsequence thereof. In another preferred aspect, the nucleic acid probe is SEQ ID NO: 25. In another preferred aspect, the nucleic acid probe is the polynucleotide sequence contained in plasmid pSMai187 which is contained in *E. coli* NRRL B-50083, wherein the polynucleotide sequence thereof encodes a polypeptide having cellulolytic enhancing activity. In another preferred aspect, the nucleic acid probe is the mature polypeptide coding sequence contained in plasmid pSMai187 which is contained in *E. coli* NRRL B-50083.

In another preferred aspect, the nucleic acid probe is the mature polypeptide coding sequence of SEQ ID NO: 27. In another preferred aspect, the nucleic acid probe is nucleotides 64 to 1112 of SEQ ID NO: 27. In another preferred aspect, the nucleic acid probe is a polynucleotide sequence that encodes the polypeptide of SEQ ID NO: 28, or a subsequence thereof. In another preferred aspect, the nucleic acid probe is SEQ ID NO: 27. In another preferred aspect, the nucleic acid probe is the polynucleotide sequence contained in plasmid pXYZ1473 which is contained in *E. coli* DSM 22075, wherein the polynucleotide sequence thereof encodes a polypeptide having cellulolytic enhancing activity. In another preferred aspect, the nucleic acid probe is the mature polypeptide coding sequence contained in plasmid pXYZ1473 which is contained in *E. coli* DSM 22075.

In another preferred aspect, the nucleic acid probe is the mature polypeptide coding sequence of SEQ ID NO: 29. In another preferred aspect, the nucleic acid probe is nucleotides 64 to 859 of SEQ ID NO: 29. In another preferred aspect, the nucleic acid probe is a polynucleotide sequence that encodes the polypeptide of SEQ ID NO: 30, or a subsequence thereof. In another preferred aspect, the nucleic acid probe is SEQ ID NO: 29.

In another preferred aspect, the nucleic acid probe is the mature polypeptide coding sequence of SEQ ID NO: 31. In another preferred aspect, the nucleic acid probe is nucleotides 64 to 1018 of SEQ ID NO: 31. In another preferred aspect, the nucleic acid probe is a polynucleotide sequence that encodes the polypeptide of SEQ ID NO: 32, or a subsequence thereof. In another preferred aspect, the nucleic acid probe is SEQ ID NO: 31. In another preferred aspect, the nucleic acid probe is the polynucleotide sequence contained in plasmid pGEM-T-Ppin7 which is contained in *E. coli* DSM 22711, wherein the polynucleotide sequence thereof encodes a polypeptide having cellulolytic enhancing activity. In another preferred aspect, the nucleic acid probe is the mature polypeptide coding sequence contained in plasmid pGEM-T-Ppin7 which is contained in *E. coli* DSM 22711.

In another preferred aspect, the nucleic acid probe is the mature polypeptide coding sequence of SEQ ID NO: 33. In another preferred aspect, the nucleic acid probe is nucleotides 70 to 1483 of SEQ ID NO: 33. In another preferred aspect, the nucleic acid probe is a polynucleotide sequence that encodes the polypeptide of SEQ ID NO: 34, or a subsequence thereof. In another preferred aspect, the nucleic acid probe is SEQ ID NO: 33. In another preferred aspect, the nucleic acid probe is the polynucleotide sequence contained in plasmid pXYZ1483 which is contained in *E. coli* DSM 22600, wherein the polynucleotide sequence thereof encodes a polypeptide having cellulolytic enhancing activity. In another preferred aspect, the nucleic acid probe is the mature polypeptide coding sequence contained in plasmid pXYZ1483 which is contained in *E. coli* DSM 22600.

In another preferred aspect, the nucleic acid probe is the mature polypeptide coding sequence of SEQ ID NO: 35. In another preferred aspect, the nucleic acid probe is nucleotides 76 to 832 of SEQ ID NO: 35. In another preferred aspect, the nucleic acid probe is a polynucleotide sequence that encodes the polypeptide of SEQ ID NO: 36, or a subsequence thereof. In another preferred aspect, the nucleic acid probe is SEQ ID NO: 35. In another preferred aspect, the nucleic acid probe is the polynucleotide sequence contained in plasmid pGEM-T-GH61D23Y4 which is contained in *E. coli* DSM 22882, wherein the polynucleotide sequence thereof encodes a polypeptide having cellulolytic enhancing activity. In another preferred aspect, the nucleic acid probe is the mature polypeptide coding sequence contained in plasmid pGEM-T-GH61D23Y4 which is contained in *E. coli* DSM 22882.

In another preferred aspect, the nucleic acid probe is the mature polypeptide coding sequence of SEQ ID NO: 37. In another preferred aspect, the nucleic acid probe is nucleotides 58 to 974 of SEQ ID NO: 37. In another preferred aspect, the nucleic acid probe is a polynucleotide sequence that encodes the polypeptide of SEQ ID NO: 38, or a subsequence thereof. In another preferred aspect, the nucleic acid probe is SEQ ID NO: 37. In another preferred aspect, the nucleic acid probe is the polynucleotide sequence contained in plasmid pSMai213 which is contained in *E. coli* NRRL B-50300, wherein the polynucleotide sequence thereof encodes a polypeptide having cellulolytic enhancing activity. In another preferred aspect, the nucleic acid probe is the mature polypeptide coding sequence contained in plasmid pSMai213 which is contained in *E. coli* NRRL B-50300.

In another preferred aspect, the nucleic acid probe is the mature polypeptide coding sequence of SEQ ID NO: 39. In another preferred aspect, the nucleic acid probe is nucleotides 52 to 875 of SEQ ID NO: 39. In another preferred aspect, the nucleic acid probe is a polynucleotide sequence that encodes the polypeptide of SEQ ID NO: 40, or a subsequence thereof. In another preferred aspect, the nucleic acid probe is SEQ ID NO: 39. In another preferred aspect, the nucleic acid probe is the polynucleotide sequence contained in plasmid pSMai216 which is contained in *E. coli* NRRL B-50301, wherein the polynucleotide sequence thereof encodes a polypeptide having cellulolytic enhancing activity. In another preferred aspect, the nucleic acid probe is the mature polypeptide coding sequence contained in plasmid pSMai216 which is contained in *E. coli* NRRL B-50301.

In another preferred aspect, the nucleic acid probe is the mature polypeptide coding sequence of SEQ ID NO: 41. In another preferred aspect, the nucleic acid probe is nucleotides 58 to 1250 of SEQ ID NO: 41. In another preferred aspect, the nucleic acid probe is a polynucleotide sequence that encodes the polypeptide of SEQ ID NO: 42, or a subsequence thereof. In another preferred aspect, the nucleic acid probe is SEQ ID NO: 41. In another preferred aspect, the nucleic acid probe is the polynucleotide sequence contained in plasmid p pSMai217 which is contained in *E. coli* NRRL B-50302, wherein the polynucleotide sequence thereof encodes a polypeptide having cellulolytic enhancing activity. In another preferred aspect, the nucleic acid probe is the mature polypeptide coding sequence contained in plasmid pSMai217 which is contained in *E. coli* NRRL B-50302.

In another preferred aspect, the nucleic acid probe is the mature polypeptide coding sequence of SEQ ID NO: 43. In another preferred aspect, the nucleic acid probe is nucleotides 52 to 795 of SEQ ID NO: 43. In another preferred aspect, the nucleic acid probe is a polynucleotide sequence that encodes the polypeptide of SEQ ID NO: 44, or a subsequence thereof. In another preferred aspect, the nucleic acid probe is SEQ ID NO: 43. In another preferred aspect, the nucleic acid probe is the polynucleotide sequence contained in plasmid pSMai218 which is contained in *E. coli* NRRL B-50303, wherein the polynucleotide sequence thereof encodes a polypeptide having cellulolytic enhancing activity. In another preferred aspect, the nucleic acid probe is the mature polypeptide coding sequence contained in plasmid pSMai218 which is contained in *E. coli* NRRL B-50303.

In another preferred aspect, the nucleic acid probe is the mature polypeptide coding sequence of SEQ ID NO: 45. In another preferred aspect, the nucleic acid probe is nucleotides 64 to 1104 of SEQ ID NO: 45. In another preferred aspect, the nucleic acid probe is a polynucleotide sequence that encodes the polypeptide of SEQ ID NO: 46, or a subsequence thereof. In another preferred aspect, the nucleic acid probe is SEQ ID NO: 45. In another preferred aspect, the nucleic acid probe is the polynucleotide sequence contained in plasmid pAG68 which is contained in *E. coli* NRRL B-50320, wherein the polynucleotide sequence thereof encodes a polypeptide having cellulolytic enhancing activity. In another preferred aspect, the nucleic acid probe is the mature polypeptide coding sequence contained in plasmid pAG68 which is contained in *E. coli* NRRL B-50320.

In another preferred aspect, the nucleic acid probe is the mature polypeptide coding sequence of SEQ ID NO: 47. In another preferred aspect, the nucleic acid probe is nucleotides 73 to 990 of SEQ ID NO: 47. In another preferred aspect, the nucleic acid probe is a polynucleotide sequence that encodes the polypeptide of SEQ ID NO: 48, or a subsequence thereof. In another preferred aspect, the nucleic acid probe is SEQ ID NO: 47. In another preferred aspect, the nucleic acid probe is the polynucleotide sequence contained in plasmid pAG69 which is contained in *E. coli* NRRL B-50321, wherein the polynucleotide sequence thereof encodes a polypeptide having cellulolytic enhancing activity. In another preferred aspect, the nucleic acid probe is the mature polypeptide coding sequence contained in plasmid pAG69 which is contained in *E. coli* NRRL B-50321.

In another preferred aspect, the nucleic acid probe is the mature polypeptide coding sequence of SEQ ID NO: 49. In another preferred aspect, the nucleic acid probe is nucleotides 49 to 1218 of SEQ ID NO: 49. In another preferred aspect, the nucleic acid probe is a polynucleotide sequence that encodes the polypeptide of SEQ ID NO: 50, or a subsequence thereof. In another preferred aspect, the nucleic acid probe is SEQ ID NO: 49. In another preferred aspect, the nucleic acid probe is the polynucleotide sequence contained in plasmid pAG75 which is contained in *E. coli* NRRL B-50322, wherein the polynucleotide sequence thereof encodes a polypeptide having cellulolytic enhancing activity. In another preferred aspect, the nucleic acid probe is the mature polypeptide coding sequence contained in plasmid pAG75 which is contained in *E. coli* NRRL B-50322.

In another preferred aspect, the nucleic acid probe is the mature polypeptide coding sequence of SEQ ID NO:

51. In another preferred aspect, the nucleic acid probe is nucleotides 55 to 930 of SEQ ID NO: 51. In another preferred aspect, the nucleic acid probe is a polynucleotide sequence that encodes the polypeptide of SEQ ID NO: 52, or a subsequence thereof. In another preferred aspect, the nucleic acid probe is SEQ ID NO: 51. In another preferred aspect, the nucleic acid probe is the polynucleotide sequence contained in plasmid pAG76 which is contained in *E. coli* NRRL B-50323, wherein the polynucleotide sequence thereof encodes a polypeptide having cellulolytic enhancing activity. In another preferred aspect, the nucleic acid probe is the mature polypeptide coding sequence contained in plasmid pAG76 which is contained in *E. coli* NRRL B-50323.

In another preferred aspect, the nucleic acid probe is the mature polypeptide coding sequence of SEQ ID NO: 53. In another preferred aspect, the nucleic acid probe is nucleotides 67 to 1581 of SEQ ID NO: 53. In another preferred aspect, the nucleic acid probe is a polynucleotide sequence that encodes the polypeptide of SEQ ID NO: 54, or a subsequence thereof. In another preferred aspect, the nucleic acid probe is SEQ ID NO: 53. In another preferred aspect, the nucleic acid probe is the polynucleotide sequence contained in plasmid pAG77 which is contained in *E. coli* NRRL B-50324, wherein the polynucleotide sequence thereof encodes a polypeptide having cellulolytic enhancing activity. In another preferred aspect, the nucleic acid probe is the mature polypeptide coding sequence contained in plasmid pAG77 which is contained in *E. coli* NRRL B-50324.

In another preferred aspect, the nucleic acid probe is the mature polypeptide coding sequence of SEQ ID NO: 55. In another preferred aspect, the nucleic acid probe is nucleotides 49 to 865 of SEQ ID NO: 55. In another preferred aspect, the nucleic acid probe is a polynucleotide sequence that encodes the polypeptide of SEQ ID NO: 56, or a subsequence thereof. In another preferred aspect, the nucleic acid probe is SEQ ID NO: 55. In another preferred aspect, the nucleic acid probe is the polynucleotide sequence contained in plasmid pAG78 which is contained in *E. coli* NRRL B-50325, wherein the polynucleotide sequence thereof encodes a polypeptide having cellulolytic enhancing activity. In another preferred aspect, the nucleic acid probe is the mature polypeptide coding sequence contained in plasmid pAG78 which is contained in *E. coli* NRRL B-50325.

In another preferred aspect, the nucleic acid probe is the mature polypeptide coding sequence of SEQ ID NO: 57. In another preferred aspect, the nucleic acid probe is nucleotides 58 to 1065 of SEQ ID NO: 57. In another preferred aspect, the nucleic acid probe is a polynucleotide sequence that encodes the polypeptide of SEQ ID NO: 58, or a subsequence thereof. In another preferred aspect, the nucleic acid probe is SEQ ID NO: 57. In another preferred aspect, the nucleic acid probe is the polynucleotide sequence contained in plasmid p pAG79 which is contained in *E. coli* NRRL B-50326, wherein the polynucleotide sequence thereof encodes a polypeptide having cellulolytic enhancing activity. In another preferred aspect, the nucleic acid probe is the mature polypeptide coding sequence contained in plasmid pAG79 which is contained in *E. coli* NRRL B-50326.

In another preferred aspect, the nucleic acid probe is the mature polypeptide coding sequence of SEQ ID NO: 59. In another preferred aspect, the nucleic acid probe is nucleotides 67 to 868 of SEQ ID NO: 59. In another preferred aspect, the nucleic acid probe is a polynucleotide sequence that encodes the polypeptide of SEQ ID NO: 60, or a subsequence thereof. In another preferred aspect, the nucleic acid probe is SEQ ID NO: 59. In another preferred aspect, the nucleic acid probe is the polynucleotide sequence contained in plasmid plasmid pGEM-T-GH61a51486 which is contained in *E. coli* DSM 22656, wherein the polynucleotide sequence thereof encodes a polypeptide having cellulolytic enhancing activity. In another preferred aspect, the nucleic acid probe is the mature polypeptide coding sequence contained in plasmid plasmid pGEM-T-GH61a51486 which is contained in *E. coli* DSM 22656.

In another preferred aspect, the nucleic acid probe is the mature polypeptide coding sequence of SEQ ID NO: 61. In another preferred aspect, the nucleic acid probe is nucleotides 55 to 1099 of SEQ ID NO: 61. In another preferred aspect, the nucleic acid probe is a polynucleotide sequence that encodes the polypeptide of SEQ ID NO: 62, or a subsequence thereof. In another preferred aspect, the nucleic acid probe is SEQ ID NO: 61. In another preferred aspect, the nucleic acid probe is the polynucleotide sequence contained in plasmid pGEM-T-GH61DYF which is contained in *E. coli* DSM 22654, wherein the polynucleotide sequence thereof encodes a polypeptide having cellulolytic enhancing activity. In another preferred aspect, the nucleic acid probe is the mature polypeptide coding sequence contained in plasmid pGEM-T-GH61DYF which is contained in *E. coli* DSM 22654.

In another preferred aspect, the nucleic acid probe is the mature polypeptide coding sequence of SEQ ID NO: 63. In another preferred aspect, the nucleic acid probe is nucleotides 70 to 1483 of SEQ ID NO: 63. In another preferred aspect, the nucleic acid probe is a polynucleotide sequence that encodes the polypeptide of SEQ ID NO: 64, or a subsequence thereof. In another preferred aspect, the nucleic acid probe is SEQ ID NO: 63. In another preferred aspect, the nucleic acid probe is the polynucleotide sequence contained in plasmid pGEM-T-GH61D14YH which is contained in *E. coli* DSM 22657, wherein the polynucleotide sequence thereof encodes a polypeptide having cellulolytic enhancing activity. In another preferred aspect, the nucleic acid probe is the mature polypeptide coding sequence contained in plasmid pGEM-T-GH61D14YH which is contained in *E. coli* DSM 22657.

In another preferred aspect, the nucleic acid probe is the mature polypeptide coding sequence of SEQ ID NO: 143. In another preferred aspect, the nucleic acid probe is nucleotides 61 to 1032 of SEQ ID NO: 143. In another preferred aspect, the nucleic acid probe is a polynucleotide sequence that encodes the polypeptide of SEQ ID NO: 143, or a subsequence thereof. In another preferred aspect, the nucleic acid probe is SEQ ID NO: 143.

In another preferred aspect, the nucleic acid probe is the mature polypeptide coding sequence of SEQ ID NO: 145. In another preferred aspect, the nucleic acid probe is nucleotides 61 to 1167 of SEQ ID NO: 145. In another preferred aspect, the nucleic acid probe is a polynucleotide sequence that encodes the polypeptide of SEQ ID NO: 145, or a subsequence thereof. In another preferred aspect, the nucleic acid probe is SEQ ID NO: 145.

In another preferred aspect, the nucleic acid probe is the mature polypeptide coding sequence of SEQ ID NO: 147. In another preferred aspect, the nucleic acid probe is nucleotides 64 to 1218 of SEQ ID NO: 147. In another preferred aspect, the nucleic acid probe is a polynucleotide sequence that encodes the polypeptide of SEQ ID NO: 147, or a subsequence thereof. In another preferred aspect, the nucleic acid probe is SEQ ID NO: 147.

In another preferred aspect, the nucleic acid probe is the mature polypeptide coding sequence of SEQ ID NO: 149. In another preferred aspect, the nucleic acid probe is nucleotides 58 to 1281 of SEQ ID NO: 149. In another preferred aspect, the nucleic acid probe is a polynucleotide sequence that encodes the polypeptide of SEQ ID NO: 149, or a subsequence thereof. In another preferred aspect, the nucleic acid probe is SEQ ID NO: 149.

In another preferred aspect, the nucleic acid probe is the mature polypeptide coding sequence of SEQ ID NO: 151. In another preferred aspect, the nucleic acid probe is nucleotides 52 to 801 of SEQ ID NO: 151. In another preferred aspect, the nucleic acid probe is a polynucleotide sequence that encodes the polypeptide of SEQ ID NO: 151, or a subsequence thereof. In another preferred aspect, the nucleic acid probe is SEQ ID NO: 151.

In another preferred aspect, the nucleic acid probe is the mature polypeptide coding sequence of SEQ ID NO: 153. In another preferred aspect, the nucleic acid probe is nucleotides 61 to 819 of SEQ ID NO: 153. In another preferred aspect, the nucleic acid probe is a polynucleotide sequence that encodes the polypeptide of SEQ ID NO: 153, or a subsequence thereof. In another preferred aspect, the nucleic acid probe is SEQ ID NO: 153.

In another preferred aspect, the nucleic acid probe is the mature polypeptide coding sequence of SEQ ID NO: 155. In another preferred aspect, the nucleic acid probe is nucleotides 61 to 966 of SEQ ID NO: 155. In another preferred aspect, the nucleic acid probe is a polynucleotide sequence that encodes the polypeptide of SEQ ID NO: 155, or a subsequence thereof. In another preferred aspect, the nucleic acid probe is SEQ ID NO: 155.

In another preferred aspect, the nucleic acid probe is the mature polypeptide coding sequence of SEQ ID NO: 157. In another preferred aspect, the nucleic acid probe is nucleotides 52 to 702 of SEQ ID NO: 157. In another preferred aspect, the nucleic acid probe is a polynucleotide sequence that encodes the polypeptide of SEQ ID NO: 157, or a subsequence thereof. In another preferred aspect, the nucleic acid probe is SEQ ID NO: 157.

In another preferred aspect, the nucleic acid probe is the mature polypeptide coding sequence of SEQ ID NO: 159. In another preferred aspect, the nucleic acid probe is nucleotides 70 to 699 of SEQ ID NO: 159. In another preferred aspect, the nucleic acid probe is a polynucleotide sequence that encodes the polypeptide of SEQ ID NO: 159, or a subsequence thereof. In another preferred aspect, the nucleic acid probe is SEQ ID NO: 159.

In another preferred aspect, the nucleic acid probe is the mature polypeptide coding sequence of SEQ ID NO: 161. In another preferred aspect, the nucleic acid probe is nucleotides 49 to 711 of SEQ ID NO: 161. In another preferred aspect, the nucleic acid probe is a polynucleotide sequence that encodes the polypeptide of SEQ ID NO: 161, or a subsequence thereof. In another preferred aspect, the nucleic acid probe is SEQ ID NO: 161.

In another preferred aspect, the nucleic acid probe is the mature polypeptide coding sequence of SEQ ID NO: 163. In another preferred aspect, the nucleic acid probe is nucleotides 76 to 1452 of SEQ ID NO: 163. In another preferred aspect, the nucleic acid probe is a polynucleotide sequence that encodes the polypeptide of SEQ ID NO: 163, or a subsequence thereof. In another preferred aspect, the nucleic acid probe is SEQ ID NO: 163.

In another preferred aspect, the nucleic acid probe is the mature polypeptide coding sequence of SEQ ID NO: 165. In another preferred aspect, the nucleic acid probe is nucleotides 64 to 1018 of SEQ ID NO: 165. In another preferred aspect, the nucleic acid probe is a polynucleotide sequence that encodes the polypeptide of SEQ ID NO: 165, or a subsequence thereof. In another preferred aspect, the nucleic acid probe is SEQ ID NO: 165.

For long probes of at least 100 nucleotides in length, very low to very high stringency conditions are defined as prehybridization and hybridization at 42°C in 5X SSPE, 0.3% SDS, 200 micrograms/ml sheared and denatured salmon sperm DNA, and either 25% formamide for very low and low stringencies, 35% formamide for medium and medium-high stringencies, or 50% formamide for high and very high stringencies, following standard Southern blotting procedures for 12 to 24 hours optimally. The carrier material is finally washed three times each for 15 minutes using 2X SSC, 0.2% SDS at 45°C (very low stringency), at 50°C (low stringency), at 55°C (medium stringency), at 60°C (medium-high stringency), at 65°C (high stringency), and at 70°C (very high stringency).

For short probes of about 15 nucleotides to about 70 nucleotides in length, stringency conditions are defined as prehybridization and hybridization at about 5°C to about 10°C below the calculated $T_m$ using the calculation according to Bolton and McCarthy (1962, Proc. Natl. Acad. Sci. USA 48:1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml following standard Southern blotting procedures for 12 to 24 hours optimally. The carrier material is finally washed once in 6X SCC plus 0.1% SDS for 15 minutes and twice each for 15 minutes using 6X SSC at 5°C to 10°C below the calculated $T_m$.

In a fifth aspect, the polypeptide having cellulolytic enhancing activity is encoded by a polynucleotide comprising or consisting of a nucleotide sequence that has a degree of identity to the mature polypeptide coding sequence of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, or SEQ ID NO: 163 of preferably at least 60%, more preferably at least 65%, more preferably at least 70%, more preferably at least 75%, more preferably at least 80%, more preferably at least 85%, even more preferably at least 90%, most preferably at least 91%, at least 92%, at least 93%, at least 94%, or at least 95%, and even most preferably at least 96%, at least 97%, at least 98%, at least 99%, or at least 100%.

In a sixth aspect, the polypeptide having cellulolytic enhancing activity is an artificial variant comprising a substitution, deletion, and/or insertion of one or more (e.g., several) amino acids of the mature polypeptide of SEQ ID NO: 2, SEQ ID NO: 4, SEQ ID NO: 6, SEQ ID NO: 8, SEQ ID NO: 10, SEQ ID NO: 12, or SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 144, SEQ ID NO: 146, SEQ ID NO: 148, SEQ ID NO: 150, SEQ ID NO: 152, SEQ ID NO: 154, SEQ ID NO: 156, SEQ ID NO: 158, SEQ ID NO: 160, SEQ ID NO: 162, SEQ ID NO: 164, or SEQ ID NO: 166; or a homologous sequence thereof. Preferably, amino acid changes are of a minor nature, that is conservative amino acid substitutions or insertions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a parent polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for cellulolytic enhancing activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identities of essential amino acids can also be inferred from analysis of identities with polypeptides that are related to the parent polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.,* Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

The total number of amino acid substitutions, deletions and/or insertions of the mature polypeptide of SEQ ID NO: 2, SEQ ID NO: 4, SEQ ID NO: 6, SEQ ID NO: 8, SEQ ID NO: 10, SEQ ID NO: 12, or SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 144, SEQ ID NO: 146, SEQ ID NO: 148, SEQ ID NO: 150, SEQ ID NO: 152, SEQ ID NO: 154, SEQ ID NO: 156, SEQ ID NO: 158, SEQ ID NO: 160, SEQ ID NO: 162, SEQ ID NO: 164, or SEQ ID NO: 166, is not more than 10, *e.g.,* 1, 2, 3, 4, 5, 6, 7, 8 or 9.

A polypeptide having cellulolytic enhancing activity may be obtained from microorganisms of any genus. For purposes of the present invention, the term "obtained from" as used herein in connection with a given source shall mean that the polypeptide encoded by a polynucleotide is produced by the source or by a strain in which the polynucleotide from the source has been inserted. In one aspect, the polypeptide obtained from a given source is secreted extracellularly.

A polypeptide having cellulolytic enhancing activity may be a bacterial polypeptide. For example, the polypeptide may be a gram positive bacterial polypeptide such as a *Bacillus, Streptococcus, Streptomyces, Staphylococcus, Enterococcus, Lactobacillus, Lactococcus, Clostridium, Geobacillus,* or *Oceanobacillus* polypeptide having cellulolytic enhancing activity, or a Gram negative bacterial polypeptide such as an *E. coli, Pseudomonas, Salmonella, Campylobacter, Helicobacter, Flavobacterium, Fusobacterium, Ilyobacter, Neisseria,* or *Ureaplasma* polypeptide having cellulolytic enhancing activity.

In one aspect, the polypeptide is a *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* or *Bacillus thuringiensis* polypeptide having cellulolytic enhancing activity.

In another aspect, the polypeptide is a *Streptococcus equisimilis, Streptococcus pyogenes, Streptococcus uberis,* or *Streptococcus equi* subsp. *Zooepidemicus* polypeptide having cellulolytic enhancing activity.

In another aspect, the polypeptide is a *Streptomyces achromogenes, Streptomyces avermitilis, Streptomyces coelicolor, Streptomyces griseus,* or *Streptomyces lividans* polypeptide having cellulolytic enhancing activity.

The polypeptide having cellulolytic enhancing activity may also be a fungal polypeptide, and more preferably a yeast polypeptide such as a *Candida, Kluyveromyces, Pichia, Saccharomyces, Schizosaccharomyces,* or *Yarrowia* polypeptide having cellulolytic enhancing activity; or more preferably a filamentous fungal polypeptide such as aan *Acremonium, Agaricus, Alternaria, Aspergillus, Aureobasidium, Botryospaeria, Ceriporiopsis, Chaetomidium, Chrysosporium, Claviceps, Cochliobolus, Coprinopsis, Coptotermes, Corynascus, Cryphonectria, Cryptococcus, Diplodia, Exidia, Filibasidium, Fusarium, Gibberella, Holomastigotoides, Humicola, Irpex, Lentinula, Leptospaeria, Magnaporthe, Melanocarpus, Meripilus, Mucor, Myceliophthora, Neocallimastix, Neurospora, Paecilomyces, Penicillium, Phanerochaete, Piromyces, Poitrasia, Pseudoplectania, Pseudotrichonympha, Rhizomucor, Schizophyllum, Scytalidium, Talaromyces, Thermoascus, Thielavia, Tolypocladium, Trichoderma, Trichophaea, Verticillium, Volvariella,* or *Xylaria* polypeptide having cellulolytic enhancing activity.

In another aspect, the polypeptide is a *Saccharomyces carlsbergensis, Saccharomyces cerevisiae, Saccharomyces diastaticus, Saccharomyces douglasii, Saccharomyces kluyveri, Saccharomyces norbensis,* or *Saccharomyces oviformis* polypeptide having cellulolytic enhancing activity.

In another aspect, the polypeptide is an *Acremonium cellulolyticus, Aspergillus aculeatus, Aspergillus awamori, Aspergillus fumigatus, Aspergillus foetidus, Aspergillus japonicus, Aspergillus nidulans, Aspergillus niger, Aspergillus oryzae, Chrysosporium keratinophilum, Chrysosporium lucknowense, Chrysosporium tropicum, Chrysosporium merdarium, Chrysosporium inops, Chrysosporium pannicola, Chrysosporium queenslandicum, Chrysosporium zonatum, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola grisea, Humicola insolens, Humicola lanuginosa, Irpex lacteus, Mucor miehei, Myceliophthora thermophila, Neurospora crassa, Penicillium funiculosum, Penicillium pinophilum, Penicillium purpurogenum, Phanerochaete chrysosporium, Thielavia achromatica, Thielavia albomyces, Thielavia albopilosa, Thielavia australeinsis, Thielavia fimeti, Thielavia microspora, Thielavia ovispora, Thielavia peruviana, Thielavia spededonium, Thielavia setosa, Thielavia subthermophila, Thielavia terrestris, Trichoderma harzianum, Trichoderma koningii, Trichoderma longibrachiatum, Trichoderma reesei, Trichoderma viride,* or *Trichophaea saccata* polypeptide having cellulolytic enhancing activity.

It will be understood that for the aforementioned species the invention encompasses both the perfect and imperfect states, and other taxonomic equivalents, *e.g.,* anamorphs, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL).

Furthermore, polypeptides having cellulolytic enhancing activity may be identified and obtained from other sources including microorganisms isolated from nature (*e.g.,* soil, composts, water, etc.) using the above-mentioned probes. Techniques for isolating microorganisms from natural habitats are well known in the art. The polynucleotide may then be obtained by similarly screening a genomic DNA or cDNA library of such a microorganism. Once a polynucleotide encoding a polypeptide has been detected with the probe(s), the polynucleotide can be isolated or cloned by utilizing techniques that are well known to those of ordinary skill in the art (see, *e.g.,* Sambrook *et al.,* 1989, *supra)*

Polynucleotides comprising nucleotide sequences that encode polypeptide having cellulolytic enhancing activity can be isolated and utilized to express the polypeptide having cellulolytic enhancing activity for evaluation in the methods of the present invention.

The techniques used to isolate or clone a polynucleotide encoding a polypeptide are known in the art and include isolation from genomic DNA, preparation from cDNA, or a combination thereof. The cloning of the polynucleotides from such genomic DNA can be effected, *e.g.,* by using the well known polymerase chain reaction (PCR) or antibody screening of expression libraries to detect cloned DNA fragments with shared structural features. See, *e.g.,* Innis et al., 1990, PCR: A Guide to Methods and Application, Academic Press, New York. Other nucleic acid amplification procedures such as ligase chain reaction (LCR), ligation activated transcription (LAT) and polynucleotide-based amplification (NASBA) may be used. The polynucleotides may be cloned from a strain or a related organism and thus, for example, may be an allelic or species variant of the polypeptide encoding region of the polynucleotide.

The polynucleotides comprise nucleotide sequences that have a degree of identity to the mature polypeptide coding sequence of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, or SEQ ID NO: 163 of preferably at least 60%, more preferably at least 65%, more preferably at least 70%, more preferably at least 75%, more preferably at least 80%, more preferably at least 85%, even more preferably at least 90%, most preferably at least 91%, at least 92%, at least 93%, at least 94%, or at least 95%, and even most preferably at least 96%, at least 97%, at least 98%, or at least 99%, which encode a polypeptide having cellulolytic enhancing activity.

The polynucleotide may also be a polynucleotide encoding a polypeptide having cellulolytic enhancing activity that hybridizes under at least very low stringency conditions, preferably at least low stringency conditions, more preferably at least medium stringency conditions, more preferably at least medium-high stringency conditions, even more preferably at least high stringency conditions, and most preferably at least very high stringency conditions with (i) the mature polypeptide coding sequence of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, or SEQ ID NO: 163, (ii) the genomic DNA sequence of the mature polypeptide coding sequence of SEQ ID NO: 7, SEQ ID NO: 9, SEQ ID NO: 11, SEQ ID NO: 15, SEQ ID NO: 155, SEQ ID NO: 157, or SEQ ID NO: 159 or the cDNA sequence of the mature polypeptide coding sequence of SEQ ID NO: 1, SEQ ID NO: 3, SEQ ID NO: 5, SEQ ID NO: 13, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 143, SEQ ID NO: 145, SEQ ID NO: 147, SEQ ID NO: 149, SEQ ID NO: 151, SEQ ID NO: 153, SEQ ID NO: 161, SEQ ID NO: 163, or SEQ ID NO: 165, or (iii) a full-length complementary strand of (i) or (ii); or allelic variants and subsequences thereof (Sambrook *et al.,* 1989, *supra),* as defined herein.

As described earlier, the techniques used to isolate or clone a polynucleotide encoding a polypeptide are known in the art and include isolation from genomic DNA, preparation from cDNA, or a combination thereof.

## Enzyme Compositions

The enzyme compositions used in accordance with the methods of the invention comprise a cellulase.

In one aspect, the enzyme composition comprises or further comprises one or more (*e.g.,* several) proteins selected from the group consisting of a cellulase, a hemicellulase, an esterase, an expansin, a laccase, a ligninolytic enzyme, a pectinase, a peroxidase, a protease, and a swollenin. In another aspect, the cellulase is preferably one or more (*e.g.,* several) enzymes selected from the group consisting of an endoglucanase, a cellobiohydrolase, and a beta-glucosidase. In another aspect, the hemicellulase is preferably one or more (*e.g.,* several) enzymes selected from the group consisting of an acetylmannan esterase, an acetylxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase.

In another aspect, the enzyme composition comprises one or more (e.g., several) cellulolytic enzymes. In another aspect, the enzyme composition comprises or further comprises one or more (*e.g.,* several) hemicellulolytic enzymes. In another aspect, the enzyme composition comprises one or more (*e.g.,* several) cellulolytic enzymes and one or more (*e.g.,* several) hemicellulolytic enzymes. In another aspect, the enzyme composition comprises one or more (*e.g.,* several) enzymes selected from the group of cellulolytic enzymes and hemicellulolytic enzymes. In another aspect, the enzyme composition comprises an endoglucanase. In another aspect, the enzyme composition comprises a cellobiohydrolase. In another aspect, the enzyme composition comprises a beta-glucosidase. In another aspect, the enzyme composition comprises an endoglucanase and a cellobiohydrolase. In another aspect, the enzyme composition comprises an endoglucanase and a beta-glucosidase. In another aspect, the enzyme composition comprises a cellobiohydrolase and a beta-glucosidase. In another aspect, the enzyme composition comprises an endoglucanase, a cellobiohydrolase, and a beta-glucosidase.

In another aspect, the enzyme composition comprises an acetylmannan esterase. In another aspect, the enzyme composition comprises an acetylxylan esterase. In another aspect, the enzyme composition comprises an arabinanase (*e.g.,* alpha-L-arabinanase). In another aspect, the enzyme composition comprises an arabinofuranosidase (*e.g.,* alpha-L-arabinofuranosidase). In another aspect, the enzyme composition comprises a coumaric acid esterase. In another aspect, the enzyme composition comprises a feruloyl esterase. In another aspect, the enzyme composition comprises a galactosidase (e.g., alpha-galactosidase and/or beta-galactosidase). In another aspect, the enzyme composition comprises a glucuronidase (*e.g.,* alpha-D-glucuronidase). In another aspect, the enzyme composition comprises a glucuronoyl esterase. In another aspect, the enzyme composition comprises a mannanase. In another aspect, the enzyme composition comprises a mannosidase (*e.g.,* beta-mannosidase). In another aspect, the enzyme composition comprises a xylanase. In a preferred aspect, the xylanase is a Family 10 xylanase. In another aspect, the enzyme composition comprises a xylosidase (*e.g.,* beta-xylosidase).

In another aspect, the enzyme composition comprises an esterase. In another aspect, the enzyme composition comprises an expansin. In another aspect, the enzyme composition comprises a laccase. In another aspect, the enzyme composition comprises a ligninolytic enzyme. In a preferred aspect, the ligninolytic enzyme is a manganese peroxidase. In another preferred aspect, the ligninolytic enzyme is a lignin peroxidase. In another preferred aspect, the ligninolytic enzyme is a $H_2O_2$-producing enzyme. In another aspect, the enzyme composition comprises a pectinase. In another aspect, the enzyme composition comprises a peroxidase. In another aspect, the enzyme composition comprises a protease. In another aspect, the enzyme composition comprises a swollenin.

In the methods of the present invention, the enzyme(s) can be added prior to or during fermentation, *e.g.,* during saccharification or during or after propagation of the fermenting microorganism(s).

One or more (*e.g.,* several) components of the enzyme composition may be wild-type proteins, recombinant proteins, or a combination of wild-type proteins and recombinant proteins. For example, one or more (*e.g.,* several) components may be native proteins of a cell, which is used as a host cell to express recombinantly one or more (*e.g.,* several) other components of the enzyme composition. One or more (*e.g.,* several) components of the enzyme composition may be produced as monocomponents, which are then combined to form the enzyme composition. The enzyme composition may be a combination of multicomponent and monocomponent protein preparations.

The enzymes used in the methods of the present invention may be in any form suitable for use, such as, for example, a crude fermentation broth with or without cells removed, a cell lysate with or without cellular debris, a semi-purified or purified enzyme preparation, or a host cell as a source of the enzymes. The enzyme composition may be a dry powder or granulate, a non-dusting granulate, a liquid, a stabilized liquid, or a stabilized protected enzyme. Liquid enzyme preparations may, for instance, be stabilized by adding stabilizers such as a sugar, a sugar alcohol or another polyol, and/or lactic acid or another organic acid according to established processes.

The enzymes can be derived or obtained from any suitable origin, including, bacterial, fungal, yeast, plant, or mammalian origin. The term "obtained" means herein that the enzyme may have been isolated from an organism that naturally produces the enzyme as a native enzyme. The term "obtained" also means herein that the enzyme may have been produced recombinantly in a host organism employing methods described herein, wherein the recombinantly produced enzyme is either native or foreign to the host organism or has a modified amino acid sequence, *e.g.,* having one or more (*e.g.,* several) amino acids that are deleted, inserted and/or substituted, *i.e.,* a recombinantly produced enzyme that is a mutant and/or a fragment of a native amino acid sequence or an enzyme produced by nucleic acid shuffling processes known in the art. Encompassed within the meaning of a native enzyme are natural variants and within the meaning of a foreign enzyme are variants obtained recombinantly, such as by site-directed mutagenesis or shuffling.

The polypeptide having enzyme activity may be a bacterial polypeptide. For example, the polypeptide may be a gram positive bacterial polypeptide such as a *Bacillus, Streptococcus, Streptomyces, Staphylococcus, Enterococcus, Lactobacillus, Lactococcus, Clostridium, Geobacillus,* or *Oceanobacillus* polypeptide having enzyme activity, or a Gram negative bacterial polypeptide such as an *E. coli, Pseudomonas, Salmonella, Campylobacter, Helicobacter, Flavobacterium, Fusobacterium, Ilyobacter, Neisseria,* or *Ureaplasma* polypeptide having enzyme activity.

In a preferred aspect, the polypeptide is a *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* or *Bacillus thuringiensis* polypeptide having enzyme activity.

In another preferred aspect, the polypeptide is a *Streptococcus equisimilis, Streptococcus pyogenes, Streptococcus uberis,* or *Streptococcus equi* subsp. *Zooepidemicus* polypeptide having enzyme activity.

In another preferred aspect, the polypeptide is a *Streptomyces achromogenes, Streptomyces avermitilis, Streptomyces coelicolor, Streptomyces griseus,* or *Streptomyces lividans* polypeptide having enzyme activity.

The polypeptide having enzyme activity may also be a fungal polypeptide, and more preferably a yeast polypeptide such as a *Candida, Kluyveromyces, Pichia, Saccharomyces, Schizosaccharomyces,* or *Yarrowia* polypeptide having enzyme activity; or more preferably a filamentous fungal polypeptide such as an *Acremonium, Agaricus, Alternaria, Aspergillus, Aureobasidium, Botryospaeria, Ceriporiopsis, Chaetomidium, Chrysosporium, Claviceps, Cochliobolus, Coprinopsis, Coptotermes, Corynascus, Cryphonectria, Cryptococcus, Diplodia, Exidia, Filibasidium, Fusarium, Gibberella, Holomastigotoides, Humicola, Irpex, Lentinula, Leptospaeria, Magnaporthe, Melanocarpus, Meripilus, Mucor, Myceliophthora, Neocallimastix, Neurospora, Paecilomyces, Penicillium, Phanerochaete, Piromyces, Poitrasia, Pseudoplectania, Pseudotrichonympha, Rhizomucor, Schizophyllum, Scytalidium, Talaromyces, Thermoascus, Thielavia, Tolypocladium, Trichoderma, Trichophaea, Verticillium, Volvariella,* or *Xylaria* polypeptide having enzyme activity.

In a preferred aspect, the polypeptide is a *Saccharomyces carlsbergensis, Saccharomyces cerevisiae, Saccharomyces diastaticus, Saccharomyces douglasii, Saccharomyces kluyveri, Saccharomyces norbensis,* or *Saccharomyces oviformis* polypeptide having enzyme activity.

In another preferred aspect, the polypeptide is an *Acremonium cellulolyticus, Aspergillus aculeatus, Aspergillus awamori, Aspergillus fumigatus, Aspergillus foetidus, Aspergillus japonicus, Aspergillus nidulans, Aspergillus niger, Aspergillus oryzae, Chrysosporium keratinophilum, Chrysosporium lucknowense, Chrysosporium tropicum, Chrysosporium merdarium, Chrysosporium inops, Chrysosporium pannicola, Chrysosporium queenslandicum, Chrysosporium zonatum, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola grisea, Humicola insolens, Humicola lanuginosa, Irpex lacteus, Mucor miehei, Myceliophthora thermophila, Neurospora crassa, Penicillium funiculosum, Penicillium purpurogenum, Phanerochaete chrysosporium, Thielavia achromatica, Thielavia albomyces, Thielavia albopilosa, Thielavia australeinsis, Thielavia fimeti, Thielavia microspora, Thielavia ovispora, Thielavia peruviana, Thielavia spededonium, Thielavia setosa, Thielavia subthermophila, Thielavia terrestris, Trichoderma harzianum, Trichoderma koningii, Trichoderma longibrachiatum, Trichoderma reesei, Trichoderma viride,* or *Trichophaea* saccata polypeptide having enzyme activity.

Chemically modified or protein engineered mutants of the polypeptides having enzyme activity may also be used.

One or more (*e.g.,* several) components of the enzyme composition may be a recombinant component, *i.e.,* produced by cloning of a DNA sequence encoding the single component and subsequent cell transformed with the DNA sequence and expressed in a host (see, for example, WO 91/17243 and WO 91/17244). The host is preferably a heterologous host (enzyme is foreign to host), but the host may under certain conditions also be a homologous host (enzyme is native to host). Monocomponent cellulolytic enzymes may also be prepared by purifying such a protein from a fermentation broth.

In one aspect, the one or more (*e.g.,* several) cellulolytic enzymes comprise a commercial cellulolytic enzyme preparation. Examples of commercial cellulolytic enzyme preparations suitable for use in the present invention include, for example, CELLIC™ CTec (Novozymes A/S), CELLIC™ CTec2 (Novozymes A/S), CELLUCLAST™ (Novozymes A/S), NOVOZYM™ 188 (Novozymes A/S), CELLUZYME™ (Novozymes A/S), CEREFLO™ (Novozymes A/S), and ULTRAFLO™ (Novozymes A/S), ACCELERASE™ (Genencor Int.), LAMINEX™ (Genencor Int.), SPEZYME™ CP (Genencor Int.), FILTRASE® NL (DSM); METHAPLUS® S/L 100 (DSM), ROHAMENT™ 7069 W (Rohm GmbH), FIBREZYME® LDI (Dyadic International, Inc.), FIBREZYME® LBR (Dyadic International, Inc.), or VISCOSTAR® 150L (Dyadic International, Inc.). The cellulase enzymes are added in amounts effective from about 0.001 to about 5.0 wt % of solids, more preferably from about 0.025 to about 4.0 wt % of solids, and most preferably from about 0.005 to about 2.0 wt % of solids. The cellulase enzymes are added in amounts effective from about 0.001 to about 5.0 wt % of solids, more preferably from about 0.025 to about 4.0 wt % of solids, and most preferably from about 0.005 to about 2.0 wt % of solids.

Examples of bacterial endoglucanases that can be used in the methods of the present invention, include, but are not limited to, an *Acidothermus cellulolyticus* endoglucanase (WO 91/05039; WO 93/15186; U.S. Patent No. 5,275,944; WO 96/02551; U.S. Patent No. 5,536,655, WO 00/70031, WO 05/093050); *Thermobifida fusca* endoglucanase III (WO 05/093050); and *Thermobifida fusca* endoglucanase V (WO 05/093050).

Examples of fungal endoglucanases that can be used in the present invention include, but are not limited to, a *Trichoderma reesei* endoglucanase I (Penttila et al., 1986, Gene 45: 253-263; *Trichoderma reesei* Cel7B endoglucanase I; GENBANK™ accession no. M15665; SEQ ID NO: 66); *Trichoderma reesei* endoglucanase II (Saloheimo, et al., 1988, Gene 63:11-22; *Trichoderma reesei* Cel5A endoglucanase II; GENBANK™ accession no. M19373; SEQ ID NO: 68); *Trichoderma reesei* endoglucanase III (Okada et al., 1988, Appl. Environ. Microbiol. 64: 555-563; GENBANK™ accession no. AB003694; SEQ ID NO: 70); *Trichoderma reesei* endoglucanase V (Saloheimo et al., 1994, Molecular Microbiology 13: 219-228; GENBANK™ accession no. Z33381; SEQ ID NO: 72); *Aspergillus aculeatus* endoglucanase (Ooi et al., 1990, Nucleic Acids Research 18: 5884); *Aspergillus kawachii* endoglucanase (Sakamoto et al., 1995, Current Genetics 27: 435-439); *Erwinia carotovara* endoglucanase (Saarilahti et al., 1990, Gene 90: 9-14); *Fusarium oxysporum* endoglucanase (GENBANK™ accession no. L29381); *Humicola grisea* var. *thermoidea* endoglucanase (GENBANK™ accession no. AB003107); *Melanocarpus albomyces* endoglucanase (GENBANK™accession no. MAL515703); *Neurospora crassa* endoglucanase (GENBANK™accession no. XM_324477); *Humicola insolens* endoglucanase V (SEQ ID NO: 74); *Myceliophthora thermophila* CBS 117.65 endoglucanase (SEQ ID NO: 76); basidiomycete CBS 495.95 endoglucanase (SEQ ID NO: 78); basidiomycete CBS 494.95 endoglucanase (SEQ ID NO: 80); *Thielavia terrestris* NRRL 8126 CEL6B endoglucanase (SEQ ID NO: 82); *Thielavia terrestris* NRRL 8126 CEL6C endoglucanase (SEQ ID NO: 84); *Thielavia terrestris* NRRL 8126 CEL7C endoglucanase (SEQ ID NO: 86); *Thielavia terrestris* NRRL 8126 CEL7E endoglucanase (SEQ ID NO: 88); *Thielavia terrestris* NRRL 8126 CEL7F endoglucanase (SEQ ID NO: 90); *Cladorrhinum foecundissimum* ATCC 62373 CEL7A endoglucanase (SEQ ID NO: 92); and *Trichoderma reesei* strain No. VTT-D-80133 endoglucanase (SEQ ID NO: 94; GENBANK™ accession no. M15665). The endoglucanases of SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 90, SEQ ID NO: 92, and SEQ ID NO: 94 described above are encoded by the mature polypeptide coding sequence of SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, and SEQ ID NO: 93, respectively.

Examples of cellobiohydrolases useful in the present invention include, but are not limited to, *Trichoderma reesei* cellobiohydrolase I (SEQ ID NO: 96); *Trichoderma reesei* cellobiohydrolase II (SEQ ID NO: 98); *Humicola insolens* cellobiohydrolase I (SEQ ID NO: 100); *Myceliophthora thermophila* cellobiohydrolase II (SEQ ID NO: 102 and SEQ ID NO: 104); *Thielavia terrestris* cellobiohydrolase II (CEL6A) (SEQ ID NO: 106); *Chaetomium thermophilum* cellobiohydrolase I (SEQ ID NO: 108); and *Chaetomium thermophilum* cellobiohydrolase II (SEQ ID NO: 110). The cellobiohydrolases of SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, and SEQ ID NO: 112 described above are encoded by the mature polypeptide coding sequence of SEQ ID NO: 97, SEQ ID NO: 99, SEQ ID NO: 101, SEQ ID NO: 103, SEQ ID NO: 105, SEQ ID NO: 107, and SEQ ID NO: 109, respectively.

Examples of beta-glucosidases useful in the present invention include, but are not limited to, *Aspergillus oryzae* beta-glucosidase (SEQ ID NO: 112); *Aspergillus fumigatus* beta-glucosidase (SEQ ID NO: 114); *Penicillium brasilianum* IBT 20888 beta-glucosidase (SEQ ID NO: 116); *Aspergillus niger* beta-glucosidase (SEQ ID NO: 118); and *Aspergillus aculeatus* beta-glucosidase (SEQ ID NO: 120). The beta-glucosidases of SEQ ID NO: 112, SEQ ID NO: 114, SEQ ID NO: 116, SEQ ID NO: 118, and SEQ ID NO: 120 described above are encoded by the mature polypeptide coding sequence of SEQ ID NO: 111, SEQ ID NO: 113, SEQ ID NO: 115, SEQ ID NO: 117, and SEQ ID NO: 119, respectively.

Examples of other beta-glucosidases useful in the present invention include a *Aspergillus oryzae* beta-glucosidase variant fusion protein of SEQ ID NO: 122 or the *Aspergillus oryzae* beta-glucosidase fusion protein of SEQ ID NO: 124. The beta-glucosidase fusion proteins of SEQ ID NO: 122 and SEQ ID NO: 124 are encoded by SEQ ID NO: 121 and SEQ ID NO: 123, respectively.

The *Aspergillus oryzae* polypeptide having beta-glucosidase activity can be obtained according to WO 2002/095014. The *Aspergillus fumigatus* polypeptide having beta-glucosidase activity can be obtained according to WO 2005/047499. The *Penicillium brasilianum* polypeptide having beta-glucosidase activity can be obtained according to WO 2007/019442. The *Aspergillus niger* polypeptide having beta-glucosidase activity can be obtained according to Dan et a*l*., 2000, J. Biol. Chem. 275: 4973-4980. The *Aspergillus aculeatus* polypeptide having beta-glucosidase activity can be obtained according to Kawaguchi et al., 1996, Gene 173: 287-288.

Other useful endoglucanases, cellobiohydrolases, and beta-glucosidases are disclosed in numerous Glycosyl Hydrolase families using the classification according to Henrissat B., 1991, A classification of glycosyl hydrolases based on amino-acid sequence similarities, Biochem. J. 280: 309-316, and Henrissat B., and Bairoch A., 1996, Updating the sequence-based classification of glycosyl hydrolases, Biochem. J. 316: 695-696.

Other cellulolytic enzymes that may be useful in the present invention are described in EP 495,257, EP 531,315, EP 531,372, WO 89/09259, WO 94/07998, WO 95/24471, WO 96/11262, WO 96/29397, WO 96/034108, WO 97/14804, WO 98/08940, WO 98/012307, WO 98/13465, WO 98/015619, WO 98/015633, WO 98/028411, WO 99/06574, WO 99/10481, WO 99/025846, WO 99/025847, WO 99/031255, WO 2000/009707, WO 2002/050245, WO 2002/0076792, WO 2002/101078, WO 2003/027306, WO 2003/052054, WO 2003/052055, WO 2003/052056, WO 2003/052057, WO 2003/052118, WO 2004/016760, WO 2004/043980, WO 2004/048592, WO 2005/001065, WO 2005/028636, WO 2005/093050, WO 2005/093073, WO 2006/074005, WO 2006/117432, WO 2007/071818, WO 2007/071820, WO 2008/008070, WO 2008/008793, U.S. Patent No. 4,435,307, U.S. Patent No. 5,457,046, U.S. Patent No. 5,648,263, U.S. Patent No. 5,686,593, U.S. Patent No. 5,691,178, U.S. Patent No. 5,763,254, and U.S. Patent No. 5,776,757.

In one aspect, the one or more (*e.g.,* several) hemicellulolytic enzymes comprise a commercial hemicellulolytic enzyme preparation. Examples of commercial hemicellulolytic enzyme preparations suitable for use in the present invention include, for example, SHEARZYME™ (Novozymes A/S), CELLIC™ HTec (Novozymes A/S), CELLIC™ HTec2 (Novozymes A/S), VISCOZYME® (Novozymes A/S), ULTRAFLO® (Novozymes A/S), PULPZYME® HC (Novozymes A/S), MULTIFECT® Xylanase (Genencor), ACCELLERASE® XY (Genencor), ACCELLERASE® XC (Genencor), ECOPULP® TX-200A (AB Enzymes), HSP 6000 Xylanase (DSM), DEPOL™ 333P (Biocatalysts Limit, Wales, UK), DEPOL™ 740L. (Biocatalysts Limit, Wales, UK), and DEPOL™ 762P (Biocatalysts Limit, Wales, UK).

Examples of xylanases useful in the methods of the present invention include, but are not limited to, *Aspergillus aculeatus* xylanase (GeneSeqP:AAR63790; WO 94/21785), *Aspergillus fumigatus* xylanases (WO 2006/078256), and *Thielavia terrestris* NRRL 8126 xylanases (WO 2009/079210).

Examples of beta-xylosidases useful in the methods of the present invention include, but are not limited to, *Trichoderma reesei* beta-xylosidase (UniProtKB/TrEMBL accession number Q92458), *Talaromyces emersonii* (Swiss-Prot accession number Q8X212), and *Neurospora crassa* (SwissProt accession number Q7SOW4).

Examples of acetylxylan esterases useful in the methods of the present invention include, but are not limited to, *Hypocrea jecorina* acetylxylan esterase (WO 2005/001036), *Neurospora crassa* acetylxylan esterase (UniProt accession number q7s259), *Thielavia terrestris* NRRL 8126 acetylxylan esterase (WO 2009/042846), *Chaetomium globosum* acetylxylan esterase (Uniprot accession number Q2GWX4), *Chaetomium gracile* acetylxylan esterase (GeneSeqP accession number AAB82124), *Phaeosphaeria nodorum* acetylxylan esterase (Uniprot accession number Q0UHJ1), and *Humicola insolens* DSM 1800 acetylxylan esterase (WO 2009/073709).

Examples of ferulic acid esterases useful in the methods of the present invention include, but are not limited to, *Humicola insolens* DSM 1800 feruloyl esterase (WO 2009/076122), *Neurospora crassa* feruloyl esterase (UniProt accession number Q9HGR3), and *Neosartorya fischeri* feruloyl esterase (UniProt Accession number A1 D9T4).

Examples of arabinofuranosidases useful in the methods of the present invention include, but are not limited to, *Humicola insolens* DSM 1800 arabinofuranosidase (WO 2009/073383) and *Aspergillus niger* arabinofuranosidase (GeneSeqP accession number AAR94170).

Examples of alpha-glucuronidases useful in the methods of the present invention include, but are not limited to, *Aspergillus clavatus* alpha-glucuronidase (UniProt accession number alcc12), *Trichoderma reesei* alpha-glucuronidase (Uniprot accession number Q99024), *Talaromyces emersonii* alpha-glucuronidase (UniProt accession number Q8X211), *Aspergillus niger* alpha-glucuronidase (Uniprot accession number Q96WX9), *Aspergillus terreus* alpha-glucuronidase (SwissProt accession number Q0CJP9), and *Aspergillus fumigatus* alpha-glucuronidase (SwissProt accession number Q4WW45).

The enzymes and proteins used in the methods of the present invention may be produced by fermentation of the above-noted microbial strains on a nutrient medium containing suitable carbon and nitrogen sources and inorganic salts, using procedures known in the art (see, *e.g.,* Bennett, J.W. and LaSure, L. (eds.), More Gene Manipulations in Fungi, Academic Press, CA, 1991). Suitable media are available from commercial suppliers or may be prepared according to published compositions (*e.g.,* in catalogues of the American Type Culture Collection). Temperature ranges and other conditions suitable for growth and enzyme production are known in the art (see, *e.g.,* Bailey, J.E., and Ollis, D.F., Biochemical Engineering Fundamentals, McGraw-Hill Book Company, NY, 1986).

The fermentation can be any method of cultivation of a cell resulting in the expression or isolation of an enzyme. Fermentation may, therefore, be understood as comprising shake flask cultivation, or small- or large-scale fermentation (including continuous, batch, fed-batch, or solid state fermentations) in laboratory or industrial fermentors performed in a suitable medium and under conditions allowing the enzyme to be expressed or isolated. The resulting enzymes produced by the methods described above may be recovered from the fermentation medium and purified by conventional procedures.

## Nucleic Acid Constructs

An isolated polynucleotide encoding a polypeptide, *e.g.,* a polypeptide having cellulolytic enhancing activity, a cellulolytic enzyme, a hemicellulolytic enzyme, etc., may be manipulated in a variety of ways to provide for expression of the polypeptide by constructing a nucleic acid construct comprising an isolated polynucleotide encoding the polypeptide operably linked to one or more (*e.g.,* several) control sequences that direct the expression of the coding sequence in a suitable host cell under conditions compatible with the control sequences. Manipulation of the polynucleotide's sequence prior to its insertion into a vector may be desirable or necessary depending on the expression vector. The techniques for modifying polynucleotide sequences utilizing recombinant DNA methods are well known in the art.

The control sequence may be a promoter sequence, a polynucleotide that is recognized by a host cell for expression of a polynucleotide encoding a polypeptide. The promoter sequence contains transcriptional control sequences that mediate the expression of the polypeptide. The promoter may be any polynucleotide that shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription of the nucleic acid constructs in the present invention in a bacterial host cell are the promoters obtained from the *Bacillus amyloliquefaciens* alpha-amylase gene (*amyQ*), *Bacillus licheniformis* alpha-amylase gene (*amyL*), *Bacillus licheniformis* penicillinase gene (*penP*), *Bacillus stearothermophilus* maltogenic amylase gene (*amyM*), *Bacillus subtilis* levansucrase gene (*sacB*), *Bacillus subtilis xylA* and *xylB* genes, *E. coli lac* operon, *Streptomyces coelicolor* agarase gene (*dagA*), and prokaryotic beta-lactamase gene (Villa-Kamaroff et al., 1978, Proc. Natl. Acad. Sci. USA 75: 3727-3731), as well as the *tac* promoter (DeBoer et a/., 1983, Proc. Natl. Acad. Sci. USA 80: 21-25). Further promoters are described in "Useful proteins from recombinant bacteria" in Gilbert et a/., 1980, Scientific American, 242: 74-94; and in Sambrook *et al.,* 1989, *supra.*

Examples of suitable promoters for directing the transcription of the nucleic acid constructs in the present invention in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase (*glaA*), *Aspergillus oryzae* TAKA amylase, *Aspergillus oryzae* alkaline protease, *Aspergillus oryzae* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* beta-xylosidase, as well as the NA2-tpi promoter (a modified promoter from a gene encoding a neutral alpha-amylase in *Aspergilli* in which the untranslated leader has been replaced by an untranslated leader from a gene encoding triose phosphate isomerase in *Aspergilli;* non-limiting examples include modified promoters from the gene encoding neutral alpha-amylase in *Aspergillus niger* in which the untranslated leader has been replaced by an untranslated leader from the gene encoding triose phosphate isomerase in *Aspergillus nidulans* or *Aspergillus oryzae*); and mutant, truncated, and hybrid promoters thereof.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The control sequence may also be a suitable transcription terminator sequence, which is recognized by a host cell to terminate transcription. The terminator sequence is operably linked to the 3'-terminus of the polynucleotide encoding the polypeptide. Any terminator that is functional in the host cell of choice may be used in the present invention.

Preferred terminators for filamentous fungal host cells are obtained from the genes for *Aspergillus nidulans* anthranilate synthase, *Aspergillus niger* glucoamylase, *Aspergillus niger* alpha-glucosidase, *Aspergillus oryzae* TAKA amylase, and *Fusarium oxysporum* trypsin-like protease.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.,* 1992, *supra.*

The control sequence may also be a suitable leader sequence, when transcribed is a nontranslated region of an mRNA that is important for translation by the host cell. The leader sequence is operably linked to the 5'-terminus of the polynucleotide encoding the polypeptide. Any leader sequence that is functional in the host cell of choice may be used.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polynucleotide and, when transcribed, is recognized by the host cell as a signal to add polyadenosine residues to transcribed mRNA. Any polyadenylation sequence that is functional in the host cell of choice may be used.

Preferred polyadenylation sequences for filamentous fungal host cells are obtained from the genes for *Aspergillus oryzae* TAKA amylase, *Aspergillus niger* glucoamylase, *Aspergillus nidulans* anthranilate synthase, *Fusarium oxysporum* trypsin-like protease, and *Aspergillus niger* alpha-glucosidase.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of a polypeptide and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. The foreign signal peptide coding sequence may be required where the coding sequence does not naturally contain a signal peptide coding sequence. Alternatively, the foreign signal peptide coding sequence may simply replace the natural signal peptide coding sequence in order to enhance secretion of the polypeptide. However, any signal peptide coding sequence that directs the expressed polypeptide into the secretory pathway of a host cell of choice may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (*nprT, nprS, nprM*), and *Bacillus subtilis prsA.* Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.,* 1992, *supra.*

The control sequence may also be a propeptide coding sequence that encodes a propeptide positioned at the N-terminus of a polypeptide. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present at the N-terminus of a polypeptide, the propeptide sequence is positioned next to the N-terminus of a polypeptide and the signal peptide sequence is positioned next to the N-terminus of the propeptide sequence.

It may also be desirable to add regulatory sequences that allow the regulation of the expression of the polypeptide relative to the growth of the host cell. Examples of regulatory systems are those that cause the expression of the gene to be turned on or off in response to a chemical or physical stimulus, including the presence of a regulatory compound. Regulatory systems in prokaryotic systems include the *lac, tac,* and *trp* operator systems. In yeast, the ADH2 system or GAL1 system may be used. In filamentous fungi, the *Aspergillus niger* glucoamylase promoter, *Aspergillus* oryzae TAKA alpha-amylase promoter, and *Aspergillus oryzae* glucoamylase promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the polypeptide would be operably linked with the regulatory sequence.

## Expression Vectors

The various nucleotide and control sequences may be joined together to produce a recombinant expression vector that may include one or more (*e.g.,* several) convenient restriction sites to allow for insertion or substitution of a polynucleotide encoding a polypeptide, *e.g.,* a polypeptide having cellulolytic enhancing activity, a cellulolytic enzyme, a hemicellulolytic enzyme, etc., at such sites. Alternatively, the polynucleotide may be expressed by inserting the polynucleotide or a nucleic acid construct comprising the sequence into an appropriate vector for expression. In creating the expression vector, the coding sequence is located in the vector so that the coding sequence is operably linked with the appropriate control sequences for expression.

The recombinant expression vector may be any vector (*e.g.,* a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, *i.e.,* a vector that exists as an extrachromosomal entity, the replication of which is independent of chromosomal replication, *e.g.,* a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more (*e.g.,* several) selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are the *dal* genes from *Bacillus subtilis* or *Bacillus licheniformis,* or markers that confer antibiotic resistance such as ampicillin, chloramphenicol, kanamycin, or tetracycline resistance. Suitable markers for yeast host cells are ADE2, HIS3, LEU2, LYS2, MET3, TRP1, and URA3. Selectable markers for use in a filamentous fungal host cell include, but are not limited to, *amdS* (acetamidase), *argB* (ornithine carbamoyltransferase), bar (phosphinothricin acetyltransferase), *hph* (hygromycin phosphotransferase), *niaD* (nitrate reductase), *pyrG* (orotidine-5'-phosphate decarboxylase), *sC* (sulfate adenyltransferase), and *trpC* (anthranilate synthase), as well as equivalents thereof. Preferred for use in an *Aspergillus* cell are the *amdS* and *pyrG* genes of *Aspergillus nidulans* or *Aspergillus oryzae* and the bar gene of *Streptomyces hygroscopicus.*

The vector preferably contains an element(s) that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

Examples of bacterial origins of replication are the origins of replication of plasmids pBR322, pUC19, pACYC177, and pACYC184 permitting replication in *E. coli,* and pUB110, pE194, pTA1060, and pAMβ1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors are well known to one skilled in the art (see, e.g., Sambrook *et al.,* 1989, *supra*).

## Host Cells

Recombinant host cells comprising a polynucleotide encoding a polypeptide, e.g., a polypeptide having cellulolytic enhancing activity, a cellulolytic enzyme, a hemicellulolytic enzyme, etc., can be advantageously used in the recombinant production of the polypeptide. A construct or vector comprising such a polynucleotide is introduced into a host cell so that the vector is maintained as a chromosomal integrant or as a self-replicating extrachromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be any cell useful in the recombinant production of a polypeptide, *e.g.,* a prokaryote or a eukaryote.

The prokaryotic host cell may be any gram-positive or gram-negative bacterium. Gram-positive bacteria include, but not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but not limited to, *Campylobacter, E. coli, Flavobacterium, Fusobacterium, Helicobacter, Ilyobacter, Neisseria, Pseudomonas, Salmonella,* and *Ureaplasma.*

The bacterial host cell may be any *Bacillus* cell including, but not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells.

The bacterial host cell may also be any *Streptococcus* cell including, but not limited to, *Streptococcus equisimilis, Streptococcus pyogenes, Streptococcus uberis,* and *Streptococcus equi* subsp. *Zooepidemicus* cells.

The bacterial host cell may also be any *Streptomyces* cell including, but not limited to, *Streptomyces achromogenes, Streptomyces avermitilis, Streptomyces coelicolor, Streptomyces griseus,* and *Streptomyces lividans* cells.

The introduction of DNA into a *Bacillus* cell may, for instance, be effected by protoplast transformation (see, *e.g.,* Chang and Cohen, 1979, Mol. Gen. Genet. 168: 111-115), by using competent cells (see, *e.g.,* Young and Spizizen, 1961, J. Bacteriol. 81: 823-829, or Dubnau and Davidoff-Abelson, 1971, J. Mol. Biol. 56: 209-221), by electroporation (see, *e.g.,* Shigekawa and Dower, 1988, Biotechniques 6: 742-751), or by conjugation (see, *e.g.,* Koehler and Thorne, 1987, J. Bacteriol. 169: 5271-5278). The introduction of DNA into an *E. coli* cell may, for instance, be effected by protoplast transformation (see, *e.g.,* Hanahan, 1983, J. Mol. Biol. 166: 557-580) or electroporation (see, *e.g.,* Dower et al., 1988, Nucleic Acids Res. 16: 6127-6145). The introduction of DNA into a *Streptomyces* cell may, for instance, be effected by protoplast transformation and electroporation (see, *e.g.,* Gong et al., 2004, Folia Microbiol. (Praha) 49: 399-405), by conjugation (see, *e.g.,* Mazodier et al., 1989, J. Bacteriol. 171: 3583-3585), or by transduction (see, *e.g.,* Burke et al., 2001, Proc. Natl. Acad. Sci. USA 98: 6289-6294). The introduction of DNA into a *Pseudomonas* cell may, for instance, be effected by electroporation (see, *e.g.,* Choi et a/., 2006, J. Microbiol. Methods 64: 391-397) or by conjugation (see, *e.g.,* Pinedo and Smets, 2005, Appl. Environ. Microbiol. 71: 51-57). The introduction of DNA into a *Streptococcus* cell may, for instance, be effected by natural competence (see, *e.g.,* Perry and Kuramitsu, 1981, Infect. Immun. 32: 1295-1297), by protoplast transformation (see, *e.g.,* Catt and Jollick, 1991, Microbios 68: 189-207), by electroporation (see, *e.g.,* Buckley et al., 1999, Appl. Environ. Microbiol. 65: 3800-3804) or by conjugation (see, *e.g.,* Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein includes the phyla Ascomycota, Basidiomycota, Chytridiomycota, and Zygomycota (as defined by Hawksworth et al., In, Ainsworth and Bisby's Dictionary of The Fungi, 8th edition, 1995, CAB International, University Press, Cambridge, UK) as well as the Oomycota (as cited in Hawksworth *et al.,* 1995, *supra,* page 171) and all mitosporic fungi (Hawksworth *et al.,* 1995, *supra).*

The fungal host cell may be a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, F.A., Passmore, S.M., and Davenport, R.R., eds, Soc. App. Bacteriol. Symposium Series No. 9, 1980).

The yeast host cell may be a *Candida, Hansenula, Kluyveromyces, Pichia, Saccharomyces, Schizosaccharomyces,* or *Yarrowia* cell such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

The fungal host cell may be a filamentous fungal cell. "Filamentous fungi" include all filamentous forms of the subdivision Eumycota and Oomycota (as defined by Hawksworth *et al.,* 1995, *supra).* The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus,*

*Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et a/., 1984, Proc. Natl. Acad. Sci. USA 81: 1470-1474, and Christensen et al., 1988, Bio/Technology 6: 1419-1422. Suitable methods for transforming *Fusarium* species are described by Malardier et a/., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito etal., 1983, J. Bacteriol. 153: 163; and Hinnen et al., 1978, Proc. Natl. Acad. Sci. USA 75: 1920.

## Methods of Production

Methods for producing a polypeptide, *e.g.,* a polypeptide having cellulolytic enhancing activity, a cellulolytic enzyme, a hemicellulolytic enzyme, etc., comprise (a) cultivating a cell, which in its wild-type form is capable of producing the polypeptide, under conditions conducive for production of the polypeptide; and (b) recovering the polypeptide.

Alternatively, methods for producing a polypeptide, e.g., a polypeptide having cellulolytic enhancing activity, a cellulolytic enzyme, a hemicellulolytic enzyme, etc., comprise (a) cultivating a recombinant host cell under conditions conducive for production of the polypeptide; and (b) recovering the polypeptide.

In the production methods, the cells are cultivated in a nutrient medium suitable for production of the polypeptide using methods well known in the art. For example, the cell may be cultivated by shake flask cultivation, and small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.,* in catalogues of the American Type Culture Collection). If the polypeptide is secreted into the nutrient medium, the polypeptide can be recovered directly from the medium. If the polypeptide is not secreted, it can be recovered from cell lysates.

The polypeptide may be detected using methods known in the art that are specific for the polypeptides. These detection methods may include use of specific antibodies, formation of an enzyme product, or disappearance of an enzyme substrate. For example, an enzyme assay may be used to determine the activity of the polypeptide. The polypeptides having cellulolytic enhancing activity are detected using the methods described herein.

The resulting broth may be used as is or the polypeptide may be recovered using methods known in the art. For example, the polypeptide may be recovered from the nutrient medium by conventional procedures including, but not limited to, centrifugation, filtration, extraction, spray-drying, evaporation, or precipitation.

The polypeptides may be purified by a variety of procedures known in the art including, but not limited to, chromatography (*e.g.*, ion exchange, affinity, hydrophobic, chromatofocusing, and size exclusion), electrophoretic procedures (*e.g.*, preparative isoelectric focusing), differential solubility (*e.g.*, ammonium sulfate precipitation), SDS-PAGE, or extraction (see, *e.g.,* Protein Purification, J.-C. Janson and Lars Ryden, editors, VCH Publishers, New York, 1989) to obtain substantially pure polypeptides.

In an alternative aspect, the polypeptide is not recovered, but rather a host cell expressing a polypeptide is used as a source of the polypeptide.

## Methods for Processing Cellulosic Material

The methods of the present invention can be used to saccharify a cellulosic material to fermentable sugars and convert the fermentable sugars to many useful substances, *e.g.,* fuel, potable ethanol, and/or fermentation products (*e.g.,* acids, alcohols, ketones, gases, and the like). The production of a desired fermentation product from cellulosic material typically involves pretreatment, enzymatic hydrolysis (saccharification), and fermentation.

The present invention relates to methods for degrading or converting a cellulosic material, comprising: treating the cellulosic material with an enzyme composition comprising a cellulase in the presence of a GH61 polypeptide having cellulolytic enhancing activity and a liquor arising from treatment of a lignocellulose material, a hemicellulose material, a lignacious material, monosaccharides of the lignocellulose material, monosaccharides of the hemicellulose material, or a combination thereof, wherein the liquor is present in an amount that minimizes inhibition of a cellulase composition of 1 to 20% (v/v), wherein the combination of the GH61 polypeptide having cellulolytic enhancing activity and the liquor enhances hydrolysis of the cellulosic material by the enzyme composition, wherein the enhanced hydrolysis is assessed according to Equation 3:

$$\textit{GH61 effect} = \frac{\%\ \text{conversion}_{(+\ \text{GH61+ liquor})}}{\%\ \text{conversion}_{(\text{no GH61+ liquor})}} \qquad \text{(Equation 3)}$$

and the equation ratio is greater than 1,
wherein the GH61 polypeptide having cellulolytic enhancing activity comprises the following motifs:
[ILMV]-P-X(4,5)-G-X-Y-[ILMV]-X-R-X-[EQ]-X(4)-[HNQ] (SEQ ID NO: 127 or SEQ ID NO: 128) and [FW]-[TF]-K-[AIV], wherein X is any amino acid, X(4,5) is any amino acid at 4 or 5 contiguous positions, and X(4) is any amino acid at 4 contiguous positions.

In one aspect, the method above further comprises recovering the degraded or converted cellulosic material. Soluble products of degradation or conversion of the cellulosic material can be separated from the insoluble cellulosic material using technology well known in the art such as, for example, centrifugation, filtration, and gravity settling.

The present invention also relates to methods for producing a fermentation product, comprising:

(a) saccharifying a cellulosic material with an enzyme composition comprising a cellulase in the presence of a GH61 polypeptide having cellulolytic enhancing activity and a liquor arising from treatment of a lignocellulose material, a hemicellulose material, a lignacious material, monosaccharides of the lignocellulose material, monosaccharides of the hemicellulose material, or a combination thereof, wherein the liquor is present in an amount that minimizes inhibition of a cellulase composition of about 1 to about 20% (v/v), wherein the liquor is present in an amount that minimizes inhibition of a cellulase composition of 1 to 20% (v/v), wherein the combination of the GH61 polypeptide having cellulolytic enhancing activity and the liquor enhances hydrolysis of the cellulosic material by the enzyme composition, wherein the enhanced hydrolysis is assessed according to Equation 3:

$$\textit{GH61 effect} = \frac{\%\ \text{conversion}_{(+\ \text{GH61+ liquor})}}{\%\ \text{conversion}_{(\text{no GH61+ liquor})}} \qquad \text{(Equation 3)}$$

and the equation ratio is greater than 1,
wherein the polypeptide having cellulolytic enhancing activity comprises the following motifs:
[ILMV]-P-X(4,5)-G-X-Y-[ILMV]-X-R-X-[EQ]-X(4)-[HNQ] (SEQ ID NO: 127 or SEQ ID NO: 128) and [FW]-[TF]-K-[AIV], wherein X is any amino acid, X(4,5) is any amino acid at 4 or 5 contiguous positions, and X(4) is any amino acid at 4 contiguous positions;
(b) fermenting the saccharified cellulosic material with one or more fermenting microorganisms to produce the fermentation product; and
(c) recovering the fermentation product from the fermentation.

The present invention also relates to methods of fermenting a cellulosic material, comprising:

(a) saccharifying a cellulosic material with an enzyme composition comprising a cellulase in the presence of a GH61 polypeptide having cellulolytic enhancing activity and a liquor arising from treatment of a lignocellulose material, a hemicellulose material, a lignacious material, monosaccharides of the lignocellulose material, monosaccharides of the hemicellulose material, or a combination thereof, wherein the liquor is present in an amount that minimizes inhibition of a cellulase composition of about 1 to about 20% (v/v), wherein the liquor is present in an amount that minimizes inhibition of a cellulase composition of 1 to 20% (v/v), wherein the combination of the GH61 polypeptide having cellulolytic enhancing activity and the liquor enhances hydrolysis of the cellulosic material by the enzyme composition, wherein the enhanced hydrolysis is assessed according to Equation 3:

$$GH61\ effect = \frac{\%\ conversion_{(+\ GH61+\ liquor)}}{\%\ conversion_{(no\ GH61+\ liquor)}} \quad \text{(Equation 3)}$$

and the equation ratio is greater than 1,
wherein the polypeptide having cellulolytic enhancing activity comprises the following motifs:
[ILMV]-P-X(4,5)-G-X-Y-[ILMV]-X-R-X-[EQ]-X(4)-[HNQ] (SEQ ID NO: 127 or SEQ ID NO: 128) and [FW]-[TF]-K-[AIV], wherein X is any amino acid, X(4,5) is any amino acid at 4 or 5 contiguous positions, and X(4) is any amino acid at 4 contiguous positions;
(b) fermenting the saccharified cellulosic material with one or more fermenting microorganisms to produce the fermentation product; and
(c) recovering the fermentation product from the fermentation.

In one aspect, the liquor is recovered following saccharification or fermentation and recycled back to a new saccharification reaction. Recycling of the liquor can be accomplished using processes conventional in the art.

The processing of cellulosic material according to the present invention can be accomplished using processes conventional in the art. Moreover, the methods of the present invention can be implemented using any conventional biomass processing apparatus configured to operate in accordance with the invention.

Hydrolysis (saccharification) and fermentation, separate or simultaneous, include, but are not limited to, separate hydrolysis and fermentation (SHF); simultaneous saccharification and fermentation (SSF); simultaneous saccharification and cofermentation (SSCF); hybrid hydrolysis and fermentation (HHF); separate hydrolysis and co-fermentation (SHCF); hybrid hydrolysis and co-fermentation (HHCF); and direct microbial conversion (DMC), also sometimes called consolidated bioprocessing (CBP). SHF uses separate process steps to first enzymatically hydrolyze cellulosic material to fermentable sugars, e.g., glucose, cellobiose, cellotriose, and pentose monomers, and then ferment the fermentable sugars to ethanol. In SSF, the enzymatic hydrolysis of cellulosic material and the fermentation of sugars to ethanol are combined in one step (Philippidis, G. P., 1996, Cellulose bioconversion technology, in Handbook on Bioethanol: Production and Utilization, Wyman, C. E., ed., Taylor & Francis, Washington, DC, 179-212). SSCF involves the cofermentation of multiple sugars (Sheehan, J., and Himmel, M., 1999, Enzymes, energy and the environment: A strategic perspective on the U.S. Department of Energy's research and development activities for bioethanol, Biotechnol. Prog. 15: 817-827). HHF involves a separate hydrolysis step, and in addition a simultaneous saccharification and hydrolysis step, which can be carried out in the same reactor. The steps in an HHF process can be carried out at different temperatures, *i.e.,* high temperature enzymatic saccharification followed by SSF at a lower temperature that the fermentation strain can tolerate. DMC combines all three processes (enzyme production, hydrolysis, and fermentation) in one or more (e.g., several) steps where the same organism is used to produce the enzymes for conversion of the cellulosic material to fermentable sugars and to convert the fermentable sugars into a final product (Lynd, L. R., Weimer, P. J., van Zyl, W. H., and Pretorius, I. S., 2002, Microbial cellulose utilization: Fundamentals and biotechnology, Microbiol. Mol. Biol. Reviews 66: 506-577). It is understood herein that any method known in the art comprising pretreatment, enzymatic hydrolysis (saccharification), fermentation, or a combination thereof, can be used in the practicing the methods of the present invention.

A conventional apparatus can include a fed-batch stirred reactor, a batch stirred reactor, a continuous flow stirred reactor with ultrafiltration, and/or a continuous plug-flow column reactor (Fernanda de Castilhos Corazza, Flávio Faria de Moraes, Gisella Maria Zanin and Ivo Neitzel, 2003, Optimal control in fed-batch reactor for the cellobiose hydrolysis, Acta Scientiarum. Technology 25: 33-38; Gusakov, A. V., and Sinitsyn, A. P., 1985, Kinetics of the enzymatic hydrolysis of cellulose: 1. A mathematical model for a batch reactor process, Enz. Microb. Technol. 7: 346-352), an attrition reactor (Ryu, S. K., and Lee, J. M., 1983, Bioconversion of waste cellulose by using an attrition bioreactor, Biotechnol. Bioeng. 25: 53-65), or a reactor with intensive stirring induced by an electromagnetic field (Gusakov, A. V., Sinitsyn, A. P., Davydkin, I. Y., Davydkin, V. Y., Protas, O. V., 1996, Enhancement of enzymatic cellulose hydrolysis using a novel type of bioreactor with intensive stirring induced by electromagnetic field, Appl. Biochem. Biotechnol. 56: 141-153). Additional reactor types include: fluidized bed, upflow blanket, immobilized, and extruder type reactors for hydrolysis and/or fermentation.

Pretreatment. In practicing the methods of the present invention, any pretreatment process known in the art can be used to disrupt plant cell wall components of cellulosic material (Chandra et al., 2007, Substrate pretreatment: The key to effective enzymatic hydrolysis of lignocellulosics? Adv. Biochem. Engin./Biotechnol. 108: 67-93; Galbe and Zacchi, 2007, Pretreatment of lignocellulosic materials for efficient bioethanol production, Adv. Biochem. Engin. / Biotechnol. 108: 41-65; Hendriks and Zeeman, 2009, Pretreatments to enhance the digestibility of lignocellulosic biomass, Bioresource Technol. 100: 10-18; Mosier et al., 2005, Features of promising technologies for pretreatment of lignocellulosic biomass, Bioresource Technol. 96: 673-686; Taherzadeh and Karimi, 2008, Pretreatment of lignocellulosic wastes to improve ethanol and biogas production: A review, Int. J. of Mol. Sci. 9: 1621-1651; Yang and Wyman, 2008, Pretreatment: the key to unlocking low-cost cellulosic ethanol, Biofuels Bioproducts and Biorefining-Biofpr. 2: 26-40).

The cellulosic material can also be subjected to particle size reduction, pre-soaking, wetting, washing, or conditioning prior to pretreatment using methods known in the art.

Conventional pretreatments include, but are not limited to, steam pretreatment (with or without explosion), dilute acid pretreatment, hot water pretreatment, alkaline pretreatment, lime pretreatment, wet oxidation, wet explosion, ammonia fiber explosion, organosolv pretreatment, and biological pretreatment. Additional pretreatments include ammonia percolation, ultrasound, electroporation, microwave, supercritical $CO_2$, supercritical $H_2O$, ozone, and gamma irradiation pretreatments.

The cellulosic material can be pretreated before hydrolysis and/or fermentation. Pretreatment is preferably performed prior to the hydrolysis. Alternatively, the pretreatment can be carried out simultaneously with enzyme hydrolysis to release fermentable sugars, such as glucose, xylose, and/or cellobiose. In most cases the pretreatment step itself results in some conversion of biomass to fermentable sugars (even in absence of enzymes).

Steam Pretreatment: In steam pretreatment, cellulosic material is heated to disrupt the plant cell wall components, including lignin, hemicellulose, and cellulose to make the cellulose and other fractions, *e.g.,* hemicellulose, accessible to enzymes. Cellulosic material is passed to or through a reaction vessel where steam is injected to increase the temperature to the required temperature and pressure and is retained therein for the desired reaction time. Steam pretreatment is preferably done at 140-230°C, more preferably 160-200°C, and most preferably 170-190°C, where the optimal temperature range depends on any addition of a chemical catalyst. Residence time for the steam pretreatment is preferably 1-15 minutes, more preferably 3-12 minutes, and most preferably 4-10 minutes, where the optimal residence time depends on temperature range and any addition of a chemical catalyst. Steam pretreatment allows for relatively high solids loadings, so that cellulosic material is generally only moist during the pretreatment. The steam pretreatment is often combined with an explosive discharge of the material after the pretreatment, which is known as steam explosion, that is, rapid flashing to atmospheric pressure and turbulent flow of the material to increase the accessible surface area by fragmentation (Duff and Murray, 1996, Bioresource Technology 855: 1-33; Galbe and Zacchi, 2002, Appl. Microbiol. Biotechnol. 59: 618-628; U.S. Patent Application No. 20020164730). During steam pretreatment, hemicellulose acetyl groups are cleaved and the resulting acid autocatalyzes partial hydrolysis of the hemicellulose to monosaccharides and oligosaccharides. Lignin is removed to only a limited extent.

A catalyst such as $H_2SO_4$ or $SO_2$ (typically 0.3 to 3% w/w) is often added prior to steam pretreatment, which decreases the time and temperature, increases the recovery, and improves enzymatic hydrolysis (Ballesteros et al., 2006, Appl. Biochem. Biotechnol. 129-132: 496-508; Varga et a/., 2004, Appl. Biochem. Biotechnol. 113-116: 509-523; Sassner et a/., 2006, Enzyme Microb. Technol. 39: 756-762).

Chemical Pretreatment: The term "chemical treatment" refers to any chemical pretreatment that promotes the separation and/or release of cellulose, hemicellulose, and/or lignin. Examples of suitable chemical pretreatment processes include, for example, dilute acid pretreatment, lime pretreatment, wet oxidation, ammonia fiber/freeze explosion (AFEX), ammonia percolation (APR), and organosolv pretreatments.

In dilute acid pretreatment, cellulosic material is mixed with dilute acid, typically $H_2SO_4$, and water to form a slurry, heated by steam to the desired temperature, and after a residence time flashed to atmospheric pressure. The dilute acid pretreatment can be performed with a number of reactor designs, *e.g.,* plug-flow reactors, counter-current reactors, or continuous counter-current shrinking bed reactors (Duff and Murray, 1996, *supra;* Schell et a/., 2004, Bioresource Technol. 91: 179-188; Lee et al., 1999, Adv. Biochem. Eng. Biotechnol. 65: 93-115).

Several methods of pretreatment under alkaline conditions can also be used. These alkaline pretreatments include, but are not limited to, lime pretreatment, wet oxidation, ammonia percolation (APR), and ammonia fiber/freeze explosion (AFEX).

Lime pretreatment is performed with calcium carbonate, sodium hydroxide, or ammonia at low temperatures of 85-150°C and residence times from 1 hour to several days (Wyman et al., 2005, Bioresource Technol. 96: 1959-1966; Mosier et al., 2005, Bioresource Technol. 96: 673-686). WO 2006/110891, WO 2006/11899, WO 2006/11900, and WO 2006/110901 disclose pretreatment methods using ammonia.

Wet oxidation is a thermal pretreatment performed typically at 180-200°C for 5-15 minutes with addition of an oxidative agent such as hydrogen peroxide or over-pressure of oxygen (Schmidt and Thomsen, 1998, Bioresource Technol. 64: 139-151; Palonen et al., 2004, Appl. Biochem. Biotechnol. 117: 1-17; Varga et al., 2004, Biotechnol. Bioeng. 88: 567-574; Martin et al., 2006, J. Chem. Technol. Biotechnol. 81: 1669-1677). The pretreatment is performed at preferably 1-40% dry matter, more preferably 2-30% dry matter, and most preferably 5-20% dry matter, and often the initial pH is increased by the addition of alkali such as sodium carbonate.

A modification of the wet oxidation pretreatment method, known as wet explosion (combination of wet oxidation and steam explosion), can handle dry matter up to 30%. In wet explosion, the oxidizing agent is introduced during pretreatment after a certain residence time. The pretreatment is then ended by flashing to atmospheric pressure (WO 2006/032282).

Ammonia fiber explosion (AFEX) involves treating cellulosic material with liquid or gaseous ammonia at moderate temperatures such as 90-100°C and high pressure such as 17-20 bar for 5-10 minutes, where the dry matter content can be as high as 60% (Gollapalli et al., 2002, Appl. Biochem. Biotechnol. 98: 23-35; Chundawat et al., 2007, Biotechnol. Bioeng. 96: 219-231; Alizadeh et al., 2005, Appl. Biochem. Biotechnol. 121: 1133-1141; Teymouri et al., 2005, Bioresource Technol. 96: 2014-2018). AFEX pretreatment results in the depolymerization of cellulose and partial hydrolysis of hemicellulose. Lignin-carbohydrate complexes are cleaved.

Organosolv pretreatment delignifies cellulosic material by extraction using aqueous ethanol (40-60% ethanol) at 160-200°C for 30-60 minutes (Pan et al., 2005, Biotechnol. Bioeng. 90: 473-481; Pan et al., 2006, Biotechnol. Bioeng. 94: 851-861; Kurabi et al., 2005, Appl. Biochem. Biotechnol. 121: 219-230). Sulphuric acid is usually added as a catalyst. In organosolv pretreatment, the majority of hemicellulose is removed.

Other examples of suitable pretreatment methods are described by Schell et al., 2003, Appl. Biochem. and Biotechnol. Vol. 105-108, p. 69-85, and Mosier et al., 2005, Bioresource Technology 96: 673-686, and U.S. Published Application 2002/0164730.

In one aspect, the chemical pretreatment is preferably carried out as an acid treatment, and more preferably as a continuous dilute and/or mild acid treatment. The acid is typically sulfuric acid, but other acids can also be used, such as acetic acid, citric acid, nitric acid, phosphoric acid, tartaric acid, succinic acid, hydrogen chloride, or mixtures thereof. Mild acid treatment is conducted in the pH range of preferably 1-5, more preferably 1-4, and most preferably 1-3. In one aspect, the acid concentration is in the range from preferably 0.01 to 20 wt % acid, more preferably 0.05 to 10 wt % acid, even more preferably 0.1 to 5 wt % acid, and most preferably 0.2 to 2.0 wt % acid. The acid is contacted with cellulosic material and held at a temperature in the range of preferably 160-220°C, and more preferably 165-195°C, for periods ranging from seconds to minutes to, e.g., 1 second to 60 minutes.

In another aspect, pretreatment is carried out as an ammonia fiber explosion step (AFEX pretreatment step).

In another aspect, pretreatment takes place in an aqueous slurry. In preferred aspects, cellulosic material is present during pretreatment in amounts preferably between 10-80 wt %, more preferably between 20-70 wt %, and most preferably between 30-60 wt %, such as around 50 wt %. The pretreated cellulosic material can be unwashed or washed using any method known in the art, *e.g.,* washed with water.

Mechanical Pretreatment: The term "mechanical pretreatment" refers to various types of grinding or milling (*e.g.,* dry milling, wet milling, or vibratory ball milling).

Physical Pretreatment: The term "physical pretreatment" refers to any pretreatment that promotes the separation and/or release of cellulose, hemicellulose, and/or lignin from cellulosic material. For example, physical pretreatment can involve irradiation (*e.g.,* microwave irradiation), steaming/steam explosion, hydrothermolysis, and combinations thereof.

Physical pretreatment can involve high pressure and/or high temperature (steam explosion). In one aspect, high pressure means pressure in the range of preferably about 300 to about 600 psi, more preferably about 350 to about 550 psi, and most preferably about 400 to about 500 psi, such as around 450 psi. In another aspect, high temperature means temperatures in the range of about 100 to about 300°C, preferably about 140 to about 235°C. In a preferred aspect, mechanical pretreatment is performed in a batch-process, steam gun hydrolyzer system that uses high pressure and high temperature as defined above, *e.g.,* a Sunds Hydrolyzer available from Sunds Defibrator AB, Sweden.

Combined Physical and Chemical Pretreatment: Cellulosic material can be pretreated both physically and chemically. For instance, the pretreatment step can involve dilute or mild acid treatment and high temperature and/or pressure treatment. The physical and chemical pretreatments can be carried out sequentially or simultaneously, as desired. A mechanical pretreatment can also be included.

Accordingly, in a preferred aspect, cellulosic material is subjected to mechanical, chemical, or physical pretreatment, or any combination thereof, to promote the separation and/or release of cellulose, hemicellulose, and/or lignin.

Biological Pretreatment: The term "biological pretreatment" refers to any biological pretreatment that promotes the separation and/or release of cellulose, hemicellulose, and/or lignin from cellulosic material. Biological pretreatment techniques can involve applying lignin-solubilizing microorganisms (see, for example, Hsu, T.-A., 1996, Pretreatment of biomass, in Handbook on Bioethanol: Production and Utilization, Wyman, C. E., ed., Taylor & Francis, Washington, DC, 179-212; Ghosh and Singh, 1993, Physicochemical and biological treatments for enzymatic/microbial conversion of cellulosic biomass, Adv. Appl. Microbiol. 39: 295-333; McMillan, J. D., 1994, Pretreating lignocellulosic biomass: a review, in Enzymatic Conversion of Biomass for Fuels Production, Himmel, M. E., Baker, J. O., and Overend, R. P., eds., ACS Symposium Series 566, American Chemical Society, Washington, DC, chapter 15; Gong, C. S., Cao, N. J., Du, J., and Tsao, G. T., 1999, Ethanol production from renewable resources, in Advances in Biochemical Engineering/Biotechnology, Scheper, T., ed., Springer-Verlag Berlin Heidelberg, Germany, 65: 207-241; Olsson and Hahn-Hagerdal, 1996, Fermentation of lignocellulosic hydrolysates for ethanol production, Enz. Microb. Tech. 18: 312-331; and Vallander and Eriksson, 1990, Production of ethanol from lignocellulosic materials: State of the art, Adv. Biochem. Eng./Biotechnol. 42: 63-95).

Saccharification. In the hydrolysis step, also known as saccharification, the cellulosic material, e.g., pretreated, is hydrolyzed to break down cellulose and alternatively also hemicellulose to fermentable sugars, such as glucose, cellobiose, xylose, xylulose, arabinose, mannose, galactose, and/or soluble oligosaccharides. The hydrolysis is performed enzymatically by an enzyme composition in the presence of a polypeptide having cellulolytic enhancing activity and a liquor. The enzyme and protein components of the compositions can be added sequentially.

Enzymatic hydrolysis is preferably carried out in a suitable aqueous environment under conditions that can be readily determined by one skilled in the art. In a preferred aspect, hydrolysis is performed under conditions suitable for the activity of the enzyme(s), *i.e.,* optimal for the enzyme(s). The hydrolysis can be carried out as a fed batch or continuous process where the pretreated cellulosic material (substrate) is fed gradually to, for example, an enzyme containing hydrolysis solution.

The saccharification is generally performed in stirred-tank reactors or fermentors under controlled pH, temperature, and mixing conditions. Suitable process time, temperature and pH conditions can readily be determined by one skilled in the art. For example, the saccharification can last up to 200 hours, but is typically performed for preferably about 12 to about 96 hours, more preferably about 16 to about 72 hours, and most preferably about 24 to about 48 hours. The temperature is in the range of preferably about 25°C to about 70°C, more preferably about 30°C to about 65°C, and more preferably about 40°C to 60°C, in particular about 50°C. The pH is in the range of preferably about 3 to about 8, more preferably about 3.5 to about 7, and most preferably about 4 to about 6, in particular about pH 5. The dry solids content is in the range of preferably about 5 to about 50 wt %, more preferably about 10 to about 40 wt %, and most preferably about 20 to about 30 wt %.

The optimum amounts of the enzymes and polypeptides having cellulolytic enhancing activity depend on several factors including, but not limited to, the mixture of component cellulolytic enzymes, the cellulosic substrate, the concentration of cellulosic substrate, the pretreatment(s) of the cellulosic substrate, temperature, time, pH, and inclusion of fermenting organism (e.g., yeast for Simultaneous Saccharification and Fermentation).

In one aspect, an effective amount of cellulolytic or hemicellulolytic enzyme protein to cellulosic material is about 0.5 to about 50 mg, preferably at about 0.5 to about 40 mg, more preferably at about 0.5 to about 25 mg, more preferably at about 0.75 to about 20 mg, more preferably at about 0.75 to about 15 mg, even more preferably at about 0.5 to about 10 mg, and most preferably at about 2.5 to about 10 mg per g of cellulosic material.

In another aspect, an effective amount of a polypeptide having cellulolytic enhancing activity to cellulosic material is about 0.01 to about 50.0 mg, preferably about 0.01 to about 40 mg, more preferably about 0.01 to about 30 mg, more preferably about 0.01 to about 20 mg, more preferably about 0.01 to about 10 mg, more preferably about 0.01 to about 5 mg, more preferably at about 0.025 to about 1.5 mg, more preferably at about 0.05 to about 1.25 mg, more preferably at about 0.075 to about 1.25 mg, more preferably at about 0.1 to about 1.25 mg, even more preferably at about 0.15 to about 1.25 mg, and most preferably at about 0.25 to about 1.0 mg per g of cellulosic material.

In another aspect, an effective amount of a polypeptide having cellulolytic enhancing activity to cellulolytic enzyme protein is about 0.005 to about 1.0 g, preferably at about 0.01 to about 1.0 g, more preferably at about 0.15 to about 0.75 g, more preferably at about 0.15 to about 0.5 g, more preferably at about 0.1 to about 0.5 g, even more preferably at about 0.1 to about 0.5 g, and most preferably at about 0.05 to about 0.2 g per g of cellulolytic enzyme protein.

Fermentation. The fermentable sugars obtained from the hydrolyzed cellulosic material can be fermented by one or more (*e.g.,* several) fermenting microorganisms capable of fermenting the sugars directly or indirectly into a desired fermentation product. "Fermentation" or "fermentation process" refers to any fermentation process or any process comprising a fermentation step. Fermentation processes also include fermentation processes used in the consumable alcohol industry (*e.g.,* beer and wine), dairy industry (*e.g.,* fermented dairy products), leather industry, and tobacco industry. The fermentation conditions depend on the desired fermentation product and fermenting organism and can easily be determined by one skilled in the art.

In the fermentation step, sugars, released from cellulosic material as a result of the pretreatment and enzymatic hydrolysis steps, are fermented to a product, *e.g.,* ethanol, by a fermenting organism, such as yeast. Hydrolysis (saccharification) and fermentation can be separate or simultaneous, as described herein.

Any suitable hydrolyzed cellulosic material can be used in the fermentation step in practicing the present invention. The material is generally selected based on the desired fermentation product, *i.e.,* the substance to be obtained from the fermentation, and the process employed, as is well known in the art.

The term "fermentation medium" is understood herein to refer to a medium before the fermenting microorganism(s) is(are) added, such as, a medium resulting from a saccharification process, as well as a medium used in a simultaneous saccharification and fermentation process (SSF).

"Fermenting microorganism" refers to any microorganism, including bacterial and fungal organisms, suitable for use in a desired fermentation process to produce a fermentation product. The fermenting organism can be $C_6$ and/or $C_5$ fermenting organisms, or a combination thereof. Both $C_6$ and $C_5$ fermenting organisms are well known in the art. Suitable fermenting microorganisms are able to ferment, *i.e.,* convert, sugars, such as glucose, xylose, xylulose, arabinose, maltose, mannose, galactose, or oligosaccharides, directly or indirectly into the desired fermentation product.

Examples of bacterial and fungal fermenting organisms producing ethanol are described by Lin et al., 2006, Appl. Microbiol. Biotechnol. 69: 627-642.

Examples of fermenting microorganisms that can ferment $C_6$ sugars include bacterial and fungal organisms, such as yeast. Preferred yeast includes strains of the *Saccharomyces* spp., preferably *Saccharomyces cerevisiae.*

Examples of fermenting organisms that can ferment $C_5$ sugars include bacterial and fungal organisms, such as some yeast. Preferred $C_5$ fermenting yeast include strains of *Pichia,* preferably *Pichia stipitis,* such as *Pichia stipitis* CBS 5773; strains of *Candida,* preferably *Candida boidinii, Candida brassicae, Candida sheatae, Candida diddensii, Candida pseudotropicalis,* or *Candida utilis.*

Other fermenting organisms include strains of *Zymomonas,* such as *Zymomonas mobilis; Hansenula,* such as *Hansenula anomala; Kluyveromyces,* such as *K. fragilis; Schizosaccharomyces,* such as S. *pombe; E. coli,* especially *E. coli* strains that have been genetically modified to improve the yield of ethanol; *Clostridium,* such as *Clostridium acetobutylicum, Chlostridium thermocellum,* and *Chlostridium phytofermentans; Geobacillus sp.; Thermoanaerobacter,* such as *Thermoanaerobacter saccharolyticum;* and *Bacillus,* such as *Bacillus coagulans.*

In a preferred aspect, the yeast is a *Saccharomyces* spp. In a more preferred aspect, the yeast is *Saccharomyces cerevisiae.* In another more preferred aspect, the yeast is *Saccharomyces distaticus.* In another more preferred aspect, the yeast is *Saccharomyces uvarum.* In another preferred aspect, the yeast is a *Kluyveromyces.* In another more preferred aspect, the yeast is *Kluyveromyces marxianus.* In another more preferred aspect, the yeast is *Kluyveromyces fragilis.* In another preferred aspect, the yeast is a *Candida.* In another more preferred aspect, the yeast is *Candida boidinii.* In another more preferred aspect, the yeast is *Candida brassicae.* In another more preferred aspect, the yeast is *Candida diddensii.* In another more preferred aspect, the yeast is *Candida pseudotropicalis.* In another more preferred aspect, the yeast is *Candida utilis.* In another preferred aspect, the yeast is a *Clavispora.* In another more preferred aspect, the yeast is *Clavispora lusitaniae.* In another more preferred aspect, the yeast is *Clavispora opuntiae.* In another preferred aspect, the yeast is a *Pachysolen.* In another more preferred aspect, the yeast is *Pachysolen tannophilus.* In another preferred aspect, the yeast is a *Pichia.* In another more preferred aspect, the yeast is a *Pichia stipitis.* In another preferred aspect, the yeast is a *Bretannomyces.* In another more preferred aspect, the yeast is *Bretannomyces clausenii* (Philippidis, G. P., 1996, Cellulose bioconversion technology, in Handbook on Bioethanol: Production and Utilization, Wyman, C. E., ed., Taylor & Francis, Washington, DC, 179-212).

Bacteria that can efficiently ferment hexose and pentose to ethanol include, for example, *Zymomonas mobilis, Clostridium acetobutylicum, Clostridium thermocellum, Chlostridium phytofermentans, Geobacillus* sp., *Thermoanaerobacter saccharolyticum,* and *Bacillus coagulans* (Philippidis, 1996, *supra).*

In a preferred aspect, the bacterium is a *Zymomonas.* In a more preferred aspect, the bacterium is *Zymomonas mobilis.* In another preferred aspect, the bacterium is a *Clostridium.* In another more preferred aspect, the bacterium is *Clostridium thermocellum.*

Commercially available yeast suitable for ethanol production includes, e.g., ETHANOL RED™ yeast (available from Fermentis/Lesaffre, USA), FALI™ (available from Fleischmann's Yeast, USA), SUPERSTART™ and THERMOSACC™ fresh yeast (available from Ethanol Technology, WI, USA), BIOFERM™ AFT and XR (available from NABC - North American Bioproducts Corporation, GA, USA), GERT STRAND™ (available from Gert Strand AB, Sweden), and FERMIOL™ (available from DSM Specialties).

In a preferred aspect, the fermenting microorganism has been genetically modified to provide the ability to ferment pentose sugars, such as xylose utilizing, arabinose utilizing, and xylose and arabinose co-utilizing microorganisms.

The cloning of heterologous genes into various fermenting microorganisms has led to the construction of organisms capable of converting hexoses and pentoses to ethanol (cofermentation) (Chen and Ho, 1993, Cloning and improving the expression of Pichia stipitis xylose reductase gene in Saccharomyces cerevisiae, Appl. Biochem. Biotechnol. 39-40: 135-147; Ho et al., 1998, Genetically engineered Saccharomyces yeast capable of effectively cofermenting glucose and xylose, Appl. Environ. Microbiol. 64: 1852-1859; Kotter and Ciriacy, 1993, Xylose fermentation by Saccharomyces cerevisiae, Appl. Microbiol. Biotechnol. 38: 776-783; Walfridsson et al., 1995, Xylose-metabolizing Saccharomyces cerevisiae strains overexpressing the TKL1 and TAL1 genes encoding the pentose phosphate pathway enzymes transketolase and transaldolase, Appl. Environ. Microbiol. 61: 4184-4190; Kuyper et al., 2004, Minimal metabolic engineering of Saccharomyces cerevisiae for efficient anaerobic xylose fermentation: a proof of principle, FEMS Yeast Research 4: 655-664; Beall et al., 1991, Parametric studies of ethanol production from xylose and other sugars by recombinant Escherichia coli, Biotech. Bioeng. 38: 296-303; Ingram et al., 1998, Metabolic engineering of bacteria for ethanol production, Biotechnol. Bioeng. 58: 204-214; Zhang et al., 1995, Metabolic engineering of a pentose metabolism pathway in ethanologenic Zymomonas mobilis, Science 267: 240-243; Deanda et al., 1996, Development of an arabinose-fermenting Zymomonas mobilis strain by metabolic pathway engineering, Appl. Environ. Microbiol. 62: 4465-4470; WO 2003/062430, xylose isomerase).

In a preferred aspect, the genetically modified fermenting microorganism is *Saccharomyces cerevisiae.* In another preferred aspect, the genetically modified fermenting microorganism is *Zymomonas mobilis.* In another preferred aspect, the genetically modified fermenting microorganism is *Escherichia coli.* In another preferred aspect, the genetically modified fermenting microorganism is *Klebsiella oxytoca.* In another preferred aspect, the genetically modified fermenting microorganism is *Kluyveromyces* sp.

It is well known in the art that the organisms described above can also be used to produce other substances, as described herein.

The fermenting microorganism is typically added to the degraded lignocellulose or hydrolysate and the fermentation is performed for about 8 to about 96 hours, such as about 24 to about 60 hours. The temperature is typically between about 26°C to about 60°C, in particular about 32°C or 50°C, and at about pH 3 to about pH 8, such as around pH 4-5, 6, or 7.

In a preferred aspect, the yeast and/or another microorganism is applied to the degraded cellulosic material and the fermentation is performed for about 12 to about 96 hours, such as typically 24-60 hours. In a preferred aspect, the temperature is preferably between about 20°C to about 60°C, more preferably about 25°C to about 50°C, and most preferably about 32°C to about 50°C, in particular about 32°C or 50°C, and the pH is generally from about pH 3 to about pH 7, preferably around pH 4-7. However, some fermenting organisms, e.g., bacteria, have higher fermentation temperature optima. Yeast or another microorganism is preferably applied in amounts of approximately $10^5$ to $10^{12}$, preferably from approximately $10^7$ to $10^{10}$, especially approximately 2 x $10^8$ viable cell count per ml of fermentation broth. Further guidance in respect of using yeast for fermentation can be found in, e.g., "The Alcohol Textbook" (Editors K. Jacques, T.P. Lyons and D.R. Kelsall, Nottingham University Press, United Kingdom 1999).

For ethanol production, following the fermentation the fermented slurry is distilled to extract the ethanol. The ethanol obtained according to the methods of the invention can be used as, *e.g.,* fuel ethanol, drinking ethanol, *i.e.,* potable neutral spirits, or industrial ethanol.

A fermentation stimulator can be used in combination with any of the processes described herein to further improve the fermentation process, and in particular, the performance of the fermenting microorganism, such as, rate enhancement and ethanol yield. A "fermentation stimulator" refers to stimulators for growth of the fermenting microorganisms, in particular, yeast. Preferred fermentation stimulators for growth include vitamins and minerals. Examples of vitamins include multivitamins, biotin, pantothenate, nicotinic acid, meso-inositol, thiamine, pyridoxine, para-aminobenzoic acid, folic acid, riboflavin, and Vitamins A, B, C, D, and E. See, for example, Alfenore et al., Improving ethanol production and viability of Saccharomyces cerevisiae by a vitamin feeding strategy during fed-batch process, Springer-Verlag (2002). Examples of minerals include minerals and mineral salts that can supply nutrients comprising P, K, Mg, S, Ca, Fe, Zn, Mn, and Cu.

Fermentation products: A fermentation product can be any substance derived from the fermentation. The fermentation product can be, without limitation, an alcohol (*e.g.*, arabinitol, n-butanol, isobutanol, ethanol, glycerol, methanol, ethylene glycol, 1,3-propanediol [propylene glycol], butanediol, glycerin, sorbitol, and xylitol); an alkane (*e.g.*, pentane, hexane, heptane, octane, nonane, decane, undecane, and dodecane), a cycloalkane (*e.g.*, cyclopentane, cyclohexane, cycloheptane, and cyclooctane), an alkene (*e.g.* pentene, hexene, heptene, and octene); an amino acid (*e.g.*, aspartic acid, glutamic acid, glycine, lysine, serine, and threonine); a gas (*e.g.*, methane, hydrogen ($H_2$), carbon dioxide ($CO_2$), and carbon monoxide (CO)); isoprene; a ketone (*e.g.,* acetone); an organic acid (*e.g.,* acetic acid, acetonic acid, adipic acid, ascorbic acid, citric acid, 2,5-diketo-D-gluconic acid, formic acid, fumaric acid, glucaric acid, gluconic acid, glucuronic acid, glutaric acid, 3-hydroxypropionic acid, itaconic acid, lactic acid, malic acid, malonic acid, oxalic acid, oxaloacetic acid, propionic acid, succinic acid, and xylonic acid); and polyketide. The fermentation product can also be protein as a high value product.

In a preferred aspect, the fermentation product is an alcohol. It will be understood that the term "alcohol" encompasses a substance that contains one or more hydroxyl moieties. In a more preferred aspect, the alcohol is n-butanol. In another more preferred aspect, the alcohol is isobutanol. In another more preferred aspect, the alcohol is ethanol. In another more preferred aspect, the alcohol is methanol. In another more preferred aspect, the alcohol is arabinitol. In another more preferred aspect, the alcohol is butanediol. In another more preferred aspect, the alcohol is ethylene glycol. In another more preferred aspect, the alcohol is glycerin. In another more preferred aspect, the alcohol is glycerol. In another more preferred aspect, the alcohol is 1,3-propanediol. In another more preferred aspect, the alcohol is sorbitol. In another more preferred aspect, the alcohol is xylitol. See, for example, Gong, C. S., Cao, N. J., Du, J., and Tsao, G. T., 1999, Ethanol production from renewable resources, in Advances in Biochemical Engineering/Biotechnology, Scheper, T., ed., Springer-Verlag Berlin Heidelberg, Germany, 65: 207-241; Silveira, M. M., and Jonas, R., 2002, The biotechnological production of sorbitol, Appl. Microbiol. Biotechnol. 59: 400-408; Nigam, P., and Singh, D., 1995, Processes for fermentative production of xylitol - a sugar substitute, Process Biochemistry 30 (2): 117-124; Ezeji, T. C., Qureshi, N. and Blaschek, H. P., 2003, Production of acetone, butanol and ethanol by Clostridium beijerinckii BA101 and in situ recovery by gas stripping, World Journal of Microbiology and Biotechnology 19 (6): 595-603.

In another preferred aspect, the fermentation product is an alkane. The alkane can be an unbranched or a branched alkane. In another more preferred aspect, the alkane is pentane. In another more preferred aspect, the alkane is hexane. In another more preferred aspect, the alkane is heptane. In another more preferred aspect, the alkane is octane. In another more preferred aspect, the alkane is nonane. In another more preferred aspect, the alkane is decane. In another more preferred aspect, the alkane is undecane. In another more preferred aspect, the alkane is dodecane.

In another preferred aspect, the fermentation product is a cycloalkane. In another more preferred aspect, the cycloalkane is cyclopentane. In another more preferred aspect, the cycloalkane is cyclohexane. In another more preferred aspect, the cycloalkane is cycloheptane. In another more preferred aspect, the cycloalkane is cyclooctane.

In another preferred aspect, the fermentation product is an alkene. The alkene can be an unbranched or a branched alkene. In another more preferred aspect, the alkene is pentene. In another more preferred aspect, the alkene is hexene. In another more preferred aspect, the alkene is heptene. In another more preferred aspect, the alkene is octene.

In another preferred aspect, the fermentation product is an amino acid. In another more preferred aspect, the organic acid is aspartic acid. In another more preferred aspect, the amino acid is glutamic acid. In another more preferred aspect, the amino acid is glycine. In another more preferred aspect, the amino acid is lysine. In another more preferred aspect, the amino acid is serine. In another more preferred aspect, the amino acid is threonine. See, for example, Richard, A., and Margaritis, A., 2004, Empirical modeling of batch fermentation kinetics for poly(glutamic acid) production and other microbial biopolymers, Biotechnology and Bioengineering 87 (4): 501-515.

In another preferred aspect, the fermentation product is a gas. In another more preferred aspect, the gas is methane. In another more preferred aspect, the gas is $H_2$. In another more preferred aspect, the gas is $CO_2$. In another more preferred aspect, the gas is CO. See, for example, Kataoka, N., A. Miya, and K. Kiriyama, 1997, Studies on hydrogen production by continuous culture system of hydrogen-producing anaerobic bacteria, Water Science and Technology 36 (6-7): 41-47; and Gunaseelan V.N. in Biomass and Bioenergy, Vol. 13 (1-2), pp. 83-114, 1997, Anaerobic digestion of biomass for methane production: A review.

In another preferred aspect, the fermentation product is isoprene.

In another preferred aspect, the fermentation product is a ketone. It will be understood that the term "ketone" encompasses a substance that contains one or more ketone moieties. In another more preferred aspect, the ketone is acetone. See, for example, Qureshi and Blaschek, 2003, *supra.*

In another preferred aspect, the fermentation product is an organic acid. In another more preferred aspect, the organic acid is acetic acid. In another more preferred aspect, the organic acid is acetonic acid. In another more preferred aspect, the organic acid is adipic acid. In another more preferred aspect, the organic acid is ascorbic acid. In another more preferred aspect, the organic acid is citric acid. In another more preferred aspect, the organic acid is 2,5-diketo-D-gluconic acid. In another more preferred aspect, the organic acid is formic acid. In another more preferred aspect, the organic acid is fumaric acid. In another more preferred aspect, the organic acid is glucaric acid. In another more preferred aspect, the organic acid is gluconic acid. In another more preferred aspect, the organic acid is glucuronic acid. In another more preferred aspect, the organic acid is glutaric acid. In another preferred aspect, the organic acid is 3-hydroxypropionic acid. In another more preferred aspect, the organic acid is itaconic acid. In another more preferred aspect, the organic acid is lactic acid. In another more preferred aspect, the organic acid is malic acid. In another more preferred aspect, the organic acid is malonic acid. In another more preferred aspect, the organic acid is oxalic acid. In another more preferred aspect, the organic acid is propionic acid. In another more preferred aspect, the organic acid is succinic acid. In another more preferred aspect, the organic acid is xylonic acid. See, for example, Chen, R., and Lee, Y. Y., 1997, Membrane-mediated extractive fermentation for lactic acid production from cellulosic biomass, Appl. Biochem. Biotechnol. 63-65: 435-448.

In another preferred aspect, the fermentation product is polyketide.

Recovery. The fermentation product(s) can be optionally recovered from the fermentation medium using any method known in the art including, but not limited to, chromatography, electrophoretic procedures, differential solubility, distillation, or extraction. For example, alcohol is separated from the fermented cellulosic material and purified by conventional methods of distillation. Ethanol with a purity of up to about 96 vol.% can be obtained, which can be used as, for example, fuel ethanol, drinking ethanol, *i.e.,* potable neutral spirits, or industrial ethanol.

The present invention is further described by the following examples that should not be construed as limiting the scope of the invention.

## Examples

### Media

2X YT plates were composed of 16 g of tryptone, 10 g of yeast extract, 5 g of NaCl, 15 g of Noble agar, and deionized water to 1 liter.

PDA plates were composed of 39 grams of potato dextrose agar and deionized water to 1 liter.

MDU2BP medium was composed of 45 g of maltose, 1 g of $MgSO_4 \cdot 7H_2O$, 1 g of NaCl, 2 g of $K_2SO_4$, 12 g of $KH_2PO_4$, 7 g of yeast extract, 2 g of urea, 0.5 ml of AMG trace metals solution, and deionized water to 1 liter; pH 5.0.

AMG trace metals solution was composed of 14.3 g of $ZnSO_4 \cdot 7H_2O$, 2.5 g of $CuSO_4 \cdot 5H_2O$, 0.5 g of $NiCl_2 \cdot 6H_2O$, 13.8 g of $FeSO_4 \cdot 7H_2O$, 8.5 g of $MnSO_4 \cdot 7H_2O$, 3 g of citric acid, and deionized water to 1 liter.

**Example 1: Pretreatment of corn stover**

Corn stover was pretreated at the U.S. Department of Energy National Renewable Energy Laboratory (NREL) using 1.4% (w/v) sulfuric acid for 8 minutes at 165°C and 107 psi. The water-insoluble solids in the pretreated corn stover contained 57.5% cellulose, 4.6% hemicelluloses, and 28.4% lignin. Cellulose and hemicellulose were determined by a two-stage sulfuric acid hydrolysis with subsequent analysis of sugars by high performance liquid chromatography using NREL Standard Analytical Procedure #002. Lignin was determined gravimetrically after hydrolyzing the cellulose and hemicellulose fractions with sulfuric acid using NREL Standard Analytical Procedure #003.

The pretreated corn stover was adjusted to pH 5.0 by repeated addition of 10 N NaOH in aliquots of a few milliliters, followed by thorough mixing and incubation at room temperature for approximately 1 hour. The pH was confirmed after overnight incubation at 4°C, and the pH-adjusted corn stover was autoclaved for 20 minutes at approximately 120°C, and then stored at 4°C to minimize the risk of microbial contamination. The dry weight of the pretreated corn stover was 33% TS (total solids), which was confirmed before each use.

The pretreated corn stover was milled prior to use. Milled pretreated corn stover (initial dry weight 32.35% TS) was prepared by milling in a Cosmos ICMG 40 wet multi-utility grinder (EssEmm Corporation, Tamil Nadu, India). Milled pretreated corn stover was also, in some cases, subsequently washed repeatedly with deionized water followed by decanting off the supernatant fraction. The dry weight of the milled, water-washed pretreated corn stover was 7.114% TS.

Alternatively, milled, water-washed pretreated corn stover was washed extensively with water at 50°C. Approximately 600 ml of water washed pretreated corn stover was diluted with approximately 500 ml of distilled, deionized water and incubated at 50°C with shaking for 7 days. Three to four times per day, the diluted pretreated corn stover was permitted to settle, and the supernatant water was decanted and replaced with 500 ml of fresh deionized water. The dry weight of the milled, hot-water washed pretreated corn stover was 6.74% TS.

**Example 2: Separation of pretreated corn stover liquor**

Acid pretreated corn stover liquor was obtained by vacuum-filtration of the pH-adjusted NREL pretreated corn stover (Example 1) using Whatman #3 filter paper in a Buchner funnel, or through a 0.22 μm STERICUP® sterile vacuum-filter (Millipore, Bedford, MA, USA). For the Whatman-filtered liquor, the liquor was additionally sterile-filtered using a 0.22 μm STERICUP® sterile vacuum-filter to minimize the risk of microbial contamination.

In later experiments, pretreated corn stover liquor was obtained by squeezing acid-pretreated corn stover in the following manner. Approximately 20 kg of dilute acid pretreated corn stover was loaded into the cotton sheet lining of a SRL Water Press Model BP40-S/S (Zambelli Enotech, Camisano Vicentino, Italy). Municipal water pressure (approximately 35 psi) was applied for 20 minutes, and the resulting liquid pressed out was captured as liquor. This acidic liquor was stored at 4°C, and was subsequently pH-adjusted to 5.0 by addition of 10 N NaOH, and sterile-filtered using a 0.22 μm STERICUP® sterile vacuum-filter.

**Example 3: Hydrolysis of cellulose and assay for GH61 polypeptide enhancement thereof**

The hydrolysis of pretreated corn stover was conducted using 2.2 ml, 96-deep well plates (Axygen, Union City, CA, USA) containing a total reaction mass of 1 g. The hydrolysis was performed with 5% total solids of either washed milled pretreated corn stover, unwashed pretreated corn stover, equivalent to 28.75 or 14.75 mg of cellulose per ml, respectively, or with a concentration of microcrystalline cellulose (AVICEL®, EM Science, Gibbstown, NJ, USA) equivalent to 28.75 mg of cellulose per ml. Later hydrolysis reactions were performed with milled, washed or milled, unwashed pretreated corn stover with a cellulose content of 59%, equivalent to 29.5 mg of cellulose per ml, or an equivalent concentration of microcrystalline cellulose (AVICEL®, Sigma-Aldrich, St. Louis, MO, USA). Hydrolysis reactions were performed in 50 mM sodium acetate pH 5.0 containing 1 mM manganese sulfate using a *Trichoderma reesei* cellulase preparation (CELLUCLAST® supplemented with *Aspergillus oryzae* beta-glucosidase available from Novozymes A/S, Bagsvaerd, Denmark; the cellulase composition is designated herein in the Examples as "*Trichoderma reesei* cellulase composition") at 4 mg per g of cellulose. *Thermoascus aurantiacus* GH61A or *Thelavia terrestris* GH61E polypeptide having cellulolytic enhancing activity was added at concentrations between 0 and 50% (w/w) of total protein. Pretreated corn stover liquors, enzymatically- or chemically-treated corn stover liquors, synthetic mixtures containing mono- and disaccharides at equivalent concentrations to corn stover liquors, pretreated biomass component liquors, and post-fermentation residual liquors were added between 0 and 20% (v/v) as indicated. Plates were sealed using an ALPS-300™ plate heat sealer (Abgene, Epsom, United Kingdom) and incubated at 50°C for 0-168 hours with mixing at 150 rpm. All experiments were performed in duplicate or triplicate. Other hydrolysis reactions were performed similarly, with the following differences: plates were sealed using an ALPS-3000™ plate heat sealer (Abgene, Epsom, United Kingdom), and incubated at 50°C with vigorous initial mixing at each sampling time, but mixing was not continuous.

At various time points between 24 and 168 hours of incubation, 100 μl aliquots were removed and the extent of hydrolysis was assayed by high-performance liquid chromatography (HPLC) using the protocol described below.

For HPLC analysis, samples were filtered using a 0.45 μm MULTISCREEN® 96-well filter plate (Millipore, Bedford, MA, USA) and filtrates were analyzed for sugar content as described below. The sugar concentrations of samples diluted in 0.005 M $H_2SO_4$ were measured using a 4.6 x 250 mm AMINEX® HPX-87H column (Bio-Rad Laboratories, Inc., Hercules, CA, USA) by elution with 0.5% w/w benzoic acid-5 mM $H_2SO_4$ at a flow rate of 0.6 ml per minute at 65°C for 11 minutes, and quantification by integration of glucose and cellobiose signals from refractive index detection (CHEMSTATION®, AGILENT® 1100 HPLC, Agilent Technologies, Santa Clara, CA, USA) calibrated by pure sugar samples. The resultant equivalents were used to calculate the fraction or percentage of cellulose conversion for each reaction. The extent of each hydrolysis was determined as the fraction of total cellulose converted to cellobiose + glucose, and was not corrected for soluble sugars present in pretreated corn stover liquor, or was corrected for soluble sugars present in liquor as indicated.

All HPLC data processing was performed using KALEIDAGRAPH® software (Synergy software, Reading, PA, USA) or MICROSOFT EXCEL® (Microsoft, Seattle, WA, USA). Measured sugar concentrations were adjusted for the appropriate dilution factor. Glucose and cellobiose were chromatographically separated and integrated and their respective concentrations determined independently. To calculate fractional conversion the glucose and cellobiose values were combined. Fractional hydrolysis is reported as the ratio of the mass corrected concentrations of glucose and cellobiose to the initial concentration of cellulose as given by Equation 1. Triplicate data points were averaged and standard deviation was calculated.

$$\text{fractional hydrolysis} = \frac{(([\text{cellobiose}]\ (\text{mg/ml})\ \times 1.053)+([\text{glucose}](\text{mg/ml}))/1.111)}{[\text{cellulose}]\ (\text{mg/ml})} \qquad \text{(Equation 1)}$$

The concentration-dependence of GH61 polypeptide-dependent enhancement of cellulose hydrolysis by the *T. reesei* cellulase composition was determined by titration of the GH61 polypeptide between 0 and 50% (w/w) total protein added to a constant *T. reesei* cellulase concentration of 4 mg per g cellulose, plotting fractional hydrolysis against GH61 polypeptide concentration, and fitting using a modified saturation-binding model as given by Equation 2.

$$\text{fractional hydrolysis} = \frac{\Delta\text{fractional hydrolysis}\times[\text{GH61}] + \text{fractional hydrolysis}_{(0)}\left(K_{\frac{1}{2}\text{apparent}} + [\text{GH61}]\right)}{K_{\frac{1}{2}\text{apparent}} + [\text{GH61}]} \qquad \text{(Equation 2)}$$

In Equation 2 "fractional hydrolysis(o)" was the hydrolysis in the absence of a GH61 polypeptide; Δfractional hydrolysis was the total GH61 polypeptide-dependent enhancement; *i.e.,* the difference between the fractional hydrolysis at apparent "saturating" GH61 polypeptide concentration and the fractional hydrolysis in the absence of the GH61 polypeptide; and $K_{1/2\ apparent}$ was the GH61 polypeptide concentration necessary to observe a half-maximal enhancement of hydrolysis.

**Example 4: Preparation of *Thermoascus aurantiacus* GH61A polypeptide having cellulolytic enhancing activity**

*Thermoascus aurantiacus* GH61A polypeptide having cellulolytic enhancing activity (SEQ ID NO: 13 [DNA sequence] and SEQ ID NO: 14 [deduced amino acid sequence]) was recombinantly produced in *Aspergillus oryzae* JaL250 according to WO 2005/074656. The recombinantly produced *Thermoascus aurantiacus* GH61A polypeptide was first concentrated by ultrafiltration using a 10 kDa membrane, buffer exchanged into 20 mM Tris-HCl pH 8.0, and then purified using a 100 ml Q-SEPHAROSE® Big Beads column (GE Healthcare, Piscataway, NJ, USA) with a 600 ml 0-600 mM NaCl linear gradient in the same buffer. Fractions of 10 ml were collected and pooled based on SDS-PAGE.

The pooled fractions (90 ml) were then further purified using a 20 ml MONO Q® column (GE Healthcare, Piscataway, NJ, USA) with a 500 ml 0-500 mM NaCl linear gradient in the same buffer. Fractions of 6 ml were collected and pooled based on SDS-PAGE. The pooled fractions (24 ml) were concentrated by ultrafiltration using a 10 kDa membrane, and chromatographed using a 320 ml SUPERDEX® 200 SEC column (GE Healthcare, Piscataway, NJ, USA) with isocratic elution of approximately 1.3 liter of 150 mM NaCl-20 mM Tris-HCl pH 8.0. Fractions of 5 ml were collected and pooled based on SDS-PAGE. Protein concentration was determined using a Microplate BCA™ Protein Assay Kit (Thermo Fisher Scientific Inc., Rockford, IL, USA) in which bovine serum albumin was used as a protein standard.

**Example 5: Preparation of *Thielavia terrestris* GH61E polypeptide having cellulolytic enhancing activity**

*Thielavia terrestris* GH61E polypeptide having cellulolytic enhancing activity (SEQ ID NO: 7 [DNA sequence] and SEQ ID NO: 8 [deduced amino acid sequence]) was recombinantly produced in *Aspergillus oryzae* JaL250 according to U.S. Patent No. 7,361,495. The *Thielavia terrestris* GH61E polypeptide was desalted and buffer-exchanged into 20 mM sodium acetate-150 mM NaCl pH 5.0 using a HIPREP® 26/10 desalting column according to the manufacturer's instructions. Protein concentration was determined using a Microplate BCA™ Protein Assay Kit in which bovine serum albumin was used as a protein standard.

**Example 6: Preparation of *Aspergillus fumigatus* GH61B polypeptide having cellulolytic enhancing activity**

*Aspergillus fumigatus* GH61 B polypeptide having cellulolytic enhancing activity (SEQ ID NO: 29 [DNA sequence] and SEQ ID NO: 30 [deduced amino acid sequence]) was recombinantly produced using *Aspergillus oryzae* JaL355 as a host according to WO 2010/138754. The recombinantly produced *A. fumigatus* GH61B polypeptide was desalted and concentrated into 20 mM Tris pH 8.0 using a 10 kDa MWCO membrane and purified by size exclusion chromatography using SUPERDEX® S75 (GE Healthcare, Piscataway, NJ, USA). The purification buffer was 150 mM NaCl, 20 mM Tris 8.0. Homogeneity was confirmed by SDS-PAGE.

**Example 7: Preparation of *Penicillium pinophilum* GH61A polypeptide having cellulolytic enhancing activity**

*Penicillium pinophilum* GH61A polypeptide having cellulolytic enhancing activity (SEQ ID NO: 31 [DNA sequence] and SEQ ID NO: 32 [deduced amino acid sequence]) was recombinantly produced using *Aspergillus oryzae* HowB101 as a host according to WO 2011/005867. The recombinantly produced P. *pinophilum* GH61A polypeptide was desalted and concentrated into 20 mM Tris pH 8.0 using a 10 kDa MWCO membrane and purified by size exclusion chromatography using SUPERDEX® S75. The purification buffer was 150 mM NaCl, 20 mM Tris 8.0. Homogeneity was confirmed by SDS-PAGE.

**Example 8: Preparation of *Trichoderma reesei* CEL7B endoglucanase I**

*Trichoderma reesei* CEL7B endoglucanase I (EGI) (SEQ ID NO: 65 [DNA sequence] and SEQ ID NO: 66 [deduced amino acid sequence]) was cloned and expressed in *Aspergillus oryzae* JaL250 as described in WO 2005/067531. Filtered broth was concentrated and buffer exchanged using a tangential flow concentrator equipped with a 10 kDa polyethersulfone membrane with 20 mM Tris-HCl pH 8.5. The sample was loaded onto a Q SEPHAROSE® High Performance column (GE Healthcare, Piscataway, NJ, USA) equilibrated in 20 mM Tris pH 8.5, and bound proteins were eluted with a linear gradient from 0-600 mM sodium chloride. The fractions were concentrated and desalted into 20 mM Tris pH 8.0, 150 mM NaCl using VIVASPIN 20® 10 kDa MWCO centrifugal concentration devices (GE Healthcare UK limited, Little Chalfont, Buckinghamshire, UK). Protein concentration was determined using a Microplate BCA™ Protein Assay Kit in which bovine serum albumin was used as a protein standard.

**Example 9: Preparation of *Trichoderma reesei* CEL5A endoglucanase II**

The *Trichoderma reesei* RutC30 Cel5A endoglucanase II gene (SEQ ID NO: 67 [DNA sequence] and SEQ ID NO: 68 [deduced amino acid sequence]) was cloned and expressed in *Aspergillus oryzae* as described below.

Two synthetic oligonucleotide primers, shown below, were designed to PCR amplify the endoglucanase II gene from *Trichoderma reesei* RutC30 genomic DNA. Genomic DNA was isolated using a DNEASY® Plant Maxi Kit (QIAGEN Inc., Valencia, CA, USA). An IN-FUSION™ PCR Cloning Kit (BD Biosciences, Palo Alto, CA, USA) was used to clone the fragment directly into pAILo2 (WO 2004/099228).

Forward primer:
5'-ACTGGATTT**ACCATGAACAAGTCCGTGGCTCCATTGCT**-3' (SEQ ID NO: 125)
Reverse primer:
5'-TCACCTCTAGTTAATTAA**CTACTTTCTTGCGAGACACG**-3' (SEQ ID NO: 126)

Bold letters represent coding sequence. The remaining sequence contains sequence identity to insertion sites of pAILo2.

Fifty picomoles of each of the primers above were used in a PCR reaction containing 200 ng of *Trichoderma reesei* genomic DNA, 1X *Pfx* Amplification Buffer (Invitrogen, Carlsbad, CA, USA), 6 μl of 10 mM blend of dATP, dTTP, dGTP, and dCTP, 2.5 units of PLATINUM® *Pfx* DNA polymerase (Invitrogen Corp., Carlsbad, CA, USA), and 1 μl of 50 mM $MgSO_4$ in a final volume of 50 μl. The amplification reaction was incubated in an EPPENDORF® MASTERCYCLER® 5333 (Eppendorf Scientific, Inc., Westbury, NY, USA) programmed for one cycle at 98°C for 2 minutes; and 35 cycles each at 94°C for 30 seconds, 61°C for 30 seconds, and 68°C for 1.5 minutes. After the 35 cycles, the reaction was incubated at 68°C for 10 minutes and then cooled at 10°C. A 1.5 kb PCR reaction product was isolated on a 0.8% GTG® agarose gel (Cambrex Bioproducts, East Rutherford, NJ, USA) using 40 mM Tris base, 20 mM sodium acetate, 1 mM disodium EDTA (TAE) buffer and 0.1 μg of ethidium bromide per ml. The DNA band was visualized with the aid of a DARKREADER™ Transilluminator (Clare Chemical Research, Dolores, CO, USA). The 1.5 kb DNA band was excised with a disposable razor blade and purified using an ULTRAFREE® DA spin cup (Millipore, Billerica, MA, USA) according to the manufacturer's instructions.

Plasmid pAILo2 was linearized by digestion with Nco I and Pac I. The plasmid fragment was purified by gel electrophoresis and ultrafiltration as described above. Cloning of the purified PCR fragment into the linearized and purified pAILo2 vector was performed using an IN-FUSION™ PCR Cloning Kit. The reaction (20 μl) contained 1X IN-FUSION™ Buffer (BD Biosciences, Palo Alto, CA, USA), 1X BSA, 1 μl of IN-FUSION™ enzyme (diluted 1:10) (BD Biosciences, Palo Alto, CA, USA), 100 ng of pAILo2 digested with Nco I and Pac I, and 100 ng of the *Trichoderma reesei* Cel5A endoglucanase II PCR product. The reaction was incubated at room temperature for 30 minutes. A 2 μl sample of the reaction was used to transform *E. coli* XL10 SOLOPACK® Gold cells (Stratagene, La Jolla, CA, USA) according to the manufacturer's instructions. After a recovery period, two 100 μl aliquots from the transformation reaction were plated onto 150 mm 2X YT plates supplemented with 100 μg of ampicillin per ml. The plates were incubated overnight at 37°C. A set of 3 putative recombinant clones was recovered from the selection plates and plasmid DNA was prepared from each one using a BIOROBOT® 9600 (QIAGEN Inc., Valencia, CA, USA). Clones were analyzed by *Pci* I/*Bsp* LU11 I restriction digestion. One clone with the expected restriction digestion pattern was then sequenced to confirm that there were no mutations in the cloned insert. Clone #3 was selected and designated pAILo27.

*Aspergillus oryzae* JaL250 protoplasts were prepared according to the method of Christensen *et al.,* 1988, *supra.* Five micrograms of pAILo27 (as well as pAILo2 as a control) were used to transform *Aspergillus oryzae* JaL250 protoplasts. The transformation of *Aspergillus oryzae* JaL250 with pAILo27 yielded about 50 transformants. Eleven transformants were isolated to individual PDA plates and incubated for five days at 34°C.

Confluent spore plates were washed with 3 ml of 0.01% TWEEN® 80 (a nonionic surfactant and emulsifier derived from polyethoxylated sorbitan and oleic acid) and the spore suspension was used to inoculate 25 ml of MDU2BP medium in 125 ml glass shake flasks. Transformant cultures were incubated at 34°C with constant shaking at 200 rpm. At day five post-inoculation, cultures were centrifuged at 6000 x *g* and their supernatants collected. Five microliters of each supernatant were mixed with an equal volume of 2X loading buffer (10% beta-mercaptoethanol) and loaded onto a 1.5 mm 8%-16% Tris-glycine SDS-PAGE gel and stained with SIMPLYBLUE™ SafeStain (Invitrogen Corp., Carlsbad, CA, USA). SDS-PAGE profiles of the culture broths showed that ten out of eleven transformants produced a new protein band of approximately 45 kDa. Transformant number 1, designated *Aspergillus oryzae* JaL250AILo27, was cultivated in a fermentor.

One hundred ml of shake flask medium were added to a 500 ml shake flask. The shake flask medium was composed per liter of 50 g of sucrose, 10 g of $KH_2PO_4$, 0.5 g of CaCl2, 2 g of $MgSO_4.7H_2O$, 2 g of $K_2SO_4$, 2 g of urea, 10 g of yeast extract, 2 g of citric acid, and 0.5 ml of trace metals solution. The trace metals solution was composed per liter of 13.8 g of $FeSO_4{\cdot}7H_2O$, 14.3 g of $ZnSO_4{\cdot}7H_2O$, 8.5 g of $MnSO_4{\cdot}H_2O$, 2.5 g of $CuSO_4{\cdot}5H_2O$, and 3 g of citric acid. The shake flask was inoculated with two plugs of *Aspergillus oryzae* JaL250AILo27 from a PDA plate and incubated at 34°C on an orbital shaker at 200 rpm for 24 hours. Fifty ml of the shake flask broth was used to inoculate a 3 liter fermentation vessel.

A total of 1.8 liters of the fermentation batch medium was added to a three liter glass jacketed fermentor (Applikon Biotechnology, Schiedam, Netherlands). The fermentation batch medium was composed per liter of 10 g of yeast extract, 24 g of sucrose, 5 g of $(NH_4)_2SO_4$, 2 g of $KH_2PO_4$, 0.5 g of $CaCl_2{\cdot}2H_2O$, 2 g of $MgSO_4{\cdot}7H_2O$, 1 g of citric acid, 2 g of $K_2SO_4$, 0.5 ml of anti-foam, and 0.5 ml of trace metals solution. The trace metals solution was composed per liter of 13.8 g of $FeSO_4{\cdot}7H_2O$, 14.3 g of $ZnSO_4{\cdot}7H_2O$, 8.5 g of $MnSO_4{\cdot}H_2O$, 2.5 g of $CuSO_4{\cdot}5H_2O$, and 3 g of citric acid. Fermentation feed medium was composed of maltose, which was. dosed at a rate of 0 to 4.4 g/l/hr for a period of 185 hours. The fermentation vessel was maintained at a temperature of 34°C and pH was controlled using an Applikon 1030 control system (Applikon Biotechnology, Schiedam, Netherlands) to a set-point of 6.1 +/- 0.1. Air was added to the vessel at a rate of 1 vvm and the broth was agitated by a Rushton impeller rotating at 1100 to 1300 rpm. At the end of the fermentation, whole broth was harvested from the vessel and centrifuged at 3000 x *g* to remove the biomass. The supernatant was sterile filtered and stored at 5 to 10°C.

The supernatant was desalted and buffer-exchanged into 20 mM Bis-Tris pH 6.0 using a HIPREP® 26/10 desalting column (GE Healthcare, Piscataway, NJ, USA) according to the manufacturer's instructions. The buffer exchanged sample was loaded onto a MonoQ® column (GE Healthcare, Piscataway, NJ, USA) equilibrated with 20 mM Bis-Tris pH 6.0, and the bound protein was eluted with a linear gradient from 0 to 1000 mM sodium chloride. Protein fractions were pooled and buffer exchanged into 1.2 M $(NH_4)_2SO_4$-20 mM Tris-HCl pH 8.5. The sample was loaded onto a Phenyl SUPEROSE™ column (HR 16/10) equilibrated with 1.2 M $(NH_4)_2SO_4$-20 mM Tris-HCl pH 8.0. Bound proteins were eluted with a linear gradient over 20 column volumes from 1.2 to 0 M $(NH_4)_2SO_4$ in 20 mM Tris-HCl pH 8.5. The fractions were pooled, concentrated, and loaded onto a SUPERDEX® 75 HR 26/60 column (GE Healthcare, Piscataway, NJ, USA) equilibrated with 20 mM Tris-150 mM sodium chloride pH 8.5. Fractions were pooled and concentrated in 20 mM Tris-150 mM sodium chloride pH 8.5. Protein concentration was determined using a Microplate BCA™ Protein Assay Kit in which bovine serum albumin was used as a protein standard.

**Example 10: Preparation of *Trichoderma reesei* CEL7A cellobiohydrolase I**

*Trichoderma reesei* CEL7A cellobiohydrolase I (SEQ ID NO: 95 [DNA sequence] and SEQ ID NO: 96 [deduced amino acid sequence]) was prepared as described by Ding and Xu, 2004, "Productive cellulase adsorption on cellulose" in Lignocellulose Biodegradation (Saha, B. C. ed.), Symposium Series 889, pp.154-169, American Chemical Society, Washington, DC. Protein concentration was determined using a Microplate BCA™ Protein Assay Kit in which bovine serum albumin was used as a protein standard.

**Example 11: Preparation of *Trichoderma reesei* CEL6A cellobiohydrolase II**

The *Trichoderma reesei* RutC30 CEL6A cellobiohydrolase II gene (SEQ ID NO: 97 [DNA sequence] and SEQ ID NO: 98 [deduced amino acid sequence]) was isolated from *Trichoderma reesei* RutC30 as described in WO 2005/056772. The *Trichoderma reesei* CEL6A cellobiohydrolase II gene was expressed in *Fusarium venenatum* using pEJG61 as an expression vector according to the procedures described in U.S. Published Application No. 20060156437. Fermentation was performed as described in U.S. Published Application No. 20060156437. Filtered broth was desalted and buffer-exchanged into 20 mM sodium acetate-150 mM NaCl pH 5.0 using a HIPREP® 26/10 Desalting Column according to the manufacturer's instructions. Protein concentration was determined using a Microplate BCA™ Protein Assay Kit in which bovine serum albumin was used as a protein standard.

**Example 12: Preparation of *Aspergillus oryzae* CEL3A beta-glucosidase**

*Aspergillus oryzae* CEL3A beta-glucosidase (SEQ ID NO: 111 [DNA sequence] and SEQ ID NO: 112 [deduced amino acid sequence]) was recombinantly prepared as described in WO 2004/099228, and purified as described by Langston et al., 2006, Biochim. Biophys. Acta Proteins Proteomics 1764: 972-978. Protein concentration was determined using a Microplate BCA™ Protein Assay Kit.

**Example 13: Effect of GH61 polypeptides having cellulolytic enhancing activity on hydrolysis of microcrystalline cellulose or PCS by the *Trichoderma reesei* cellulase composition**

The effect of the *Thermoascus aurantiacus* GH61A polypeptide on the hydrolysis of AVICEL® or milled washed PCS by the *Trichoderma reesei* cellulase composition was determined using the same experimental conditions and procedures according to Example 3. In general, in experiments performed in subsequent examples, a control reaction was included in which increasing concentrations of GH61 polypeptide were added to the hydrolysis of either AVICEL® or pretreated corn stover with the *T. reesei* cellulase composition in the absence of other liquors, compounds, or variously treated liquors.

The presence of the *T. aurantiacus* GH61A polypeptide did not enhance the hydrolysis of AVICEL® by the *T. reesei* cellulase composition. Conversion of AVICEL® was 0.119 ± 0.00251 and 0.339 ± 0.00222 at 1 and 3 days, respectively, in the absence of the *T. aurantiacus* GH61A polypeptide compared to 0.112 ± 0.00376 and 0.333 ± 0.00328 at 1 and 3, respectively, in the presence of 24% (w/w) of the *T. aurantiacus* GH61A polypeptide.

The presence of the *T. aurantiacus* GH61A polypeptide enhanced the hydrolysis of milled washed PCS by the *T. reesei* cellulase composition. Conversion of milled washed PCS was 0.249 ± 0.00104 and 0.545 ± 0.00656 at 1 and 3 days, respectively, in the presence of the *T. aurantiacus* GH61A polypeptide compared to 0.222 ± 0.00464 and 0.412 ± 0.0237 at 1 and 3 days, respectively, in the absence of the *T. aurantiacus* GH61A polypeptide.

The presence of the *T. aurantiacus* GH61A polypeptide marginally enhanced the hydrolysis of milled hot-washed PCS by the *T. reesei* cellulase composition. Conversion of hot washed PCS was 0.315 ± 0.00267 and 0.383 ± 0.00498, at 1 and 3 days, respectively, in the absence of the *T. aurantiacus* GH61A polypeptide compared to 0.331 ± 0.0115 and 0.409 ± 0.0145 at 1 and 3, respectively, in the presence of 8% (w/w) of the *T. aurantiacus* GH61A polypeptide.

The presence of the *Thelavia terrestris* GH61E polypeptide did not enhance the hydrolysis of AVICEL® by the *T. reesei* cellulase composition. Conversion of AVICEL® was 0.122 ± 0.00426, 0.242 ± 0.00813 and 0.315 ± 0.00814, at 1, 3, and 5 days, respectively, in the absence of the *T. terrestris* GH61E polypeptide compared with 0.121 ± 0.000824, 0.228 ± 0.000978 and 0.307 ± 0.00348 at 1, 3, and 5 days respectively, in the presence of 24% (w/w) of the *T. terrestris* GH61E polypeptide.

The presence of the *Aspergillus fumigatus* GH61B polypeptide did not significantly enhance the hydrolysis of AVICEL® by the *T. reesei* cellulase composition. Conversion of AVICEL® was 0.150 ± 0.009, 0.31 ± 0.001, and 0.48 ± 0.001 at 1, 3, and 7 days, respectively, in the absence of the *A. fumigatus* GH61B polypeptide compared to 0.148 ± 0.002%, 0.311 ± 0.001 %, and 0.54 ± 0.02% at 1, 3, and 7 days, respectively, in the presence of the *A. fumigatus* GH61B polypeptide.

**Example 14: Effect of *Thermoascus aurantiacus* GH61A polypeptide on cellulolysis of unwashed and washed acid-pretreated corn stover**

The effect of *Thermoascus aurantiacus* GH61A polypeptide on hydrolysis of both milled washed and milled unwashed NREL pretreated corn stover by the *Trichoderma reesei* cellulase composition were assayed for comparison. To an equivalent, fixed concentration of the *T. reesei* cellulase composition at 4 mg per gram of cellulose, increasing concentrations of *T. aurantiacus* GH61A polypeptide between 0 and 24% (w/w) were added to equivalent dry masses of milled unwashed pretreated corn stover or milled washed pretreated corn stover, and the fractional hydrolysis was assayed according to Example 3.

Figure 1 shows the fractional hydrolysis of variously washed pretreated corn stover substrates. The total enhancement in total conversion from the *T. aurantiacus* GH61A polypeptide was larger in magnitude and was apparent at earlier stages of hydrolysis for unwashed corn stover in comparison to washed pretreated corn stover. Over the range of concentrations tested, there was no GH61 polypeptide concentration-dependence of hydrolysis after 1 day of hydrolysis for washed milled pretreated corn stover (open squares), whereas a slight but significant increase of hydrolysis as GH61 polypeptide concentration increased was observed for unwashed milled corn stover (open circles). After 3 days of hydrolysis, washed pretreated corn stover showed a sharp, saturable enhancement of hydrolysis with GH61 polypeptide addition, well-fitted by a square hyperbolic binding function (closed squares). Conversely, there was a linear increase in hydrolysis with GH61 polypeptide concentration for the unwashed pretreated corn stover that extrapolated to a higher overall conversion at GH61 polypeptide concentrations greater than 50% (w/w) (closed circles). For hot-water washed pretreated corn stover prepared according to Example 1, this trend was even more apparent; after 3 days of hydrolysis, no GH61 polypeptide enhancement was observed (open triangles), and GH61 polypeptide-dependent enhancement of cellulolysis was not apparent until 5 days of hydrolysis (closed triangles). The total magnitude of GH61 polypeptide-dependent enhancement for hot-water washed pretreated corn stover was only 5% at 7 days, whereas for the GH61 polypeptide enhancement for unwashed pretreated corn stover was at least 15% at 7 days of hydrolysis (data not shown). In each case, the conversion by the *T. reesei* cellulase composition in the absence of the GH61 polypeptide (y-intercept) was higher for the more extensively washed substrates, indicating the removal of soluble cellulase inhibitors by washing.

**Example 15: The effect of addition of acid-pretreated corn stover liquor to washed milled pretreated corn stover**

Acid-pretreated corn stover liquor, fractionated according to Example 2, was added at concentrations between 0 and 15% (v/v) to milled, water-washed acid-pretreated corn stover and hydrolyzed by 4 mg of the *Trichoderma reesei* cellulase composition per g cellulose plus increasing concentrations of the *Thermoascus aurantiacus* GH61A polypeptide according to Example 3.

Figure 2A shows the extent of hydrolysis for the various additions of the *T. aurantiacus* GH61A polypeptide and NREL acid-pretreated corn stover liquor to milled, water-washed acid-pretreated corn stover at 1 day (white bars) and 3 days (gray bars) of hydrolysis.

Figure 2B shows a replot of the data presented in Figure 2A with non-linear least square fits to Equation 2 according to Example 3 or with linear least square fits. The Figure demonstrates that the functional dependence of the extent of hydrolysis was square hyperbolic, *i.e.,* saturating, for concentrations of liquor <10% (v/v), and became linear or exponential at concentrations of liquor ≥10% (v/v) at 3 days of hydrolysis. The extent of hydrolysis at the highest added concentration of the *T. aurantiacus* GH61A polypeptide was greater than that observed in the absence of liquor, and extrapolation of the trends to predict fractional hydrolysis levels at higher GH61 polypeptide concentrations indicated a greater conversion at higher liquor concentrations. From fits of Equation 2, the total enhancement from the GH61 polypeptide, Δfractional hydrolysis, was 0.150±0.000550 in the absence of added liquor, 0.166±0.0155 with 2% liquor, 0.227±0.0827 with 5% liquor, and 0.417±0.240 with 10% liquor, and the increase was linear with GH61 polypeptide concentration at 15% liquor.

**Example 16: Effect of addition of acid-pretreated corn stover liquor on *Thermoascus aurantiacus* GH61A polypeptide during hydrolysis of microcrystalline cellulose**

Pretreated corn stover liquor, fractionated according to Example 2, was added to a saccharification reaction of microcrystalline cellulose by the *Trichoderma reesei* cellulase composition according to Example 3 at concentrations between 0 and 20% (v/v). The *Thermoascus aurantiacus* GH61A polypeptide was titrated between 0 and 24% (w/w) of total protein.

Figure 3 shows that the *T. aurantiacus* GH61A polypeptide did not enhance on hydrolysis of microcrystalline cellulose by the *T. reesei* cellulase composition in the absence of liquor. However, in the presence of NREL pretreated corn stover liquor, the *T. aurantiacus* GH61A polypeptide enhanced cellulolysis. Figure 3A shows fractional hydrolysis at various GH61 polypeptide concentrations for increasing concentrations of NREL pretreated corn stover liquor at 1 day (open circles) and 3 days of hydrolysis (closed circles). As pretreated corn stover liquor was added from 0% v/v (circles) to 5% (diamonds), 10% (triangles), and 15% (inverted triangles), in the absence of the *T. aurantiacus* GH61A polypeptide, there was increasing inhibition of the *T. reesei* cellulase composition, as was apparent from the reduction in fractional hydrolysis. As the concentration of the *T. aurantiacus* GH61A polypeptide was increased, for those samples containing acid-pretreated corn stover liquor, the extent of hydrolysis increased to an extent beyond the level observed for hydrolysis in the absence of liquor. These data were corrected for saccharides present in the added liquor, and the possibility that either the *T. aurantiacus* GH61A polypeptide or the *T. reesei* cellulase composition in combination with the *T. aurantiacus* GH61A polypeptide were converting some substrate in pretreated corn stover liquor to glucose was unlikely, as the extent of enhancement from the *T. aurantiacus* GH61A polypeptide at 5% liquor addition would correspond to a conversion of 55 g/L glucose equivalents from the added liquor.

Figure 3B shows the results of addition of a synthetic mixture of the major sugar components of pretreated corn stover liquor; glucose, cellobiose, xylose, and arabinose with or without added phenol to mimic the phenolic lignin degradation compounds present in pretreated corn stover liquor, at the concentrations present in actual liquor. The synthetic sugar mixture was added between 0% and 15%, (symbols as in Figure 3A), and the synthetic sugar mixture containing phenol was added at either 5% (squares) or 15% (right triangles). For this example, added liquor sugar concentrations were not subtracted from the overall apparent hydrolysis, as was evident from the upward shift in apparent hydrolysis with increasing liquor addition. From Figure 3B, fractional hydrolysis was independent of GH61 polypeptide concentration, thus there was no apparent GH61 polypeptide cellulolytic enhancing activity in the presence of the synthetic liquor mixtures on microcrystalline cellulose. These data indicated that the GH61 polypeptide cellulolytic enhancing activity derives from the minor components of NREL pretreated corn stover liquor.

### Example 17: Effect of preconditioning acid-pretreated corn stover liquor with *Thelavia terrestris* GH61E polypeptide

Pretreated corn stover liquor, extracted according to Example 2, was added to a saccharification reaction of microcrystalline cellulose by the *Trichoderma reesei* cellulase composition according to Example 3 at concentrations between 0 and 20% (v/v). Alternatively, 10 ml of liquor were incubated at 50°C overnight with 0.43 mg protein of the *T. reesei* cellulase composition per ml, or with 20 μg protein of the *Thielavia terrestris* GH61E polypeptide per ml, or both (pre-conditioned liquor). The enzyme was removed from these samples using a 3 kDa MWCO AMICON® centrifuge filter (Millipore, Bedford, MA, USA), and the filtered flow-through liquor was added to saccharifications of microcrystalline cellulose at concentrations between 0 and 20% (v/v). The *T. terrestris* GH61E polypeptide was titrated between concentrations of 0 and 24% (w/w) of total protein.

Figure 4 shows that, like the *T. aurantiacus* GH61A polypeptide demonstrated in Example 11, the *Thelavia terrestris* GH61E polypeptide had cellulolytic enhancing activity on microcrystalline cellulose in the presence of NREL pretreated corn stover liquor and did not enhance cellulolysis in the absence of liquor. Figure 4 (all panels, circles) shows that increasing *T. terrestris* GH61E polypeptide concentration on microcrystalline cellulose in the absence of liquor did not enhance hydrolysis. As liquor was added from 0% (v/v) (circles) to 5% (squares), 10% (diamonds), and 15% (triangles) in the absence of the *T. terrestris* GH61E polypeptide, there was increasing inhibition of the *T. reesei* cellulase composition, as was apparent from the reduction in fractional hydrolysis (all panels, y-intercepts). As the *T. terrestris* GH61E polypeptide concentration was increased, for those samples containing either liquor or enzymatically pre-conditioned liquor, the extent of hydrolysis increased. In most cases, despite the inhibition of cellulolysis arising from the liquor addition, the increase in hydrolysis led to an extent of saccharification beyond the level observed for hydrolysis in the absence of liquor at high *Thelavia terrestris* GH61E polypeptide concentrations. Figure 4A shows the effects of liquor that had not been enzymatically treated, Figure 4B shows the effects of liquor that had been saccharified using the *T. reesei* cellulase composition, Figure 4C shows the effects of liquor that had been incubated with both the *T. reesei* cellulase composition and the *T. terrestris* GH61E polypeptide. In each case, GH61 polypeptide cellulolytic enhancing activity was apparent in the presence of pretreated corn stover liquor. Control reactions containing identical concentrations of liquor or pretreated liquor and enzyme in identical concentrations to those present in the cellulose-containing samples were performed in parallel, and sugars produced from these control reactions were subtracted from the saccharification totals. In each case the conversion of pretreated corn stover liquor to glucose equivalents produced minimal total sugar, and far less than was necessary to account for the large cellulolytic enhancement observed in saccharifications containing both the *Thielavia terrestris* GH61E polypeptide and liquor.

**Example 18: Effect of acid-pretreated corn stover liquor or steam-pretreated corn stover liquor on *Thelavia terrestris* GH61E polypeptide activity**

Pretreated corn stover liquor was extracted according to Example 2 with the following exceptions: 0.6 kg of steam explosion-pretreated corn stover or NREL acid-pretreated corn stover were suspended in 900 ml of deionized water and mixed for 2 hours. The solids were filtered using filter paper and sterile filtered with a 0.45 μm filter. Two hundred ml of each was ultracentrifuged using a 44.5 mM diameter 1 kDa MWCO centrifuge filter (Millipore, Bedford, MA, USA). The concentrated retentate was restored to the original volume by addition of water. The original liquors and the molecular weight separated fractions of each were added to saccharification reactions of microcrystalline cellulose with the *Trichoderma reesei* cellulase composition according to Example 3 at concentrations of 5% and 15% (v/v). The *Thelavia terrestris* GH61E polypeptide was titrated at concentrations between 0 and 24% (w/w) total protein.

Figure 5A shows that increasing concentrations of the *Thelavia terrestris* GH61E polypeptide increased the hydrolysis of microcrystalline cellulose by the *T. reesei* cellulase composition in the presence of the acid-pretreated corn stover liquor. Thus in the presence of the acid-pretreated corn stover liquor, the *T. terrestris* GH61E polypeptide significantly enhanced cellulolysis. Figure 5B shows that increasing concentrations of the *Thelavia terrestris* GH61E polypeptide marginally increased hydrolysis in the presence of steam explosion-pretreated corn stover, thus demonstrating minimal enhancement of cellulolysis by *Thelavia terrestris* GH61E under similar conditions.

Overall, the results indicated that conditions of pretreatment are critical for the production of liquor components necessary for GH61 cellulolytic enhancing activity on microcrystalline cellulose. More severe pretreatments, exemplified herein by the acid-pretreated corn stover (*e.g.*, NREL PCS), generate soluble components or higher concentrations of the soluble components than do milder pretreatments, as illustrated by steam-pretreated corn stover above.

**Example 19: Separation of GH61 polypeptide-enhancing liquor components from non-enhancing components and inhibitory components in NREL pretreated corn stover liquor**

Pretreated corn stover liquor was extracted and separated by ultrafiltration into nominal molecular weight fractions above and below 1 kDa according to Example 18. The molecular weight separated fractions were added to a saccharification reaction of microcrystalline cellulose with the *T. reesei* cellulase composition at concentrations between 0 and 15% (v/v) according to Example 3. The *Thelavia terrestris* GH61E polypeptide was titrated at concentrations between 0 and 24% (w/w) total protein.

As shown in Figure 5A, at zero *T. terrestris* GH61E polypeptide concentration, both the whole liquor and the lower molecular weight fractions showed inhibition at higher liquor concentrations (circles, squares). The inhibition from liquor has been discussed previously in Examples 10, 12 and 13. Conversely, there was no difference in hydrolysis at either 5 or 15% (v/v) of the higher molecular weight fraction (triangles). Increasing concentrations of the *T. terrestris* GH61E polypeptide with all these liquor molecular weight fractions increased cellulose hydrolysis. The unfractionated pretreated corn stover liquor yielded the highest GH61 polypeptide-dependent enhancement, however the high molecular weight pretreated corn stover liquor fraction also yielded substantial GH61 polypeptide-dependent enhancement.

The efficacy of molecular weight-based filtration was confirmed by fractionation of PCS liquor using AMICON® 30, 10, and 3 kDa MWCO centrifuge filters (Millipore, Bedford, MA, USA) and assaying for GH61 cellulolytic enhancing activity. NREL acid-pretreated corn stover liquor was extracted according to Example 2, and then filtered through successively smaller MWCO filters. The retentates were repeatedly washed with 3 to 5 volumes of water. The retentates for each molecular weight filter were assayed for GH61 cellulolytic enhancing activity according to Example 3 with the following exceptions: 5% (v/v) of each retentate was added to saccharification reactions of microcrystalline cellulose (AVICEL®), and the extent of hydrolysis was determined at 1 and 6 days of saccharification. Figure 6 shows the fractional hydrolysis of AVICEL® by the *T. reesei* cellulase composition with added *T. aurantiacus* GH61A polypeptide as indicated, in the presence of the filter rententates indicated. Figure 6 shows that the GH61 polypeptide-enhancing factor was not retained, or was only marginally retained by a 3 kDa filter. Increasing concentrations of GH61 polypeptide increased the hydrolysis of AVICEL® in the presence of flow-through from the 3 kDa MWCO filter, whereas a decrease, or no change in hydrolysis with GH61 polypeptide concentration was observed when incubated in the presence of retentates from molecular weight filters of 3 kDa and greater. These data, combined with the data presented in Figure 5A, indicated that the nominal molecular weight of the compounds facilitating GH61 polypeptide cellulolytic enhancing activity may lie between 1 and 3 kDa, and that molecular weight separation of the NREL acid-pretreated corn stover liquor can separate enhancing from inhibitory factors.

**Example 20: Effect of *Thermoascus aurantiacus* GH61 polypeptide and pretreated corn stover liquor enhances cellulolytic activity of individual cellulases on AVICEL®**

Individual monocomponent cellulases Cel5A endoglucanase II, Cel6A cellobiohydrolase, Cel7A cellobiohydrolase, and Cel7B endoglucanase I from *Trichoderma reesei* and beta-glucosidase from *Aspergillus oryzae* were assayed for enhancement of their ability to hydrolyze microcrystalline cellulose when incubated with the *Thermoascus aurantiacus* GH61A polypeptide with or without 5% NREL acid-pretreated corn stover liquor. AVICEL® hydrolyses were performed according to Example 3, with the following exceptions. Instead of the *Trichoderma reesei* cellulase composition, purified *T. reesei* monocomponents and purified *Aspergillus oryzae* beta-glucosidase were used at a concentration of 10 mg of enzyme protein per g cellulose. Alternatively, mixtures of monocomponent cellulases were used, with each cellulase dosed at 10 mg of enzyme protein per g cellulose. Saccharification reactions were performed with or without 1 mg of the *T. aurantiacus* GH61A polypeptide per g cellulose and with or without 5% NREL pretreated corn stover liquor at 50°C for 7 days.

Figure 7A shows the fractional hydrolysis achieved by each monocomponent and monocomponent mixture at 3, 5, and 7 days of hydrolysis, with and without the *T. aurantiacus* GH61A polypeptide in the presence of acid-pretreated corn stover liquor. In each case, in the absence of liquor, no GH61 polypeptide-dependent enhancement was observed (data not shown). Conversely, when acid-pretreated corn stover liquor was present, the *T. aurantiacus* GH61A polypeptide enhanced the cellulase activity of each monocomponent or monocomponent mixture by 5% or more at 7 days of hydrolysis.

Figure 7B shows the enhancement of cellulase activity for each monocomponent arising from the *T. aurantiacus* GH61A polypeptide in the presence of NREL acid-pretreated corn stover liquor at 3, 5, and 7 days of hydrolysis, which is given by the ratio of fractional hydrolysis in the presence to the absence of the GH61 polypeptide:

$$GH61\ effect = \frac{\%\ \text{conversion}_{(+\ \text{GH61}+\ \text{liquor})}}{\%\ \text{conversion}_{(\text{no GH61}+\ \text{liquor})}} \qquad \text{(Equation 3)}$$

Enhancement of hydrolysis by the GH61 polypeptide yields a ratio >1; inhibition of hydrolysis yields a ratio <1, and no effect on hydrolysis yields a ratio = 1

In each case at 5 and 7 days of hydrolysis, every monocomponent and every monocomponent mixture had enhanced cellulolytic activity, indicated by a *GH61 effect* > 1. The mixtures containing 3 to 5 cellulase components had the highest overall cellulase concentration in the reactions, thus had the highest fractional hydrolysis, and the lowest apparent *GH61 effect.* Despite this, the *T. aurantiacus* GH61A polypeptide still provided an enhancement of 1.05 ± 0.00604 for the 5-component cellulose mixture (Cel5A endoglucanase II, Cel6A cellobiohydrolase, Cel7A cellobiohydrolase, and Cel7B endoglucanase I from *T. reesei* and beta-glucosidase from *Aspergillus oryzae*) at 7 days of hydrolysis. The largest relative enhancements by the *T. aurantiacus* GH61A polypeptide to AVICEL® saccharification by cellulose monocomponents or mixtures thereof were observed for GH61 polypeptide enhancement of *T. reesei* Cel7A cellobiohydrolase (1.33 ± 0.127), or mixtures containing the *T. reesei* Cel7A cellobiohydrolase and *T. reesei* Cel5A endoglucanase II (1.33 ± 0.00719).

**Example 21: Enrichment of NREL acid-pretreated corn stover liquor components**

NREL acid-pretreated corn stover liquor was fractionated by adsorption to microcrystalline cellulose as described below. NREL acid-pretreated corn stover liquor (1.2 liters) was incubated with 10 g of AVICEL® overnight at room temperature. The supernatant fraction was removed by vacuum filtration through Whatman #3 filter paper. The AVICEL® and adsorbed liquor components were washed 6 times with 500 ml of acetonitrile (occasionally acetone was used), followed by elution with 2 liters of water in 300-400 ml fractions. The liquor components that in combination with a GH61 polypeptide demonstrated a GH61 polypeptide-dependent enhancement of cellulolysis by the *Trichoderma reesei* cellulase composition were largely eluted in the first 2 water elution fractions (Figure 8B), though later elution fractions contained some small amount of residual liquor. Water-eluted liquor components were concentrated approximately 10-fold using a Macrosep 1kD Omega centrifuge filter (Pall Corporation, East Hills, NY, USA) and applied to an 8.0 mm x 300 mm Shodex Sugar SP0810 HPLC chromatography column (Showa Denka America, Inc., NY, USA) using an AGILENT® 1100 HPLC and CHEMSTATION® software (Agilent Technologies, Santa Clara, CA, USA) and separated by isocratic elution with water at a flow rate of 0.5 ml per minute at 80°C for 50 minutes, and collecting 250 μl fractions in 2.2 ml, 96-deep well plates (Axygen, Union City, CA, USA). Repeated 50 μl injections of the liquor followed by repeated fraction collection yielded approximately 8 ml of each fraction. Peaks were identified by diode array detection at 210, 280 and 340 nm using a CHEMSTATION®, AGILENT® 1100 HPLC (Agilent Technologies, Santa Clara, CA, USA) (Figure 8, dashed lines).

Peak fractions were assayed according to Example 3 with the following exceptions. 400 μl of each fraction

were added to the hydrolysis reactions containing 4 mg of the *T. reesei* cellulase composition and 1 mg of *Thermoascus aurantiacus* GH61A polypeptide. Aliquots were removed and analyzed for sugar content at 1, 3, and 10 days of saccharification. Control reactions containing the original NREL acid-pretreated corn stover liquor and control reactions containing no liquor and no GH61 polypeptide were run in parallel. Figure 8B shows that addition of the various fractions resulted in fluctuation between fractions around a mean fractional hydrolysis value of 0.617 (Figure 8, solid lines). Several peaks of activity consisting of multiple sequential fractions with high cellulolytic activity in the presence of the *T. aurantiacus* GH61A polypeptide were observed, including one broad peak of activity centered at fraction 7C. This peak corresponded with a peak in $A_{(280)}$. The extent of the cellulolytic enhancement by the fraction appeared small, indicating that the amount fractionated was very small. Absorbance at 280 nm was consistent with contents of the fractions possessing aromatic characteristics.

**Example 22: Effect of NREL pretreated corn stover liquor fractions and the *Thermoascus aurantiacus* GH61A polypeptide on hydrolysis of microcrystalline cellulose by the *Trichoderma reesei* cellulase composition**

Pooled, lyophilized fractions of the NREL acid-pretreated corn stover liquor were each added to approximately 25 mg per ml of AVICEL® in 700 µl of 50 mM sodium acetate pH 5.0 in the presence of 3 mM calcium chloride with 37.5 µg of the *Thermoascus aurantiacus* GH61A polypeptide per ml and were incubated in 1.7 ml microcentrifuge tubes at 50°C with shaking at 1500 rpm for 48 hours in a Thermomixer (Eppendorf, Hamburg, Germany). Following the incubation, the samples were centrifuged at 31,000 rpm in a microcentrifuge for 5 minutes. A series of AVICEL® masses were weighed out and suspended in 700 µl of equivalent buffer, incubated for an equivalent length of time, and pelleted equivalently. The height of the AVICEL® was measured using a transparent ruler, and assuming the microcentrifuge tubes were roughly equivalent in volume, the volume of the conical portion of the tube was given by the following equation:

$$V = \frac{1}{3}\pi r^2 h \qquad \text{(Equation 4)}$$

Thus the volume of the AVICEL® scales proportionally with the height of the pellet, and a measurement of pellet height could therefore be used to approximate the volume.

Figure 9 shows a standard curve of AVICEL® height vs. mass of AVICEL®. The measurement of the pellet heights for various masses of AVICEL® scaled linearly within a volume region spanning the conical portion of the tube.

Figure 10 shows the height of the AVICEL® for several of the pooled NREL acid-pretreated corn stover liquor HPLC fractions incubated with the *T. aurantiacus* GH61A polypeptide. One of these pooled fractions (denoted 6H-7A) incubated with the *T. aurantiacus* GH61A polypeptide was notably higher, 9 mM compared with an average of all other samples of 5.2 mM, a 1.7-fold larger volume. The AVICEL® in this sample had swollen to a volume equivalent to an AVICEL® mass of approximately 90 mg. Pooled fractions 6H-7A along with the *T. aurantiacus* GH61A polypeptide clearly induced swelling of the AVICEL®, whereas inclusion of other liquor fractions with the GH61 polypeptide did not induce swelling.

The supernatants from the AVICEL® incubations were then decanted off and the pellet dried using a GeneVac EZ-2 Plus® vacuum concentrator (Genevac Inc., Gardiner, NY, USA). The dried residues were dissolved in deuterated water and were analyzed by $^1$H NMR using a VARIAN® MercuryVx 400 MHz NMR (Varian, Palo Alto, CA, USA). While the components of the liquor could not be identified by NMR spectroscopy, the NREL liquor fractions that produced swelling of the AVICEL® on incubation with the *T. aurantiacus* GH61 polypeptide generated NMR peaks corresponding to saccharide chemical shifts.

The solid residual cellulose was then tested for reducing end content using a method modified from Zhang and Lynd, 2005, Biomacromolecules 6: 1510-151. The cellulose was washed in 1 ml of 1.1% sodium dodecyl sulfate (SDS) and incubated at 95°C for 10 minutes with shaking, and then the suspension was pelleted by centrifugation at 31,000 rpm in a microcentrifuge. The SDS solution was decanted off and the pellet was washed by repeated resuspension in 1.5 ml of 70% ethanol and pelleting. The pellets were finally incubated with a 1:1 solution of $H_2O$ and BCA working solution (1:1 mixtures of 0.624 g of $CuSO_45H_2O$, and 0.631 g of L-serine per 500 ml of $H_2O$ with 0.971 g of disodium 2,2'-bichinchoninate, 27.12 g of $Na_2CO_3$, and 12.1 g of $NaHCO_3$ per 500 ml of $H_2O$) at 65°C for 30 minutes. The concentration of reducing ends was determined by comparison to a standard curve generated by serial dilution of glucose in the same BCA working solution and measurement of $A_{(600nm)}$ in a 96-well microtiter plate using a POWERWAVE X™ microplate spectrophotometer (Biotek Instruments, Winooski, VT USA).

Figure 11A shows the glucose reducing end standard curve of $A_{600\ nm}$ vs. glucose concentration. The concentration of reducing ends in the *T. aurantiacus* GH61A polypeptide incubated samples was calculated from this standard curve. Figure 11B shows the number of reducing end equivalents for AVICEL® incubated with the GH61 polypeptide and the indicated NREL acid-pretreated corn stover HPLC fractions. The same NREL acid-pretreated corn stover fractions that induced GH61 polypeptide-dependent swelling of the AVICEL®, showed a much higher apparent concentration of reducing end equivalents in the insoluble, washed residual cellulose suggesting that hydration of these reducing ends was the likely cause of the swelling of the cellulose.

**Example 23: Enrichment of acid-pretreated xylan components**

Xylan was acid-pretreated and then fractionated by HPLC chromatography according to Example 21 for NREL acid-pretreated corn stover liquor with the following exceptions. Acid pretreated xylan was generated by incubating 45 g of beechwood xylan with 400 g of 1.1% $H_2SO_4$ at 190°C for 2 minutes in a a 1 gallon, high-pressure horizontal stirred reactor (Parr Instrument Company, Moline, IL, USA). No preincubation with cellulose and organic phase wash was performed. The acid-pretreated xylan was filtered with a 3 kDa MWCO VIVASPIN 20® centrifuge filter (GE Healthcare, Piscataway, NJ, USA), retained on a Macrosep 1kD Omega centrifuge filter (Pall, Ann Arbor, MI), washed with 3-fold volumes of deionized water, and applied to a 8.0 mm x 300 mm Shodex Sugar SP0810 chromatography column (Showa Denka America, Inc., NY, USA) using an AGILENT® 1100 HPLC and CHEMSTATION® software, and separated by isocratic elution with water at a flow rate of 0.5 ml per minute at 80°C for 50 minutes, collecting 250 μl fractions in 2.0 ml, 96-deep well plates (Axygen, Union City, CA, USA). Repeated 100 μl injections of the liquor followed by repeated fraction collection yielded approximately 5 ml of each fraction, which were pooled and lyophilized to dryness. Later separations included an overnight incubation of the acid-pretreated xylan with endoxylanase (SHEARZYME™, Novozymes A/S, Bagsvaerd, Denmark) in 50 mM sodium acetate pH 5.0 at 50°C to hydrolyze xylo-oligomers, followed by removal of the enzyme with a 3 kDa MWCO VIVASPIN 20® centrifuge filter prior to chromatography. *Thermoascus aurantiacus* GH61A polypeptide titrations to AVICEL® hydrolysis reactions containing either the endoxylanase-treated or the untreated xylan liquor were performed according to Example 3 and confirmed that endoxylanase treatment had not altered the effects of acid-pretreated xylan on GH61 polypeptide enhancement of cellulolysis in these samples. Absorbance of the eluted fractions was not determined. Fractions were assayed by dissolution of the lyophilized fractions in 200 μl of water, and addition of 100 μl to saccharification reactions according to Example 3, containing 4 mg of the *T. reesei* cellulase composition and 1 mg of the *T. aurantiacus* GH61A polypeptide, or no GH61A.

Figure 12 shows the hydrolysis of AVICEL® by the *T. reesei* cellulase composition with supplemented *T. aurantiacus* GH61A polypeptide and the HPLC fractions as indicated. This elution profile demonstrated a broad elution peak of fractions that in the presence of the GH61 polypeptide resulted in higher hydrolysis by the *T. reesei* cellulase composition. Thus, these fractions contained compounds that in the presence of GH61 polypeptide promoted GH61 polypeptide-dependent cellulolytic enhancement. Several inhibitory fractions were also observed, as is evident in Figure 12, fraction 5D. The elution of the components that facilitate GH61 polypeptide cellulolytic enhancement centered on the same fractions that had previously been shown to facilitate GH61 polypeptide cellulolytic enhancement in chromatography of NREL pretreated corn stover liquor (Example 15). It is likely that the width of the elution was due to overloading of the HPLC column and concomitant loss of theoretical plate. This hypothesis was further supported by the magnitude of the enhancement in cellulolysis by the HPLC fractions examined, which was much higher than observed following NREL acid-pretreated corn stover liquor.

The fractions that had higher fractional hydrolysis in the presence of the *Thermoascus aurantiacus* GH61A polypeptide were analyzed by LC-MS in the following manner. Samples were diluted in 0.1% formic acid to 20 μg per ml approximate concentrations. Samples were then either filtered using Ultrafree-MC centrifugal filter devices (Millipore Corporation, Billerica, MA, USA) or were centrifuged for 10 minutes at 21,000 x g and then stored at 4°C if analysis was not performed immediately. UPLC® tandem mass spectrometry, was performed using a Q-Tof *micro*™ hybrid orthogonal quadrupole time-of-flight mass spectrometer (Waters Micromass MS Technologies, Milford, MA, USA) using MASSLYNX™ software version 4.1 (Waters Micromass MS Technologies, Milford, MA, USA). The Q-TOF *MICRO*™ was fitted with an ACQUITY UPLC® using a 1.0 x 50 mm, C18, 1.7 μm, BEH Acquity column (Waters Corp, Milford, MA, USA) to permit chromatographic separation of analytes. The following elution gradient was applied over a 37 minute interval at a flow rate of 100 μl per minute: 0-15 minutes from 1-15% acetonitrile with 0.1% formic acid, 15-20 minutes from 15-40% acetonitrile with 0.1% formic acid, 20-25 minutes from 40-80% acetonitrile with 0.1% formic acid, 25-30 minutes with 80% acetonitrile with 0.1% formic acid, 30-31 minutes from 80-1 % acetonitrile with 0.1% formic acid, and 31-37 minutes with 1% acetonitrile with 0.1% formic acid. Elution was monitored at 280 nm through a diode array detector and eluents from the column were introduced directly into the Q-TOF *MICRO*™ via electrospray ion source. A cone voltage of 20 volts was typically used and the collision energy was varied in the range of 5-15 volts. Data were acquired in survey scan mode from a mass range of m/z 50 to 1000 with switching criteria for MS to MS/MS that included an ion intensity of greater than 50.0 counts per second. The acquired spectra were combined, smoothed, and centered in an automated fashion. Analytes were identified by comparison to spectra of standard compounds when possible. Standard compounds were analyzed before and after the samples to confirm the mass accuracy and retention time stability of the sample analyses.

Figure 13 shows a representative LC-MS chromatogram of a *T. aurantiacus* GH61A polypeptide cellulolytic enhancing acid-pretreated xylan HPLC fraction. It is clear from the number of peaks that significant heterogeneity was observed. The bulk of the components were eluted from the LC in the first 6-minutes, suggesting a highly polar or charged composition.

**Example 24: Using GH61 polypeptide-binding affinity to enrich NREL acid-pretreated corn stover liquor for components that are functional in GH61 polypeptide-dependent cellulolytic enhancement**

*Thermoascus aurantiacus* GH61A polypeptide affinity was used as a means to enrich NREL acid-pretreated corn stover liquor components that bind to the GH61 polypeptide. Seventy mg of the *T. aurantiacus* GH61A polypeptide were incubated overnight with 50 ml of liquor. The GH61 polypeptide-bound liquor components were separated from the free components by ultracentrifugation using a 10 kDa MWCO VIVASPIN 20® centrifuge filter, concentrating the protein and protein-bound fraction 10-fold, followed by washing with an equivalent volume of water to the starting material (50 ml total volume), and repeating 5-times. Finally, the protein was denatured by incubation at 90°C for 30 minutes, and centrifuged using a 10 kDa MWCO VIVASPIN20® centrifuge filter. The flow-through from the filter was analyzed using liquid chromatography mass spectrometry according to Example 23, with electrospray mass spectrometry performed in both positive and negative ionization modes.

Figure 14 shows the LC-MS chromatogram of the NREL acid-pretreated corn stover liquor components that bound to the *T. aurantiacus* GH61A polypeptide and were eluted by denaturation. Both positive and negative mode ion chromatographs were combined, yielding a small set of unique mass ions, many of which could be excluded as dimers, fragments of larger parent ions or contaminants derived from the buffer or from the centrifuge columns, leading to identification of approximately 30 unique mass ions of reasonable intensity. Based on the monoisotopic masses obtained, chemical formulae were determined and a database search of chemical compounds (ChemSpider, Royal Society of Chemistry) yielded a list of putative compounds for GH61 polypeptide assay. In the chromatograms, 3 broadly classified sets of compounds could be identified: the first set eluted in the first 2-minutes, had smaller mass and were likely small polar or charged compounds; a second lower abundance set, corresponding to a broadly varied set of molecular weights but likely to be less polar, eluted with intermediate retention times; and finally a series of larger molecular weight ions eluted between 12 and 20 minutes, corresponding to less polar or higher molecular weight compounds. It was observed that the search of monoisotopic masses frequently yielded compounds consistent with plant flavonols, flavanols, oxidized flavonoids, oxidized flavanols and similar compounds, thus a large set of these compounds were tested for GH61 cellulolytic enhancing activity.

**Example 25: Effect of NREL pretreated corn stover liquor and the *Thermoascus aurantiacus* GH61A polypeptide on generating soluble cellodextrin from microcrystalline cellulose or phosphoric acid-swollen cellulose**

Monosaccharide, disaccharide, and aldonic and uronic acid standards (Sigma-Aldrich, St. Louis, MO, USA) were dissolved in water at 1 mg per ml and were diluted to 50 μg per ml in 10 mM NaOH. Cellooligosaccharides (Sigma-Aldrich, St, Louis, MO, USA) were dissolved in water at 25 to 30 mg per ml and were diluted as above. Xylooligosaccharides (Megazyme Bray, Co. Wicklow, Ireland) were dissolved in water at 10 to 30 mg per ml and were diluted as above. Lactobionic acid was purchased from Sigma-Aldrich (St. Louis, MO, USA), dissolved in water at 2.5 mg per ml. The aldonic acids of each cellooligosaccharide were generated by incubation of 17 μg of *Humicola insolens* cellobiose dehydrogenase (WO 2010/080527) per ml with 2.5 mg per ml of each cellooligosaccharide in 50 mM sodium acetate pH 5.0 at 50°C and 10 mM dichloroindophenol (DCIP; Sigma-Aldrich, St. Louis, MO, USA) was added incrementally. The equivalence point was achieved when DCIP was not reduced by the solution, and the color remained purple. Formation of the aldonate products was confirmed by LC-MS according to Example 25. Cellobiose dehydrogenase was then removed by ultrafiltration through a VIVASPIN 20® 10 kDa MWCO centrifuge filter.

The *Thermoascus aurantiacus* GH61A polypeptide was incubated at 37.5 μg/ml with either 29.5 mg per ml microcrystalline cellulose (AVICEL®™, Sigma Aldrich, St. Louis, MO, USA) or 1.2% (w/w) phosphoric acid-swollen cellulose (PASC) in 50 mM sodium acetate, 1 mM $MnSO_4$ for 5 days at 50°C with or without 10% (v/v) NREL pretreated corn stover liquor. NREL pretreated corn stover liquor was extracted according to Example 2. Phosphoric acid swollen cellulose was prepared from AVICEL® PH101 using the protocol described by Zhang et al., 2006, Biomacromolecules 7: 644-648. Control incubations containing AVICEL® alone, PASC alone, AVICEL® or PASC with NREL acid-pretreated corn stover liquor in the absence of the *T. aurantiacus* GH61A polypeptide were performed in the same manner. The samples were then filtered through a 0.22 μm centrifuge filter, and the filtrates were diluted 1:10 in 1 ml final volume of 10 mM NaOH and analyzed by DIONEX® Ion Chromatography with pulsed amperometry detection (IC-PAD, Dionex Corporation, Sunnyvale, CA) using DIONEX® Chromeleon or PeakNet Software. Ten μl of 2.5 mg per ml lactobionic acid was added as an external loading control. Chromatographic separation was obtained using a PA-20 column (Dionex Corporation, Sunnyvale, CA, USA), with relevant guard, borate and amine-trap pre-columns, and elution was achieved with an isocratic gradient of 13 mM sodium hydroxide, 2.5 mM sodium acetate for 20 minutes, followed by a linear gradient from 13 to 50 mM NaOH, isocratic gradient for 10 minutes and linear gradient from 0.5 to 40 mM sodium acetate in 50 mM NaOH.

Figure 15A shows the chromatograms of AVICEL® and PASC incubations with GH61 polypeptide, with GH61 polypeptide and pretreated corn stover liquor, and control incubations. The chromatograms indicated that soluble oligosaccharide products were evolved from the incubations containing the GH61 polypeptide, liquor, buffer, and cellulose in both PASC and microcrystalline forms. The arrows indicate unique elution peaks that were present only in the incubations containing the full set of components. Figure 15B shows a comparison of chromatogram of AVICEL® incubated with the *T. aurantiacus* GH61A polypeptide, acid-pretreated corn stover liquor, and buffer to the chromatograms of standard compounds. Figure 15B indicated that the retention times of the novel products were consistent with those of cellopentaose, cellotetraose, and cellotriose. Three additional elution peaks were also evident; a large peak with retention time of 49 minutes matched no available standard but was likely cellohexaose based on its retention time in comparison to the cellooligosaccharides and aldonic acid standards. The relative peak intensities indicated that the major product may be cellotetraose, though the sparing solubility of cello-oligosaccharides of DP $\geq 6$ may have understated the actual production of these oligomers.

**Example 26: Effect of water-extracted and alkaline pretreated corn stover liquors in combination with the *Thermoascus aurantiacus* GH61A on cellulolysis by the *T. reesei* cellulase composition**

The *Thermoascus aurantiacus* GH61A polypeptide was assayed for cellulolytic enhancing activity according to Example 3 with 10% (v/v) added liquor from either alkaline pretreated corn stover or 10% (v/v) added liquor from water-extracted acid-pretreated corn stover according to Example 2. Alkaline pretreated corn stover was prepared in the following manner. Milled and washed, raw PCS was treated with 4%, 6%, 8% or 10% (w/w) NaOH for 1 hr at 90°C in a 2-L stirred tank reactor (IKA Works, Inc., Wilmington, NC, USA). Water extracted corn stover liquors were generated in the following manner. Raw corn stover was milled in a WILEY® Mill (Model 4; Thomas Scientific, Swedesboro, NJ, USA) with a nominal screen size of 2 mm. The milled stover was sieved through a #40 mesh screen and the fines were discarded. A Dionex Accelerated Solvent Extractor (ASE) 350 (Dionex Corporation, Sunnyvale, CA, USA) instrument was used for all pretreatments with two cycles for each pretreatment. The first cycle of each pretreatment was a pressurized hot water extraction run in standard flow mode of operation. The second cycle of the pretreatment was the same for each experimental sample, and was a mid-severity dilute acid pretreatment run in pressure solvent saver mode of operation. Approximately 20.0 g of the sieved corn stover were packed into a 100 ml stainless steel extraction cell. During the pressurized hot water extraction the stover was extracted at temperatures of 100-170°C in 10°C increments for a static reaction time of 7 minutes. After extraction the cell was purged with nitrogen for 60 seconds, and all extraction liquor collected. Static pretreatment time was defined from the time the internal temperature of the cell reached equilibrium with the heating chamber. Liquors from the first, water-extraction step were filtered using a 10 kDa MWCO VIVASPIN 20® centrifuge filter and were pH adjusted to 5.0 by addition of NaOH or HCl, prior to use.

As shown previously, GH61 polypeptide titrations in the absence of added liquors did not significantly enhance microcrystalline cellulose hydrolysis by the *T. reesei* cellulase composition (Examples 15, 16, and 18). Figure 16A shows the fractional hydrolysis of AVICEL® by the *T. reesei* cellulase composition with increasing concentrations of the *T. aurantiacus* GH61A polypeptide in the presence of liquors from various severities of alkaline pretreated corn stover. Addition of alkaline corn stover liquors increasing in severity from 4% NaOH to 10% NaOH showed reduced fractional hydrolysis, thus increasing inhibition in the absence of the GH61 polypeptide. Unlike acid-pretreated corn stover liquors, however, addition of the GH61 polypeptide in the presence of alkaline liquors provided no substantial improvement over saccharifications without liquor.

The higher severity alkaline pretreated corn stover liquors in combination with the *T. aurantiacus* GH61A polypeptide did provide a small amount of cellulolytic enhancing activity at 7 days of hydrolysis. Comparing zero to 50% GH61 polypeptide additions, hydrolysis in the presence of 6% NaOH liquor was $0.473 \pm 0.00483$ and $0.488 \pm 0.00724$, 8% NaOH was $0.454 \pm 0.00352$ and $0.485 \pm 0.000119$, and 10% NaOH was $0.445 \pm 0.00625$ and $0.476 \pm 0.00732$ The addition of the *T. aurantiacus* GH61A polypeptide was sufficient to partially mitigate the inhibition arising from the liquor additions; as the hydrolysis in the absence of liquor was $0.502 \pm 0.0109$ and $0.499 \pm 0.00642$ at zero and 50% (w/w) GH61A polypeptide, respectively.

Figure 16B shows the fractional hydrolysis of AVICEL® by the *T. reesei* cellulase composition with increasing concentrations of the *T. aurantiacus* GH61A polypeptide in the presence of corn stover liquors extracted with water at the indicated temperatures. Pressurized water-extracted corn stover liquors did not appreciably enhance fractional hydrolysis in the presence of the GH61 polypeptide. Water-extracted liquors generated at 150°C and above show some inhibition, both in the absence and presence of the GH61 polypeptide. These data indicated that pressurized water-extraction did not extract the liquor components required to observe GH61 polypeptide cellulolytic enhancing activity on microcrystalline cellulose.

**Example 27: Effect of acid-pretreated xylan, acid-pretreated biomass component mixtures containing xylan, or acid-pretreated monosaccharide components of xylan including xylose and arabinose in combination with *Thermascus aurantiacus* GH61A polypeptide on cellulose hydrolysis by the *Trichoderma reesei* cellulase composition**

The cellulolytic enhancing effect of the *T. aurantiacus* GH61A polypeptide on microcrystalline cellulose was assayed with components of biomass that had been acid-pretreated.

The *Thermoascus aurantiacus* GH61A polypeptide was assayed for cellulolytic enhancing activity according to the procedure described in Example 3 with the following exceptions: hydrolysis reactions were performed with 10% (v/v) added liquor from acid-pretreated corn stover (Example 2) or with 10% (v/v) added liquor from acid-pretreated biomass components including xylan, cellulose, protein, lipid, monosaccharide, and combinations thereof. AVICEL® (1.8 g), oatspelt xylan (1.1 g; TCI), lignin (0.9 g; MeadWestvaco), corn gluten (0.25 g; Sigma Aldrich, St. Louis, MO, USA), gallic acid (0.25 g; Sigma Aldrich, St. Louis, MO, USA), ferulic acid (0.25 g; Sigma Aldrich, St. Louis, MO, USA), or corn oil (0.25 g; Sigma Aldrich, St. Louis, MO, USA) were pretreated by dissolution in 25 ml of 1% $H_2SO_4$ and incubation in an SBL-2 fluidized sand bath reactor with TC-8D Temperature Controller (Techne Inc., Burlington, NJ, USA) for a 5 minutes temperature ramp followed by 5 minutes at 190°C. Aliquots from saccharification reaction were removed for analysis at 1 and 5 days. Liquors were filtered using a 10 kDa MWCO VIVASPIN 20® centrifuge filter and pH adjusted to 5.0 by addition of NaOH or HCl, prior to use.

Figure 17A and 17B show fractional hydrolysis of AVICEL® by the *T. reesei* cellulase composition with various concentrations of the *T. aurantiacus* GH61A polypeptide in the presence of liquors derived from acid-pretreatment of various biomass components. Titration of the *T. aurantiacus* GH61A polypeptide from 0 to 50% (w/w) addition to the *T. reesei* cellulase composition yielded, in the absence of liquor, no enhancement of AVICEL® hydrolysis (Example 15, 16, 18, and Figure 19B). Increasing concentrations of the *T. aurantiacus* GH61A polypeptide increases fractional hydrolysis in the presence of a subset of the liquors examined. Small GH61 polypeptide concentration-dependent hydrolysis enhancements were observed in the presence of liquors of acid-pretreated lignin, lipid, protein, cellulose, lignin model compounds such as gallic acid, and hemicellulase components such as ferulic acid. The extent of GH61 polypeptide enhancement of fractional hydrolysis in the presence of acid-liquors was from 0.390 $\pm$ 0.007 to 0.435 $\pm$ 0.010 in the presence of lignin liquor, from 0.305 $\pm$ 0.013 to 0.341 $\pm$ 0.016 in the presence of corn oil liquor, 0.493 $\pm$ 0.007 to 0.524 $\pm$ 0.004 in the presence of corn gluten liquor, 0.134 $\pm$ 0.021 to 0.184 $\pm$ 0.023 in the presence of gallic acid liquor, 0.352 $\pm$.011 to 0.397 $\pm$ 0.029 in the presence of ferulic acid liquor at 7 days of hydrolysis. Conversely, liquor derived from acid-pretreatment of xylan yielded a substantial GH61 polypeptide concentration-dependent enhancement of cellulolysis, from 0.390 $\pm$ 0.009 to 0.540 $\pm$ 0.013 at 7 days of hydrolysis. Where possible, biomass components had been acid pretreated at their respective concentrations in NREL acid-pretreated corn stover, thus the observed GH61 cellulolytic enhancements should scale proportionally with their contributions to enhancements in pretreated corn stover. The bulk of the apparent GH61 polypeptide concentration-dependent cellulolytic enhancements on cellulose are thus derived from the xylan components.

**Example 28: Effect of residual liquors post-fermentation in combination with the *Thermoascus aurantiacus* GH61A polypeptide on cellulose hydrolysis by the *T. reesei* cellulase composition**

The *Thermoascus aurantiacus* GH61A polypeptide was assayed for cellulolytic-enhancing activity according to Example 3 with the following exceptions: 10% (v/v) added liquor from post-fermentation residues was added, and aliquots were removed at 1, 3, and 7 days of saccharification. Liquors were filtered using a 10 kDa MWCO VIVASPIN 20® centrifuge filter and pH adjusted to 5.0 by addition of NaOH or HCl, prior to use. Fermentation liquors were obtained as follows: 20% total solids NREL unwashed, unmilled pretreated corn stover was hydrolyzed by 5 mg per gram cellulose of a *T. reesei* cellulase composition for 5-days. The hydrolysate was fermented for 2 days using a *Saccharomyces cerevisiae* strain comprising a xylose isomerase gene (WO 2003/062430) at 3% cell density for 2 days. Fifty ml of the fermentation liquor was filtered as described and assayed for GH61 polypeptide concentration-dependent cellulolytic enhancement activity.

Figure 18 shows the fractional hydrolysis of AVICEL® by the *T. reesei* cellulase composition with various concentrations of the *T. aurantiacus* GH61A polypeptide in the presence of post-fermentation liquors. The addition of increasing *T. aurantiacus* GH61A polypeptide concentrations significantly enhanced hydrolysis of AVICEL® in the presence of the post-fermentation liquors from 0.246 $\pm$ 0.007 to 0.265 $\pm$ 0.007 at 1 day of hydrolysis and from 0.616 $\pm$ 0.006 to 0.895 $\pm$ 0.002 at 7 days of hydrolysis.

**Example 29: Effect of corn stover liquors from increasing severity of acid pretreatments in combination with the *Thermoascus aurantiacus* GH61A polypeptide on cellulose hydrolysis by the *T. reesei* cellulase composition**

The *Thermoascus aurantiacus* GH61A polypeptide was assayed for cellulolytic enhancing activity according to Example 3 with 10% (v/v) added liquor from acid-pretreated corn stover or microcrystalline cellulose (AVICEL®) of various severity pretreatments. Liquors were filtered using a 10 kDa MWCO VIVASPIN 20® centrifuge filter and pH adjusted to 5.0 by addition of NaOH or HCl, prior to use. Liquors were generated using an SBL-2 fluidized sand bath reactor with TC-8D Temperature Controller, incubating corn stover with 1% $H_2SO_4$ in 25 ml and incubating at temperatures between 140°C and 170°C for 1 to 5 minutes, or by incubating AVICEL® with 1% $H_2SO_4$ in 25 ml at temperatures between 110°C and 190°C for 5 minutes.

Figures 19A and 19B show the fractional hydrolysis of AVICEL® containing various severity acid-treatments of corn stover by the *T. reesei* cellulase composition and various concentrations of the *T. aurantiacus* GH61A polypeptide. Liquors were generated by systematically varying both temperature between 140°C and 170°C and varying time at each temperature between 1 and 5 minutes. For saccharification reactions containing liquors produced by acid-pretreatment at temperatures greater than 140°C and 5 minutes, increasing *T. aurantiacus* GH61A polypeptide concentrations increased fractional hydrolysis. The higher severity pretreatments, particularly those of 160°C and above, showed a greater increase in fractional hydrolysis from GH61 polypeptide addition than did the lower severity pretreatments of 150°C and less. Additionally, higher severity pretreatment liquors were increasingly inhibitory to cellulolysis, thus the greatest overall cellulose conversion was obtained by addition of high GH61 polypeptide concentrations (50% w/w) at intermediate pretreatment severity, specifically 150°C for 5 minutes, 160°C for 3-5 minutes, or 170°C for 1 minute. These data demonstrated that pretreatment of a biomass can be tailored to maximize hydrolysis for a given GH61 polypeptide concentration in a cellulase composition. There is an optimum pretreatment condition that balances GH61 polypeptide cellulolytic-enhancing activity with production of soluble inhibitor compounds.

Figure 19C shows the fractional hydrolysis of AVICEL® containing various severity acid-treatments of AVICEL® by the *T. reesei* cellulase composition and various concentrations of the *T. aurantiacus* GH61A polypeptide. In calculating the fractional hydrolysis, sugars derived from liquor addition were not subtracted, and contributed up to 3% of conversion. From Figure 19C it is clear that liquors derived from acid-treatment of microcrystalline cellulose in combination with the *T. aurantiacus* GH61A polypeptide enhanced hydrolysis only marginally.

**Example 30: Effect of xylan liquors from increasing severity of acid pretreatments in combination with the *Thermoascus aurantiacus* GH61A polypeptide on cellulose hydrolysis by the *T. reesei* cellulase composition**

The *Thermoascus aurantiacus* GH61A polypeptide was assayed for cellulolytic enhancing activity according to Example 3 with 10% (v/v) added liquor from xylan pretreated with varying severity. Liquors were generated by incubation of 1.1 g of either oatspelt xylan or wheat flour with 1% $H_2SO_4$ or 3% HCl in 25 ml total volume in an SBL-2 fluidized sand bath reactor with TC-8D Temperature Controller for 10 minutes total; a 5 minute ramp period followed by a 5 minute incubation at the indicated temperature. Incubation temperatures were varied between 60°C and 180°C. Pretreatment liquors were filtered using a 10 kDa MWCO VIVASPIN 20® centrifuge filter and pH adjusted to 5.0 by addition of NaOH or HCl, prior to use.

Figures 20A and 20B show the effect of acid-pretreated xylan liquors added to the *T. reesei* cellulase compositions with increasing concentrations of the *T. aurantiacus* GH61A polypeptide. In the absence of the GH61 polypeptide, liquors of increasing pretreatment severity were increasingly inhibitory to the cellulolysis of AVICEL®, particularly those generated with 3% HCl. Titration of increasing GH61 polypeptide concentration in the presence of xylan acid-pretreated at temperatures greater than 140°C led to increasing cellulolysis, and the net change in hydrolysis with GH61 polypeptide addition was higher for higher severity pretreatments. Hydrolysis was not improved by the GH61 polypeptide on addition of xylan pretreated at 60°C to 120°C. Hydrolysis was improved by 0.057, from 0.349 $\pm$ 0.00510 to 0.406 $\pm$ 0.00394 with 160°C liquor, by 0.089 from 0.287 $\pm$ 0.00233 to 0.376 $\pm$ 0.00118 with 180°C liquor, and by 0.0740, from 0.216 $\pm$ 0.00225 to 0.293 $\pm$ 0.00843 with 180°C HCl derived liquor. The effect of increasing pretreatment severity on *T. aurantiacus* GH61A polypeptide cellulolytic enhancing activity with xylan liquors was similar to those of corn stover pretreatment severity (Example 26), and again suggested a maximal overall hydrolysis was obtained by high GH61 polypeptide concentrations in the presence of liquors of intermediate severity. In this case, 50% (w/w) *T. aurantiacus* GH61A polypeptide in the presence of 160°C acid-pretreated xylan produced the highest cellulose conversion. Additionally, as previously observed for corn stover liquors, there appeared to be an optimum balance between GH61 polypeptide cellulolytic enhancing activity and the production of soluble inhibitors. The addition of a specific concentration and tailoring of pretreatment severity can be accomplished for a given GH61 polypeptide concentration within a cellulase composition.

Figure 20C shows the fractional hydrolysis of AVICEL® containing various severity acid-treatments of beechwood xylan by the *T. reesei* cellulase composition and various concentrations of the *T. aurantiacus* GH61A polypeptide. From Figure 20C, it was clear that the maximum conversion was achieved in the presence of a high concentration of the *T. aurantiacus* GH61A polypeptide (50%) and liquor produced at 170°C. In the presence of liquor generated at 190°C, the observed hydrolysis was lower for all concentrations of the *T. aurantiacus* GH61A polypeptide tested, and in the presence of liquors generated below 170°C; the conversion levels reached were not as great. These data suggest that treatment severity of some substrates may be optimized to generate liquors for a given GH61 polypeptide concentration within a cellulase composition.

**Example 31: Effect of solid-phase extracted acid pretreated corn stover liquors in combination with the *Thermoascus aurantiacus* GH61A polypeptide on cellulose hydrolysis by the *T. reesei* cellulase composition**

The *Thermoascus aurantiacus* GH61A polypeptide was assayed for cellulolytic enhancing activity according to Example 3 with the following exceptions. *T. aurantiacus* GH61 polypeptide was either not added or was added at 50% (w/w) of total protein and 10% (v/v) solid phase extraction resin-eluted liquor was added to each hydrolysis reaction. Both NREL acid-pretreated corn stover liquor prepared according to Example 2 and acid-pretreated xylan prepared according to Example 23, and then pH adjusted to 5.0, were applied to one of two solid phase extraction cartridges, either a BOND ELUT™ C-18 column (Varian, Palo Alto, CA, USA) or a STRATA™-X Polymeric column (Phenomenex, Torrance, CA, USA) equilibrated in water. The samples were washed 3-times with 1 ml of water and then eluted with two aliquots of 600 μl methanol. The eluted samples were diluted back to their original volumes with water and assayed for GH61 polypeptide cellulolytic enhancing activity.

Figure 21 shows the fractional hydrolysis of AVICEL® by the *T. reesei* cellulase composition with and without 50% (w/w) *T. aurantiacus* GH61A polypeptide addition in the presence of solid-phase extracted liquors as indicated. The fractional hydrolysis was equivalent within error for all samples, indicating that no enhancement of cellulolysis from GH61 polypeptide addition was observed in the presence of solid-phase extracted liquors from acid-pretreated corn stover or from acid-pretreated xylan. A cellulolytic enhancement of the *T. reesei* cellulase composition was previously demonstrated in the presence of these liquors in combination with addition of the GH61 polypeptide, prior to solid-phase extraction. It was therefore concluded that the compounds present in these liquors that were correlated with the observed GH61 polypeptide cellulolytic enhancing activity were eluted during the wash steps.

**Example 32: Effect of electrodialyzed acid-pretreated corn stover liquors on *Thermoascus aurantiacus* GH61A polypeptide enhancing the *Trichoderma reesei* cellulase composition**

The *Thermoascus aurantiacus* GH61A polypeptide was assayed for cellulolytic-enhancing activity according to Example 3 with the following exceptions: 10% (v/v) NREL pretreated corn stover liquor, acid-pretreated xylan (Example 23) or electrodialyzed NREL pretreated corn stover liquor was added to each hydrolysis reaction. Electrodialyzed NREL pretreated corn stover liquor was generated in the following manner. Two kg of NREL pretreated corn stover liquor was filtered and. the pH was adjusted to 5.0 using concentrated NaOH. The pretreatment liquor was subjected to concentrating electrodialysis using a EUR2B pilot scale electrodialysis unit (Ameridia, Somerset, NJ, USA) equipped with a EUR2B-10 stack Ameridia, Somerset, NJ, USA). The solution of pH adjusted liquor had a conductance of 16.5 mS/cm and a pH of 5.0 and was charged to the diluate tank. A solution of potassium nitrate (20 mS/cm, 2.0 kg) was charged to the electrode rinse tank. A dilute solution of NREL pretreatment liquor (0.657 mS/cm) was charged to the concentrate tank. The solutions described above were circulated through the EUR2B-10 stack at a flow rate of approximately 0.9 gallon per minute (gpm) using a DC power supply set to 14 volts. After 55 minutes, the conductivity change in the diluate and concentrate tanks remained stable and the run was ended. The final conductance in the concentrate tank was 14.01 mS/cm.

Figure 22 shows that increasing the *T. aurantiacus* GH61A polypeptide concentration in the presence of NREL pretreated corn stover liquor, electrodialyzed NREL acid-pretreated corn stover liquor, and acid-pretreated xylan increased the fractional hydrolysis of AVICEL®. At the highest concentrations of GH61 polypeptide, the electrodialyzed NREL liquor showed slightly lower conversion than the undialyzed sample, 0.541 ± 0.0006 compared with 0.524 ± 0.0118, though at intermediate GH61 polypeptide concentrations the conversion was higher for the electrodialyzed liquor, 0.467 ± 0.00671 compared with 0.502 ± 0.00215.

**Example 33: Effect of addition of acid-pretreated corn stover liquors and *Thermoascus aurantiacus* GH61A polypeptide to non-acid pretreated biomass feedstocks**

The *Thermoascus aurantiacus* GH61A polypeptide was assayed for cellulolytic-enhancing activity according to Example 3 with the following exceptions: 5% (v/v) added NREL acid-pretreated corn stover liquor was added to either 3% or 5% total solids of various milled biomass feedstocks, and aliquots were removed for analysis at 1, 3, and 7 days of saccharification. Control incubations of each biomass containing either no *T. reesei* cellulase composition and no GH61 polypeptide, or containing GH61 polypeptide in the absence of the *T. reesei* cellulase composition with and without liquor, and control incubations containing an equivalent concentration of liquor with the *T. reesei* cellulase composition and GH61 polypeptide without biomass were performed in parallel. The composition of total accessible cellulose in each biomass was determined by measurement of glucose equivalents produced by saccharification by 50 mg of Cellic CTec™ (available Novozymes A/S, Bagsvaerd, Denmark) over 7 days of hydrolysis. Liquors were filtered using a 10 kDa MWCO VIVASPIN 20® centrifuge filter and pH adjusted to 5.0 by addition of NaOH or HCl, prior to use. Biomass feedstocks included raw sugarcane bagasse and corn stovers pretreated in the following manners: organosolv low severity ethanol, organosolv medium severity ethanol, organosolv high severity ethanol, and organosolv glycerol.

Figures 23A, 23B, 23C and 23D show the fractional hydrolysis of various biomass feedstocks by the *T. reesei* cellulase composition with varying *T. aurantiacus* GH61A polypeptide concentrations, with and without supplemented NREL acid-pretreated corn stover liquor, as indicated. For each biomass, addition of 5% (v/v) NREL acid-pretreated corn stover liquor in the absence of the *T. reesei* cellulase composition added a background sugar concentration equivalent to approximately 0.05 increase in apparent hydrolysis, depending on the relative amount of cellulose present in each substrate. This was not substantially changed by addition of the *T. reesei* cellulase composition or the GH61 polypeptide. Since these liquor-derived sugars are not subtracted from the fractional hydrolysis values, for each biomass the presence of liquor shifts the apparent hydrolysis to a higher value.

For each biomass, a higher fractional hydrolysis was obtained by addition of higher *T. aurantiacus* GH61A polypeptide concentrations from 0 to 50% (w/w) total protein to the *T. reesei* cellulase composition. For some of these biomasses, the presence of the NREL acid-pretreated corn stover liquor increased the total cellulolytic enhancement by the *T. aurantiacus* GH61A polypeptide. This was particularly apparent at 3 days of hydrolysis (gray bars, Figure 24), as exemplified by the enhancement of low and medium severity ethanol organosolv pretreatments. In the absence of GH61 polypeptide, fractional hydrolysis of low severity ethanol organosolv corn stover was 0.148 $\pm$ 0.00447 without liquor and 0.165 $\pm$ 0.00126 with liquor, whereas at 50% GH61, fractional hydrolysis was increased from 0.237 $\pm$ 0.00737 to 0.273 $\pm$ 0.00113. Similarly, in the absence of GH61 polypeptide, fractional hydrolysis of medium severity ethanol corn stover was 0.179 $\pm$ 0.000737 without liquor and 0.190 $\pm$ 0.00401 with liquor, whereas at 50% GH61 polypeptide, fractional hydrolysis was increased from 0.281 $\pm$ 0.0136 to 0.328 $\pm$ 0.00543. Substrates that showed this augmentation of the GH61 polypeptide cellulolytic enhancing effect included organosolv low and medium severity ethanol and sugarcane bagasse. It appeared that cellulolysis of lower severity pretreatment biomass feedstocks were better enhanced by the *T. aurantiacus* GH61A polypeptide in the presence of supplemented acid-pretreated biomass liquor. The augmentation of the GH61 polypeptide cellulolytic enhancing activity on the low severity pretreatment by supplemented liquor appeared most dramatic at the early stages of saccharification.

**Example 34: Effect of addition of acid-pretreated monosaccharides on *Thermoascus aurantiacus* GH61A enhancement of *Trichoderma reesei* cellulase composition cellulolysis of microcrystalline cellulose**

The *Thermoascus aurantiacus* GH61A polypeptide was assayed for cellulolytic enhancing activity as described in Example 3 with the following exceptions. Saccharification reactions were performed with 5 mM $MnSO_4$, and either 5% or 10% (v/v) liquor derived from acid-pretreatment of monosaccharides was added. Liquors were generated in the following manner: 20 mg per ml of each monosaccharide was incubated with 1% $H_2SO_4$ in an SBL-2 fluidized sand bath reactor with TC-8D Temperature Controller at 190°C for 5 minutes, and the resulting liquors were then filtered using a 10 kDa MWCO VIVASPIN 20® centrifuge filter and pH adjusted to 5.0 by addition of NaOH or HCl prior to use. Ozonolysis was performed in the following manner: NREL acid-pretreated corn stover was slurried at 15% total solids, and the liquor extracted by vacuum filtration using a glass fiber filter. This liquor was incubated with a low concentration of ozone for 30 minutes prior to pH adjustment.

Figure 24 shows the fractional hydrolysis of AVICEL® by the *T. reesei* cellulase composition with either no GH61A polypeptide or 24% (w/w) *T. aurantiacus* GH61A polypeptide in the presence of 5% or 10% (v/v) of various acid-pretreated monosaccharides, and with ozone-treated NREL pretreated corn stover liquor. Figure 25 demonstrates that acid-pretreated monosaccharides were strongly inhibitory to cellulolysis by the *T. reesei* cellulase composition in the absence of GH61 polypeptide. In the presence of acid-pretreated hemicellulose-derived sugars, e.g., C5 sugars, including arabinose and xylose, the fractional hydrolysis was increased by addition of the *T. aurantiacus* GH61A polypeptide. In the presence of 5% acid-pretreated arabinose, addition of the GH61 polypeptide increased fractional hydrolysis from 0.183 $\pm$ 0.00372 to 0.223 $\pm$ 0.00548 at 3 days of hydrolysis, and from 0.229 $\pm$ 0.00379 to 0.328 $\pm$ 0.00272 at 7 days of hydrolysis. Similarly, in the presence of 5% acid-pretreated xylose, addition of the GH61 polypeptide increased fractional hydrolysis from 0.113 $\pm$ 0.0148 to 0.140 $\pm$ 0.00356 at 3 days of hydrolysis, and from 0.117 $\pm$ 0.00252 to 0.177 $\pm$ 0.00602 at 7 days of hydrolysis. Conversely, addition of the *T. aurantiacus* GH61A polypeptide in the presence of acid-pretreated glucose increased fractional hydrolysis to a much smaller extent, even at the higher concentration of 10% (v/v), from 0.172 $\pm$ 0.00658 to 0.206 $\pm$ 0.00108. Ozone treatment of NREL acid-pretreated corn stover liquor altered the liquor components in such a manner as to disrupt the ability of the liquor in combination with the GH61 polypeptide to enhance cellulolysis of AVICEL® by the *T. reesei* cellulase composition. In the presence of liquor, the GH61 polypeptide increased fractional hydrolysis from 0.522 ± 0.00612 to 0.679 ± 0.00807, whereas in the presence of the ozone-treated liquor, the fractional hydrolysis was increased only from 0.512 ± 0.00347 to 0.540 ± 0.00956.

**Example 35: Enhancement of** AVICEL® **cellulolysis by *T. reesei* cellulases using NREL PCS liquor and various GH61 polypeptides**

Microcrystalline cellulose saccharification reactions were performed as described (Example 3), using 29.5 mg of microcrystalline cellulose (AVICEL®) per ml and 4 mg of the *T. reesei* cellulase composition per g cellulose in 50 mM sodium acetate, 1 mM manganese sulfate at pH 5.0 in the presence of 10% (v/v) NREL PCS liquor prepared as described (Example 2). GH61 polypeptides including *Thermoascus aurantiacus* GH61A polypeptide, *Aspergillus fumigatus* GH61B polypeptide and *Penicillium pinophilum* GH61 polypeptide were added between 0 and 2 mg per g cellulose (0 and 50% (w/w) of the *T. reesei* cellulase composition concentration). Alternatively, liquor was added at 10% (v/v) to saccharifications containing either no GH61 polypeptide or 2 mg of the various GH61 polypeptides per g cellulose.

Figure 25 shows the fractional hydrolysis of AVICEL® by the *T. reesei* cellulase composition with various concentrations of the indicated GH61 polypeptides with 10% (v/v) NREL PCS liquor. Fractional hydrolysis is shown for the *T. reesei* cellulase composition with *Thermoascus aurantiacus* GH61A (circles), *Aspergillus fumigatus* GH61B polypeptide (diamonds) and *Penicillium pinophilum* GH61 polypeptide (squares) at 1 day (open symbols) and 3 days of hydrolysis (closed symbols). Addition of liquor in combination with all the GH61 polypeptides showed an apparent increase in hydrolysis of the cellulose. *Thermoascus aurantiacus* GH61A polypeptide produced the greatest enhancement of cellulolysis in the presence of liquor, 0.098 ± at 7 days of hydrolysis. Conversely, addition of the highest concentration of each of the GH61 polypeptides did not enhance cellulolysis in the absence of supplemented liquor. Multiple GH61 polypeptides are thus shown to enhance cellulolysis by *T. reesei* cellulases in the presence of biomass liquor.

**Example 36: Effect of addition of Kraft (indulin) lignin or oxidized Kraft (indulin) lignin on the *Thermoascus aurantiacus* GH61A enhancement of *Trichoderma reesei* cellulase composition cellulolysis of microcrystalline cellulose**

Microcrystalline cellulose saccharification were performed as described (Example 3), using 25 mg of microcrystalline cellulose (AVICEL®) per ml and 4 mg per g cellulose of a composition containing a blend of an *Aspergillus aculeatus* GH10 xylanase (WO 94/021785) and a *Trichoderma reesei* cellulase preparation containing *Aspergillus fumigatus* beta-glucosidase (WO 2005/047499) and *Thermoascus aurantiacus* GH61A polypeptide (WO 2005/074656) in 50 mM sodium acetate, 1 mM manganese sulfate at pH 5.0 in the presence of zero or 0.1% (w/w) Kraft (Indulin) lignin or oxidized Kraft (Indulin) lignin. Oxidized Kraft lignin was generated by overnight incubation of a 20% total solids slurry of lignin with sodium periodate at 4°C at a concentration of 5 g of sodium periodate per 100 g of slurry. The oxidized lignin was washed extensively with water and then freeze dried. The *T. reesei* cellulase composition was either supplemented with an additional 15% (w/w) *T. aurantiacus* GH61A polypeptide or was not supplemented.

Figure 26 shows the concentration of glucose produced by saccharification at various saccharification times as indicated. Addition of lignin or oxidized lignin enhanced the saccharification of AVICEL® by the *T. reesei* cellulase composition containing GH61A polypeptide. Addition of 15% (w/w) supplemental GH61A polypeptide to a *T. reesei* cellulase composition containing *T. aurantiacus* GH61A polypeptide further increased the extent of saccharification of a hydrolysis reaction containing Kraft lignin. Addition of 15% supplemental GH61A polypeptide to a cellulase composition containing GH61A polypeptide resulted in lower glucose conversion than comparable reactions without supplemental GH61A polypeptide in hydrolyses of AVICEL® containing no Kraft lignin or containing oxidized Kraft lignin. These data indicate that products of biomass pretreatment derived from specific lignins or the lignins themselves, in combination with GH61A polypeptide, enhance the conversion of cellulose by a *T. reesei* cellulase composition.

**Example 37: *Thermoascus aurantiacus* GH61A enhancement of cellulolysis of high total solids alkaline pretreated corn stover by a *Trichoderma reesei* cellulase composition**

Raw, washed and milled corn stover was pretreated with 8% (w/w of dry weight) sodium hydroxide at 90°C for 1 hour. The resulting whole slurry was transferred to a vacuum filtration apparatus and washed exhaustively with tap water until the pH of the filtrate was less than or equal to 8.6. The washed solids were then transferred to hydrolysis reactors to give final solids concentrations of 10%. A composition containing a blend of an *Aspergillus aculeatus* GH10 xylanase (WO 94/021785) and a *Trichoderma reesei* cellulase preparation containing *Aspergillus fumigatus* beta-glucosidase (WO 2005/047499) and *Thermoascus aurantiacus* GH61A polypeptide (WO 2005/074656) was replaced with increasing concentrations of GH61A polypeptide, maintaining a fixed total protein concentration of 4 mg protein per gram cellulose. The washed, milled, alkaline pretreated corn stover was hydrolyzed at 50°C for 120 hours in 10-20 g Oak Ridge tubes (Nalge Nunc International Corporation, Rochester, NY, USA) containing ¼ inch steel balls for agitation, using a FINEPCR Hybridization Oven (Daigger, Inc. Vernon Hills, IL, USA), rotating at 12 rpm.

Figure 27 shows that glucose concentration increased with increasing replacement of the cellulase composition with *T. aurantiacus* GH61A polypeptide, indicating that cellulose hydrolysis was enhanced with 5-10% additional GH61A polypeptide. Thus at high total solids, supplementation of a *T. reesei* cellulase composition with higher relative concentrations of GH61 polypeptide resulted in higher cellulose conversion of alkaline pretreated corn stovers.

**Example 38: *Thermoascus aurantiacus* GH61A enhancement of cellulolysis of high total solids pretreated corn stover of various pretreatment severities by a *Trichoderma reesei* cellulase composition**

Raw corn stover was milled in a Thomas Model 10 Wiley Mill (Thomas Scientific, Swedesboro, NJ, USA) with a nominal screen size of 2 mm and then thoroughly washed with tap water. The washed corn stover was allowed to dry in a 45°C convection oven until the dry solids content was above 90%. The dried solids were then sieved through a #40 mesh screen to ensure a uniform size distribution. An Accelerated Solvent Extractor (ASE) 350 instrument (Dionex Corporation, Bannockburn, IL, USA) was used for all pretreatments. Approximately 15.0 g of the washed, milled and sieved corn stover was packed into a 100 ml stainless steel extraction cell to ensure consistent solids loading during pretreatment. The extraction cell was loaded into the heating chamber and filled with sulfuric acid solution of various concentrations until the back-pressure reached 1500 psi. The heating chamber then heated the filled cell to the desired temperature. At the end of the heating phase, the pretreatment was stopped by immediately releasing the pressure in the extraction cell, purging with nitrogen gas, and collecting the pretreatment liquor in a designated glass vial. The extraction cell was then immediately removed from the ASE 350 instrument and quenched in ice. After cooling to room temperature, the pretreated solids were removed from the cell and re-slurried with the pretreatment liquor. The following pretreatment conditions were varied: temperature from 150-190°C, static residence times between 1 and 15 minutes, and acid concentrations between 0.4%-1.0% (w/w) $H_2SO_4$. The range for combined severity factor ranged from 0.5 to 2.0.

Pretreated corn stover at each pretreatment severity was adjusted to pH 5.0 and a final TS of 15%. Hydrolysis was initiated by adding to each PCS batch either 2 mg of a composition containing a blend of an *Aspergillus aculeatus* GH10 xylanase (WO 94/021785) and a *Trichoderma reesei* cellulase preparation containing *Aspergillus fumigatus* beta-glucosidase (WO 2005/047499) and *Thermoascus aurantiacus* GH61A polypeptide (WO 2005/074656) with 0.4 mg of *T. aurantiacus* GH61A polypeptide per gram cellulose or 1.6 mg of the composition containing a blend of an *Aspergillus aculeatus* GH10 xylanase (WO 94/021785) and a *Trichoderma reesei* cellulase preparation containing *Aspergillus fumigatus* beta-glucosidase (WO 2005/047499) and *Thermoascus aurantiacus* GH61A polypeptide (WO 2005/074656) with 0.4 mg of *T. aurantiacus* GH61A polypeptide per gram cellulose and incubating at 50°C for up to 216 hrs in 10-20 g Oak Ridge tubes containing ¼ inch steel balls for agitation, using a FINEPCR Hybridization Oven, rotating at 12 rpm.

Figure 28 shows the cellulose conversion of high total solids (15% dry weight) corn stover of various severity acid pretreatments. At each set of pretreatment conditions, except for a single, low severity pretreatment, replacement of the cellulase composition with 20% additional *T. aurantiacus* GH61A polypeptide resulted in greater cellulose conversion, particularly at 216 hours of hydrolysis. Gray bars: 120 hours of saccharification; black bars: 216 hours of saccharification. Thus at high total solids, supplementation of a *T. reesei* cellulase composition with higher relative concentrations of GH61 polypeptide resulted in higher cellulose conversion when the pretreatment severity was sufficient to generate suitable biomass liquor.

**Example 39: *Thermoascus aurantiacus* GH61A enhancement of cellulolysis of high total solids pretreated giant cane (*Arundo donax*) of various pretreatment severities by a *Trichoderma reesei* cellulase composition**

Raw *Arundo donax* was milled in a Thomas Model 10 Wiley Mill with a nominal screen size of 2 mm and then thoroughly washed with tap water. The washed *A. donax* biomass was allowed to dry in a 45°C convection oven until the dry solids content was above 90%. The dried solids were then sieved through a #40 mesh screen to ensure a uniform size distribution. An Accelerated Solvent Extractor (ASE) 350 instrument was used for all pretreatments. Approximately 20.0 g of the washed, milled and sieved *Arundo donax* was packed into a 100 ml stainless steel extraction cell to ensure consistent solids loading during pretreatment. The extraction cell was loaded into the heating chamber and filled with sulfuric acid solution of various concentrations until the back-pressure reached 1500 psi. The heating chamber then heated the filled cell to the desired temperature. At the end of the heating phase, the pretreatment was stopped by immediately releasing the pressure in the extraction cell, purging with nitrogen gas and collecting the pretreatment liquor in a designated glass vial. The extraction cell was then immediately removed from the ASE 350 instrument and quenched in ice. After cooling to room temperature, the pretreated solids were removed from the cell and re-slurried with the pretreatment liquor. The following pretreatment conditions were varied: temperature from 170-190°C, static residence times between 1 and 5 minutes, and acid concentrations between 0.5%-1.0% (w/w) $H_2SO_4$. The range for combined severity factor ranged from 0.35 to 2.13.

*Arundo donax* biomass of each pretreatment severity was adjusted to pH 5.0 and a final TS of 15%. Hydrolysis was initiated by adding either 4 mg of a composition containing a blend of an *Aspergillus aculeatus* GH10 xylanase (WO 94/021785) and a *Trichoderma reesei* cellulase preparation containing *Aspergillus fumigatus* beta-glucosidase (WO 2005/047499) and *Thermoascus aurantiacus* GH61A polypeptide (WO 2005/074656) per gram cellulose or 3.4 mg of the a composition containing a blend of an *Aspergillus aculeatus* GH10 xylanase (WO 94/021785) and a *Trichoderma reesei* cellulase preparation containing *Aspergillus fumigatus* beta-glucosidase (WO 2005/047499) and *Thermoascus aurantiacus* GH61A polypeptide (WO 2005/074656) and 0.6 mg of *T. aurantiacus* GH61A per gram cellulose and incubating at 50°C for up to 120 hours in 10-20 g Oak Ridge tubes containing ¼ inch steel balls for agitation, using a FINEPCR Hybridization Oven, rotating at 12 rpm.

Figure 29 shows the cellulose conversion of high total solids (15% dry weight) *Arundo donax* of various severity acid pretreatments. At each set of pretreatment conditions, replacement of the cellulase composition with 15% additional *T. aurantiacus* GH61A polypeptide resulted in greater cellulose conversion, particularly at 216 hours of hydrolysis. Gray bars: 72 hours of saccharification; black bars: 120 hours of saccharification. Thus at high total solids, supplementation of a *T. reesei* cellulase composition with higher relative concentrations of GH61 polypeptide resulted in higher cellulose conversion of *A. donax* biomass.

## Claims

1. A method for degrading or converting a cellulosic material, comprising: treating the cellulosic material with an enzyme composition comprising a cellulase in the presence of a GH61 polypeptide having cellulolytic enhancing activity and a liquor arising from treatment of a lignocellulose material, a hemicellulose material, a lignacious material, monosaccharides of the lignocellulose material, monosaccharides of the hemicellulose material, or a combination thereof, wherein the liquor is present in an amount that minimizes inhibition of a cellulase composition of 1 to 20% (v/v), wherein the combination of the GH61 polypeptide having cellulolytic enhancing activity and the liquor enhances hydrolysis of the cellulosic material by the enzyme composition, wherein the enhanced hydrolysis is assessed according to Equation 3:

$$GH61\ effect = \frac{\%\ \text{conversion}_{(+\ \text{GH61+ liquor})}}{\%\ \text{conversion}_{(\text{no GH61+ liquor})}} \qquad \text{(Equation 3)}$$

and the equation ratio is greater than 1,
wherein the GH61 polypeptide having cellulolytic enhancing activity comprises the following motifs:
[ILMV]-P-X(4,5)-G-X-Y-[ILMV]-X-R-X-[EQ]-X(4)-[HNQ] (SEQ ID NO: 127 or SEQ ID NO: 128) and [FW]-[TF]-K-[AIV], wherein X is any amino acid, X(4,5) is any amino acid at 4 or 5 contiguous positions, and X(4) is any amino acid at 4 contiguous positions.

2. The method of claim 1, further comprising recovering the degraded cellulosic material.

3. A method for producing a fermentation product, comprising:

(a) saccharifying a cellulosic material with an enzyme composition comprising a cellulase in the presence of a GH61 polypeptide having cellulolytic enhancing activity and a liquor arising from treatment of a lignocellulose material, a hemicellulose material, a lignacious material, monosaccharides of the lignocellulose material, monosaccharides of the hemicellulose material, or a combination thereof, wherein the liquor is present in an amount that minimizes inhibition of a cellulase composition of about 1 to about 20% (v/v), wherein the liquor is present in an amount that minimizes inhibition of a cellulase composition of 1 to 20% (v/v), wherein the combination of the GH61 polypeptide having cellulolytic enhancing activity and the liquor enhances hydrolysis of the cellulosic material by the enzyme composition, wherein the enhanced hydrolysis is assessed according to Equation 3:

$$GH61\ effect = \frac{\%\ conversion_{(+\ GH61+\ liquor)}}{\%\ conversion_{(no\ GH61+\ liquor)}} \quad \text{(Equation 3)}$$

and the equation ratio is greater than 1,
wherein the polypeptide having cellulolytic enhancing activity comprises the following motifs:
[ILMV]-P-X(4,5)-G-X-Y-[ILMV]-X-R-X-[EQ]-X(4)-[HNQ] (SEQ ID NO: 127 or SEQ ID NO: 128) and [FW]-[TF]-K-[AIV], wherein X is any amino acid, X(4,5) is any amino acid at 4 or 5 contiguous positions, and X(4) is any amino acid at 4 contiguous positions;
(b) fermenting the saccharified cellulosic material with one or more fermenting microorganisms to produce the fermentation product; and
(c) recovering the fermentation product from the fermentation.

**4.** A method of fermenting a cellulosic material, comprising: fermenting the cellulosic material with one or more fermenting microorganisms, wherein the cellulosic material is saccharified with an enzyme composition comprising a cellulase in the presence of a GH61 polypeptide having cellulolytic enhancing activity and a liquor arising from treatment of a lignocellulose material, a hemicellulose material, a lignacious material, monosaccharides of the lignocellulose material, monosaccharides of the hemicellulose material, or a combination thereof, wherein the liquor is present in an amount that minimizes inhibition of a cellulase composition of 1 to 20% (v/v), wherein the combination of the GH61 polypeptide having cellulolytic enhancing activity and the liquor enhances hydrolysis of the cellulosic material by the enzyme composition, wherein the enhanced hydrolysis is assessed according to Equation 3:

$$GH61\ effect = \frac{\%\ conversion_{(+\ GH61+\ liquor)}}{\%\ conversion_{(no\ GH61+\ liquor)}} \quad \text{(Equation 3)}$$

and the equation ratio is greater than 1,
wherein the polypeptide having cellulolytic enhancing activity comprises the following motifs:
[ILMV]-P-X(4,5)-G-X-Y-[ILMV]-X-R-X-[EQ]-X(4)-[HNQ] (SEQ ID NO: 127 or SEQ ID NO: 128) and [FW]-[TF]-K-[AIV], wherein X is any amino acid, X(4,5) is any amino acid at 4 or 5 contiguous positions, and X(4) is any amino acid at 4 contiguous positions.

**5.** The method of claim 4, wherein the fermenting of the cellulosic material produces a fermentation product.

**6.** The method of claim 5, further comprising recovering the fermentation product from the fermentation.

**7.** The method of any of claims 1-6, wherein the enzyme composition further comprises one or more enzymes selected from the group consisting of a cellulase, a hemicellulase, an esterase, an expansin, a laccase, a ligninolytic enzyme, a pectinase, a peroxidase, a protease, and a swollenin.

**8.** The method of any of claims 1-7, wherein the pretreated cellulosic material is unwashed.

**9.** The method of any of claims 1-8, wherein the pretreated cellulosic material is washed.

**10.** The method of any of claims 1-9, wherein an effective amount of a polypeptide having cellulolytic enhancing activity to cellulosic material is 0.01 to 50.0 mg per g of cellulosic material.

**11.** The method of claims 1-10, wherein an effective amount of a polypeptide having cellulolytic enhancing activity to cellulolytic enzyme protein is about 0.005 to about 1.0 g per g of cellulolytic enzyme protein.

**Patentansprüche**

1. Verfahren zum Abbauen oder Umwandeln eines cellulosischen Materials, umfassend: Behandeln des cellulosischen Materials mit einer Enzymzusammensetzung, die eine Cellulase umfasst, in Gegenwart eines GH61-Polypeptids mit cellulolytisch verstärkender Aktivität und einer Lösung, die aus der Behandlung eines Lignocellulosematerials, eines Hemicellulosematerials, eines Ligninmaterials, Monosacchariden des Lignocellulosematerials, Monosacchariden des Hemicellulosematerials oder einer Kombination davon stammt, wobei die Lösung in einer Menge vorliegt, die Hemmung einer Cellulasezusammensetzung von 1 bis 20% (Vol./Vol.) minimiert, wobei die Kombination des GH61-Polypeptids mit cellulolytisch verstärkender Aktivität und der Lösung die Hydrolyse des cellulosischen Materials durch die Enzymzusammensetzung verstärkt, wobei die verstärkte Hydrolyse gemäß Gleichung 3 bewertet wird:

$$\textit{GH61-Wirkung} = \frac{\%\ \text{Umwandlung}_{(+\ \text{GH61+ Lösung})}}{\%\ \text{Umwandlung}_{(\text{kein GH61+ Lösung})}} \qquad \text{(Gleichung 3)}$$

und das Gleichungsverhältnis größer als 1 ist,
wobei das GH61-Polypeptid mit cellulolytisch verstärkender Aktivität die folgenden Motive umfasst:
[ILMV]-P-X(4,5)-G-X-Y-[ILMV]-X-R-X-[EQ]-X(4)-[HNQ] (SEQ ID NO: 127 oder SEQ ID NO: 128) und [FW]-[TF]-K-[AIV], wobei X eine beliebige Aminosäure ist, X(4,5) eine beliebige Aminosäure an 4 oder 5 zusammenhängenden Positionen ist, und X(4) eine beliebige Aminosäure an 4 zusammenhängenden Positionen ist.

2. Verfahren nach Anspruch 1, das des Weiteren Gewinnen des abgebauten cellulosischen Materials umfasst.

3. Verfahren zum Herstellen eines Fermentationsprodukts, umfassend:

(a) Verzuckern eines cellulosischen Materials mit einer Enzymzusammensetzung, die eine Cellulase umfasst, in Gegenwart eines GH61-Polypeptids mit cellulolytisch verstärkender Aktivität und einer Lösung, die aus der Behandlung eines Lignocellulosematerials, eines Hemicellulosematerials, eines Ligninmaterials, Monosacchariden des Lignocellulosematerials, Monosacchariden des Hemicellulosematerials oder einer Kombination davon stammt, wobei die Lösung in einer Menge vorhanden ist, die Hemmung einer Cellulasezusammensetzung von etwa 1 bis etwa 20% (Vol./Vol.) minimiert, wobei die Lösung in einer Menge vorhanden ist, die Hemmung einer Cellulasezusammensetzung von 1 bis 20% (Vol./Vol.) minimiert, wobei die Kombination des GH61-Polypeptids mit cellulolytisch verstärkender Aktivität und der Lösung die Hydrolyse des cellulosischen Materials durch die Enzymzusammensetzung verstärkt, wobei die verstärkte Hydrolyse gemäß Gleichung 3 bewertet wird:

$$\textit{GH61-Wirkung} = \frac{\%\ \text{Umwandlung}_{(+\ \text{GH61+ Lösung})}}{\%\ \text{Umwandlung}_{(\text{kein GH61+ Lösung})}} \qquad \text{(Gleichung 3)}$$

und das Gleichungsverhältnis größer als 1 ist, wobei das Polypeptid mit cellulolytisch verstärkender Aktivität die folgenden Motive umfasst:
[ILMV]-P-X(4,5)-G-X-Y-[ILMV]-X-R-X-[EQ]-X(4)-[HNQ] (SEQ ID NO: 127 oder SEQ ID NO: 128) und [FW]-[TF]-K-[AIV], wobei X eine beliebige Aminosäure ist, X(4,5) eine beliebige Aminosäure an 4 oder 5 zusammenhängenden Positionen ist, und X(4) eine beliebige Aminosäure an 4 zusammenhängenden Positionen ist;
(b) Fermentieren des verzuckerten cellulosischen Materials mit einem oder mehreren fermentierenden Mikroorganismen, um das Fermentationsprodukt herzustellen; und
(c) Gewinnen des Fermentationsprodukts aus der Fermentation.

4. Verfahren zum Fermentieren eines cellulosischen Materials, umfassend: Fermentieren des cellulosischen Materials mit einem oder mehreren fermentierenden Mikroorganismen, wobei das cellulosische Material mit einer Enzymzusammensetzung, die eine Cellulase umfasst, in Gegenwart eines GH61-Polypeptids mit cellulolytisch verstärkender Aktivität und einer Lösung verzuckert wird, die aus der Behandlung eines Lignocellulosematerials, eines Hemicellulosematerials, eines Ligninmaterials, Monosacchariden des Lignocellulosematerials, Monosacchariden des Hemicellulosematerials oder einer Kombination davon stammt, wobei die Lösung in einer Menge vorhanden ist, die Hemmung einer Cellulasezusammensetzung von 1 bis 20% (Vol./Vol.) minimiert, wobei die Kombination des GH61-Polypeptids mit cellulolytisch verstärkender Aktivität und der Lösung die Hydrolyse des cellulosischen Materials durch die Enzymzusammensetzung verstärkt, wobei die verstärkte Hydrolyse gemäß Gleichung 3 bewertet wird:

$$GH61\text{-}Wirkung = \frac{\%\ \text{Umwandlung}_{(+\ \text{GH61+ Lösung})}}{\%\ \text{Umwandlung}_{(\text{kein GH61+ Lösung})}} \qquad \text{(Gleichung 3)}$$

und das Gleichungsverhältnis größer als 1 ist,
wobei das Polypeptid mit cellulolytisch verstärkender Aktivität die folgenden Motive umfasst:
[ILMV]-P-X(4,5)-G-X-Y-[ILMV]-X-R-X-[EQ]-X(4)-[HNQ] (SEQ ID NO: 127 oder SEQ ID NO: 128) und [FW]-[TF]-K-[AIV],
wobei X eine beliebige Aminosäure ist, X(4,5) eine beliebige Aminosäure an 4 oder 5 zusammenhängenden Positionen ist, und X(4) eine beliebige Aminosäure an 4 zusammenhängenden Positionen ist.

**5.** Verfahren nach Anspruch 4, wobei das Fermentieren des cellulosischen Materials ein Fermentationsprodukt herstellt.

**6.** Verfahren nach Anspruch 5, das des Weiteren Gewinnen des Fermentationsprodukts aus der Fermentation umfasst.

**7.** Verfahren nach einem beliebigen der Ansprüche 1-6, wobei die Enzymzusammensetzung des Weiteren ein oder mehrere Enzyme umfasst, die aus der Gruppe bestehend aus einer Cellulase, einer Hemicellulase, einer Esterase, einem Expansin, einer Laccase, einem ligninolytischen Enzym, einer Pectinase, einer Peroxidase, einer Protease und einem Swollenin ausgewählt sind.

**8.** Verfahren nach einem beliebigen der Ansprüche 1-7, wobei das vorbehandelte cellulosische Material ungewaschen ist.

**9.** Verfahren nach einem beliebigen der Ansprüche 1-8, wobei das vorbehandelte cellulosische Material gewaschen ist.

**10.** Verfahren nach einem beliebigen der Ansprüche 1-9, wobei eine wirksame Menge eines Polypeptids mit cellulolytisch verstärkender Aktivität gegenüber cellulosischem Material 0,01 bis 50,0 mg pro g cellulosischem Material beträgt.

**11.** Verfahren nach Ansprüchen 1-10, wobei eine wirksame Menge eines Polypeptids mit cellulolytisch verstärkender Aktivität gegenüber cellulolytischem Enzymprotein etwa 0,005 bis etwa 1,0 g pro g cellulolytischem Enzymprotein beträgt.

## Revendications

**1.** Méthode pour dégrader ou convertir un matériau cellulosique, comprenant : le traitement du matériau cellulosique avec une composition enzymatique comprenant une cellulase en présence d'un polypeptide GH61 ayant une activité d'amplification cellulolytique et d'une liqueur issue du traitement d'un matériau de type lignocellulose, d'un matériau de type hémicellulose, d'un matériau ligninacé, de monosaccharides du matériau de type lignocellulose, de monosaccharides du matériau de type hémicellulose, ou d'une de leurs combinaisons, dans laquelle la liqueur est présente en une quantité qui minimise l'inhibition d'une composition de cellulase de 1 à 20 % (v/v), dans laquelle la combinaison du polypeptide GH61 ayant une activité d'amplification cellulolytique et de la liqueur amplifie l'hydrolyse du matériau cellulosique par la composition enzymatique, dans laquelle l'hydrolyse amplifiée est déterminée conformément à l'équation 3 :

$$\text{Effet de GH61} = \frac{\%\ \text{conversion}_{(+\ \text{GH61 + liqueur})}}{\%\ \text{conversion}_{(\text{sans GH61 + liqueur})}} \qquad \text{(Equation 3)}$$

et le rapport de l'équation est supérieur à 1,
dans laquelle le polypeptide GH61 ayant une activité d'amplification cellulolytique comprend les motifs suivants : [ILMV]-P-X(4,5)-G-X-Y-[ILMV]-X-R-X-[EQ]-X(4)-[HNQ] (SEQ ID NO: 127 ou SEQ ID NO : 128) et [FW]-[TF]-K-[AIV], où X est un acide aminé quelconque, X(4,5) est un acide aminé quelconque en 4 ou 5 positions contiguës, et X(4) est un acide aminé quelconque en 4 positions contiguës.

2. Méthode selon la revendication 1, comprenant en outre la récupération du matériau cellulosique dégradé.

3. Méthode pour produire un produit de fermentation, comprenant :

(a) la saccharification d'un matériau cellulosique avec une composition enzymatique comprenant une cellulase en présence d'un polypeptide GH61 ayant une activité d'amplification cellulolytique et d'une liqueur issue du traitement d'un matériau de type lignocellulose, d'un matériau de type hémicellulose, d'un matériau ligninacé, de monosaccharides du matériau de type lignocellulose, de monosaccharides du matériau de type hémicellulose, ou d'une de leurs combinaisons, dans laquelle la liqueur est présente en une quantité qui minimise l'inhibition d'une composition de cellulase d'environ 1 à environ 20 % (v/v), dans laquelle la liqueur est présente en une quantité qui minimise l'inhibition d'une composition de cellulase de 1 à 20 % (v/v), dans laquelle la combinaison du polypeptide GH61 ayant une activité d'amplification cellulolytique et de la liqueur amplifie l'hydrolyse du matériau cellulosique par la composition enzymatique, dans laquelle l'hydrolyse amplifiée est déterminée conformément à l'équation 3 :

$$\text{Effet de GH61} = \frac{\%\ \text{conversion}_{(+\ \text{GH61}\ +\ \text{liqueur})}}{\%\ \text{conversion}_{(\text{sans GH61}\ +\ \text{liqueur})}} \qquad \text{(Equation 3)}$$

et le rapport de l'équation est supérieur à 1,
dans laquelle le polypeptide ayant une activité d'amplification cellulolytique comprend les motifs suivants : [ILMV]-P-X(4,5)-G-X-Y-[ILMV]-X-R-X-[EQ]-X(4)-[HNQ] (SEQ ID NO: 127 ou SEQ ID NO : 128) et [FW]-[TF]-K-[AIV], où X est un acide aminé quelconque, X(4,5) est un acide aminé quelconque en 4 ou 5 positions contiguës, et X(4) est un acide aminé quelconque en 4 positions contiguës ;
(b) la fermentation du matériau cellulosique saccharifié avec un ou plusieurs microorganismes de fermentation pour produire le produit de fermentation ; et
(c) la récupération du produit de fermentation à partir de la fermentation.

4. Méthode de fermentation d'un matériau cellulosique, comprenant : la fermentation du matériau cellulosique avec un ou plusieurs microorganismes de fermentation, dans laquelle le matériau cellulosique est saccharifié avec une composition enzymatique comprenant une cellulase en présence d'un polypeptide GH61 ayant une activité d'amplification cellulolytique et d'une liqueur issue du traitement d'un matériau de type lignocellulose, d'un matériau de type hémicellulose, d'un matériau ligninacé, de monosaccharides du matériau de type lignocellulose, de monosaccharides du matériau de type hémicellulose, ou d'une de leurs combinaisons, dans laquelle la liqueur est présente en une quantité qui minimise l'inhibition d'une composition de cellulase de 1 à 20 % (v/v), dans laquelle la combinaison du polypeptide GH61 ayant une activité d'amplification cellulolytique et de la liqueur amplifie l'hydrolyse du matériau cellulosique par la composition enzymatique, dans laquelle l'hydrolyse amplifiée est déterminée conformément à l'équation 3 :

$$\text{Effet de GH61} = \frac{\%\ \text{conversion}_{(+\ \text{GH61}\ +\ \text{liqueur})}}{\%\ \text{conversion}_{(\text{sans GH61}\ +\ \text{liqueur})}} \qquad \text{(Equation 3)}$$

et le rapport de l'équation est supérieur à 1,
dans laquelle le polypeptide ayant une activité d'amplification cellulolytique comprend les motifs suivants : [ILMV]-P-X(4,5)-G-X-Y-[ILMV]-X-R-X-[EQ]-X(4)-[HNQ] (SEQ ID NO: 127 ou SEQ ID NO : 128) et [FW]-[TF]-K-[AIV], où X est un acide aminé quelconque, X(4,5) est un acide aminé quelconque en 4 ou 5 positions contiguës, et X(4) est un acide aminé quelconque en 4 positions contiguës.

5. Méthode selon la revendication 4, dans laquelle la fermentation du matériau cellulosique produit un produit de fermentation.

6. Méthode selon la revendication 5, comprenant en outre la récupération du produit de fermentation à partir de la fermentation.

7. Méthode selon l'une quelconque des revendications 1 à 6, dans laquelle la composition enzymatique comprend en outre une ou plusieurs enzymes choisies dans le groupe constitué par une cellulase, une hémicellulase, une estérase, une expansine, une laccase, une enzyme ligninolytique, une pectinase, une peroxydase, une protéase, et une swollénine.

8. Méthode selon l'une quelconque des revendications 1 à 7, dans laquelle le matériau cellulosique prétraité n'est pas lavé.

9. Méthode selon l'une quelconque des revendications 1 à 8, dans laquelle le matériau cellulosique prétraité est lavé.

10. Méthode selon l'une quelconque des revendications 1 à 9, dans laquelle la quantité efficace d'un polypeptide ayant une activité d'amplification cellulolytique vis-à-vis du matériau cellulosique est de 0,01 à 50,0 mg par g de matériau cellulosique.

11. Méthode selon l'une quelconque des revendications 1 à 10, dans laquelle la quantité efficace d'un polypeptide ayant une activité d'amplification cellulolytique vis-à-vis d'une protéine enzymatique cellulolytique est d'environ 0,005 à environ 1,0 g par g de protéine enzymatique cellulolytique.

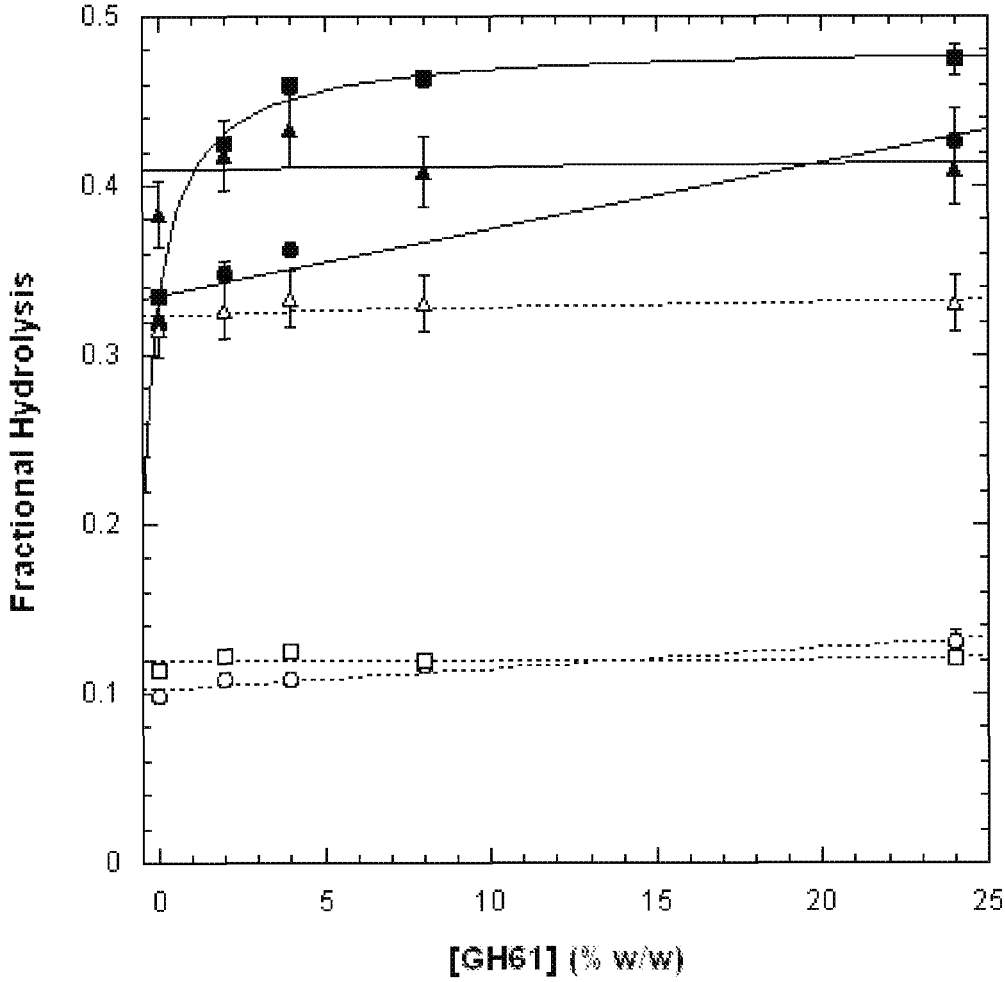


# Fig. 1

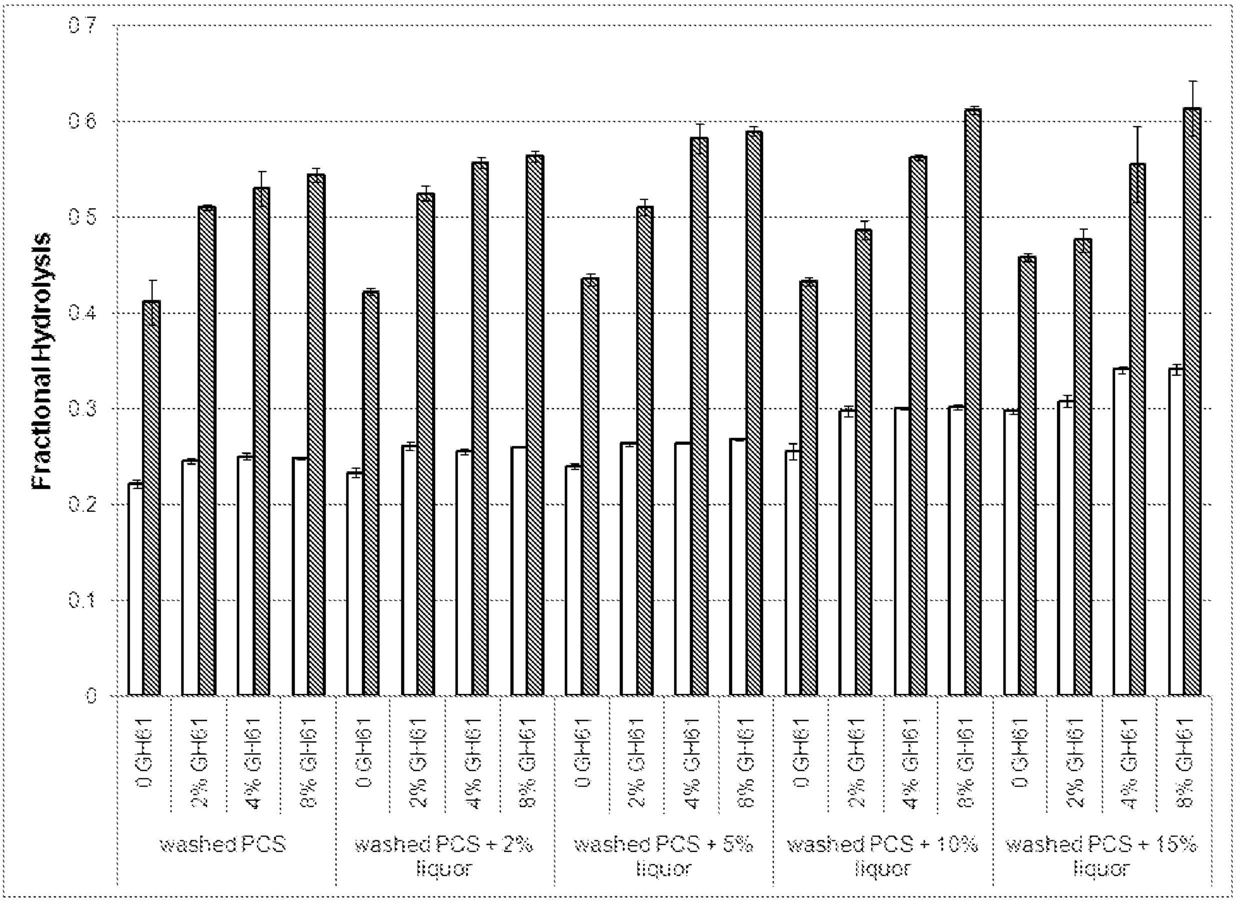
Fractional Hydrolysis
0.7
0.6
0.5
0.4
0.3
0.2
0.1
0
0 GH61
2% GH61
4% GH61
8% GH61
washed PCS
washed PCS + 2% liquor
washed PCS + 5% liquor
washed PCS + 10% liquor
washed PCS + 15% liquor

Fig. 2A

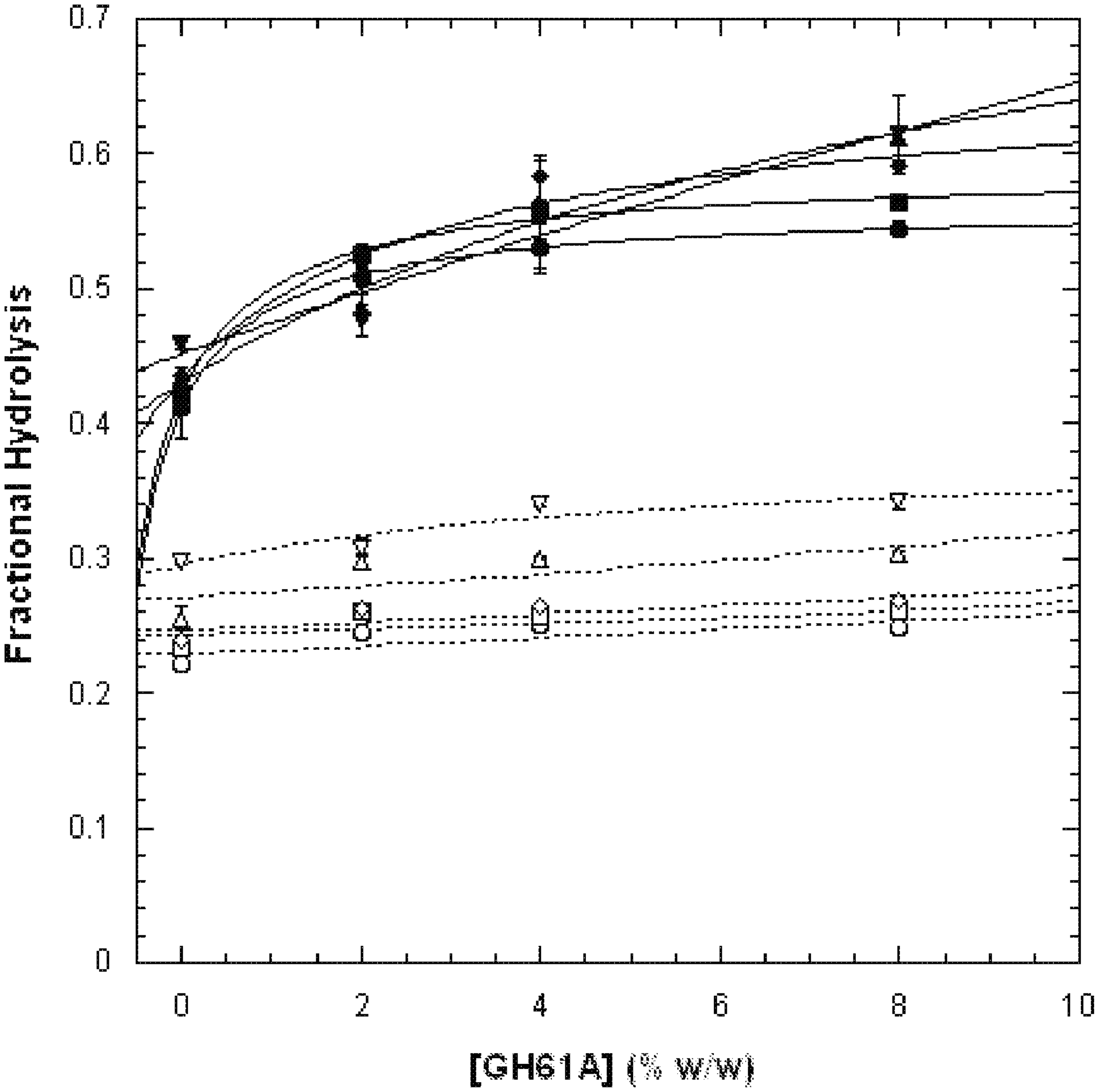


Fig. 2B

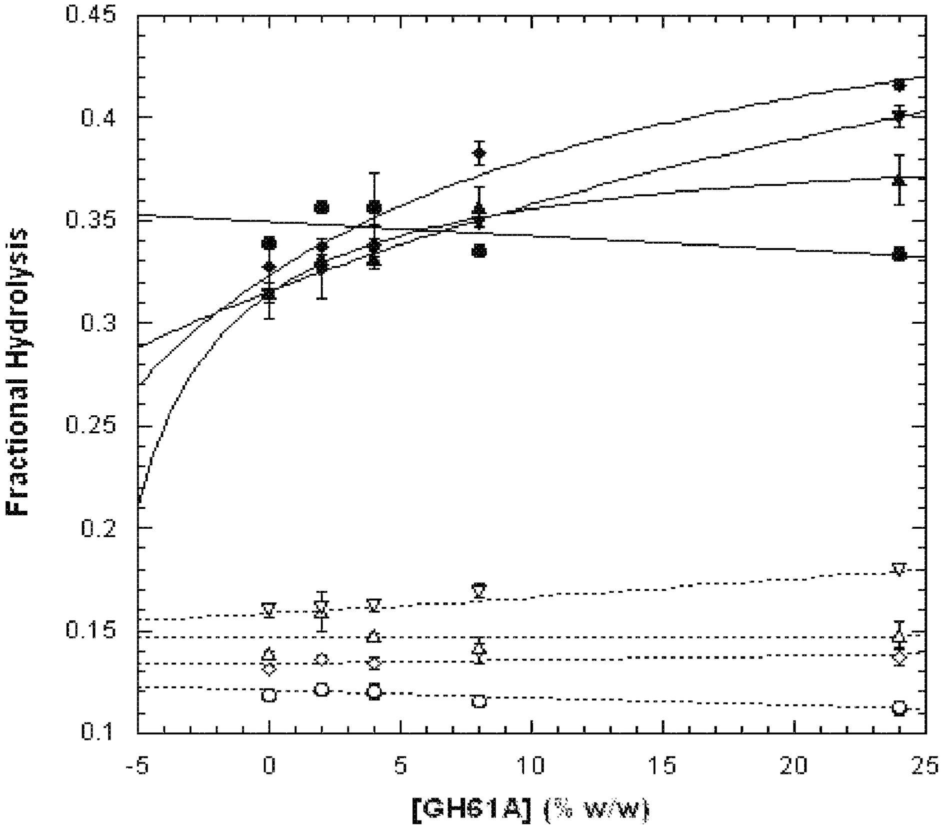

Fractional Hydrolysis
[GH61A] (% w/w)


Fig. 3A

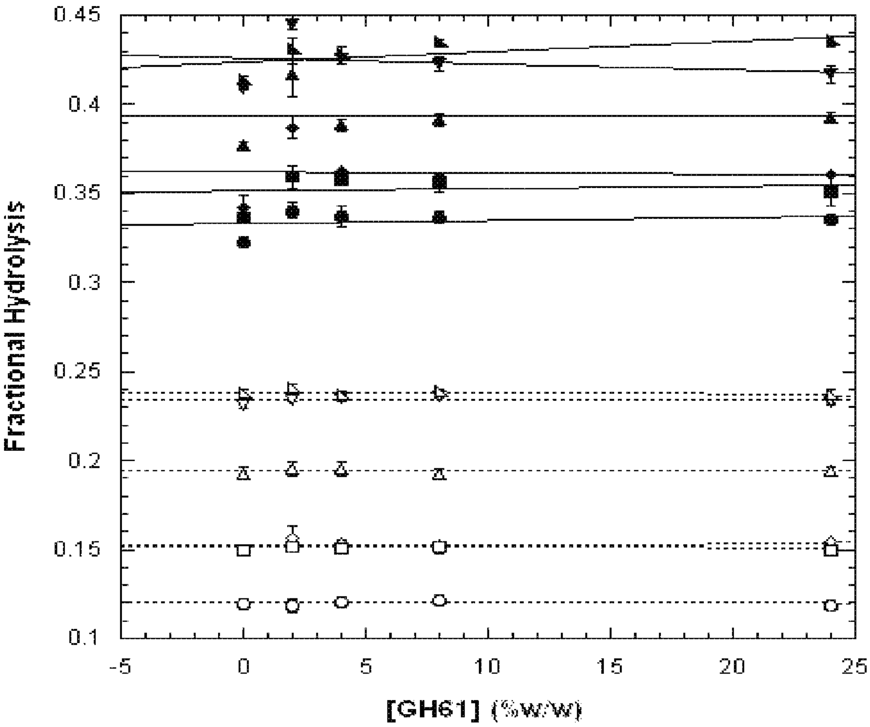

Fractional Hydrolysis
[GH61] (%w/w)


Fig. 3B

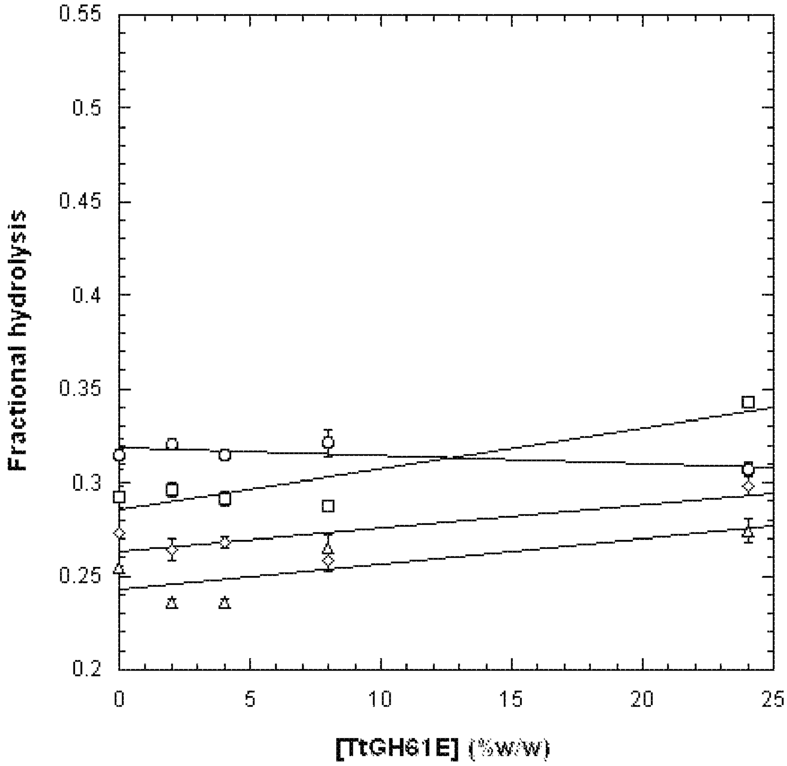
0.55
0.5
0.45
0.4
0.35
0.3
0.25
0.2
Fractional hydrolysis
0
5
10
15
20
25
[TtGH61E] (%w/w)

Fig. 4A

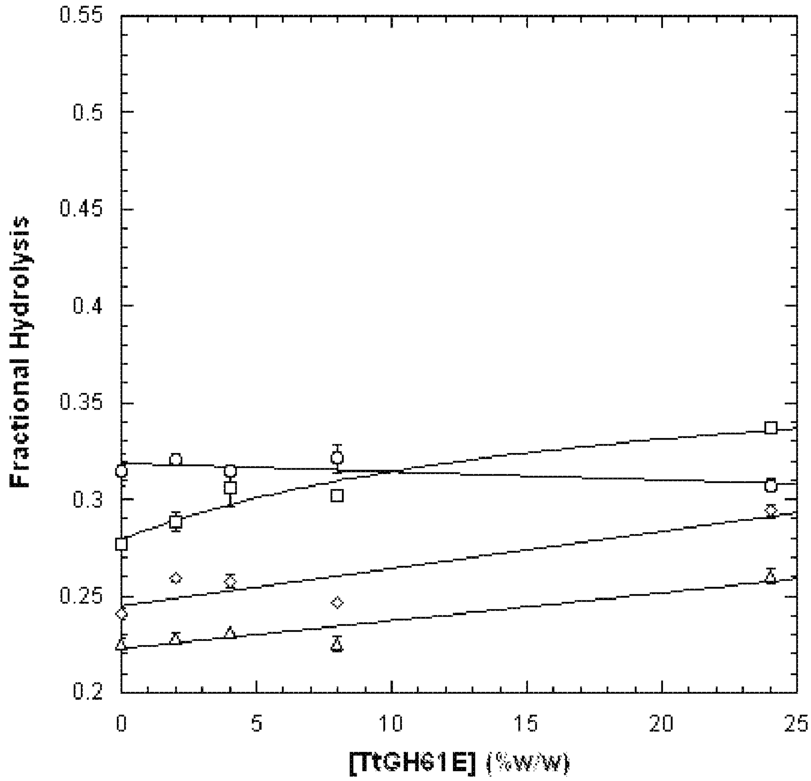
0.55
0.5
0.45
0.4
0.35
0.3
0.25
0.2
Fractional Hydrolysis
0
5
10
15
20
25
[TtGH61E] (%w/w)

Fig. 4B

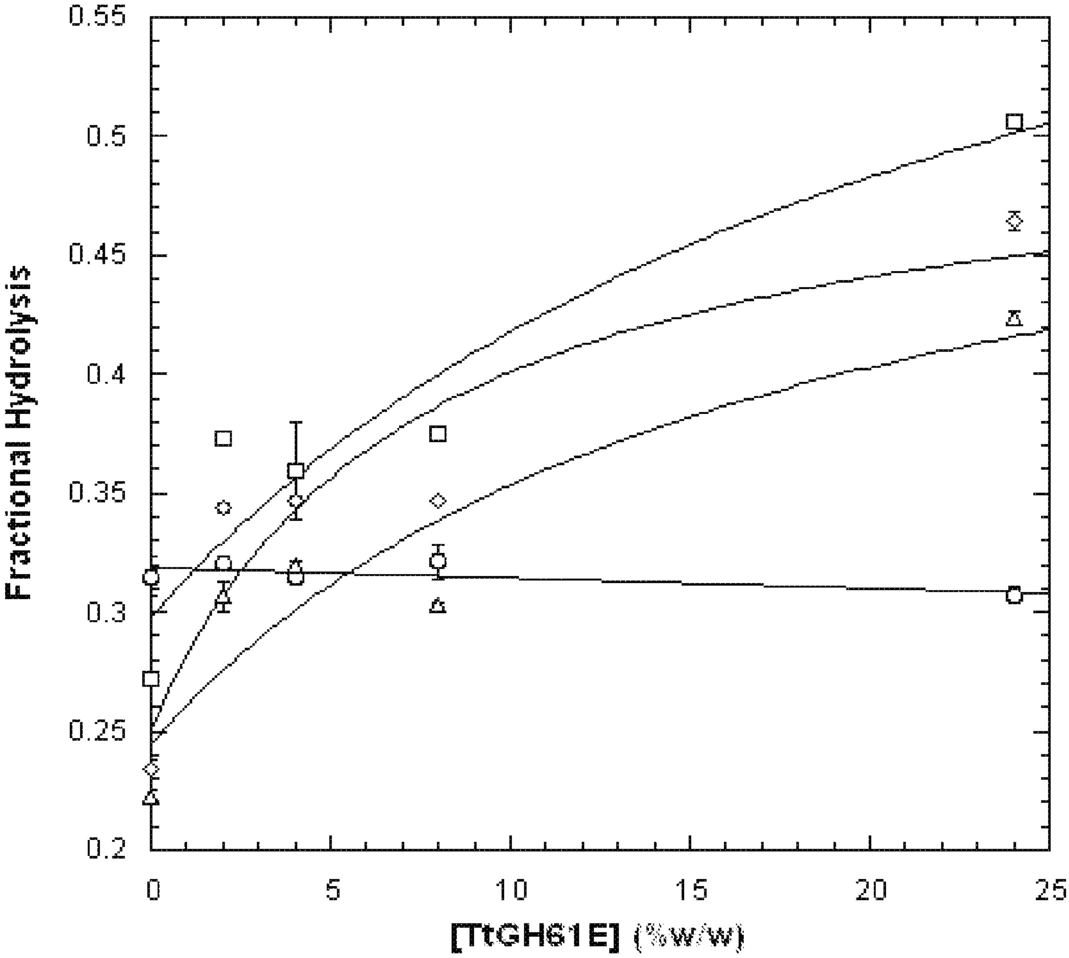
0.55
0.5
0.45
0.4
0.35
0.3
0.25
0.2
Fractional Hydrolysis
0
5
10
15
20
25
[TtGH61E] (%w/w)

Fig. 4C

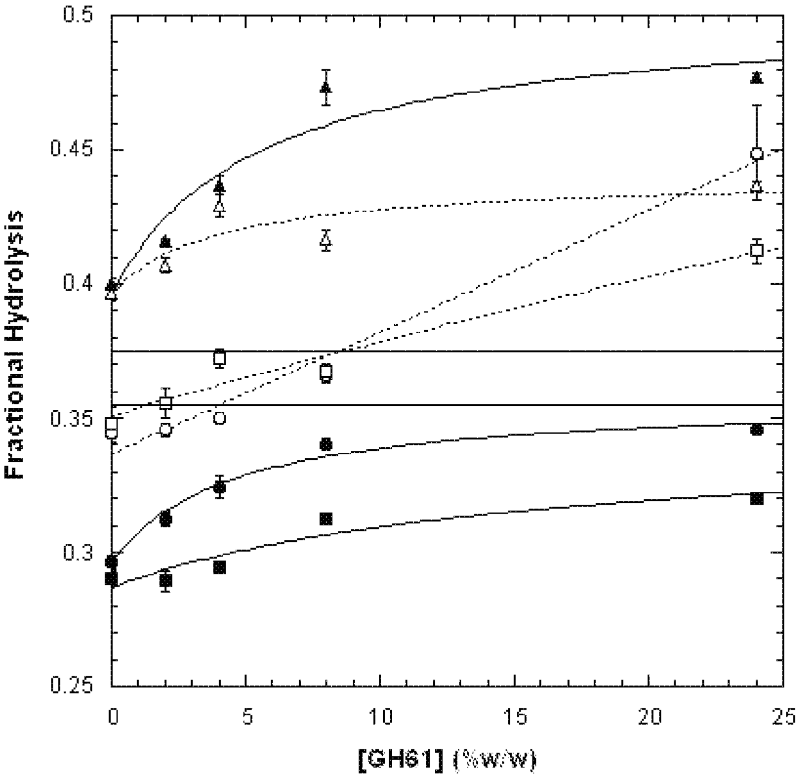
Fractional Hydrolysis
0.5
0.45
0.4
0.35
0.3
0.25
0
5
10
15
20
25
[GH61] (%w/w)

Fig. 5A

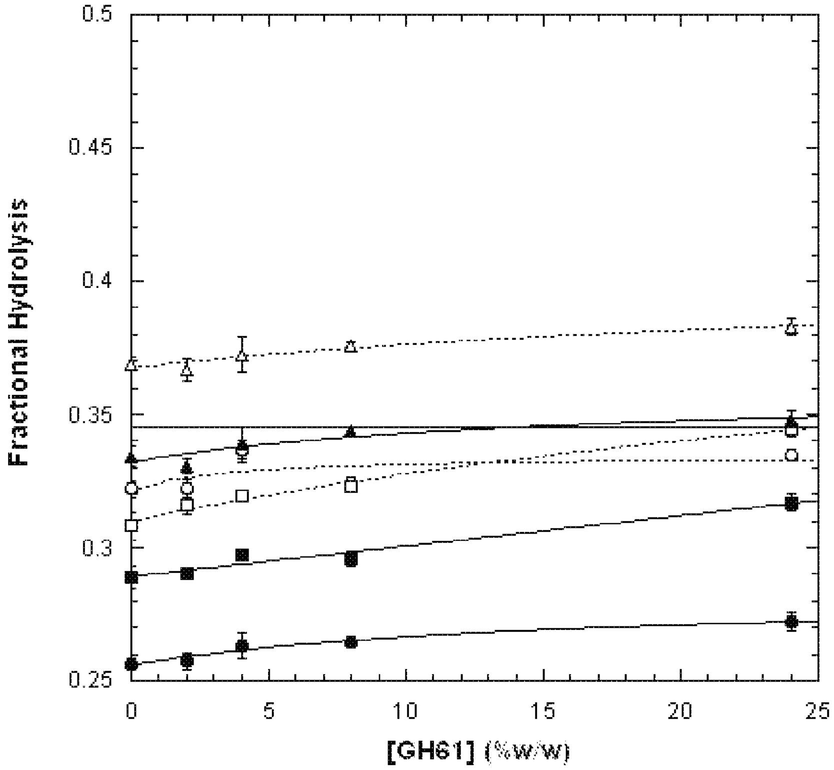
Fractional Hydrolysis
0.5
0.45
0.4
0.35
0.3
0.25
0
5
10
15
20
25
[GH61] (%w/w)

Fig. 5B

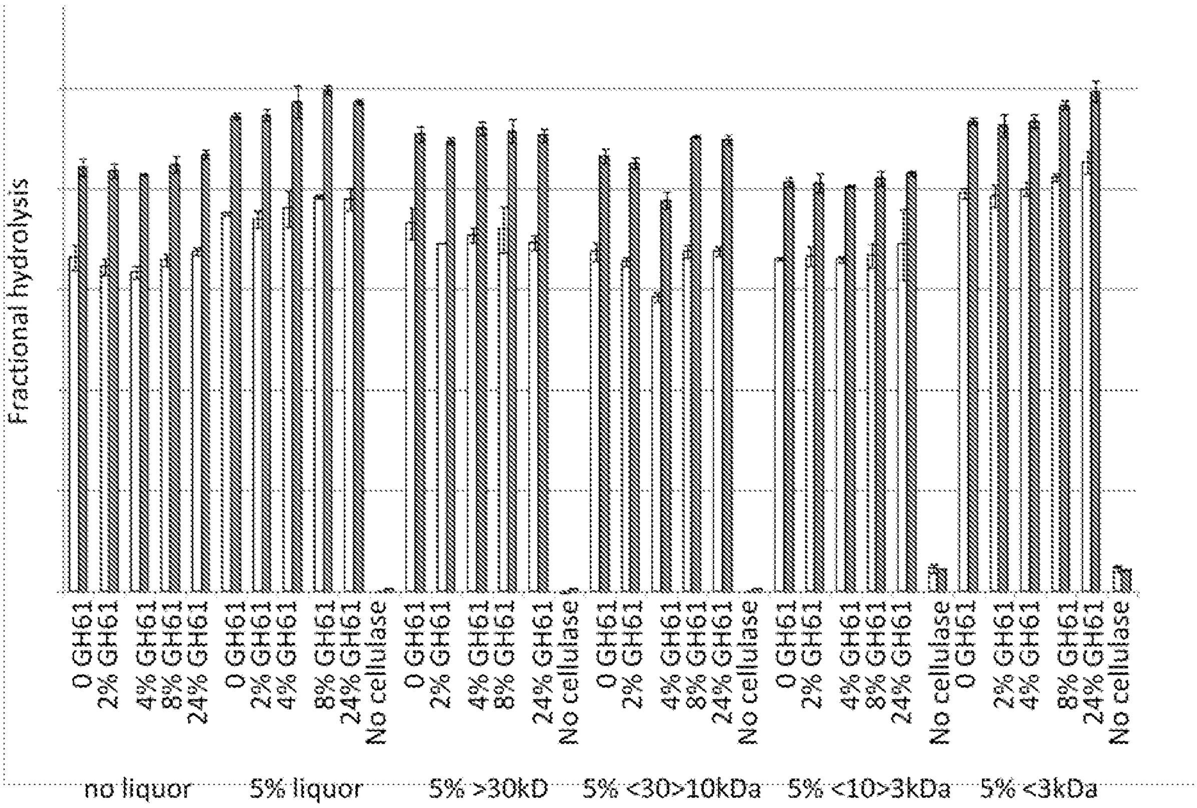
Fractional hydrolysis
0 GH61
2% GH61
4% GH61
8% GH61
24% GH61
No cellulase
no liquor
5% liquor
5% >30kD
5% <30>10kDa
5% <10>3kDa
5% <3kDa

Fig. 6

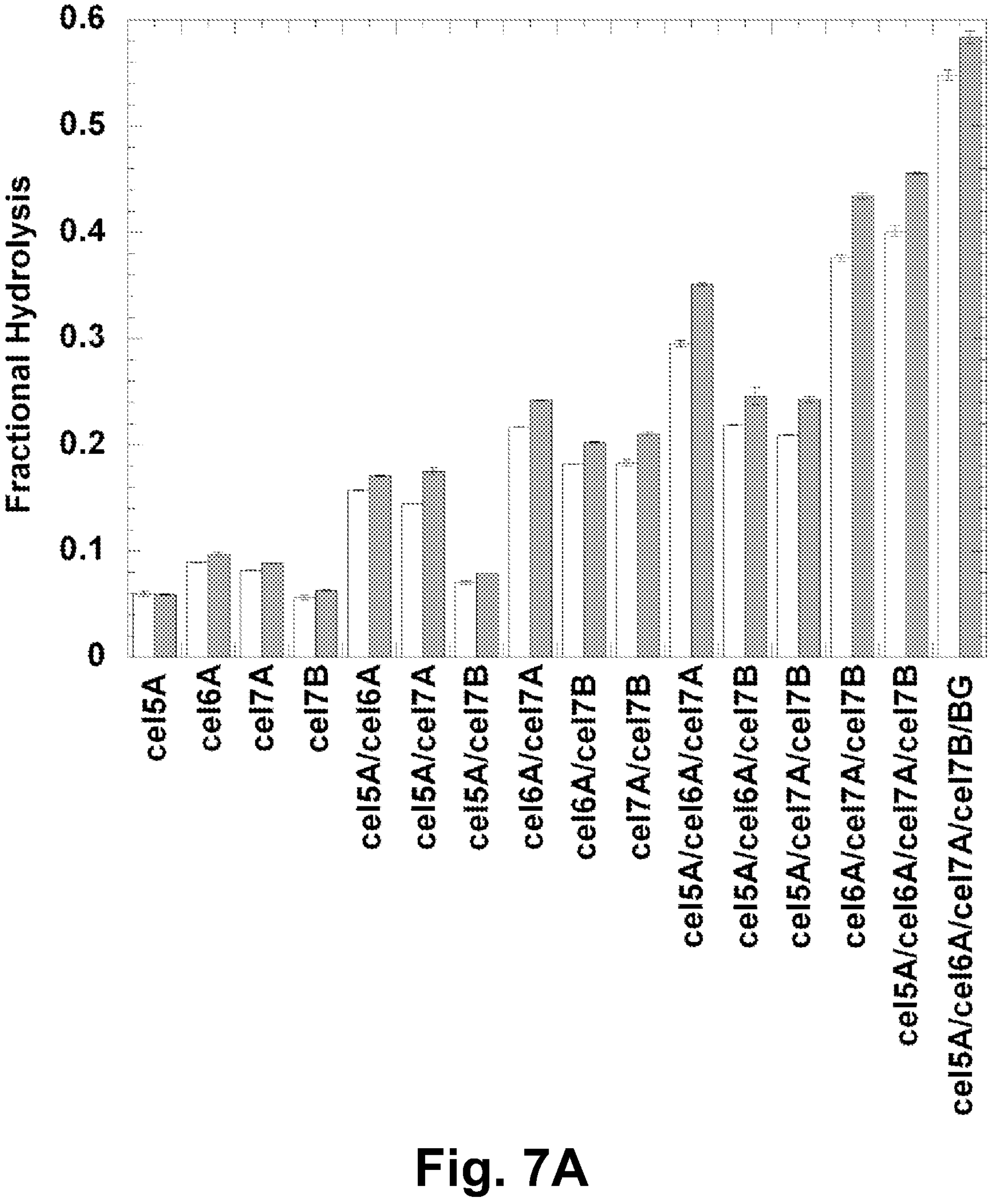
0.6
0.5
0.4
0.3
0.2
0.1
0
Fractional Hydrolysis
cel5A
cel6A
cel7A
cel7B
cel5A/cel6A
cel5A/cel7A
cel5A/cel7B
cel6A/cel7A
cel6A/cel7B
cel7A/cel7B
cel5A/cel6A/cel7A
cel5A/cel6A/cel7B
cel5A/cel7A/cel7B
cel6A/cel7A/cel7B
cel5A/cel6A/cel7A/cel7B
cel5A/cel6A/cel7A/cel7B/BG

Fig. 7A

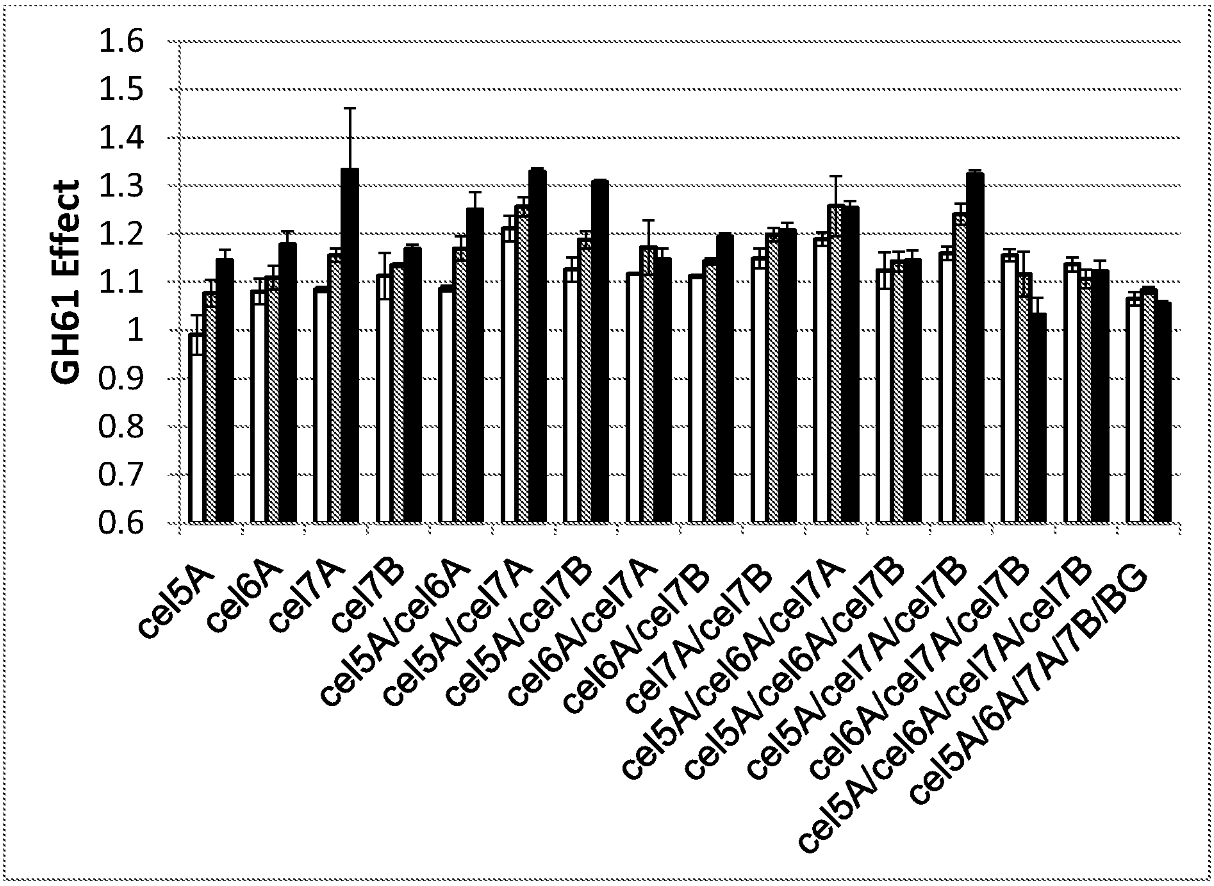
GH61 Effect
1.6
1.5
1.4
1.3
1.2
1.1
1
0.9
0.8
0.7
0.6
cel5A
cel6A
cel7A
cel7B
cel5A/cel6A
cel5A/cel7A
cel5A/cel7B
cel6A/cel7A
cel6A/cel7B
cel7A/cel7B
cel5A/cel6A/cel7A
cel5A/cel6A/cel7B
cel5A/cel7A/cel7B
cel6A/cel7A/cel7B
cel5A/cel6A/cel7A/cel7B
cel5A/6A/7A/7B/BG

Fig. 7B

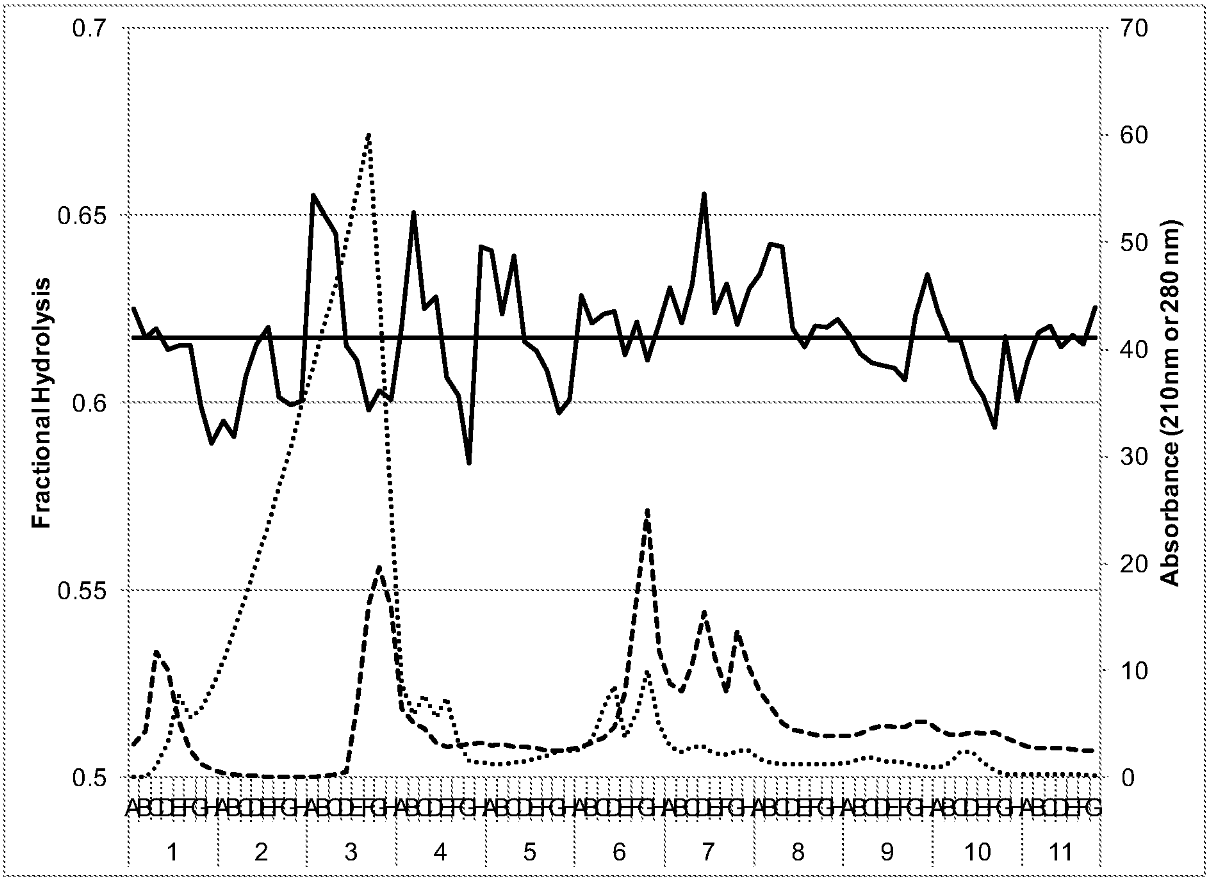
Fractional Hydrolysis
Absorbance (210nm or 280 nm)
0.7
0.65
0.6
0.55
0.5
70
60
50
40
30
20
10
0
1
2
3
4
5
6
7
8
9
10
11

# Fig. 8A

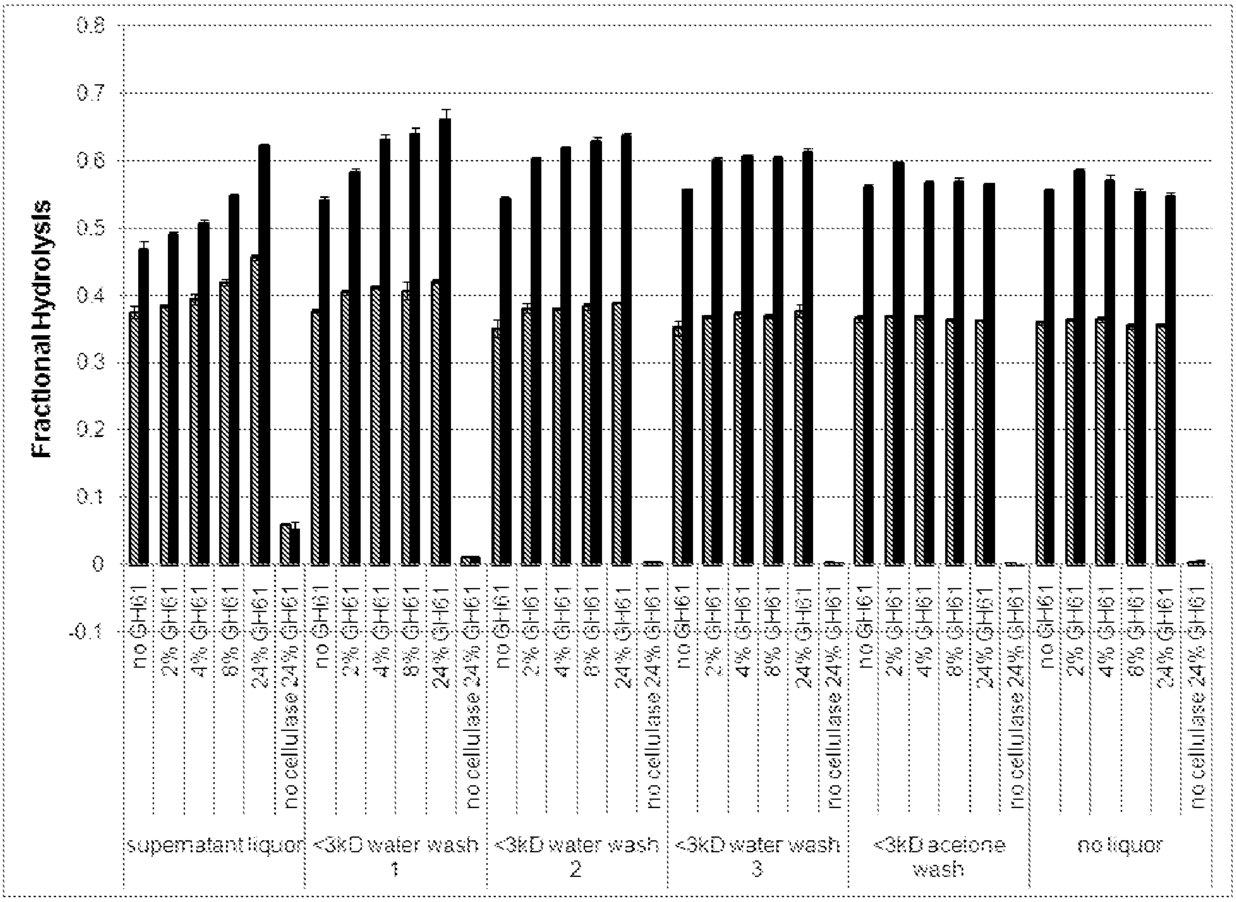
Fractional Hydrolysis
0.8
0.7
0.6
0.5
0.4
0.3
0.2
0.1
0
-0.1
no GH61
2% GH61
4% GH61
8% GH61
24% GH61
no cellulase 24% GH61
supernatant liquor
<3kD water wash 1
<3kD water wash 2
<3kD water wash 3
<3kD acetone wash
no liquor

Fig. 8B

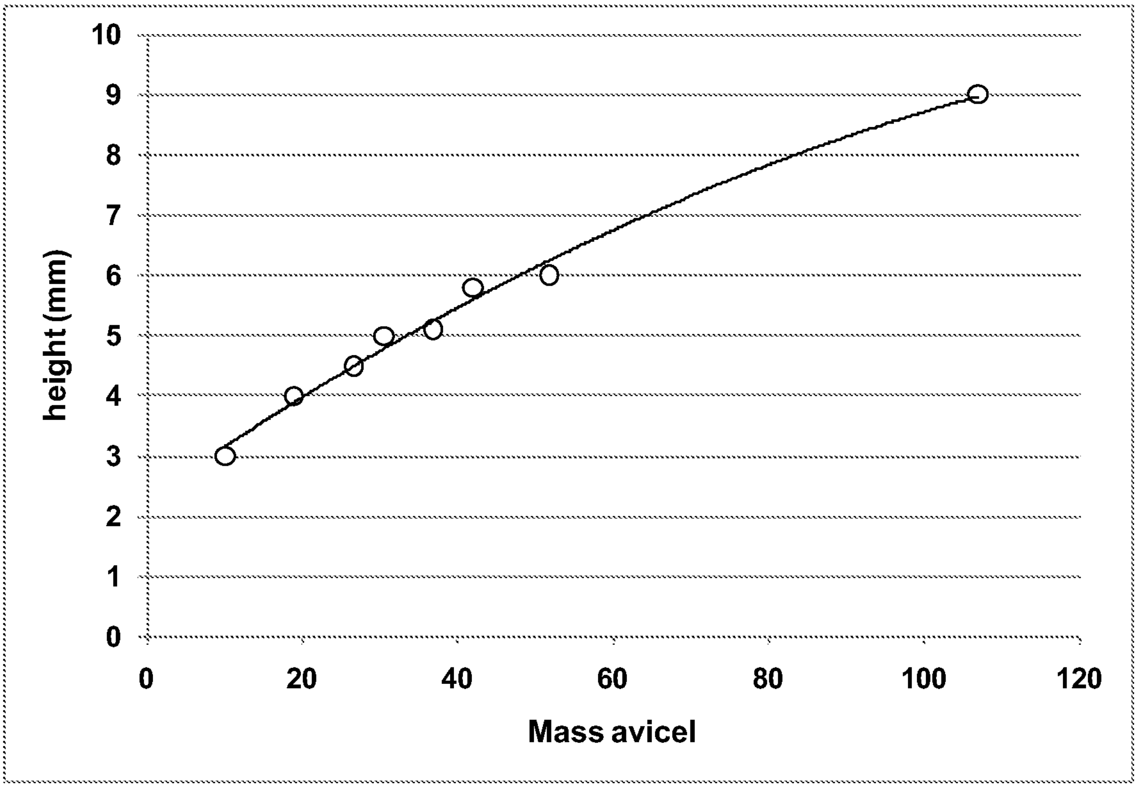


Fig. 9

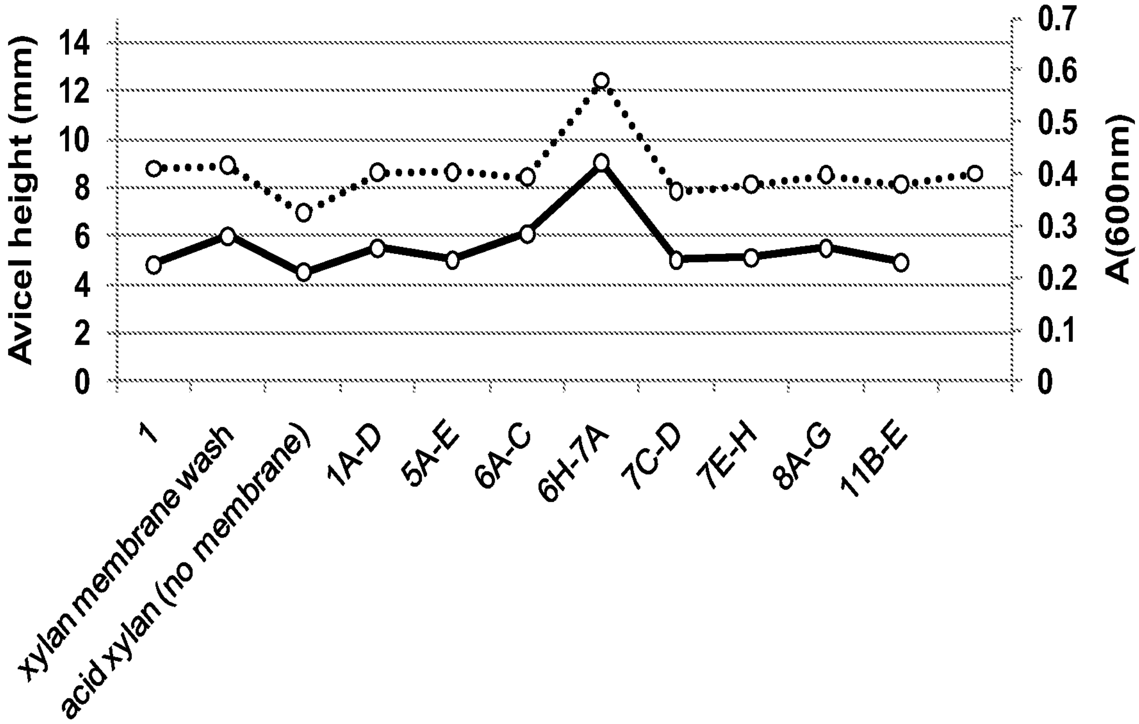
Avicel height (mm)
14
12
10
8
6
4
2
0
0.7
0.6
0.5
0.4
0.3
0.2
0.1
0
A(600nm)
1
xylan membrane wash
acid xylan (no membrane)
1A-D
5A-E
6A-C
6H-7A
7C-D
7E-H
8A-G
11B-E

Fig. 10

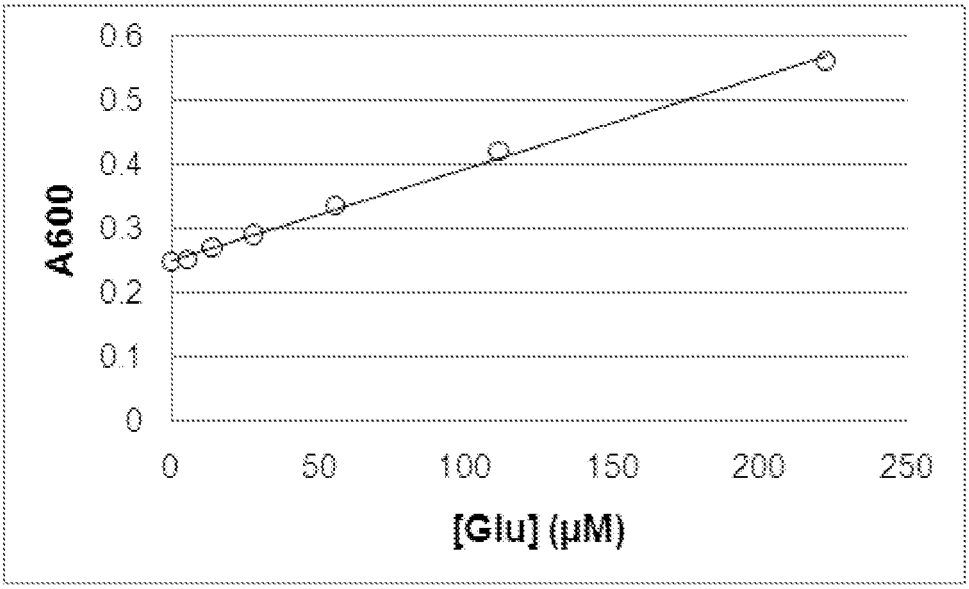


Fig. 11A

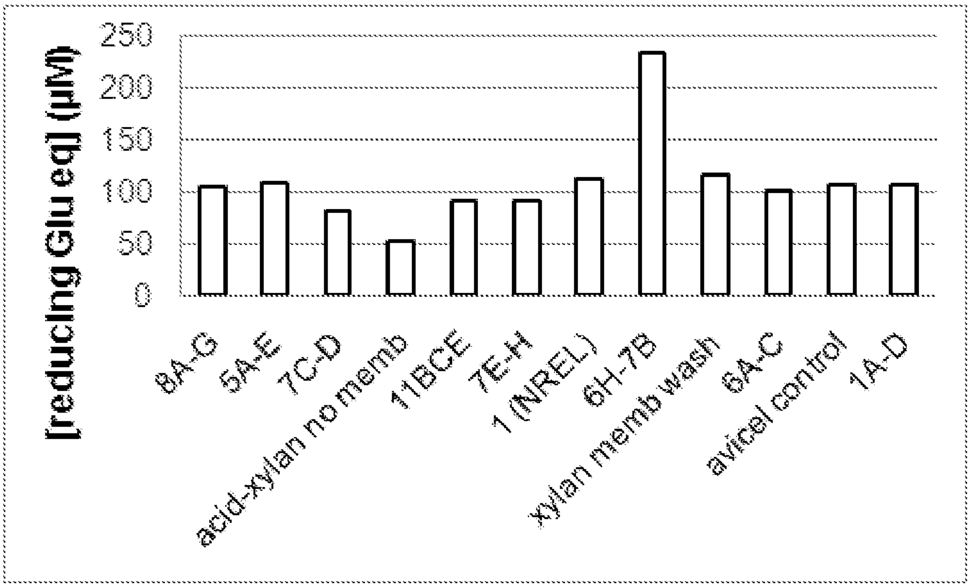


Fig. 11B

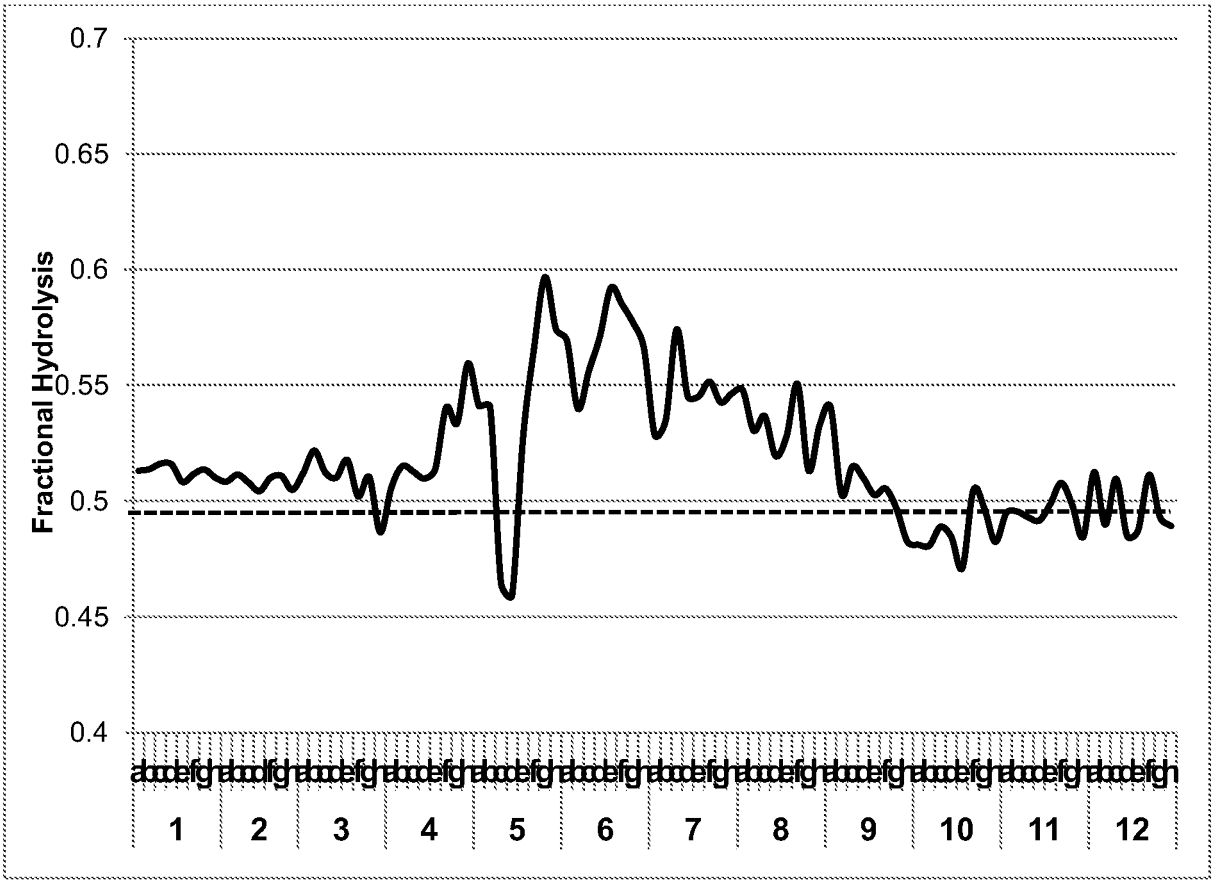
0.7
0.65
0.6
0.55
0.5
0.45
0.4
Fractional Hydrolysis
abcdefgh abcdefgh abcdefgh abcdefgh abcdefgh abcdefgh abcdefgh abcdefgh abcdefgh abcdefgh abcdefgh abcdefgh
1 2 3 4 5 6 7 8 9 10 11 12

Fig. 12

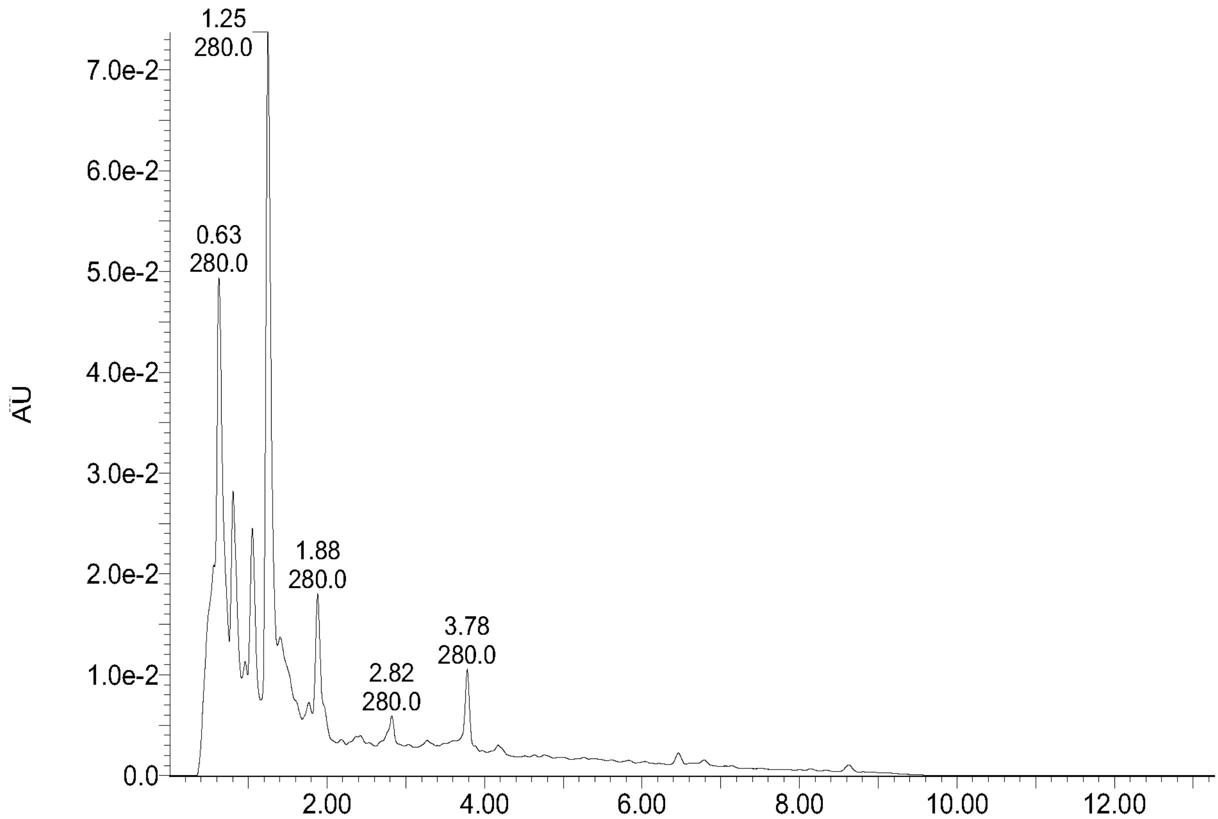
1.25
280.0
0.63
280.0
1.88
280.0
2.82
280.0
3.78
280.0
7.0e-2
6.0e-2
5.0e-2
4.0e-2
3.0e-2
2.0e-2
1.0e-2
0.0
AU
2.00
4.00
6.00
8.00
10.00
12.00

**Fig. 13**

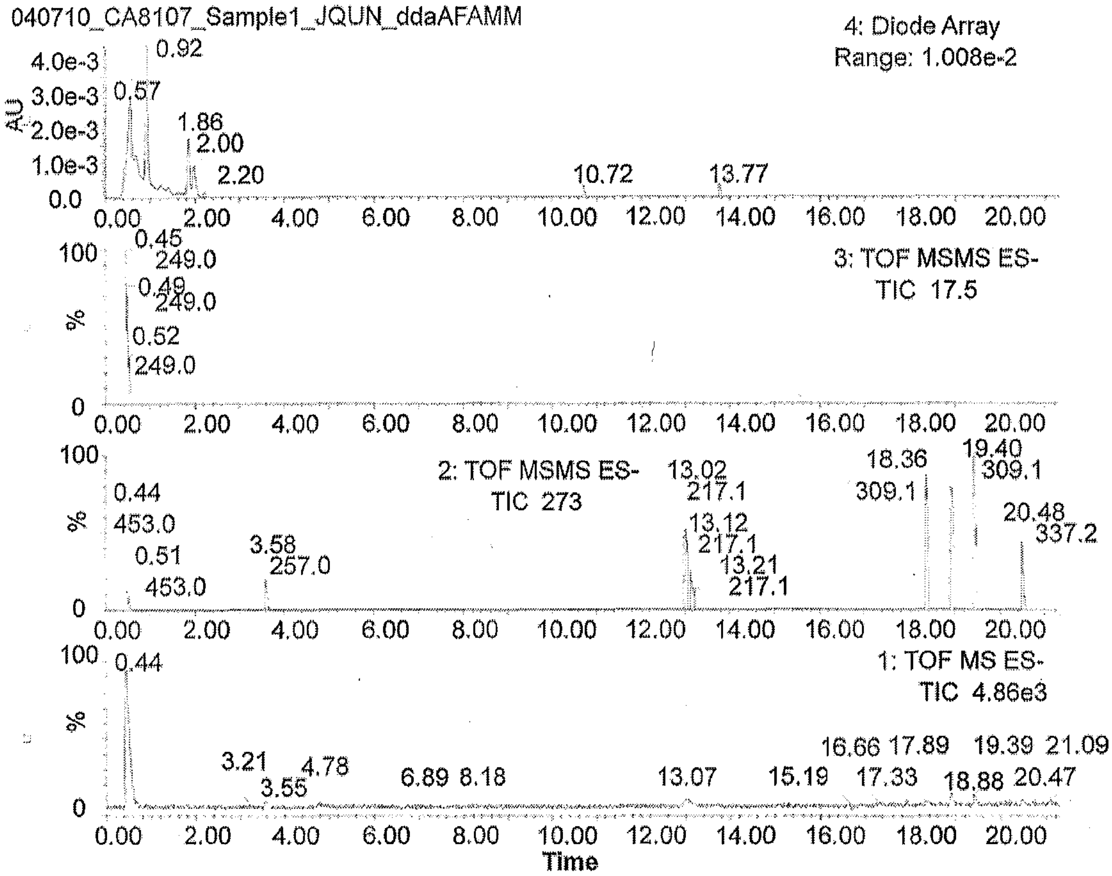
040710_CA8107_Sample1_JQUN_ddaAFAMM
4: Diode Array
Range: 1.008e-2
AU
4.0e-3
3.0e-3
2.0e-3
1.0e-3
0.0
0.92
0.57
1.86
2.00
2.20
10.72
13.77
3: TOF MSMS ES-
TIC 17.5
0.45
249.0
0.49
249.0
0.52
249.0
2: TOF MSMS ES-
TIC 273
0.44
453.0
0.51
453.0
3.58
257.0
13.02
217.1
13.12
217.1
13.21
217.1
18.36
309.1
19.40
309.1
20.48
337.2
1: TOF MS ES-
TIC 4.86e3
0.44
3.21
3.55
4.78
6.89 8.18
13.07
15.19
16.66 17.89
17.33
19.39 21.09
18.88 20.47
100
%
0
0.00 2.00 4.00 6.00 8.00 10.00 12.00 14.00 16.00 18.00 20.00
Time

Fig. 14

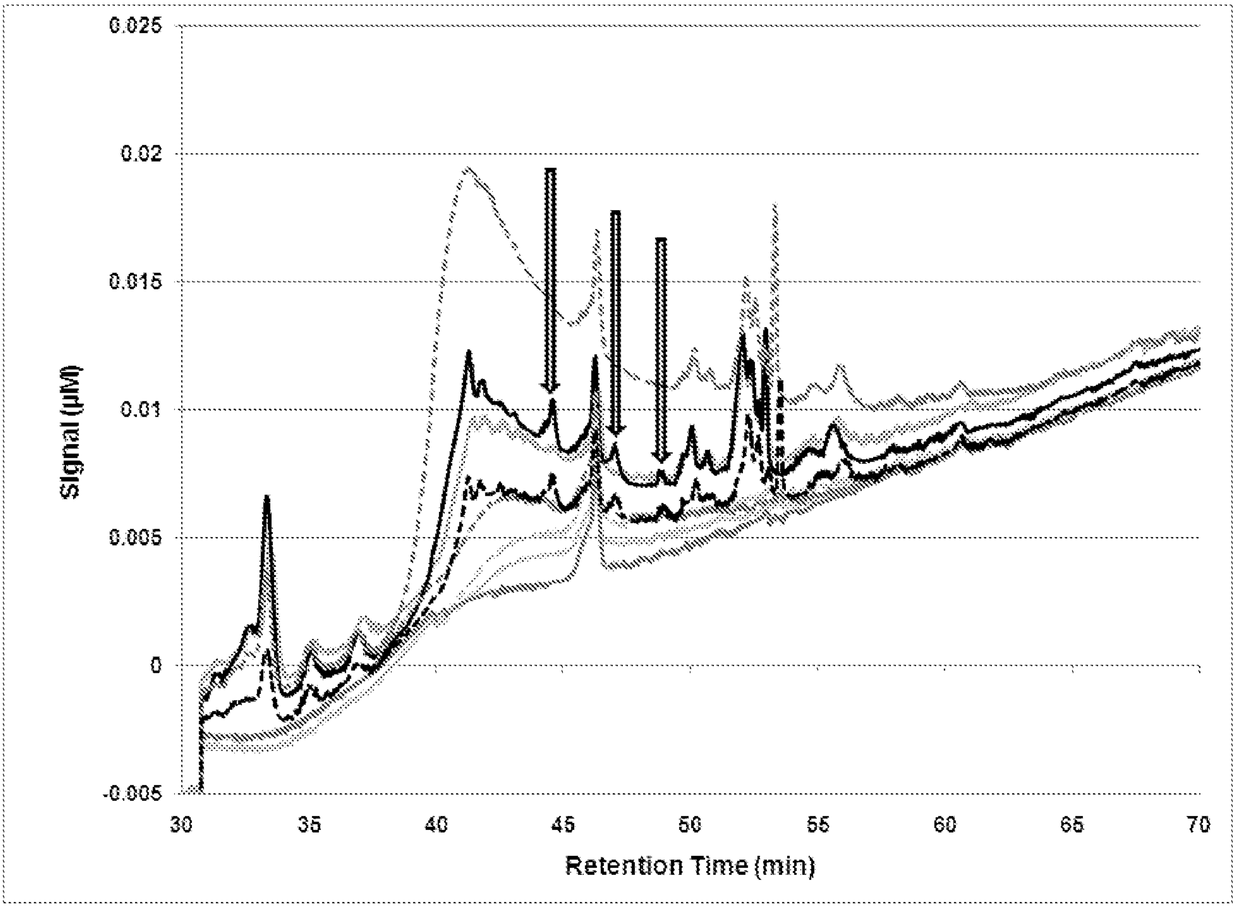
0.025
0.02
0.015
0.01
0.005
0
-0.005
Signal (µM)
30
35
40
45
50
55
60
65
70
Retention Time (min)

Fig. 15A

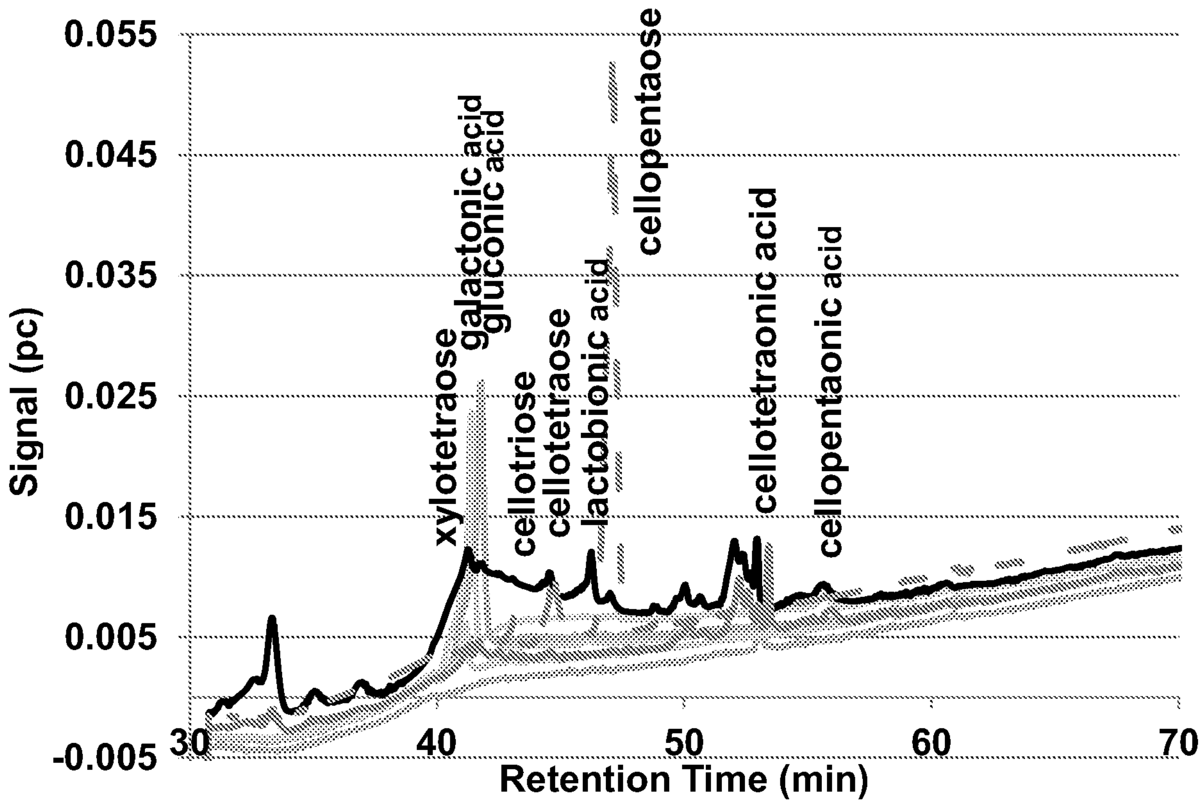
Signal (pc)
0.055
0.045
0.035
0.025
0.015
0.005
-0.005
30
40
50
60
70
Retention Time (min)
xylotetraose
galactonic acid
gluconic acid
cellotriose
cellotetraose
lactobionic acid
cellopentaose
cellotetraonic acid
cellopentaonic acid

Fig. 15B

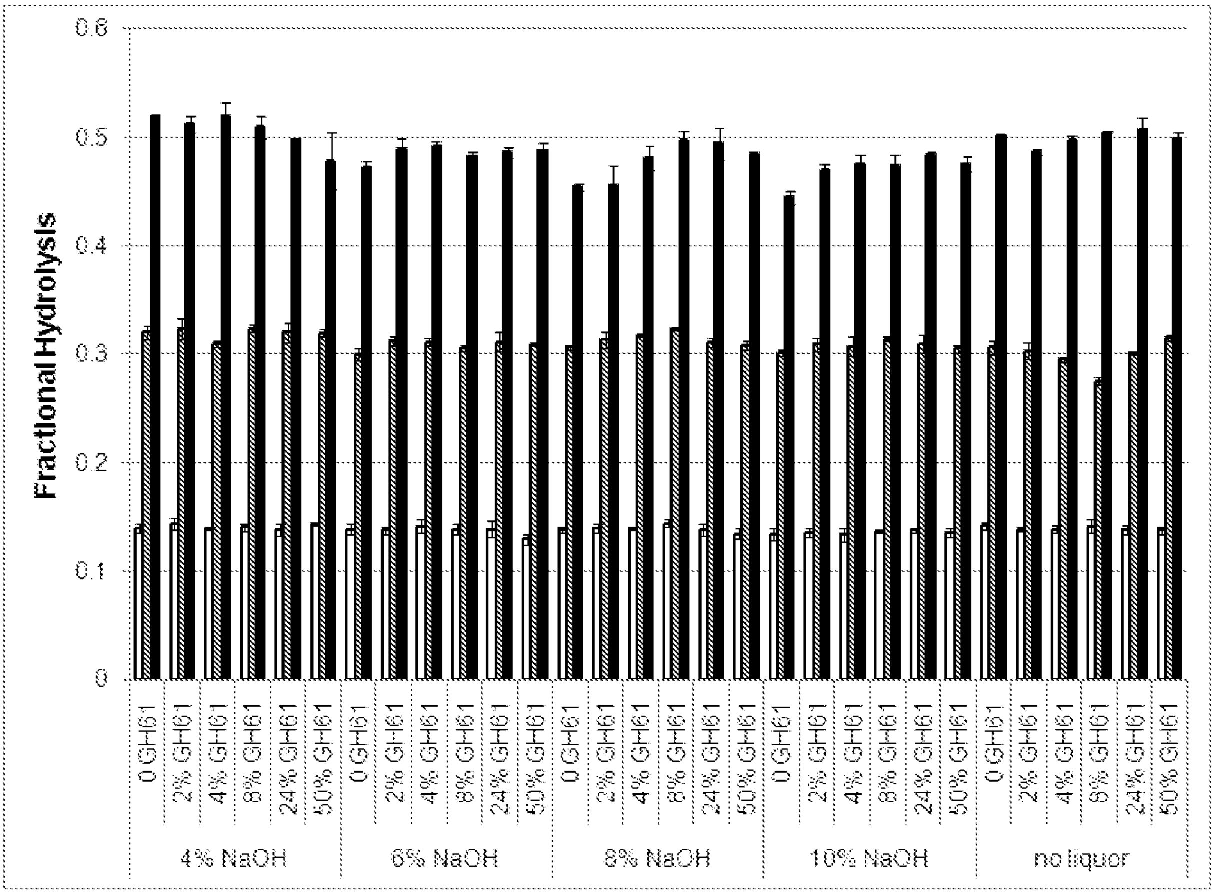
Fractional Hydrolysis
0.6
0.5
0.4
0.3
0.2
0.1
0
0 GH61
2% GH61
4% GH61
8% GH61
24% GH61
50% GH61
4% NaOH
6% NaOH
8% NaOH
10% NaOH
no liquor

Fig. 16A

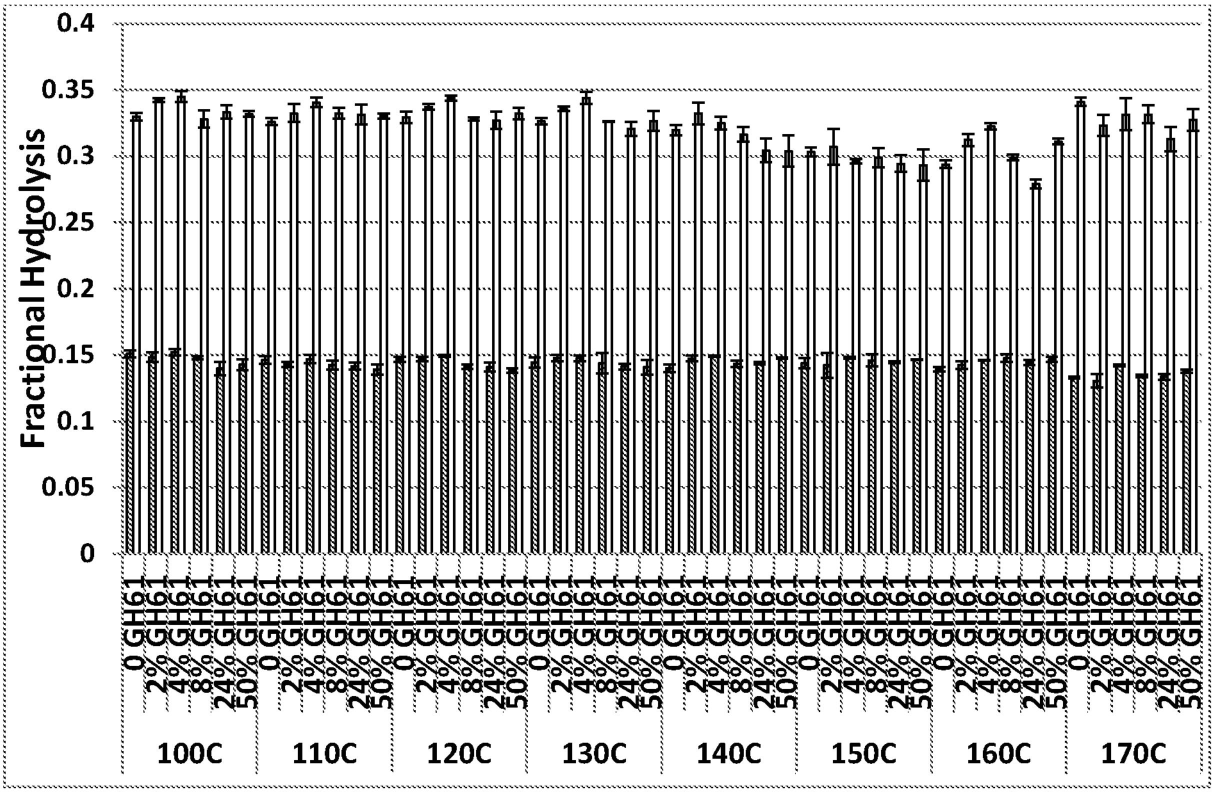
Fractignal Hydrolysis
0.4
0.35
0.3
0.25
0.2
0.15
0.1
0.05
0
0 GH61
2% GH61
4% GH61
8% GH61
24% GH61
50% GH61
100C
110C
120C
130C
140C
150C
160C
170C

# Fig. 16B

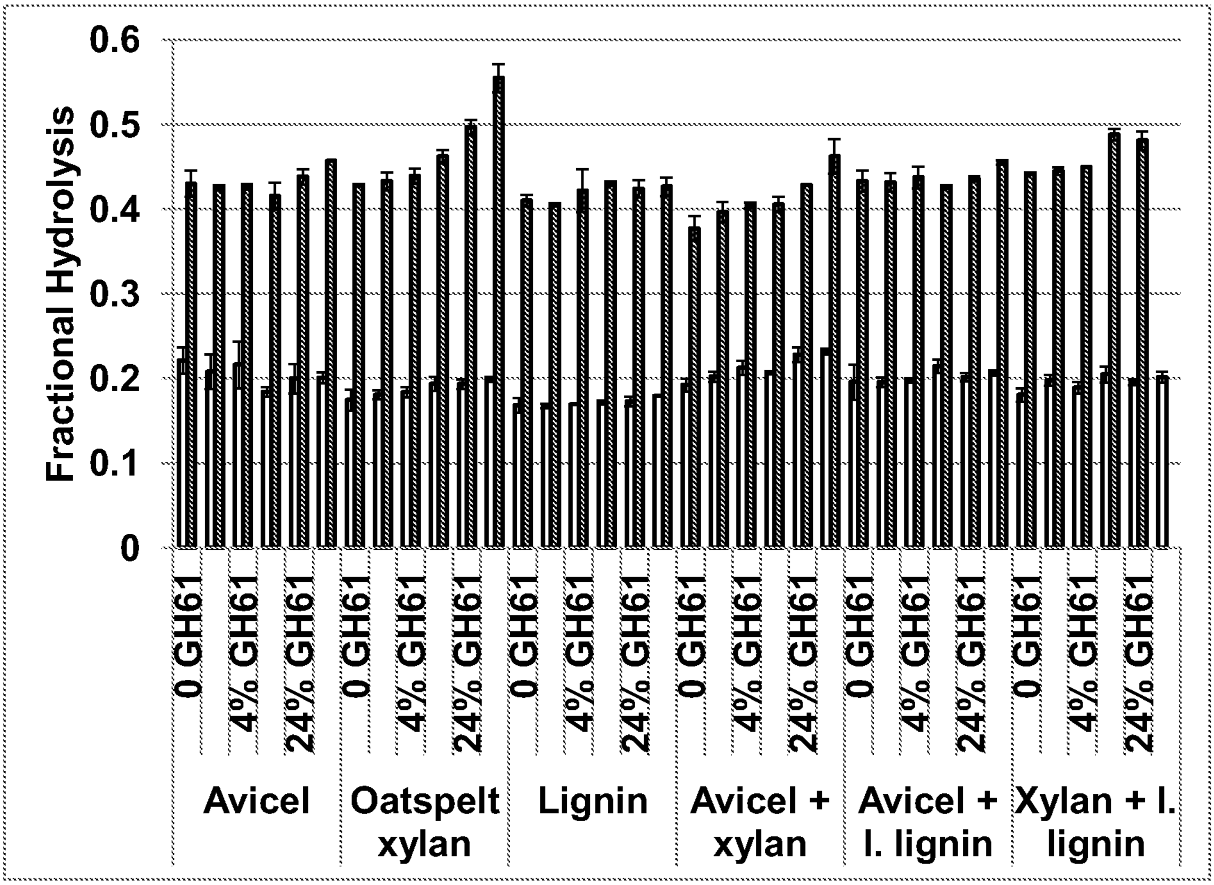
0.6
0.5
0.4
0.3
0.2
0.1
0
Fractional Hydrolysis
0 GH61
4% GH61
24% GH61
Avicel
Oatspelt xylan
Lignin
Avicel + xylan
Avicel + l. lignin
Xylan + l. lignin

Fig. 17A

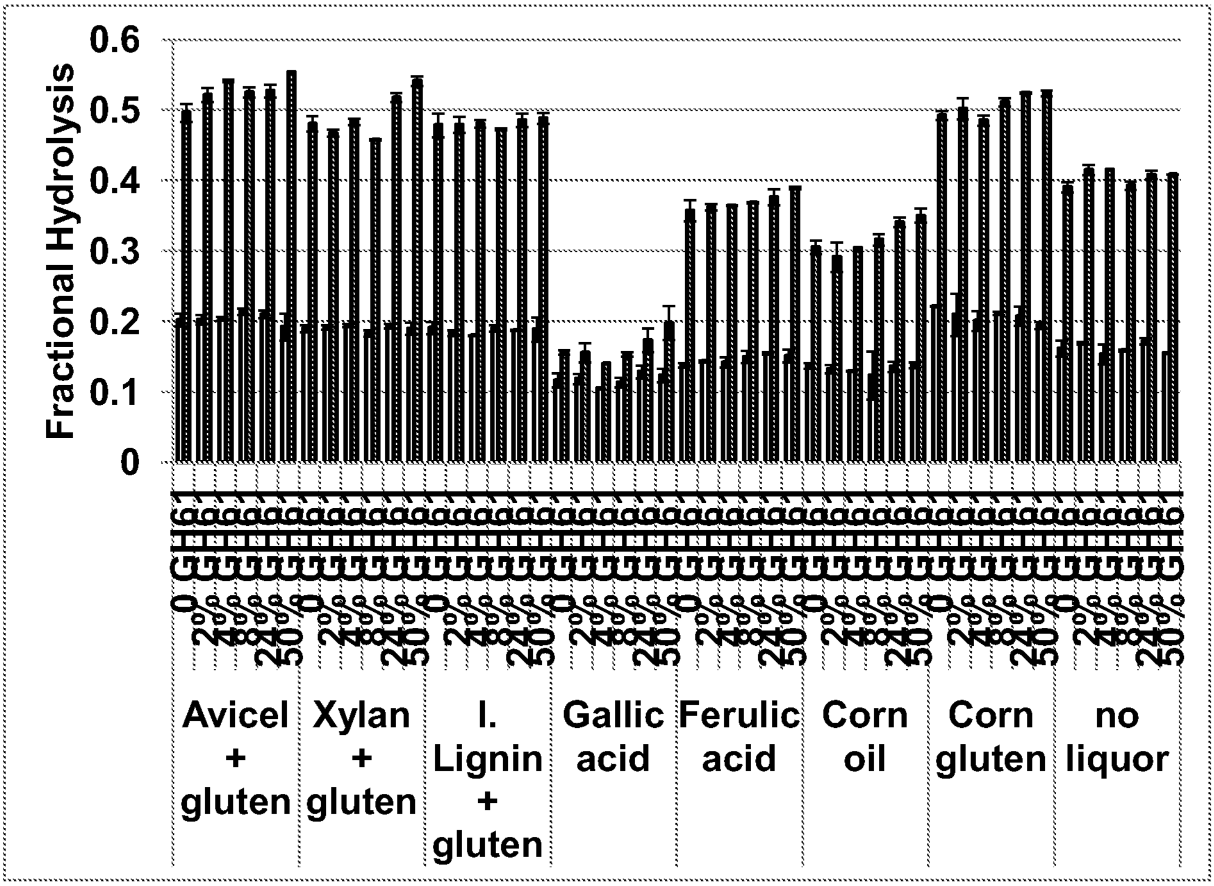
Fractional Hydrolysis
0.6
0.5
0.4
0.3
0.2
0.1
0
Avicel + gluten
Xylan + gluten
I. Lignin + gluten
Gallic acid
Ferulic acid
Corn oil
Corn gluten
no liquor

Fig. 17B

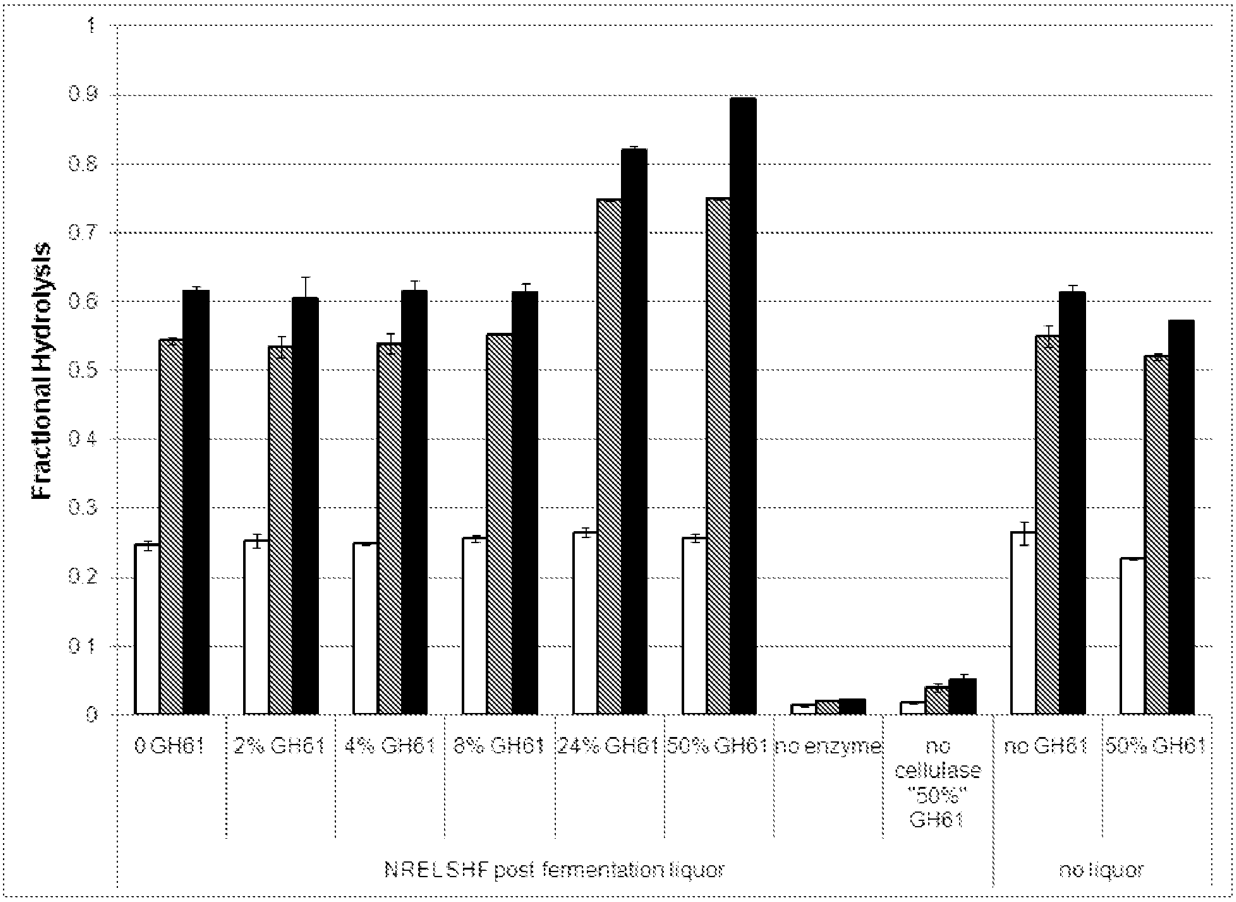
Fractional Hydrolysis
1
0.9
0.8
0.7
0.6
0.5
0.4
0.3
0.2
0.1
0
0 GH61
2% GH61
4% GH61
8% GH61
24% GH61
50% GH61
no enzyme
no cellulase "50%" GH61
no GH61
50% GH61
NREL SHF post fermentation liquor
no liquor

Fig. 18

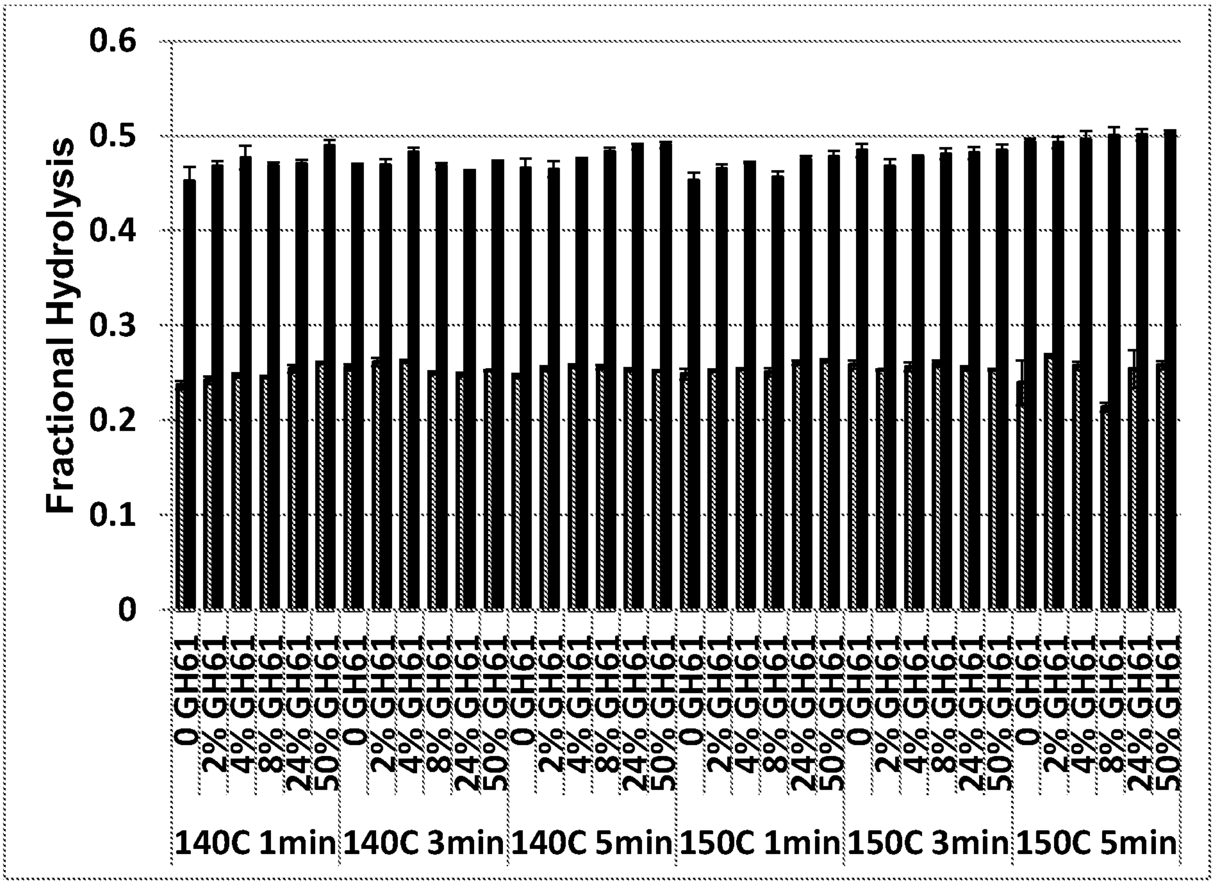
Fractional Hydrolysis
0.6
0.5
0.4
0.3
0.2
0.1
0
0 GH61
2% GH61
4% GH61
8% GH61
24% GH61
50% GH61
140C 1min
140C 3min
140C 5min
150C 1min
150C 3min
150C 5min

Fig. 19A

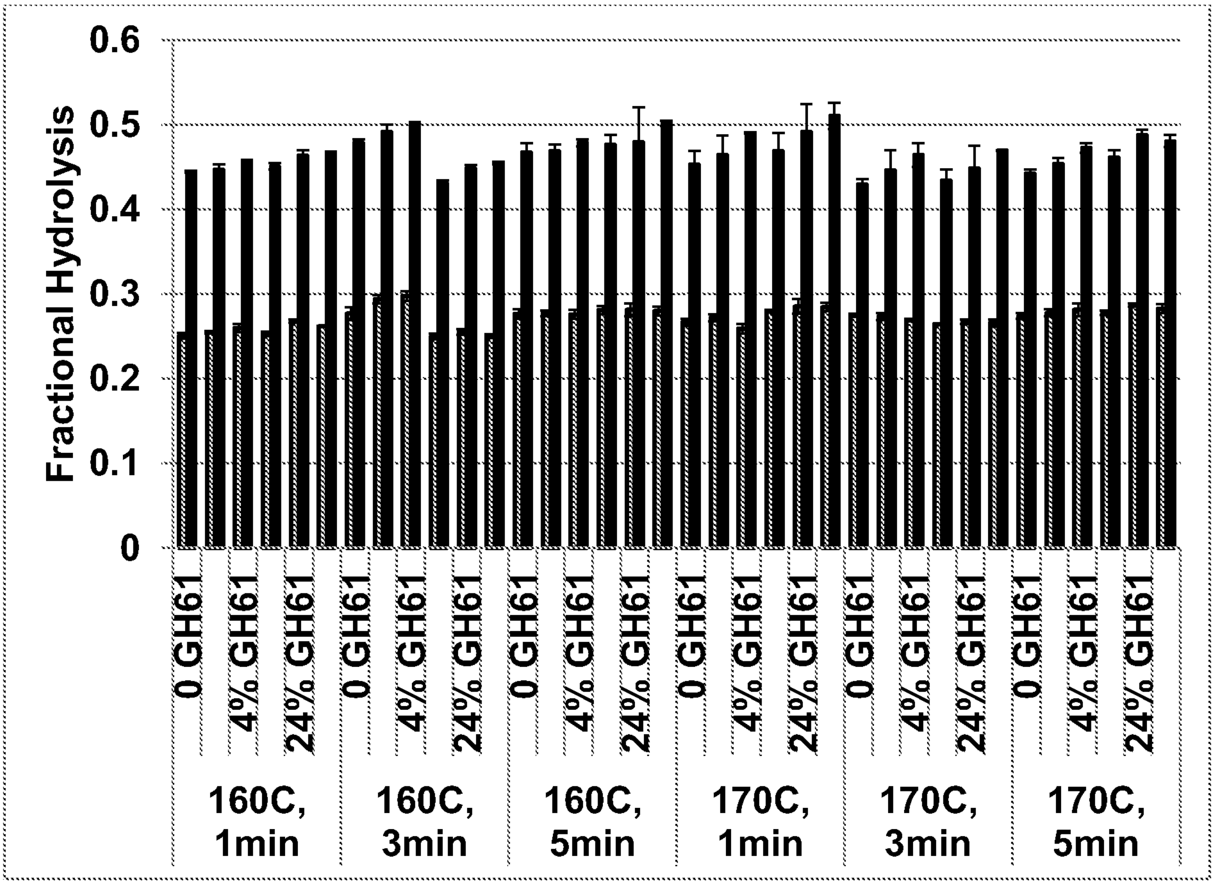

Fractional Hydrolysis
0.6
0.5
0.4
0.3
0.2
0.1
0
0 GH61
4% GH61
24% GH61
160C, 1min
160C, 3min
160C, 5min
170C, 1min
170C, 3min
170C, 5min


Fig. 19B

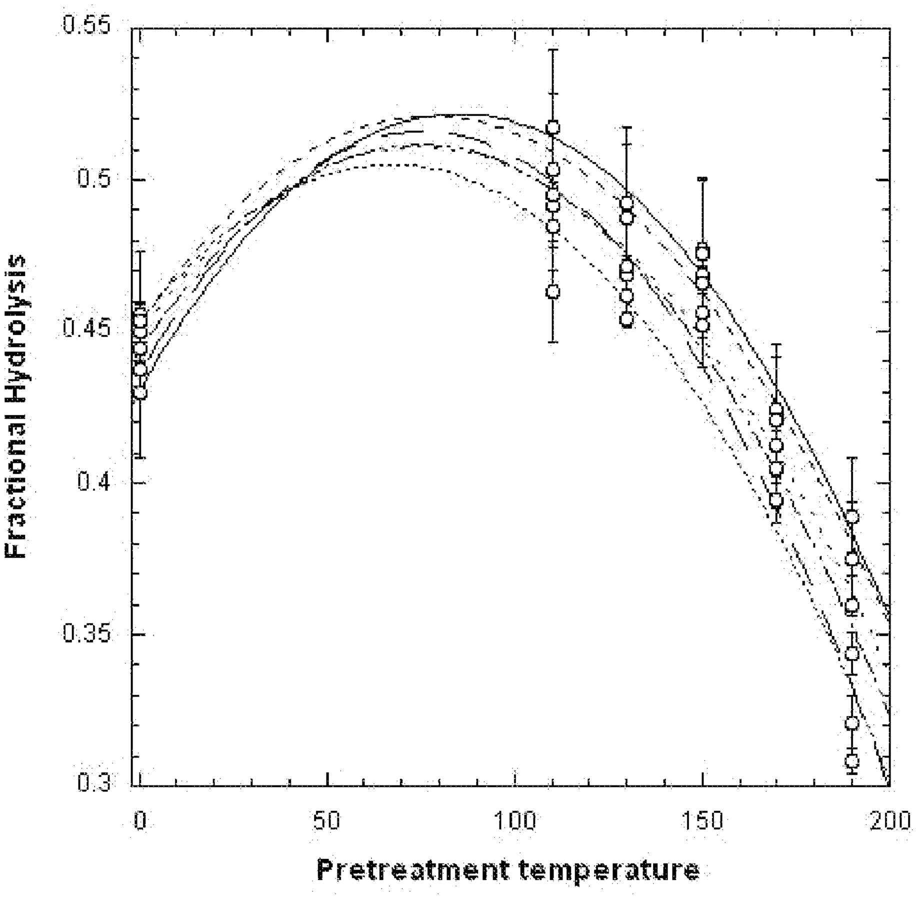
Fractional Hydrolysis
0.55
0.5
0.45
0.4
0.35
0.3
0
50
100
150
200
Pretreatment temperature

Fig. 19C

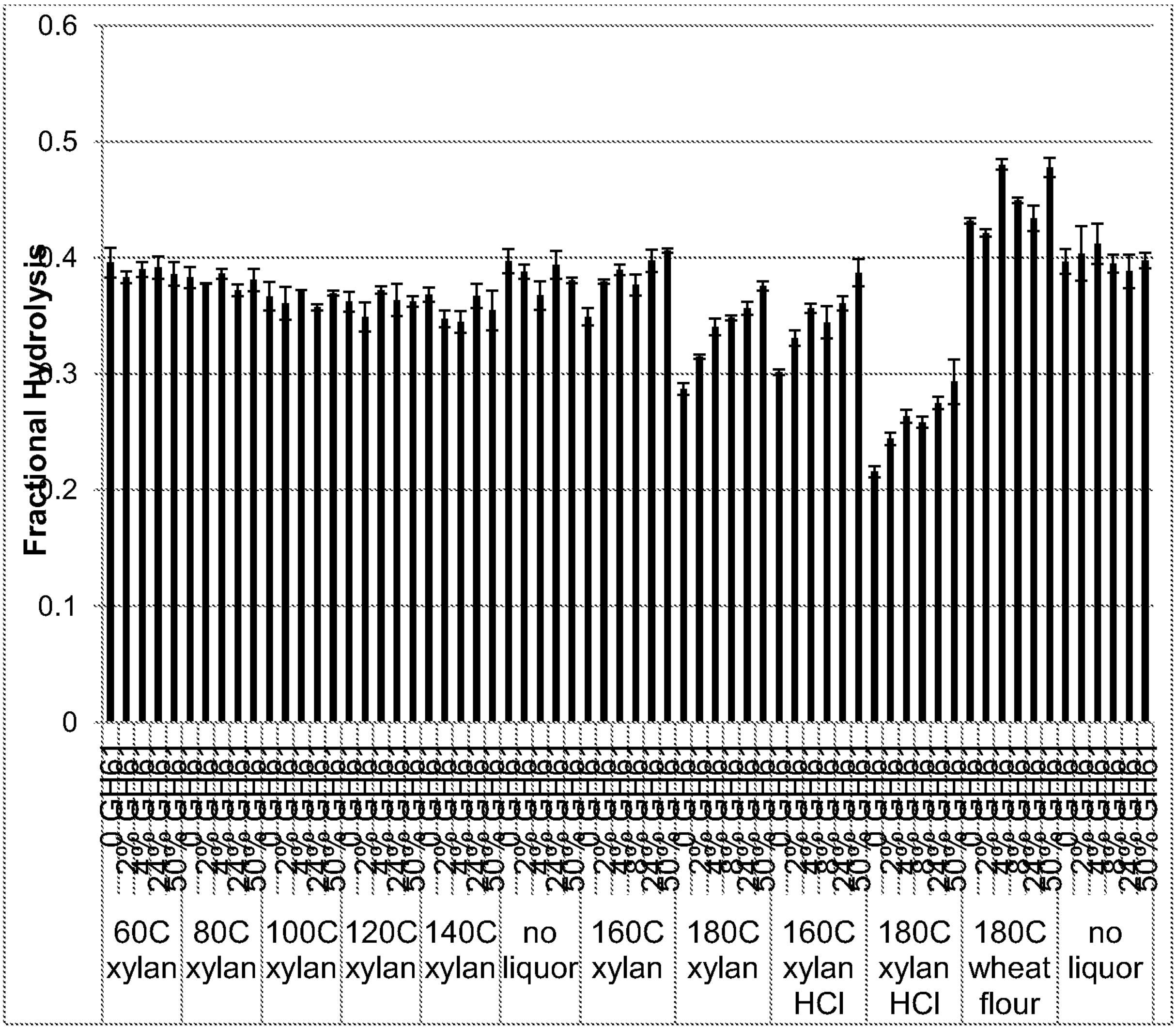
Fractional Hydrolysis
0.6
0.5
0.4
0.3
0.2
0.1
0
60C xylan
80C xylan
100C xylan
120C xylan
140C xylan
no liquor
160C xylan
180C xylan
160C xylan HCl
180C xylan HCl
180C wheat flour
no liquor

# Fig. 20A

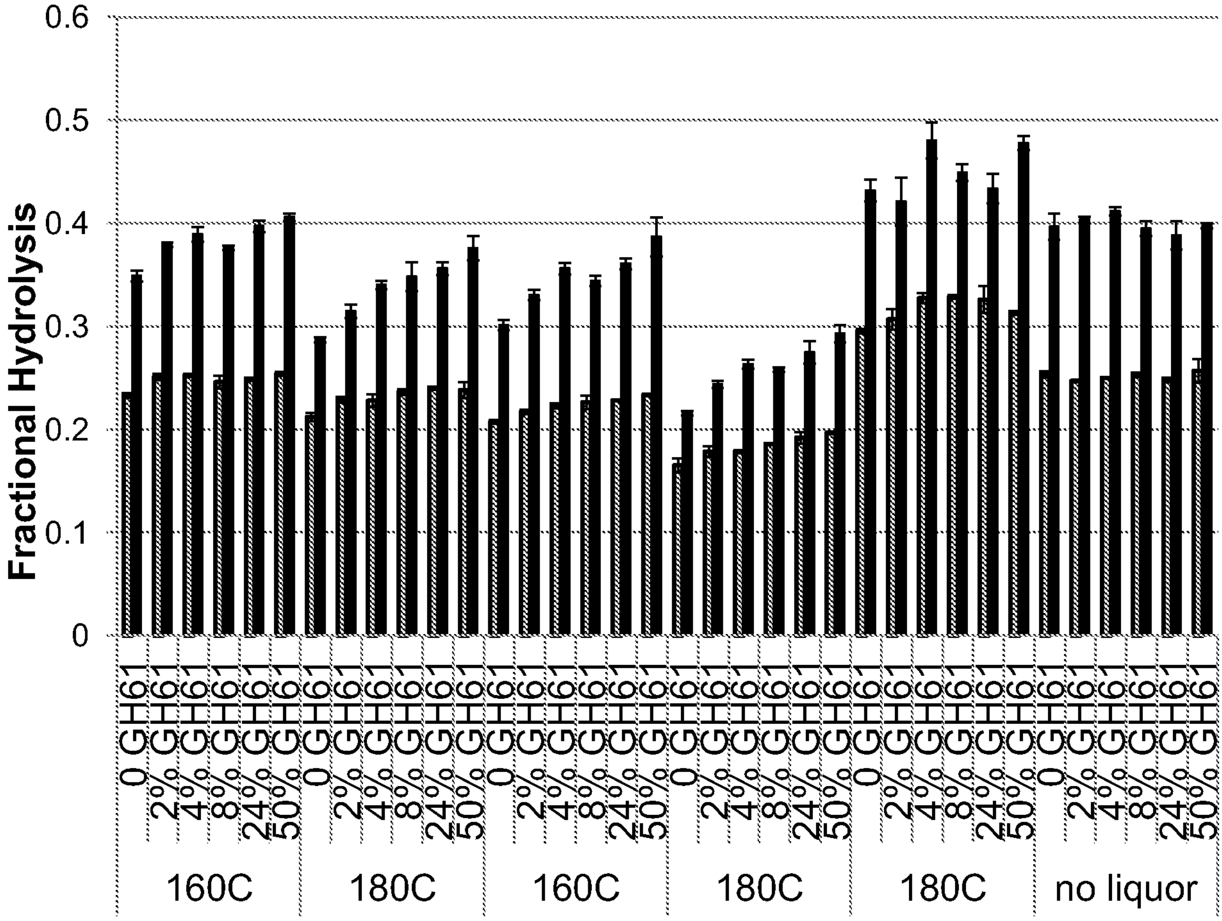
0.6
0.5
0.4
0.3
0.2
0.1
0
Fractional Hydrolysis
0 GH61
2% GH61
4% GH61
8% GH61
24% GH61
50% GH61
160C
180C
160C
180C
180C
no liquor

Fig. 20B

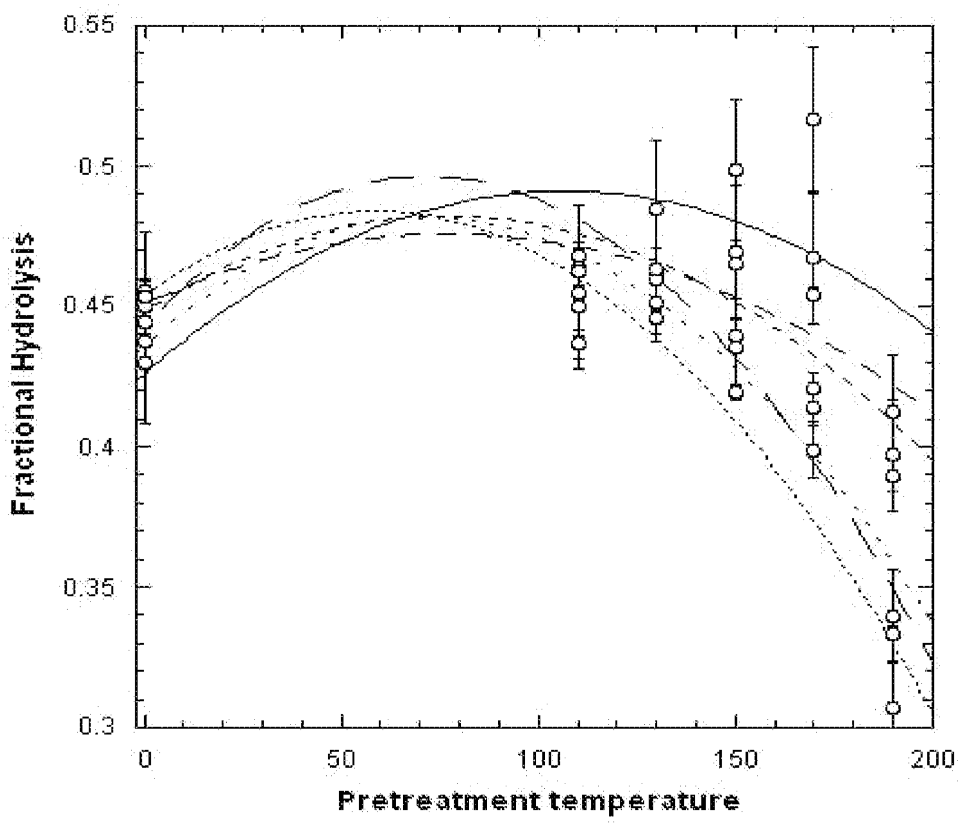
0.55
0.5
0.45
0.4
0.35
0.3
Fractional Hydrolysis
0
50
100
150
200
Pretreatment temperature

Fig. 20C

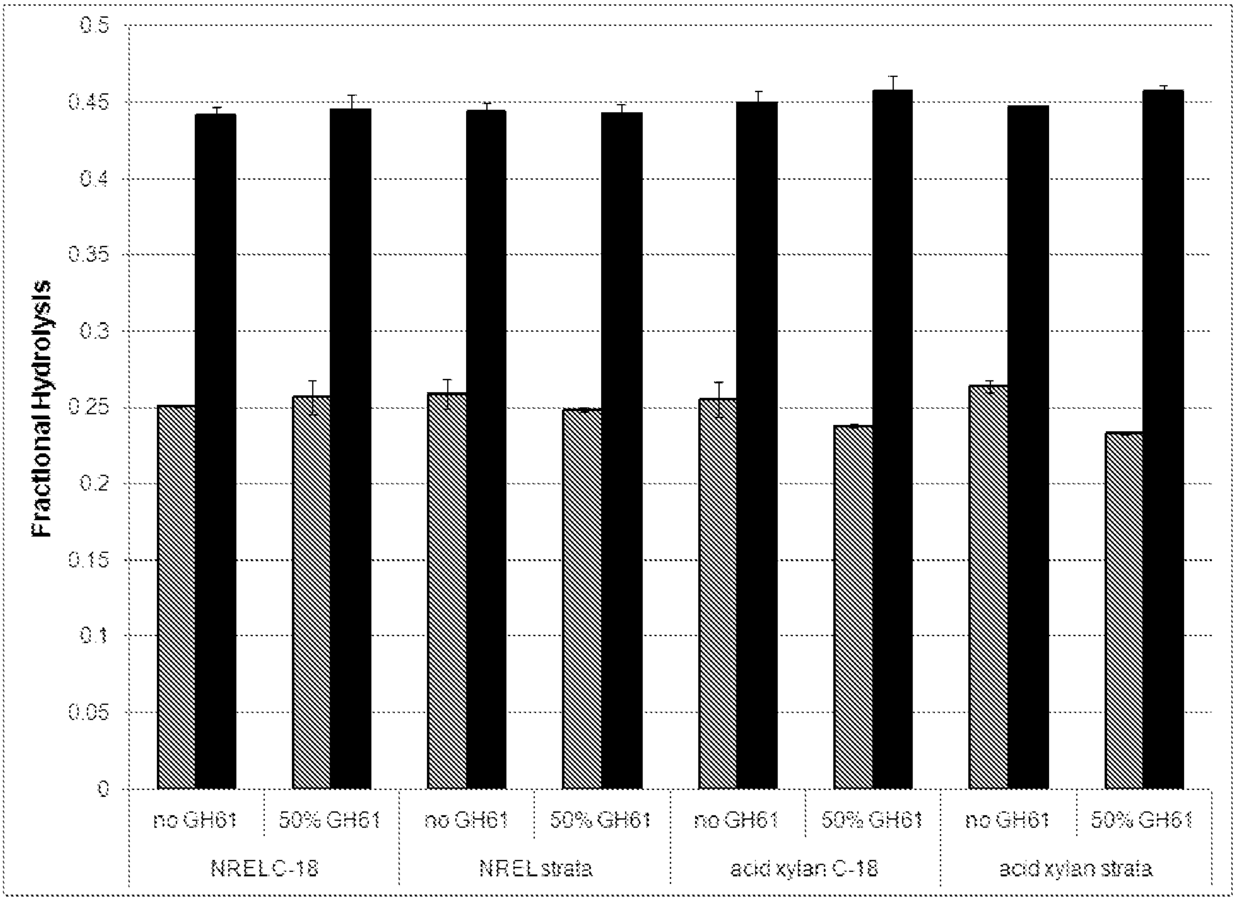
Fractional Hydrolysis
0.5
0.45
0.4
0.35
0.3
0.25
0.2
0.15
0.1
0.05
0
no GH61
50% GH61
no GH61
50% GH61
no GH61
50% GH61
no GH61
50% GH61
NREL C-18
NREL strata
acid xylan C-18
acid xylan strata

**Fig. 21**

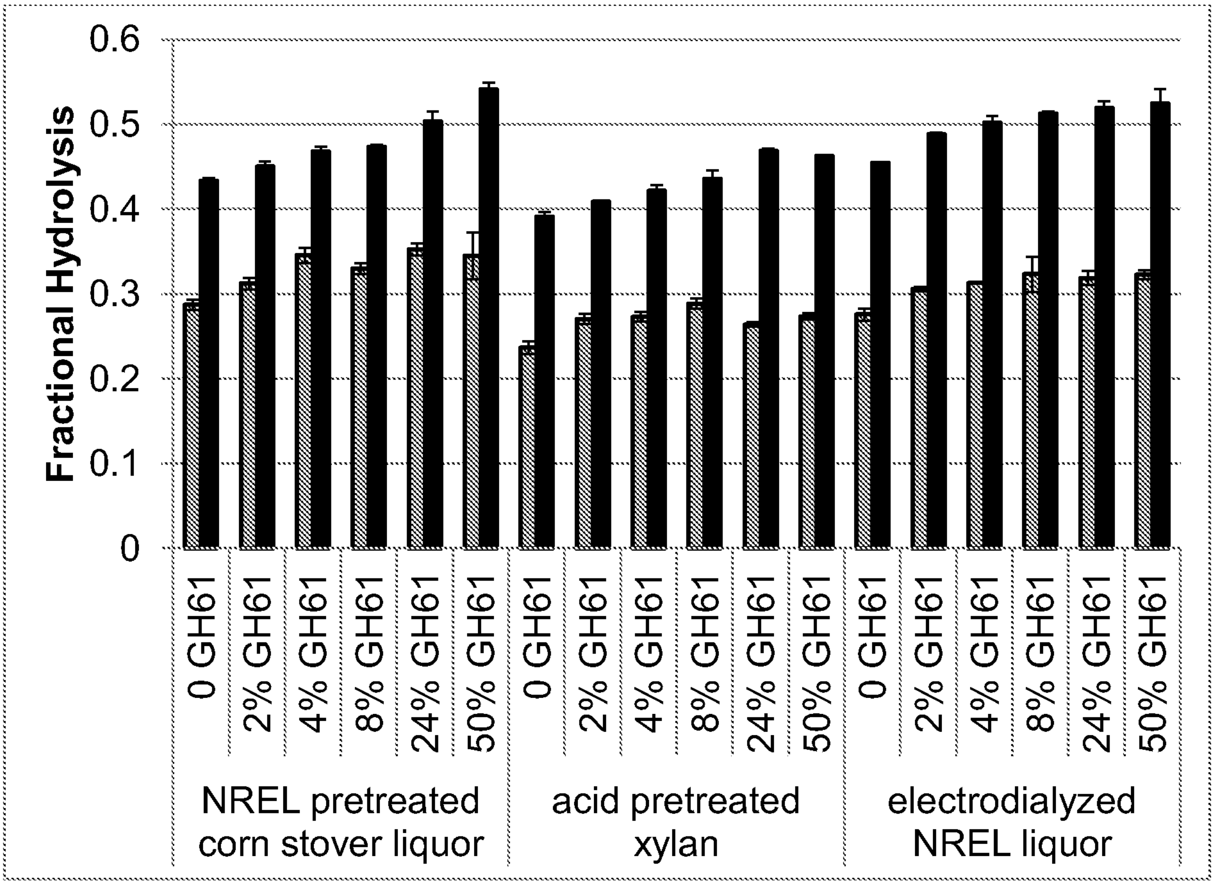
Fractional Hydrolysis
0.6
0.5
0.4
0.3
0.2
0.1
0
0 GH61
2% GH61
4% GH61
8% GH61
24% GH61
50% GH61
0 GH61
2% GH61
4% GH61
8% GH61
24% GH61
50% GH61
0 GH61
2% GH61
4% GH61
8% GH61
24% GH61
50% GH61
NREL pretreated corn stover liquor
acid pretreated xylan
electrodialyzed NREL liquor

Fig. 22

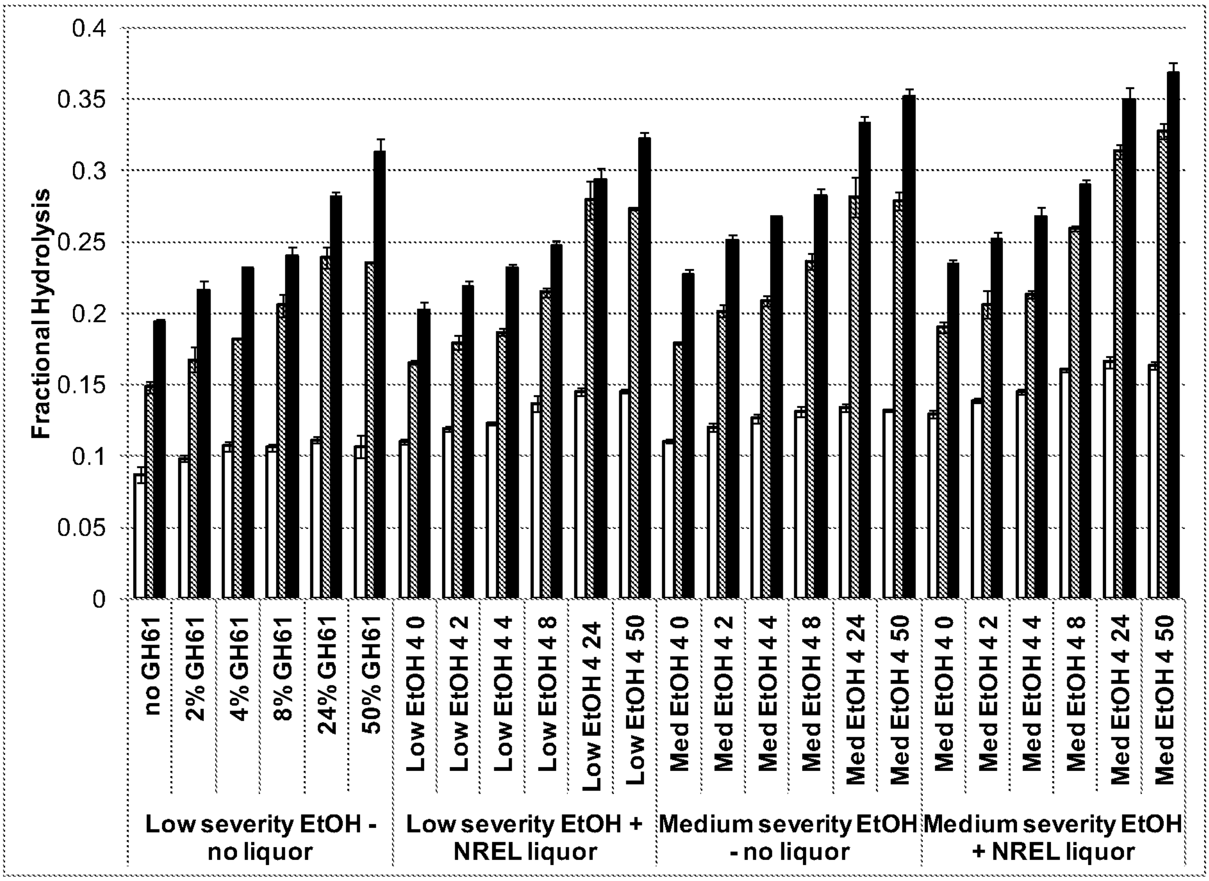
Fractional Hydrolysis
0.4
0.35
0.3
0.25
0.2
0.15
0.1
0.05
0
no GH61
2% GH61
4% GH61
8% GH61
24% GH61
50% GH61
Low EtOH 4 0
Low EtOH 4 2
Low EtOH 4 4
Low EtOH 4 8
Low EtOH 4 24
Low EtOH 4 50
Med EtOH 4 0
Med EtOH 4 2
Med EtOH 4 4
Med EtOH 4 8
Med EtOH 4 24
Med EtOH 4 50
Med EtOH 4 0
Med EtOH 4 2
Med EtOH 4 4
Med EtOH 4 8
Med EtOH 4 24
Med EtOH 4 50
Low severity EtOH - no liquor
Low severity EtOH + NREL liquor
Medium severity EtOH - no liquor
Medium severity EtOH + NREL liquor

# Fig. 23A

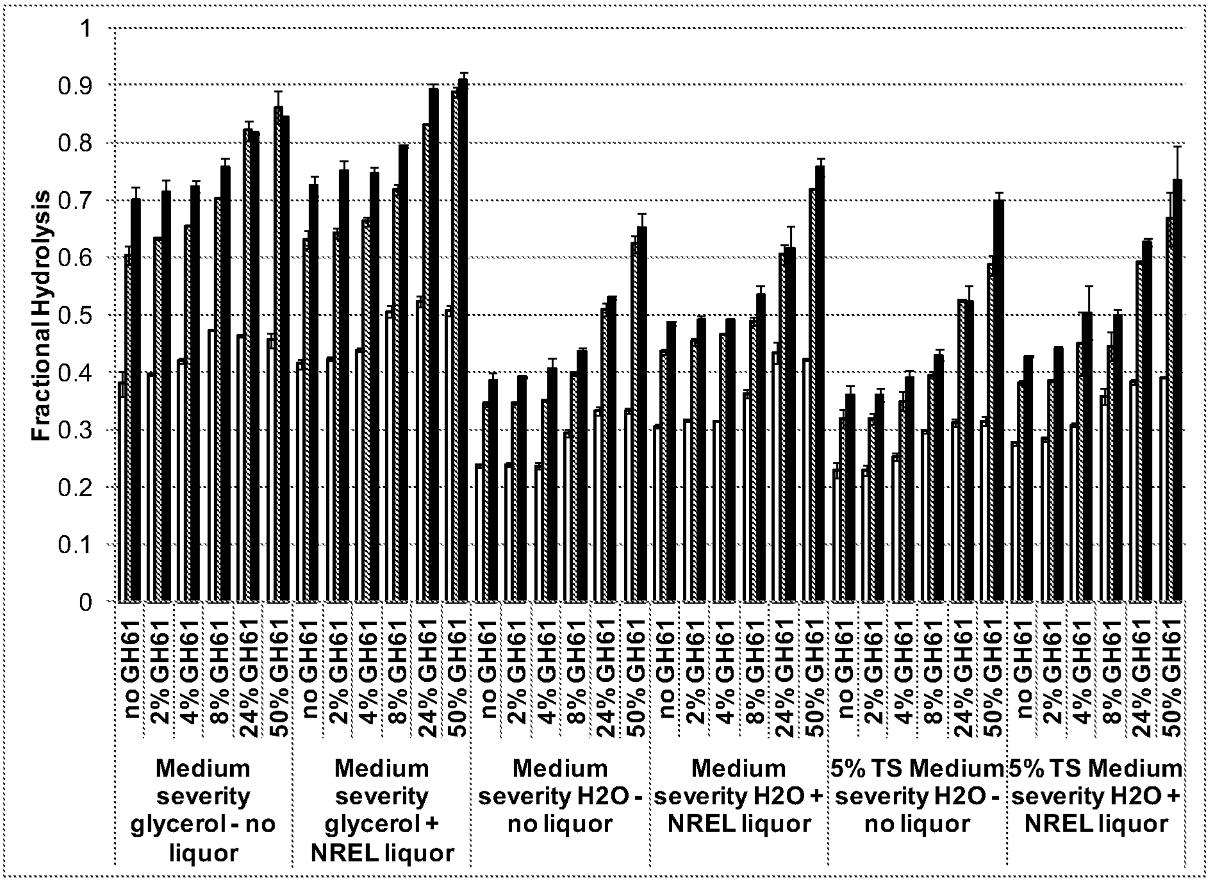
Fractional Hydrolysis
1
0.9
0.8
0.7
0.6
0.5
0.4
0.3
0.2
0.1
0
no GH61
2% GH61
4% GH61
8% GH61
24% GH61
50% GH61
Medium severity glycerol - no liquor
Medium severity glycerol + NREL liquor
Medium severity H2O - no liquor
Medium severity H2O + NREL liquor
5% TS Medium severity H2O - no liquor
5% TS Medium severity H2O + NREL liquor

Fig. 23B

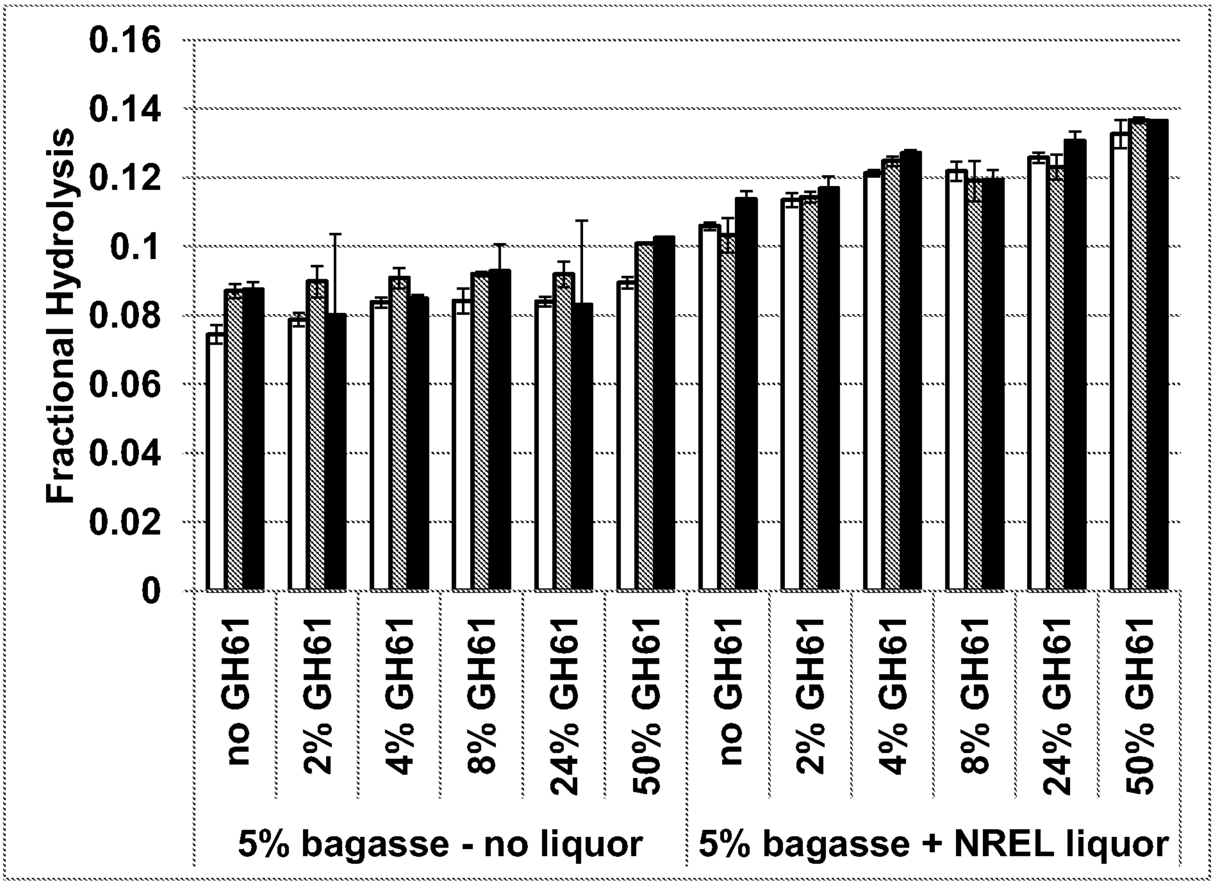
Fractional Hydrolysis
0.16
0.14
0.12
0.1
0.08
0.06
0.04
0.02
0
no GH61
2% GH61
4% GH61
8% GH61
24% GH61
50% GH61
no GH61
2% GH61
4% GH61
8% GH61
24% GH61
50% GH61
5% bagasse - no liquor
5% bagasse + NREL liquor

Fig. 23C

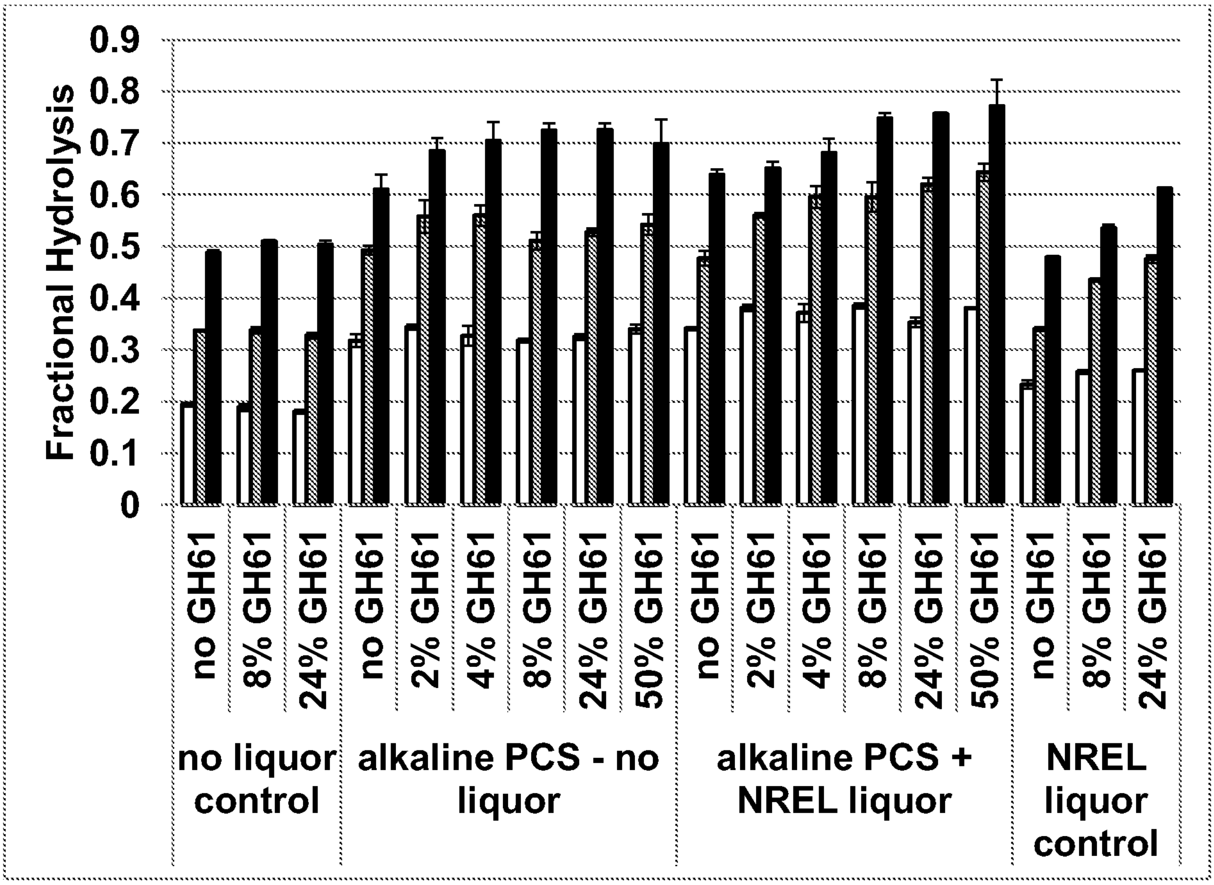
Fractional Hydrolysis
0.9
0.8
0.7
0.6
0.5
0.4
0.3
0.2
0.1
0
no GH61
8% GH61
24% GH61
no GH61
2% GH61
4% GH61
8% GH61
24% GH61
50% GH61
no GH61
2% GH61
4% GH61
8% GH61
24% GH61
50% GH61
no GH61
8% GH61
24% GH61
no liquor control
alkaline PCS - no liquor
alkaline PCS + NREL liquor
NREL liquor control

Fig. 23D

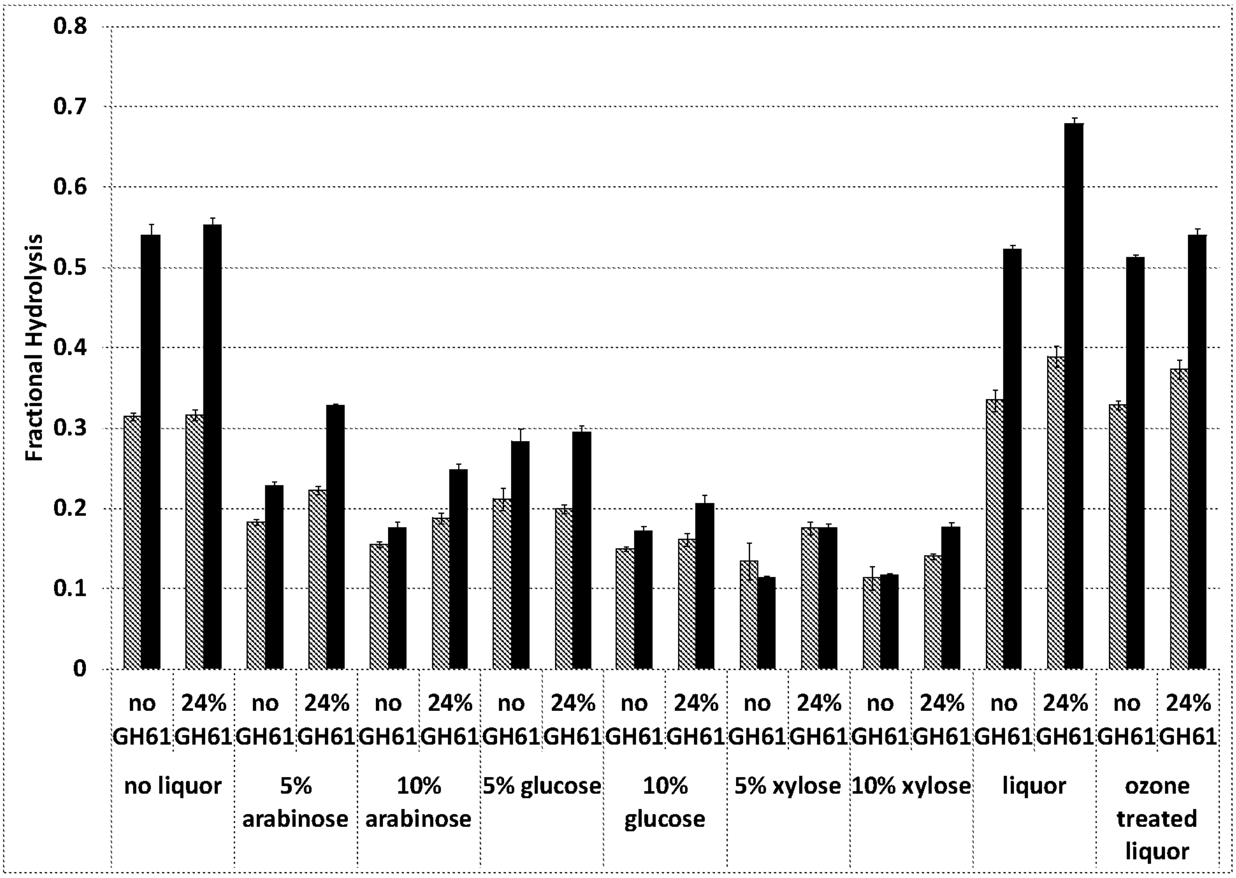
Fractional Hydrolysis
0.8
0.7
0.6
0.5
0.4
0.3
0.2
0.1
0
no GH61
24% GH61
no liquor
5% arabinose
10% arabinose
5% glucose
10% glucose
5% xylose
10% xylose
liquor
ozone treated liquor

Fig. 24

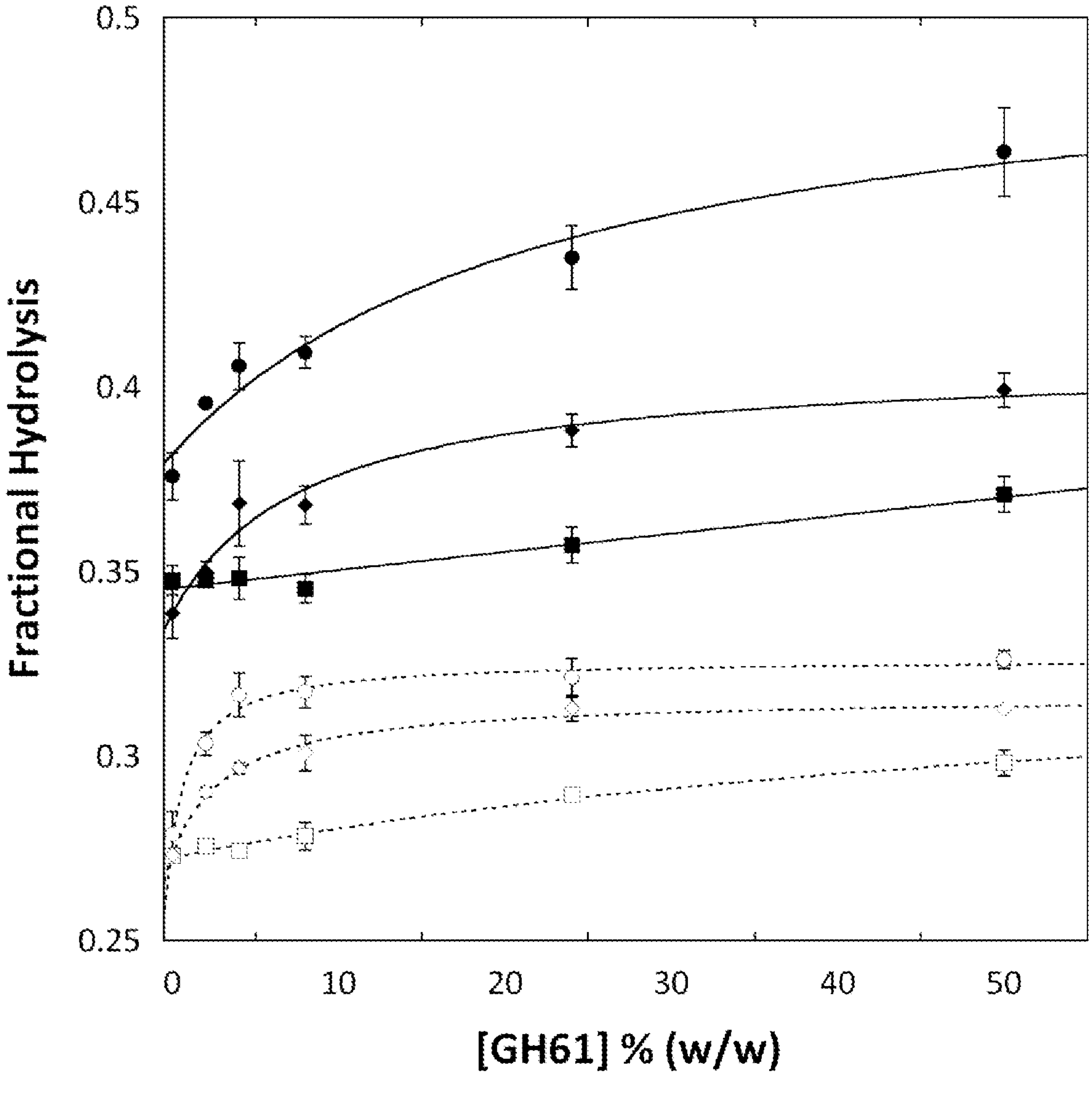
0.5
0.45
0.4
0.35
0.3
0.25
Fractional Hydrolysis
0
10
20
30
40
50
[GH61] % (w/w)

Fig. 25

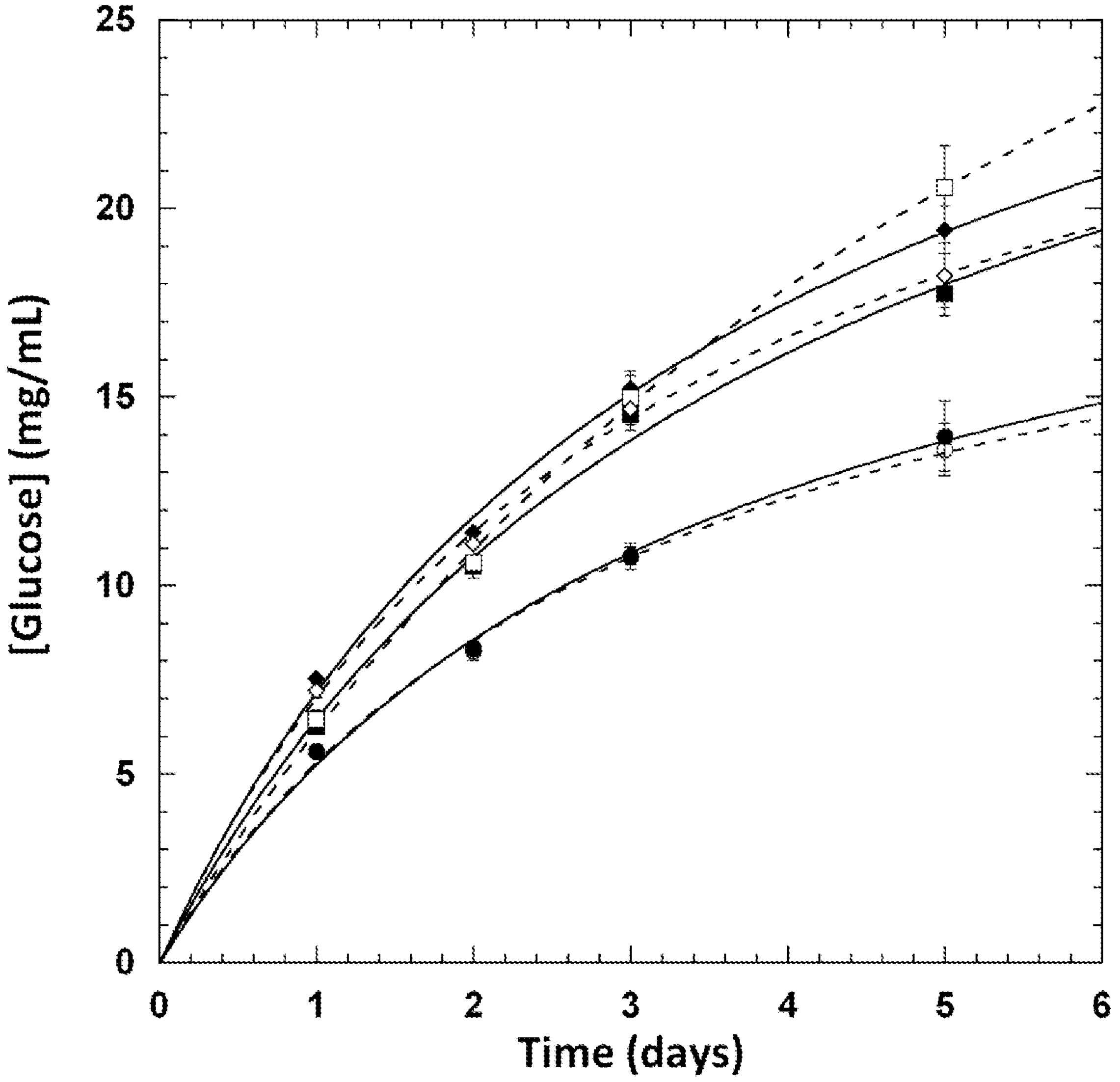


Fig. 26

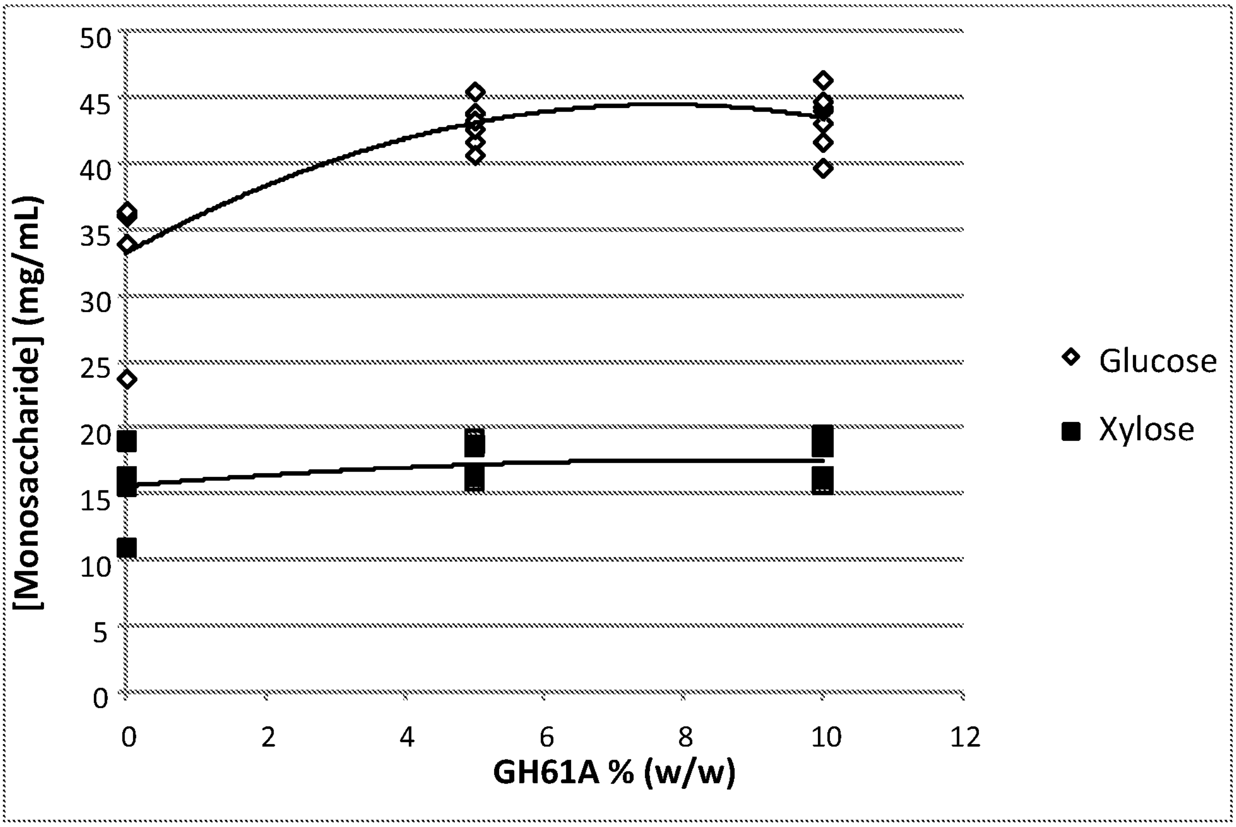

[Monosaccharide] (mg/mL)
50
45
40
35
30
25
20
15
10
5
0
0
2
4
6
8
10
12
GH61A % (w/w)
Glucose
Xylose


Fig. 27

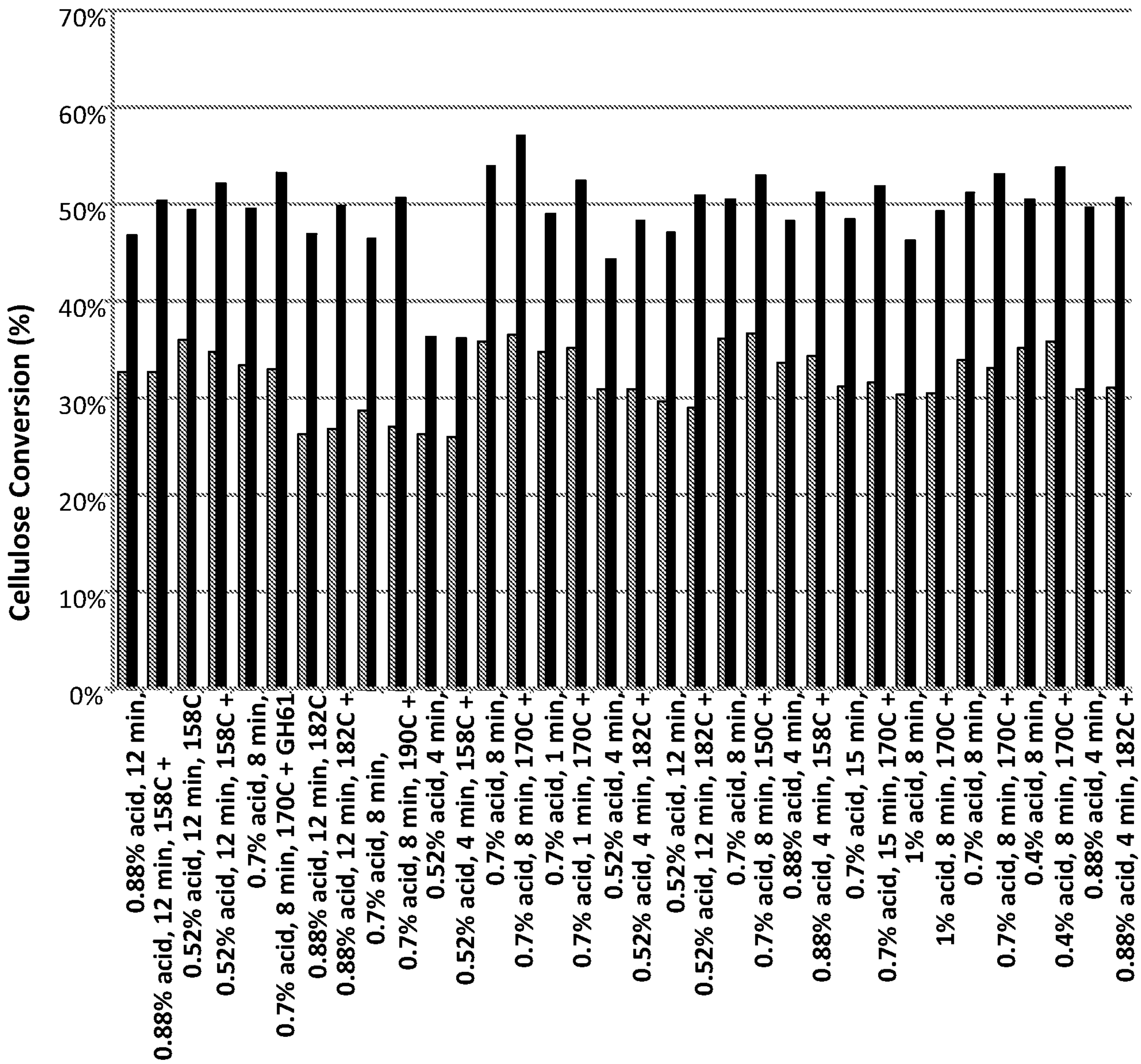
Cellulose Conversion (%)
70%
60%
50%
40%
30%
20%
10%
0%
0.88% acid, 12 min,
0.88% acid, 12 min, 158C +
0.52% acid, 12 min, 158C
0.52% acid, 12 min, 158C +
0.7% acid, 8 min,
0.7% acid, 8 min, 170C + GH61
0.88% acid, 12 min, 182C
0.88% acid, 12 min, 182C +
0.7% acid, 8 min,
0.7% acid, 8 min, 190C +
0.52% acid, 4 min,
0.52% acid, 4 min, 158C +
0.7% acid, 8 min,
0.7% acid, 8 min, 170C +
0.7% acid, 1 min,
0.7% acid, 1 min, 170C +
0.52% acid, 4 min,
0.52% acid, 4 min, 182C +
0.52% acid, 12 min,
0.52% acid, 12 min, 182C +
0.7% acid, 8 min,
0.7% acid, 8 min, 150C +
0.88% acid, 4 min,
0.88% acid, 4 min, 158C +
0.7% acid, 15 min,
0.7% acid, 15 min, 170C +
1% acid, 8 min,
1% acid, 8 min, 170C +
0.7% acid, 8 min,
0.7% acid, 8 min, 170C +
0.4% acid, 8 min,
0.4% acid, 8 min, 170C +
0.88% acid, 4 min,
0.88% acid, 4 min, 182C +

Fig. 28

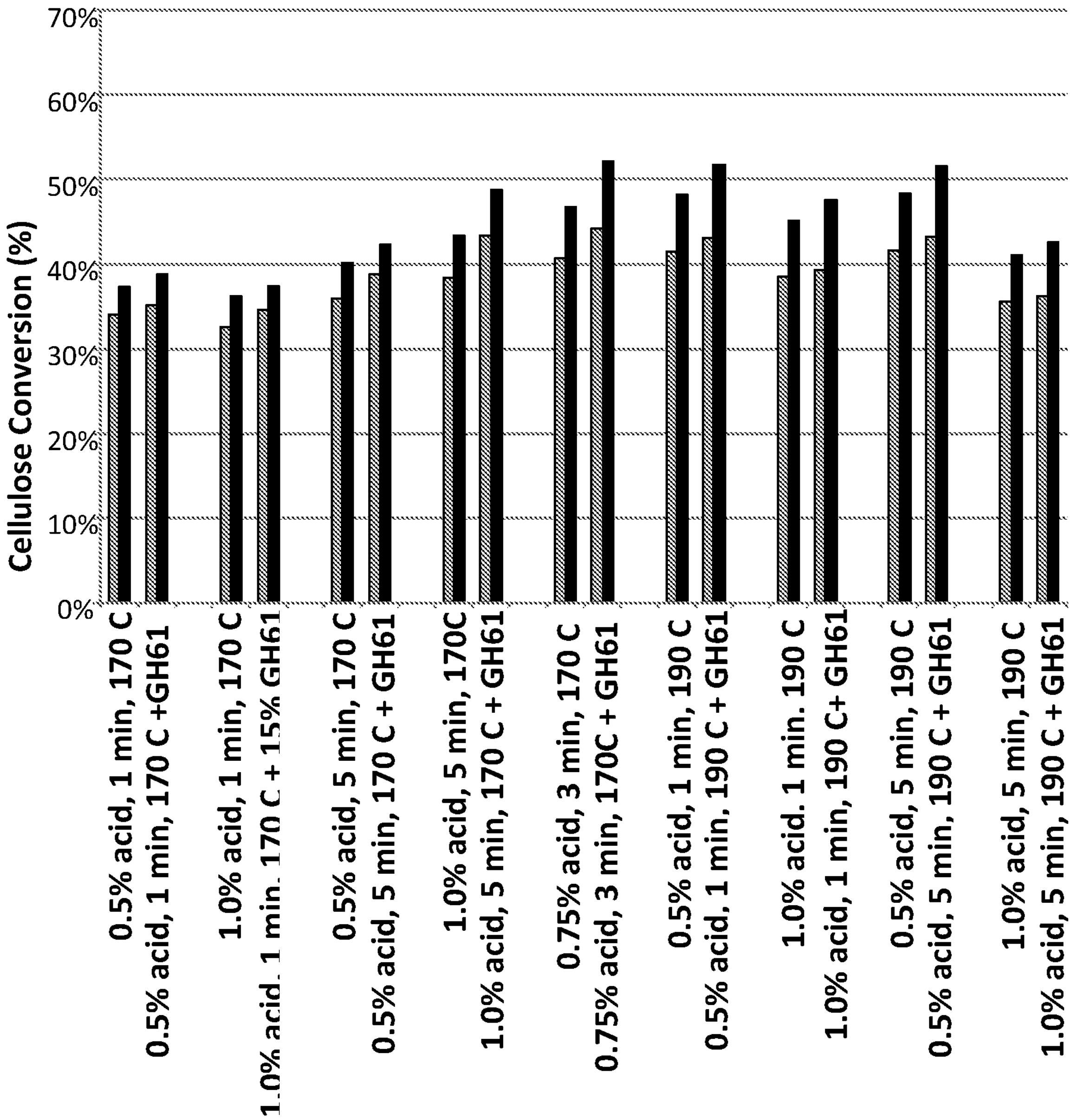
Cellulose Conversion (%)
70%
60%
50%
40%
30%
20%
10%
0%
0.5% acid, 1 min, 170 C
0.5% acid, 1 min, 170 C +GH61
1.0% acid, 1 min, 170 C
1.0% acid, 1 min, 170 C + 15% GH61
0.5% acid, 5 min, 170 C
0.5% acid, 5 min, 170 C + GH61
1.0% acid, 5 min, 170C
1.0% acid, 5 min, 170 C + GH61
0.75% acid, 3 min, 170 C
0.75% acid, 3 min, 170C + GH61
0.5% acid, 1 min, 190 C
0.5% acid, 1 min, 190 C + GH61
1.0% acid, 1 min, 190 C
1.0% acid, 1 min, 190 C+ GH61
0.5% acid, 5 min, 190 C
0.5% acid, 5 min, 190 C + GH61
1.0% acid, 5 min, 190 C
1.0% acid, 5 min, 190 C + GH61

Fig. 29

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005074647 A
- WO 2008148131 A
- WO 2011035027 A
- WO 2005074656 A
- WO 2010065830 A
- WO 2007089290 A
- WO 2009085935 A
- WO 2009085859 A
- WO 2009085864 A
- WO 2009085868 A
- WO 2010138754 A
- WO 2011005867 A
- WO 2011039319 A
- WO 2011041397 A
- WO 2011041504 A
- WO 2008151043 A
- WO 94021785 A
- WO 2005047499 A
- WO 02095014 A
- WO 2002095014 A
- WO 9517413 A
- WO 9522625 A
- US 5223409 A
- WO 9206204 A
- WO 9117243 A
- WO 9117244 A
- WO 9105039 A
- WO 9315186 A
- US 5275944 A
- WO 9602551 A
- US 5536655 A
- WO 0070031 A
- WO 05093050 A
- WO 2007019442 A
- EP 495257 A
- EP 531315 A
- EP 531372 A
- WO 8909259 A
- WO 9407998 A
- WO 9524471 A
- WO 9611262 A
- WO 9629397 A
- WO 96034108 A
- WO 9714804 A
- WO 9808940 A
- WO 98012307 A
- WO 9813465 A
- WO 98015619 A
- WO 98015633 A
- WO 98028411 A
- WO 9906574 A
- WO 9910481 A
- WO 99025846 A
- WO 99025847 A
- WO 99031255 A
- WO 2000009707 A
- WO 2002050245 A
- WO 20020076792 A
- WO 2002101078 A
- WO 2003027306 A
- WO 2003052054 A
- WO 2003052055 A
- WO 2003052056 A
- WO 2003052057 A
- WO 2003052118 A
- WO 2004016760 A
- WO 2004043980 A
- WO 2004048592 A
- WO 2005001065 A
- WO 2005028636 A
- WO 2005093050 A
- WO 2005093073 A
- WO 2006074005 A
- WO 2006117432 A
- WO 2007071818 A
- WO 2007071820 A
- WO 2008008070 A
- WO 2008008793 A
- US 4435307 A
- US 5457046 A
- US 5648263 A
- US 5686593 A
- US 5691178 A
- US 5763254 A
- US 5776757 A
- WO 9421785 A
- WO 2006078256 A
- WO 2009079210 A
- WO 2005001036 A
- WO 2009042846 A
- WO 2009073709 A
- WO 2009076122 A
- WO 2009073383 A
- WO 9600787 A
- WO 0056900 A
- WO 9533836 A

- WO 0024883 A
- EP 238023 A
- US 20020164730 A
- WO 2006110891 A
- WO 200611899 A
- WO 200611900 A
- WO 2006110901 A
- WO 2006032282 A
- WO 2003062430 A
- US 7361495 B
- WO 2005067531 A
- WO 2004099228 A
- WO 2005056772 A
- US 20060156437 A
- WO 2010080527 A
- US 61373124 B
- US 61373128 B
- US 61373145 B
- US 61373150 B
- US 61373157 B
- US 61373166 B
- US 61373170 B
- US 61373210 B


**Non-patent literature cited in the description**


- **VRIES.** *J. Bacteriol.,* 1998, vol. 180, 243-249
- **VENTURI et al.** Extracellular beta-D-glucosidase from Chaetomium thermophilum var. coprophilum: production, purification and some biochemical properties. *J. Basic Microbiol.,* 2002, vol. 42, 55-66
- **TEERI.** Crystalline cellulose degradation: New insight into the function of cellobiohydrolases. *Trends in Biotechnology,* 1997, vol. 15, 160-167
- **TEERI et al.** hoderma reesei cellobiohydrolases: why so efficient on crystalline cellulose. *Biochem. Soc. Trans.,* 1998, vol. 26, 173-178
- **LEVER et al.** *Anal. Biochem.,* 1972, vol. 47, 273-279
- **VAN TILBEURGH et al.** *FEBS Letters,* 1982, vol. 149, 152-156
- **VAN TILBEURGH ; CLAEYSSENS.** *FEBS Letters,* 1985, vol. 187, 283-288
- **TOMME et al.** *Eur. J. Biochem.,* 1988, vol. 170, 575-581
- **ZHANG et al.** Outlook for cellulase improvement: Screening and selection strategies. *Biotechnology Advances,* 2006, vol. 24, 452-481
- **GHOSE.** Measurement of cellulase activities. *Pure Appl. Chem.,* 1987, vol. 59, 257-68
- **WISELOGEL et al.** Handbook on Bioethanol. Taylor & Francis, 1995, 105-118
- **WYMAN.** *Bioresource Technology,* 1994, vol. 50, 3-16
- **LYND.** *Applied Biochemistry and Biotechnology,* 1990, vol. 24/25, 695-719
- Recent Progress in Bioconversion of Lignocellulosics. **MOSIER et al.** Advances in Biochemical Engineering/Biotechnology. Springer-Verlag, 1999, vol. 65, 23-40
- **ZHANG et al.** *Biotechnology Advances,* 2006, vol. 24, 452-481
- **GHOSE.** *Pure and Appl. Chem.,* 1987, vol. 59, 257-268
- **HENRISSAT B.** A classification of glycosyl hydrolases based on amino-acid sequence similarities. *Biochem. J,* 1991, vol. 280, 309-316
- **HENRISSAT B. ; BAIROCH A.** Updating the sequence-based classification of glycosyl hydrolases. *Biochem. J,* 1996, vol. 316, 695-696
- **SHALLOM, D. ; SHOHAM, Y.** Microbial hemicellulases. *Current Opinion In Microbiology,* 2003, vol. 6 (3), 219-228
- **GHOSE ; BISARIA.** *Pure & Appl. Chem.,* 1987, vol. 59, 1739-1752
- **NIELSEN et al.** *Protein Engineering,* 1997, vol. 10, 1-6
- **NEEDLEMAN ; WUNSCH.** *J. Mol. Biol.,* 1970, vol. 48, 443-453
- **RICE et al.** The European Molecular Biology Open Software Suite. *Trends Genet.,* 2000, vol. 16, 276-277
- **EBRINGEROVA et al.** *Adv. Polym. Sci.,* 2005, vol. 186, 1-67
- **BIELY ; PUCHARD.** Recent progress in the assays of xylanolytic enzymes. *Journal of the Science of Food and Agriculture,* 2006, vol. 86 (11), 1636-1647
- **SPANIKOVA ; BIELY.** Glucuronoyl esterase - Novel carbohydrate esterase produced by Schizophyllum commune. *FEBS Letters,* 2006, vol. 580 (19), 4597-4601
- **HERRMANN ; VRSANSKA ; JURICKOVA ; HIRSCH ; BIELY ; KUBICEK.** The beta-D-xylosidase of Trichoderma reesei is a multifunctional beta-D-xylan xylohydrolase. *Biochemical Journal,* 1997, vol. 321, 375-381
- **BAILEY ; BIELY ; POUTANEN.** Interlaboratory testing of methods for assay of xylanase activity. *Journal of Biotechnology,* 1992, vol. 23 (3), 257-270
- **LEVER.** A new reaction for colorimetric determination of carbohydrates. *Anal. Biochem,* 1972, vol. 47, 273-279
- **BOLTON ; MCCARTHY.** *Proc. Natl. Acad. Sci. USA,* 1962, vol. 48, 1390
- **H. NEURATH ; R.L. HILL.** In, The Proteins. Academic Press, 1979
- **CUNNINGHAM ; WELLS.** *Science,* 1989, vol. 244, 1081-1085

- **HILTON et al.** *J. Biol. Chem.,* 1996, vol. 271, 4699-4708
- **VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **INNIS et al.** PCR: A Guide to Methods and Application. Academic Press, 1990
- **PENTTILA et al.** *Gene,* 1986, vol. 45, 253-263
- **SALOHEIMO et al.** *Gene,* 1988, vol. 63, 11-22
- **OKADA et al.** *Appl. Environ. Microbiol.,* 1988, vol. 64, 555-563
- **SALOHEIMO et al.** *Molecular Microbiology,* 1994, vol. 13, 219-228
- **OOI et al.** *Nucleic Acids Research,* 1990, vol. 18, 5884
- **SAKAMOTO et al.** *Current Genetics,* 1995, vol. 27, 435-439
- **SAARILAHTI et al.** *Gene,* 1990, vol. 90, 9-14
- **DAN.** *J. Biol. Chem.,* 2000, vol. 275, 4973-4980
- **KAWAGUCHI et al.** *Gene,* 1996, vol. 173, 287-288
- **HENRISSAT B.** A classification of glycosyl hydrolases based on amino-acid sequence similarities. *Biochem. J.,* 1991, vol. 280, 309-316
- **HENRISSAT B. ; BAIROCH A.** Updating the sequence-based classification of glycosyl hydrolases. *Biochem. J.,* 1996, vol. 316, 695-696
- More Gene Manipulations in Fungi. Academic Press, 1991
- **BAILEY, J.E. ; OLLIS, D.F.** Biochemical Engineering Fundamentals. McGraw-Hill Book Company, 1986
- **VILLA-KAMAROFF et al.** *Proc. Natl. Acad. Sci. USA,* 1978, vol. 75, 3727-3731
- **DEBOER.** *Proc. Natl. Acad. Sci. USA,* 1983, vol. 80, 21-25
- **GILBERT.** *Scientific American,* 1980, vol. 242, 74-94
- **ROMANOS et al.** *Yeast,* 1992, vol. 8, 423-488
- **GUO ; SHERMAN.** *Mol. Cellular Biol.,* 1995, vol. 15, 5983-5990
- **SIMONEN ; PALVA.** *Microbiological Reviews,* 1993, vol. 57, 109-137
- **GEMS et al.** *Gene,* 1991, vol. 98, 61-67
- **CULLEN et al.** *Nucleic Acids Res.,* 1987, vol. 15, 9163-9175
- **CHANG ; COHEN.** *Mol. Gen. Genet.,* 1979, vol. 168, 111-115
- **YOUNG ; SPIZIZEN.** *J. Bacteriol.,* 1961, vol. 81, 823-829
- **DUBNAU ; DAVIDOFF-ABELSON.** *J. Mol. Biol.,* 1971, vol. 56, 209-221
- **SHIGEKAWA ; DOWER.** *Biotechniques,* 1988, vol. 6, 742-751
- **KOEHLER ; THORNE.** *J. Bacteriol.,* 1987, vol. 169, 5271-5278
- **HANAHAN.** *J. Mol. Biol.,* 1983, vol. 166, 557-580
- **DOWER et al.** *Nucleic Acids Res.,* 1988, vol. 16, 6127-6145
- **GONG et al.** *Folia Microbiol. (Praha),* 2004, vol. 49, 399-405
- **MAZODIER et al.** *J. Bacteriol.,* 1989, vol. 171, 3583-3585
- **BURKE et al.** *Proc. Natl. Acad. Sci. USA,* 2001, vol. 98, 6289-6294
- **CHOI.** *J. Microbiol. Methods,* 2006, vol. 64, 391-397
- **PINEDO ; SMETS.** *Appl. Environ. Microbiol.,* 2005, vol. 71, 51-57
- **PERRY ; KURAMITSU.** *Infect. Immun.,* 1981, vol. 32, 1295-1297
- **CATT ; JOLLICK.** *Microbios,* 1991, vol. 68, 189-207
- **BUCKLEY et al.** *Appl. Environ. Microbiol.,* 1999, vol. 65, 3800-3804
- **CLEWELL.** *Microbiol. Rev.,* 1981, vol. 45, 409-436
- **HAWKSWORTH et al.** In, Ainsworth and Bisby's Dictionary of The Fungi. CAB International, University Press, 1995
- Biology and Activities of Yeast. Symposium Series, 1980
- **YELTON.** *Proc. Natl. Acad. Sci. USA,* 1984, vol. 81, 1470-1474
- **CHRISTENSEN et al.** *Bio/Technology,* 1988, vol. 6, 1419-1422
- **MALARDIER.** *Gene,* 1989, vol. 78, 147-156
- Guide to Yeast Genetics and Molecular Biology. **BECKER ; GUARENTE.** Methods in Enzymology. Academic Press, Inc, vol. 194, 182-187
- **ITO et al.** *J. Bacteriol.,* 1983, vol. 153, 163
- **HINNEN et al.** *Proc. Natl. Acad. Sci. USA,* 1978, 75
- Protein Purification. VCH Publishers, 1989
- Cellulose bioconversion technology. **PHILIPPIDIS, G. P.** Handbook on Bioethanol: Production and Utilization. Taylor & Francis, 1996, 179-212

- **SHEEHAN, J. ; HIMMEL, M.** Enzymes, energy and the environment: A strategic perspective on the U.S. Department of Energy's research and development activities for bioethanol. *Biotechnol. Prog.,* 1999, vol. 15, 817-827
- **LYND, L. R. ; WEIMER, P. J. ; VAN ZYL, W. H. ; PRETORIUS, I. S.** Microbial cellulose utilization: Fundamentals and biotechnology. *Microbiol. Mol. Biol. Reviews,* 2002, vol. 66, 506-577
- **FERNANDA DE CASTILHOS CORAZZA ; FLÁVIO FARIA DE MORAES ; GISELLA MARIA ZANIN ; IVO NEITZEL.** mal control in fed-batch reactor for the cellobiose hydrolysis. *Acta Scientiarum. Technology,* 2003, vol. 25, 33-38
- **GUSAKOV, A. V. ; SINITSYN, A. P.** Kinetics of the enzymatic hydrolysis of cellulose: 1. A mathematical model for a batch reactor process. *Enz. Microb. Technol.,* 1985, vol. 7, 346-352
- **RYU, S. K. ; LEE, J. M.** Bioconversion of waste cellulose by using an attrition bioreactor. *Biotechnol. Bioeng.,* 1983, vol. 25, 53-65
- **GUSAKOV, A. V. ; SINITSYN, A. P. ; DAVYDKIN, I. Y. ; DAVYDKIN, V. Y. ; PROTAS, O. V.** Enhancement of enzymatic cellulose hydrolysis using a novel type of bioreactor with intensive stirring induced by electromagnetic field. *Appl. Biochem. Biotechnol.,* 1996, vol. 56, 141-153
- **CHANDRA et al.** Substrate pretreatment: The key to effective enzymatic hydrolysis of lignocellulosics. *Adv. Biochem. Engin./Biotechnol.,* 2007, vol. 108, 67-93
- **GALBE ; ZACCHI.** Pretreatment of lignocellulosic materials for efficient bioethanol production. *Adv. Biochem. Engin. / Biotechnol.,* 2007, vol. 108, 41-65
- **HENDRIKS ; ZEEMAN.** Pretreatments to enhance the digestibility of lignocellulosic biomass. *Bioresource Technol.,* 2009, vol. 100, 10-18
- **MOSIER et al.** Features of promising technologies for pretreatment of lignocellulosic biomass. *Bioresource Technol.,* 2005, vol. 96, 673-686
- **TAHERZADEH ; KARIMI.** tment of lignocellulosic wastes to improve ethanol and biogas production: A review. *Int. J. of Mol. Sci.,* 2008, vol. 9, 1621-1651
- **YANG ; WYMAN.** Pretreatment: the key to unlocking low-cost cellulosic ethanol. *Biofuels Bioproducts and Biorefining-Biofpr.,* 2008, vol. 2, 26-40
- **DUFF ; MURRAY.** *Bioresource Technology,* 1996, vol. 855, 1-33
- **GALBE ; ZACCHI.** *Appl. Microbiol. Biotechnol.,* 2002, vol. 59, 618-628
- **BALLESTEROS et al.** *Appl. Biochem. Biotechnol.,* 2006, vol. 129-132, 496-508
- **VARGA.** *Appl. Biochem. Biotechnol.,* 2004, vol. 113-116, 509-523
- **SASSNER.** *Enzyme Microb. Technol.,* 2006, vol. 39, 756-762
- **SCHELL.** *Bioresource Technol.,* 2004, vol. 91, 179-188
- **LEE et al.** *Adv. Biochem. Eng. Biotechnol.,* 1999, vol. 65, 93-115
- **WYMAN et al.** *Bioresource Technol.,* 2005, vol. 96, 1959-1966
- **MOSIER et al.** *Bioresource Technol.,* 2005, vol. 96, 673-686
- **SCHMIDT ; THOMSEN.** *Bioresource Technol,* 1998, vol. 64, 139-151
- **PALONEN et al.** *Appl. Biochem. Biotechnol.,* 2004, vol. 117, 1-17
- **VARGA et al.** *Biotechnol. Bioeng.,* 2004, vol. 88, 567-574
- **MARTIN et al.** *J. Chem. Technol. Biotechnol.,* 2006, vol. 81, 1669-1677
- **GOLLAPALLI et al.** *Appl. Biochem. Biotechnol.,* 2002, vol. 98, 23-35
- **CHUNDAWAT et al.** *Biotechnol. Bioeng.,* 2007, vol. 96, 219-231
- **ALIZADEH et al.** *Appl. Biochem. Biotechnol.,* 2005, vol. 121, 1133-1141
- **TEYMOURI et al.** *Bioresource Technol.,* 2005, vol. 96, 2014-2018
- **PAN et al.** *Biotechnol. Bioeng.,* 2005, vol. 90, 473-481
- **PAN et al.** *Biotechnol. Bioeng.,* 2006, vol. 94, 851-861
- **KURABI et al.** *Appl. Biochem. Biotechnol.,* 2005, vol. 121, 219-230
- **SCHELL et al.** *Appl. Biochem. and Biotechnol.,* 2003, vol. 105 (108), 69-85
- **MOSIER et al.** *Bioresource Technology,* 2005, vol. 96, 673-686
- Pretreatment of biomass. **HSU, T.-A.** Handbook on Bioethanol: Production and Utilization. Taylor & Francis, 1996, 179-212
- **GHOSH ; SINGH.** emical and biological treatments for enzymatic/microbial conversion of cellulosic biomass. *Adv. Appl. Microbiol.,* 1993, vol. 39, 295-333
- eating lignocellulosic biomass: a review. **MCMILLAN, J. D.** Enzymatic Conversion of Biomass for Fuels Production. ACS Symposium Series, 1994, 566
- Ethanol production from renewable resources. **GONG, C. S. ; CAO, N. J. ; DU, J. ; TSAO, G. T.** Advances in Biochemical Engineering/Biotechnology. Springer-Verlag Berlin Heidelberg, 1999, vol. 65, 207-241
- **OLSSON ; HAHN-HAGERDAL.** Fermentation of lignocellulosic hydrolysates for ethanol production. *Enz. Microb. Tech.,* 1996, vol. 18, 312-331
- **VALLANDER ; ERIKSSON.** Production of ethanol from lignocellulosic materials: State of the art. *Adv. Biochem. Eng./Biotechnol.,* 1990, vol. 42, 63-95

- **LIN et al.** *Appl. Microbiol. Biotechnol.,* 2006, vol. 69, 627-642
- **CHEN ; HO.** Cloning and improving the expression of Pichia stipitis xylose reductase gene. *Saccharomyces cerevisiae, Appl. Biochem. Biotechnol.,* 1993, vol. 39-40, 135-147
- **HO et al.** Genetically engineered Saccharomyces yeast capable of effectively cofermenting glucose and xylose. *Appl. Environ. Microbiol.,* 1998, vol. 64, 1852-1859
- **KOTTER ; CIRIACY.** Xylose fermentation by Saccharomyces cerevisiae. *Appl. Microbiol. Biotechnol.,* 1993, vol. 38, 776-783
- **WALFRIDSSON et al.** Xylose-metabolizing Saccharomyces cerevisiae strains overexpressing the TKL1 and TAL1 genes encoding the pentose phosphate pathway enzymes transketolase and transaldolase. *Appl. Environ. Microbiol.,* 1995, vol. 61, 4184-4190
- **KUYPER et al.** Minimal metabolic engineering of Saccharomyces cerevisiae for efficient anaerobic xylose fermentation: a proof of principle. *FEMS Yeast Research,* 2004, vol. 4, 655-664
- **BEALL et al.** Parametric studies of ethanol production from xylose and other sugars by recombinant. *Escherichia coli, Biotech. Bioeng,* 1991, vol. 38, 296-303
- **INGRAM et al.** Metabolic engineering of bacteria for ethanol production. *Biotechnol. Bioeng.,* 1998, vol. 58, 204-214
- **ZHANG et al.** Metabolic engineering of a pentose metabolism pathway in ethanologenic. *Zymomonas mobilis, Science,* 1995, vol. 267, 240-243
- **DEANDA et al.** Development of an arabinose-fermenting Zymomonas mobilis strain by metabolic pathway engineering. *Appl. Environ. Microbiol.,* 1996, vol. 62, 4465-4470
- The Alcohol Textbook. Nottingham University Press, 1999
- **ALFENORE et al.** Improving ethanol production and viability of Saccharomyces cerevisiae by a vitamin feeding strategy during fed-batch process. Springer-Verlag, 2002
- Ethanol production from renewable resources. **GONG, C. S. ; CAO, N. J. ; DU, J. ; TSAO, G. T.** Advances in Biochemical Engineering/Biotechnology. Springer-Verlag, 1999, vol. 65, 207-241
- **SILVEIRA, M. M. ; JONAS, R.** The biotechnological production of sorbitol. *Appl. Microbiol. Biotechnol.,* 2002, vol. 59, 400-408
- **NIGAM, P. ; SINGH, D.** Processes for fermentative production of xylitol - a sugar substitute. *Process Biochemistry,* 1995, vol. 30 (2), 117-124
- **EZEJI, T. C. ; QURESHI, N. ; BLASCHEK, H. P.** Production of acetone, butanol and ethanol by Clostridium beijerinckii BA101 and in situ recovery by gas stripping. *World Journal of Microbiology and Biotechnology,* 2003, vol. 19 (6), 595-603
- **RICHARD, A. ; MARGARITIS, A.** Empirical modeling of batch fermentation kinetics for poly(glutamic acid) production and other microbial biopolymers. *Biotechnology and Bioengineering,* 2004, vol. 87 (4), 501-515
- **KATAOKA, N. ; A. MIYA ; K. KIRIYAMA.** Studies on hydrogen production by continuous culture system of hydrogen-producing anaerobic bacteria. *Water Science and Technology,* 1997, vol. 36 (6-7), 41-47
- **GUNASEELAN V.N.** *Biomass and Bioenergy,* 1997, vol. 13 (1-2), 83-114
- **CHEN, R. ; LEE, Y. Y.** Membrane-mediated extractive fermentation for lactic acid production from cellulosic biomass. *Appl. Biochem. Biotechnol.,* 1997, vol. 63-65, 435-448
- Productive cellulase adsorption on cellulose. **DING ; XU.** Lignocellulose Biodegradation. American Chemical Society, 2004, vol. 889, 154-169
- **LANGSTON et al.** *Biochim. Biophys. Acta Proteins Proteomics,* 2006, vol. 1764, 972-978
- **ZHANG ; LYND.** *Biomacromolecules,* 2005, vol. 6, 1510-151
- ChemSpider. Royal Society of Chemistry
- **ZHANG et al.** *Biomacromolecules,* 2006, vol. 7, 644-648
- **THOMAS MODEL 10 WILEY MILL.** Thomas Scientific. Swedesboro